(19) Europäisches Patentamt
European Patent Office
Office européen des brevets

(11) EP 4 188 551 B1

(12) # EUROPEAN PATENT SPECIFICATION

(45) Date of publication and mention of the grant of the patent:
**18.12.2024 Bulletin 2024/51**

(21) Application number: **21758611.4**

(22) Date of filing: **27.07.2021**

(51) International Patent Classification (IPC):
***A61P 35/00*** *(2006.01)* ***C07D 471/04*** *(2006.01)*
***A61K 31/437*** *(2006.01)*

(52) Cooperative Patent Classification (CPC):
**C07D 471/04; A61P 35/00**

(86) International application number:
**PCT/EP2021/070994**

(87) International publication number:
**WO 2022/023339 (03.02.2022 Gazette 2022/05)**

(54) **ARYL SUBSTITUTED PYRROLO-PYRIDINONES AND THERAPEUTIC USES THEREOF**

ARYLSUBSTITUIERTE PYRROLOPYRIDINONE UND IHRE THERAPEUTISCHE VERWENDUNG

PYRROLO-PYRIDINONES SUBSTITUÉES PAR ARYLE ET LEURS UTILISATIONS THÉRAPEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2020 US 202063058229 P**

(43) Date of publication of application:
**07.06.2023 Bulletin 2023/23**

(73) Proprietors:
- **Bayer Aktiengesellschaft**
  **51373 Leverkusen (DE)**
- **The Broad Institute, Inc.**
  **Cambridge, MA 02142 (US)**
- **Dana-Farber Cancer Institute, Inc.**
  **Boston, Massachusetts 02215 (US)**

(72) Inventors:
- **SCHULZE, Volker**
  **16562 Hohen Neuendorf, OT Bergfelde (DE)**
- **MENGEL, Anne**
  **13187 Berlin (DE)**
- **FARIA ALVARES DE LEMOS, Adelaide Clara**
  **10823 Berlin (DE)**
- **RAUH, Ulrike**
  **13465 Berlin (DE)**
- **BÖMER, Ulf**
  **16548 Glienicke (DE)**
- **HILLIG, Roman**
  **12159 Berlin (DE)**
- **LECHNER, Christian**
  **10117 Berlin (DE)**
- **MORTIER, Jeremie, Xavier G.**
  **12055 Berlin (DE)**
- **KAULFUSS, Stefan**
  **10245 Berlin (DE)**
- **CORSELLO, Steven**
  **Boston, MA 02215 (US)**
- **HANDING, Kartarzyna**
  **Cambridge, MA 02142 (US)**
- **MADEC, Amael**
  **Cambridge, MA 02142 (US)**
- **FURST, Laura**
  **Cambridge, MA 02142 (US)**
- **SHEKHAR, Mrinal**
  **Cambridge, MA 02142 (US)**
- **MCKINNEY, David**
  **Cambridge, MA 02142 (US)**

(74) Representative: **HGF**
**HGF Limited**
**1 City Walk**
**Leeds LS11 9DX (GB)**

(56) References cited:
**WO-A1-2016/100166 WO-A1-2016/120196**
**WO-A1-2020/161257**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 188 551 B1

## Description

## INTRODUCTION

The invention relates to substituted 2-[2-(acylamino)pyridin-4-yl]-4H-pyrrolo[3,2-c]pyridin-4-one compounds, a process for their production and uses thereof.

## BACKGROUND OF THE INVENTION

One of the most fundamental characteristics of cancer cells is their ability to sustain chronic proliferation whereas in normal tissues the entry into and progression through the cell division cycle is tightly controlled to ensure a homeostasis of cell number and maintenance of normal tissue function. Loss of proliferation control was emphasized as one of the six hallmarks of cancer [Hanahan D and Weinberg RA, Cell 100, 57, 2000; Hanahan D and Weinberg RA, Cell 144, 646, 2011].

The members of the casein kinase 1 (CSNK1) family are highly conserved and are expressed in many eukaryotes ranging from yeast to humans. Mammalian CSNK1 isoforms (α, β, γ, δ, ε) and their splice variants are involved in diverse cellular processes including membrane trafficking, circadian rhythm, cell cycle progression, chromosome segregation, apoptosis and cellular differentiation. Mutations and deregulation of CSNK1 expression and activity have been linked to proliferative diseases such as cancer (Knippschild, Onkologie 2005;28:508-514).

CSNK1 substrates are enzymes, transcription factors, splice factors, cytoskeleton proteins, receptors, membrane-associated proteins and cell signaling proteins. Since recognition motifs for CSNK1 are found on most cellular proteins, more than 140 in vitro and in vivo substrates have been reported thus far (Knippschild et al., Front Oncol. 2014 May 19;4:96). Several known substrates especially of the CSNK1α and δ isoforms are involved in oncogenic signaling pathways as Wnt/β-catenin (β-catenin; dishevelled (DVL); adenomatous polyposis coli (APC); nuclear factor of activated Tcells, cytoplasmic 3 (NFATC3)), p53 (p53; p53/E3 ubiquitin-protein ligase Mdm2 (MDM2)), PI3K/AKT (forkhead box protein O1 (Foxo1)), death receptor signaling (Fas-associated death domain protein (FADD); and BH3-interactive domain death agonist (BID)) (Schittek and Sinnberg Molecular Cancer 2014, 13:231). A distinctive feature of CSNK1 family members is their exclusive need of ATP to phosphorylate their substrates and their independency of other co-factors.

CSNK1α plays a role in the mitotic spindle formation during cell division and in DNA repair mechanisms, and participates in RNA metabolism. Antibodies specific for CSNK1α block cell cycle progression during M phase in mouse oocytes, which indicates that CSNK1α is required for proper cell cycle progression in these cells. CSNK1α can be found at the centrosomes, microtubule asters and the kinetochore. Similarly, CSNK1α regulates apoptotic signaling pathways, however, there seems to be cell type-specific differences. CSNK1α has been shown to have an anti-apoptotic function in the extrinsic apoptosis pathway. Its inhibition increased Fas-induced apoptosis in Hela cells, whereas the overexpression of CSNK1α delayed cell death, caused by the phosphorylation of BID, prevented the caspase 8 dependent cleavage of BID. In addition, CSNK1α inhibits TRAIL induced apoptosis by modification of the TNF receptor or FADD at the death-inducing signaling complex (DISC). Therefore, downregulation of CSNK1α leads to an enhancement of TRAIL-induced cell death. Likewise, CSNK1α promotes cell survival by interacting with the retinoid X receptor (RXR). Downregulation of CSNK1α enhances the apoptotic effect of RXR agonists (Schittek and Sinnberg, Molecular Cancer 2014, 13:231).

Knockdown or downregulation of CSNK1α in the intestinal epithelium of mice, in human colon cancers or in leukemia cells triggers p53 activation. Similarly, one study showed that CSNK1α stably associates with MDM2, stimulates MDM2-p53 binding, and cooperates with MDM2 to inactivate p53. These data suggest that inhibition of CSNK1α activity increases p53 activity. The knockdown of CSNK1α induces p53 transcriptional activity by reducing the inhibitory effect of MDM2 for p53 since MDM2 phosphorylation is necessary for interaction with p53 (Schittek and Sinnberg, Molecular Cancer 2014, 13:231).

Ribosomal protein S6 (RPS6) is a critical component of the 40S ribosomal subunit that mediates translation initiation. RPS6 activity is regulated by phosphorylation by CSNK1α, which phosphorylates serine residue 247, enhancing the phosphorylation of upstream sites (Hutchinson et al., JBC, 2011, 286, 10, 8688). CSNK1α inhibition leads to dramatic reduction in RPS6 phosphorylation and activation of p53, resulting in selective elimination of solid tumor and AML cells. Pharmacological inhibition of CSNK1α in p53wt colon and lung carcinoma as well as in AML induces p53 accumulation along with apoptosis. Targeting of CSNK1α provides a potential approach to the therapeutic activation of p53 in AML, a disorder predominantly associated with non-mutated p53 (Jaras et al., J. Exp. Med. 2014, 211, 4, 605).

CSNK1α is an essential participant in the aberrant NF-kB activity required for ABC DLBCL subtype survival. CSNK1α knockdown is specifically lethal to ABC DLBCL cells (Bidere, Nature, 458, 5 March 2009). Pharmacological inhibition of CSNK1α will specifically kill ABC-DLBCL due to the blocking of the CARD1 1-Bcl-10-MALT1 complex (CBM complex).

Thus, pharmacological inhibition of CSNK1α represents a new approach for the treatment of proliferative disorders, including solid tumors such as carcinomas, sarcomas, leukaemias and lymphoid malignancies or other disorders,

associated with uncontrolled cellular proliferation.

Due to the fact that especially cancer disease, being expressed by uncontrolled proliferative cellular processes in tissues of different organs of the human- or animal body, is still not considered to be a controlled disease in that sufficient drug therapies do not already exist, there is a strong need to provide further new therapeutically useful drugs, preferably those inhibiting new targets and providing new therapeutic options.

Therefore, inhibitors of Casein kinase 1 alpha and/or delta represent valuable compounds as single agent therapies that in some instances, can complement other therapeutic options either as single agents or in combination with other drugs.

WO 2016/120196 discloses 4H-pyrrolo[3,2-c]pyridin-4-one derivatives, which may be useful as Bub1 kinase inhibitors.

WO 2016/100166 disloses substituted dichydro-1H-pyrrolo[3,2-C]pyridine-4(5H)-ones, which may be useful as RIPK3 inhibitors.

WO 2014/149164 discloses MK2 inhibitors and uses thereof.

WO 99/20624 discloses p38 MAP kinase inhibitors.

## SUMMARY OF THE INVENTION

The invention provides compounds that inhibit Casein kinase 1 alpha and/or Casein kinase 1 delta.

It has now been found that compounds of the present invention have surprising and advantageous properties.

In particular, compounds of the present invention have surprisingly been found to effectively inhibit CSNK1A1. In certain embodiments, compounds of the present invention display an $IC_{50}$ of below 100 nM in a CSNK1A1 kinase assay in the presence of 1 $\mu$M ATP. Furthermore, in certain embodiments, compounds of the present invention additionally show low inhibition of wild type-EGFR kinase. In certain embodiments, compounds of the present invention are less potent than 600 nM in a wild type-EGFR kinase assay in the presence of 2 mM ATP. In certain embodiments, compounds of the present invention display an $IC_{50}$ below 100 nM in a CSNK1A1 kinase assay in the presence of 1 mM ATP and are less potent than 100 nM in a wild type-EGFR kinase assay in the presence of 2 mM ATP.

In general, reduced or no inhibition of other kinases and specifically reduced or no inhibition of wild type EGFR kinase, in particular in a high ATP assay (e.g. with a concentration of 2 mM ATP), is considered to be relevant in the clinical setting to avoid/reduce unwanted side effects associated with the inhibition of said wild type EGFR kinase, such as, for example, skin rash and GI toxicity.

## BRIEF DESCRIPTION OF THE FIGURE

Figure 1 shows an electron density map of the compound of Intermediate 42. For clarity only the ligand atoms are shown. Carbon atom C6 unambiguously features the (S)-configuration.

## DESCRIPTION OF THE INVENTION

The invention is set out in the appended set of claims.

In accordance with a first aspect, the invention relates to compounds of formula (I),

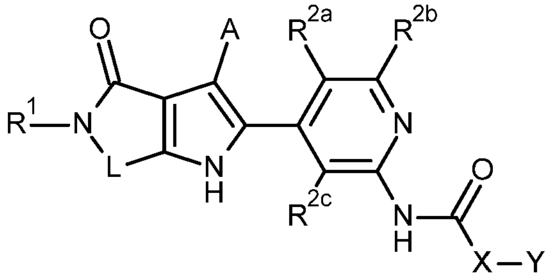

(I)

in which:

$R^1$ represents hydrogen or a group selected from $C_1$-$C_4$-alkyl, $C_3$-$C_6$-cycloalkyl, $C_3$-$C_6$-cycloalkyl-$C_1$-$C_4$-alkyl-, $C_1$-$C_3$-alkoxy-$C_1$-$C_3$-alkyl-, and $C_1$-$C_5$-hydroxyalkyl, wherein said groups are each independently optionally substituted, one or more times, with halogen;

$R^{2a}$ represents hydrogen, hydroxy, halogen, cyano, $-NH_2$, -NHMe, $-NMe_2$, $C_1$-$C_2$-alkoxy, $C_1$-$C_2$-haloalkyl, or $C_1$-$C_2$-haloalkoxy;

$R^{2b}$ represents hydrogen, hydroxy, halogen, cyano, $-NH_2$, $-NHR^9$, or-$NMe_2$;

$R^{2c}$ represents hydrogen, halogen, cyano, $-NMe_2$, $C_1$-$C_2$-alkoxy, or $C_1$-$C_2$-haloalkoxy;

L represents

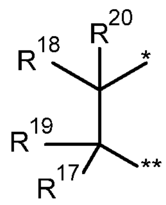

or

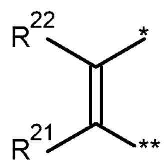

wherein * represents the point of direct attachment to the adjacent N-atom and ** represents the point of direct attachment to the adjacent C-atom;

$R^{17}$ represents hydrogen, $C_1$-$C_3$-alkyl, $C_1$-$C_3$-alkoxy, $C_1$-$C_3$-haloalkyl, or $C_3$-$C_6$-cycloalkyl-$C_1$-$C_4$-alkyl-;

$R^{18}$ represents hydrogen, $C_1$-$C_3$-alkyl, $C_1$-$C_3$-haloalkyl, or $C_3$-$C_6$-cycloalkyl-$C_1$-$C_4$-alkyl-, or

$R^{17}$ and $R^{18}$, together with the carbon atoms to which they are attached, form a $C_3$-$C_6$-cycloalkyl ring;

$R^{19}$ represents hydrogen, $C_1$-$C_3$-alkyl, $C_1$-$C_3$-haloalkyl, or $C_3$-$C_6$-cycloalkyl-$C_1$-$C_4$-alkyl-;

$R^{20}$ represents hydrogen, $C_1$-$C_3$-alkyl, $C_1$-$C_3$-alkoxy, $C_1$-$C_3$-haloalkyl, or $C_3$-$C_6$-cycloalkyl-$C_1$-$C_4$-alkyl-, or

$R^{18}$ and $R^{20}$, together with the carbon atom to which they are attached, form a $C_3$-$C_6$-cycloalkyl ring;

$R^{21}$ represents hydrogen, halogen, $C_1$-$C_3$-alkyl, $C_1$-$C_3$-haloalkyl, or $C_3$-$C_6$-cycloalkyl-$C_1$-$C_4$-alkyl-;

$R^{22}$ represents hydrogen, $C_1$-$C_3$-alkyl, $C_1$-$C_3$-haloalkyl, or $C_3$-$C_6$-cycloalkyl-$C_1$-$C_4$-alkyl-;

X represents methylene or ethylene, wherein said methylene and ethylene groups are each independently optionally substituted, one or more times, with $R^3$;

Y represents phenyl or a heteroaryl group, wherein said phenyl and heteroaryl groups are each independently optionally substituted, one or more times, with $R^4$;

$R^3$ represents hydroxy, halogen, cyano, $C_1$-$C_3$-alkyl, $C_1$-$C_3$-alkoxy, $C_1$-$C_3$-haloalkyl, $C_1$-$C_2$-haloalkoxy, $C_3$-$C_4$-cycloalkyl, $-NHR^6$, $R^5O$-$C_1$-$C_3$-alkyl, $R^6R^7N$-$C_1$-$C_5$-alkyl, or $R^6R^7N$-X'-C($R^{10}$)($R^{11}$)-$C_1$-$C_2$-alkyl-;

X' represents methylene or ethylene;

$R^4$ represents hydroxy, halogen, cyano, $C_1$-$C_4$-alkyl, $C_1$-$C_4$-alkoxy, $C_1$-$C_4$-haloalkyl, $C_1$-$C_4$-haloalkoxy, $-NMe_2$, $-CO_2R^{14}$, $-CONR^{15}R^{16}$, or $-SO_2Me$;

$R^5$ represents hydrogen, $C_1$-$C_2$-alkyl, $C_1$-$C_2$-haloalkyl, or $C_3$-$C_4$-cycloalkyl;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl, or

$R^6$ and $R^7$, together with the nitrogen atom to which they are attached, represent a 4- to 7-membered heterocyclic ring, wherein said heterocyclic ring optionally contains one or more further heteroatoms selected from N, O, S, or groups selected from C(=O), S(=O), and $SO_2$, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;

A represents a group selected from:

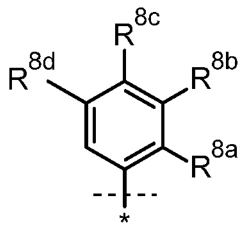, 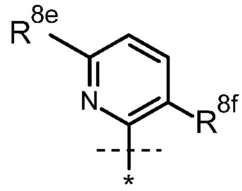, 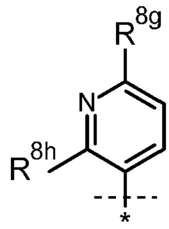, 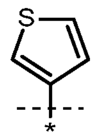,

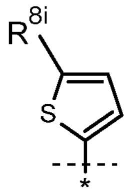, 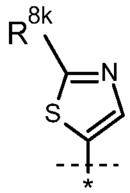, 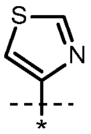, 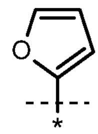, 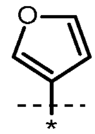, and 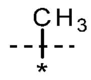,

wherein * indicates the point of attachment of said group to the rest of the compound of formula (I);

$R^{8a}$ represents hydrogen, hydroxy, halogen, cyano, $C_1$-$C_2$-alkyl, or $C_1$-$C_2$-haloalkyl;

$R^{8b}$ represents hydrogen, hydroxy, halogen, cyano, $C_1$-$C_2$-alkyl, or $C_1$-$C_2$-haloalkyl;

$R^{8c}$ represents hydrogen, hydroxy, halogen, $C_1$-$C_2$-alkyl, or $C_1$-$C_2$-haloalkyl;

$R^{8d}$ represents hydrogen, hydroxy, halogen, cyano, $C_1$-$C_2$-alkyl, or $C_1$-$C_2$-haloalkyl;

$R^{8e}$ represents hydrogen, hydroxy, halogen, cyano, $C_1$-$C_2$-alkyl, or $C_1$-$C_2$-haloalkyl;

$R^{8f}$ represents hydrogen, hydroxy, halogen, cyano, $C_1$-$C_2$-alkyl, or $C_1$-$C_2$-haloalkyl;

$R^{8g}$ represents hydrogen, hydroxy, amino, halogen, cyano, $C_1$-$C_2$-alkyl, or $C_1$-$C_2$-haloalkyl;

$R^{8h}$ represents hydrogen, hydroxy, halogen, cyano, $C_1$-$C_2$-alkyl, or $C_1$-$C_2$-haloalkyl;

$R^{8i}$ represents hydrogen, hydroxy, halogen, cyano, $C_1$-$C_2$-alkyl, or $C_1$-$C_2$-haloalkyl;

$R^{8k}$ represents hydrogen, hydroxy, halogen, cyano, $C_1$-$C_2$-alkyl, or $C_1$-$C_2$-haloalkyl;

$R^9$ represents $C_1$-$C_3$-alkyl or cyclopropyl;

$R^{9a}$ represents $C_1$-$C_3$-alkyl or cyclopropyl;

$R^{10}$ represents methyl, halogen, or hydroxy;

$R^{11}$ represents methyl, halogen, or hydroxy;

$R^{14}$ represents hydrogen, methyl, or ethyl;

$R^{15}$ represents hydrogen, methyl, or ethyl;

$R^{16}$ represents hydrogen, methyl, ethyl, methoxyethyl, or dimethylaminoethyl, or

$R^{15}$ and $R^{16}$, together with the nitrogen atom to which they are attached, represent a 5- to 6-membered heterocyclic ring, wherein said heterocyclic ring optionally contains one or two further heteroatoms selected from N, O, S, or groups selected from C(=O), S(=O), and $SO_2$;

or an N-oxide, a salt, a tautomer or a stereoisomer of said compound, or a salt of said N-oxide, tautomer or stereoisomer.

In accordance with an embodiment of the first aspect, the present invention relates to compounds of formula (I) supra, wherein:

$R^1$ represents hydrogen or a group selected from $C_1$-$C_2$-alkyl and $C_3$-$C_4$-cycloalkyl, wherein said groups are each independently optionally substituted, one or more times, with halogen;

$R^{2a}$ represents hydrogen or halogen;

$R^{2b}$ represents hydrogen, halogen, -$NH_2$, or-$NHR^9$;

$R^{2c}$ represents hydrogen or halogen;

L represents

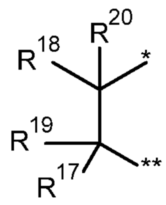

or

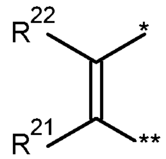

wherein * represents the point of direct attachment to the adjacent N-atom and ** represents the point of direct attachment to the adjacent C-atom;

$R^{17}$ represents hydrogen, $C_1$-$C_3$-alkyl, $C_1$-$C_3$-haloalkyl, or $C_3$-$C_6$-cycloalkyl-$C_1$-$C_4$-alkyl-;

$R^{18}$ represents hydrogen, $C_1$-$C_3$-alkyl, $C_1$-$C_3$-haloalkyl, or $C_3$-cycloalkyl-$C_1$-alkyl-, or

$R^{17}$ and $R^{18}$, together with the carbon atoms to which they are attached, form a $C_3$-$C_5$-cycloalkyl ring;

$R^{19}$ represents hydrogen, $C_1$-$C_3$-alkyl, $C_1$-$C_3$-haloalkyl, or $C_3$-$C_6$-cycloalkyl-$C_1$-$C_4$-alkyl-;

$R^{20}$ represents hydrogen or $C_1$-$C_3$-alkyl, or

$R^{18}$ and $R^{20}$, together with the carbon atom to which they are attached, form a $C_3$-$C_6$-cycloalkyl ring;

$R^{21}$ represents hydrogen, halogen, $C_1$-$C_3$-alkyl, $C_1$-$C_3$-haloalkyl, or $C_3$-$C_6$-cycloalkyl-$C_1$-$C_4$-alkyl-;

$R^{22}$ represents hydrogen or $C_1$-$C_3$-alkyl;

X represents methylene,
wherein said methylene group is optionally substituted, one or more times, with $R^3$;

Y represents phenyl or a heteroaryl group,
wherein said phenyl and heteroaryl groups are each independently optionally substituted, one or more times, with $R^4$;

$R^3$ represents $C_1$-$C_3$-alkyl, $C_1$-$C_3$-haloalkyl, -$NHR^6$, $R^5O$-$C_1$-$C_3$-alkyl, or $R^6R^7N$-$C_1$-$C_4$-alkyl;

$R^4$ represents, halogen, $C_1$-$C_4$-alkyl, $C_1$-$C_4$-haloalkyl, $C_1$-$C_4$-haloalkoxy, -$CO_2R^{14}$, -$CONR^{15}R^{16}$, or -$SO_2Me$;

$R^5$ represents hydrogen, $C_1$-$C_2$-alkyl, or $C_1$-$C_2$-haloalkyl;

$R^6$ represents hydrogen, $C_1$-$C_2$-alkyl, $C_1$-$C_2$-haloalkyl, or $C_3$-$C_4$-cycloalkyl;

$R^7$ represents hydrogen, $C_1$-$C_2$-alkyl, $C_1$-$C_2$-haloalkyl, $C_3$-$C_4$-cycloalkyl, or -$CO_2$-$C_1$-$C_4$-alkyl, or

$R^6$ and $R^7$, together with the nitrogen atom to which they are attached, represent a 4- to 7-membered heterocyclic ring, wherein said heterocyclic ring optionally contains one or more further heteroatoms selected from N, O, S, and is independently optionally substituted, one or more times, with halogen, $C_1$-$C_3$-alkyl, $C_3$-cycloalkyl-$C_1$-$C_2$-alkyl-,-$NH_2$, -$NHR^9$, -$NR^9R^{9a}$, or hydroxy;

A represents a group selected from:

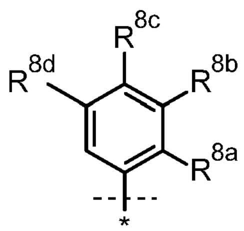
,
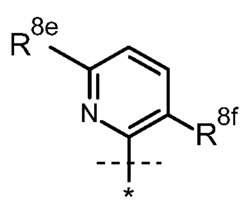
,
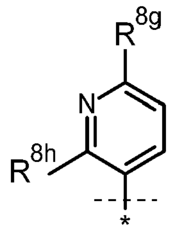
,
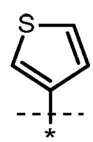
,

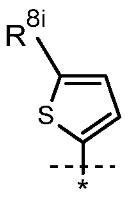 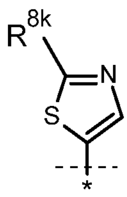, 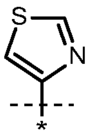, 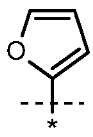, 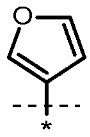, and 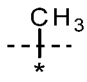,

wherein * indicates the point of attachment of said group to the rest of the formula (I);

$R^{8a}$ represents hydrogen, hydroxy, halogen, cyano, or $C_1$-alkyl;

$R^{8b}$ represents hydrogen, hydroxy, halogen, or $C_1$-alkyl;

$R^{8c}$ represents hydrogen, hydroxy, halogen, or $C_1$-alkyl;

$R^{8d}$ represents hydrogen, hydroxy, or halogen;

$R^{8e}$ represents hydrogen;

$R^{8f}$ represents hydrogen;

$R^{8g}$ represents hydrogen, amino, or halogen;

$R^{8h}$ represents hydrogen or halogen;

$R^{8i}$ represents hydrogen, halogen, or $C_1$-$C_2$-alkyl;

$R^{8k}$ represents hydrogen or $C_1$-alkyl;

$R^9$ represents $C_1$-$C_3$-alkyl or cyclopropyl;

$R^{9a}$ represents $C_1$-$C_3$-alkyl or cyclopropyl;

$R^{14}$ represents hydrogen, methyl, or ethyl;

$R^{15}$ represents hydrogen, methyl, or ethyl;

$R^{16}$ represents hydrogen, methyl, ethyl, methoxyethyl, or dimethylaminoethyl, or

$R^{15}$ and $R^{16}$, together with the nitrogen atom to which they are attached, represent a 5- to 6-membered heterocyclic ring, wherein said heterocyclic ring optionally contains one or two further heteroatoms selected from N, and O;

or an N-oxide, a salt, a tautomer or a stereoisomer of said compound, or a salt of said N-oxide, tautomer or stereoisomer.

In accordance with an embodiment of the first aspect, the present invention relates to compounds of formula (I) supra, wherein:

$R^1$ represents hydrogen or a group selected from $C_1$-$C_2$-alkyl and $C_3$-$C_4$-cycloalkyl;

$R^{2a}$ represents hydrogen or halogen;

$R^{2b}$ represents hydrogen;

$R^{2c}$ represents hydrogen or halogen;

L represents

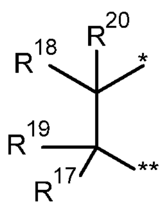

or

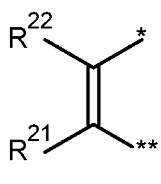

wherein * represents the point of direct attachment to the adjacent N-atom and ** represents the point of direct attachment to the adjacent C-atom;

| | |
|---|---|
| $R^{17}$ | represents hydrogen, $C_1$-$C_3$-alkyl, $C_1$-$C_3$-haloalkyl, or $C_3$-$C_4$-cycloalkyl-$C_1$-alkyl-; |
| $R^{18}$ | represents hydrogen or $C_1$-$C_3$-alkyl; |
| $R^{19}$ | represents hydrogen, $C_1$-$C_3$-alkyl, $C_1$-$C_3$-haloalkyl, or $C_3$-$C_4$-cycloalkyl-$C_1$-alkyl-; |
| $R^{20}$ | represents hydrogen or $C_1$-$C_3$-alkyl, or |
| $R^{18}$ and $R^{20}$, | together with the carbon atom to which they are attached, form a $C_3$-$C_6$-cycloalkyl ring; |
| $R^{21}$ | represents hydrogen, halogen, $C_1$-$C_3$-alkyl, $C_1$-$C_3$-haloalkyl, or $C_3$-$C_4$-cycloalkyl-$C_1$-alkyl-; |
| $R^{22}$ | represents hydrogen; |
| X | represents methylene, wherein said methylene group is optionally substituted, one or two times, with $R^3$; |
| Y | represents phenyl, wherein said phenyl is optionally substituted, one or more times, with $R^4$; |
| $R^3$ | represents $C_1$-$C_3$-alkyl, $C_1$-$C_3$-haloalkyl, or $R^5O$-$C_1$-$C_3$-alkyl; |
| $R^4$ | represents, halogen, $C_1$-$C_4$-alkyl, $C_1$-$C_4$-haloalkyl, $-CONR^{15}R^{16}$, or $-SO_2Me$; |
| $R^5$ | represents hydrogen; |
| A | represents a group selected from: |

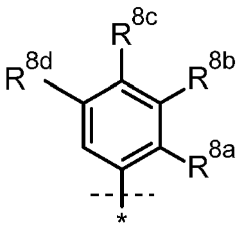 , 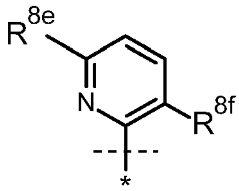 , 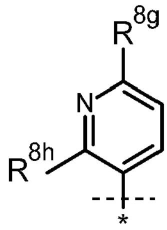 , 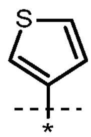 ,

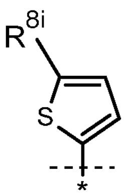 , 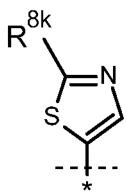 , 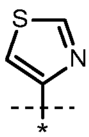 , 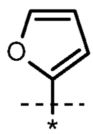 , 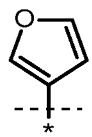 , and 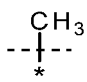 ,

wherein * indicates the point of attachment of said group to the rest of the formula (I);

| | |
|---|---|
| $R^{8a}$ | represents hydrogen, hydroxy, halogen, or cyano; |
| $R^{8b}$ | represents hydrogen, halogen, or $C_1$-alkyl; |
| $R^{8c}$ | represents hydrogen, halogen, or $C_1$-alkyl; |
| $R^{8d}$ | represents hydrogen or halogen; |
| $R^{8e}$ | represents hydrogen; |
| $R^{8f}$ | represents hydrogen; |
| $R^{8g}$ | represents hydrogen or halogen; |
| $R^{8h}$ | represents hydrogen; |
| $R^{8i}$ | represents hydrogen, halogen, or $C_1$-$C_2$-alkyl; |
| $R^{8k}$ | represents hydrogen or $C_1$-alkyl; |
| $R^{15}$ | represents hydrogen or methyl; |
| $R^{16}$ | represents methyl or methoxyethyl, or |
| $R^{15}$ and $R^{16}$, | together with the nitrogen atom to which they are attached, represent a 5- to 6-membered heterocyclic ring, wherein said heterocyclic ring optionally contains one further heteroatoms selected from N, and O; |

or an N-oxide, a salt, a tautomer or a stereoisomer of said compound, or a salt of said N-oxide, tautomer or stereoisomer.

In accordance with an embodiment of the first aspect, the present invention relates to compounds of formula (I) supra, wherein:

| | |
|---|---|
| $R^1$ | represents hydrogen or a group selected from $C_1$-alkyl and $C_3$-cycloalkyl; |
| $R^{2a}$ | represents hydrogen; |
| $R^{2b}$ | represents hydrogen; |

$R^{2c}$ represents hydrogen;

L represents

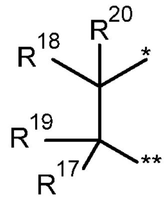

or

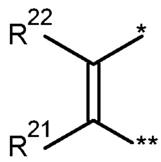

wherein * represents the point of direct attachment to the adjacent N-atom and ** represents the point of direct attachment to the adjacent C-atom;

$R^{17}$ represents hydrogen or $C_1$-alkyl;

$R^{18}$ represents hydrogen or $C_1$-alkyl;

$R^{19}$ represents hydrogen, $C_1$-$C_3$-alkyl, $C_1$-$C_3$-haloalkyl, or $C_3$--cycloalkyl-$C_1$-alkyl-;

$R^{20}$ represents hydrogen or $C_1$-alkyl, or

$R^{18}$ and $R^{20}$, together with the carbon atom to which they are attached, form a $C_3$-$C_4$-cycloalkyl ring;

$R^{21}$ represents hydrogen, $C_1$-$C_3$-alkyl, $C_1$-$C_3$-haloalkyl, or $C_3$-cycloalkyl-$C_1$-alkyl-;

$R^{22}$ represents hydrogen;

X represents methylene, wherein said methylene group is optionally substituted once with $R^3$;

Y represents phenyl, wherein said phenyl is optionally substituted once with $R^4$;

$R^3$ represents $C_1$-$C_3$-alkyl or $C_1$-$C_2$-haloalkyl;

$R^4$ represents halogen or -CON$R^{15}R^{16}$;

A represents a group selected from:

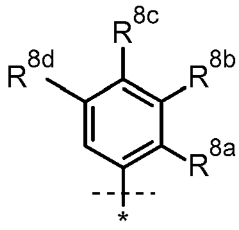

,

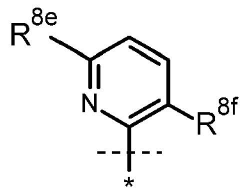

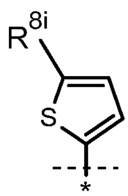

,

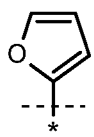

and

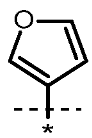

,

wherein * indicates the point of attachment of said group to the rest of the formula (I);

$R^{8a}$ represents hydrogen or fluoro;

| | |
|---|---|
| $R^{8b}$ | represents hydrogen or fluoro; |
| $R^{8c}$ | represents hydrogen, fluoro, or chloro; |
| $R^{8d}$ | represents hydrogen; |
| $R^{8e}$ | represents hydrogen; |
| $R^{8f}$ | represents hydrogen; |
| $R^{8i}$ | represents hydrogen, chloro, or $C_1$-alkyl; |
| $R^{15}$ | represents hydrogen; |
| $R^{16}$ | represents methoxyethyl, or |
| $R^{15}$ and $R^{16}$, | together with the nitrogen atom to which they are attached, represent a 5- to 6-membered heterocyclic ring, wherein said heterocyclic ring optionally contains one further heteroatom N; |

or an N-oxide, a salt, a tautomer, or a stereoisomer of said compound, or a salt of said N-oxide, tautomer or stereoisomer.

One aspect of the invention are compounds of formula (I) as described in the examples, as characterized by their names in the title, as claimed in claim 5, and/or their structures as well as the subcombinations of all residues specifically disclosed in the compounds of the examples.

Another aspect of the present disclosure are the intermediates used for their synthesis.

The invention provides an intermediate compound of formula 1-3, Ia or 1-9 or a salt thereof as set out in claim 12.

One special aspect of the invention are intermediates 1-3, or a salt thereof:

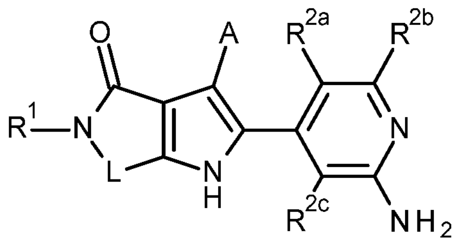

1-3,

in which $R^1$, $R^{2a}$, $R^{2b}$, $R^{2c}$, A, and L are as defined herein for the compound of general formula (I), with the proviso that R' is not hydrogen or methyl.

Another aspect of the invention relates to the use of intermediates 1-3, or a salt thereof:

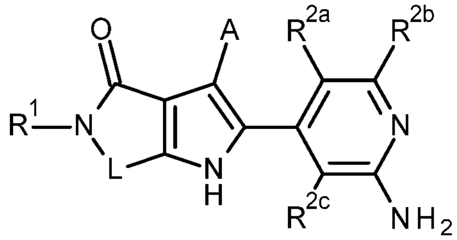

,

1-3,

in which $R^1$, $R^{2a}$, $R^{2b}$, $R^{2c}$, A, and L are as defined herein for the compound of general formula (I), for preparing a compound of formula (I) as defined herein or an N-oxide, a salt, a tautomer or a stereoisomer of said compound, or a salt of said N-oxide, tautomer or stereoisomer.

Another aspect of the invention relates to the use as set out in the claims of any of the intermediates described herein for preparing a compound of formula (I) as defined herein or an N-oxide, a salt, a tautomer or a stereoisomer of said compound, or a salt of said N-oxide, tautomer or stereoisomer.

In further embodiments of the above-mentioned aspects, the invention relates to compounds of formula (I), wherein:

R' represents hydrogen or a group selected from $C_1$-$C_4$-alkyl, $C_3$-$C_6$-cycloalkyl, $C_3$-$C_6$-cycloalkyl-$C_1$-$C_4$-alkyl-, $C_1$-$C_3$-alkoxy-$C_1$-$C_3$-alkyl-, and $C_1$-$C_5$-hydroxyalkyl, wherein said groups are each independently optionally substituted, one or more times, with halogen.

In further embodiments of the above-mentioned aspects, the invention relates to compounds of formula (I), wherein:

$R^1$ represents hydrogen or a group selected from $C_1$-$C_2$-alkyl and $C_3$-$C_4$-cycloalkyl, wherein said groups are each independently optionally substituted, one or more times, with halogen.

In further embodiments of the above-mentioned aspects, the invention relates to compounds of formula (I), wherein:

$R^1$ represents hydrogen or a group selected from $C_1$-$C_2$-alkyl and $C_3$-$C_4$-cycloalkyl.

In further embodiments of the above-mentioned aspects, the invention relates to compounds of formula (I), wherein:

$R^1$ represents hydrogen or a group selected from $C_1$-alkyl and $C_3$-cycloalkyl.

In further embodiments of the above-mentioned aspects, the invention relates to compounds of formula (I), wherein:

$R^{2a}$ represents hydrogen, hydroxy, halogen, cyano, $-NH_2$, -NHMe, $-NMe_2$, $C_1$-$C_2$-alkoxy, $C_1$-$C_2$-haloalkyl, or $C_1$-$C_2$-haloalkoxy.

In further embodiments of the above-mentioned aspects, the invention relates to compounds of formula (I), wherein:

$R^{2a}$ represents hydrogen or halogen.

In further embodiments of the above-mentioned aspects, the invention relates to compounds of formula (I), wherein:

$R^{2a}$ represents hydrogen.

In further embodiments of the above-mentioned aspects, the invention relates to compounds of formula (I), wherein:

$R^{2b}$ represents hydrogen, hydroxy, halogen, cyano, $-NH_2$, $-NHR^9$, or-$NMe_2$.

In further embodiments of the above-mentioned aspects, the invention relates to compounds of formula (I), wherein:

$R^{2b}$ represents hydrogen, halogen, $-NH_2$, or-$NHR^9$.

In further embodiments of the above-mentioned aspects, the invention relates to compounds of formula (I), wherein:

$R^{2b}$ represents hydrogen.

In further embodiments of the above-mentioned aspects, the invention relates to compounds of formula (I), wherein:

$R^{2c}$ represents hydrogen, halogen, cyano, $-NMe_2$, $C_1$-$C_2$-alkoxy, or $C_1$-$C_2$-haloalkoxy.

In further embodiments of the above-mentioned aspects, the invention relates to compounds of formula (I), wherein:

$R^{2c}$ represents hydrogen or halogen.

In further embodiments of the above-mentioned aspects, the invention relates to compounds of formula (I),

wherein:

$R^{2c}$ represents hydrogen.

In further embodiments of the above-mentioned aspects, the invention relates to compounds of formula (I), wherein:

L represents

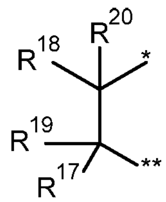

or

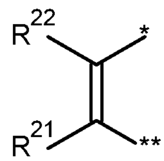

wherein * represents the point of direct attachment to the adjacent N-atom and ** represents the point of direct attachment to the adjacent C-atom.

In further embodiments of the above-mentioned aspects, the invention relates to compounds of formula (I), wherein:

L represents

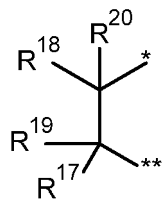

, wherein * represents the point of direct attachment to the adjacent N-atom and ** represents the point of direct attachment to the adjacent C-atom.

In further embodiments of the above-mentioned aspects, the invention relates to compounds of formula (I), wherein:

L represents

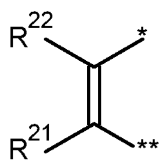

,

wherein * represents the point of direct attachment to the adjacent N-atom and ** represents the point of direct attachment to the adjacent C-atom.

In further embodiments of the above-mentioned aspects, the invention relates to compounds of formula (I), wherein:

$R^{17}$ represents hydrogen, $C_1$-$C_3$-alkyl, $C_1$-$C_3$-alkoxy, $C_1$-$C_3$-haloalkyl, or $C_3$-$C_6$-cycloalkyl-$C_1$-$C_4$-alkyl-.

In further embodiments of the above-mentioned aspects, the invention relates to compounds of formula (I), wherein:

$R^{17}$ represents hydrogen, $C_1$-$C_3$-alkyl, $C_1$-$C_3$-haloalkyl, or $C_3$-$C_6$-cycloalkyl-$C_1$-$C_4$-alkyl-.

In further embodiments of the above-mentioned aspects, the invention relates to compounds of formula (I), wherein:

$R^{17}$ represents hydrogen, $C_1$-$C_3$-alkyl, $C_1$-$C_3$-haloalkyl, or $C_3$-$C_4$-cycloalkyl-$C_1$-alkyl-.

In further embodiments of the above-mentioned aspects, the invention relates to compounds of formula (I), wherein:

$R^{17}$ represents hydrogen or $C_1$-alkyl.

In further embodiments of the above-mentioned aspects, the invention relates to compounds of formula (I), wherein:

$R^{18}$ represents hydrogen, $C_1$-$C_3$-alkyl, $C_1$-$C_3$-haloalkyl, or $C_3$-$C_6$-cycloalkyl-$C_1$-$C_4$-alkyl-.

In further embodiments of the above-mentioned aspects, the invention relates to compounds of formula (I), wherein:

$R^{18}$ represents hydrogen, $C_1$-$C_3$-alkyl, $C_1$-$C_3$-haloalkyl, or $C_3$-cycloalkyl-$C_1$-alkyl-.

In further embodiments of the above-mentioned aspects, the invention relates to compounds of formula (I), wherein:

$R^{18}$ represents hydrogen or $C_1$-$C_3$-alkyl.

In further embodiments of the above-mentioned aspects, the invention relates to compounds of formula (I), wherein:

$R^{18}$ represents hydrogen or $C_1$-alkyl.

In further embodiments of the above-mentioned aspects, the invention relates to compounds of formula (I), wherein:
$R^{17}$ and $R^{18}$, together with the carbon atoms to which they are attached, form a $C_3$-$C_6$-cycloalkyl ring.
In further embodiments of the above-mentioned aspects, the invention relates to compounds of formula (I), wherein:
$R^{17}$ and $R^{18}$, together with the carbon atoms to which they are attached, form a $C_3$-$C_5$-cycloalkyl ring.
In further embodiments of the above-mentioned aspects, the invention relates to compounds of formula (I), wherein:

$R^{19}$ represents hydrogen, $C_1$-$C_3$-alkyl, $C_1$-$C_3$-haloalkyl, or $C_3$-$C_6$-cycloalkyl-$C_1$-$C_4$-alkyl-.

In further embodiments of the above-mentioned aspects, the invention relates to compounds of formula (I), wherein:

$R^{19}$ represents hydrogen, $C_1$-$C_3$-alkyl, $C_1$-$C_3$-haloalkyl, or $C_3$-$C_4$-cycloalkyl-$C_1$-alkyl-.

In further embodiments of the above-mentioned aspects, the invention relates to compounds of formula (I), wherein:

$R^{19}$ represents hydrogen, $C_1$-$C_3$-alkyl, $C_1$-$C_3$-haloalkyl, or $C_3$--cycloalkyl-$C_1$-alkyl-.

In further embodiments of the above-mentioned aspects, the invention relates to compounds of formula (I), wherein:

$R^{20}$ represents hydrogen, $C_1$-$C_3$-alkyl, $C_1$-$C_3$-alkoxy, $C_1$-$C_3$-haloalkyl, or $C_3$-$C_6$-cycloalkyl-$C_1$-$C_4$-alkyl-.

In further embodiments of the above-mentioned aspects, the invention relates to compounds of formula (I), wherein:

$R^{20}$ represents hydrogen or $C_1$-$C_3$-alkyl.

In further embodiments of the above-mentioned aspects, the invention relates to compounds of formula (I), wherein:

$R^{20}$ represents hydrogen or $C_1$-alkyl.

In further embodiments of the above-mentioned aspects, the invention relates to compounds of formula (I), wherein:
$R^{18}$ and $R^{20}$, together with the carbon atom to which they are attached, form a $C_3$-$C_6$-cycloalkyl ring.
In further embodiments of the above-mentioned aspects, the invention relates to compounds of formula (I), wherein:
$R^{18}$ and $R^{20}$, together with the carbon atom to which they are attached, form a $C_3$-$C_4$-cycloalkyl ring.
In further embodiments of the above-mentioned aspects, the invention relates to compounds of formula (I), wherein:

$R^{21}$ represents hydrogen, halogen, $C_1$-$C_3$-alkyl, $C_1$-$C_3$-haloalkyl, or $C_3$-$C_6$-cycloalkyl-$C_1$-$C_4$-alkyl-.

In further embodiments of the above-mentioned aspects, the invention relates to compounds of formula (I), wherein:

$R^{21}$ represents hydrogen, halogen, $C_1$-$C_3$-alkyl, $C_1$-$C_3$-haloalkyl, or $C_3$-$C_4$-cycloalkyl-$C_1$-alkyl-.

In further embodiments of the above-mentioned aspects, the invention relates to compounds of formula (I), wherein:

$R^{21}$ represents hydrogen, $C_1$-$C_3$-alkyl, $C_1$-$C_3$-haloalkyl, or $C_3$-cycloalkyl-$C_1$-alkyl-.

In further embodiments of the above-mentioned aspects, the invention relates to compounds of formula (I), wherein:

$R^{22}$ represents hydrogen, $C_1$-$C_3$-alkyl, $C_1$-$C_3$-haloalkyl, or $C_3$-$C_6$-cycloalkyl-$C_1$-$C_4$-alkyl-.

In further embodiments of the above-mentioned aspects, the invention relates to compounds of formula (I), wherein:

$R^{22}$ represents hydrogen or $C_1$-$C_3$-alkyl.

In further embodiments of the above-mentioned aspects, the invention relates to compounds of formula (I), wherein:

$R^{22}$ represents hydrogen.

In further embodiments of the above-mentioned aspects, the invention relates to compounds of formula (I), wherein:

X represents methylene or ethylene,
wherein said methylene and ethylene groups are each independently optionally substituted, one or more times, with $R^3$.

In further embodiments of the above-mentioned aspects, the invention relates to compounds of formula (I), wherein:

X represents methylene,
wherein said methylene group is optionally substituted, one or more times, with $R^3$.

In further embodiments of the above-mentioned aspects, the invention relates to compounds of formula (I), wherein:

X represents methylene,
wherein said methylene group is optionally substituted, one or two times, with $R^3$.

In further embodiments of the above-mentioned aspects, the invention relates to compounds of formula (I), wherein:

X represents methylene,
wherein said methylene group is optionally substituted once with $R^3$.

In further embodiments of the above-mentioned aspects, the invention relates to compounds of formula (I), wherein:

Y represents phenyl or a heteroaryl group,
wherein said phenyl and heteroaryl groups are each independently optionally substituted, one or more times, with $R^4$.

In further embodiments of the above-mentioned aspects, the invention relates to compounds of formula (I), wherein:

Y represents phenyl,
wherein said phenyl is optionally substituted, one or more times, with $R^4$.

In further embodiments of the above-mentioned aspects, the invention relates to compounds of formula (I), wherein:

Y represents phenyl,
wherein said phenyl is optionally substituted once with $R^4$.

In further embodiments of the above-mentioned aspects, the invention relates to compounds of formula (I), wherein:

$R^3$ represents hydroxy, halogen, cyano, $C_1$-$C_3$-alkyl, $C_1$-$C_3$-alkoxy, $C_1$-$C_3$-haloalkyl, $C_1$-$C_2$-haloalkoxy, $C_3$-$C_4$-cycloalkyl, -$NHR^6$, $R^5O$-$C_1$-$C_3$-alkyl, $R^6R^7N$-$C_1$-$C_5$-alkyl, or $R^6R^7N$-X'-C($R^{10}$)($R^{11}$)-$C_1$-$C_2$-alkyl-.

In further embodiments of the above-mentioned aspects, the invention relates to compounds of formula (I), wherein:

$R^3$ represents $C_1$-$C_3$-alkyl, $C_1$-$C_3$-haloalkyl, -$NHR^6$, $R^5O$-$C_1$-$C_3$-alkyl, or $R^6R^7N$-$C_1$-$C_4$-alkyl.

In further embodiments of the above-mentioned aspects, the invention relates to compounds of formula (I), wherein:

$R^3$ represents $C_1$-$C_3$-alkyl, $C_1$-$C_3$-haloalkyl, or $R^SO$-$C_1$-$C_3$-alkyl.

In further embodiments of the above-mentioned aspects, the invention relates to compounds of formula (I), wherein:

$R^3$ represents $C_1$-$C_3$-alkyl or $C_1$-$C_2$-haloalkyl.

In further embodiments of the above-mentioned aspects, the invention relates to compounds of formula (I), wherein:

X' represents methylene or ethylene.

In further embodiments of the above-mentioned aspects, the invention relates to compounds of formula (I), wherein:

X' represents methylene.

In further embodiments of the above-mentioned aspects, the invention relates to compounds of formula (I), wherein:

$R^4$ represents hydroxy, halogen, cyano, $C_1$-$C_4$-alkyl, $C_1$-$C_4$-alkoxy, $C_1$-$C_4$-haloalkyl, $C_1$-$C_4$-haloalkoxy, $-NMe_2$, $-CO_2R^{14}$, $-CONR^{15}R^{16}$, or $-SO_2Me$.

In further embodiments of the above-mentioned aspects, the invention relates to compounds of formula (I), wherein:

$R^4$ represents, halogen, $C_1$-$C_4$-alkyl, $C_1$-$C_4$-haloalkyl, $C_1$-$C_4$-haloalkoxy, $-CO_2R^{14}$, $-CONR^{15}R^{16}$, or $-SO_2Me$.

In further embodiments of the above-mentioned aspects, the invention relates to compounds of formula (I), wherein:

$R^4$ represents, halogen, $C_1$-$C_4$-alkyl, $C_1$-$C_4$-haloalkyl, $-CONR^{15}R^{16}$, or $-SO_2Me$.

In further embodiments of the above-mentioned aspects, the invention relates to compounds of formula (I), wherein:

$R^4$ represents halogen or $-CONR^{15}R^{16}$.

In further embodiments of the above-mentioned aspects, the invention relates to compounds of formula (I), wherein:

$R^5$ represents hydrogen, $C_1$-$C_2$-alkyl, $C_1$-$C_2$-haloalkyl, or $C_3$-$C_4$-cycloalkyl.

In further embodiments of the above-mentioned aspects, the invention relates to compounds of formula (I), wherein:

$R^5$ represents hydrogen, $C_1$-$C_2$-alkyl, or $C_1$-$C_2$-haloalkyl.

In further embodiments of the above-mentioned aspects, the invention relates to compounds of formula (I), wherein:

$R^5$ represents hydrogen.

In further embodiments of the above-mentioned aspects, the invention relates to compounds of formula (I), wherein:

$R^6$ represents hydrogen, $C_1$-$C_2$-alkyl, $C_1$-$C_2$-haloalkyl, or $C_3$-$C_4$-cycloalkyl.

In further embodiments of the above-mentioned aspects, the invention relates to compounds of formula (I), wherein:

$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl.

In further embodiments of the above-mentioned aspects, the invention relates to compounds of formula (I), wherein:

$R^6$ and $R^7$, together with the nitrogen atom to which they are attached, represent a 4- to 7-membered heterocyclic ring, wherein said heterocyclic ring optionally contains one or more further heteroatoms selected from N, O, S, or groups selected from C(=O), S(=O), and $SO_2$, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy.

In further embodiments of the above-mentioned aspects, the invention relates to compounds of formula (I), wherein:

$R^6$ and $R^7$, together with the nitrogen atom to which they are attached, represent a 4- to 7-membered heterocyclic ring, wherein said heterocyclic ring optionally contains one or more further heteroatoms selected from N, O, S, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy.

In further embodiments of the above-mentioned aspects, the invention relates to compounds of formula (I), wherein:

A represents a group selected from:

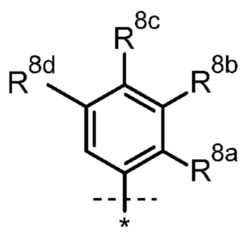 , 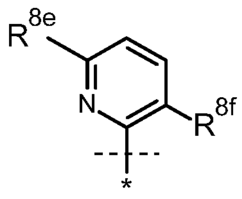 , 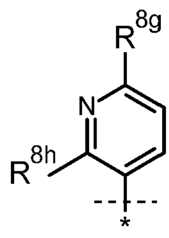 , 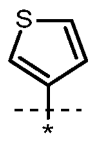 ,

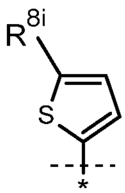 , 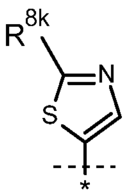 , 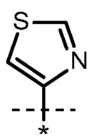 , 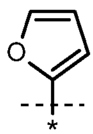 , 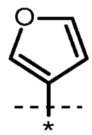 , and 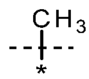 ,

wherein * indicates the point of attachment of said group to the rest of the formula (I).

In further embodiments of the above-mentioned aspects, the invention relates to compounds of formula (I), wherein:

A represents a group selected from:

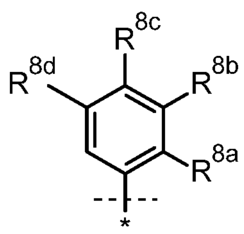 , 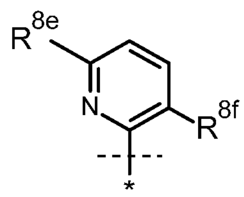 ,

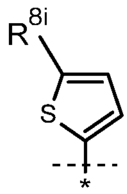 , 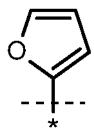 and 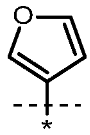 ,

wherein * indicates the point of attachment of said group to the rest of the formula (I).

In further embodiments of the above-mentioned aspects, the invention relates to compounds of formula (I), wherein:

A represents a group selected from:

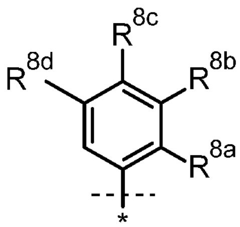 and 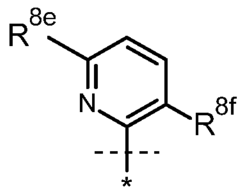 ,

wherein * indicates the point of attachment of said group to the rest of the formula (I).

In further embodiments of the above-mentioned aspects, the invention relates to compounds of formula (I), wherein:

$R^{8a}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8a}$ represents hydrogen, hydroxy, halogen, cyano, or $C_1$-alkyl.

In further embodiments of the above-mentioned aspects, the invention relates to compounds of formula (I), wherein:

$R^{8a}$ represents hydrogen, hydroxy, halogen, or cyano.

In further embodiments of the above-mentioned aspects, the invention relates to compounds of formula (I), wherein:

$R^{8a}$ represents hydrogen or fluoro.

In further embodiments of the above-mentioned aspects, the invention relates to compounds of formula (I), wherein:

$R^{8b}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8b}$ represents hydrogen, hydroxy, halogen, or $C_1$-alkyl.

In further embodiments of the above-mentioned aspects, the invention relates to compounds of formula (I), wherein:

$R^{8b}$ represents hydrogen, halogen, or $C_1$-alkyl.

In further embodiments of the above-mentioned aspects, the invention relates to compounds of formula (I), wherein:

$R^{8b}$ represents hydrogen or fluoro.

In further embodiments of the above-mentioned aspects, the invention relates to compounds of formula (I), wherein:

$R^{8c}$ represents hydrogen, hydroxy, halogen, $C_1$-$C_2$-alkyl, or $C_1$-$C_2$-haloalkyl.

In further embodiments of the above-mentioned aspects, the invention relates to compounds of formula (I), wherein:

$R^{8c}$ represents hydrogen, hydroxy, halogen, or $C_1$-alkyl.

In further embodiments of the above-mentioned aspects, the invention relates to compounds of formula (I), wherein:

$R^{8c}$ represents hydrogen, halogen, or $C_1$-alkyl.

In further embodiments of the above-mentioned aspects, the invention relates to compounds of formula (I), wherein:

$R^{8c}$ represents hydrogen, fluoro, or chloro.

In further embodiments of the above-mentioned aspects, the invention relates to compounds of formula (I), wherein:

$R^{8d}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8d}$ represents hydrogen, hydroxy, or halogen.

In further embodiments of the above-mentioned aspects, the invention relates to compounds of formula (I), wherein:

$R^{8d}$ represents hydrogen or halogen.

In further embodiments of the above-mentioned aspects, the invention relates to compounds of formula (I), wherein:

$R^{8d}$ represents hydrogen.

In further embodiments of the above-mentioned aspects, the invention relates to compounds of formula (I), wherein:

$R^{8e}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8e}$ represents hydrogen.

In further embodiments of the above-mentioned aspects, the invention relates to compounds of formula (I), wherein:

$R^{8f}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8f}$ represents hydrogen.

In further embodiments of the above-mentioned aspects, the invention relates to compounds of formula (I), wherein:

$R^{8g}$ represents hydrogen, hydroxy, amino, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8g}$ represents hydrogen, amino, or halogen.

In further embodiments of the above-mentioned aspects, the invention relates to compounds of formula (I), wherein:

$R^{8g}$ represents hydrogen or halogen.

In further embodiments of the above-mentioned aspects, the invention relates to compounds of formula (I), wherein:

$R^{8h}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8h}$ represents hydrogen or halogen.

In further embodiments of the above-mentioned aspects, the invention relates to compounds of formula (I), wherein:

$R^{8h}$ represents hydrogen.

In further embodiments of the above-mentioned aspects, the invention relates to compounds of formula (I), wherein:

$R^{8i}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8i}$ represents hydrogen, halogen, or $C_1$-$C_2$-alkyl.

In further embodiments of the above-mentioned aspects, the invention relates to compounds of formula (I), wherein:

$R^{8i}$ represents hydrogen, chloro, or $C_1$-alkyl.

In further embodiments of the above-mentioned aspects, the invention relates to compounds of formula (I),

wherein:

$R^{8k}$ represents hydrogen, hydroxy, halogen, cyano, $C_1$-$C_2$-alkyl, or $C_1$-$C_2$-haloalkyl.

In further embodiments of the above-mentioned aspects, the invention relates to compounds of formula (I), wherein:

$R^{8k}$ represents hydrogen or $C_1$-alkyl.

In further embodiments of the above-mentioned aspects, the invention relates to compounds of formula (I), wherein:

$R^9$ represents $C_1$-$C_3$-alkyl or cyclopropyl.

In further embodiments of the above-mentioned aspects, the invention relates to compounds of formula (I), wherein:

$R^{9a}$ represents $C_1$-$C_3$-alkyl or cyclopropyl.

In further embodiments of the above-mentioned aspects, the invention relates to compounds of formula (I), wherein:

$R^{10}$ represents methyl, halogen, or hydroxy.

In further embodiments of the above-mentioned aspects, the invention relates to compounds of formula (I), wherein:

$R^{11}$ represents methyl, halogen, or hydroxy.

In further embodiments of the above-mentioned aspects, the invention relates to compounds of formula (I), wherein:

$R^{14}$ represents hydrogen, methyl, or ethyl.

In further embodiments of the above-mentioned aspects, the invention relates to compounds of formula (I), wherein:

$R^{15}$ represents hydrogen, methyl, or ethyl.

In further embodiments of the above-mentioned aspects, the invention relates to compounds of formula (I), wherein:

$R^{15}$ represents hydrogen or methyl.

In further embodiments of the above-mentioned aspects, the invention relates to compounds of formula (I), wherein:

$R^{15}$ represents hydrogen.

In further embodiments of the above-mentioned aspects, the invention relates to compounds of formula (I), wherein:

$R^{16}$ represents hydrogen, methyl, ethyl, methoxyethyl, or dimethylaminoethyl.

In further embodiments of the above-mentioned aspects, the invention relates to compounds of formula (I), wherein:

$R^{16}$ represents methyl or methoxyethyl.

In further embodiments of the above-mentioned aspects, the invention relates to compounds of formula (I), wherein:

$R^{16}$ represents methoxyethyl.

In further embodiments of the above-mentioned aspects, the invention relates to compounds of formula (I), wherein:
$R^{15}$ and $R^{16}$, together with the nitrogen atom to which they are attached, represent a 5- to 6-membered heterocyclic ring, wherein said heterocyclic ring optionally contains one or two further heteroatoms selected from N, O, S, or groups selected from C(=O), S(=O), and $SO_2$.
In further embodiments of the above-mentioned aspects, the invention relates to compounds of formula (I), wherein:
$R^{15}$ and $R^{16}$, together with the nitrogen atom to which they are attached, represent a 5- to 6-membered heterocyclic ring, wherein said heterocyclic ring optionally contains one or two further heteroatoms selected from N, and O.
In further embodiments of the above-mentioned aspects, the invention relates to compounds of formula (I), wherein:
$R^{15}$ and $R^{16}$, together with the nitrogen atom to which they are attached, represent a 5- to 6-membered heterocyclic ring, wherein said heterocyclic ring optionally contains one further heteroatoms selected from N, and O.
In further embodiments of the above-mentioned aspects, the invention relates to compounds of formula (I), wherein:
$R^{15}$ and $R^{16}$, together with the nitrogen atom to which they are attached, represent a 5- to 6-membered heterocyclic ring, wherein said heterocyclic ring optionally contains one further heteroatom N.
A further aspect of the invention are compounds of formula (I), which are present as their salts.
It is to be understood that the present invention relates to any sub-combination within any embodiment or aspect of the present invention of compounds of general formula (I), supra.
More particularly still, the present invention covers compounds of general formula (I) which are disclosed in the Example section of this text, infra.
In accordance with another aspect, the present invention covers methods of preparing compounds of the present invention, said methods comprising the steps as described in the Experimental Section herein.
In other embodiments of the invention, compounds are defined according to the claims as disclosed in the Claims section wherein the definitions are limited according to the preferred or more preferred definitions as disclosed below or specifically disclosed residues of the exemplified compounds and subcombinations thereof.
In certain embodiments, compounds of the present invention have surprising and advantageous properties.
In particular, compounds of the present invention have surprisingly been found to effectively inhibit Casein kinase 1 alpha and/or delta and may therefore be used for the treatment or prophylaxis of diseases of uncontrolled cell growth, proliferation and/or survival, inappropriate cellular immune responses, or inappropriate cellular inflammatory responses or diseases which are accompanied with uncontrolled cell growth, proliferation and/or survival, inappropriate cellular immune responses, or inappropriate cellular inflammatory responses, In particular, the disclosed compounds can be used for treatment or prophylaxis of diseases in which the uncontrolled cell growth, proliferation and/or survival, inappropriate cellular immune responses, or inappropriate cellular inflammatory responses is mediated by Casein kinase 1 alpha and/or delta, such as, for example, haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

**Definitions**

Constituents which are optionally substituted as stated herein, may be substituted, unless otherwise noted, one or more times, independently of one another at any possible position. When any variable occurs more than one time in any constituent, each definition is independent. For example, when in which $R^2$, $R^3$, $R^4$, $R^5$, $R^6$, $R^7$, and/or $R^8$, occur more than one time in any compound of formula (I) each definition of $R^2$, $R^3$, $R^4$, $R^5$, $R^6$, $R^7$, and $R^8$ is independent.
Should a constituent be composed of more than one part, e.g. $C_3$-$C_6$-cycloalkyl-$C_1$-$C_4$-alkyl-, unless stated otherwise, the position of a possible substituent can be at any of these parts at any suitable position (e.g. in the case of a substituted $C_3$-$C_6$-cycloalkyl-$C_1$-$C_4$-alkyl- group, substituents may be present at the $C_3$-$C_6$-cycloalkyl part of the group, at the $C_1$-$C_4$-alkyl part of the group or both). A hyphen at the beginning or at the end of the constituent marks the point of attachment to the rest of the molecule. Should a ring be substituted the substituent could be at any suitable position of the ring, also on a ring nitrogen atom if suitable.

The term "comprising" when used in the specification includes "consisting of".

If it is referred to "as mentioned above" or "mentioned above", *"supra"* within the description it is referred to any of the disclosures made within the specification in any of the preceding pages, or above on the same page.

If it is referred to "as mentioned herein", "described herein", "provided herein" or "stated herein" within the description it is referred to any of the disclosures made within the specification in any of the preceding or subsequent pages.

"suitable" within the sense of the invention means chemically possible to be made by methods within the knowledge of a skilled person.

The terms as mentioned in the present text have preferably the following meanings:

The term "halogen atom", "halo-" or "Hal-" is to be understood as meaning a fluorine, chlorine, bromine or iodine atom, preferably a fluorine, chlorine or bromine atom.

The term "$C_1$-$C_6$-alkyl" is to be understood as meaning a linear or branched, saturated, monovalent hydrocarbon group having 1, 2, 3, 4, 5, or 6 carbon atoms, e.g. a methyl, ethyl, propyl, butyl, pentyl, hexyl, iso-propyl, iso-butyl, sec-butyl, tert-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl group, or an isomer thereof. Preferably, said group has 1, 2, 3 or 4 carbon atoms ("$C_1$-$C_4$-alkyl"), e.g. a methyl, ethyl, propyl, butyl, iso-propyl, iso-butyl, sec-butyl, tert-butyl group, more preferably 1, 2 or 3 carbon atoms ("$C_1$-$C_3$-alkyl"), e.g. a methyl, ethyl, n-propyl or iso-propyl group.

The term "$C_1$-$C_4$-haloalkyl" is to be understood as meaning a linear or branched, saturated, monovalent hydrocarbon group in which the term "$C_1$-$C_4$-alkyl" is defined *supra,* and in which one or more hydrogen atoms are replaced by a halogen atom, identically or differently, *i.e.* one halogen atom being independent from another. Preferably, said halogen atom is F. Said $C_1$-$C_4$-haloalkyl group is, for example, $-CF_3$, $-CHF_2$, $-CH_2F$, $-CF_2CF_3$, $-CH_2CH_2F$, $-CH_2CHF_2$, $-CH_2CF_3$, $-CH_2CH_2CF_3$, or $-CH(CH_2F)_2$. Preferably, said group has 1, 2, or 3 carbon atoms ("$C_1$-$C_3$-haloalkyl"). More preferably, said group has 1, or 2 carbon atoms ("$C_1$-$C_2$-haloalkyl").

The term "$C_1$-$C_5$-hydroxyalkyl" means a linear or branched, saturated, monovalent hydrocarbon group in which the term "$C_1$-$C_5$-alkyl" is defined *supra,* and in which 1 or 2 hydrogen atoms are replaced with a hydroxy group, e.g. a hydroxymethyl, 1-hydroxyethyl, 2-hydroxyethyl, 1,2-dihydroxyethyl, 3-hydroxypropyl, 2-hydroxypropyl, 1-hydroxypropyl, 1-hydroxypropan-2-yl, 2-hydroxypropan-2-yl, 2,3-dihydroxypropyl, 1,3-dihydroxypropan-2-yl, 3-hydroxy-2-methyl-propyl, 2-hydroxy-2-methyl-propyl or 1-hydroxy-2-methyl-propyl group.

The term "$C_1$-$C_4$-alkoxy" is to be understood as meaning a linear or branched, saturated, monovalent, hydrocarbon group of formula -O-alkyl, in which the term "alkyl" is defined *supra,* e.g. a methoxy, ethoxy, n-propoxy, iso-propoxy, n-butoxy, iso-butoxy, tert-butoxy or sec-butoxy group, or an isomer thereof.

The term "$C_1$-$C_4$-haloalkoxy" is to be understood as meaning a linear or branched, saturated, monovalent $C_1$-$C_4$-alkoxy group, as defined *supra,* in which one or more of the hydrogen atoms are replaced, identically or differently, by a halogen atom. Preferably, said halogen atom is F. Said $C_1$-$C_4$-haloalkoxy group is, for example, $-OCF_3$, $-OCHF_2$, $-OCH_2F$, $-OCF_2CF_3$, or - $OCH_2CF_3$. Preferably, said group has 1, 2, or 3 carbon atoms ("$C_1$-$C_3$-haloalkoxy"). More particularly, said group has 1, or 2 carbon atoms ("$C_1$-$C_2$-haloalkoxy").

The term "$C_3$-$C_6$-cycloalkyl" is to be understood as meaning a saturated, monovalent, mono- or bicyclic hydrocarbon ring which contains 3, 4, 5 or 6 carbon atoms ("$C_3$-$C_6$-cycloalkyl"). Said $C_3$-$C_6$-cycloalkyl group is for example, a monocyclic hydrocarbon ring, e.g. a cyclopropyl, cyclobutyl, cyclopentyl or cyclohexyl, or a bicyclic hydrocarbon ring.

The term "halocyclopropyl" is to be understood as meaning a cyclopropyl group in which one or more hydrogen atoms are replaced by a halogen atom, identically or differently, *i.e.* one halogen atom being independent from another. Particularly, said halogen atom is F.

The term "4- to 7-membered heterocyclic ring" mean a monocyclic, saturated heterocycle with 4, 5, 6 or 7 ring atoms in total, wherein said heterocyclic ring contains one N atom and optionally one or more heteroatoms from the series N, O and S, or groups from the series C(=O), S(=O), and $SO_2$.

Said heterocyclic ring, without being limited thereto, can be a 4-membered ring, such as azetidinyl, oxetanyl or thietanyl, for example; or a 5-membered ring, such as tetrahydrofuranyl, 1,3-dioxolanyl, thiolanyl, pyrrolidinyl, imidazolidinyl, pyrazolidinyl, 1,1-dioxidothiolanyl, 1,2-oxazolidinyl, 1,3-oxazolidinyl or 1,3-thiazolidinyl, for example; or a 6-membered ring, such as tetrahydropyranyl, tetrahydrothiopyranyl, piperidinyl, morpholinyl, dithianyl, thiomorpholinyl, piperazinyl, 1,3-dioxanyl, 1,4-dioxanyl or 1,2-oxazinanyl, for example, or a 7-membered ring, such as azepanyl, 1,4-diazepanyl or 1,4-oxazepanyl, for example.

The term "heteroaryl" means a monovalent, monocyclic, bicyclic or tricyclic aromatic ring having 5, 6, 8, 9, 10, 11, 12, 13 or 14 ring atoms (a "5- to 14-membered heteroaryl" group), preferably 5, 6, 9 or 10 ring atoms, which contains at least one ring heteroatom and optionally one, two or three further ring heteroatoms from the series: N, O and/or S, and which is bound via a ring carbon atom or optionally via a ring nitrogen atom (if allowed by valency).

Said heteroaryl group can be a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group, such as, for example, pyridinyl, pyridazinyl, pyrimidinyl, pyrazinyl or triazinyl; or a tricyclic heteroaryl group, such as, for example, carbazolyl, acridinyl or phenazinyl; or a 9-membered heteroaryl group, such as, for example, benzofuranyl, benzothienyl, benzoxazolyl, benzisoxazolyl, benzimidazolyl, benzothiazolyl, benzotriazolyl, indazolyl, indolyl, isoindolyl, indolizinyl or purinyl; or a 10-membered heteroaryl group, such as, for example, quinolinyl, quinazolinyl, isoquinolinyl, cinnolinyl, phthalazinyl, quinoxalinyl or pteridinyl.

In general, and unless otherwise mentioned, the heteroaryl or heteroarylene groups include all possible isomeric forms thereof, e.g.: tautomers and positional isomers with respect to the point of linkage to the rest of the molecule. Thus, for some illustrative non-restricting examples, the term pyridinyl includes pyridin-2-yl, pyridin-3-yl and pyridin-4-yl; or the term thienyl includes thien-2-yl and thien-3-yl.

The term "$C_1$-$C_6$", as used throughout this text, e.g. in the context of the definition of "$C_1$-$C_6$-alkyl" is to be understood as meaning an alkyl group having a finite number of carbon atoms of 1 to 6, *i.e.* 1, 2, 3, 4, 5 or 6 carbon atoms. It is to be understood further that said term "$C_1$-$C_6$" is to be interpreted as any sub-range comprised therein, e.g. $C_1$-$C_6$, $C_2$-$C_6$ , $C_3$-$C_6$ , $C_1$-$C_2$ , $C_1$-$C_3$ , particularly $C_1$-$C_2$, $C_1$-$C_3$, $C_1$-$C_4$,

The term "$C_1$-$C_4$", as used throughout this text, e.g. in the context of the definition of "$C_1$-$C_4$-alkyl", "$C_1$-$C_4$-haloalkyl", "$C_1$-$C_4$-alkoxy", or "$C_1$-$C_4$-haloalkoxy" is to be understood as meaning an alkyl group having a finite number of carbon atoms of 1 to 4, *i.e.* 1, 2, 3 or 4 carbon atoms. It is to be understood further that said term "$C_1$-$C_4$" is to be interpreted as any sub-range comprised therein, e.g. $C_1$-$C_4$, $C_2$-$C_4$, $C_3$-$C_4$, $C_1$-$C_2$, $C_1$-$C_3$, preferably$C_1$-$C_2$, $C_1$-$C_3$, $C_1$-$C_4$, in the case of "$C_1$-$C_4$-haloalkyl" or "$C_1$-$C_4$-haloalkoxy" even more preferably $C_1$-$C_2$.

Further, as used herein, the term "$C_3$-$C_6$", as used throughout this text, e.g. in the context of the definition of "$C_3$-$C_6$-cycloalkyl", is to be understood as meaning a cycloalkyl group having a finite number of carbon atoms of 3 to 6, *i.e.* 3, 4, 5 or 6 carbon atoms. It is to be understood further that said term "$C_3$-$C_6$" is to be interpreted as any sub-range comprised therein, e.g. $C_3$-$C_6$, $C_4$-$C_5$, $C_3$-$C_5$, $C_3$-$C_4$, $C_4$-$C_6$, $C_5$-$C_6$; preferably$C_3$-$C_6$.

The term "substituted" means that one or more hydrogens on the designated atom is replaced with a selection from the indicated group, provided that the substituted atom's normal valency under the existing circumstances is not exceeded, and that the substitution results in a stable compound. Combinations of substituents and/or variables are permissible only if such combinations result in stable compounds.

The term "optionally substituted" means optional substitution with the specified groups, radicals or moieties.

Ring system substituent means a substituent attached to an aromatic or nonaromatic ring system which, for example, replaces an available hydrogen on the ring system.

As used herein, the term "one or more", e.g. in the definition of the substituents of the compounds of the general formulae of the present invention, is understood as meaning "one, two, three, four or five, preferably one, two, three or four, more particularly one, two or three, even more preferably one or two".

The compounds of general formula (I) may exist as isotopic variants. The invention therefore includes one or more isotopic variant(s) of the compounds of general formula (I), particularly deuterium-containing compounds of general formula (I).

The term "Isotopic variant" of a compound or a reagent is defined as a compound exhibiting an unnatural proportion of one or more of the isotopes of the elements that constitute such a compound.

The term "Isotopic variant of the compound of general formula (I)" is defined as a compound of general formula (I) exhibiting an unnatural proportion of one or more of the isotopes of the elements that constitute such a compound.

The expression "unnatural proportion" is to be understood as meaning a proportion of such isotope which is higher than its natural abundance. The natural abundances of isotopes to be applied in this context are described in "Isotopic Compositions of the Elements 1997", Pure Appl. Chem., 70(1), 217-235, 1998.

Examples of such isotopes include stable and radioactive isotopes of hydrogen, carbon, nitrogen, oxygen, phosphorus, sulfur, fluorine, chlorine, bromine and iodine, such as $^{2}H$ (deuterium), $^{3}H$ (tritium), $^{11}C$, $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$ and $^{131}I$, respectively. Accordingly, recitation of "hydrogen" or "H" should be understood to encompass $^{1}H$ (protium), $^{2}H$ (deuterium), and $^{3}H$ (tritium) unless otherwise specified.

With respect to the treatment and/or prophylaxis of the disorders specified herein, isotopic variant(s) of the compounds of general formula (I) preferably contain elevated levels of deuterium ("deuterium-containing compounds of general formula (I)"). Isotopic variants of the compounds of general formula (I) in which one or more radioactive isotopes, such as $^{3}H$ or $^{14}C$, are incorporated are useful e.g. in drug and/or substrate tissue distribution studies. These isotopes are particularly preferred for the ease of their incorporation and detectability. Positron emitting isotopes such as $^{18}F$ or $^{11}C$ may be incorporated into a compound of general formula (I). These isotopic variants of the compounds of general formula (I) are useful for in vivo imaging applications. Deuterium-containing and $^{13}C$-containing compounds of general formula (I) can be used in mass spectrometry analyses (H. J. Leis et al., Curr. Org. Chem., 1998, 2, 131) in the context of preclinical or clinical studies.

Isotopic variants of the compounds of general formula (I) can generally be prepared by methods known to a person skilled in the art, such as those described in the schemes and/or examples herein, e.g., by substituting a reagent

for an isotopic variant of said reagent, preferably for a deuterium-containing reagent. Depending on the desired sites of deuteration, in some cases deuterium from $D_2O$ can be incorporated either directly into the compounds or into reagents that are useful for synthesizing such compounds (Esaki et al., Tetrahedron, 2006, 62, 10954; Esaki et al., Chem. Eur. J., 2007, 13, 4052). Deuterium gas is also a useful reagent for incorporating deuterium into molecules. Catalytic deuteration of olefinic bonds (H. J. Leis et al., Curr. Org. Chem., 1998, 2, 131; J. R. Morandi et al., J. Org. Chem., 1969, 34 (6), 1889) and acetylenic bonds (N. H. Khan, J. Am. Chem. Soc., 1952, 74 (12), 3018; S. Chandrasekhar et al., Tetrahedron, 2011, 52, 3865) is a rapid route for incorporation of deuterium. Metal catalysts (i.e. e.g., Pd, Pt, and Rh) in the presence of deuterium gas can be used to directly exchange deuterium for hydrogen in functional groups containing hydrocarbons (J. G. Atkinson et al., US Patent 3966781). A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA. Further information on the state of the art with respect to deuterium-hydrogen exchange is given for example in Hanzlik et al., J. Org. Chem. 55, 3992-3997, 1990; R. P. Hanzlik et al., Biochem. Biophys. Res. Commun. 160, 844, 1989; P. J. Reider et al., J. Org. Chem. 52, 3326-3334, 1987; M. Jarman et al., Carcinogenesis 16(4), 683-688, 1993; J. Atzrodt et al., Angew. Chem., Int. Ed. 2007, 46, 7744; K. Matoishi et al., J. Chem. Soc, Chem. Commun. 2000, 1519-1520; K. Kassahun et al., WO2012/112363.

The term "deuterium-containing compound of general formula (I)" is defined as a compound of general formula (I), in which one or more protium ($^1H$) atom(s) is/are replaced by one or more deuterium atom(s) and in which the abundance of deuterium at each deuterated position of the compound of general formula (I) is higher than the natural abundance of deuterium, which is about 0.015%. Particularly, in a deuterium-containing compound of general formula (I) the abundance of deuterium at each deuterated position of the compound of general formula (I) is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).

The selective incorporation of one or more deuterium atom(s) into a compound of general formula (I) may alter the physicochemical properties (such as for example acidity [A. Streitwieser et al., J. Am. Chem. Soc., 1963, 85, 2759; C. L. Perrin, et al., J. Am. Chem. Soc., 2007, 129, 4490], basicity [C. L. Perrin, et al., J. Am. Chem. Soc., 2003, 125, 15008; C. L. Perrin in Advances in Physical Organic Chemistry, 44, 144; C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (D. J. Kushner et al., Can. J. Physiol. Pharmacol., 1999, 77, 79; A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula (I). In some cases deuterium substitution reduces or eliminates the formation of an undesired or toxic metabolite and/or enhances the formation of a desired metabolite (e.g. Nevirapine: A. M. Sharma et al., Chem. Res.Toxicol., 2013, 26, 410; Uetrecht etal., Chemical Research in Toxicology, 2008, 21, 9, 1862; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels, i.e., reduced peak-trough variation. This could result in lower side effects and/or enhanced efficacy, depending on the particular compound's pharmacokinetic/ pharmacodynamic relationship. Indiplon (A. J. Morales et al., Abstract 285, The 15th North American Meeting of the International Society of Xenobiotics, San Diego, CA, October 12-16, 2008), ML-337 (C. J. Wenthur et al., J. Med. Chem., 2013, 56, 5208), and Odanacatib (K. Kassahun et al., WO2012/112363) are examples of this deuterium effect. Still other cases have been reported in which reduced rates of metabolism result in an increase in exposure of the drug without changing the rate of systemic clearance (e.g. Rofecoxib: F. Schneider et al., Arzneim. Forsch. Drug. Res., 2006, 56, 295; Telaprevir: F. Maltais et al., J. Med. Chem., 2009, 52, 7993). Deuterated drugs showing this effect may have reduced dosing requirements (e.g. lower number of doses or lower dosage to achieve the desired effect) and/or may produce lower metabolite loads.

A compound of general formula (I) may have multiple potential sites of vulnerabillity tometabolism. To optimize the above-described effects on physicochemical properties and metabolic profile, deuterium-containing compounds of general formula (I) having a certain pattern of one or more deuterium-hydrogen exchange(s) can be selected. Particularly, the deuterium atom(s) of deuterium-containing compound(s) of general formula (I) is/are attached to a carbon atom and/or is/are located at those positions of the compound of general formula (I), which are sites of attack for metabolizing enzymes such as e.g. cytochrome $P_{450}$.

Where the plural form of the word compounds, salts, polymorphs, hydrates, solvates and the like, is used herein, this is taken to include also a single compound, salt, polymorph, isomer, hydrate, solvate or the like.

By "stable compound' or "stable structure" is meant a compound that is sufficiently robust to survive isolation to a useful degree of purity from a reaction mixture, and is capable of being subjected to further chemical transformation or, preferably, formulation into an efficacious therapeutic agent.

The compounds of this invention may contain one or more asymmetric centres, depending upon the location and nature of the various substituents desired. Asymmetric carbon atoms may be present in the (R) or (S) configuration, resulting in racemic mixtures in the case of a single asymmetric centre, and diastereomeric mixtures in the case of multiple asymmetric centres. In certain instances, asymmetry may also be present due to restricted rotation about a given bond, for example, the central bond adjoining two substituted aromatic rings of the specified compounds, (i.e., atropisomers).

Substituents on a non-planar ring may also be present in either cis or trans configurations. It is intended that all such configurations (including enantiomers and diastereomers), are included within the scope of the present invention.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

Separated optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (e.g., chiral HPLC columns), with or without conventional derivatisation, optimally chosen to maximise the separation of the enantiomers. Suitable chiral HPLC columns are manufactured by Daicel, e.g., Chiracel OD and Chiracel OJ among many others, all routinely selectable. Enzymatic separations, with or without derivatisation, are also useful. The optically active compounds of this invention can likewise be obtained by chiral syntheses utilizing optically active starting materials or optically active catalysts.

In order to distinguish different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, e.g. R- or S- isomers, or E- or Z-isomers, in any ratio. Isolation of a single stereoisomer, e.g. a single enantiomer or a single diastereomer, of a compound of the present invention may be achieved by any suitable state of the art method, such as chromatography, especially chiral chromatography, for example.

Further, the compounds of the present invention may exist as tautomers. For example, any compound of the present invention which contains a pyrazole moiety as a heteroaryl group for example can exist as a 1H tautomer, or a 2H tautomer, or even a mixture in any amount of the two tautomers, or a triazole moiety for example can exist as a 1H tautomer, a 2H tautomer, or a 4H tautomer, or even a mixture in any amount of said 1H, 2H and 4H tautomers, namely :

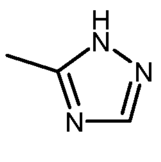

1H-tautomer

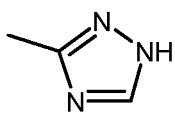

2H-tautomer

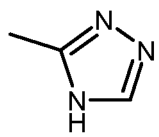

4H-tautomer.

Specifically, the compound of formula (I) may exist, at least as the following tautomers:

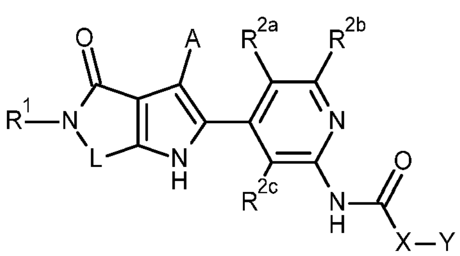

,

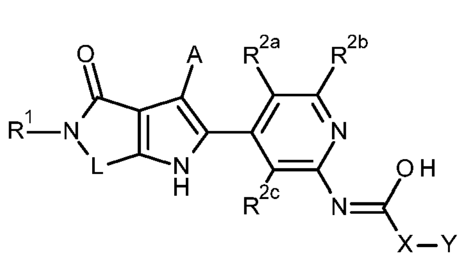

,

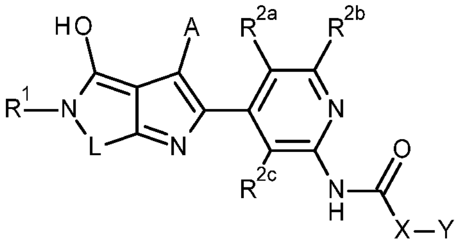

, and

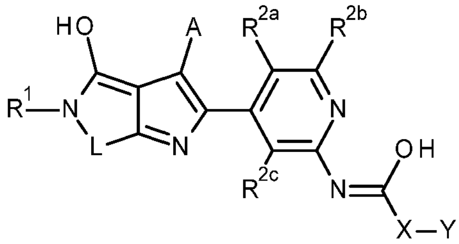

.

More specifically, when R' is hydrogen, the compound of formula (I) may exist, at least as the following tautomers:

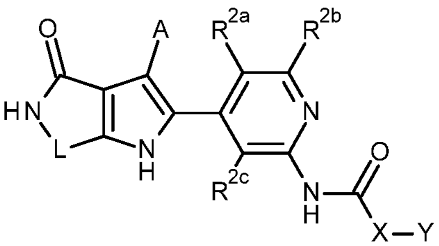

,

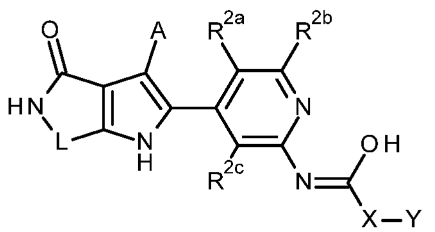

,

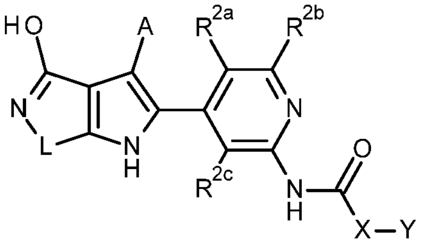

, and

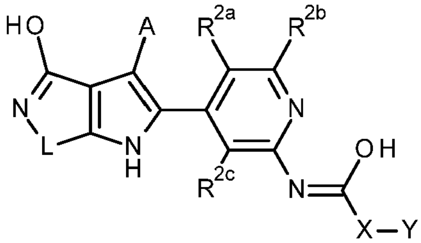

.

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also relates to useful forms of the compounds as disclosed herein, such as metabolites, hydrates, solvates, prodrugs, salts, in particular pharmaceutically acceptable salts, and co-precipitates.

The compounds of the present invention can exist as a hydrate, or as a solvate, wherein the compounds of the present invention contain polar solvents, preferably water, methanol or ethanol for example as structural element of the crystal lattice of the compounds. The amount of polar solvents, in particular water, may exist in a stoichiometric or non-stoichiometric ratio. In the case of stoichiometric solvates, e.g. a hydrate, hemi-, (semi-), mono-, sesqui-, di-, tri-, tetra-, penta- *etc.* solvates or hydrates, respectively, are possible. The present invention includes all such hydrates or solvates.

Further, the compounds of the present invention can exist in free form, e.g. as a free base, or as a free acid, or as a zwitterion, or can exist in the form of a salt. Said salt may be any salt, either an organic or inorganic addition salt, particularly any pharmaceutically acceptable organic or inorganic addition salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, para-toluenesulfonic, methansulfonic, 2-naphthalenesulfonic, naphthalenedisulfonic, camphorsulfonic acid, citric, tartaric, stearic, lactic, oxalic, malonic, succinic, malic,

adipic, alginic, maleic, fumaric, D-gluconic, mandelic, ascorbic, glucoheptanoic, glycerophosphoric, aspartic, sulfosalicylic, hemisulfuric or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and stearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention can be prepared by reacting the compounds of the invention with the appropriate base via any of a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae such as "hydrochloride", "trifluoroacetate", "sodium salt", or "x HCl", "x $CF_3COOH$", "x $Na^+$", for example, are to be understood as not a stoichiometric specification, but simply as a salt form.

This applies analogously to cases in which synthesis intermediates or example compounds or salts thereof have been obtained, by the preparation and/or purification processes described, as solvates, such as hydrates with (if defined) unknown stoichiometric composition.

The salts include water-insoluble and, particularly, water-soluble salts.

Furthermore, derivatives of the compounds of formula (I) and the salts thereof which are converted into a compound of formula (I) or a salt thereof in a biological system (bioprecursors or pro-drugs) are covered by the invention. Said biological system is e.g. a mammalian organism, particularly a human subject. The bioprecursor is, for example, converted into the compound of formula (I) or a salt thereof by metabolic processes.

As used herein, the term "*in vivo* hydrolysable ester" is understood as meaning an *in vivo* hydrolysable ester of a compound of the present invention containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, $C_1$-$C_6$ alkoxymethyl esters, *e.g.* methoxymethyl, $C_1$-$C_6$ alkanoyloxymethyl esters, e.g. pivaloyloxymethyl, phthalidyl esters, $C_3$-$C_8$ cycloalkoxy-carbonyloxy-$C_1$-$C_6$ alkyl esters, e.g. 1-cyclohexylcarbonyloxyethyl, 1,3-dioxolen-2-onylmethyl esters, *e.g.* 5-methyl-1,3-dioxolen-2-onylmethyl, and $C_1$-$C_6$-alkoxycarbonyloxyethyl esters, *e.g.* 1-methoxycarbonyloxyethyl, and may be formed at any carboxy group in the compounds of this invention.

An *in vivo* hydrolysable ester of a compound of the present invention containing a hydroxy group includes inorganic esters such as phosphate esters and [alpha]-acyloxyalkyl ethers and related compounds which as a result of the *in vivo* hydrolysis of the ester breakdown to give the parent hydroxy group. Examples of [alpha]-acyloxyalkyl ethers include acetoxymethoxy and 2,2-dimethylpropionyloxymethoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The present invention covers all such esters.

Furthermore, the present invention includes all possible crystalline forms, or polymorphs, of the compounds of the present invention, either as single polymorphs, or as a mixture of more than one polymorphs, in any ratio.

In the context of the properties of the compounds of the present invention the term "pharmacokinetic profile" means one single parameter or a combination thereof including permeability, bioavailability, exposure, and pharmacodynamic parameters such as duration, or magnitude of pharmacological effect, as measured in a suitable experiment. Compounds with improved pharmacokinetic profiles can, for example, be used in lower doses to achieve the same effect, may achieve a longer duration of action, or a may achieve a combination of both effects.

The term "combination" in the present invention is used as known to persons skilled in the art and may be present as a fixed combination, a non-fixed combination or kit-of-parts.

A "fixed combination" in the present invention is used as known to persons skilled in the art and is defined as a combination wherein the said first active ingredient and the said second active ingredient are present together in one

unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A non-fixed combination or "kit-of-parts" in the present invention is used as known to persons skilled in the art and is defined as a combination wherein the said first active ingredient and the said second active ingredient are present in more than one unit. One example of a non-fixed combination or kit-of-parts is a combination wherein the said first active ingredient and the said second active ingredient are present separately. The components of the non-fixed combination or kit-of-parts may be administered separately, sequentially, simultaneously, concurrently or chronologically staggered. Any such combination of a compound of formula (I) of the present invention with an anti-cancer agent as defined below is an embodiment of the invention.

The term "(chemotherapeutic) anti-cancer agents" includes but is not limited to: 131I-chTNT, abarelix, abiraterone, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, aprepitant, arcitumomab, arglabin, arsenic trioxide, asparaginase, atezolizumab, axitinib, azacitidine, basiliximab, belotecan, bendamustine, besilesomab, belinostat, bevacizumab, bexarotene, bicalutamide, bisantrene, bleomycin, blinatumomab, bortezomib, buserelin, bosutinib, brentuximab vedotin, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, copanlisib , crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, eculizumab, edrecolomab, elliptinium acetate, elotuzumab, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (123I), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, necitumumab, nedaplatin, nelarabine, neridronic acid, netupitant/palonosetron, nivolumab, pentetreotide, nilotinib, nilutamide, nimorazole, nimotuzumab, nimustine, nintedanib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, rolapitant, romidepsin, romiplostim, romurtide, rucaparib, samarium (153Sm) lexidronam, sargramostim, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin,

tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

General Procedures

The compounds according to the invention can be prepared according to the following Schemes 1 through 6.

The schemes and procedures described below illustrate synthetic routes to the compounds of general formula (I) of the invention and are not intended to be limiting. It is obvious to the person skilled in the art that the order of transformations as exemplified in the schemes can be modified in various ways. The order of transformations exemplified in the schemes is therefore not intended to be limiting. In addition, interconversion of any of the substituents $R^1$, $R^{2a}$, $R^{2b}$, $R^{2c}$, $R^{17}$, $R^{18}$,$R^{19}$, $R^{20}$, $R^{21}$, $R^{22}$, A, X, Y, Z, $Z^1$, $Z^2$, $Z^3$, $Z^4$, and PG can be achieved before and/or after the exemplified transformations. These modifications can be such as the introduction of protecting groups, cleavage of protecting groups, reduction or oxidation of functional groups, dehydrogenation, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art.

Specific examples are described in the subsequent paragraphs.

## Scheme 1

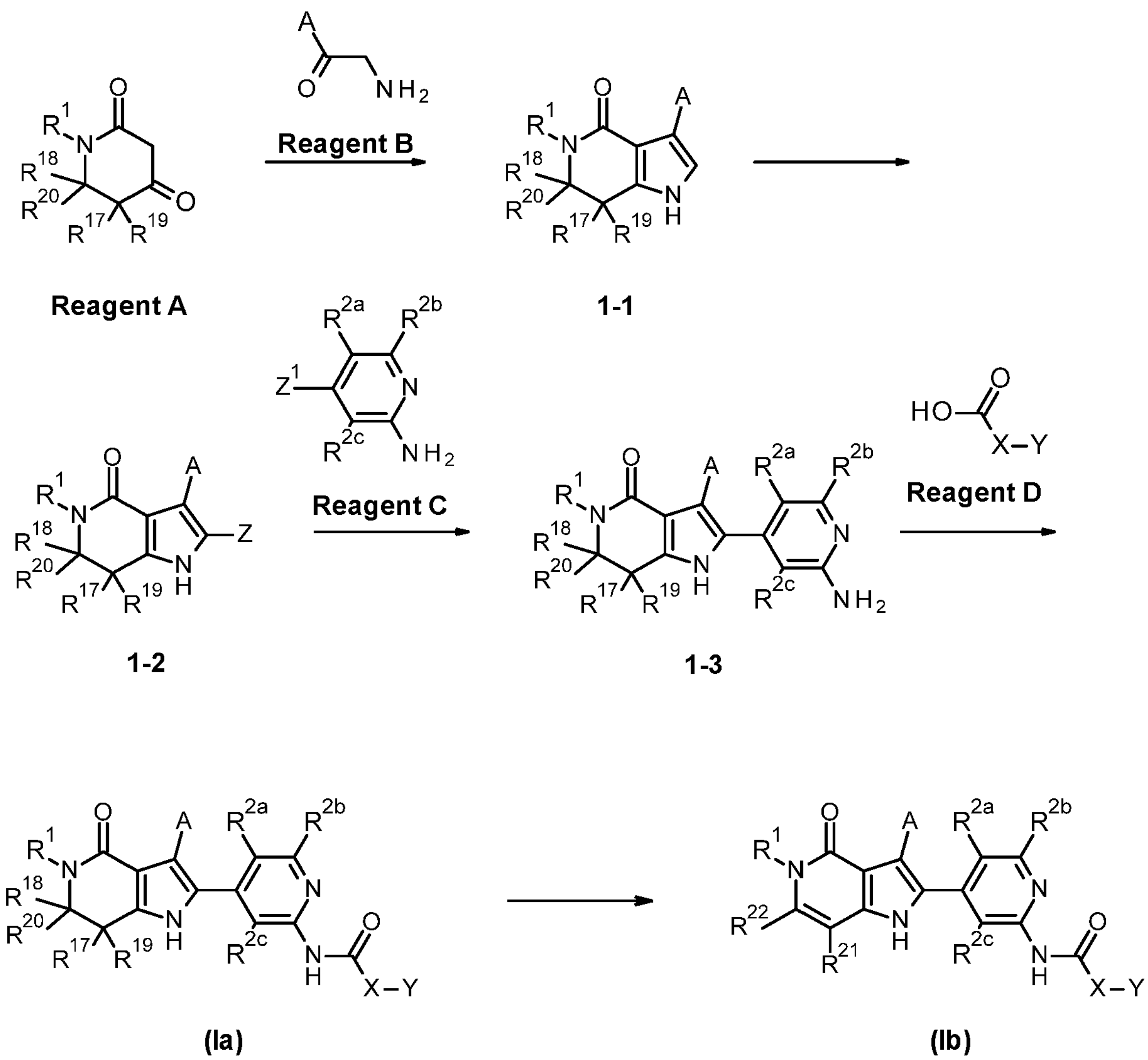


Reagent A, 1-1, 1-2, 1-3, (Ia), (Ib)

*Scheme* 1: Route for the preparation of compounds of general formula (Ia) and (Ib), wherein $R^1$, $R^{2a}$, $R^{2b}$, $R^{2c}$,

$R^{17}$, $R^{18}$,$R^{19}$, $R^{20}$, $R^{21}$, $R^{22}$, A, X, and Y have the meaning as given for general formula (I), supra. Z represents a chloro, a bromo or an iodo, preferably a bromo or an iodo and $Z^1$ represents a suitable stannane, boronic acid or boronic ester. In addition, interconversion of any of the substituents, $R^1$, $R^{2a}$, $R^{2b}$, $R^{2c}$, $R^{17}$, $R^{18}$,$R^{19}$, $R^{20}$, $R^{21}$, $R^{22}$, A, X, Y, Z and $Z^1$ can be achieved before and/or after the exemplified transformation.

Reagent A, reagent B, reagent C, and reagent D are either commercially available or can be prepared according to procedures available from the public domain, as understandable to the person skilled in the art. Specific examples are described in the subsequent paragraphs.

A suitably substituted piperidine-2,4-dione of general formula (reagent A), such as, for example, N-methyl-piperidine-2,4-dione, can be reacted with a suitably substituted amino-keton (reagent B), such as, for example, 2-amino-1-phenylethan-1-one, in a suitable solvent system, such as, for example, water, in the presence of a suitable base, such as, for example, potassium acetate, at temperatures ranging from rt to +100°C. Preferably the reaction is carried out at 90°C to furnish compounds of general formula **1-1.** Similar reactions have been performed in the literature (Hart et al., WO2016/100166).

Intermediates of general formula '**1-1** can be reacted to introduce a substituent Z, which is preferably a halide, such reactions are known to those skilled in the art (see Menichincheri et al., WO2014/72220 A1 (introduction of bromide and iodide); Smith et al., Bioorg. Med. Chem. Lett., 2007, 17, 673 - 678 (introduction of bromide) Cee et al., WO2014/22752 A1 (introduction of bromide)) to furnish intermediates of the formula **1-2.**

Intermediates of general formula **1-2** can be reacted with reagent C to introduce the substituted pyridinyl substituent, using metal-catalyzed reactions, such as, for example, the Suzuki reaction. Such reactions are known to those skilled in the art (WO2007/39740 A2; Cee et al., WO2014/22752 A1; Smith et al., Bioorg. Med. Chem. Lett., 2007, 17, 673 - 678) and can be used to furnish intermediates of the formula **1-3.**

Intermediates of general formula **1-3** can be subjected to a peptide coupling by reaction with a carboxylic acid (reagent D) of formula Y-X-(=O)OH, in which X and Y are as defined for compounds of general formula (I), in the presence of a peptide coupling reagent, selected from HATU (O-(7-azabenzotriazol-1-yl)-N,N,N',N'-tetramethyluronium hexafluorophosphate), TBTU (O-(benzotriazol-1-yl)-N,N,N',N'-tetramethyluronium tetrafluoroborate), PyBOP (benzotriazol-1-yl-oxytripyrrolidinophosphonium hexafluorophosphate), or T3P (2,4,6-tripropyl-1,3,5,2,4,6-trioxatriphosphinane 2,4,6-trioxide), all of them being well known to the person skilled in the art and all of them being commercially available, in the presence of a base such as a tertiary aliphatic amine of the formula $N(C_1-C_4\text{-alkyl})_3$, or sodium bicarbonate, or potassium carbonate, in an appropriate solvent such as N,N-dimethylformamide, N,N-dimethylacetamide, dimethylsulfoxide, THF, dichloromethane or N-methyl pyrrolidin-2-one in a temperature range from 0°C to the boiling point of the respective solvent to furnish compounds of general formula (Ia). Specific examples are described in the Experimental Section.

Compounds of general formula (Ia) can be oxidized to general fomula (Ib) by reaction with an oxidizing agent, such as, for example, 2,3-dichloro-5,6-dicyano-1,4-benzochinone, in a suitable solvent system, such as, for example, 1,4-dioxane, at temperatures ranging from rt to +80°C. Preferably the reaction is carried out at 80°C to furnish compounds of general formula (1b). Similar reactions have been performed in the literature (Hart et al., WO2016/100166).

## Scheme 2

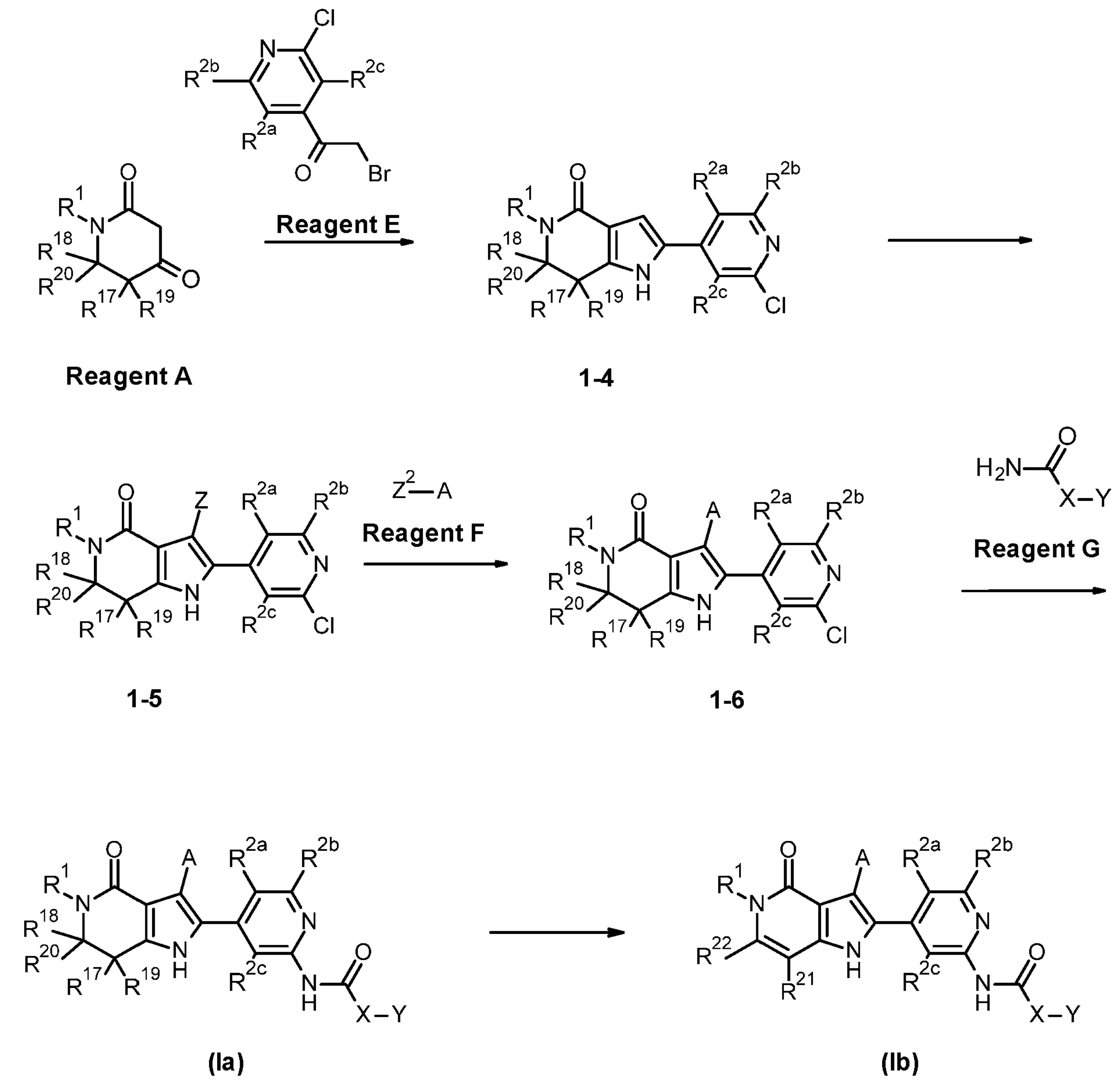


Reagent A

1-4

1-5

1-6

(Ia)

(Ib)

*Scheme 2:* Route for the preparation of compounds of general formula (Ia) and (Ib), wherein $R^1$, $R^{2a}$, $R^{2b}$, $R^{2c}$, $R^{17}$, $R^{18}$,$R^{19}$, $R^{20}$, $R^{21}$, $R^{22}$, A, X, and Y have the meaning as given for general formula (I), supra. Z represents a chloro, a bromo or an iodo, preferably a bromo or an iodo and $Z^2$ represents a suitable stannane, boronic acid or boronic ester. In addition, interconversion of any of the substituents, $R^1$, $R^{2a}$, $R^{2b}$, $R^{2c}$, $R^{17}$, $R^{18}$,$R^{19}$, $R^{20}$, $R^{21}$, $R^{22}$, A, X, Y, Z and $Z^2$ can be achieved before and/or after the exemplified transformation.

Reagent A, reagent E, reagent F, and reagent G are either commercially available or can be prepared according to procedures available from the public domain, as understandable to the person skilled in the art. Specific examples are described in the subsequent paragraphs.

A suitably substituted piperidine-2,4-dione of general formula (reagent A), such as, for example, N-methyl-piperidine-2,4-dione, can be reacted with a suitably substituted bromo-keton (reagent E), such as, for example, 2-bromo-1-(2-chloro-4-pyridyl)ethanone, in the presence of a nitrogen-source, such as, for example, ammonium acetate, in a suitable solvent system, such as, for example, ethanol, at temperatures ranging from 0 °C to rt. Preferably the reaction is carried out at rt to furnish intermediate of general formula **1-4.** Similar reactions have been performed in the literature (Anderson et al., Journal of Medicinal Chemistry, 2007, vol. 50, # 11, p. 2647 - 2654).

Intermediates of general formula **1-4** can be reacted to introduce a substituent Z, which is preferably a halide, such reactions are known to those skilled in the art (see Menichincheri et al., WO2014/72220 A1 (introduction of bromide and iodide); Smith et al., Bioorg. Med. Chem. Lett., 2007, 17, 673 - 678 (introduction of bromide) Cee et al., WO2014/22752

A1 (introduction of bromide)) to furnish intermediates of the formula **1-5.**

Intermediates of general formula **1-5** can be reacted with reagent F, a suitable stannane like A-Sn(nBu)$_3$, a boronic acid like A-B(OH)$_2$ or a suitable boronic ester, to introduce the substituted A, using metal-catalyzed reactions, such as, for example, the Suzuki reaction. Such reactions are known to those skilled in the art (WO2007/39740 A2; Cee et al., WO2014/22752 A1; Smith et al., Bioorg. Med. Chem. Lett., 2007, 17, 673 - 678) and can be used to furnish intermediates of the formula **1-6.**

Intermediates of general formula **1-6** can be subjected to a reaction with an amide (reagent G) of formula Y-X-(=O)NH2, in which X and Y are as defined for compounds of general formula (I), in the presence of a ligand, such as, for example, Xanthphos, a Palladium-catalyst, such as, for example, palladium acetate, a suitable base, such as, for example potassium carbonate, in a suitable solvent system, such as, for example, THF, in a temperature range from 0°C to the boiling point of the respective solvent to furnish compounds of general formula (Ia). Specific examples are described in the Experimental Section.

Compounds of general formula (Ia) can be oxidized to general fomula (Ib) by reaction with an oxidizing agent, such as, for example, 2,3-dichloro-5,6-dicyano-1,4-benzochinone, in a suitable solvent system, such as, for example, 1,4-dioxane, at temperatures ranging from rt to +80°C. Preferably the reaction is carried out at 80°C to furnish compounds of general formula (1b). Similar reactions have been performed in the literature (Hart et al.,WO2016/100166).

**Scheme 3**

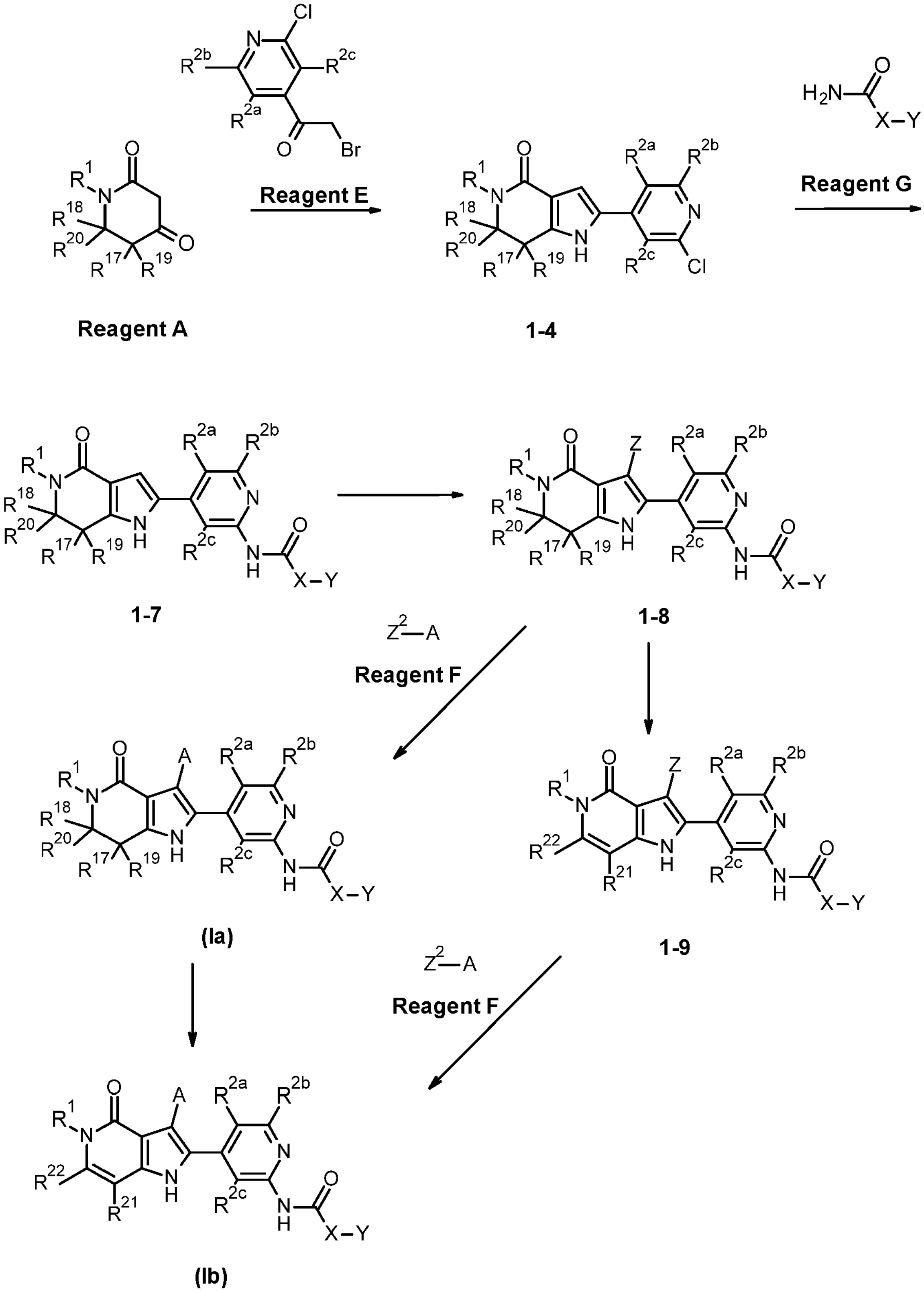


Reagent A

1-4

1-7

1-8

(Ia)

1-9

(Ib)

*Scheme 3:* Route for the preparation of compounds of general formula (Ia) and (Ib), wherein $R^1$, $R^{2a}$, $R^{2b}$, $R^{2c}$, $R^{17}$, $R^{18}$,$R^{19}$, $R^{20}$, $R^{21}$, $R^{22}$, A, X, and Y have the meaning as given for general formula (I), supra. Z represents a chloro, a bromo or an iodo, preferably a bromo or an iodo and $Z^2$ represents a suitable stannane, boronic acid or boronic ester.

In addition, interconversion of any of the substituents, $R^1$, $R^{2a}$, $R^{2b}$, $R^{2c}$, $R^{17}$, $R^{18}$,$R^{19}$, $R^{20}$, $R^{21}$, $R^{22}$, A, X, Y, Z and $Z^2$ can be achieved before and/or after the exemplified transformation.

Reagent A, reagent E, reagent F, and reagent G are either commercially available or can be prepared according to procedures available from the public domain, as understandable to the person skilled in the art. Specific examples are described in the subsequent paragraphs.

A suitably substituted piperidine-2,4-dione of general formula (reagent A), such as, for example, N-methyl-piperidine-2,4-dione, can be reacted with a suitably substituted bromo-keton (reagent E), such as, for example, 2-bromo-1-(2-chloro-4-pyridyl)ethanone, in the presence of a nitrogen-source, such as, for example, ammonium acetate, in a suitable solvent system, such as, for example, ethanol, at temperatures ranging from 0 °C to rt. Preferably the reaction is carried out at rt to furnish intermediate of general formula **1-4.** Similar reactions have been performed in the literature (Anderson et al., Journal of Medicinal Chemistry, 2007, vol. 50, # 11, p. 2647 - 2654).

Intermediates of general formula **1-4** can be subjected to a reaction with an amide (reagent G) of formula Y-X-(=O)NH2, in which X and Y are as defined for compounds of general formula (I), in the presence of a ligand, such as, for example, Xanthphos, a Palladium-catalyst, such as, for example, palladium acetate, a suitable base, such as, for example potassium carbonate, in a suitable solvent system, such as, for example, THF, in a temperature range from 0°C to the boiling point of the respective solvent to furnish intermediate of general formula **1-7.** Specific examples are described in the Experimental Section.

Intermediates of general formula **1-7** can be reacted to introduce a substituent Z, which is preferably a halide, such reactions are known to those skilled in the art (see Menichincheri et al., WO2014/72220 A1 (introduction of bromide and iodide); Smith et al., Bioorg. Med. Chem. Lett., 2007, 17, 673 - 678 (introduction of bromide) Cee et al., WO2014/22752 A1 (introduction of bromide)) to furnish intermediates of the formula **1-8.**

Intermediates of general formula **1-8** or **1-9** can be reacted with reagent F, a suitable stannane like A-Sn(nBu)$_3$, a boronic acid like A-B(OH)$_2$ or a suitable boronic ester, to introduce the substituted A, using metal-catalyzed reactions, such as, for example, the Suzuki reaction. Such reactions are known to those skilled in the art (WO2007/39740 A2; Cee et al., WO2014/22752 A1; Smith et al., Bioorg. Med. Chem. Lett., 2007, 17, 673 - 678) and can be used to furnish compounds of general formula **(Ia)** or **(Ib).**

Compounds of general formula (Ia) or intermediats of general formula **1-9** can be oxidized to general fomula (Ib) by reaction with an oxidizing agent, such as, for example, 2,3-dichloro-5,6-dicyano-1,4-benzochinone, in a suitable solvent system, such as, for example, 1,4-dioxane, at temperatures ranging from rt to +80°C. Preferably the reaction is carried out at 80°C to furnish compounds of general formula **(1b).** Similar reactions have been performed in the literature (Hart et al., WO2016/100166).

**Scheme 4**

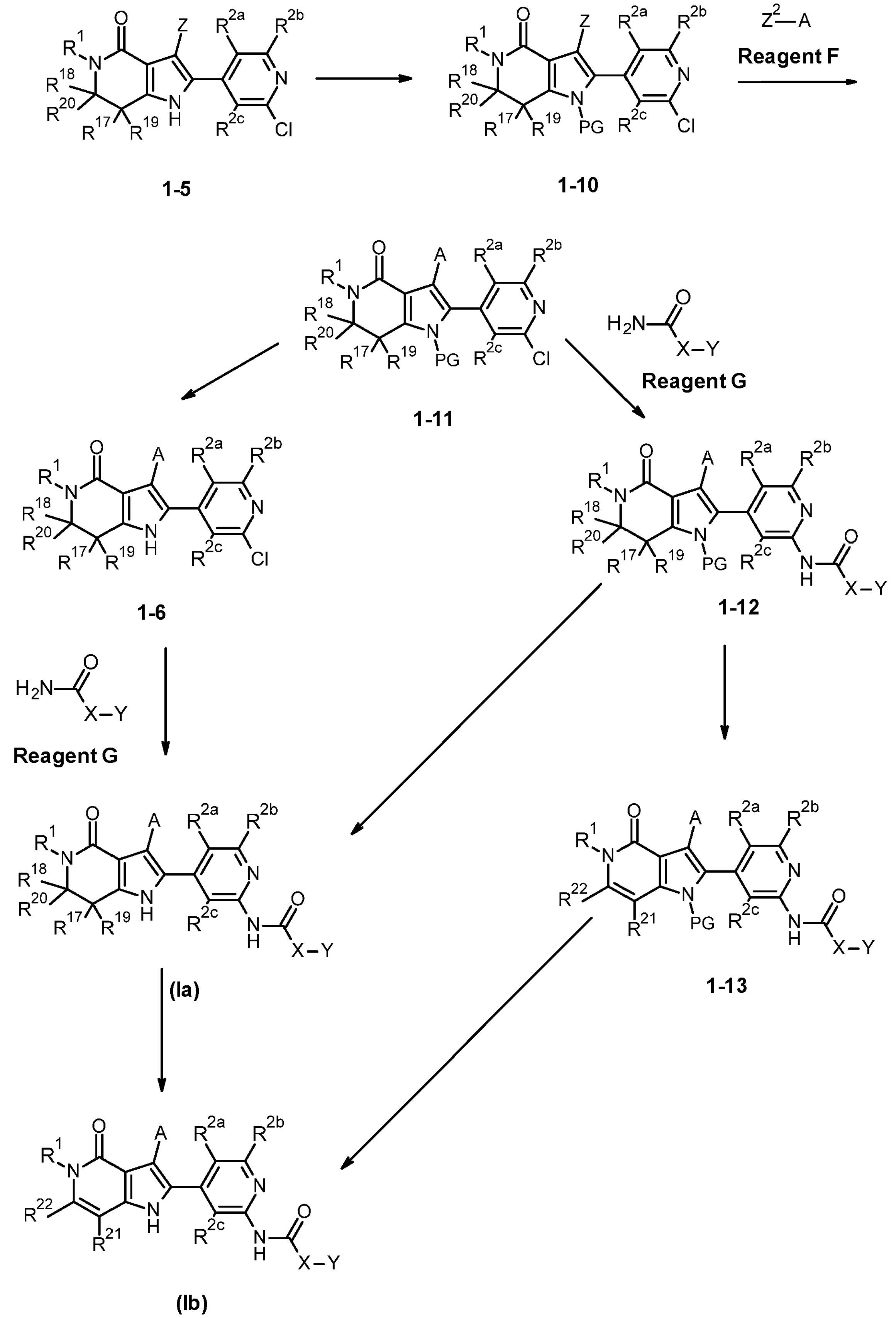


1-5 1-10 1-11 1-6 1-12 1-13 (Ia) (Ib)

*Scheme 4:* Route for the preparation of compounds of general formula (Ia) and (Ib), wherein $R^1$, $R^{2a}$, $R^{2b}$, $R^{2c}$, $R^{17}$, $R^{18}$,$R^{19}$, $R^{20}$, $R^{21}$, $R^{22}$, A, X, and Y have the meaning as given for general formula (I), supra. Z represents a chloro, a bromo or an iodo, preferably a bromo or an iodo and $Z^2$ represents a suitable stannane, boronic acid or boronic ester. In addition, interconversion of any of the substituents, $R^1$, $R^{2a}$, $R^{2b}$, $R^{2c}$, $R^{17}$, $R^{18}$,$R^{19}$, $R^{20}$, $R^{21}$, $R^{22}$, A, X, Y, Z and $Z^2$ can be achieved before and/or after the exemplified transformation.

Reagent F and reagent G are either commercially available or can be prepared according to procedures available from the public domain, as understandable to the person skilled in the art. Specific examples are described in the subsequent paragraphs.

The reactions shown in Scheme 2 can be performed with compounds that are protected at the pyrol-nitrogen. Examplified it is shown in scheme 4. The same protection strategy can be used for scheme 3.

Intermediates of general formula 1-5 can be reacted to introduce the PG group, such as, for example, a Tosyl-group or a SEM-group via alkylation under basic conditions (Marchionni et al., WO2009/40399 A1, also see for example T.W. Greene and P.G.M. Wutts in Protective Groups in Organic Synthesis, 3rd edition, Wiley 1999) or a tert-butoxycarbonyl (Boc) group (Kim et al., WO2013/62344 A1; Voss et al., WO2015/22073 A1) to furnish intermediates of the formula **1-10.**

Intermediates of general formula **1-10** can be reacted with reagent F, a suitable stannane like A-$Sn(nBu)_3$, a boronic acid like A-$B(OH)_2$ or a suitable boronic ester, to introduce the substituted A, using metal-catalyzed reactions, such as, for example, the Suzuki reaction. Such reactions are known to those skilled in the art (WO2007/39740 A2; Cee et al., WO2014/22752 A1; Smith et al., Bioorg. Med. Chem. Lett., 2007, 17, 673 - 678) and can be used to furnish intermediates of the formula **1-11.**

Intermediates of general formula **1-11** or **1-6** can be subjected to a reaction with an amide (reagent G) of formula Y-X-C(=O)NH2, in which X and Y are as defined for compounds of general formula (I), in the presence of a ligand, such as, for example, Xanthphos, a Palladium-catalyst, such as, for example, palladium acetate, a suitable base, such as, for example potassium carbonate, in a suitable solvent system, such as, for example, THF, in a temperature range from 0°C to the boiling point of the respective solvent to furnish intermediate of general fomular **1-12** or compounds of general formula (Ia). Specific examples are described in the Experimental Section.

Compounds of general formula (Ia) or intermediates of general formula **1-12** can be oxidized to general fomula (Ib) or intermediates of general formula **1-13** by reaction with an oxidizing agent, such as, for example, 2,3-dichloro-5,6-dicyano-1,4-benzochinone, in a suitable solvent system, such as, for example, 1,4-dioxane, at temperatures ranging from rt to +80°C. Preferably the reaction is carried out at 80°C to furnish compounds of general formula (**Ib**) or intermediates of general formula **1-13.** Similar reactions have been performed in the literature (Hart et al., WO2016/100166).

Intermediates of general formula **1-11** or **1-13** can be subjected to reaction conditions for the removal of protecting groups to furnish intermediates of general formula **1-6** or compounds of general formula (**Ib**), such as for example acidic conditions for the removal of a Boc group and TBAF for the removal of a SEM group. A comprehensive overview of conditions for possible protecting groups is outlined for example in T.W. Greene and P.G.M. Wutts in Protective Groups in Organic Synthesis, 3rd edition, Wiley 1999).

## Scheme 5

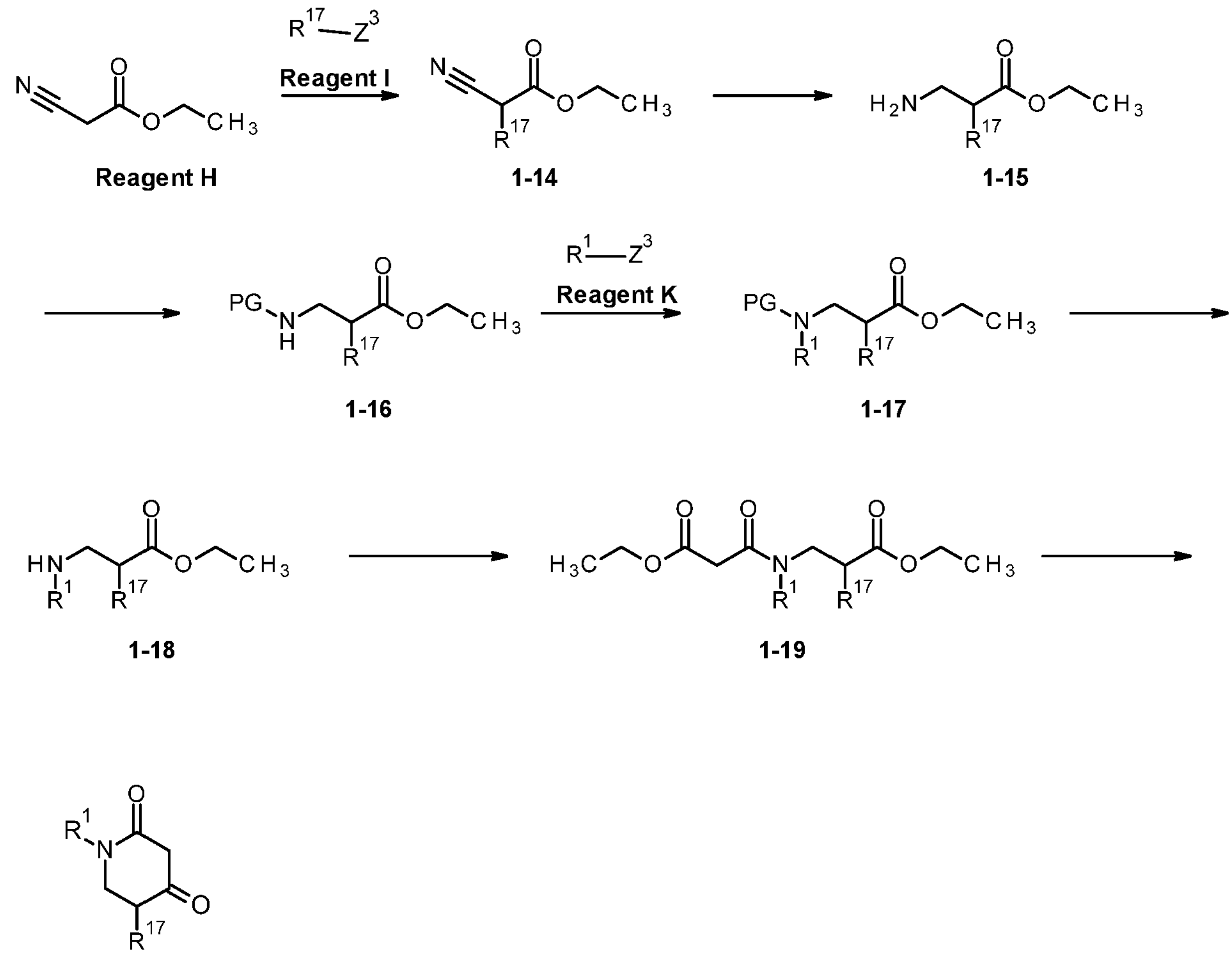


Reagent H

1-14

1-15

1-16

1-17

1-18

1-19

Reagent A-a

*Scheme 5:* Route for the preparation of reagent of general formula (A-a), wherein $R^1$ and $R^{17}$ have the meaning as given for general formula (I), supra. $Z^3$ represents a leaving group like a chloro, bromo, iodo, mesylate or a triflate. In addition, interconversion of any of the substituents, $R^1$, $R^{17}$ and $Z^3$ can be achieved before and/or after the exemplified transformation.

Reagent H, and reagent I are either commercially available or can be prepared according to procedures available from the public domain, as understandable to the person skilled in the art. Specific examples are described in the subsequent paragraphs.

Ethyl cyanoacetate (Reagent H) can be reacted with a suitably substituted reagent I, such as, for example, 2,2,2-trifluoroethyl trifluoromethanesulfonate, in the presence of a strong base, such as, for example, lithium bis(trimethylsilyl)amide, in a suitable solvent system, such as, for example, THF, at temperatures ranging from -70 °C to rt. Preferably the reaction is carried out at -70°C to furnish intermediate of general formula **1-14.**

An intermediate of general formula **1-14** can be reduced by hydrogenation in the presence of a catalyst, such as, for example platinum(IV) oxide, in a suitable solvent system, such as, for example, methanol, at temperatures ranging from 0°C to rt. Preferably the reaction is carried out at rt to furnish compounds of general formula **1-15.**

Intermediates of general formula **1-15** can be reacted to introduce the PG group, such as, for example, a Tosyl-group or a SEM-group via alkylation under basic conditions (Marchionni et al., WO2009/40399 A1, also see for example T.W. Greene and P.G.M. Wutts in Protective Groups in Organic Synthesis, 3rd edition, Wiley 1999) or a tert-butoxycarbonyl (Boc) group (Kim et al., WO2013/62344 A1; Voss et al., WO2015/22073 A1) or a Benzyloxycarbonyl (Z) group to furnish intermediates of the formula **1-16.**

An intermediate of general formula **1-16** can be alkylated with reagent K, such as, for example iodomethane, in the presence of a base, such as, for example, sodium hydride in a suitable solvent system, such as, for example, THF, at temperatures ranging from 0°C to rt. Preferably the reaction is carried out at 0 °C to furnish compounds of

general formula **1-17.**

Intermediates of general formula **1-17** can be subjected to reaction conditions for the removal of protecting groups to furnish intermediates of general formula **1-18,** such as for example acidic conditions for the removal of a Boc group and TBAF for the removal of a SEM group and hydrogynation for the removal of Z group. A comprehensive overview of conditions for possible protecting groups is outlined for example in T.W. Greene and P.G.M. Wutts in Protective Groups in Organic Synthesis, 3rd edition, Wiley 1999).

An intermediate of general formula **1-18** can build an amide bond by the addition of ethyl 3-chloro-3-oxopropanoate, in the presence of a base, such as, for example, N,N-Diisopropylethylamine and 4-dimethylaminopyridine, in a suitable solvent system, such as, for example, dichloromethane, at temperatures ranging from 0°C to rt. Preferably the reaction is carried out below 15 °C to furnish compounds of general formula **1-19.**

An intermediate of general formula **1-19** can cyclize and decarboxylate by the addition of a base, such as, for example, sodium ethoxide in a suitable solvent system, such as, for example, ethanol, at temperatures ranging from 0°C to rt. Preferably the reaction is carried out at 0°C and after workup dissolved in a sutiable solvent system, such as, for example acetonitrile and water, at temperatures ranging from rt to reflux. Preferably the reaction is carried out at reflux to furnish reagent of general formula A-a.

## Scheme 6

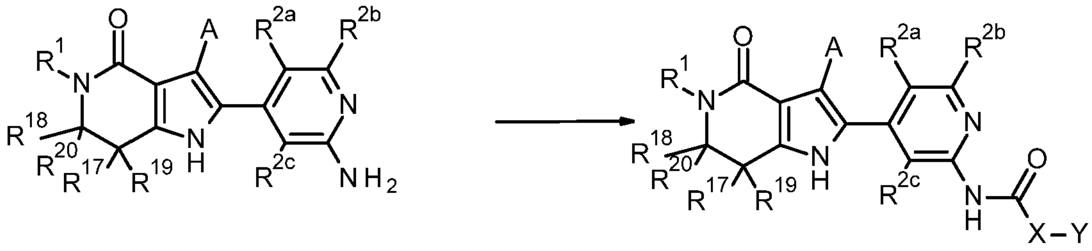

**1-3** **(Ia)**

*Scheme 6:* Route for the preparation of compounds of general formula (Ia), wherein $R^1$, $R^{2a}$, $R^{2b}$, $R^{2c}$, $R^{17}$, $R^{18}$,$R^{19}$, $R^{20}$, A, X, and Y have the meaning as given for general formula (I), supra. In addition, interconversion of any of the substituents, $R^1$, $R^{2a}$, $R^{2b}$, $R^{2c}$, $R^{17}$, $R^{18}$,$R^{19}$, $R^{20}$, A, X, and Y can be achieved before and/or after the exemplified transformation.

Intermediates of general formula **1-3** are reacted with an acylating reagent, an acylating agent which can be generated *in situ,* to furnish compounds of general formula (**I**). These types of reactions are well-known (selected literature examples are: S. Miwatashi, et al., J. Med. Chem., 2005, 48, 5966 - 5979; J. Zhao, et al., Bioorg. Med. Chem. Lett., 2014, 24,. 2802 - 2806; M. P. Hay, et al., J. Med. Chem., 2010, 53, 787 - 797; J. M. Keith, et al., Med. Chem. Lett, 2012, 3, 823 - 827; J. Liang, et al., Eur. J. Med. Chem., 2013, 67, 175 - 187).

Not-limiting examples of these types of reagents are:

i) carboxylic acids with dehydrating reagents typically used in amide bond formation, such as, for example (HBTU, HATU, PyBOP, BOP, T3P, EDC, DIC, DCC)
ii) acid fluorides, acid chlorides or acid bromides, preferably in the presence of a base
iii) acid anhydrides of the general formulae

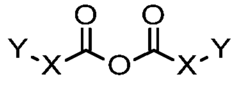

or

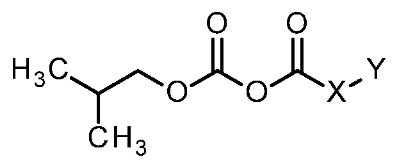

preferably in the presence of a base

Said acylating agent can be in a protected form, containing a protecting group like Boc for example leading to a protected form of compounds of general formula **(I)** which furnishes compounds of general formula **(I)** after an additional deprotection step. Further protecting groups are well known to the person skilled in the art. A detailed description for the use of a large number of proven protective groups is found, for example, in T. W. Greene, Protective Groups in

Organic Synthesis, John Wiley & Sons, 1999, 3rd Ed., or in P. Kocienski, Protecting Groups, Thieme Medical Publishers, 2000.

It is known to the person skilled in the art that, if there are a number of reactive centers on a starting or intermediate compound, it may be necessary to block one or more reactive centers temporarily by protective groups in order to allow a reaction to proceed specifically at the desired reaction center. A detailed description for the use of a large number of proven protective groups is found, for example, in T. W. Greene, Protective Groups in Organic Synthesis, John Wiley & Sons, 1999, 3rd Ed., or in P. Kocienski, Protecting Groups, Thieme Medical Publishers, 2000.

The compounds according to the invention are isolated and purified in a manner known per se, e.g. by distilling off the solvent *in vacuo* and recrystallizing the residue obtained from a suitable solvent or subjecting it to one of the customary purification methods, such as chromatography on a suitable support material. Furthermore, reverse phase preparative HPLC may be applied. The compounds of the present invention which possess a sufficiently basic or acidic functionality, may result as a salt, such as, in the case of a compound of the present invention which is sufficiently basic, a trifluoroacetate or formate salt for example, or, in the case of a compound of the present invention which is sufficiently acidic, an ammonium salt for example. Salts of this type can either be transformed into its free base or free acid form, respectively, by various methods known to the person skilled in the art, or be used as salts in subsequent biological assays. Additionally, the drying process during the isolation of the compounds of the present invention may not fully remove traces of cosolvents, especially such as formic acid or trifluoroacetic acid, to give solvates or inclusion complexes. The person skilled in the art will recognise which solvates or inclusion complexes are acceptable to be used in subsequent biological assays. It is to be understood that the specific form (e.g. salt, free base, free acid, solvate, inclusion complex) of a compound of the present invention as isolated and described herein is not necessarily the only form in which said compound can be applied to a biological assay in order to quantify the specific biological activity.

Salts of the compounds of formula (I) according to the invention can be obtained by dissolving the free compound in a suitable solvent (for example a ketone such as acetone, methylethylketone or methyl isobutyl ketone, an ether such as diethyl ether, tetrahydrofuran or dioxane, a chlorinated hydrocarbon such as methylene chloride or chloroform, or a low molecular weight aliphatic alcohol such as methanol, ethanol or isopropanol) which contains the desired acid or base, or to which the desired acid or base is then added. The acid or base can be employed in salt preparation, depending on whether a mono- or polybasic acid or base is concerned and depending on which salt is desired, in an equimolar ratio or one differing therefrom. The salts are obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art. Especially preferred are hydrochlorides and the process used in the example section.

Pure diastereomers and pure enantiomers of the compounds and salts according to the invention can be obtained e.g. by asymmetric synthesis, by using chiral starting compounds or optically active catalysts in synthesis or by separating enantiomeric and diasteriomeric mixtures obtained in synthesis.

Enantiomeric and diastereomeric mixtures can be separated into the pure enantiomers and pure diastereomers by methods known to the person skilled in the art. Preferably, diastereomeric mixtures are separated by crystallization, in particular fractional crystallization, or chromatography. Enantiomeric mixtures can be separated e.g. by forming diastereomers with a chiral auxiliary agent, resolving the diastereomers obtained and removing the chiral auxiliary agent. As chiral auxiliary agents, for example, chiral acids can be used to separate enantiomeric bases such as e.g. mandelic acid and chiral bases can be used to separate enantiomeric acids by formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be separated using chiral separating columns in chromatography. Another suitable method for the isolation of enantiomers is the enzymatic separation.

One preferred aspect of the disclosure is the process for the preparation of the compounds of claims 1 to 5 according to the examples as well as the intermediates used for their preparation.

The intermediates used for the synthesis of the compounds of claims of formula (I) as described herein, as well as their use for the synthesis of the compounds of formula (I), are one further aspect of the present disclosure. Preferred intermediates are the Intermediate Examples as disclosed herein. The invention relates to intermediates, uses thereof and methods using intermediates, as set out in the claims.

Optionally, compounds of the formula (I) can be converted into their salts, or, optionally, salts of the compounds of the formula (I) can be converted into the free compounds. Corresponding processes are customary for the skilled person.

**Commercial utility**

As mentioned supra, the compounds of the present invention have surprisingly been found to effectively inhibit Casein kinase 1 alpha and/or delta finally resulting in cell death e.g. apoptosis and may therefore be used for the treatment or prophylaxis of diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses. In particular, the disclosed compounds can be used for the treatment or prophylaxis of diseases in which the uncontrolled cell growth, proliferation and/or survival, inappropriate cellular immune responses, or inappropriate cellular inflammatory responses are mediated by Casein kinase 1 alpha and/or delta, such as, for example, benign and malignant neoplasia, more specifically haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours including esophageal, gastric and colorectal tumours, endocrine tumours, mammary and other gynaecological tumours, urological tumours including renal, bladder and prostate tumours, skin tumours, and sarcomas, and/or metastases thereof, especially haematological tumours, solid tumours, and/or metastases of breast, bladder, bone, brain, central and peripheral nervous system, cervix, colon, endocrine glands (e.g. thyroid and adrenal cortex), endocrine tumours, endometrium, esophagus, gastrointestinal tumours, germ cells, kidney, liver, lung, larynx and hypopharynx, mesothelioma, ovary, pancreas, prostate, rectum, renal, small intestine, soft tissue, stomach, skin, testis, ureter, vagina and vulva as well as malignant neoplasias including primary tumors in said organs and corresponding secondary tumors in distant organs ("tumor metastases"). Haematological tumors can e.g be exemplified by aggressive and indolent forms of leukemia and lymphoma, namely non-Hodgkins lymphoma, chronic and acute myeloid leukemia (CML / AML), acute lymphoblastic leukemia (ALL), Hodgkins lymphoma, multiple myeloma and T-cell lymphoma. Also included are myelodysplastic syndrome, plasma cell neoplasia, paraneoplastic syndromes, and cancers of unknown primary site as well as AIDS related malignancies.

The term "inappropriate" within the context of the present invention, in particular in the context of "inappropriate cellular immune responses, or inappropriate cellular inflammatory responses", as used herein, is to be understood as preferably meaning a response which is associated with, responsible for, or results in, the pathology of said diseases.

The invention relates to a compound of general formula (I) according to any of claims 1 to 5 for use in the treatment or prophylaxis of a hyperproliferative disease and/or a disorder responsive to induction of cell death. Preferably, the compound is for use in the treatment or prophylaxis of diseases, especially the treatment, wherein the diseases are haematological tumours, solid tumours and/or metastases thereof. In another aspect the compound of formula (I) is for use in the prophylaxis and/or treatment of cervical tumours, lung tumours (such as lung carcinoma), colon tumours (such as colorectal carcinoma), or lymphoma (such as diffuse large B-cell lymphoma) and/or metastases thereof, especially preferred for the treatment thereof.

Also described herein is the use of a compound of formula (I) or a stereoisomer, a tautomer, an N-oxide, a hydrate, a solvate, or a salt thereof, particularly a pharmaceutically acceptable salt thereof, or a mixture of same, as described herein, in the manufacture of a medicament for the treatment or prophylaxis of a disease, wherein such disease is a hyperproliferative disorder or a disorder responsive to induction of cell death e.g. apoptosis. In an instance the disease is a haematological tumour, a solid tumour and/or metastases thereof. In another embodiment the disease is is a cervical tumour, a lung tumour (such as lung carcinoma), a colon tumour (such as colorectal carcinoma), or a lymphoma (such as diffuse large B-cell lymphoma and/or metastases thereof.

Methods of treating hyper-proliferative disorders

The present disclosure relates to a method for using the compounds of the present invention and compositions thereof, to treat mammalian hyper-proliferative disorders. Compounds can be utilized to inhibit, block, reduce, decrease, etc., cell proliferation and/or cell division, and/or produce cell death e.g. apoptosis. This method comprises administering to a mammal in need thereof, including a human, an amount of a compound of this invention, or a pharmaceutically acceptable salt, isomer, polymorph, metabolite, hydrate, solvate or ester thereof; etc. which is effective to treat the disorder. Hyper-proliferative disorders include but are not limited, e.g., psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypothalamic glioma, cerebellar and cerebral astrocytoma, medulloblastoma, ependymoma, as well as neuroectodermal and pineal tumour.

Tumours of the male reproductive organs include, but are not limited to prostate and testicular cancer. Tumours of the female reproductive organs include, but are not limited to endometrial, cervical, ovarian, vaginal, and vulvar cancer, as well as sarcoma of the uterus.

Tumours of the digestive tract include, but are not limited to anal, colon, colorectal, oesophageal, gallbladder, gastric, pancreatic, rectal, small-intestine, and salivary gland cancers.

Tumours of the urinary tract include, but are not limited to bladder, penile, kidney, renal pelvis, ureter, urethral and human papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer and squamous cell. Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this document is used conventionally, e.g., the management or care of a subject for the purpose of combating, alleviating, reducing, relieving, improving the condition of, *etc.,* of a disease or disorder, such as cancer.

Methods of treating kinase disorders

The present disclosure also provides methods for the treatment of disorders associated with aberrant mitogen extracellular kinase activity, including, but not limited to stroke, heart failure, hepatomegaly, cardiomegaly, diabetes, Alzheimer's disease, cystic fibrosis, symptoms of xenograft rejections, septic shock or asthma.

Effective amounts of compounds of the present invention can be used to treat such disorders, including those diseases (e.g., cancer) mentioned in the Background section above. Nonetheless, such cancers and other diseases can be treated with compounds of the present invention, regardless of the mechanism of action and/or the relationship between the kinase and the disorder.

The phrase "aberrant kinase activity" or "aberrant tyrosine kinase activity," includes any abnormal expression or activity of the gene encoding the kinase or of the polypeptide it encodes. Examples of such aberrant activity, include, but are not limited to, over-expression of the gene or polypeptide; gene amplificatio ; mutations which produce constitutively-active or hyperactive kinase activity; gene mutations, deletions, substitutions, additions, etc.

The present disclosure also provides for methods of inhibiting a kinase activity, especially of mitogen extracellular kinase, comprising administering an effective amount of a compound of the present invention, including salts, polymorphs, metabolites, hydrates, solvates, prodrugs (e.g.: esters) thereof, and diastereoisomeric forms thereof. Kinase activity can be inhibited in cells (*e.g., in vitro*), or in the cells of a mammalian subject, especially a human patient in need of treatment.

Methods of treating angiogenic disorders

The present disclosure also provides methods of treating disorders and diseases associated with excessive and/or abnormal angiogenesis.

Inappropriate and ectopic expression of angiogenesis can be deleterious to an organism. A number of pathological conditions are associated with the growth of extraneous blood vessels. These include, e.g., diabetic retinopathy, ischemic retinal-vein occlusion, and retinopathy of prematurity [Aiello et al. New Engl. J. Med. 1994, 331, 1480 ; Peer et al. Lab. Invest. 1995, 72, 638], age-related macular degeneration [AMD ; see, Lopez et al. Invest. Opththalmol. Vis. Sci. 1996, 37, 855], neovascular glaucoma, psoriasis, retrolental fibroplasias, angiofibroma, inflammation, rheumatoid arthritis (RA), restenosis, in-stent restenosis, vascular graft restenosis, etc. In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer. Thus, compounds of the present invention can be utilized to treat and/or prevent any of the aforementioned angiogenesis disorders, e.g., by inhibiting and/or reducing blood vessel formation; by inhibiting, blocking, reducing, decreasing, etc. endothelial cell proliferation or other types involved in angiogenesis, as well as causing cell death, e.g., apoptosis, of such cell types.

Preferably, the diseases of said method are haematological tumours, solid tumour and/or metastases thereof.

The compounds of the present invention can be used in particular in therapy and prevention, e.g., prophylaxis, especially in therapy of tumour growth and metastases, especially in solid tumours of all indications and stages with or without pre-treatment of the tumour growth.

**Pharmaceutical compositions of the compounds of the invention**

This invention also relates to pharmaceutical compositions containing one or more compounds of the present invention. These compositions can be utilised to achieve the desired pharmacological effect by administration to a patient in need thereof. A patient, for the purpose of this invention, is a mammal, including a human, in need of treatment for the particular condition or disease.

Therefore, the present invention includes pharmaceutical compositions that are comprised of a pharmaceutically acceptable carrier or auxiliary and a pharmaceutically effective amount of a compound, or salt thereof, of the present invention.

Another aspect of the disclosure is a pharmaceutical composition comprising a pharmaceutically effective amount of a compound of formula (I) and a pharmaceutically acceptable auxiliary for the treatment of a disease mentioned *supra,* especially for the treatment of haematological tumours, solid tumours and/or metastases thereof.

A pharmaceutically acceptable carrier or auxiliary is preferably a carrier that is non-toxic and innocuous to a patient at concentrations consistent with effective activity of the active ingredient so that any side effects ascribable to the carrier do not vitiate the beneficial effects of the active ingredient. Carriers and auxiliaries are all kinds of additives assisting to the composition to be suitable for administration.

A pharmaceutically effective amount of compound is preferably that amount which produces a result or exerts the intended influence on the particular condition being treated.

The compounds of the present invention can be administered with pharmaceutically-acceptable carriers or auxiliaries well known in the art using any effective conventional dosage unit forms, including immediate, slow and timed release preparations, orally, parenterally, topically, nasally, ophthalmically, optically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatine type containing auxiliaries, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatine, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, colouring agents, and flavouring agents such as peppermint, oil of wintergreen, or cherry flavouring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or

to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavouring and colouring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan

monooleate, (4) condensation products of said partial esters with ethylene oxide, for example, polyoxyethylene sorbitan monooleate. The emulsions may also contain sweetening and flavouring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more colouring agents; one or more flavouring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavouring and colouring agents.

The compounds of this invention may also be administered parenterally, that is, subcutaneously, intravenously, intraocularly, intrasynovially, intramuscularly, or interperitoneally, as injectable dosages of the compound in preferably a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates ; anionic detergents, for example, alkyl, aryl, and olefin sulfonates, alkyl, olefin, ether, and monoglyceride sulfates, and sulfosuccinates ; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimise or eliminate irritation at the site of injection, such compositions may contain a non-ionic surfactant having a hydrophile-lipophile balance (HLB) preferably of from about 12 to about 17. The quantity of surfactant in such formulation preferably ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia ; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Controlled release formulations for parenteral administration include liposomal, polymeric microsphere and polymeric gel formulations that are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for administration, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions of the invention can also contain other conventional pharmaceutically acceptable compounding ingredients, generally referred to as carriers or diluents, as necessary or desired. Conventional procedures for preparing such compositions in appropriate dosage forms can be utilized.

Such ingredients and procedures include those described in the following references, each of which is incorporated herein by reference: Powell, M.F. et al., "Compendium of Excipients for Parenteral Formulations" PDA Journal of Pharmaceutical Science & Technology 1998, 52(5), 238-311 ; Strickley, R.G "Parenteral Formulations of Small Molecule Therapeutics Marketed in the United States (1999)-Part-1" PDA Journal of Pharmaceutical Science & Technology 1999, 53(6), 324-349 ; and Nema, S. et al., "Excipients and Their Use in Injectable Products" PDA Journal of Pharmaceutical Science & Technology 1997, 51(4), 166-171.

Commonly used pharmaceutical ingredients that can be used as appropriate to formulate the composition for its intended route of administration include:

acidifying agents (examples include, but are not limited to acetic acid, citric acid, fumaric acid, hydrochloric acid, nitric acid);

alkalinizing agents (examples include, but are not limited to ammonia solution, ammonium carbonate, diethanolamine, monoethanolamine, potassium hydroxide, sodium borate, sodium carbonate, sodium hydroxide, triethanolamine, trolamine);

adsorbents (examples include, but are not limited to powdered cellulose and activated charcoal);

aerosol propellants (examples include, but are not limited to carbon dioxide, $CCl_2F_2$, $F_2ClC\text{-}CClF_2$ and $CClF_3$);

air displacement agents - examples include, but are not limited to nitrogen and argon;

antifungal preservatives (examples include, but are not limited to benzoic acid, butylparaben, ethylparaben, methylparaben, propylparaben, sodium benzoate);

antimicrobial preservatives (examples include, but are not limited to benzalkonium chloride, benzethonium chloride, benzyl alcohol, cetylpyridinium chloride, chlorobutanol, phenol, phenylethyl alcohol, phenylmercuric nitrate and thimerosal);

antioxidants (examples include, but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);

binding materials (examples include, but are not limited to block polymers, natural and synthetic rubber, polyacrylates, polyurethanes, silicones, polysiloxanes and styrene-butadiene copolymers);

buffering agents (examples include, but are not limited to potassium metaphosphate, dipotassium phosphate, sodium acetate, sodium citrate anhydrous and sodium citrate dihydrate);

carrying agents (examples include, but are not limited to acacia syrup, aromatic syrup, aromatic elixir, cherry syrup, cocoa syrup, orange syrup, corn oil, mineral oil, peanut oil, sesame oil, bacteriostatic sodium chloride injection and bacteriostatic water for injection);

chelating agents (examples include, but are not limited to edetate disodium and edetic acid);

colourants (examples include, but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);

clarifying agents (examples include, but are not limited to bentonite);

emulsifying agents (examples include, but are not limited to acacia, cetomacrogol, cetyl alcohol, glyceryl monostearate, lecithin, sorbitan monooleate, polyoxyethylene 50 monostearate);

encapsulating agents (examples, include but are not limited to gelatin and cellulose acetate phthalate);

flavourants (examples include, but are not limited to anise oil, cinnamon oil, cocoa, menthol, orange oil, peppermint oil and vanillin);

humectants (examples include, but are not limited to glycerol, propylene glycol and sorbitol);

levigating agents (examples include, but are not limited to mineral oil and glycerin);

oils (examples include, but are not limited to arachis oil, mineral oil, olive oil, peanut oil, sesame oil and vegetable oil);

ointment bases (examples include, but are not limited to lanolin, hydrophilic ointment, polyethylene glycol ointment, petrolatum, hydrophilic petrolatum, white ointment, yellow ointment, and rose water ointment);

penetration enhancers (transdermal delivery) (examples include, but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas);

plasticizers (examples include, but are not limited to diethyl phthalate and glycerol);

solvents (examples include, but are not limited to ethanol, corn oil, cottonseed oil, glycerol, isopropanol, mineral oil, oleic acid, peanut oil, purified water, water for injection, sterile water for injection and sterile water for irrigation);

stiffening agents (examples include, but are not limited to cetyl alcohol, cetyl esters wax, microcrystalline wax, paraffin, stearyl alcohol, white wax and yellow wax);

suppository bases (examples include, but are not limited to cocoa butter and polyethylene glycols (mixtures));

surfactants (examples include, but are not limited to benzalkonium chloride, nonoxynol 10, oxtoxynol 9, polysorbate 80, sodium lauryl sulfate and sorbitan mono-palmitate);

suspending agents (examples include, but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);

sweetening agents (examples include, but are not limited to aspartame, dextrose, glycerol, mannitol, propylene glycol, saccharin sodium, sorbitol and sucrose);

tablet anti-adherents (examples include, but are not limited to magnesium stearate and talc);

tablet binders (examples include, but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);

tablet and capsule diluents (examples include, but are not limited to dibasic calcium phosphate, kaolin, lactose, mannitol, microcrystalline cellulose, powdered cellulose, precipitated calcium carbonate, sodium carbonate, sodium phosphate, sorbitol and starch);

tablet coating agents (examples include, but are not limited to liquid glucose, hydroxyethyl cellulose, hydroxypropyl cellulose, hydroxypropyl methylcellulose, methylcellulose, ethylcellulose, cellulose acetate phthalate and shellac);

tablet direct compression excipients (examples include, but are not limited to dibasic calcium phosphate);

tablet disintegrants (examples include, but are not limited to alginic acid, carboxymethylcellulose calcium, microcrystalline cellulose, polacrillin potassium, cross-linked polyvinylpyrrolidone, sodium alginate, sodium starch glycollate and starch);

tablet glidants (examples include, but are not limited to colloidal silica, corn starch and talc);

tablet lubricants (examples include, but are not limited to calcium stearate, magnesium stearate, mineral oil, stearic acid and zinc stearate);

tablet/capsule opaquants (examples include, but are not limited to titanium dioxide);

tablet polishing agents (examples include, but are not limited to carnuba wax and white wax);

thickening agents (examples include, but are not limited to beeswax, cetyl alcohol and paraffin);

tonicity agents (examples include, but are not limited to dextrose and sodium chloride);

viscosity increasing agents (examples include, but are not limited to alginic acid, bentonite, carbomers, carboxymethylcellulose sodium, methylcellulose, polyvinyl pyrrolidone, sodium alginate and tragacanth); and

wetting agents (examples include, but are not limited to heptadecaethylene oxycetanol, lecithins, sorbitol monooleate, polyoxyethylene sorbitol monooleate, and polyoxyethylene stearate).

Pharmaceutical compositions according to the present invention can be illustrated as follows:
Sterile i.v. solution: A 5 mg/mL solution of the desired compound of this invention can be made using sterile, injectable water, and the pH is adjusted if necessary. The solution is diluted for administration to 1 - 2 mg/mL with sterile 5% dextrose and is administered as an i.v. infusion over about 60 minutes.

Lyophilised powder for i.v. administration: A sterile preparation can be prepared with (i) 100 - 1000 mg of the desired compound of this invention as a lyophilised powder, (ii) 32- 327 mg/mL sodium citrate, and (iii) 300 - 3000 mg Dextran 40. The formulation is reconstituted with sterile, injectable saline or dextrose 5% to a concentration of 10 to 20 mg/mL, which is further diluted with saline or dextrose 5% to 0.2 - 0.4 mg/mL, and is administered either IV bolus or by IV infusion over 15 - 60 minutes.

Intramuscular suspension: The following solution or suspension can be prepared, for intramuscular injection:

50 mg/mL of the desired, water-insoluble compound of this invention
5 mg/mL sodium carboxymethylcellulose
4 mg/mL TWEEN 80
9 mg/mL sodium chloride
9 mg/mL benzyl alcohol

Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.

Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

Tablets: A large number of tablets are prepared by conventional procedures so that the dosage unit is 100 mg of active ingredient, 0.2 mg. of colloidal silicon dioxide, 5 mg of magnesium stearate, 275 mg of microcrystalline cellulose, 11 mg of starch, and 98.8 mg of lactose. Appropriate aqueous and non-aqueous coatings may be applied to increase palatability, improve elegance and stability or delay absorption.

Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

Dose and administration

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

**Combination Therapies**

The compounds of this invention can be administered as the sole pharmaceutical agent or in combination with one or more other pharmaceutical agents where the combination causes no unacceptable adverse effects. Those combined pharmaceutical agents can be other agents having anti proliferative effects such as for example for the treatment of haematological tumours, solid tumours and/or metastases thereof and/or agents for the treatment of undesired side effects. The present invention relates also to such combinations.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), which is hereby incorporated by reference, especially (chemotherapeutic) anti-cancer agents as defined supra. The combination can be a non-fixed combination or a fixed-dose combination as the case may be.

Methods of testing for a particular pharmacological or pharmaceutical property are well known to persons skilled in the art.

The example testing experiments described herein serve to illustrate the present invention and the invention is not limited to the examples given.

As will be appreciated by persons skilled in the art, the invention is not limited to the particular embodiments described herein, but covers all modifications of said embodiments that are within the spirit and scope of the invention as defined by the appended claims.

The following examples illustrate the invention in greater detail, without restricting it. Further compounds according to the invention, of which the preparation is not explicitly described, can be prepared in an analogous way.

The compounds, which are mentioned in the examples and the salts thereof represent preferred embodiments of the invention as well as a claim covering all subcombinations of the residues of the compound of formula (I) as disclosed by the specific examples.

The term "according to" within the experimental section is used in the sense that the procedure referred to is to be used "analogously to".

## EXPERIMENTAL PART

Table 1 lists the abbreviations used in this paragraph and in the Intermediates and Examples sections as far as they are not explained within the text body.

**Table 1**

| Abbreviation | Meaning |
|---|---|
| AcOH | acetic acid (ethanoic acid) |
| aq. | aqueous |
| Boc | t-butoxycarbonyl |
| BOP | (benzotriazol-1-yloxy)tris(dimethylamino)phosphonium hexafluorophosphate |
| br | broad |
| CI | chemical ionisation |
| COMU | {[(Z)-(1-cyano-2-ethoxy-2-oxoethylidene)amino]oxy}-N,N-dimethylmorpholin-4-ylmethaniminium hexafluorophosphate(1-) |
| $CS_2CO_3$ | caesium carbonate |
| d | doublet |
| DAD | diode array detector |
| DBU | 1,8-diazabicyclo(5.4.0)undec-7-ene |
| DCC | N,N'-dicyclohexylcarbodiimide |
| DCM | dichloromethane |
| dd | double-doublet |
| DIC | N,N'-diisopropylcarbodiimide |
| DIPEA | diisopropylethylamine |
| DMA | Dimethylacetamide |
| DMF | *N,N*-dimethylformamide |
| DMSO | dimethyl sulfoxide |
| dt | double-triplet |
| EDC | 1-ethyl-3-(3-dimethylaminopropyl)carbodiimide |
| ELSD | Evaporative Light Scattering Detector |
| EtOAc | ethyl acetate |
| EtOH | ethanol |
| eq. | equivalent |
| ESI | electrospray (ES) ionisation |
| h | hour |
| HATU | 1-[bis(dimethylamino)methylene]-1H-1,2,3-triazolo[4,5-b]pyridinium 3-oxid hexafluorophosphate |
| HBTU | (o-benzotriazole-10yl)-N,N,N',N,-tetramethyluronium hexafluorophosphate |
| HCl | hydrochloric acid |
| HPLC | high performance liquid chromatography |
| $K_2CO_3$ | potassium carbonate |
| LC-MS | liquid chromatography mass spectrometry |

(continued)

| Abbreviation | Meaning |
|---|---|
| m | multiplet |
| mCPBA | meta-Chloroperbenzoic acid |
| min | minute |
| MeCN | acetonitrile |
| MeOH | methanol |
| MS | mass spectrometry |
| MTBE | methyl-tert-butyl ether |
| NaCl | sodium chloride |
| NBS | 1-bromopyrrolidine-2,5-dione, N-Bromosuccinimide |
| $NaHCO_3$ | sodium hydrogen carbonate or sodium bicarbonate |
| NMR | nuclear magnetic resonance spectroscopy: chemical shifts (δ) are given in ppm. The chemical shifts were |
| | corrected by setting the DMSO signal to 2.50 ppm unless otherwise stated. |
| $Na_2SO_4$ | sodium sulfate |
| PDA | Photo Diode Array |
| Pd/C | palladium on activated charcoal |
| PyBOP | (benzotriazol-1-yloxy)tripyrrolidinophosphonium hexafluorophosphate |
| q | quartet |
| r.t. or rt or RT | room temperature |
| | |
| Rt | retention time (as measured either with HPLC or UPLC) in minutes |
| s | singlet |
| sat. | saturated |
| SEM | (2-methoxyethyl)(trimethyl)silyl group |
| SEM-Cl | [2-(chloromethoxy)ethyl](trimethyl)silane |
| SFC | supercritical fluid chromatography |
| SIBX | stabilized 2-iodoxybenzoic acid |
| SM | starting material |
| SQD | Single-Quadrupole-Detector |
| T3P | propylphosphonic anhydride |
| t | triplet |
| tBuBrettPhos or *t*-BuBrett Phos or *t*-BuBrett Phos | di-tert-butyl(2',4',6'-triisopropyl-3,6-dimethoxy[biphenyl]-2-yl)phosphine (CAS 1160861-53-9) |

(continued)

| Abbreviation | Meaning |
|---|---|
| tBuBrettPhos Pd G3<br>or<br>*t*-BuBrettPhos Pd G3<br>or<br>*t*-BuBrettPhos-gen 3 | (2'-amino[biphenyl]-2-yl)(methanesulfonato-kappaO)palladium - di-tert-butyl(2',4',6'-triisopropyl-3,6-dimethoxy[biphenyl]-2-yl)phosphine (1:1) (CAS: 1536473-72-9) |
| td | triple-doublet |
| TEA | triethylamine |
| TFA | trifluoroacetic acid |
| THF | tetrahydrofuran |
| UPLC | ultra performance liquid chromatography |
| XPhos or X-Phos | Dicyclohexyl[2',4',6'-tris(propan-2-yl)[1,1'-biphenyl]-2-yl]phosphane;<br>Dicyclohexyl(2',4',6'-triisopropyl[biphenyl]-2-yl)phosphine;<br>CAS-RN:[564483-18-7] |
| XPhos Pd G3; or XPhos-Pd-G3 | (2-Dicyclohexylphosphino-2',4',6'-triisopropyl-1,1'-biphenyl)[2-(2'-amino-1,1'-biphenyl)]palladium(II) methanesulfonate |

Other abbreviations have their meanings customary per se to the skilled person.

The various aspects of the invention described in this application are illustrated by the following examples which are not meant to limit the invention in any way.

**Specific Experimental Descriptions**

NMR peak forms in the following specific experimental descriptions are stated as they appear in the spectra, possible higher order effects have not been considered.

Reactions employing microwave irradiation may be run with a Biotage Initiator® microwave oven optionally equipped with a robotic unit. The reported reaction times employing microwave heating are intended to be understood as fixed reaction times after reaching the indicated reaction temperature.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example pre-packed silica gel cartridges, e.g. from Separtis such as Isolute® Flash silica gel or Isolute® Flash $NH_2$ silica gel in combination with a Isolera® autopurifier (Biotage) and eluents such as gradients of e.g. hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (*e.g.* salt, free base etc) of a compound of the present invention as isolated as described herein is not necessarily the only form in which said compound can be applied to a biological assay in order to quantify the specific biological activity.

The percentage yields reported in the following examples are based on the starting component that was used in the lowest molar amount. Air and moisture sensitive liquids and solutions were transferred via syringe or cannula and introduced into reaction vessels through rubber septa. Commercial grade reagents and solvents were used without further purification. The term "concentrated in vacuo" refers to the use of a Buchi rotary evaporator at a minimum pressure

of approximately 15 mm of Hg. All temperatures are reported uncorrected in degrees Celsius (°C). In order that this invention may be better understood, the following examples are set forth. These examples are for the purpose of illustration only, and are not to be construed as limiting the scope of the invention in any manner. All publications mentioned herein are incorporated by reference in their entirety.

**Analytical conditions**

**UPLC-MS Standard Procedures**

UPLC-MS-data given in the subsequent specific experimental descriptions refer (unless otherwise noted) to the following conditions:

**Method 1:**

| | |
|---|---|
| ***System:*** | Waters Acquity UPLC-MS: Binary Solvent Manager, Sample Manager/Organizer, Column Manager, PDA, ELSD, SQD 3001 |
| ***Column:*** | Acquity BEH C18 1.7 50x2.1mm |
| ***Solvent:*** | A = water + 0.1% vol. formic acid (99%) |
| | B = acetonitrile |
| ***Gradient:*** | 0-1.6 min 1-99% B, 1.6-2.0 min 99% B |
| ***Flow:*** | 0.8 mL/min |
| ***Temperature:*** | 60 °C |
| ***Injection:*** | 2.0 µL |
| ***Detection:*** | DAD scan range 210-400 nm |
| | MS ESI+, ESI-, scan range 160-1000 m/z |
| | ELSD |

**Method 2:**

| | |
|---|---|
| ***System:*** | Waters Acquity UPLC-MS: Binary Solvent Manager, Sample Manager/Organizer, Column Manager, PDA, ELSD, SQD 3001 |
| ***Column:*** | Acquity BEH C18 1.7 50x2.1mm |
| ***Solvent:*** | A = water + 0.2% vol. ammonia (32%) |
| | B = acetonitrile |
| ***Gradient:*** | 0-1.6 min 1-99% B, 1.6-2.0 min 99% B |
| ***Flow:*** | 0.8 mL/min |
| ***Temperature:*** | 60 °C |
| ***Injection:*** | 2.0 µL |
| ***Detection:*** | DAD scan range 210-400 nm |
| | MS ESI+, ESI-, scan range 160-1000 m/z |
| | ELSD |

**Method 3:**

Instrument: Waters Acquity; Column: Waters Acquity BEH C18 50 mm × 2.1 mm × 1.7 μm; eluent A: Water (MilliQ) + 0.01 vol % formic acid, eluent B: acetonitrile + 0.01 vol % formic acid; gradient: 0-0.3 min 3-4% B, 0.3-1.5 min 4-95% B, 1.5-1.9 min 95% B; 1.9-2.0 min 5% B; flow: 0.65 mL/min; temperature: 50°C; DAD scan: 200-500 nm.

**Method** 4:

Instrument: Waters Acquity; Column: Waters Acquity BEH C18 50 mm × 2.1 mm x 1.7 μm; eluent A: Water (MilliQ) + 0.01 vol % formic acid, eluent B: acetonitrile + 0.01 vol % formic acid; gradient: 0-0.5 min 2-5% B, 0.5-4.0 min 5-95% B, 4.0-4.5 min 95% B, 4.5-5.0 min 95-2% B; flow: 0.65 mL/min; temperature: 45°C; DAD scan: 200-500 nm.

**Method 5:**

Instrument: Waters Acquity; Column: Waters Acquity BEH C18 50 mm × 2.1 mm × 1.7 μm; eluent A: Water (MilliQ) + 0.01 vol % formic acid, eluent B: acetonitrile + 0.01 vol % formic acid; gradient: 0-0.5 min 5% B, 0.5-9.5 min 5-95% B, 9.5-10.0 min 5% B, flow: 0.65 mL/min; temperature: 45°C; DAD scan: 200-500 nm.

**Method 6:**

Instrument: SHIMADZU LCMS-2020 SingleQuad; Column: Chromolith@Flash RP-18E 25-2 MM; eluent A: water + 0.0375 vol % trifluoroacetic acid, eluent B: acetonitrile + 0.01875 vol % trifluoroacetic acid; gradient: 0-0.8 min, 5-95% B, 0.8-1.2 min 95% B; flow 1.5 mL/min; temperature: 50 °C; PDA: 220 nm & 254 nm.

**Method 7:**

| 5-95AB_4MIN | | | | |
|---|---|---|---|---|
| Instrument | | SHIMADZU LCMS-2020; | | |
| Software | | LabSolution Version 5.97SP1 | | |
| HPLC | Column | Kinetex® EVO C18 2.1x30mm 5um | | |
| | Mobile Phase | A: 0.0375% TFA in water (v/v) | | |
| | | B: 0.01875% TFA in Acetonitrile (v/v) | | |
| | Gradient | Time(min) | B(%) | Flow(mL/min) |
| | | 0.0 | 5 | 0.8 |
| | | 3.0 | 95 | 0.8 |
| | | 3.60 | 95 | 0.8 |
| | | 3.61 | 5 | 0.8 |
| | | 4.00 | 5 | 0.8 |
| | Column Temp | 40°C | | |
| | Detector | PDA (220nm&254nm) | | |

(continued)

| | | |
|---|---|---|
| MS | Ionization source | ESI |
| | Drying Gas | N2 |
| | Drying Gas Flow | 15(L/min) |
| | DL Voltage | 120(v) |
| | Qarray DC Voltage | 20(V) |
| | MS Polarity | Positive |
| | MS Mode | Scan |
| | Mass range | 100-1000 |

**Flash column chromatography conditions**

"Purification by (flash) column chromatography" as stated in the subsequent specific experimental descriptions refers to the use of a Biotage Isolera purification system. For technical specifications see "Biotage product catalogue" on www.biotage.com.

Chemical names were generated using the ACD/Name software from ACD/Labs. In some cases generally accepted names of commercially available reagents were used in place of ACD/Name generated names.

Optical rotations were measured using a JASCO P2000 Polarimeter. Typical, a solution of the compound with a concentration of 1 mg/mL to 15 mg/mL was used for the measurement. The specific rotation $[\alpha]_D$ was calculated according to the following formula:

$$[\alpha]D = \frac{\propto}{\beta \times d}$$

In this equation, $\alpha$ is the measured rotation in degrees; d is the path length in decimetres and $\beta$ is the concentration in g/mL.

**EXPERIMENTAL SECTION - INTERMEDIATES**

**Intermediate** 1

5-methyl-3-phenyl-1,5,6,7-tetrahydro-4H-pyrrolo[3,2-c]pyridin-4-one

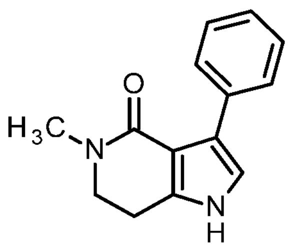

1-Methylpiperidine-2,4-dione (CAS-RN:[118263-97-1], 3.00 g, 23.6 mmol), 2-amino-1-phenylethan-1-one hydrogen chloride salt (CAS-RN:[5468-37-1], 4.05 g, 23.6 mmol) and potassium acetate (4.63 g, 47.2 mmol) were suspended in water (43 mL) and stirred at rt for 5 min. The mixture was heated at 90 °C over night. The suspension was diluted with some water. The solid in the mixture was filtered off under vacuum. The filter cake was washed with water twice and dried in a vacuum drying oven to provide the title compound: 5.22 g

LC-MS (Method 2): Rt = 0.84 min; MS (ESIpos): m/z = 227 $[M+H]^+$

$^1$H-NMR (400MHz, DMSO-$d_6$): δ [ppm]= 2.85 (t, 2H), 2.90 (s, 3H), 3.53 (t, 2H), 6.87 (d, 1H), 7.10 - 7.19 (m, 1H), 7.21 - 7.29 (m, 2H), 7.60 - 7.72 (m, 2H), 11.27 (br s, 1H)

**Intermediate** 2

2-bromo-5-methyl-3-phenyl-1,5,6,7-tetrahydro-4H-pyrrolo[3,2-c]pyridin-4-one

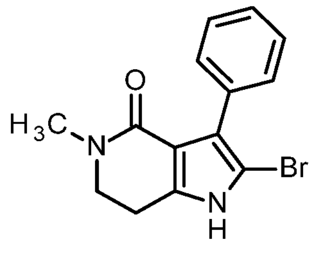

5-Methyl-3-phenyl-1,5,6,7-tetrahydro-4H-pyrrolo[3,2-c]pyridin-4-one (see Intermediate 1, 5.22 g) was suspended in DMF (53 mL). N-bromosuccinimide (CAS-RN:[128-08-5], 4.11 g, 23.1 mmol) was added and the mixture was stirred under $N_2$ at room temperature for 15 min. The reaction mixture was concentrated under reduced pressure and the residue was diluted with methanol. The resulting precipitate was filtered off. The filter cake was washed with methanol twice to provide the title compound as a grey solid: 5.51 g

LC-MS (Method 2): Rt = 0.94 min; MS (ESIpos): m/z = 305 $[M+H]^+$

$^1$H-NMR (400MHz, DMSO-$d_6$): δ [ppm]= 2.76 - 2.88 (m, 5H), 3.53 (t, 2H), 7.14 - 7.26 (m, 1H), 7.26 - 7.43 (m, 4H), 12.01 (br s, 1H)

**Intermediate 3**

2-(2-aminopyridin-4-yl)-5-methyl-3-phenyl-1,5,6,7-tetrahydro-4H-pyrrolo[3,2-c]pyridin-4-one

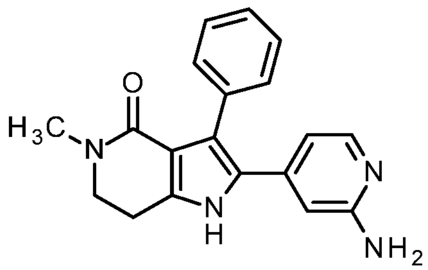

2-Bromo-5-methyl-3-phenyl-1,5,6,7-tetrahydro-4H-pyrrolo[3,2-c]pyridin-4-one (see Intermediate 2, 734 mg), 4-(4,4,5,5-tetramethyl-1,3,2-dioxaborolan-2-yl)pyridin-2-amine (794 mg, 3.61 mmol), cesium carbonate (2.35 g, 7.21 mmol) and XPhos Pd G3 (CAS-RN:[1445085-55-1], 204 mg, 240 μmol) were suspended in a degassed mixture of 1,4-dioxane (10 mL) and water (1.1 mL) and stirred at 80 °C overnight. The reaction mixture was diluted with a mixture of methylene chloride/isopropanole (7:3) and water. The layers were separated and the aqueous layer was extracted with methylene chloride/isopropanole once. The combined organic layers were dried using a waterresistant filter and concentrated under reduced pressure. The crude product was diluted with DCM. A beige solid precipitated. It was filtered off and dried at 50 °C for one hour to provide the title compound: 571 mg.

LC-MS (Method 2): Rt = 0.78 min; MS (ESIpos): m/z = 319 $[M+H]^+$

$^1$H-NMR (400 MHz, DMSO-$d_6$) δ [ppm]: -0.002 (1.74), 1.026 (1.66), 1.042 (1.70), 1.066 (2.47), 2.518 (1.30), 2.522 (0.82), 2.838 (16.00), 2.888 (1.55), 2.905 (3.49), 2.923 (1.69), 3.526 (1.94), 3.543 (3.76), 3.560 (1.71), 3.565 (2.80),

5.744 (3.88), 5.757 (0.90), 6.057 (0.75), 6.071 (2.01), 6.075 (2.02), 6.084 (1.84), 6.088 (1.91), 6.256 (2.84), 6.258 (2.80), 6.634 (0.54), 6.636 (0.60), 6.676 (0.41), 6.689 (0.41), 7.178 (1.49), 7.182 (2.15), 7.186 (1.08), 7.191 (0.94), 7.196 (3.08), 7.202 (4.12), 7.215 (1.71), 7.224 (2.40), 7.231 (5.03), 7.241 (1.22), 7.245 (1.54), 7.250 (2.30), 7.253 (0.59), 7.260 (0.41), 7.263 (0.74), 7.270 (0.42), 7.640 (2.48), 7.653 (2.48), 7.965 (0.48), 7.978 (0.47), 11.635 (1.59).

## Intermediate 4

5-methyl-3-(pyridin-3-yl)-1,5,6,7-tetrahydro-4H-pyrrolo[3,2-c]pyridin-4-one

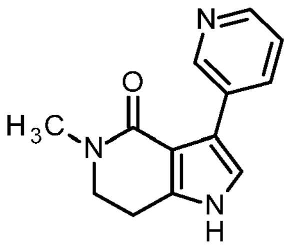

1-Methylpiperidine-2,4-dione (CAS-RN:[118263-97-1], 438 mg, 3.44 mmol), 2-amino-1-(pyridin-3-yl)ethan-1-one-hydrogen chloride salt (CAS-RN:[ 51746-82-8], 720 mg, 3.44 mmol) and potassium acetate (676 mg, 6.89 mmol) were suspended in water (6.2 mL) and stirred at rt for 5 min. The mixture was heated at 90 °C over night. The suspension was diluted with some water and a mixture of methylene chloride / isopropanole (7:3). The layers were separated and the aqueous layer was extracted with methylene chloride/isopropanole twice. The combined organic layers were dried using a waterresistant filter and concentrated under reduced pressure to provide the title compound: 703.3 mg.

LC-MS (Method 2): Rt = 0.63 min; MS (ESIpos): m/z = 228 $[M+H]^+$

$^1$H-NMR (400MHz, DMSO-$d_6$): δ [ppm]= 2.86 (t, 2H), 2.91 (s, 3H), 3.50 - 3.61 (m, 2H), 7.02 (d, 1H), 7.28 (ddd, 1H), 8.07 (dt, 1H), 8.34 (dd, 1H), 8.80 (dd, 1H), 11.42 (br s, 1H), 11.98 (br s, 1H).

## Intermediate 5

2-bromo-5-methyl-3-(pyridin-3-yl)-1,5,6,7-tetrahydro-4H-pyrrolo[3,2-c]pyridin-4-one

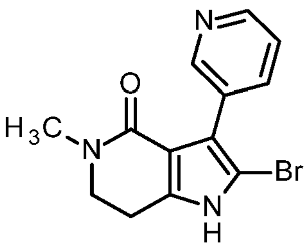

5-Methyl-3-(pyridin-3-yl)-1,5,6,7-tetrahydro-4H-pyrrolo[3,2-c]pyridin-4-one (see Intermediate 4, 700 mg, 3.08 mmol) was suspended in DMF (7.1 mL). N-bromosuccinimide (CAS-RN:[128-08-5], 548 mg, 3.08 mmol) was added and the mixture was stirred under $N_2$ at room temperature for 15 min. The reaction mixture was concentrated under reduced pressure and the residue was purified by chromatography (28 g amino column, gradient: dichloromethane/ethanol 0-20% ethanol) to provide the title compound: 390 mg.

LC-MS (Method 2): Rt = 0.70 min; MS (ESIpos): m/z = 306 $[M+H]^+$

$^1$H-NMR (400MHz, DMSO-$d_6$): δ [ppm]= 2.81 - 2.89 (m, 6H), 3.54 (t, 2H), 7.35 (ddd, 1H), 7.74 (dt, 1H), 8.43 (dd, 1H), 8.53 (dd, 1H), 12.18 (br s, 1H).

**Intermediate 6**

2-(2-aminopyridin-4-yl)-5-methyl-3-(pyridin-3-yl)-1,5,6,7-tetrahydro-4H-pyrrolo[3,2-c]pyridin-4-one

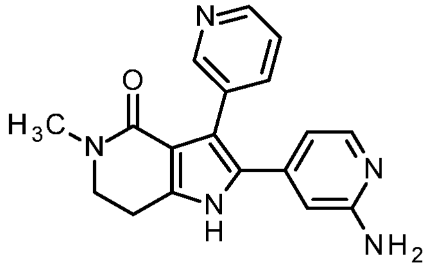

2-Bromo-5-methyl-3-(pyridin-3-yl)-1,5,6,7-tetrahydro-4H-pyrrolo[3,2-c]pyridin-4-one (see Intermediate 5, 380 mg), 4-(4,4,5,5-tetramethyl-1,3,2-dioxaborolan-2-yl)pyridin-2-amine (CAS-RN:[1195995-72-2], 410 mg, 1.86 mmol), caesium carbonate (1.21 g, 3.72 mmol) and XPhos Pd G3 (CAS-RN:[1445085-55-1], 105 mg, 124 μmol) were suspended in a degassed mixture of 1,4-dioxane (5.3 mL) and water (560 μL) and stirred at 80 °C overnight. The reaction mixture was diluted with a mixture of methylene chloride/isopropanole (7:3) and water. A beige solid in the layers was filtered off under vacuum to provide the title compound: 168 mg.

LC-MS (Method 2): Rt = 0.62 min; MS (ESIpos): m/z = 320 $[M+H]^+$

$^1$H-NMR (400MHz, DMSO-$d_6$): δ [ppm]= 2.85 (s, 3H), 2.92 (t, 2H), 3.53 - 3.60 (m, 2H), 5.83 (s, 2H), 6.13 (dd, 1H), 6.23 (s, 1H), 7.29 (dd, 1H), 7.62 (dt, 1H), 7.72 (d, 1H), 8.33 (d, 1H), 8.40 (dd, 1H), 11.43 - 12.06 (m, 1H)

**Intermediate** 7

ethyl-2-cyano-4,4,4-trifluorobutanoate (Racemate)

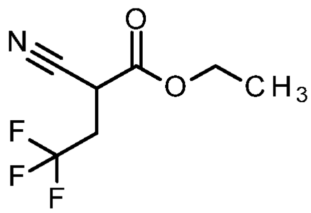

To a solution of ethyl cyanoacetate (CAS-RN:[ 105-56-6], 75.0 g, 663 mmol) in tetrahydrofuran (700 mL) was added lithium bis(trimethylsilyl)amide (663.03 mL, 1.0 M in tetrahydrofuran, 663.03 mmol) drop-wise at -70 °C. After addition, the mixture was stirred at -70 °C for 1 hour, a solution of 2,2,2-trifluoroethyl trifluoromethanesulfonate (CAS-RN:[ 6226-25-1], 154 g, 663 mmol) in tetrahydrofuran (100 mL) was added drop-wise and the resulting mixture was stirred at -70 °C for another 0.5 hour. Then the mixture was warmed to room temperature and stirred for 14 hours. Hydrochloric acid solution (1 M in water) was added and the mixture was extracted with ethyl acetate. The combined organic phase was dried over anhydrous sodium sulfate, filtered and concentrated to give a residue. The residue was purified by silica gel chromatography (petroleum ether: ethyl acetate = 100: 1 to 50: 1) to give 42 g ethyl-2-cyano-4,4,4-trifluorobutanoate (Racemate) as a yellow oil.

$^1$H NMR (400 MHz, $CDCl_3$): δ [ppm] = 4.34 (q, $J$ = 7.2 Hz, 2H), 3.78 (dd, J = 8.4, 5.2 Hz, 1H), 2.92-2.87 (m, 1H), 2.79-2.75 (m, 1H), 1.36 (t, $J$= 7.2 Hz, 1H).

**Intermediate 8**

ethyl-2-(aminomethyl)-4,4,4-trifluorobutanoate-hydrogen chloride salt (Racemate)

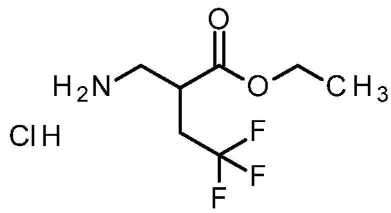

The reactions were performed as two batches in parallel: A mixture of ethyl-2-cyano-4,4,4-trifluorobutanoate (Racemate) (see Intermediate 7, 21.0 g, 108 mmol) and platinum(IV) oxide (2.44 g, 10.8 mmol) in methanol (300 mL) and hydrogen chloride (11 mL, 12 M in water, 130 mmol) was stirred at room temperature for 16 hours under hydrogen (15 psi). The mixture (combined with another batch starting from 21 g) was filtered and the filtrate was concentrated by evaporation in vacuum. Water and ethyl acetate were added and the mixture was separated. The aqueous phase was dried by lyophilization to give 45 g ethyl-2-(aminomethyl)-4,4,4-trifluorobutanoate hydrochloride salt (Racemate) as a white solid.

$^1H$ NMR (400 MHz, DMSO-$d_6$): δ [ppm] = 8.40 (brs, 2H), 4.18-4.12 (m, 2H), 3.10-3.00 (m, 3H), 2.82-2.68 (m, 2H), 1.21 (t, *J*= 7.2 Hz, 3H).

**Intermediate 9**

ethyl-2-({[(benzyloxy)carbonyl]amino}methyl)-4,4,4-trifluorobutanoate (Racemate)

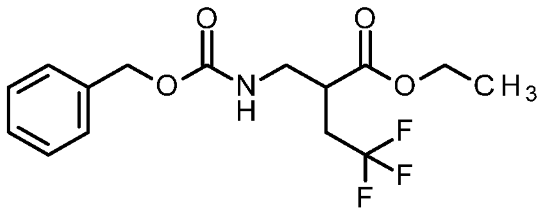

A mixture of ethyl-2-(aminomethyl)-4,4,4-trifluorobutanoate-hydrogen chloride salt (Racemate) (see Intermediate 8, 44.8 g, 190 mmol), benzyl carbonochloridate (41 mL, 290 mmol) and sodium hydrogen carbonate (63.9 g, 760 mmol) in water (400 mL) and dichloromethane (400 mL) was stirred at room temperature for 2 hours. Water was added and the mixture was extracted with dichloromethane. The combined organic phase was dried over anhydrous sodium sulfate, filtered and concentrated to give a residue. The residue was purified by column chromatography (300-400 mesh, petroleum ether: ethyl acetate = 20: 1 then 10: 1) to give 63 g ethyl-2-({[(benzyloxy)carbonyl]amino}methyl)-4,4,4-trifluorobutanoate (Racemate) as yellow oil.

$^1H$ NMR (400 MHz, $CDCl_3$): δ [ppm] = 7.39-7.35 (m, 5H), 5.11 (s, 2H), 4.71 (d, *J*= 6.0 Hz, 1H), 4.25-4.10 (m, 2H), 3.51-3.44 (m, 2H), 2.95-2.92 (m, 1H), 2.61-2.52 (m, 1H), 2.37-2.29 (m, 1H), 1.27 (t, *J*= 7.2 Hz, 3H).

**Intermediate 10**

ethyl-2-({[(benzyloxy)carbonyl](methyl)amino}methyl)-4,4,4-trifluorobutanoate (Racemate)

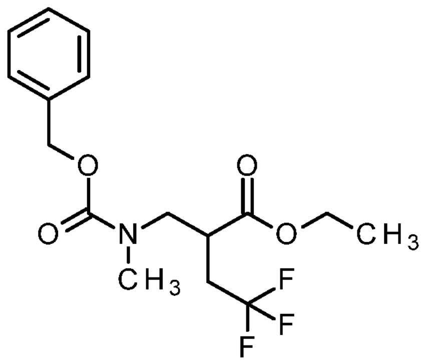

To a solution of ethyl-2-({[(benzyloxy)carbonyl]amino}methyl)-4,4,4-trifluorobutanoate (Racemate) (see Intermediate 9, 10.0 g, 30.0 mmol) in tetrahydrofuran (120 mL) was added sodium hydride (1.44 g, 60% purity, contained mineral oil) in portions at 0 °C. The mixture was stirred at 0 °C for 1 hour, iodomethane (2.8 mL, 45 mmol) was added drop-wise. After addition, the resulting mixture was stirred at 0 °C for 0.5 hour. Then the mixture was warmed to room temperature and stirred for 3 hours. Saturated ammonium chloride aqueous solution was added and the mixture was extracted with ethyl acetate. The organic phase was washed with brine, dried over anhydrous sodium sulfate, filtered and concentrated to give a residue. The residue was purified by silica gel chromatography (200-300mesh, petroleum ether: ethyl acetate = 20: 1 to 10: 1) to give 4.70 g ethyl-2-({[(benzyloxy)carbonyl](methyl)amino}methyl)-4,4,4-trifluorobutanoate (Racemate) as yellow oil.

$^1H$ NMR (400 MHz, $CDCl_3$): $\delta$ [ppm] = 7.29-7.28 (m, 5H), 5.06-5.03 (m, 2H), 4.09-4.02 (m, 2H), 3.70-3.43 (m, 2H), 2.95 (s, 3H), 2.75-2.53 (m, 2H), 2.25-2.09 (m, 1H), 1.25 (t, *J*= 7.2 Hz, 3H).

**Intermediate 11**

ethyl-4,4,4-trifluoro-2-[(methylamino)methyl]butanoate·hydrogen chloride salt (Racemate)

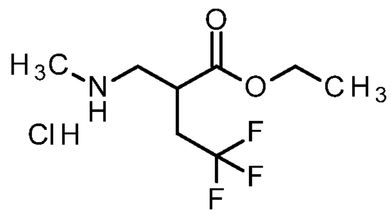

To a solution of ethyl-2-({[(benzyloxy)carbonyl](methyl)amino}methyl)-4,4,4-trifluorobutanoate (see Intermediate 10, 4.70 g, 13.5 mmol,) and hydrochloric acid (1.4 mL, 12 M in water, 16 mmol) in methanol (50 mL) was added palladium on carbon (500 mg, 10 % purity, contained 50% water, 470 μmol) at room temperature. The reaction mixture was stirred at room temperature for 16 hours under hydrogen atmosphere (15 psi). The mixture was filtered and the filtrate was concentrated by evaporation in vacuum to give ethyl-4,4,4-trifluoro-2-[(methylamino)methyl]butanoate·hydrogen chloride salt (Racemate) (3.20 g). The residue was used for next step directly without further purification.

**Intermediate 12**

ethyl-2-{[(3-ethoxy-3-oxopropanoyl)(methyl)amino]methyl}-4,4,4-trifluorobutanoate (Racemate)

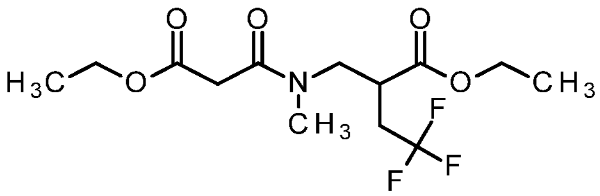

N,N-Diisopropylethylamine (6.7 mL, 38 mmol) and 4-dimethylaminopyridine (157 mg, 1.28 mmol) were added to a solution of ethyl-4,4,4-trifluoro-2-[(methylamino)methyl]-butanoate·hydrogen chloride salt (Racemate) (see Intermediate 11, 3.20 g, 12.8 mmol, in 50 mL dichloromethane) at 0°C and ethyl 3-chloro-3-oxopropanoate (1.5 mL, 12 mmol) was added to the mixture slowly below 15 °C. The mixture was stirred at room temperature for 16 hours. The mixture was adjusted to pH~3 by addtion hydrochloric acid solution (2 M in water). Then the mixture was extracted with dichloromethane and the combined organic layer was washed with brine, dried over anhydrous sodium sulfate, filtered and concentrated by evaporation in vacuum to give ethyl-2-{[(3-ethoxy-3-oxopropanoyl)(methyl)amino]methyl}-4,4,4-trifluorobutanoate (3.30 g). The residue was used for next step directly without further purification.

__Intermediate 13__

1-methyl-5-(2,2,2-trifluoroethyl)piperidine-2,4-dione (Racemate)

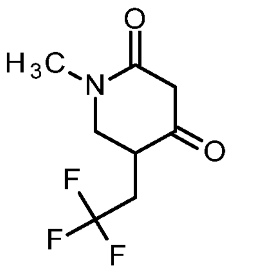

To a solution of ethyl-2-{[(3-ethoxy-3-oxopropanoyl)(methyl)amino]methyl}-4,4,4-trifluorobutanoate (Racemate) (see Intermediate 12, 24.0 g) in ethanol (200 mL) was added sodium ethoxide (5.99 g, 88.0 mmol, in ethanol) at 0-5 °C. Then the mixture was stirred at room temperature for 1 hour. The mixture was treated with aqueous hydrogen chloride solution (2 M), the aqueous phase was extracted with ethyl acetate. The combined organic phase was washed with brine, dried over anhydrous sodium sulfate, filtered and concentrated to give a residue. Then the mixture dissolved in acetonitrile (200 mL) and water (10 mL) and the resulting mixture was heated at reflux for 16 hours. The mixture was concentrated by evaporation in vacuum. The residue (combined with another batch starting from 15 g) was purified by silica gel chromatography (330 g, 100-200 mesh, petroleum ether: ethyl acetate = 20: 1 to 3: 1) to give 1-methyl-5-(2,2,2-trifluoroethyl)piperidine-2,4-dione (Racemate) 8.11 g as a yellow oil.

LC-MS (Method 6): Rt = 0.249 min; MS (ESIpos): m/z = 210.1 $[M+H]^+$.

$^1$H-NMR (400 MHz, $CDCl_3$): $\delta$ [ppm] = 3.68-3.66 (m, 1H), 3.44-3.33 (m, 3H), 3.10 (s, 3H), 2.97-2.94 (m, 2H), 2.14-2.07 (m, 1H).

__Intermediate 14__

5-methyl-3-phenyl-7-(2,2,2-trifluoroethyl)-1,5,6,7-tetrahydro-4H-pyrrolo[3,2-c]pyridin-4-one (Racemate)

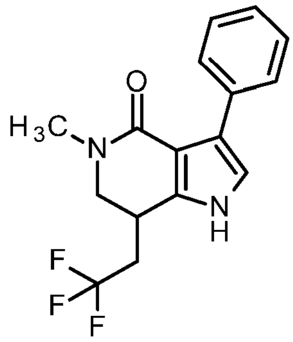

1-Methyl-5-(2,2,2-trifluoroethyl)piperidine-2,4-dione (Racemate) (see Intermediate 13, 1.00 g), 2-amino-1-phenylethan-1-one-hydrogen chloride salt (CAS-RN:[5468-37-1], 821 mg, 4.78 mmol) and potassium acetate (938 mg, 9.56 mmol) were suspended in water (8.6 mL) and stirred at room temperature for 10min. Then the mixture was stirred at 90°C over night. The reaction mixture (combined with another batch starting from 50 mg) was diluted with water. The unsolved precipitate was filtered off, washed with water and dryed in vacuum to provide the title compound as beige/brownish solid: m= 1.48 g

LC-MS (Method 2): Rt = 1.04 min; MS (ESIpos): m/z = 309 $[M+H]^+$

$^1$H-NMR (400MHz, DMSO-$d_6$): δ [ppm]= 2.58 - 2.82 (m, 2H), 2.93 (s, 3H), 3.36 - 3.47 (m, 2H), 3.72 (dd, 1H), 6.95 (d, 1H), 7.12 - 7.19 (m, 1H), 7.22 - 7.29 (m, 2H), 7.60 - 7.66 (m, 2H), 11.41 (br s, 1H).

## Intermediate 15

2-bromo-5-methyl-3-phenyl-7-(2,2,2-trifluoroethyl)-1,5,6,7-tetrahydro-4H-pyrrolo[3,2-c]pyridin-4-one (Racemate)

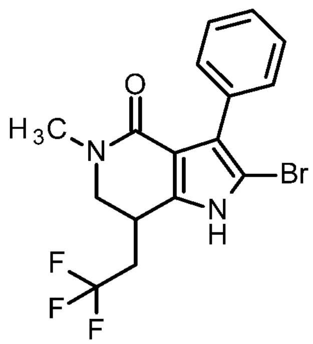

5-Methyl-3-phenyl-7-(2,2,2-trifluoroethyl)-1,5,6,7-tetrahydro-4H-pyrrolo[3,2-c]pyridin-4-one (Racemate) (see Intermediate 14, 1.48 g) was suspended in DMF (11 mL), treated with N-bromosuccinimide (CAS-RN:[128-08-5], 854 mg, 4.80 mmol) portion wise and stirred at room temperature for 15 min. The reaction mixture was concentrated under reduced pressure. The residue was treated with methanol (~10 mL) and it was sonicated for 5 minutes. The unsolved precipitate was filtered off, washed with methanol and dried at 50 °C under vacuum to provide the title compound: 1.451 g

LC-MS (Method 2): Rt = 1.10 min; MS (ESIpos): m/z = 387 $[M+H]^+$

$^1$H-NMR (400MHz, DMSO-$d_6$): δ [ppm]= 2.60 - 2.82 (m, 2H), 2.87 (s, 3H), 3.40 - 3.48 (m, 2H), 3.71 (dd, 1H), 7.04 - 7.56 (m, 5H), 12.13 (br s, 1H).

**Intermediate 16**

2-(2-aminopyridin-4-yl)-5-methyl-3-phenyl-7-(2,2,2-trifluoroethyl)-1,5,6,7-tetrahydro-4H-pyrrolo[3,2-c]pyridin-4-one (Racemate)

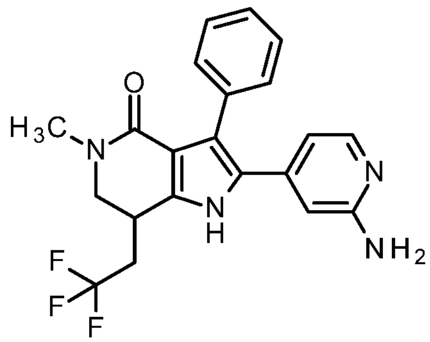

2-Bromo-5-methyl-3-phenyl-7-(2,2,2-trifluoroethyl)-1,5,6,7-tetrahydro-4H-pyrrolo[3,2-c]pyridin-4-one (Racemate) (see Intermediate 15, 1.45 g), 4-(4,4,5,5-tetramethyl-1,3,2-dioxaborolan-2-yl)pyridin-2-amine (CAS-RN:[1195995-72-2], 1.24 g, 5.62 mmol), cesium carbonate (3.66 g, 11.2 mmol) and XPhos Pd G3 (CAS-RN:[1445085-55-1], 317 mg, 374 µmol) were suspended in a degassed mixture of 1,4-dioxane (16 mL) and water (1.7 mL) and stirred at 80 °C overnight. The reaction mixture was diluted with a mixture of methylene chloride/isopropanole (7:3) and water. The layers were separated and the aqueous layer was extracted with methylene chloride/isopropanole three times. The combined organic layers were dried using a waterresistant filter and concentrated under reduced pressure. The crude product was purified by chromatography (25g column, silica ULTRA, gradient: ethyl acetate/ethanol 0-20% ethanol) to provide the title compound: 1.23 g.

LC-MS (Method 2): $R_t$ = 0.97 min; MS (ESIpos): m/z = 401.6 $[M+H]^+$

$^1$H-NMR (400MHz, DMSO-$d_6$): δ [ppm]= 2.57 - 2.78 (m, 1H), 2.87 (s, 3H), 2.88 - 2.99 (m, 1H), 3.39 - 3.50 (m, 2H), 3.67 - 3.78 (m, 1H), 5.81 (s, 2H), 6.07 (dd, 1H), 6.30 (d, 1H), 7.13 - 7.29 (m, 5H), 7.67 (d, 1H), 11.67 (s, 1H).

**Intermediate 17**

3-(4-fluorophenyl)-5-methyl-7-(2,2,2-trifluoroethyl)-1,5,6,7-tetrahydro-4H-pyrrolo[3,2-c]pyridin-4-one (Racemate)

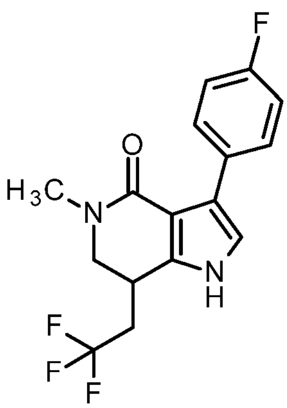

1-Methyl-5-(2,2,2-trifluoroethyl)piperidine-2,4-dione (Racemate) (see Intermediate 13, 1.00 g, 4.78 mmol), 2-amino-1-(4-fluorophenyl)ethan-1-one-hydrogen chloride salt (CAS-RN:[456-00-8], 907 mg, 4.78 mmol) and potassium acetate (938 mg, 9.56 mmol) were suspended in water (8.6 mL) and stirred at 90 °C over night under argon atmosphere. The suspension was diluted with some water. The solid in the mixture was filtered off under vacuum. The filter cake was

washed with water and dried in a vacuum drying oven to provide the title compound: 1.19 g LC-MS (Method 2): Rt = 1.08 min; MS (ESIpos): m/z = 327.5 $[M+H]^+$
$^1$H-NMR (400MHz, DMSO-$d_6$): δ [ppm]= 2.58 - 2.83 (m, 2H), 2.93 (s, 3H), 3.35 - 3.39 (m, 1H), 3.39 - 3.46 (m, 1H), 3.71 (dd, 1H), 6.96 (d, 1H), 7.02 - 7.13 (m, 2H), 7.62 - 7.75 (m, 2H), 11.43 (br s, 1H).

**Intermediate 18**

2-bromo-3-(4-fluorophenyl)-5-methyl-7-(2,2,2-trifluoroethyl)-1,5,6,7-tetrahydro-4H-pyrrolo[3,2-c]pyridin-4-one (Racemate)

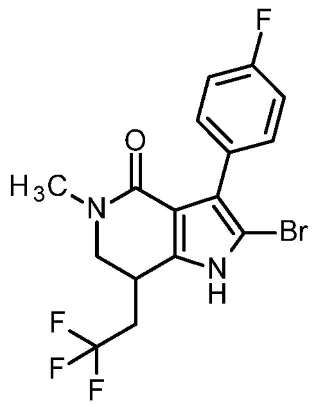

3-(4-Fluorophenyl)-5-methyl-7-(2,2,2-trifluoroethyl)-1,5,6,7-tetrahydro-4H-pyrrolo[3,2-c]pyridin-4-one (Racemate) (see Intermediate 17, 1.19 g) was suspended in DMF (8.4 mL), treated with N-bromosuccinimide (CAS-RN:[128-08-5], 647 mg, 3.63 mmol) portion wise and stirred at room temperature for 15 min. The reaction mixture was concentrated under reduced pressure. The residue was treated with methanol (~10 mL) and it was sonicated for 5 minutes. The unsolved precipitate was filtered off, washed with methanol and dried at 50 °C under vacuum to provide the title compound: 1.03 g

LC-MS (Method 2): Rt = 1.13 min; MS (ESIpos): m/z = 405 $[M+H]^+$

$^1$H-NMR (400MHz, DMSO-$d_6$): δ [ppm]= 2.60 - 2.84 (m, 2H), 2.87 (s, 3H), 3.34 - 3.47 (m, 2H), 3.70 (dd, 1H), 7.09 - 7.18 (m, 2H), 7.31 - 7.41 (m, 2H), 12.15 (s, 1H).

**Intermediate 19**

2-(2-aminopyridin-4-yl)-3-(4-fluorophenyl)-5-methyl-7-(2,2,2-trifluoroethyl)-1,5,6,7-tetrahydro-4H-pyrrolo[3,2-c]pyridin-4-one (Racemate)

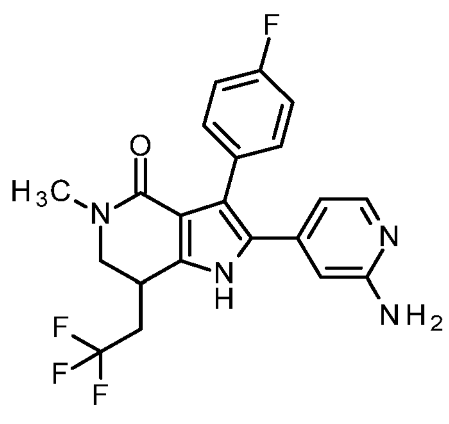

2-Bromo-3-(4-fluorophenyl)-5-methyl-7-(2,2,2-trifluoroethyl)-1,5,6,7-tetrahydro-4H-pyrrolo[3,2-c]pyridin-4-one (Racemate) (see Intermediate 18, 1.03 g), 4-(4,4,5,5-tetramethyl-1,3,2-dioxaborolan-2-yl)pyridin-2-amine (CAS-RN:[1195995-72-2], 839 mg, 3.81 mmol), caesium carbonate (2.48 g, 7.63 mmol) and XPhos Pd G3 (CAS-RN:[1445085-55-1], 215 mg, 254 μmol) were suspended in a degassed mixture of 1,4-dioxane (11 mL) and water (1.1 mL) and stirred at 80 °C overnight under argon atmosphere. The reaction mixture was diluted with a mixture of methylene chloride/isopropanole (7:3) and water. The aqueous layer was extracted three times with methylene chloride. The combined organic layers were filtered through a silicone coated filter and concentrated under reduced pressure. The crude product was purified by chromatography (25g column, silica ULTRA, gradient: ethyl acetate/ethanol 0-20% ethanol) to provide the title compound: 720 mg.

LC-MS (Method 2): Rt = 1.00 min; MS (ESIpos): m/z = 419.6 $[M+H]^+$

$^1$H-NMR (400MHz, DMSO-$d_6$): δ [ppm]= 2.60 - 2.77 (m, 1H), 2.81 - 3.00 (m, 4H), 3.38 - 3.48 (m, 2H), 3.73 (q, 1H), 5.83 (s, 2H), 6.13 (dd, 1H), 6.24 - 6.29 (m, 1H), 7.02 - 7.12 (m, 2H), 7.16 - 7.26 (m, 2H), 7.72 (d, 1H), 11.69 (s, 1H).

**Intermediate 20**

3-(4-fluorophenyl)-5,7-dimethyl-1,5,6,7-tetrahydro-4H-pyrrolo[3,2-c]pyridin-4-one (Racemate)

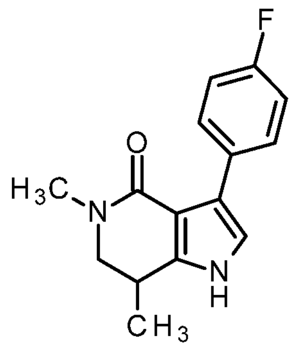

1,5-Dimethylpiperidine-2,4-dione (Racemate) (CAS-RN:[115497-23-9], 1.50 g, 10.6 mmol), 2-amino-1-(4-fluorophenyl)ethan-1-one-hydrogen chloride salt (CAS-RN:[456-00-8], 2.01 g, 10.6 mmol) and potassium acetate (2.09 g, 21.3 mmol) were suspended in water (19 mL) and stirred at 90 °C over night under argon atmosphere. The suspension was diluted with some water. The solid in the mixture was filtered off. The filter cake was washed with water and dried in a vacuum drying oven to provide the title compound: 2.13 g

LC-MS (Method 2): $R_t$ = 0.94 min; MS (ESIpos): m/z = 259 $[M+H]^+$

$^1$H-NMR (400MHz, DMSO-$d_6$): δ [ppm]= 1.24 (d, 3H), 2.90 (s, 3H), 3.11 (dt, 1H), 3.19 - 3.27 (m, 1H), 3.53 (dd, 1H), 6.89 (d, 1H), 7.02 - 7.13 (m, 2H), 7.62 - 7.73 (m, 2H), 11.33 (br s, 1H).

**Intermediate 21**

2-bromo-3-(4-fluorophenyl)-5,7-dimethyl-1,5,6,7-tetrahydro-4H-pyrrolo[3,2-c]pyridin-4-one (Racemate)

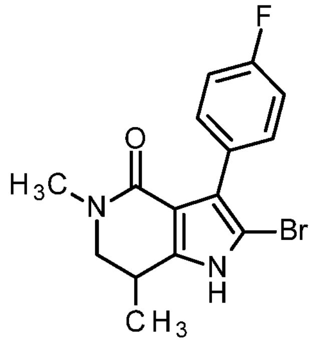

3-(4-Fluorophenyl)-5,7-dimethyl-1,5,6,7-tetrahydro-4H-pyrrolo[3,2-c]pyridin-4-one (Racemate) (see Intermediate 20, 2.13 g) was suspended in DMF (19 mL), treated with N-bromosuccinimide (CAS-RN:[128-08-5], 1.46 g, 8.23 mmol) portion wise and stirred at room temperature for 15 min. The reaction mixture was concentrated under reduced pressure. The residue was treated with methanol (~10 mL) and it was sonicated for 5 minutes. The unsolved precipitate was filtered off, washed with methanol and dried at 50 °C under vacuum to provide the title compound: 603 mg The methanol filtrate contains also the title compound and was concentrated under reduced pressure and treated with ethyl acetate and minimal methanol. It was sonicated for 15 minutes, the unsolved precipitate was filtered off, washed with ethyl acetate and dried at 50°C under vacuum to provide the title compound again: 665 mg

Both fractions of the title compound were combined and used for next step.

LC-MS (Method 2): Rt = 1.03 min; MS (ESIpos): m/z = 337 $[M+H]^+$

$^1$H-NMR (500MHz, DMSO-$d_6$): δ [ppm]= 1.24 (d, 3H), 2.84 (s, 3H), 3.04 - 3.17 (m, 1H), 3.20 - 3.28 (m, 1H), 3.53 (dd, 1H), 7.13 (t, 2H), 7.27 - 7.46 (m, 2H), 12.01 (s, 1H).

**Intermediate 22**

2-(2-aminopyridin-4-yl)-3-(4-fluorophenyl)-5,7-dimethyl-1,5,6,7-tetrahydro-4H-pyrrolo[3,2-c]pyridin-4-one (Racemate)

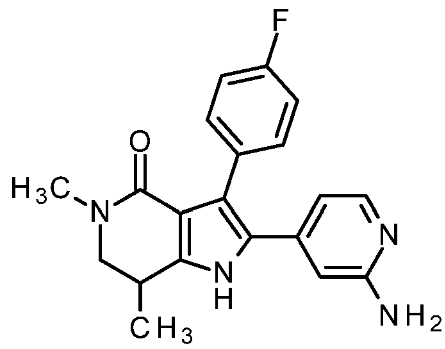

2-Bromo-3-(4-fluorophenyl)-5,7-dimethyl-1,5,6,7-tetrahydro-4H-pyrrolo[3,2-c]pyridin-4-one (Racemate) (see Intermediate 21, 1.26 g), 4-(4,4,5,5-tetramethyl-1,3,2-dioxaborolan-2-yl)pyridin-2-amine (CAS-RN:[1195995-72-2], 1.23 g, 5.61 mmol), caesium carbonate (3.65 g, 11.2 mmol) and XPhos Pd G3 (CAS-RN:[1445085-55-1], 316 mg, 374 μmol) were suspended in a degassed mixture of 1,4-dioxane (16 mL) and water (1.7 mL) and stirred at 80 °C overnight under argon atmosphere. The reaction mixture was diluted with a mixture of methylene chloride/isopropanole (7:3) and water. The aqueous layer was extracted three times with methylene chloride. The combined organic layers were filtered through a silicone coated filter and concentrated under reduced pressure. The crude product was purified by chromatography (55g column, aminophase, gradient: methylene chloride /ethanol 0-20% ethanol) to provide the title compound: 946 mg.

LC-MS (Method 2): Rt = 0.85 min; MS (ESIpos): m/z = 351 $[M+H]^+$

$^1$H-NMR (400MHz, DMSO-$d_6$): δ [ppm]= 1.30 (d, 3H), 2.84 (s, 3H), 3.09 - 3.28 (m, 2H), 3.59 (dd, 1H), 5.79 (s, 2H),

6.12 (dd, 1H), 6.25 - 6.32 (m, 1H), 7.01 - 7.12 (m, 2H), 7.17 - 7.25 (m, 2H), 7.69 (d, 1H), 11.55 (s, 1H).

**Intermediate 23**

5,7-dimethyl-3-phenyl-1,5,6,7-tetrahydro-4H-pyrrolo[3,2-c]pyridin-4-one (Racemate)

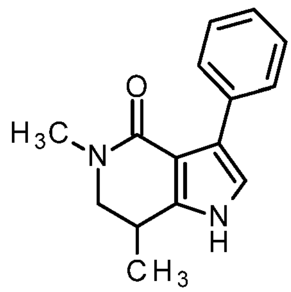

1,5-Dimethylpiperidine-2,4-dione (Racemate) (CAS-RN:[115497-23-9], 1.50 g, 10.6 mmol), 2-amino-1-phenylethan-1-one-hydrogen chloride salt (CAS-RN:[5468-37-1], 1.82 g, 10.6 mmol) and potassium acetate (2.09 g, 21.3 mmol) were suspended in water (19 mL) and stirred at 90 °C for 1 hour under argon atmosphere. The suspension was diluted with some water and stirred for 15 minutes. The unsolved precipitate was filtered off, washed with water and dried at 50°C under vacuum to provide the title compound: 2.19 g

LC-MS (Method 2): Rt = 0.90 min; MS (ESIpos): m/z = 241 $[M+H]^+$

$^1$H-NMR (400MHz, DMSO-$d_6$): δ [ppm]= 1.24 (d, 3H), 2.91 (s, 3H), 3.12 (dquin, 1H), 3.20 - 3.28 (m, 1H), 3.53 (dd, 1H), 6.88 (d, 1H), 7.10 - 7.18 (m, 1H), 7.21 - 7.30 (m, 2H), 7.60 - 7.68 (m, 2H), 11.31 (br s, 1H).

**Intermediate 24**

2-bromo-5,7-dimethyl-3-phenyl-1,5,6,7-tetrahydro-4H-pyrrolo[3,2-c]pyridin-4-one (Racemate)

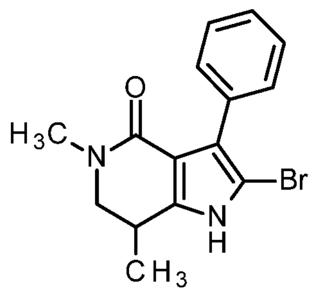

5,7-Dimethyl-3-phenyl-1,5,6,7-tetrahydro-4H-pyrrolo[3,2-c]pyridin-4-one (Racemate) (see Intermediate 23, 2.19 g, 9.13 mmol) was suspended in DMF (21 mL), treated with N-bromosuccinimide (CAS-RN:[128-08-5], 1.62 g, 9.13 mmol) portion wise and stirred at room temperature for 15 min. The reaction mixture was concentrated under reduced pressure. The residue was treated with methanol (~10 mL) and it was sonicated for 5 minutes. The unsolved precipitate was filtered off, washed with methanol and dried at 50 °C under vacuum to provide the title compound: 2.12 g

LC-MS (Method 2): Rt = 0.99 min; MS (ESIpos): m/z = 319 $[M+H]^+$

$^1$H-NMR (500MHz, DMSO-$d_6$): δ [ppm]= 1.25 (d, 3H), 2.85 (s, 3H), 3.06 - 3.16 (m, 1H), 3.23 (dd, 1H), 3.53 (dd, 1H), 7.18 - 7.47 (m, 5H), 11.98 (s, 1H).

**Intermediate** 25

2-(2-aminopyridin-4-yl)-5,7-dimethyl-3-phenyl-1,5,6,7-tetrahydro-4H-pyrrolo[3,2-c]pyridin-4-one (Racemate)

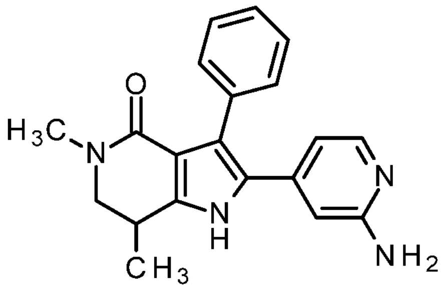

2-Bromo-5,7-dimethyl-3-phenyl-1,5,6,7-tetrahydro-4H-pyrrolo[3,2-c]pyridin-4-one (Racemate) (see Intermediate 24, 2.12 g), 4-(4,4,5,5-tetramethyl-1,3,2-dioxaborolan-2-yl)pyridin-2-amine (CAS-RN:[1195995-72-2], 2.19 g, 9.96 mmol), caesium carbonate (6.49 g, 19.9 mmol) and XPhos Pd G3 (CAS-RN:[1445085-55-1], 562 mg, 664 μmol) were suspended in a degassed mixture of 1,4-dioxane (28 mL) and water (3.0 mL) and stirred at 80 °C overnight under argon atmosphere. The reaction mixture was diluted with a mixture of methylene chloride/isopropanole (7:3) and water. The aqueous layer was extracted three times with methylene chloride. The combined organic layers were filtered through a silicone coated filter and concentrated under reduced pressure. The crude product was purified by chromatography (55 g column, aminophase, gradient: methylene chloride /ethanol 0-20% ethanol) and then was treated with methylene chloride and stirred for 10 minutes. The unsolved precipitage was filtered off, washed with methylene chloride and dried at 50°C under vacuum to provide the title compound: 1.36 g.

LC-MS (Method 2): Rt = 0.84 min; MS (ESIpos): m/z = 333 $[M+H]^+$

$^1H$-NMR (400MHz, DMSO-$d_6$): δ [ppm]= 1.31 (d, 3H), 2.84 (s, 3H), 3.10 - 3.27 (m, 2H), 3.59 (dd, 1H), 5.72 - 5.82 (m, 2H), 6.07 (dd, 1H), 6.26 - 6.37 (m, 1H), 7.15 - 7.28 (m, 5H), 7.65 (d, 1H), 11.52 (s, 1H).

**Intermediate 26**

Ethyl-3-(benzylamino)-2-methylbutanoate (mixture of 4 stereoisomers)

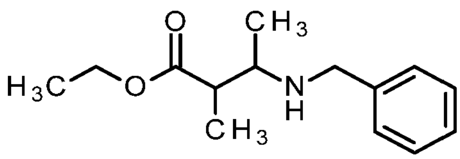

1-Phenylmethanamine (CAS-RN:[100-46-9], 3.8 mL, 35 mmol) and sodium cyanoborohydride (4.36 g, 69.4 mmol) were added to a solution of ethyl-2-methyl-3-oxobutanoate (Racemate) (CAS-RN:[609-14-3], 4.9 mL, 35 mmol) in 40 mL ethanol at room temperature and acetic acid (990 μL, 17 mmol) was carefully droped into the mixture. It was stirred at room temperature for 3 hours and then the mixture was stirred at 50 °C overnight. The reaction mixture was concentrated under reduced pressure, the residue was redissolved in ethyl acetate and washed with aqueous saturated sodium hydrogencarbonate solution. After seperation the organic layer was extracted with aqueous 1 M HCl-solution. The organic layer was discarded and the aqueous layer was basified using aqueous 1M sodium hydroxide solution. The aqueous phase was extracted with ethyl acetate twice. The combined organic layers were dried using a waterresistant filter and concentrated under reduced pressure to provide 4.73 g of the title compound. It was used without further purification for the next step.
LC-MS (Method 2): Rt = 1.25 min; MS (ESIpos): m/z = 236 $[M+H]^+$

**Intermediate 27**

ethyl-3-[benzyl(methyl)amino]-2-methylbutanoate (mixture of 4 stereoisomers)

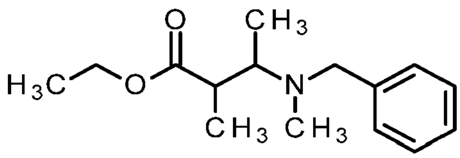

Ethyl-3-(benzylamino)-2-methylbutanoate (mixture of 4 stereoisomers) (see Intermediate 26, 2.83 g) was dissolved in DMF (23 mL). Iodomethane (370 μL, 5.9 mmol) and potassium carbonate (1.63 g, 11.8 mmol) were added and the mixture was stirred at room temperature under nitrogen atmosphere overnight. The reaction mixture was diluted with ethyl acetate and water. The aqueous layer was extracted with ethyl acetate twice. The combined organic layers were dried using a waterresistant filter and concentrated under reduced pressure to provide 5.11 g of the title compound.
LC-MS (Method 2): Rt = 1.50 min; MS (ESIpos): m/z = 250 $[M+H]^+$

**Intermediate 28**

ethyl-2-methyl-3-(methylamino)butanoate (mixture of 4 stereoisomers)

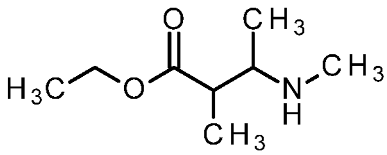

Ethyl-3-[benzyl(methyl)amino]-2-methylbutanoate (mixture of 4 stereoisomers) (see Intermediate 27, 5.11 g) was dissolved in ethanol (100 mL) and THF (13 mL) and treated with acetic acid (510 μL, 8.9 mmol ) and Palladium on activated carbon (589 mg, 10 % purity, 553 μmol). With hydrogen under pressure (20 bar) it was stirred at 60 °C for 3 days. The catalast was filtered off and the clear filtrate was concentrated under reduced pressure to provide 4.76 g of the title compound.
LC-MS (Method 2): Rt = 0.84 min; MS (ESIpos): m/z = 160 $[M+H]^+$

**Intermediate 29**

ethyl-3-[(3-ethoxy-3-oxopropanoyl)(methyl)amino]-2-methylbutanoate (mixture of 4 stereoisomers)

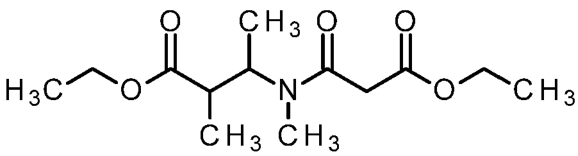

N,N-Diisopropylethylamine (3.5 mL, 20 mmol) and 4-dimethylaminopyridine (223 mg, 1.82 mmol) were added to a solution of ethyl-2-methyl-3-(methylamino)butanoate (mixture of 4 stereoisomers) (see Intermediate 28, 4.76 g) in 21 mL dichloromethane at 0°C and ethyl 3-chloro-3-oxopropanoate (CAS-RN:[36239-09-5], 2.5 mL, 20 mmol) was added to the mixture slowly below 8 °C. The mixture was stirred at 0 °C for 1.5 hours and afterwards it was stirred at room temperature for an additional hour. The reaction mixture was treated with half concentrated NH4Cl solution, the layers were separated and the aqueous phase was extracted with dichloromethane once. The combined organic layers were dried using a waterresistant filter and concentrated under reduced pressure. The crude product was purified by preparative HPLC chromatography (eluent: acetonitrile / water with ammonia as additive) to provide 1.91 g of the title compound.

LC-MS (Method 2): Rt = 0.96 min; MS (ESIpos): m/z = 274 [M+H]+

**Intermediate 30**

ethyl-1,5,6-trimethyl-2,4-dioxopiperidine-3-carboxylate (mixture of 4 stereoisomers)

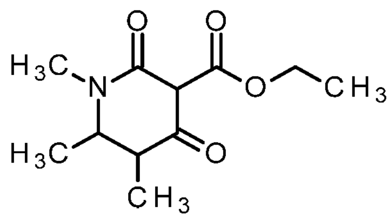

Ethyl-3-[(3-ethoxy-3-oxopropanoyl)(methyl)amino]-2-methylbutanoate (mixture of 4 stereoisomers) (see Intermediate 29, 471 mg) was dissolved in ethanol (3.1 mL) and the sodium ethoxide solution (CAS-RN:[141-52-6], 100 μL, 2.1 mmol) was added. The mixture was stirred for 72 hours at room temperature. Further sodium ethoxide solution (CAS-RN:[141-52-6], 100 μL, 2.1 mmol) was added and stirring was continued for 72 hours at room temperature. Further sodium ethoxide solution (CAS-RN:[141-52-6], 50 μL, 1.05 mmol) was added again and stirring was continued for 1 hour at room temperature. The reaction mixture (combined with another batch starting from 100 mg) was diluted with water and the pH value was adjusted to 1 by addition of aqueous HCl (2 mol/l). It was extracted with ethyl acetate twice. The combined organic layers were dried using a waterresistant filter and concentrated under reduced pressure to provide 423 mg of the title compound.
LC-MS (Method 1): Rt = 0.86 min; MS (ESIpos): m/z = 228 [M+H]+

**Intermediate 31**

1,5,6-trimethylpiperidine-2,4-dione (mixture of 4 stereoisomers)

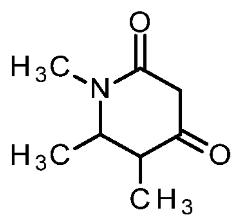

Ethyl-1,5,6-trimethyl-2,4-dioxopiperidine-3-carboxylate (mixture of 4 stereoisomers) (see Intermediate 30, 490 mg) was dissolved in acetonitrile (5.7 mL) and treated with water (290 μL). It was stirred at reflux overnight under inert atmosphere. The reaction mixture was concentrated under reduced pressure. The crude product (combined with another batch starting from 70 mg) was purified by preparative reverse phase HPLC (eluent: acetonitrile / water with ammonia as additive) to provide 65 mg of the title compound.

LC-MS (Method 2): $R_t$ = 0.47 min; MS (ESIpos): m/z = 156 [M+H]+

$^1$H-NMR (400 MHz, DMSO-$d_6$) δ [ppm]: 0.939 (10.71), 0.944 (10.91), 0.956 (10.41), 0.960 (10.83), 1.069 (1.22), 1.086 (2.74), 1.105 (2.29), 1.115 (1.27), 1.133 (1.20), 1.163 (2.22), 1.180 (2.17), 2.518 (0.51), 2.539 (0.95), 2.747 (0.51), 2.771 (2.89), 2.924 (3.34), 2.935 (16.00), 3.029 (0.86), 3.043 (1.06), 3.060 (0.86), 3.117 (1.18), 3.169 (2.88), 3.240 (2.94), 3.292 (1.18), 3.620 (1.27), 3.634 (1.30), 3.636 (1.30), 3.650 (1.18), 4.775 (0.79).

**Intermediate 32**

5,6,7-trimethyl-3-phenyl-1,5,6,7-tetrahydro-4H-pyrrolo[3,2-c]pyridin-4-one (mixture of 4 stereoisomers)

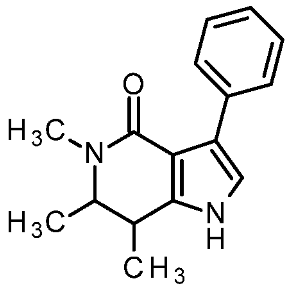

1,5,6-Trimethylpiperidine-2,4-dione (mixture of 4 stereoisomers) (see Intermediate 31, 83.0 mg), 2-amino-1-phenylethan-1-one-hydrogen chloride salt (CAS-RN:[5468-37-1], 91.8 mg, 535 μmol) and potassium acetate (105 mg, 1.07 mmol) were suspended in water (960 μL) and stirred at 90 °C overnight under argon atmosphere. The suspension was diluted with ethyl acetate and water. The layers were separated and the aqueous layer was extracted with ethyl acetate twice. The combined organic layers were dried using a waterresistant filter and concentrated under reduced pressure to provide 124.5 mg of the title compound.
LC-MS (Method 2): Rt = 0.96 min; MS (ESIpos): m/z = 255 $[M+H]^+$

**Intermediate 33**

2-bromo-5,6,7-trimethyl-3-phenyl-1,5,6,7-tetrahydro-4H-pyrrolo[3,2-c]pyridin-4-one (mixture of 4 stereoisomers)

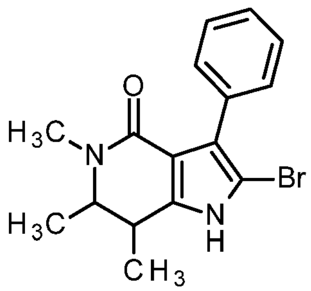

5,6,7-Trimethyl-3-phenyl-1,5,6,7-tetrahydro-4H-pyrrolo[3,2-c]pyridin-4-one (mixture of 4 stereoisomers) (see Intermediate 32, 125 mg) was suspended in DMF (760 μL), treated with N-bromosuccinimide (CAS-RN:[128-08-5], 58.4 mg, 328 μmol) portion wise and stirred at room temperature under nitrogen atmosphere for 15 min. The reaction mixture was concentrated under reduced pressure. The residue was purified by chromatography (5 g spheric silica column, gradient: dichloromethane /ethanol 1-10% ethanol) to provide 78.6 mg of the title compound.

LC-MS (Method 2): Rt = 1.05 min; MS (ESIneg): m/z = 331 $[M-H]^-$

$^1$H-NMR (500 MHz, DMSO-$d_6$) δ [ppm]: 1.017 (2.60), 1.030 (2.64), 1.161 (3.16), 1.174 (3.16), 1.199 (3.11), 1.213 (3.17), 1.225 (2.60), 1.240 (2.68), 2.213 (0.44), 2.514 (2.71), 2.518 (2.42), 2.522 (1.94), 2.562 (16.00), 2.639 (0.74), 2.729 (4.74), 2.746 (0.56), 2.758 (0.60), 2.835 (7.19), 2.852 (8.35), 2.888 (5.76), 3.370 (0.44), 3.382 (0.50), 3.396 (0.44), 3.425 (0.68), 3.428 (0.67), 3.435 (0.63), 3.567 (0.43), 3.579 (0.58), 5.758 (3.52), 7.209 (0.45), 7.212 (0.46), 7.225 (1.20), 7.238 (1.01), 7.241 (1.04), 7.281 (1.32), 7.295 (2.63), 7.297 (2.60), 7.311 (1.64), 7.335 (1.86), 7.346 (2.33), 7.349 (1.72), 7.352 (0.84), 7.360 (1.32), 7.363 (0.97), 7.951 (0.70), 11.902 (0.48), 11.964 (0.54).

**Intermediate 34**

2-(2-aminopyridin-4-yl)-5,6,7-trimethyl-3-phenyl-1,5,6,7-tetrahydro-4H-pyrrolo[3,2-c]pyridin-4-one (mixture of 4 stereoisomers)

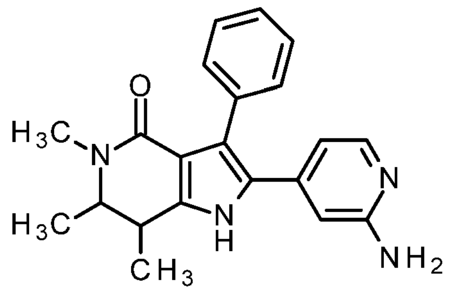

2-Bromo-5,6,7-trimethyl-3-phenyl-1,5,6,7-tetrahydro-4H-pyrrolo[3,2-c]pyridin-4-one (mixture of 4 stereoisomers) (see Intermediate 33, 75.0 mg), 4-(4,4,5,5-tetramethyl-1,3,2-dioxaborolan-2-yl)pyridin-2-amine (CAS-RN:[1195995-72-2], 74.3 mg, 338 μmol), caesium carbonate (220 mg, 675 μmol) and XPhos Pd G3 (CAS-RN:[1445085-55-1], 19.1 mg, 22.5 μmol) were suspended in a degassed mixture of 1,4-dioxane (960 μL) and water (100 μL) and stirred at 80 °C overnight under argon atmosphere. The reaction mixture was diluted with methylene chloride and water. The aqueous layer was extracted with methylene chloride twice. The combined organic layers were dried using a waterresistant filter and concentrated under reduced pressure. The crude product was purified by chromatography (11 g column, aminophase, gradient: methylene chloride /ethanol 1-10% ethanol) to provide 48.1 mg of the title compound.

LC-MS (Method 2): $R_t$ = 0.86 min; MS (ESIneg): m/z = 345 $[M-H]^-$

$^1$H-NMR (400 MHz, DMSO-$d_6$) δ [ppm]: 0.815 (0.45), 0.822 (0.46), 0.904 (0.52), 1.035 (5.48), 1.052 (6.63), 1.066 (0.56), 1.070 (1.38), 1.160 (6.20), 1.176 (6.26), 1.232 (0.73), 1.253 (6.05), 1.270 (6.22), 1.286 (4.41), 1.303 (4.32), 2.066 (0.61), 2.277 (0.87), 2.342 (0.86), 2.522 (4.63), 2.802 (0.41), 2.819 (1.47), 2.832 (13.24), 2.848 (16.00), 3.404 (0.89), 3.422 (2.86), 3.440 (2.20), 3.454 (1.65), 3.470 (0.59), 3.591 (0.81), 3.607 (1.06), 3.622 (0.73), 4.358 (0.68), 5.746 (5.53), 6.062 (3.12), 6.066 (2.96), 6.072 (2.19), 6.076 (2.44), 6.079 (2.72), 6.085 (1.86), 6.284 (3.30), 6.329 (2.57), 6.635 (1.47), 6.675 (0.94), 6.680 (0.85), 6.689 (0.94), 6.693 (0.85), 7.180 (1.16), 7.198 (6.39), 7.216 (9.68), 7.222 (5.78), 7.228 (7.56), 7.240 (2.70), 7.247 (1.90), 7.252 (2.22), 7.260 (0.96), 7.636 (3.16), 7.650 (3.23), 7.966 (1.22), 7.980 (1.20), 11.375 (1.54), 11.599 (2.00).

**Intermediate 35**

7,7-dimethyl-3-phenyl-1,5,6,7-tetrahydro-4H-pyrrolo[3,2-c]pyridin-4-one

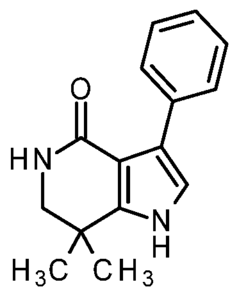

5,5-Dimethylpiperidine-2,4-dione (CAS-RN:[118263-81-3], 1.00 g, 6.73 mmol), 2-amino-1-phenylethan-1-one-hydrogen chloride salt (CAS-RN:[5468-37-1], 1.15 g, 6.73 mmol) and potassium acetate (1.32 g, 13.5 mmol) were

suspended in water (12mL) and stirred at 90 °C overnight under nitrogen atmosphere. The suspension was diluted with some water and the solid in the mixture was filtered off. The filter cake was washed with water twice and dried in vacuum at 50°C to provide 1.54 g of the title compound.

LC-MS (Method 2): $R_t$ = 0.85 min; MS (ESIneg): m/z = 239 $[M-H]^-$

$^1$H-NMR (500MHz, DMSO-$d_6$): δ [ppm]= 1.25 (s, 6H), 3.12 (d, 2H), 6.90 (d, 1H), 7.00 (br s, 1H), 7.11 - 7.18 (m, 1H), 7.21 - 7.28 (m, 2H), 7.67 (dd, 2H), 11.30 (br s, 1H).

**Intermediate 36**

2-bromo-7,7-dimethyl-3-phenyl-1,5,6,7-tetrahydro-4H-pyrrolo[3,2-c]pyridin-4-one

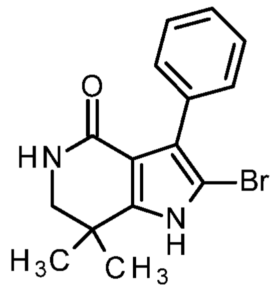

7,7-Dimethyl-3-phenyl-1,5,6,7-tetrahydro-4H-pyrrolo[3,2-c]pyridin-4-one (see Intermediate 35, 1.54 g) was suspended in DMF (15 mL), treated with N-bromosuccinimide (CAS-RN:[128-08-5], 1.14 g, 6.39 mmol) portion wise and stirred at room temperature under nitrogen atmosphere for 15 min. The reaction mixture was concentrated under reduced pressure. The residue was diluted with methanol. The unsolved precipitate was filtered off and the filter cake was washed with methanol three times. The filter cake was dried under vacuum at 50 °C to provide 666 mg of the title compound.

LC-MS (Method 2): $R_t$ = 0.93 min; MS (ESIneg): m/z = 317 $[M-H]^-$

$^1$H-NMR (400MHz, DMSO-$d_6$): δ [ppm]= 1.26 (s, 6H), 3.12 (d, 2H), 7.04 (br s, 1H), 7.16 - 7.25 (m, 1H), 7.26 - 7.33 (m, 2H), 7.33 - 7.41 (m, 2H), 11.93 (s, 1H).

**Intermediate 37**

2-(2-aminopyridin-4-yl)-7,7-dimethyl-3-phenyl-1,5,6,7-tetrahydro-4H-pyrrolo[3,2-c]pyridin-4-one

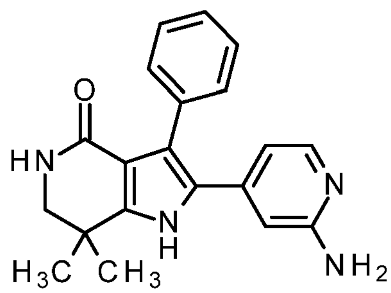

2-Bromo-7,7-dimethyl-3-phenyl-1,5,6,7-tetrahydro-4H-pyrrolo[3,2-c]pyridin-4-one (see Intermediate 36, 834 mg), 4-(4,4,5,5-tetramethyl-1,3,2-dioxaborolan-2-yl)pyridin-2-amine (CAS-RN:[1195995-72-2], 863 mg, 3.92 mmol), caesium carbonate (2.55 g, 7.84 mmol) and XPhos Pd G3 (CAS-RN:[1445085-55-1], 221 mg, 261 μmol) were suspended in a degassed mixture of 1,4-dioxane (11 mL) and water (1.2 mL) and stirred at 80 °C overnight under nitrogen atmosphere.

The reaction mixture was diluted with a mixture of methylene chloride/isopropanole (7:3) and water. The unsolved precipitate was filtered off and washed with methylene chloride/isopropanole (7:3). The filter cake was diluted again with methylene chloride/isopropanole (7:3) and stirred overnight. The residue was filterd off and dried under vacuum at 50°C to provide 737 mg of the title compound.

LC-MS (Method 2): Rt = 0.78 min; MS (ESIneg): m/z = 331 $[M-H]^-$

$^1$H-NMR (400MHz, DMSO-$d_6$): δ [ppm]= 1.32 (s, 6H), 3.13 (d, 2H), 5.79 (s, 2H), 6.05 (dd, 1H), 6.36 (s, 1H), 6.96 (br s, 1H), 7.12 - 7.30 (m, 5H), 7.64 (d, 1H), 11.27 - 11.65 (m, 1H).

**Intermediate 38**

ethyl [1-(methylamino)cyclobutyl]acetate

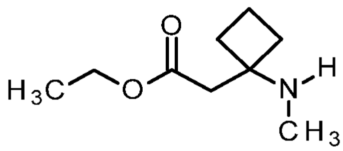

Methane amine (3.4 mL, 33 % purity in ethanol, 27 mmol) was cooled down with an ice bath and treated dropwise with ethyl cyclobutylideneacetate (CAS-RN:[27741-65-7], 3.80 g, 27.1 mmol), dissolved in 4.4 mL ethanol. The reaction mixture was stirred at rt over night under Argon atmosphere. The reaction mixture was concentrated under reduced pressure. The crude product was used without further purification: 4.3 g.

**Intermediate 39**

ethyl 3-{[1-(2-ethoxy-2-oxoethyl)cyclobutyl](methyl)amino}-3-oxopropanoate

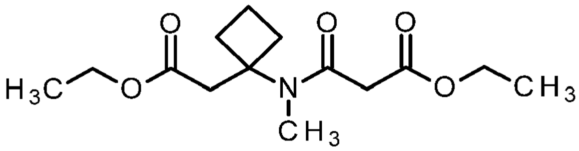

Ethyl [1-(methylamino)cyclobutyl]acetate (see Intermediate 38, 4.31 g), was dissolved in 26 mL dichloromethane and cooled down to 0°C. Then N,N-diisopropylethylamine (4.8 mL, 28 mmol) and DMAP (307 mg, 2.52 mmol) were added. Subsequentely ethyl 3-chloro-3-oxopropanoate (3.5 mL, 28 mmol) was added. The reaction mixture was stirred at 0°C for 1.5 h at rt over night. Half concentrated ammonium chloride soultion and dichloromethane were added to the reaction mixture. It was stirred for a few minutes, filtered through a silicone coated filter and concentrated under reduced pressure. The crude product was combined with the crude product of another batch starting from ethyl [1-(methylamino)cyclobutyl]acetate (see BRAL818-2, 5.0 g) and purified by flash chromatography (Isolera, Biotage, 50g column silica ULTRA; hexane / ethyl acetate 12%-100%, collection mode: collect all fractions) to provide the title compound: 3.7 g, which was used without further purification.
LC-MS (Method 2): Rt = 0.96 min; MS (ESIpos): m/z = 286 [M+H]+

**Intermediate 40**

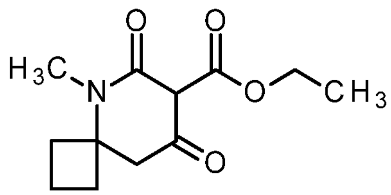

Sodium ethoxide (1.5 mL, 21 % in ethanol, 6.6 mmol) was cooled down with an ice bath and treated dropwise with ethyl 3-{[1-(2-ethoxy-2-oxoethyl)cyclobutyl](methyl)amino}-3-oxopropanoate (see Intermediate 39, 3.70 g), dissolved in 8.7 mL ethanol. The reaction mixture was stirred at 0°C for 30 minutes, then further 20 mL ethanol were added and the reaction mixture was stirred at rt over night under Argon atmosphere. The reaction mixture was diluted with dichloromethane and the pH was adjusted to 1 by addition of hydrochloric acid (4 M). It was stirred for a few minutes, filtered through a silicone coated filter and concentrated under reduced pressure to provide the title compound, which was used without further purification: 3.0 g.
LC-MS (Method 1): Rt = 0.93 min; MS (ESIpos): m/z = 241 [M+H]+

**Intermediate 41**

5-methyl-5-azaspiro[3.5]nonane-6,8-dione

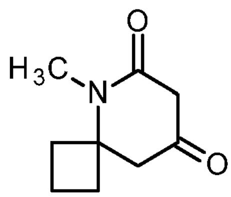

5-Methyl-6,8-dioxo-5-azaspiro[3.5]nonane-7-carboxylate (Racemate) (see Intermediate 40, 1.43 g) was dissolved in 12 mL dichloromethane and treated with hydrochloric acid (11 mL, 2.0 M, 23 mmol). It was stirred 20 min at rt under Argon atmosphere. The reaction mixture was filtered through a silicone coated filter and concentrated under reduced pressure. The crude intermediate was dissolved in 16 mL acetonitrile and treated with 1.6 mL water. It was stirred under reflux for 1 hour under Argon atmosphere. The reaction mixture was concentrated under reduced pressure. The crude product was purified by flash chromatography (50 g column, silica ULTRA; ethyl acetate / ethanol 0%-20% ethanol) to provide the analytically pure title compound: 1.4 g.

LC-MS (Method 1): Rt = 0.62 min; MS (ESIpos): m/z = 168 [M+H]+

$^1$H-NMR (400 MHz, DMSO-$d_6$) δ [ppm]: 1.680 (0.52), 1.688 (0.64), 1.705 (1.27), 1.714 (0.89), 1.716 (0.82), 1.723 (1.46), 1.730 (1.41), 1.732 (1.64), 1.741 (1.01), 1.744 (0.91), 1.748 (1.65), 1.753 (0.70), 1.757 (0.76), 1.768 (0.95), 1.792 (0.47), 1.796 (0.41), 1.819 (0.67), 1.831 (0.60), 1.837 (0.56), 1.844 (0.62), 1.851 (0.54), 1.870 (0.49), 1.876 (0.93), 1.883 (1.19), 1.891 (1.16), 1.900 (1.21), 1.902 (1.34), 1.907 (1.37), 1.909 (1.30), 1.917 (1.07), 1.927 (1.04), 1.931 (0.52), 1.934 (0.66), 2.370 (0.73), 2.376 (0.64), 2.394 (0.63), 2.401 (0.70), 2.518 (0.48), 2.527 (1.78), 2.534 (1.49), 2.552 (1.58), 2.558 (1.70), 2.582 (4.03), 2.856 (12.03), 3.001 (16.00), 3.334 (10.32), 4.884 (2.27), 10.439 (1.29).

**Intermediate 42**

5'-methyl-3'-phenyl-1',7'-dihydrospiro[cyclobutane-1,6'-pyrrolo[3,2-c]pyridin]-4'(5'H)-one

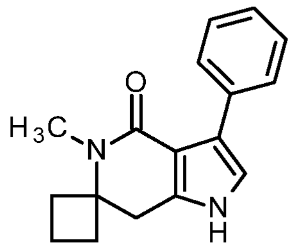

5-Methyl-5-azaspiro[3.5]nonane-6,8-dione (see Intermediate 41, 675 mg), 2-amino-1-phenylethan-1-one-hydrogen chloride salt (CAS-RN:[5468-37-1], 693 mg, 4.04 mmol) and potassium acetate (792 mg, 8.07 mmol) were suspended in water (7.3 mL) and stirred at 90 °C for one hour under argon atmosphere. The reaction mixture was diluted with water and stirred for 15 minutes.The unsolved precipitate was filtered off, washed with water and dried at 50 °C under vacuum. The crude product was purified by flash chromatography (25 g column, silica ULTRA, gradient: dichloromethane / ethanol 0-10% ethanol) to provide 275 mg of the title compound.

LC-MS (Method 2): Rt = 1.02 min; MS (ESIpos): m/z = 267.5 $[M+H]^+$

$^1$H-NMR (400MHz, DMSO-$d_6$): δ [ppm]= 1.70 - 1.84 (m, 2H), 1.85 - 1.97 (m, 2H), 2.39 - 2.48 (m, 2H), 3.00 (s, 3H), 3.05 (s, 2H), 6.88 (d, 1H), 7.10 - 7.18 (m, 1H), 7.21 - 7.29 (m, 2H), 7.60 - 7.70 (m, 2H), 11.31 (br s, 1H)

## Intermediate 43

2'-bromo-5'-methyl-3'-phenyl-1',7'-dihydrospiro[cyclobutane-1,6'-pyrrolo[3,2-c]pyridin]-4'(5'H)-one

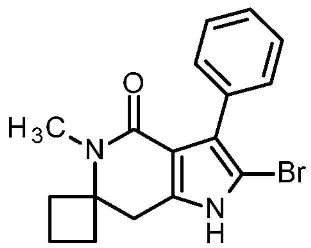

5'-Methyl-3'-phenyl-1',7'-dihydrospiro[cyclobutane-1,6'-pyrrolo[3,2-c]pyridin]-4'(5'H)-one (see Intermediate 42, 490 mg) was suspended in DMF (4.2 mL), treated with N-bromosuccinimide (CAS-RN:[128-08-5], 327 mg, 1.84 mmol) portion wise and stirred at room temperature under nitrogen atmosphere for 15 min. The reaction mixture was concentrated under reduced pressure. The residue was diluted with methanol (~5 mL) and it was sonicated for 5 minutes. The unsolved precipitate was filtered off, washed with methanol and dried under vacuum at 50 °C to provide 290 mg of the title compound.
LC-MS (Method 2): Rt = 1.10 min; MS (ESIpos): m/z = 345 $[M+H]^+$

## Intermediate 44

2'-(2-aminopyridin-4-yl)-5'-methyl-3'-phenyl-1',7'-dihydrospiro[cyclobutane-1,6'-pyrrolo[3,2-c]pyridin]-4'(5'H)-one

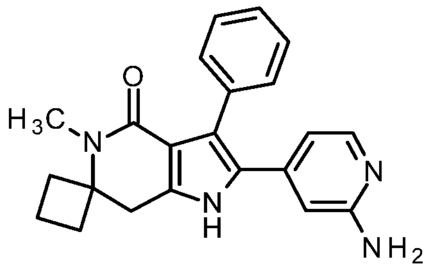

2'-Bromo-5'-methyl-3'-phenyl-1',7'-dihydrospiro[cyclobutane-1,6'-pyrrolo[3,2-c]pyridin]-4'(5'H)-one (see Intermediate 43, 290 mg), 4-(4,4,5,5-tetramethyl-1,3,2-dioxaborolan-2-yl)pyridin-2-amine (CAS-RN:[1195995-72-2], 277 mg, 1.26 mmol), caesium carbonate (821 mg, 2.52 mmol) and XPhos Pd G3 (CAS-RN:[1445085-55-1], 71.1 mg, 84.0 μmol) were suspended in a degassed mixture of 1,4-dioxane (3.6 mL) and water (380 μL) and stirred at 80 °C overnight under argon atmosphere. The reaction mixture was diluted with a mixture of methylene chloride/isopropanole (7:3) and water. The phases were separated and the aqueous layer was extracted three times with methylene chloride. The combined organic layers were dried using a silicone coated filter and concentrated under reduced pressure. The crude product was suspended in methylene chloride and diluted with MTBE. The unsolved precipitate was filtered off, washed with MTBE and dried at 50°C under vacuum to provide 164 mg of the title compound.

LC-MS (Method 2): Rt = 0.93 min; MS (ESIpos): m/z = 359 $[M+H]^+$

$^1$H-NMR (400MHz, DMSO-$d_6$): δ [ppm]= 1.71 - 1.85 (m, 2H), 1.90 - 2.02 (m, 2H), 2.41 - 2.49 (m, 2H), 2.94 (s, 3H), 3.11 (s, 2H), 5.75 (s, 2H), 6.09 (dd, 1H), 6.28 (s, 1H), 7.16 - 7.30 (m, 5H), 7.65 (d, 1H), 11.68 (s, 1H).

**Intermediate 45**

ethyl-2-cyano-3-cyclopropylpropanoate (Racemate)

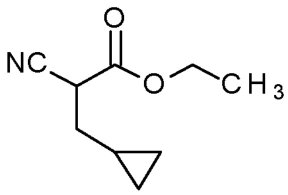

To a solution of ethyl cyanoacetate (CAS-RN:[105-56-6], 100.0 g, 884 mmol) and (bromomethyl)cyclopropane (CAS-RN:[7051-34-5], 131.0 g, 972 mmol) in acetonitrile (2000 mL) was added potassium carbonate (367 g, 2.65 mol) and sodium iodate (17.5 g, 88.4 mmol). Then the mixture was stirred at 60 °C for 14 hours. Aqueous hydrochloric acid solution (1 M) was added until pH was adjust to 3, and the mixture was extracted with ethyl acetate. The combined organic phase was dried over anhydrous sodium sulfate, filtered and concentrated to give a residue. The residue was purified by silica gel chromatography (200-300 mesh, petroleum ether: ethyl acetate = 100: 1 to 50: 1) to give 100 g ethyl-2-cyano-3-cyclopropylpropanoate (Racemate) as yellow oil.

$^1$H NMR (400 MHz, $CDCl_3$): δ [ppm] = 4.26 (q, 2H), 3.57 (t, 1H), 1.94-1.88 (m, 1H), 1.85-1.82 (m, 1H), 1.32 (t, 3H), 0.93-0.84 (m, 1H), 0.60-0.54 (m, 2H), 0.25-0.16 (m, 2H).

**Intermediate 46**

ethyl-2-(aminomethyl)-3-cyclopropylpropanoate-hydrogen chloride salt (Racemate)

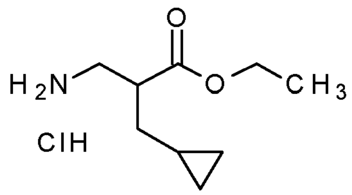

This reaction was performed parallel for 4 batched: A mixture of ethyl-2-cyano-3-cyclopropylpropanoate (Racemate)(see Intermediate 45, 17.5 g) and Platinum(IV) oxide (1.75 g, 7.7 mmol) and hydrochloric acid (12 M, 8 mL) in ethanol (530 mL) was stirred at room temperature for 16 hours under hydrogen (15 psi). The 4 batches of mixture were combined together, filtered and the filtrate was concentrated by evaporation in vacuum. The residue (65.0 g) was used to next step directely.

**Intermediate 47**

ethyl-2-({[(benzyloxy)carbonyl]amino}methyl)-3-cyclopropylpropanoate (Racemate)

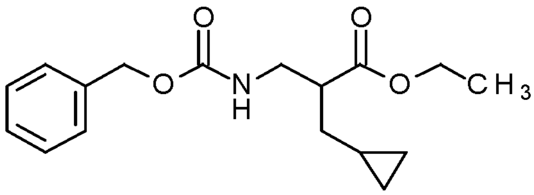

A mixture of ethyl-2-(aminomethyl)-3-cyclopropylpropanoate-hydrogen chloride salt (Racemate) (see Intermediate 46, 65.0 g), benzyl carbonochloridate (13153.4 g, 313 mmol) and sodium hydrogen carbonate (105.0 g, 1.25 mol) in water (650 mL) and dichloromethane (650 mL) was stirred at room temperature for 2 hours. The organic phase was dried over anhydrous sodium sulfate, filtered and concentrated to give a residue (combined with another batch starting from 35 g). 10 g of crude product was used next directely. The remained residue (90 g) was purified by preparative HPLC (Column: Phenomenex luna C18 (250*70mm,10 um); Mobile phase: water(0.225% FA)-CAN; Gradient B%: 45%-73%,140mL/min; 97%, 21 min; Instrument: ACSWH-PREP-HPLC-I) and then extrated with ethyl acetate and concentrated to give 44.0 g ethyl-3-{[(benzyloxy)carbonyl]amino}-2-(cyclopropylmethyl)propanoate (Racemate) as yellow oil.

LC-MS (Method 3): Rt = 0.956 min; MS (ESIpos): m/z = 306.2 $[M+H]^+$.

$^1H$ NMR (400 MHz, $CDCl_3$): δ [ppm] = 7.32-7.25 (m, 5H), 5.27 (s, 1H), 5.06 (s, 2H), 4.12 (q, 2H), 3.46-3.44 (m, 1H), 3.39-3.33 (m, 1H), 2.72-2.68 (m, 1H), 1.46 (t, 2H), 1.22 (t, 3H), 0.71-0.65 (m, 1H), 0.44-0.38 (m, 2H), 0.04-0.01 (m, 2H).

**Intermediate 48**

ethyl-2-({[(benzyloxy)carbonyl](methyl)amino}methyl)-3-cyclopropylpropanoate (Racemate)

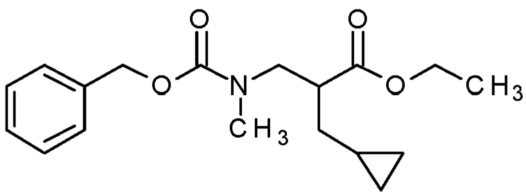

To a solution of ethyl-2-({[(benzyloxy)carbonyl]amino}methyl)-3-cyclopropylpropanoate (see Intermediate 47, 44.0 g) in tetrahydrofuran (500 mL) was drop-wise added sodium hydride (6.92 g, 60% purity, contained mineral oil, 173 mmol) at 0 °C. The mixture was stirred at 0 °C for 1 hour, iodomethane (30.7 g, 216 mmol) was drop-wise added and

the resulting mixture was stirred at 0 °C for 0.5 hour. Then the mixture was stirred at room temperature for 3 hours. The mixture was quenched by pouring into cold ammonium chloride solution, extracted with ethyl acetate. The organic phase was washed with brine, dried over anhydrous sodium sulfate, filtered and concentrated to give a residue. The residue was purified by silica gel chromatography (200-300 mesh, petroleum ether: ethyl acetate = 20: 1 to 10: 1) to give 46.0 g ethyl-3-{[(benzyloxy)carbonyl](methyl)amino}-2-(cyclopropylmethyl)propanoate as a yellow oil.

LC-MS (Method 3): Rt = 0.813 min; MS (ESIpos): m/z = 320 $[M+H]^+$.

$^1H$ NMR (400 MHz, $CDCl_3$): δ [ppm] = 7.37-7.30 (m, 5H), 5.14-5.09 (m, 2H), 4.16-4.10 (m, 2H), 3.59-3.54 (m, 1H), 3.43-3.37 (m, 1H), 2.93 (s, 3H), 1.54-1.48 (m, 1H), 1.27 (t, 3H), 0.78-0.55 (m, 1H), 0.45-0.40 (m, 2H), 0.05-0.01 (m, 2H).

**Intermediate 49**

ethyl-2-(cyclopropylmethyl)-3-(methylamino)propanoate-hydrogen chloride salt (Racemate)

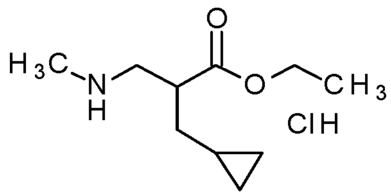

To a solution of ethyl-2-({[(benzyloxy)carbonyl](methyl)amino}methyl)-3-cyclopropylpropanoate (Racemate) (see Intermediate 48, 46.0 g) and hydrochloric acid (12 M) (14 mL, 172 mmol) in ethanol (380 mL) was added palladium on carbon (2.0 g, 10% putiry, contained 50% water) and palladium hydroxide on carbon (2.0 g, contained 50% water) at room temperature (20 °C). The reaction mixture was stirred at room temperature (20 °C) for 20 hours under hydrogen atmosphere (15 psi). The mixture was filtered and the filtrate was concentrated by evaporation in vacuum. The residue (31.0 g) was used to next step directely.

**Intermediate 50**

ethyl-2-(cyclopropylmethyl)-3-[(3-ethoxy-3-oxopropanoyl)(methyl)amino]propanoate (Racemate)

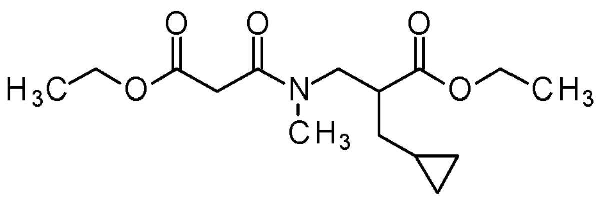

N,N-Diisopropylethylamine (73 mL, 420 mmol) and 4-Dimethylaminopyridine (1.71 g, 14.0 mmol) were added at 0 °C to a solution of ethyl-2-(cyclopropylmethyl)-3-(methylamino)propanoate-hydrogen chloride salt (see Intermediate 49, 31.0 g) in dichloromethane. And ethyl-3-chloro-3-oxopropanoate (CAS-RN:[37517-81-0], 18.9 g, 126 mmol) was added to the mixture slowly at below 15°C. The mixture was stirred at room temperature for 16 hours. The mixture was adjusted to pH~3 with aqueous hydrochloric acid solution (2 M). Then the mixture was extracted with dichloromethane and the combined organic layer was washed with brine, dried over anhydrous sodium sulfate, filtered and concentrated by evaporation in vacuum. The residue (18.0 g) was used for next step directly and no purification.

LC-MS (Method 3): Rt = 0.880 min; MS (ESIpos): m/z = 300.2 $[M+H]^+$.

**Intermediate 51**

5-(cyclopropylmethyl)-1-methylpiperidine-2,4-dione (Racemate)

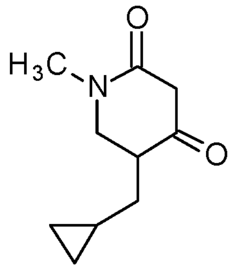

To a solution of ethyl-2-(cyclopropylmethyl)-3-[(3-ethoxy-3-oxopropanoyl)(methyl)-amino]propanoate (Racemate) (see Intermediate 50, 60.0 g) in ethanol (600 mL) was added sodium ethoxide (16.4 g, 241 mmol) at 0-5 °C. Then the mixture was stirred at room temperature for 4 hours. The mixture was treated with aqueous hydrogen chloride solution (2 M), the aqueous phase was extracted with ethyl acetate. The combined organic phase was washed with brine, dried over anhydrous sodium sulfate, filtered and concentrated to give a residue. Then the residue dissolved in acetonitrile (600 mL) and water (30 mL), and the resulting mixture was heated at reflux for 16 hours. The residue was purified by column chromatography (300-400 mesh, petroleum ether: ethyl acetate = 30: 1 then 1: 1) to give 25 g 5-(cyclopropylmethyl)-1-methylpiperidine-2,4-dione (Racemate) in 95 % purity as yellow oil.

LC-MS (Method 3): Rt = 0.489 min; MS (ESIpos): m/z = 182 $[M+H]^+$
$^1$H NMR (400 MHz, $CDCl_3$): $\delta$ [ppm] = 3.66-3.61 (m, 1H), 3.49-3.39 (m, 1H), 3.32-3.29 (m, 2H), 3.07 (s, 3H), 2.75-2.70 (m, 1H), 1.76-7-1.71 (m, 1H), 1.36-1.30 (m, 1H), 0.73-0.69 (m, 1H), 0.54-0.49 (m, 2H), 0.10-0.06 (m, 2H).

**Intermediate 52**

7-(cyclopropylmethyl)-5-methyl-3-phenyl-1,5,6,7-tetrahydro-4H-pyrrolo[3,2-c]pyridin-4-one (Racemate)

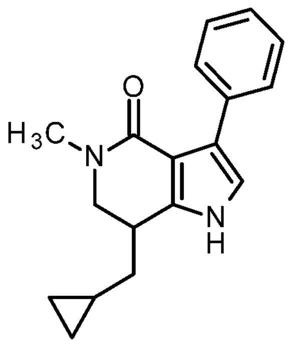

5-(Cyclopropylmethyl)-1-methylpiperidine-2,4-dione (Racemate) (see Intermediate 51, 1.00 g), 2-amino-1-phenylethan-1-one-hydrogen chloride salt (CAS-RN:[5468-37-1], 947 mg, 5.52 mmol) and potassium acetate (1.08 g, 11.0 mmol) were suspended in water (9.9 mL) and stirred at 90 °C for one hour under nitrogen atmosphere. The reaction mixture was diluted with water, the unsolved precipitate was filtered off, washed with water and dried at 50°C under vacuum to provide 1.50 g of the title compound.

LC-MS (Method 2): $R_t$ = 1.08 min; MS (ESIpos): m/z = 281.6 $[M+H]^+$

$^1$H-NMR (400MHz, DMSO-$d_6$): $\delta$ [ppm]= -0.04 - 0.16 (m, 2H), 0.32 - 0.45 (m, 1H), 0.47 - 0.61 (m, 1H), 0.70 - 0.88 (m, 1H), 1.47 (ddd, 1H), 1.54 - 1.69 (m, 1H), 2.93 (s, 3H), 2.99 - 3.11 (m, 1H), 3.43 (dd, 1H), 3.72 (dd, 1H), 6.87 (d, 1H), 7.09 - 7.18 (m, 1H), 7.21 - 7.37 (m, 2H), 7.59 - 7.75 (m, 2H), 11.25 (br s, 1H).

**Intermediate 53**

2-bromo-7-(cyclopropylmethyl)-5-methyl-3-phenyl-1,5,6,7-tetrahydro-4H-pyrrolo[3,2-c]pyridin-4-one (Racemate)

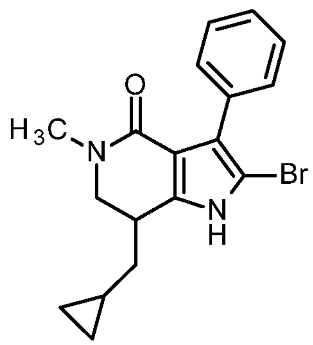

7-(Cyclopropylmethyl)-5-methyl-3-phenyl-1,5,6,7-tetrahydro-4H-pyrrolo[3,2-c]pyridin-4-one (Racemate) (see Intermediate 52, 1.50 g) was suspended in DMF (12 mL), treated with N-bromosuccinimide (CAS-RN:[128-08-5], 952 mg, 5.35 mmol) portion wise and stirred at room temperature under nitrogen atmosphere for 15 min. The reaction mixture was concentrated under reduced pressure. The residue was diluted with methanol. The unsolved precipitate was filtered off, washed with methanol and dried under vacuum at 50 °C to provide 1.53 g of the title compound.

LC-MS (Method 2): Rt = 1.15 min; MS (ESIpos): m/z = 359 $[M+H]^+$

$^1$H-NMR (400MHz, DMSO-$d_6$): δ [ppm]= -0.02 - 0.14 (m, 2H), 0.38 - 0.46 (m, 1H), 0.47 - 0.55 (m, 1H), 0.70 - 0.84 (m, 1H), 1.44 - 1.64 (m, 2H), 2.87 (s, 3H), 3.03 (dd, 1H), 3.43 (dd, 1H), 3.72 (dd, 1H), 7.20 - 7.26 (m, 1H), 7.27 - 7.36 (m, 4H), 11.94 (s, 1H).

**Intermediate 54**

2-(2-aminopyridin-4-yl)-7-(cyclopropylmethyl)-5-methyl-3-phenyl-1,5,6,7-tetrahydro-4H-pyrrolo[3,2-c]pyridin-4-one (Racemate)

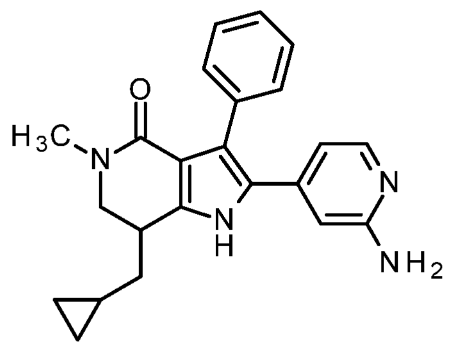

2-Bromo-7-(cyclopropylmethyl)-5-methyl-3-phenyl-1,5,6,7-tetrahydro-4H-pyrrolo[3,2-c]pyridin-4-one (Racemate) (see Intermediate 53, 500 mg), 4-(4,4,5,5-tetramethyl-1,3,2-dioxaborolan-2-yl)pyridin-2-amine (CAS-RN:[1195995-72-2], 459 mg, 2.09 mmol), caesium carbonate (1.36 g, 4.18 mmol) and XPhos Pd G3 (CAS-RN:[1445085-55-1], 118 mg, 139 μmol) were suspended in a degassed mixture of 1,4-dioxane (6.0 mL) and water (630 μL) and stirred at 80 °C overnight under nitrogen atmosphere. The reaction mixture was diluted with a mixture of methylene chloride/isopropanole (7:3) and water. The phases were separated and the aqueous layer was extracted three times with methylene chloride. The combined organic layers were dried using a waterresistant filter and concentrated under reduced pressure. The crude product was purified by flash chromatography (28 g column, amino phase, gradient: ethyl acetate / ethanol 0-10% ethanol) to provide 451 mg of the title compound.

LC-MS (Method 2): Rt = 0.99 min; MS (ESIpos): m/z = 373 $[M+H]^+$

$^1$H-NMR (400MHz, DMSO-$d_6$): δ [ppm]= -0.03 - 0.16 (m, 2H), 0.38 - 0.48 (m, 1H), 0.48 - 0.58 (m, 1H), 0.77 - 0.90 (m, 1H), 1.59 (t, 2H), 2.86 (s, 3H), 3.01 - 3.14 (m, 1H), 3.45 (dd, 1H), 3.75 (dd, 1H), 5.78 (s, 2H), 6.06 (dd, 1H), 6.25 - 6.33 (m, 1H), 7.13 - 7.28 (m, 5H), 7.64 (d, 1H), 11.51 (s, 1H).

**Intermediate 55**

7-(cyclopropylmethyl)-3-(4-fluorophenyl)-5-methyl-1,5,6,7-tetrahydro-4H-pyrrolo[3,2-c]pyridin-4-one (Racemate)

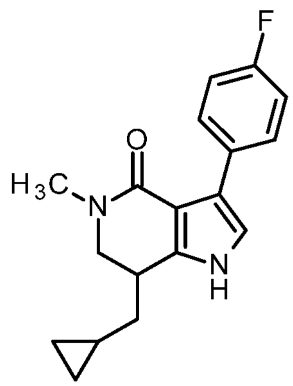

5-(Cyclopropylmethyl)-1-methylpiperidine-2,4-dione (Racemate) (see Intermediate 51, 1.00 g), 2-amino-1-(4-fluorophenyl)ethan-1-one-hydrogen chloride salt (CAS-RN:[456-00-8], 1.05 g, 5.52 mmol) and potassium acetate (1.08 g, 11.0 mmol) were suspended in water (9.9 mL) and stirred at 90 °C over night under nitrogen atmosphere. The suspension was diluted with some water. The formed precipitate was filtered off, washed with water and dried at 50°C under vacuum to provide 1.58 g of the title compound.

LC-MS (Method 2): Rt = 1.11 min; MS (ESIpos): m/z = 299.6 $[M+H]^+$

$^1$H-NMR (400MHz, DMSO-$d_6$): δ [ppm]= -0.08 - 0.13 (m, 2H), 0.36 - 0.45 (m, 1H), 0.46 - 0.58 (m, 1H), 0.71 - 0.84 (m, 1H), 1.41 - 1.53 (m, 1H), 1.54 - 1.65 (m, 1H), 2.92 (s, 3H), 2.98 - 3.10 (m, 1H), 3.42 (dd, 1H), 3.71 (dd, 1H), 6.88 (d, 1H), 7.01 - 7.12 (m, 2H), 7.64 - 7.74 (m, 2H), 11.27 (br s, 1H).

**Intermediate 56**

2-bromo-7-(cyclopropylmethyl)-3-(4-fluorophenyl)-5-methyl-1,5,6,7-tetrahydro-4H-pyrrolo[3,2-c]pyridin-4-one (Racemate)

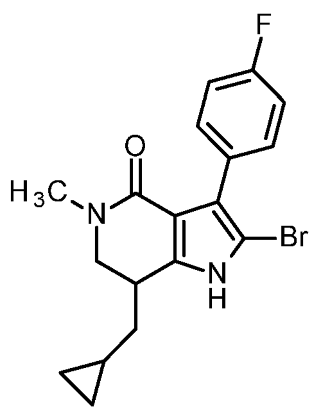

7-(Cyclopropylmethyl)-3-(4-fluorophenyl)-5-methyl-1,5,6,7-tetrahydro-4H-pyrrolo[3,2-c]pyridin-4-one (Racemate) (see Intermediate 55, 1.58 g) was suspended in DMF (12 mL), treated with N-bromosuccinimide (CAS-RN:[128-08-5], 943 mg, 5.30 mmol) portion wise and stirred at room temperature under nitrogen atmosphere for 15 min. The reaction mixture was concentrated under reduced pressure. The residue was diluted with methanol. The unsolved precipitate was filtered off, washed with methanol and dried under vacuum at 50 °C to provide 1.63 g of the title compound.

LC-MS (Method 2): Rt = 1.18 min; MS (ESIpos): m/z = 377 $[M+H]^+$

$^1$H-NMR (400MHz, DMSO-$d_6$): δ [ppm]= -0.07 - 0.12 (m, 2H), 0.37 - 0.46 (m, 1H), 0.47 - 0.56 (m, 1H), 0.68 - 0.83 (m, 1H), 1.42 - 1.66 (m, 2H), 2.87 (s, 3H), 2.99 - 3.08 (m, 1H), 3.42 (dd, 1H), 3.72 (dd, 1H), 7.05 - 7.18 (m, 2H), 7.31 - 7.42 (m, 2H), 11.97 (br s, 1H).

**Intermediate 57**

2-(2-aminopyridin-4-yl)-7-(cyclopropylmethyl)-3-(4-fluorophenyl)-5-methyl-1,5,6,7-tetrahydro-4H-pyrrolo[3,2-c]pyridin-4-one (Racemate)

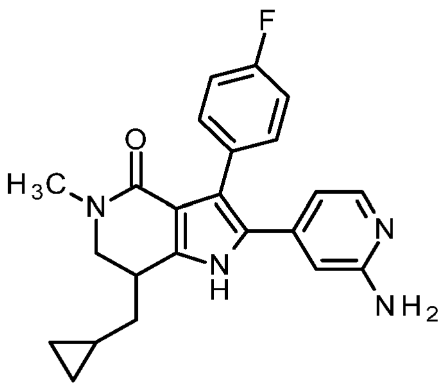

2-Bromo-7-(cyclopropylmethyl)-3-(4-fluorophenyl)-5-methyl-1,5,6,7-tetrahydro-4H-pyrrolo[3,2-c]pyridin-4-one (Racemate) (see Intermediate 56, 500 mg), 4-(4,4,5,5-tetramethyl-1,3,2-dioxaborolan-2-yl)pyridin-2-amine (CAS-RN:[1195995-72-2], 438 mg, 1.99 mmol), caesium carbonate (1.30 g, 3.98 mmol) and XPhos Pd G3 (CAS-RN:[1445085-55-1], 112 mg, 133 μmol) were suspended in a degassed mixture of 1,4-dioxane (5.7 mL) and water (600 μL) and stirred at 80 °C overnight under nitrogen atmosphere. The reaction mixture was diluted with a mixture of methylene chloride/isopropanole (7:3) and water. The phases were separated and the aqueous layer was extracted three times with methylene chloride. The combined organic layers were dried using a water resistant filter and concentrated under reduced pressure. The crude product was purified by flash chromatography (11 g column, amino phase, gradient: ethyl acetate / ethanol 0-10% ethanol) to provide 507 mg of the title compound.

LC-MS (Method 2): $R_t$ = 1.03 min; MS (ESIpos): m/z = 391 $[M+H]^+$

$^1$H-NMR (400MHz, DMSO-$d_6$): δ [ppm]= -0.01 - 0.16 (m, 2H), 0.37 - 0.47 (m, 1H), 0.48 - 0.57 (m, 1H), 0.75 - 0.88 (m, 1H), 1.58 (t, 2H), 2.86 (s, 3H), 3.01 - 3.12 (m, 1H), 3.45 (dd, 1H), 3.75 (dd, 1H), 5.79 (s, 2H), 6.11 (dd, 1H), 6.25 (d, 1H), 7.00 - 7.15 (m, 2H), 7.16 - 7.26 (m, 2H), 7.69 (d, 1H), 11.53 (s, 1H).

**Intermediate 58**

3-(2,4-difluorophenyl)-5-methyl-1,5,6,7-tetrahydro-4H-pyrrolo[3,2-c]pyridin-4-one

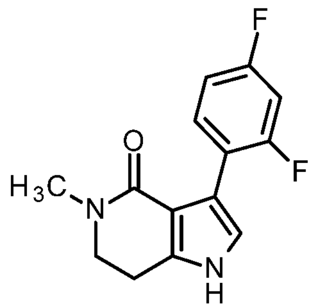

1-Methylpiperidine-2,4-dione (CAS-RN:[118263-97-1], 303 mg, 2.38 mmol), 2-amino-1-(2,4-difluorophenyl)ethan-1-one-hydrogen chloride salt (CAS-RN:[786719-60-6], 495 mg, 2.38 mmol) and potassium acetate (468 mg, 4.77 mmol) were suspended in water (4.3 mL) and stirred at room temperature for 5 min. The mixture was heated at 90 °C overnight. The mixture was diluted with some water and the precipitated solid was filtered off, the filter cake was washed with water twice and dried in a vacuum drying oven to provide 410 mg of the title compound.

LC-MS (Method 2): $R_t$ = 0.88 min; MS (ESIpos): m/z = 263 $[M+H]^+$

$^1$H-NMR (400MHz, DMSO-$d_6$): δ [ppm]= 2.80 - 2.92 (m, 5H), 3.53 (t, 2H), 6.80 (d, 1H), 6.96 - 7.06 (m, 1H), 7.09 - 7.23 (m, 1H), 7.54 (td, 1H), 11.35 (br s, 1H).

**Intermediate 59**

2-bromo-3-(2,4-difluorophenyl)-5-methyl-1,5,6,7-tetrahydro-4H-pyrrolo[3,2-c]pyridin-4-one

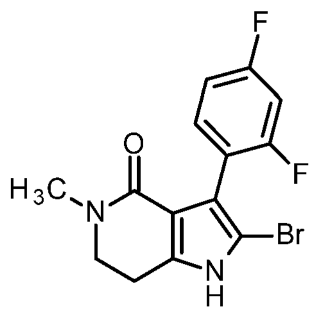

3-(2,4-Difluorophenyl)-5-methyl-1,5,6,7-tetrahydro-4H-pyrrolo[3,2-c]pyridin-4-one (see Intermediate 58, 400 mg) was suspended in DMF (3.5 mL), treated with N-bromosuccinimide (CAS-RN:[128-08-5], 271 mg, 1.53 mmol) portion wise and stirred at room temperature under nitrogen atmosphere for 15 min. The precipitate in the reaction mixture was filtered off, the filter cake was washed with DMF and dried in vacuum to give a first fraction of the title compound as a white solid.

The filtrate was concentrated under reduced pressure and the residue was diluted with MeOH. The resulting precipitate was filtered off, the filter cake was washed with MeOH twice and dried in vacuum to give a second fraction of the title compound.

Both fractions were combined to provide 438 mg of the title compound.

LC-MS (Method 2): Rt = 0.97 min; MS (ESIpos): m/z = 341 $[M+H]^+$

$^1$H-NMR (400MHz, DMSO-$d_6$): δ [ppm]= 2.80 - 2.88 (m, 5H), 3.52 (br t, 2H), 6.99 - 7.10 (m, 1H), 7.18 (td, 1H), 7.28 (td, 1H), 12.12 (br s, 1H).

**Intermediate 60**

2-(2-aminopyridin-4-yl)-3-(2,4-difluorophenyl)-5-methyl-1,5,6,7-tetrahydro-4H-pyrrolo[3,2-c]pyridin-4-one

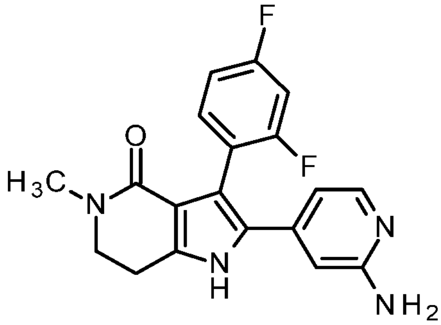

2-Bromo-3-(2,4-difluorophenyl)-5-methyl-1,5,6,7-tetrahydro-4H-pyrrolo[3,2-c]pyridin-4-one (see Intermediate 59, 430 mg), 4-(4,4,5,5-tetramethyl-1,3,2-dioxaborolan-2-yl)pyridin-2-amine (CAS-RN:[1195995-72-2], 416 mg, 1.89 mmol), caesium carbonate (1.23 g, 3.78 mmol) and XPhos Pd G3 (CAS-RN:[1445085-55-1], 107 mg, 126 μmol) were suspended in a degassed mixture of 1,4-dioxane (5.4 mL) and water (570 μL) and stirred at 80 °C overnight under nitrogen atmosphere. The reaction mixture was diluted with a mixture of methylene chloride/isopropanole (7:3) and water. The phases were separated and the aqueous layer was extracted with methylene chloride once. The combined organic layers were dried using a water resistant filter and concentrated under reduced pressure. The crude product was purified by preparative reverse phase HPLC (eluent: acetonitrile / water with ammonia as additive) to provide 281 mg of the title compound.

LC-MS (Method 2): $R_t$ = 0.81 min; MS (ESIpos): m/z = 355 $[M+H]^+$

$^1$H-NMR (400MHz, DMSO-$d_6$): δ [ppm]= 2.82 (s, 3H), 2.92 (t, 2H), 3.55 (t, 2H), 5.79 (s, 2H), 6.15 - 6.26 (m, 2H), 6.93 - 7.03 (m, 1H), 7.11 - 7.24 (m, 2H), 7.72 (d, 1H), 11.78 (s, 1H).

**Intermediate 61**

3-phenyl-1,5,6,7-tetrahydro-4H-pyrrolo[3,2-c]pyridin-4-one

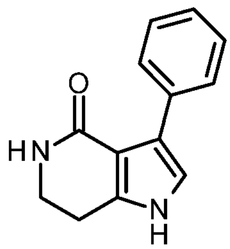

Piperidine-2,4-dione (CAS-RN:[50607-30-2], 800 mg, 7.07 mmol), 2-amino-1-phenylethan-1-one-hydrogen chloride salt (CAS-RN:[5468-37-1], 1.21 g, 7.07 mmol) and potassium acetate (1.39 g, 14.1 mmol) were suspended in water (13 mL) and stirred at room temperature for 5 min. The mixture was heated at 90 °C overnight under nitrogen atmosphere in a sealed vessel. The mixture was diluted with some water and the precipitated solid was filtered off, the filter cake was washed with water and dried at 50°C under vacuum to provide 1.91 g of the title compound.

LC-MS (Method 2): $R_t$ = 0.75 min; MS (ESIpos): m/z = 213 $[M+H]^+$

$^1$H-NMR (400MHz, DMSO-$d_6$): δ [ppm]= 2.75 (t, 2H), 3.34 - 3.41 (m, 2H), 6.85 - 6.99 (m, 2H), 7.08 - 7.18 (m, 1H), 7.21 - 7.31 (m, 2H), 7.64 - 7.77 (m, 2H), 11.32 (br s, 1H).

**Intermediate 62**

2-bromo-3-phenyl-1,5,6,7-tetrahydro-4H-pyrrolo[3,2-c]pyridin-4-one

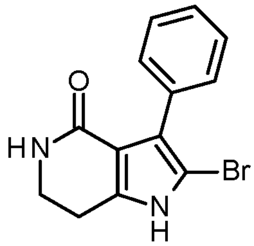

3-Phenyl-1,5,6,7-tetrahydro-4H-pyrrolo[3,2-c]pyridin-4-one (see Intermediate 61, 1.91 g) was suspended in DMF (21 mL), treated with N-bromosuccinimide (CAS-RN:[128-08-5], 1.60 g, 9.00 mmol) portion wise and stirred at room temperature under nitrogen atmosphere for 15 min. The reaction mixture was concentrated under reduced pressure. The residue was diluted with methanol. The unsolved precipitate was filtered off, washed with methanol and dried under vacuum at 50 °C to provide 1.62 g of the title compound.

LC-MS (Method 2): $R_t$ = 0.87 min; MS (ESIpos): m/z = 291 $[M+H]^+$

$^1$H-NMR (400MHz, DMSO-$d_6$): δ [ppm]= 2.74 (t, 2H), 3.36 (td, 2H), 6.97 (br s, 1H), 7.19 - 7.26 (m, 1H), 7.28 - 7.34 (m, 2H), 7.35 - 7.42 (m, 2H), 12.03 (s, 1H).

**Intermediate 63**

2-(2-aminopyridin-4-yl)-3-phenyl-1,5,6,7-tetrahydro-4H-pyrrolo[3,2-c]pyridin-4-one

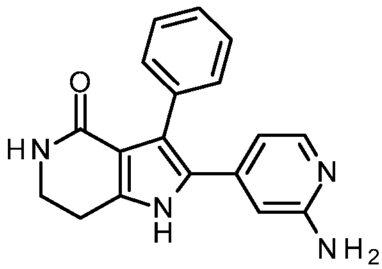

2-Bromo-3-phenyl-1,5,6,7-tetrahydro-4H-pyrrolo[3,2-c]pyridin-4-one (see Intermediate 62, 900 mg), 4-(4,4,5,5-tetramethyl-1,3,2-dioxaborolan-2-yl)pyridin-2-amine (CAS-RN:[1195995-72-2], 1.02 g, 4.64 mmol), caesium carbonate (3.02 g, 9.27 mmol) and XPhos Pd G3 (CAS-RN:[1445085-55-1], 262 mg, 309 μmol) were suspended in a degassed mixture of 1,4-dioxane (13 mL) and water (1.4 mL) and stirred at 80 °C overnight under nitrogen atmosphere. The reaction mixture was diluted with a mixture of methylene chloride/isopropanole (7:3) and water. The precipitate in both layers was filtered off and dried at 50°C under vacuum to provide 518 mg of the title compound.

LC-MS (Method 2): $R_t$ = 0.70 min; MS (ESIpos): m/z = 305 $[M+H]^+$

$^1$H-NMR (400MHz, DMSO-$d_6$): δ [ppm]= 2.81 (t, 2H), 3.36 - 3.41 (m, 2H), 5.66 - 5.80 (m, 2H), 6.09 (dd, 1H), 6.27 (d, 1H), 6.90 (br s, 1H), 7.16 - 7.30 (m, 5H), 7.65 (d, 1H), 11.52 - 11.79 (m, 1H).

**Intermediate 64**

ethyl 3-{[1- (2 -ethoxy-2 -oxoethyl) cycl opropyl] (methyl)am i no }-3-oxopropanoate

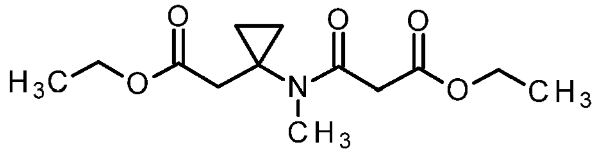

Ethyl [1-(methylamino)cyclopropyl]acetate (see US Patent: Lauffer et al., US2019/0322673 A1; 5.00 g, 31.8 mmmol) was dissolved in 32 mL dichloromethane and cooled down to 0°C. Then the N,N-diisopropylethylamine (6.1 mL, 35 mmol) and DMAP (389 mg, 3.18 mmol) were added. Subsequentely ethyl 3-chloro-3-oxopropanoate (CAS-RN:[36239-09-5], 4.4 mL, 35 mmol) was added. The reaction mixture was stirred at 0°C for 1.5 hours and at rt over night under nitrogen atmosphere. To the reaction mixture half concentrated aqueous ammoniumchloride solution and dichloromethane was added. The aqueous layer was extracted with dichloromethane three times. The combined organic layers were filtered through a silicone coated filter and concentrated under reduced pressure. The crude product was purified by flash chromatography (50g column, silica ULTRA; hexane / ethyl acetate 12%-100%) to provide the target compound in approximately 45% purity, which was used without further purification: 2.7 g.

LC-MS (Method 1): Rt = 0.87 min; MS (ESIpos): m/z = 272 [M+H]+

$^1$H-NMR (400 MHz, DMSO-$d_6$) $\delta$ [ppm]: 0.782 (1.12), 0.788 (1.22), 0.808 (1.03), 0.812 (1.25), 0.818 (1.20), 0.855 (0.43), 0.868 (0.52), 0.987 (0.45), 1.113 (0.95), 1.133 (1.13), 1.156 (3.18), 1.166 (6.99), 1.169 (6.26), 1.174 (5.91), 1.183 (14.29), 1.187 (12.08), 1.192 (3.19), 1.201 (6.98),

**Intermediate 65**

ethyl 4-methyl-5,7-dioxo-4-azaspiro[2.5]octane-6-carboxylate (Racemate)

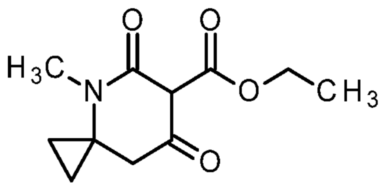

Sodium ethoxide solution (2.6 mL, 21 % purity in ethanol, 11 mmol) was cooled down with an ice bath and treated drop wise with ethyl 3-{[1-(2-ethoxy-2-oxoethyl)cyclopropyl](methyl)amino}-3-oxopropanoate (see Intermediate 64, 2.70 g), dissolved in 14 mL ethanol. It was stirred at this temperature for 30 minutes and at rt over night under nitrogen atmosphere. The reaction mixture was diluted with dichloromethane and the pH was adjusted to 1 by addition of hydrochloric acid (4 M). The reaction mixture was stirred for a few minutes, filtered through a silicone coated filter and concentrated under reduced pressure to provide 2.2 g of the target compound.

LC-MS (Method 1): Rt = 0.83 min; MS (ESIpos): m/z = 226 [M+H]+

$^1$H-NMR (400 MHz, DMSO-$d_6$) $\delta$ [ppm]: 0.658 (1.76), 0.672 (5.52), 0.674 (5.48), 0.689 (2.15), 0.781 (1.12), 0.787 (1.49), 0.801 (1.45), 0.805 (1.62), 0.807 (1.55), 0.818 (1.71), 0.838 (0.47), 0.855 (0.47), 0.867 (0.53), 0.967 (0.54), 1.018 (3.40), 1.035 (1.97), 1.052 (2.46), 1.070 (1.24), 1.082 (0.54), 1.095 (1.06), 1.099 (1.14), 1.113 (1.02), 1.133 (0.91), 1.156 (2.19), 1.165 (5.39), 1.168 (4.95), 1.174 (4.79), 1.183 (10.62), 1.186 (9.21), 1.192 (3.91), 1.201 (5.58), 1.204 (4.91), 1.211 (8.24), 1.229 (16.00), 1.247 (7.56), 2.044 (0.73), 2.412 (0.41), 2.430 (0.43), 2.450 (0.59), 2.470 (2.76), 2.518 (1.61), 2.522 (1.35), 2.544 (2.43), 2.560 (1.58), 2.662 (4.71), 2.703 (0.83), 2.720 (0.48), 2.744 (1.41), 2.751 (4.66), 2.770 (2.57), 2.795 (9.66), 2.798 (5.11), 2.822 (0.63), 2.836 (0.50), 2.864 (0.53), 2.900 (5.03), 2.908 (1.97), 3.300 (1.17), 3.397 (2.91), 3.426 (0.43), 3.461 (2.47), 3.559 (0.78), 3.635 (0.75), 4.003 (0.54), 4.021 (1.62),

4.031 (0.44), 4.039 (1.99), 4.043 (1.33), 4.048 (0.77), 4.052 (1.27), 4.057 (1.85), 4.061 (3.35), 4.069 (3.20), 4.074 (1.52), 4.079 (3.33), 4.087 (3.06), 4.091 (0.78), 4.097 (1.37), 4.105 (1.03), 4.115 (0.44), 4.137 (0.70), 4.155 (0.73), 4.168 (2.15), 4.186 (6.14), 4.204 (6.04), 4.221 (1.93), 4.732 (0.77).

**Intermediate 66**

4-methyl-4-azaspiro[2.5]octane-5,7-dione

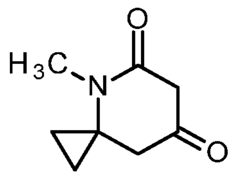

Ethyl 4-methyl-5,7-dioxo-4-azaspiro[2.5]octane-6-carboxylate (Racemate) (see Intermediate 65, 7.70 g) was dissolved in 66 mL dichloromethane and treated with aqueous hydrochloric acid (64 mL, 2.0 M, 130 mmol). The reaction mixture was stirred for 20 minutes at rt under nitrogen atmosphere. The reaction mixture was filtered through a water resistant filter and concentrated under reduced pressure. The residue was dissolved in 90 mL acetonitrile and treated with 9.2 mL water. The reaction mixture was stirred under reflux for 1 hour at 100 °C under nitrogen atmosphere. The crude product was concentrated under reduced pressure. The crude product was purified by flash chromatography (50g column, silica gel ULTRA; ethyl acetate / ethanol 0%-20%) to provide 2.4 g of the target compound.

LC-MS (Method 1): Rt = 0.50 min; MS (ESIpos): m/z = 154 [M+H]+

$^1$H-NMR (400 MHz, DMSO-$d_6$) $\delta$ [ppm] = 0.77 - 0.85 (m, 2H), 1.06 - 1.13 (m, 2H), 2.47 (s, 2H), 2.75 (s, 3H), 3.46 (s, 2H).

**Intermediate 67**

5'-methyl-3'-phenyl-1',7'-dihydrospiro[cyclopropane-1,6'-pyrrolo[3,2-c]pyridin]-4'(5'H)-one

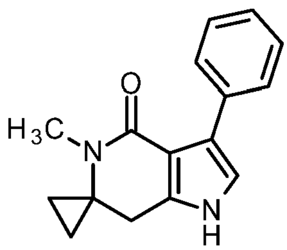

4-Methyl-4-azaspiro[2.5]octane-5,7-dione (see Intermediate 66, 700 mg), 2-amino-1-phenylethan-1-one-hydrogen chloride salt (CAS-RN:[5468-37-1], 784 mg, 4.57 mmol) and potassium acetate (897 mg, 9.14 mmol) were suspended in water (8.2 mL) and stirred at room temperature for 5 min. The mixture was heated at 90 °C overnight under argon atmosphere. The mixture was diluted with some water, stirred for 15 min and the unsolved precipitate was filtered off, the filter cake was washed with water and dried at 50°C under vacuum to provide 1.12 g of the title compound.

LC-MS (Method 2): $R_t$ = 0.93 min; MS (ESIpos): m/z = 253.5 [M+H]$^+$

$^1$H-NMR (400MHz, DMSO-$d_6$): $\delta$ [ppm]= 0.68 - 0.75 (m, 2H), 1.02 - 1.08 (m, 2H), 2.73 (s, 3H), 2.76 (s, 2H), 6.89 (d, 1H), 7.08 - 7.19 (m, 1H), 7.22 - 7.30 (m, 2H), 7.61 - 7.68 (m, 2H), 11.27 (br s, 1H).

**Intermediate 68**

2'-bromo-5'-methyl-3'-phenyl-1',7'-dihydrospiro[cyclopropane-1,6'-pyrrolo[3,2-c]pyridin]-4'(5'H)-one

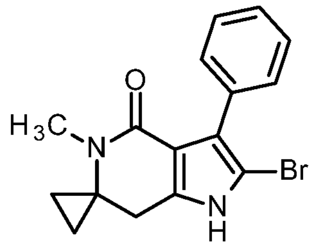

5'-Methyl-3'-phenyl-1',7'-dihydrospiro[cyclopropane-1,6'-pyrrolo[3,2-c]pyridin]-4'(5'H)-one (see Intermediate 67, 1.12 g) was suspended in DMF (10 mL), treated with N-bromosuccinimide (CAS-RN:[128-08-5], 791 mg, 4.44 mmol) portion wise and stirred at room temperature for 15 min. The reaction mixture was concentrated under reduced pressure. The residue was diluted with ethanol and it was sonicated for 5 minutes. The unsolved precipitate was filtered off, washed with methanol and dried at 50 °C under vacuum to provide 517 mg of the title compound.

LC-MS (Method 2): $R_t$ = 1.02 min; MS (ESIpos): m/z = 331 $[M+H]^+$

$^1$H-NMR (400MHz, DMSO-$d_6$): δ [ppm]= 0.69 - 0.79 (m, 2H), 0.99 - 1.10 (m, 2H), 2.67 (s, 3H), 2.74 (s, 2H), 7.18 - 7.26 (m, 1H), 7.27 - 7.42 (m, 4H), 12.02 (br s, 1H).

**Intermediate 69**

2'-(2-aminopyridin-4-yl)-5'-methyl-3'-phenyl-1',7'-dihydrospiro[cyclopropane-1,6'-pyrrolo[3,2-c]pyridin]-4'(5'H)-one

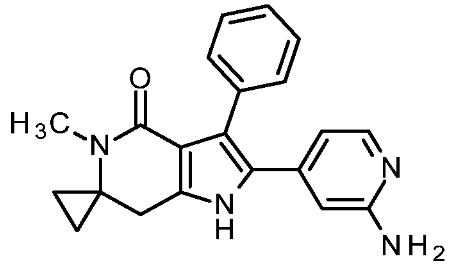

2'-Bromo-5'-methyl-3'-phenyl-1',7'-dihydro-spiro[cyclopropane-1,6'-pyrrolo[3,2-c]pyridin]-4'(5'H)-one (see Intermediate 68, 510 mg), 4-(4,4,5,5-tetramethyl-1,3,2-dioxaborolan-2-yl)pyridin-2-amine (CAS-RN:[1195995-72-2], 508 mg, 2.31 mmol), caesium carbonate (1.51 g, 4.62 mmol) and XPhos Pd G3 (CAS-RN:[1445085-55-1], 130 mg, 154 µmol) were suspended in a degassed mixture of 1,4-dioxane (6.6 mL) and water (690 µL) and stirred at 80 °C overnight under nitrogen atmosphere. The reaction mixture was diluted with a mixture of methylene chloride/isopropanole (7:3) and water. The phases were separated and the aqueous layer was extracted three times with methylene chloride. The combined organic layers were dried using a silicone coated filter and concentrated under reduced pressure. The crude product was purified by chromatography (28 g column, aminophase, gradient: methylene chloride /ethanol 0-20% ethanol) and by preparative reverse phase HPLC (eluent: acetonitrile / water with ammonia as additive) to provide 79 mg of the title compound.
LC-MS (Method 2): Rt = 0.95 min; MS (ESIpos): m/z = 345 $[M+H]^+$

**Intermediate 70**

3'-(4-fluorophenyl)-5'-methyl-1',7'-dihydrospiro[cyclopropane-1,6'-pyrrolo[3,2-c]pyridin]-4'(5'H)-one

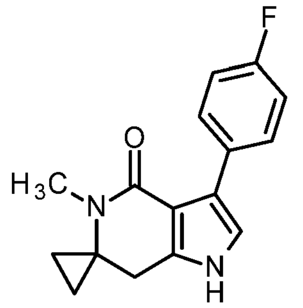

4-Methyl-4-azaspiro[2.5]octane-5,7-dione (see Intermediate 66, 700 mg), 2-amino-1-(4-fluorophenyl)ethan-1-one-hydrogen chloride salt (CAS-RN:[456-00-8], 867 mg, 4.57 mmol) and potassium acetate (897 mg, 9.14 mmol) were suspended in water (8.2 mL) and stirred at 90 °C over night under argon atmosphere. The suspension was diluted with some water and stirred for 15 min. The formed precipitate was filtered off, washed with water and dried at 50°C under vacuum to provide 1.15 g of the title compound.

LC-MS (Method 2): Rt = 0.97 min; MS (ESIpos): m/z = 271 $[M+H]^+$

$^1$H-NMR (400MHz, DMSO-$d_6$): δ [ppm]= 0.67 - 0.75 (m, 2H), 1.01 - 1.07 (m, 2H), 2.73 (s, 3H), 2.75 (s, 2H), 6.89 (d, 1H), 7.02 - 7.15 (m, 2H), 7.61 - 7.73 (m, 2H), 11.29 (br s, 1H).

**Intermediate 71**

2'-bromo-3'-(4-fluorophenyl)-5'-methyl-1',7'-dihydrospiro[cyclopropane-1,6'-pyrrolo[3,2-c]pyridin]-4'(5'H)-one

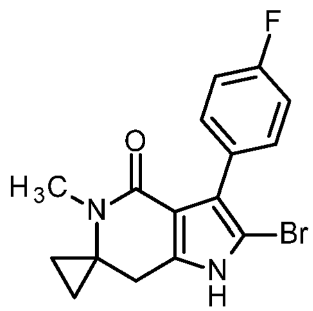

3'-(4-Fluorophenyl)-5'-methyl-1',7'-dihydrospiro[cyclopropane-1,6'-pyrrolo[3,2-c]pyridin]-4'(5'H)-one (see Intermediate 70, 1.15 g) was suspended in DMF (9.8 mL), treated with N-bromosuccinimide (CAS-RN:[128-08-5], 757 mg, 4.25 mmol) portion wise and stirred at room temperature for 2 hours. The reaction mixture was concentrated under reduced pressure. The residue was diluted with methanol (~10 mL) and it was sonicated for 5 minutes. The unsolved precipitate was filtered off, washed with methanol and dried at 50 °C under vacuum to provide 537 mg of the title compound.

LC-MS (Method 2): Rt = 1.06 min; MS (ESIpos): m/z = 349 $[M+H]^+$

$^1$H-NMR (400MHz, DMSO-$d_6$): δ [ppm]= 0.71 - 0.77 (m, 2H), 1.00 - 1.10 (m, 2H), 2.66 (s, 3H), 2.74 (s, 2H), 7.10 - 7.20 (m, 2H), 7.34 - 7.43 (m, 2H), 12.05 (s, 1H).

**Intermediate 72**

2'-(2-aminopyridin-4-yl)-3'-(4-fluorophenyl)-5'-methyl-1',7'-dihydrospiro[cyclopropane-1,6'-pyrrolo[3,2-c]pyridin]-4'(5'H)-one

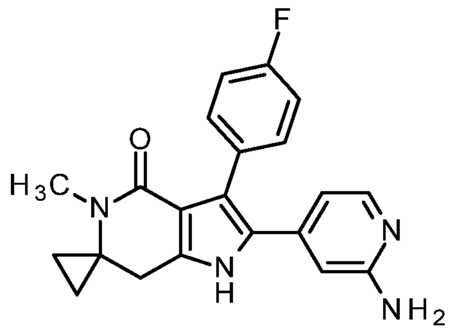

2'-Bromo-3'-(4-fluorophenyl)-5'-methyl-1',7'-dihydrospiro[cyclopropane-1,6'-pyrrolo[3,2-c]-pyridin]-4'(5'H)-one (see Intermediate 71, 530 mg), 4-(4,4,5,5-tetramethyl-1,3,2-dioxaborolan-2-yl)pyridin-2-amine (CAS-RN:[1195995-72-2], 501 mg, 2.28 mmol), caesium carbonate (1.48 g, 4.55 mmol) and XPhos Pd G3 (CAS-RN:[1445085-55-1], 128 mg, 152 μmol) were suspended in a degassed mixture of 1,4-dioxane (6.5 mL) and water (680 μL) and stirred at 80 °C overnight under argon atmosphere. The reaction mixture was diluted with a mixture of methylene chloride/isopropanole (7:3) and water. The phases were separated and the aqueous layer was extracted three times with methylene chloride. The combined organic layers were dried using a silicone coated filter and concentrated under reduced pressure. The crude product was purified by chromatography (28 g column, aminophase, gradient: methylene chloride /ethanol 0-20% ethanol) and by preparative reverse phase HPLC (eluent: acetonitrile / water with ammonia as additive) to provide 172 mg of the title compound.
LC-MS (Method 2): Rt = 0.91 min; MS (ESIpos): m/z = 363.6 $[M+H]^+$

**Intermediate 73**

6,6-dimethyl-3-phenyl-1,5,6,7-tetrahydro-4H-pyrrolo[3,2-c]pyridin-4-one

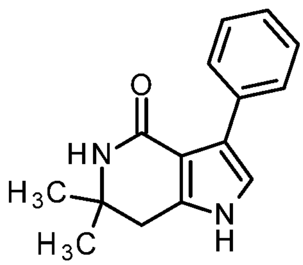

A screw cap vial with a pressure septum was loaded with 2-amino-1-phenylethan-1-one-hydrogen chloride salt (CAS-RN:[5468-37-1], 608 mg, 3.54 mmol), 6,6-dimethylpiperidine-2,4-dione (CAS-RN:[5239-39-4], 500 mg, 3.54 mmol) and potassium acetate (694 mg, 7.08 mmol). To the vial was added 7.10 mL of water. The reaction was heated at 90 °C for 2 hours. The reaction was cooled down to rt, filtered and the cake was rinsed with 10 mL of $H_2O$. The product was dried under reduce pressure, yielding the desired product: 528 mg.

LC-MS (Method 3): $R_t$ = 1.25 min; MS (ESIpos): m/z = 241 $[M+H]^+$
$^1$H-NMR (400 MHz, $CDCl_3$) δ [ppm] = 8.28 (s, 1H), 7.78 - 7.72 (m, 2H), 7.39 - 7.34 (m, 2H), 7.27 - 7.20 (m, 1H), 6.79 (d, 1H), 5.15 (s, 1H), 2.85 (s, 2H), 1.41 (s, 6H).

**Intermediate 74**

2-bromo-6,6-dimethyl-3-phenyl-1,5,6,7-tetrahydro-4H-pyrrolo[3,2-c]pyridin-4-one

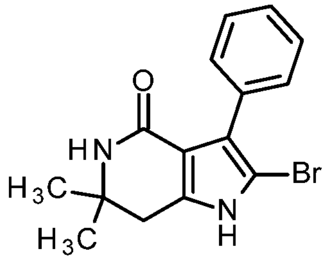

To a solution of 6,6-dimethyl-3-phenyl-1,5,6,7-tetrahydro-4H-pyrrolo[3,2-c]pyridin-4-one (see Intermediate 73, 300 mg) in 3.0 mL of N,N-dimethylformamide was added 1-bromopyrrolidine-2,5-dione (222 mg, 1.25 mmol). The reaction was stirred at rt for 15 min. The reaction was concentrated under vacuum. The crude product was triturated with 5.0 mL of cold methanol, filtered and the product was used without further purification: 306 mg.

LC-MS (Method 3): $R_t$ = 1.33 min; MS (ESIpos): m/z = 319 $[M+H]^+$

$^1$H-NMR (400 MHz, DMSO-$d_6$) δ [ppm] = 11.98 (s, 1H), 7.43 - 7.36 (m, 2H), 7.35 - 7.27 (m, 2H), 7.27 - 7.18 (m, 1H), 6.87 (s, 1H), 2.71 (s, 2H), 1.26 (s, 6H).

**Intermediate 75**

2-(2-aminopyridin-4-yl)-6,6-dimethyl-3-phenyl-1,5,6,7-tetrahydro-4H-pyrrolo[3,2-c]pyridin-4-one

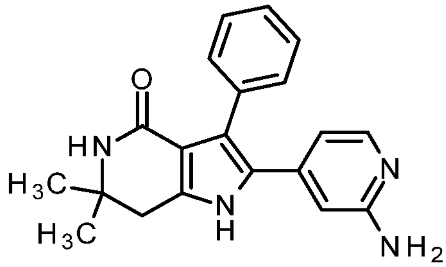

A screw cap vial with a pressure septum was loaded with 2-bromo-6,6-dimethyl-3-phenyl-1,5,6,7-tetrahydro-4H-pyrrolo[3,2-c]pyridin-4-one (see Intermediate 74, 150 mg), 4-(4,4,5,5-tetramethyl-1,3,2-dioxaborolan-2-yl)pyridin-2-amine (123 mg, 558 µmol), Pd(dppf)$Cl_2$ · $CH_2Cl_2$ (18.2 mg, 22.3 µmol) and $Cs_2CO_3$ (319 mg, 982 µmol). The vial was purged with argon and filled with 4.7 mL of 1,4-dioxane / water (4:1). The reaction was added to a preheated hot plate at 100 °C and stirred for 2 hours. The reaction was cooled down, filtered over a plug of silica gel, rinsed with methanol and concentrated under vacuum. The crude product was purified by preparative reverse phase HPLC (eluent: acetonitrile / water 0 to 100 % + 0.1 % formic acid). The title compound containing fractions were concentrated under reduced pressure to provide 19.0 mg of the title compound as a formic acid salt.

LC-MS (Method 5): Rt = 1.80 min; MS (ESIpos): m/z = 333 $[M+H]^+$

$^1$H-NMR (400 MHz, DMSO-$d_6$) δ [ppm] = 11.61 (s, 1H), 8.16 (s, 1H), 7.65 (d, 1H), 7.35 - 7.15 (m, 5H), 6.79 (s, 1H), 6.27 (d, 1H), 6.09 (dd, 1.5 Hz, 1H), 5.74 (s, 2H), 2.78 (s, 2H), 1.27 (s, 6H).

**Intermediate 76**

3-(4-fluorophenyl)-6,6-dimethyl-1,5,6,7-tetrahydro-4H-pyrrolo[3,2-c]pyridin-4-one

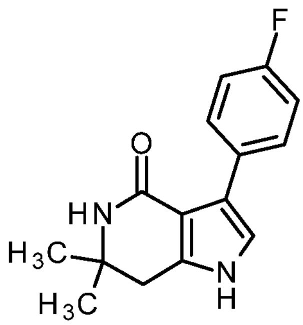

A screw cap vial with a pressure septum was loaded with 2-amino-1-(4-fluorophenyl)ethan-1-one-hydrogen chloride salt (CAS-RN:[456-00-8], 672 mg, 3.54 mmol), 6,6-dimethylpiperidine-2,4-dione (CAS-RN:[5239-39-4], 500 mg, 3.54 mmol) and potassium acetate (694 mg, 7.08 mmol). To the vial was added 7.10 mL of water. The reaction was heated at 90 °C for 14 h. The reaction mixture was cooled down to rt, filtered and the cake was rinsed with 10 mL of water. The product was dried under reduce pressure, yielding the desired product: 458 mg.

LC-MS (Method 3): Rt = 1.28 min; MS (ESIpos): m/z = 259 $[M+H]^+$

$^1$H-NMR (400 MHz, DMSO-$d_6$) $\delta$ [ppm] = $\delta$ 11.29 (s, 1H), 7.96 - 7.73 (m, 2H), 7.08 (t, 2H), 6.93 (d, 1H), 6.84 (s, 1H), 2.73 (s, 2H), 1.26 (s, 6H).

**Intermediate 77**

2-bromo-3-(4-fluorophenyl)-6,6-dimethyl-1,5,6,7-tetrahydro-4H-pyrrolo[3,2-c]pyridin-4-one

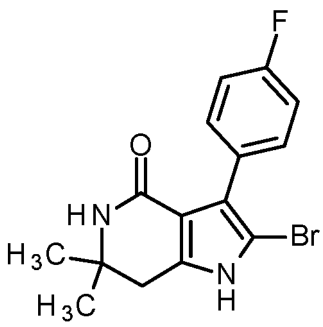

To a solution of 3-(4-fluorophenyl)-6,6-dimethyl-1,5,6,7-tetrahydro-4H-pyrrolo[3,2-c]pyridin-4-one (see Intermediate 76, 405 mg) in 3.92 mL of N,N-dimethylformamide was added 1-bromopyrrolidine-2,5-dione (279 mg, 1.57 mmol). The reaction was stirred at rt for 15 min. The reaction mixture was concentrated under vacuum. The crude product was triturated with 5.0 mL of cold methanol, filtered, dried under vacuum and the product was used without further purification: 410 mg.

LC-MS (Method 3): $R_t$ = 1.38 min; MS (ESIpos): m/z = 337 $[M+H]^+$

$^1$H-NMR (400 MHz, DMSO-$d_6$) $\delta$ [ppm] = 12.01 (s, 1H), 7.42 (dd, 2H), 7.14 (t, 2H), 6.90 (s, 1H), 2.71 (s, 2H), 1.25 (s, 6H).

**Intermediate 78**

2-(2-aminopyridin-4-yl)-3-(4-fluorophenyl)-6,6-dimethyl-1,5,6,7-tetrahydro-4H-pyrrolo[3,2-c]pyridin-4-one

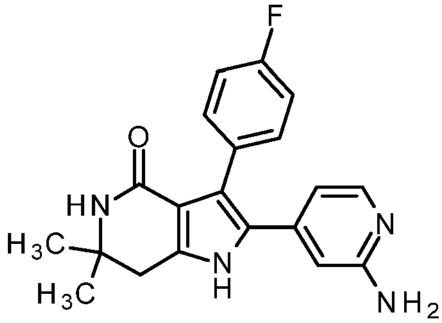

A screw cap vial with a pressure septum was loaded with 2-bromo-3-(4-fluorophenyl)-6,6-dimethyl-1,5,6,7-tetrahydro-4H-pyrrolo[3,2-c]pyridin-4-one (see Intermediate 77, 200 mg), 4-(4,4,5,5-tetramethyl-1,3,2-dioxaborolan-2-yl)pyridin-2-amine (163 mg, 741 μmol), Pd(dppf)$Cl_2$ $CH_2Cl_2$ (24.2 mg, 29.7 μmol) and $Cs_2CO_3$ (424 mg, 1.30 mmol). The vial was purged with argon and filled with 5.93 mL of 1,4-dioxane / water (4:1). The reaction was added to a preheated hot plate at 100 °C and stirred for 2 h. The reaction was cooled down, filtered over a plug of silica gel, rinsed with methanol and concentrated under vacuum. The crude product was purified by preparative reverse phase HPLC (eluent: acetonitrile / water 0 to 100 % + 0.1 % formic acid). The title compound containing fractions were concentrated under reduced pressure to provide 26.0 mg of the title compound as a formic acid salt.

LC-MS (Method 5): $R_t$ = 1.92 min; MS (ESIpos): m/z = 351 $[M+H]^+$

$^1$H-NMR (400 MHz, DMSO-$d_6$) δ [ppm] = 11.63 (s, 1H), 8.15 (s, 1H), 7.70 (d, 1H), 7.25 (dd, 2H), 7.08 (t, 2H), 6.82 (s, 1H), 6.23 (d, 1H), 6.14 (dd, 1H), 5.78 (s, 2H), 2.78 (s, 2H), 1.27 (s, 6H).

**Intermediate 79**

3-(3-fluorophenyl)-6,6-dimethyl-1,5,6,7-tetrahydro-4H-pyrrolo[3,2-c]pyridin-4-one

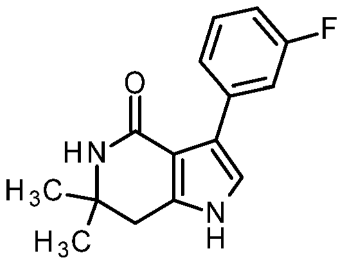

A screw cap vial with a pressure septum was loaded with 2-amino-1-(3-fluorophenyl)ethan-1-one-hydrogen chloride salt (CAS-RN:[93102-97-7], 1.57 g, 8.28 mmol), 6,6-dimethylpiperidine-2,4-dione (CAS-RN:[5239-39-4], 1.17 g, 8.28 mmol) and potassium acetate (1.62 g, 16.6 mmol). To the vial was added 16.6 mL of water. The reaction was heated at 90 °C for 1 h. The reaction mixture was cooled down to rt, filtered and the cake was rinsed with 10 mL of water. The crude product was purified by flash chromatography on silica gel (dichloromethane / methanol 0 to 15%). The title compound containing fractions were concentrated under reduced pressure to provide 896 mg of the title compound.

LC-MS (Method 4): $R_t$ = 2.03 min; MS (ESIpos): m/z = 259 $[M+H]^+$

$^1$H-NMR (400 MHz, DMSO-$d_6$) δ [ppm] = δ 11.38 (s, 1H), 7.84 (ddd, 1H), 7.61 (dt, 1H), 7.28 (td, 1H), 7.08 (d, 1H),

6.97 - 6.91 (m, 1H), 6.89 (s, 1H), 2.73 (s, 2H), 1.25 (s, 6H).

**Intermediate 80**

2-bromo-3-(3-fluorophenyl)-6,6-dimethyl-1,5,6,7-tetrahydro-4H-pyrrolo[3,2-c]pyridin-4-one

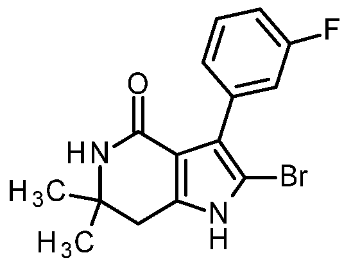

To a solution of 3-(3-fluorophenyl)-6,6-dimethyl-1,5,6,7-tetrahydro-4H-pyrrolo[3,2-c]pyridin-4-one (see Intermediate 79, 465 mg) in 3.60 mL of N,N-dimethylformamide was added 1-bromopyrrolidine-2,5-dione (320 mg, 1.80 mmol). The reaction was stirred at rt for 15 min. The reaction mixture was concentrated under vacuum. The crude product was triturated with 5.0 mL of cold methanol, filtered, dried under vacuum and the product was used without further purification: 275 mg.

LC-MS (Method 4): $R_t$ = 2.24 min; MS (ESIpos): m/z = 337 $[M+H]^+$

$^1$H-NMR (400 MHz, DMSO-$d_6$) $\delta$ [ppm] = 12.09 (s, 1H), 7.36 (td, 1H), 7.27 - 7.17 (m, 2H), 7.15 - 6.99 (m, 1H), 6.94 (s, 1H), 2.72 (s, 2H), 1.27 (s, 6H).

**Intermediate 81**

2-(2-aminopyridin-4-yl)-3-(3-fluorophenyl)-6,6-dimethyl-1,5,6,7-tetrahydro-4H-pyrrolo[3,2-c]pyridin-4-one

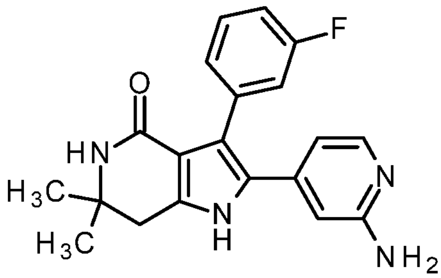

A screw cap vial with a pressure septum was loaded with 2-bromo-3-(3-fluorophenyl)-6,6-dimethyl-1,5,6,7-tetrahydro-4H-pyrrolo[3,2-c]pyridin-4-one (see Intermediate 80, 165 mg), 4-(4,4,5,5-tetramethyl-1,3,2-dioxaborolan-2-yl)pyridin-2-amine (215 mg, 979 μmol), $K_3PO_4$ (207 mg, 979 μmol), and XPhos Pd G3 (41.4 mg, 48.9 μmol). The vial was purged with Argon and filled with 2.45 mL of 1,4-dioxane / water (4:1). The reaction was added to a preheated hot plate at 80 °C and stirred for 14 h. The reaction was cooled down, filtered over a plug of silica gel, rinsed with methanol and N,N-dimethylformamide and concentrated under vacuum. The crude product was purified by preparative reverse phase HPLC (eluent: acetonitrile / water 1 to 100 % + 0.1 % formic acid). The title compound containing fractions were concentrated under reduced pressure to provide 50.1 mg of the title compound as a formic acid salt.

LC-MS (Method 4): $R_t$ = 1.31 min; MS (ESIpos): m/z = 351 $[M+H]^+$

$^1$H-NMR (400 MHz, DMSO-$d_6$) $\delta$ [ppm] = 11.73 (s, 1H), 8.13 (s, 1H), 7.71 (d, 1H), 7.27 (q, 1H), 7.11 - 6.94 (m, 3H), 6.87 (s, 1H), 6.28 (s, 1H), 6.22 - 6.14 (m, 1H), 5.96 (s, 2H), 2.79 (s, 2H), 1.27 (s, 6H).

**Intermediate 82**

3-(2-fluorophenyl)-6,6-dimethyl-1,5,6,7-tetrahydro-4H-pyrrolo[3,2-c]pyridin-4-one

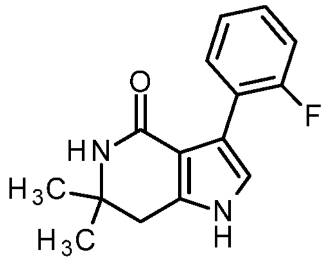

A screw cap vial with a pressure septum was loaded with 2-amino-1-(2-fluorophenyl)ethan-1-one-hydrogen chloride salt (CAS-RN:[93102-96-6], 920 mg, 4.85 mmol), 6,6-dimethylpiperidine-2,4-dione (CAS-RN:[5239-39-4], 685 mg, 4.85 mmol) and potassium acetate (951 mg, 9.70 mmol). To the vial was added 9.70 mL of water. The reaction was heated at 90 °C for 5 h. The reaction mixture was cooled down to rt, filtered and the cake was rinsed with 5 mL of water. The crude product was purified by flash chromatography on silica gel (n-hexane / acetone 15 to 50%). The title compound containing fractions were concentrated under reduced pressure to provide 890 mg of the title compound.

LC-MS (Method 4): $R_t$ = 1.91 min; MS (ESIpos): m/z = 259 $[M+H]^+$

$^1$H-NMR (400 MHz, DMSO-$d_6$) δ [ppm] = 11.32 (s, 1H), 7.67 (td, 1H), 7.21 (tdd, 1H), 7.14 - 7.02 (m, 2H), 6.83 (t, 1H), 6.78 (s, 1H), 2.74 (s, 2H), 1.26 (s, 6H).

**Intermediate 83**

2-bromo-3-(2-fluorophenyl)-6,6-dimethyl-1,5,6,7-tetrahydro-4H-pyrrolo[3,2-c]pyridin-4-one

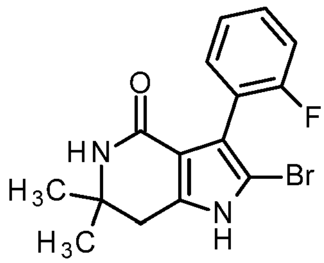

To a solution of 3-(2-fluorophenyl)-6,6-dimethyl-1,5,6,7-tetrahydro-4H-pyrrolo[3,2-c]pyridin-4-one (see Intermediate 82, 492 mg) in 4.76 mL of *N,N*-dimethylformamide was added 1-bromopyrrolidine-2,5-dione (339 mg, 1.90 mmol). The reaction was stirred at rt for 15 min. The reaction mixture was concentrated under vacuum. The crude product was triturated with 5.0 mL of cold methanol, filtered, dried under vacuum and the product was used without further purification: 470 mg.

LC-MS (Method 4): Rt = 2.13 min; MS (ESIpos): m/z = 337 $[M+H]^+$

$^1$H-NMR (400 MHz, DMSO-$d_6$) δ [ppm] = 12.06 (s, 1H), 7.36 - 7.23 (m, 2H), 7.21 - 7.07 (m, 2H), 6.86 (s, 1H), 2.72 (s, 2H), 1.25 (s, 6H).

**Intermediate 84**

2-(2-aminopyridin-4-yl)-3-(2-fluorophenyl)-6,6-dimethyl-1,5,6,7-tetrahydro-4H-pyrrolo[3,2-c]pyridin-4-one

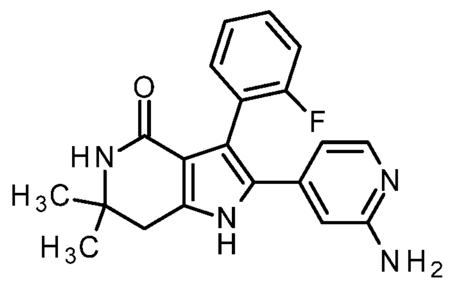

A screw cap vial with a pressure septum was loaded with 2-bromo-3-(2-fluorophenyl)-6,6-dimethyl-1,5,6,7-tetrahydro-4H-pyrrolo[3,2-c]pyridin-4-one (see Intermediate 83, 468 mg), 4-(4,4,5,5-tetramethyl-1,3,2-dioxaborolan-2-yl)pyridin-2-amine (611 mg, 2.78 mmol), $K_3PO_4$ (736 mg, 3.47 mmol), and XPhos Pd G3 (117 mg, 139 μmol). The vial was purged with argon and filled with 6.94 mL of 1,4-dioxane / water (4:1). The reaction was added to a preheated hot plate at 80 °C and stirred for 14 h. The reaction was cooled down, filtered over a plug of silica gel, rinsed with methanol and *N*,*N*-dimethylformamide and concentrated under vacuum. The crude product was purified by preparative reverse phase HPLC (eluent: acetonitrile / water 1 to 100 % + 0.1% formic acid). The title compound containing fractions were concentrated under reduced pressure to provide 170 mg of the title compound as a formic acid salt.

LC-MS (Method 4): $R_t$ = 1.31 min; MS (ESIpos): m/z = 351 $[M+H]^+$

$^1$H-NMR (400 MHz, DMSO-$d_6$) δ [ppm] = 11.72 (s, 1H), 8.14 (s, 1H), 7.67 (d, 1H), 7.32 (tdd, 1H), 7.20 (td, 1H), 7.16 - 7.08 (m, 2H), 6.80 (s, 1H), 6.26 (t, 1H), 6.12 (dd, 1H), 5.77 (s, 2H), 2.80 (s, 2H), 1.27 (s, 6H).

**Intermediate 85**

6,6-dimethyl-3-(3-methylphenyl)-1,5,6,7-tetrahydro-4H-pyrrolo[3,2-c]pyridin-4-one

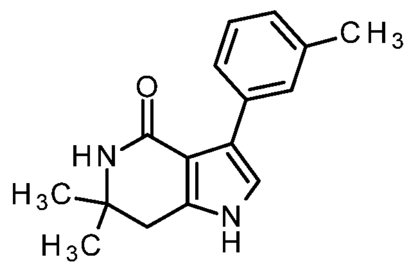

A screw cap vial with a pressure septum was loaded with 2-amino-1-(3-methylphenyl)ethan-1-one-hydrogen chloride salt (CAS-RN:[70785-83-0], 507 mg, 2.73 mmol), 6,6-dimethylpiperidine-2,4-dione (CAS-RN:[5239-39-4], 386 mg, 2.73mmol) and potassium acetate (535 mg, 5.46 mmol). To the vial was added 5.46 mL of water. The reaction was heated at 90 °C for 3 h. The reaction mixture was cooled down to rt, filtered and the cake was rinsed with 10 mL of water. The product was dried under reduce pressure, yielding 670 mg of the title compound.

LC-MS (Method 4): $R_t$ = 2.10 min; MS (ESIpos): m/z = 255 $[M+H]^+$

$^1$H-NMR (400 MHz, DMSO-$d_6$) δ [ppm] = δ 11.24 (s, 1H), 7.73 - 7.46 (m, 2H), 7.13 (t, 1H), 6.95 (dt, 1H), 6.88 (d, 1H), 6.75 (s, 1H), 2.72 (s, 2H), 2.28 (s, 3H), 1.25 (s, 6H).

**Intermediate 86**

2-bromo-6,6-dimethyl-3-(3-methylphenyl)-1,5,6,7-tetrahydro-4H-pyrrolo[3,2-c]pyridin-4-one

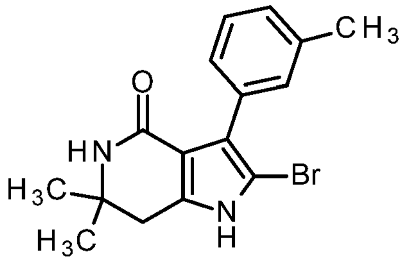

To a solution of 6,6-dimethyl-3-(3-methylphenyl)-1,5,6,7-tetrahydro-4H-pyrrolo[3,2-c]pyridin-4-one (see Intermediate 85, 620 mg) in 6.09 mL of N,N-dimethylformamide was added 1 bromopyrrolidine-2,5-dione (464 mg, 2.39 mmol). The reaction was stirred at rt for 15 min. Then reaction mixture was concentrated under vacuum. The crude product was triturated with 5.0 mL of cold methanol, filtered, dried under vacuum and the product was used without further purification: 326 mg.

LC-MS (Method 4): $R_t$ = 2.32 min; MS (ESIpos): m/z = 333 $[M+H]^+$

$^1$H-NMR (400 MHz, DMSO-$d_6$) $\delta$ [ppm] = 11.96 (s, 1H), 7.19 (td, 3H), 7.05 (d, 1H), 6.83 (s, 1H), 2.71 (s, 2H), 2.31 (s, 3H), 1.26 (s, 6H).

**Intermediate 87**

2-(2-aminopyridin-4-yl)-6,6-dimethyl-3-(3-methylphenyl)-1,5,6,7-tetrahydro-4H-pyrrolo[3,2-c]pyridin-4-one

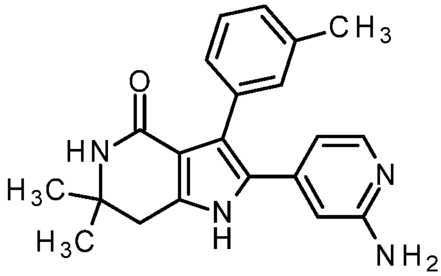

A screw cap vial with a pressure septum was loaded with 2-bromo-6,6-dimethyl-3-(3-methylphenyl)-1,5,6,7-tetrahydro-4H-pyrrolo[3,2-c]pyridin-4-one (see Intermediate 86, 450 mg), 4-(4,4,5,5-tetramethyl-1,3,2-dioxaborolan-2-yl)pyridin-2-amine (744 mg, 3.38 mmol), $K_3PO_4$ (716 mg, 3.38 mmol), and XPhos Pd G3 (105 mg, 135 $\mu$mol). The vial was purged with Argon and filled with 6.76 mL of 1,4-dioxane / water (4:1). The reaction was added to a preheated hot plate at 90 °C and heated for 16 h. The reaction was cooled down and concentrated under vacuum. The crude product was purified by preparative reverse phase HPLC (eluent: acetonitrile / water 0 to 100% + 0.1 % formic acid). The title compound containing fractions were concentrated under reduced pressure to provide 180 mg of the title compound as a formic acid salt.

LC-MS (Method 4): $R_t$ = 1.39 min; MS (ESIpos): m/z = 347 $[M+H]^+$

$^1$H-NMR (400 MHz, DMSO-$d_6$) $\delta$ [ppm] = $\delta$ 11.60 (s, 1H), 8.13 (s, 1H), 7.66 (d, 1H), 7.15 (t, 1H), 7.10 - 6.97 (m, 3H), 6.76 (s, 1H), 6.30 (d, 1H), 6.10 (dd, 1H), 5.78 (s, 2H), 2.79 (s, 2H), 2.26 (s, 3H), 1.28 (s, 6H).

**Intermediate 88**

3-(4-chlorophenyl)-6,6-dimethyl-1,5,6,7-tetrahydro-4H-pyrrolo[3,2-c]pyridin-4-one

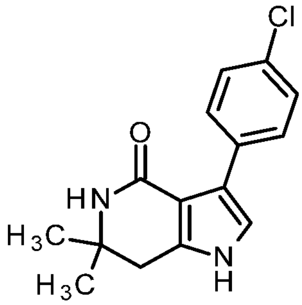

A screw cap vial with a pressure septum was loaded with 2-amino-1-(4-chlorophenyl)ethan-1-one-hydrogen chloride salt (CAS-RN:[5467-71-0], 1.00 g, 4.85 mmol), 6,6-dimethylpiperidine-2,4-dione (CAS-RN:[5239-39-4], 685 mg, 4.85 mmol) and potassium acetate (951 mg, 9.71 mmol). To the vial was added 9.71 mL of water. The reaction was heated at 90 °C for 14 h. The reaction mixture was cooled down to rt, filtered and the cake was rinsed with 10 mL of water. The product was dried under reduce pressure, yielding 690 mg of the title compound.

LC-MS (Method 3): $R_t$ = 1.35 min; MS (ESIpos): m/z = 275 $[M+H]^+$

$^1$H-NMR (400 MHz, DMSO-$d_6$) $\delta$ [ppm] = $\delta$ 11.35 (s, 1H), 7.85 (d, 2H), 7.31 (d, 2H), 7.00 (d, 1H), 6.87 (s, 1H), 2.73 (s, 2H), 1.26 (s, 6H).

**Intermediate 89**

2-bromo-3-(4-chlorophenyl)-6,6-dimethyl-1,5,6,7-tetrahydro-4H-pyrrolo[3,2-c]pyridin-4-one

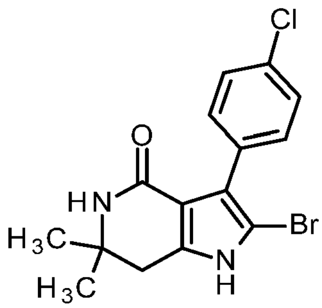

To a solution of 3-(4-chlorophenyl)-6,6-dimethyl-1,5,6,7-tetrahydro-4H-pyrrolo[3,2-c]pyridin-4-one (see Intermediate 88, 675 mg) in 12.2 mL of N,N-dimethylformamide was added 1-bromopyrrolidine-2,5-dione (437 mg, 2.46 mmol). The reaction was stirred at rt for 1 h. Then the reaction mixture was concentrated under vacuum. The crude product was triturated with 10.0 mL of cold methanol, filtered, dried under vacuum and the product was used without further purification: 710 mg.

LC-MS (Method 3): Rt = 1.42 min; MS (ESIpos): m/z = 353 $[M+H]^+$

$^1$H-NMR (400 MHz, DMSO-$d_6$) $\delta$ [ppm] = 12.07 (s, 1H), 7.43 (d, 2H), 7.41 - 7.34 (m, 2H), 6.94 (s, 1H), 2.72 (s, 2H), 1.26 (s, 6H).

**Intermediate 90**

2-(2-aminopyridin-4-yl)-3-(4-chlorophenyl)-6,6-dimethyl-1,5,6,7-tetrahydro-4H-pyrrolo[3,2-c]pyridin-4-one

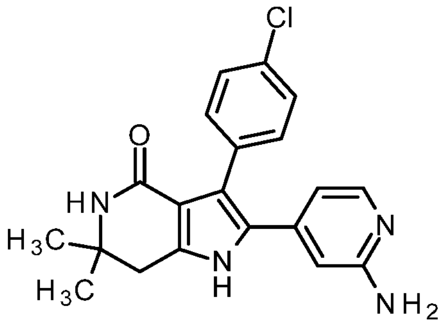

A screw cap vial with a pressure septum was loaded with 2-bromo-3-(4-chlorophenyl)-6,6-dimethyl-1,5,6,7-tetrahydro-4H-pyrrolo[3,2-c]pyridin-4-one (see Intermediate 89, 677 mg), 4-(4,4,5,5-tetramethyl-1,3,2-dioxaborolan-2-yl)pyridin-2-amine (548 mg, 2.49 mmol), $K_3PO_4$ (1.22 g, 5.74 mmol), and XPhos Pd G3 (162 mg, 191 μmol). The vial was purged with argon and filled with 6.38 mL of 1,4-dioxane / water (4:1). The reaction was added to a preheated hot plate at 90 °C and heated for 16 h. The reaction was cooled down and concentrated under vacuum. The crude product was purified by preparative reverse phase HPLC (eluent: acetonitrile / water 0 to 100 % + 0.1 % formic acid). The title compound containing fractions were concentrated under reduced pressure to provide 253 mg of the title compound as a formic acid salt.

LC-MS (Method 4): $R_t$ = 1.47 min; MS (ESIpos): m/z = 367 $[M+H]^+$

$^1$H-NMR (400 MHz, DMSO-$d_6$) δ [ppm] = 11.67 (s, 1H), 8.14 (s, 1H), 7.72 (d, 1H), 7.33 - 7.28 (m, 2H), 7.27 - 7.22 (m, 2H), 6.85 (s, 1H), 6.25 (d, 1H), 6.15 (dd, 1H), 5.81 (s, 2H), 2.78 (s, 2H), 1.27 (s, 6H).

**Intermediate 91**

6,6-dimethyl-3-(4-methylphenyl)-1,5,6,7-tetrahydro-4H-pyrrolo[3,2-c]pyridin-4-one

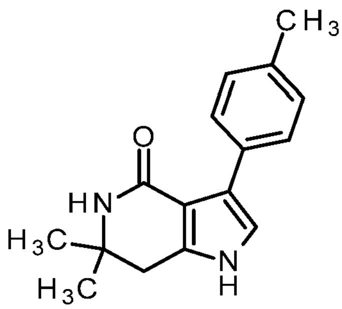

A screw cap vial with a pressure septum was loaded with 2-amino-1-(4-methylphenyl)ethan-1-one-hydrogen chloride salt (CAS-RN:[5467-70-9], 1.00 g, 5.39 mmol), 6,6-dimethylpiperidine-2,4-dione (CAS-RN:[5239-39-4], 760 mg, 5.39 mmol) and potassium acetate (1.01 g, 10.8 mmol). To the vial was added 10.7 mL of water. The reaction was heated at 90 °C for 14 h. The reaction was cooled down to rt, filtered and the cake was rinsed with 10 mL of water. The reaction mixture was concentrated, and the crude product was purified by flash chromatography on silica gel (dichloromethane / methanol 2% to 12%), yielding 650 mg of the title compound.

LC-MS (Method 3): $R_t$ = 1.32 min; MS (ESIpos): m/z = 255 $[M+H]^+$

$^1$H-NMR (400 MHz, DMSO-$d_6$) δ [ppm] = 11.21 (s, 1H), 7.65 (d, 2H), 7.06 (d, 2H), 6.86 (d, 1H), 6.76 (s, 1H), 2.71 (s, 2H), 2.28 (s, 3H), 1.24 (s, 6H).

**Intermediate 92**

2-bromo-6,6-dimethyl-3-(4-methylphenyl)-1,5,6,7-tetrahydro-4H-pyrrolo[3,2-c]pyridin-4-one

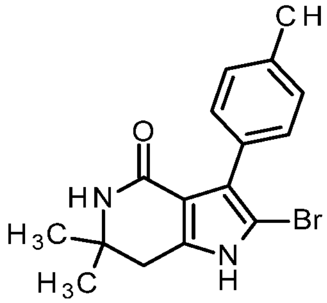

To a solution o 6,6-dimethyl-3-(4-methylphenyl)-1,5,6,7-tetrahydro-4H-pyrrolo[3,2-c]pyridin-4-one (see Intermediate 91, 606 mg) in 11.9 mL of N,N-dimethylformamide was added 1-bromopyrrolidine-2,5-dione (424 mg, 2.38 mmol). The reaction was stirred at rt for 1 h. Then the reaction mixture was concentrated under vacuum. The crude product was triturated with 10 mL of cold methanol, filtered, dried under vacuum and the product was used without further purification: 306 mg.

LC-MS (Method 3): Rt = 1.38 min; MS (ESIpos): m/z = 333 $[M+H]^+$

$^1$H-NMR (400 MHz, DMSO-$d_6$) δ [ppm] = 11.93 (s, 1H), 7.46 - 7.17 (m, 2H), 7.11 (d, 2H), 6.85 (s, 1H), 2.70 (s, 2H), 2.31 (s, 3H), 1.25 (s, 6H).

**Intermediate 93**

2-(2-aminopyridin-4-yl)-6,6-dimethyl-3-(4-methylphenyl)-1,5,6,7-tetrahydro-4H-pyrrolo[3,2-c]pyridin-4-one

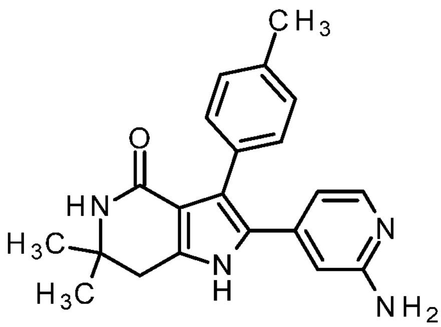

A screw cap vial with a pressure septum was loaded with 4-(4,4,5,5-tetramethyl-1,3,2-dioxaborolan-2-yl)pyridin-2-amine (533 mg, 2.42 mmol), 2-bromo-6,6-dimethyl-3-(4-methylphenyl)-1,5,6,7-tetrahydro-4H-pyrrolo[3,2-c]pyridin-4-one (see Intermediate 92, 269 mg), $K_3PO_4$ (513 mg, 2.42 mmol), and XPhos Pd G3 (68.3 mg, 80.7 μmol). The vial was purged with argon and filled 4.04 mL of 1,4-dioxane:water (4:1). The reaction was added to a preheated hot plate at 90 °C and heated for 16h. The reaction was cooled down and concentrated under vacuum. The crude product was purified by preparative reverse phase HPLC (eluent: acetonitrile / water 0 to 100% + 0.1 % formic acid). The title compound containing fractions were concentrated under reduced pressure to provide 215 mg of the title compound as a formic acid salt.

LC-MS (Method 4): $R_t$ = 1.46 min; MS (ESIpos): m/z = 347 $[M+H]^+$

$^1$H-NMR (400 MHz, DMSO-$d_6$) $\delta$ [ppm] = 11.57 (s, 1H), 8.13 (s, 1H), 7.66 (d, 1H), 7.12 (d, 2H), 7.05 (d, 2H), 6.78 (s, 1H), 6.30 (d, 1H), 6.12 (dd, 1H), 5.81 (s, 2H), 2.77 (s, 2H), 2.31 (s, 3H), 1.27 (s, 6H).

**Intermediate 94**

2-(2-chloropyridin-4-yl)-6,6-dimethyl-1,5,6,7-tetrahydro-4H-pyrrolo[3,2-c]pyridin-4-one

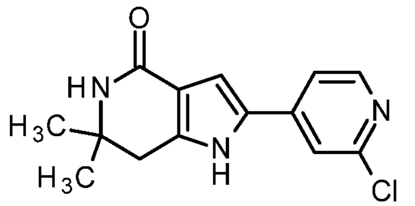

To a round bottom flask charged with 6,6-dimethylpiperidine-2,4-dione (CAS-RN:[5239-39-4], 2.65 g, 18.8 mmol), ammonium acetate (5.25 g, 68.2 mmol) was added 52 mL of ethanol. To the solution was added 2-bromo-1-(2-chloro-4-pyridyl)ethanone (CAS-RN: [23794-16-3], 4.00 g, 17.1 mmol). The reaction was stirred at rt for 90 min. To the reaction was added 120 mL of water. The mixture was stirred for 2 h at rt, cooled down to 0 °C for 30 min, filtered and rinsed with water, yielding 3.91 g.of the title compound as a green solid.

LC-MS (Method 4): $R_t$ = 1.74 min; MS (ESIpos): m/z = 276 $[M+H]^+$

$^1$H-NMR (400 MHz, DMSO-$d_6$) $\delta$ [ppm] = 11.97 (s, 1H), 8.28 (d, 1H), 7.74 (s, 1H), 7.68 - 7.58 (m, 1H), 7.13 (t, 1H), 7.07 (s, 1H), 2.80 (s, 2H), 1.26 (s, 6H).

**Intermediate 95**

N-[4-(6,6-dimethyl-4-oxo-4,5,6,7-tetrahydro-1H-pyrrolo[3,2-c]pyridin-2-yl)pyridin-2-yl]-2-(4-fluorophenyl)acetamide

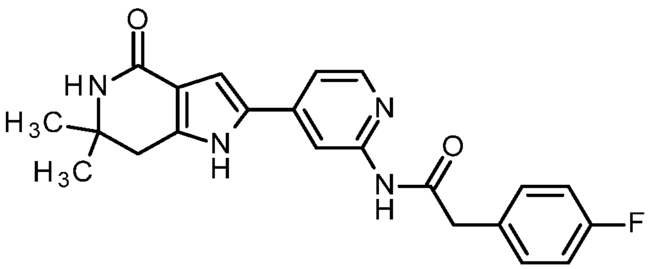

To a microwave vial is added 2-(2-chloropyridin-4-yl)-6,6-dimethyl-1,5,6,7-tetrahydro-4H-pyrrolo[3,2-c]pyridin-4-one (see Intermediate 94, 800 mg), 2-(4-fluorophenyl)acetamide (889 mg, 5.80 mmol), Xantphos (671 mg, 1.16 mmol), $K_2CO_3$ (1.60 g, 11.6 mmol) and $Pd(OAc)_2$ (65.0 mg, 290 μmol). The microwave vial was sealed and degassed with argon. The tube was filled with 14.5 mL of tetrahydrofuran, sealed and heated at 90 °C on the microwave for 16 h. The reaction was cooled down to rt and concentrated under vacuum. The crude product was purified by flash chromatography on silica gel (dichloromethane / methanol 2 to 13%). The title compound containing fractions were concentrated under reduced pressure to provide 1.61 g of the title compound.

LC-MS (Method 3): $R_t$ = 1.23 min; MS (ESIpos): m/z = 393 $[M+H]^+$

$^1$H-NMR (400 MHz, DMSO-$d_6$) $\delta$ [ppm] = 11.88 (s, 1H), 10.64 (s, 1H), 8.23 (d, 2H), 7.45 - 7.36 (m, 2H), 7.33 (d, 1H), 7.16 (td, 2H), 6.97 (s, 1H), 6.82 (d, 1H), 3.74 (s, 2H), 2.77 (s, 2H), 1.25 (d, 6H).

**Intermediate 96**

N-[4-(3-bromo-6,6-dimethyl-4-oxo-4,5,6,7-tetrahydro-1H-pyrrolo[3,2-c]pyridin-2-yl)pyridin-2-yl]-2-(4-fluorophenyl)acetamide

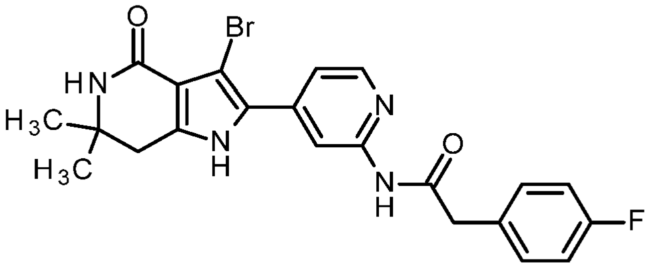

To a solution of N-[4-(6,6-dimethyl-4-oxo-4,5,6,7-tetrahydro-1H-pyrrolo[3,2-c]pyridin-2-yl)pyridin-2-yl]-2-(4-fluorophenyl)acetamide (see Intermediate 95, 228 mg) in 11.6 mL of N,N-dimethylformamide at 0° C was added 1-bromopyrrolidine-2,5-dione (107 mg, 598 μmol). The reaction was allowed to warm up to rt and stirred for 2 h. The reaction was concentrated under vacuum. The crude product was purified by flash chromatography on silica gel (dichloromethane / methanol 2 to 15%). The title compound containing fractions were concentrated under reduced pressure to provide 190 mg of the title compound.

LC-MS (Method 4): $R_t$= 2.12 min; MS (ESIpos): m/z = 471 $[M+H]^+$

$^1$H-NMR (400 MHz, DMSO-$d_6$) δ [ppm] = 12.11 (s, 1H), 10.75 (s, 1H), 8.54 (d, 1H), 8.35 (d, 1H), 7.46 - 7.34 (m, 3H), 7.24 - 7.06 (m, 3H), 3.75 (s, 2H), 2.79 (s, 2H), 1.25 (s, 6H).

**Intermediate 97**

N-[4-(6,6-dimethyl-4-oxo-4,5,6,7-tetrahydro-1H-pyrrolo[3,2-c]pyridin-2-yl)pyridin-2-yl]-2-(4-fluorophenyl)propanamide (Racemate)

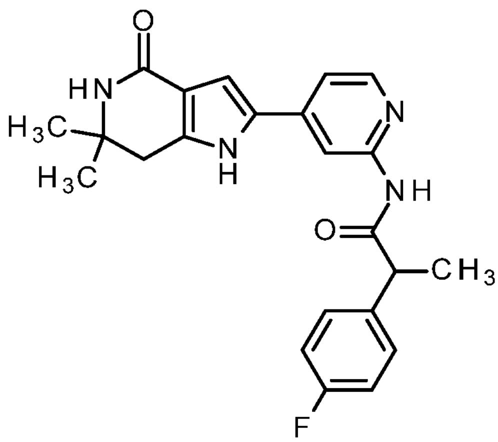

To a microwave vial is added 2-(2-chloropyridin-4-yl)-6,6-dimethyl-1,5,6,7-tetrahydro-4H-pyrrolo[3,2-c]pyridin-4-one (see Intermediate 94, 1.80 g), 2-(4-fluorophenyl)propanamide (1.09 g, 6.53 mmol), Xantphos (1.13 g, 1.96 mmol), $K_2CO_3$ (3.60 g, 26.1 mmol) and $Pd(OAc)_2$ (219 mg, 979 μmol). The microwave vial was sealed and degassed with argon. The tube was filled with 26.1 mL of tetrahydrofuran, sealed and heated at 90 °C on the microwave for 16h. The reaction was cooled down to rt and concentrated under vacuum. The crude product was purified by flash chromatography on silica gel (dichloromethane / methanol 2 to 13 %). The title compound containing fractions were concentrated under reduced pressure to provide 1.61 g of the title compound.

LC-MS (Method 3): $R_t$ = 1.27 min; MS (ESIpos): m/z = 407 $[M+H]^+$

$^1$H-NMR (400 MHz, DMSO-$d_6$) δ [ppm] = 11.89 (d, 1H), 10.59 (s, 1H), 8.26 - 8.21 (m, 1H), 8.20 (d, 1H), 7.56 - 7.42 (m, 2H), 7.32 (dd, 1H), 7.16 (t, 2H), 6.98 (s, 1H), 6.84 (d, 1H), 4.05 (q, 1H), 2.78 (s, 2H), 1.42 (d, 3H), 1.26 (s, 6H).

**Intermediate 98**

N-[4-(3-bromo-6,6-dimethyl-4-oxo-4,5,6,7-tetrahydro-1H-pyrrolo[3,2-c]pyridin-2-yl)pyridin-2-yl]-2-(4-fluorophenyl)propanamide (Racemate)

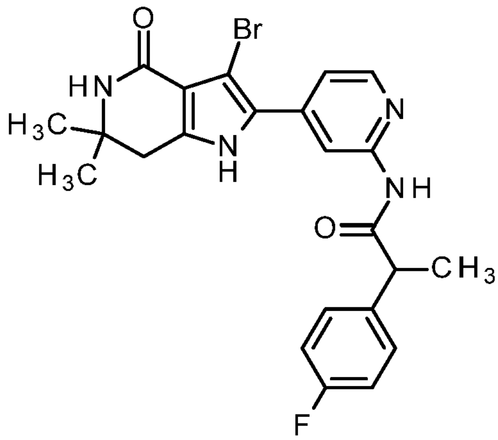

To a solution of N-[4-(6,6-dimethyl-4-oxo-4,5,6,7-tetrahydro-1H-pyrrolo[3,2-c]pyridin-2-yl)pyridin-2-yl]-2-(4-fluorophenyl)propanamide (Racemate) (see Intermediate 97, 700 mg) in 35.7 mL of N,N-dimethylformamide at 0° C was added 1-bromopyrrolidine-2,5-dione (316 mg, 1.77 mmol). The reaction was allowed to warm up to rt and stirred for 2 h. The reaction was concentrated under vacuum. The crude product was purified by flash chromatography on silica gel (dichloromethane / methanol 2 to 15%). The title compound containing fractions were concentrated under reduced pressure to provide 658 mg of the title compound.

LC-MS (Method 3): Rt = 1.36 min; MS (ESIpos): m/z = 485 $[M+H]^+$

$^1$H-NMR (400 MHz, DMSO-$d_6$) δ [ppm] = 12.12 (s, 1H), 10.70 (s, 1H), 8.61 - 8.44 (m, 1H), 8.32 (d, 1H), 7.55 - 7.41 (m, 3H), 7.22 - 7.07 (m, 3H), 4.07 (q, 1H), 2.57 (s, 2H), 1.43 (d, 3H), 1.26 (s, 6H).

**Intermediate 99**

2-(2-chloropyridin-4-yl)-5-methyl-1,5,6,7-tetrahydro-4H-pyrrolo[3,2-c]pyridin-4-one

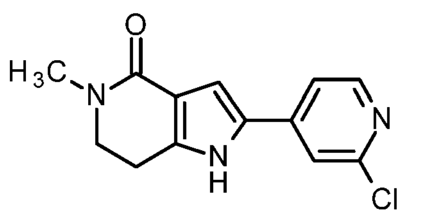

To a round bottom flask charged with 1-methylpiperidine-2,4-dione (CAS-RN:[118263-97-1], 3.80 g, 29.9 mmol), ammonium acetate (9.19 g, 119 mmol) was added 90.5 mL of ethanol. To the solution was added 2-bromo-1-(2-chloro-4-pyridyl)ethanone (CAS-RN: [23794-16-3], 7.00 g, 29.9 mmol). The reaction was stirred at rt for 2 h. To the reaction was added 90 mL of water and the mixture was cooled down to 0 °C, stirred for 1 h, filtered and rinsed with water, yielding the desired product as a grey solid. The mother liquor was concentrated under vacuum. The residual crude product was

purified by flash chromatography on silica gel (dichloromethane / methanol 2 to 15 %). The title compound containing fractions were concentrated under reduced pressure to provide 4.01 g of the title compound.

LC-MS (Method 3): $R_t$ = 1.15 min; MS (ESIpos): m/z = 262 $[M+H]^+$

$^1$H-NMR (400 MHz, DMSO-$d_6$) $\delta$ [ppm] = 11.97 (s, 1H), 8.28 (d, 1H), 7.74 (d, 1H), 7.63 (dd, 1H), 7.14 (d, 1H), 3.56 (t, 2H), 2.99 - 2.87 (m, 5H).

**Intermediate 100**

2-(4-fluorophenyl)-N-[4-(5-methyl-4-oxo-4,5,6,7-tetrahydro-1H-pyrrolo[3,2-c]pyridin-2-yl)pyridin-2-yl]propanamide (Racemate)

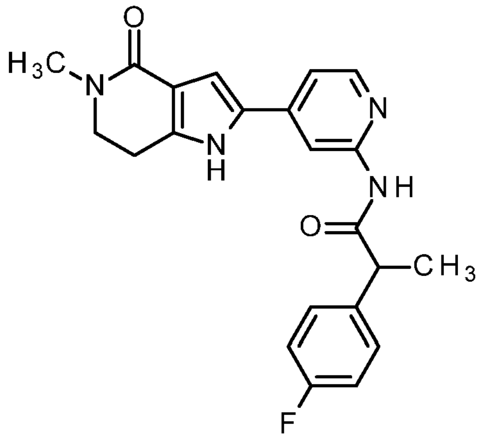

A screw cap vial with a pressure septum was loaded with 2-(2-chloropyridin-4-yl)-5-methyl-1,5,6,7-tetrahydro-4H-pyrrolo[3,2-c]pyridin-4-one (see Intermediate 99, 1.25 g), 2-(4-fluorophenyl)-propanamide (Racemate) (958 mg, 5.73 mmol), $K_2CO_3$ (2.64 g, 19.1 mmol), XantPhos (1.10 g, 1.91 mmol) and $Pd(OAc)_2$ (160 mg, 716 $\mu$mol). The vial was degassed with argon and filled with 23.9 mL of 1,4-dioxane. The reaction was heated to 100 °C. After 16 h, the reaction was cooled down and concentrated under vacuum. The crude product was purified by flash chromatography on silica gel (dichloromethane / methanol 0 to 14 %). The title compound containing fractions were concentrated under reduced pressure to provide 830 mg of the title compound.

LC-MS (Method 4): $R_t$ = 2.04 min; MS (ESIpos): m/z = 393 $[M+H]^+$

$^1$H-NMR (400 MHz, DMSO-$d_6$) $\delta$ [ppm] = 11.93 - 11.88 (m, 1H), 10.58 (s, 1H), 8.24 (d, 1H), 8.20 (d, 1H), 7.50 - 7.41 (m, 2H), 7.32 (dd, 1.6 Hz, 1H), 7.21 - 7.10 (m, 2H), 6.84 (d, 1H), 4.05 (q, 1H), 3.54 (t, 2H), 2.96 - 2.83 (m, 5H), 1.42 (d, 3H).

**Intermediate 101**

N-[4-(3-bromo-5-methyl-4-oxo-4,5,6,7-tetrahydro-1H-pyrrolo[3,2-c]pyridin-2-yl)pyridin-2-yl]-2-(4-fluorophenyl)propanamide (Racemate)

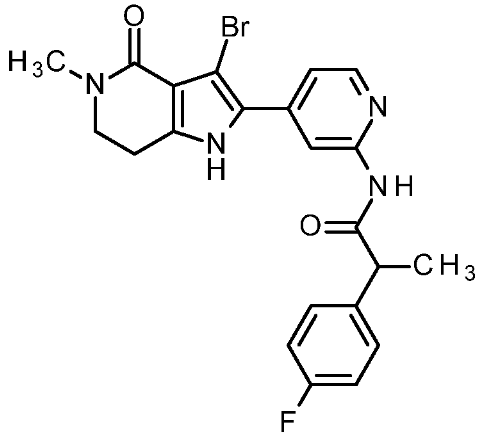

To a solution of 2-(4-fluorophenyl)-N-[4-(5-methyl-4-oxo-4,5,6,7-tetrahydro-1H-pyrrolo[3,2-c]pyridin-2-yl)pyridin-2-yl]propanamide (Racemate) (see Intermediate 100, 3.40 g) in 34.7 mL of N,N-dimethylformamide at 0° C was added 1-bromopyrrolidine-2,5-dione (1.54 g, 8.66 mmol). The reaction was allowed to warm up to rt and stirred for 30 min. The reaction mixture was diluted with a saturated aqueous solution of sodium thiosulfate. The solution was extracted 3 times with ethyl acetate. The organic layers were dried over MgSO4 and concentrated under vacuum. The crude product was purified by flash chromatography on silica gel (dichloromethane / methanol 1 to 13%). The title compound containing fractions were concentrated under reduced pressure to provide 3.85 g of the title compound.

LC-MS (Method 4): $R_t$ = 2.28 min; MS (ESIpos): m/z = 471 $[M+H]^+$

$^1$H-NMR (400 MHz, DMSO-$d_6$) $\delta$ [ppm] = 12.13 (s, 1H), 10.68 (s, 1H), 8.54 (s, 1H), 8.33 (d1H), 7.61 - 7.39 (m, 3H), 7.17 (t, 2H), 4.07 (q, 1H), 3.54 (t, 2H), 2.97 - 2.85 (m, 5H), 1.43 (d, 3H).

## Intermediate 102

N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate)

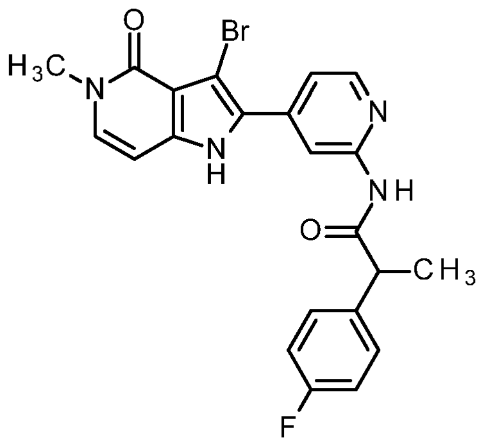

To a solution of N-[4-(3-bromo-5-methyl-4-oxo-4,5,6,7-tetrahydro-1H-pyrrolo[3,2-c]pyridin-2-yl)pyridin-2-yl]-2-(4-fluorophenyl)propanamide (Racemate) (see Intermediate 101, 950 mg) in 17.1 mL 1,4-dioxane was added 2,3-Dichloro-5,6-dicyano-1,4-benzoquinone (1.17 g, 5.14 mmol) and the mixture was heated at 90 °C for 45 min. The reaction was cooled down to rt, diluted with cold 0.5 M NaOH and extracted 3 times with ethyl acetate. The organic layers were combined, dried over $MgSO_4$ and concentrated under vacuum. The crude product was purified by flash chromatography on silica gel (dichloromethane / acetone 10 to 45%). The title compound containing fractions were concentrated under reduced pressure to provide 359 mg of the title compound.

LC-MS (Method 4): $R_t$ = 2.33 min; MS (ESIpos): m/z = 469 $[M+H]^+$

$^1$H-NMR (400 MHz, DMSO-$d_6$) δ [ppm] = 12.27 (s, 1H), 10.77 (s, 1H), 8.62 (d, 1H), 8.40 (d, 1H), 7.55 - 7.44 (m, 3H), 7.42 (d, 1H), 7.17 (t, 2H), 6.42 (d, 1H), 4.09 (q, 1H), 3.44 (s, 3H), 1.44 (d, 3H).

## Intermediate 103

4,4-difluoro-2-(4-fluorophenyl)-N-[4-(5-methyl-4-oxo-4,5,6,7-tetrahydro-1H-pyrrolo[3,2-c]-pyridin-2-yl)pyridin-2-yl]butanamide (Racemate)

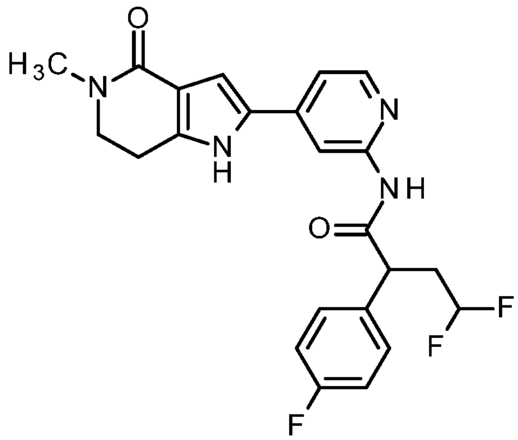

A screw cap vial with a pressure septum was loaded with 2-(2-chloropyridin-4-yl)-5-methyl-1,5,6,7-tetrahydro-4H-pyrrolo[3,2-c]pyridin-4-one (see Intermediate 99, 650 mg), 4,4-difluoro-2-(4-fluorophenyl)butanamide (Racemate) (701 mg, 3.23 mmol), $Cs_2CO_3$ (1.62 g, 4.97 mmol), *t*-BuBrettPhos-gen 3 (318 mg, 373 μmol) and *t*-BuBrett Phos (180 mg, 373 μmol). The vial was degassed with argon and filled with 12.4 mL of 1,4-dioxane. The reaction was heated to100 °C. After 1 h, the reaction was cooled down and concentrated under vacuum. The crude product was purified by flash chromatography on silica gel (dichloromethane / methanol 2 to 12 %). The title compound containing fractions were concentrated under reduced pressure to provide 720 mg of the title compound.

LC-MS (Method 4): $R_t$ = 2.22 min; MS (ESIpos): m/z = 443 $[M+H]^+$

$^1$H-NMR (400 MHz, DMSO-$d_6$) δ [ppm] = 11.91 (s, 1H), 10.74 (s, 1H), 8.30 - 8.01 (m, 2H), 7.50 (dd, 2H), 7.33 (dd, 1H), 7.19 (t, 2H), 6.84 (d, 1H), 6.03 (tt, 1H), 4.21 (t, 1H), 3.54 (t, 2H), 2.91 (d, 5H), 2.84 - 2.63 (m, 1H), 2.35 - 2.16 (m, 1H).

## Intermediate 104

N-[4-(3-bromo-5-methyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate)

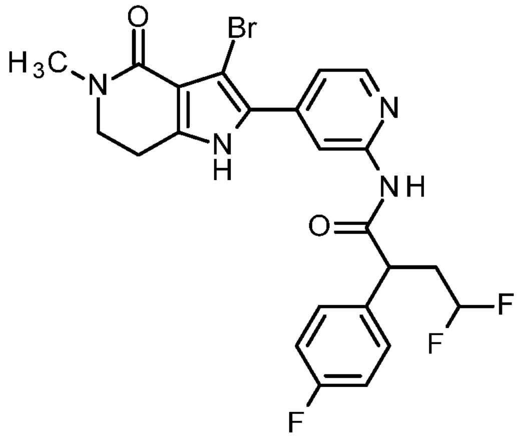

To a solution of 4,4-difluoro-2-(4-fluorophenyl)-N-[4-(5-methyl-4-oxo-4,5,6,7-tetrahydro-1H-pyrrolo[3,2-c]pyridin-2-yl)pyridin-2-yl]butanamide (Racemate) (see Intermediate 103, 1.57 g) in 14.2 mL of N,N-dimethylformamide at 0° C was added 1-bromopyrrolidine-2,5-dione (632 mg, 3.55 mmol). The reaction was allowed to warm up to rt and stirred for 30 min. The reaction mixture was diluted with a saturated aqueous solution of sodium thiosulfate. The solution was extracted 3 times with ethyl acetate. The organic layers were dried over MgSO4 and concentrated under vacuum. The crude product was purified by flash chromatography on silica gel (dichloromethane / methanol 1 to 13%). The title compound containing fractions were concentrated under reduced pressure to provide 1.65 g of the title compound.

LC-MS (Method 3): $R_t$ = 1.39 min; MS (ESIpos): m/z = 521 $[M+H]^+$
$^1$H-NMR (400 MHz, DMSO-$d_6$) $\delta$ [ppm] = 12.14 (s, 1H), 10.84 (s, 1H), 8.51 (s, 1H), 8.33 (d, 1H), 7.51 (dd, 2H), 7.42 (dd, 1H), 7.20 (t, 2H), 6.03 (tt, 1H), 4.23 (dd, 1H), 3.54 (t, 2H), 2.92 (d, 5H), 2.88 - 2.60 (m, 1H), 2.37 - 2.16 (m, 1H).

**Intermediate 105**

N-[4-(3-bromo-5-methyl-4-oxo-4,5-dihydro-1H-pyrrolo[3,2-c]pyridin-2-yl)pyridin-2-yl]-4,4-difluoro-2-(4-fluorophenyl)butanamide (Racemate)

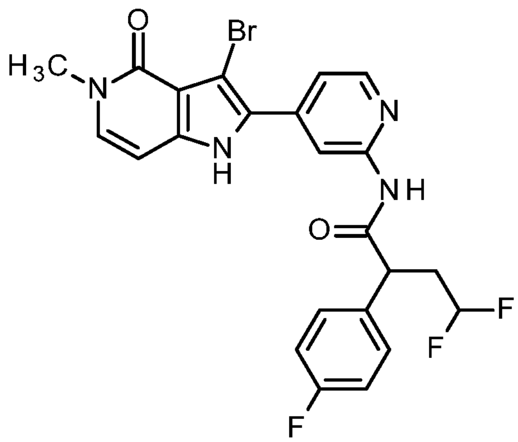

To a solution of N-[4-(3-bromo-5-methyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate) (see Intermediate 104, 350 mg) in 3.36 mL of 1,4-dioxane was added 2,3-Dichloro-5,6-dicyano-1,4-benzoquinone (229 mg, 1.01 mmol) and the mixture was heated at 80 °C for 90 min. The reaction was cooled down to rt, diluted with cold 0.5 M NaOH and extracted 3 times with ethyl acetate. The organic layers were combined, dried over $MgSO_4$ and concentrated under vacuum. The crude product was purified by flash chromatography on silica gel (dichloromethane / methanol 2 to 12%). The title compound containing fractions were concentrated under reduced pressure to provide 218 mg of the title compound.

LC-MS (Method 4): $R_t$ = 2.42 min; MS (ESIpos): m/z = 519 $[M+H]^+$

$^1$H-NMR (400 MHz, DMSO-$d_6$) δ [ppm] = 12.27 (s, 1H), 10.92 (s, 1H), 8.58 (d, 1H), 8.39 (d, 1H), 7.61 - 7.45 (m, 3H), 7.41 (d, 1H), 7.19 (t, 2H), 6.41 (d, 1H), 6.03 (tt, 1H), 4.24 (dd, 1H), 3.44 (s, 3H), 2.87 - 2.64 (m, 1H), 2.36 - 2.17 (m, 1H).

**Intermediate 106**

3-bromo-2-(2-chloropyridin-4-yl)-5-methyl-1,5,6,7-tetrahydro-4H-pyrrolo[3,2-c]pyridin-4-one

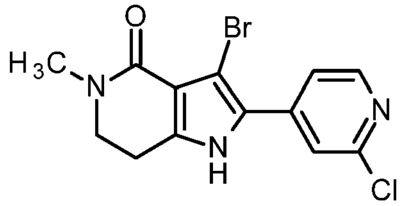

To a solution of 2-(2-chloropyridin-4-yl)-5-methyl-1,5,6,7-tetrahydro-4H-pyrrolo[3,2-c]pyridin-4-one (see Intermediate 99, 800 mg) in 15.3 mL of *N*,*N*-dimethylformamide at 0 °C was added 1-bromopyrrolidine-2,5-dione (571 mg, 3.21 mmol). The reaction was allowed to warm up to rt and stirred for 1 h. The reaction mixture was diluted with a saturated aqueous solution of sodium thiosulfate. The organic layer. The organic layers were dried over $MgSO_4$ and concentrated under vacuum. The crude product was purified by flash chromatography on silica gel (dichloromethane / methanol 1 to 15%). The title compound containing fractions were concentrated under reduced pressure to provide 390 mg of the title compound.

LC-MS (Method 3): Rt = 1.24 min; MS (ESIpos): m/z = 340 / 342 $[M+H]^+$

$^1$H-NMR (400 MHz, DMSO-$d_6$) δ [ppm] = 12.12 (s, 1H), 8.27 (d, 1H), 7.65 (dd, 2H), 3.38 (t, 2H), 2.76 (d, 5H).

**Intermediate 107**

2-(2-chloropyridin-4-yl)-5-methyl-3-phenyl-1,5,6,7-tetrahydro-4H-pyrrolo[3,2-c]pyridin-4-one

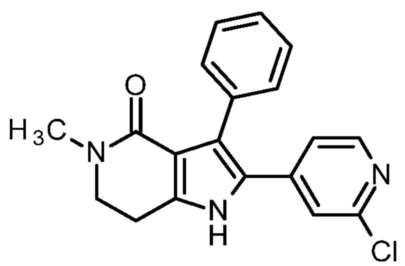

A screw cap vial was charged with 3-bromo-2-(2-chloropyridin-4-yl)-5-methyl-1,5,6,7-tetrahydro-4H-pyrrolo[3,2-c]pyridin-4-one (see Intermediate 106, 323 mg), phenylboronic acid (116 mg, 948 μmol), $Pd(dppf)Cl_2 \cdot CH_2Cl_2$ (77.4 mg, 94.8 μmol) and $K_3PO_4$ (402 mg, 1.90 mmol). The vial was purged with argon and filled with 4.74 mL of dioxane / water 4:1. The reaction was heated at 65 °C. After 90 min, the reaction was cooled down to rt. The crude product was purified by flash chromatography on silica gel (dichloromethane / methanol 2 to 15%). The title compound containing fractions were concentrated under reduced pressure to provide 146 mg of the title compound.

LC-MS (Method 4): $R_t$ = 2.21 min; MS (ESIpos): m/z = 338 $[M+H]^+$

$^1$H-NMR (400 MHz, DMSO-$d_6$) δ [ppm] = 12.01 (s, 1H), 8.13 (d, 1H), 7.44 - 7.28 (m, 3H), 7.27 - 7.19 (m, 2H), 7.13 (d, 1H), 6.96 (dd, 1H), 3.57 (t, 2H), 3.33 (s, 3H), 2.96 (t, 2H).

**Intermediate 108**

2-(2-chloropyridin-4-yl)-5-cyclopropyl-1,5,6,7-tetrahydro-4H-pyrrolo[3,2-c]pyridin-4-one

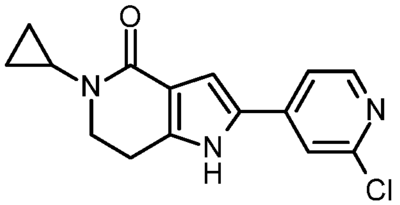

To a round bottom flask charged with 1-cyclopropylpiperidine-2,4-dione (CAS-RN:[1501330-86-4], 1.27 g, 8.28 mmol) and ammonium acetate (1.91 g, 24.8 mmol) was added 41.4 mL of ethanol. To the solution was added 2-bromo-1-(2-chloro-4-pyridyl)ethanone (CAS-RN:[23794-16-3], 1.94 g, 8.28 mmol). The reaction was stirred at rt for 90 min. To the reaction was added 60 mL of water and the mixture was cooled down to 0 °C. The reaction was concentrated under vacuum to remove the organic solvent. The aqueous layer was extracted 3 times with ethyl acetate. The organic layers were combined, dried over $MgSO_4$ and dried under vacuum. The residual crude product was purified by flash chromatography on silica gel (dichloromethane / methanol 2 to 15 %). The title compound containing fractions were concentrated under reduced pressure to provide 1.06 g of the title compound.

LC-MS (Method 4): $R_t$ = 1.83 min; MS (ESIpos): m/z = 288 $[M+H]^+$

$^1$H-NMR (400 MHz, DMSO-$d_6$) δ [ppm] = 12.07 - 11.82 (m, 1H), 8.28 (d, 1H), 7.73 (s, 1H), 7.63 (dd, 1H), 7.14 (d, 1H), 3.53 (t, 2H), 2.87 (t, 2H), 2.61 (dt, 1H), 0.73 (dt, 2H), 0.58 (q, 2H).

**Intermediate 109**

N-[4-(5-cyclopropyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate)

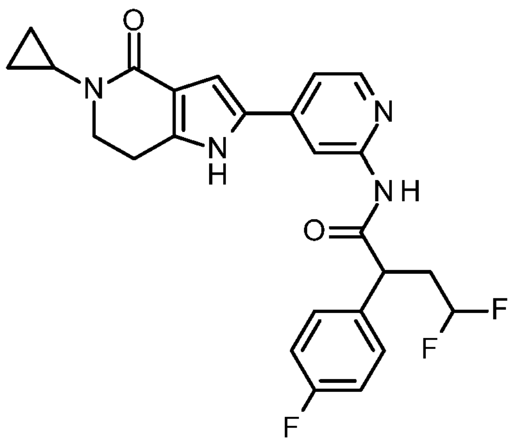

A screw cap vial with a pressure septum was loaded with 2-(2-chloropyridin-4-yl)-5-cyclopropyl-1,5,6,7-tetrahydro-4H-pyrrolo[3,2-c]pyridin-4-one (see Intermediate 108, 981 mg), 4,4-difluoro-2-(4-fluorophenyl)butanamide (963 mg, 4.43 mmol), $Cs_2CO_3$ (2.22 g, 6.82 mmol), t-BuBrettPhos-gen 3 (431 mg, 511 µmol) and t-BuBrett Phos (165 mg, 341 µmol). The vial was degassed with argon and filled with 17.1 mL of 1,4-dioxane. The reaction was heated to 105 °C. After 1 h, the reaction was cooled down and concentrated under vacuum. The crude product was purified by flash chromatography on silica gel (dichloromethane / methanol 2 to 12 %). The title compound containing fractions were concentrated under reduced pressure to provide 810 mg of the title compound.

LC-MS (Method 4): Rt = 2.33 min; MS (ESIpos): m/z = 469 $[M+H]^+$

$^1$H-NMR (400 MHz, DMSO-$d_6$) δ [ppm] = 11.94 (s, 1H), 10.78 (s, 1H), 8.29 - 8.16 (m, 2H), 7.56 - 7.45 (m, 1H), 7.41 - 7.30 (m, 2H), 7.18 (dt, 2H), 6.86 (d, 1H), 6.30 - 5.87 (m, 1H), 4.21 (dd, 1H), 3.76 - 3.61 (m, 1H), 3.53 (t, 2H), 2.86 (t, 2H), 2.63 - 2.54 (m, 1H), 2.35 - 2.08 (m, 1H), 0.74 (td, 2H), 0.63 - 0.50 (m, 2H).

**Intermediate 110**

N-[4-(3-bromo-5-cyclopropyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate)

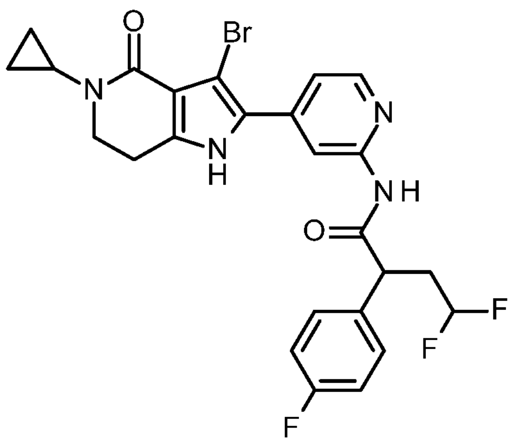

To a solution of N-[4-(5-cyclopropyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate) (see Intermediate 109, 750 mg) in 7.60 mL of N,N-dimethylformamide at 0° C was added 1-bromopyrrolidine-2,5-dione (271 mg, 1.52 mmol). The reaction was allowed to warm up to rt and stirred for 1 h. The reaction mixture was diluted with a saturated aqueous solution of sodium thiosulfate. The solution was extracted 3 times with ethyl acetate. The organic layers were dried over $MgSO_4$ and concentrated under vacuum. The crude product was purified by flash chromatography on silica gel (dichloromethane / methanol 2 to 15%). The title compound containing fractions were concentrated under reduced pressure to provide 758 mg of the title compound.

LC-MS (Method 3): Rt = 1.43 min; MS (ESIpos): m/z = 547 [M+H]+

$^1$H-NMR (400 MHz, DMSO-$d_6$) δ [ppm] = 12.14 (s, 1H), 10.87 (s, 1H), 8.50 (d, 1H), 8.32 (d, 1H), 7.67 - 7.46 (m, 2H), 7.46 - 7.28 (m, 1H), 7.24 - 6.99 (m, 2H), 6.23 - 5.81 (m, 1H), 4.22 (dd, 1H), 3.51 (t, 2H), 2.84 (t, 1H), 2.79 - 2.67 (m, 2H), 2.60 (dt, 1H), 2.36 - 2.04 (m, 1H), 0.78 - 0.65 (m, 2H), 0.57 (dd, 2H).

**Intermediate 111**

N-[4-(3-bromo-5-cyclopropyl-4-oxo-4,5-dihydro-1H-pyrrolo[3,2-c]pyridin-2-yl)pyridin-2-yl]-4,4-difluoro-2-(4-fluorophenyl)butanamide (Racemate)

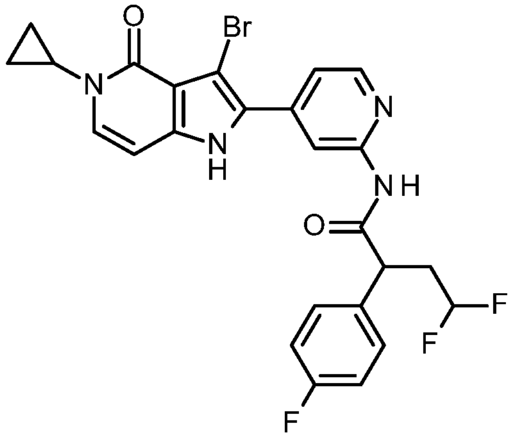

To a solution of N-[4-(3-bromo-5-cyclopropyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate) (see Intermediate 110, 750 mg) in 6.85 mL of 1,4-dioxane was added 2,3-Dichloro-5,6-dicyano-1,4-benzoquinone (467 mg, 2.06 mmol) and the mixture was heated at 80 °C for 90 min. The reaction was cooled down to rt, diluted with cold 0.5 M NaOH and extracted 3 times with ethyl acetate. The organic layers were combined, dried over $MgSO_4$ and concentrated under vacuum. The crude product was purified by preparative reverse phase HPLC (eluent: acetonitrile / water 40 to 80% +0.1% TFA). The title compound containing fractions were concentrated under reduced pressure to provide 470 mg of the title compound.

LC-MS (Method 4): Rt = 2.57 min; MS (ESIpos): m/z = 545 $[M+H]^+$

$^1$H-NMR (400 MHz, DMSO-$d_6$) $\delta$ [ppm] = 12.29 (s, 1H), 10.96 (s, 1H), 8.58 (d, 1H), 8.39 (d, 1H), 7.59 - 7.44 (m, 3H), 7.29 (d, 1H), 7.20 (t, 2H), 6.37 (d, 1H), 6.03 (tt, 1H), 4.23 (dd, 1H), 3.24 (tt, 1H), 2.93 - 2.71 (m, 1H), 2.36 - 2.15 (m, 1H), 0.99 (td, 2H), 0.83 - 0.72 (m, 2H).

## Intermediate 112

3-(4-fluorophenyl)-5-methyl-1,5,6,7-tetrahydro-4H-pyrrolo[3,2-c]pyridin-4-one

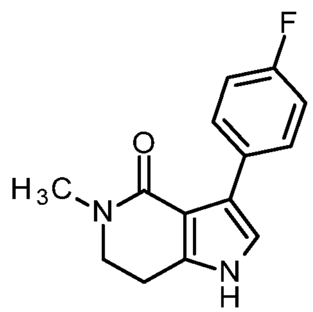

1-Methylpiperidine-2,4-dione (CAS-RN:[118263-97-1], 2.00 g, 15.7 mmol), 2-amino-1-(4-fluorophenyl)ethan-1-one hydrogen chloride salt (CAS-RN:[456-00-8], 2.98 g, 15.7 mmol) and potassium acetate (3.09 g, 31.5 mmol) were suspended in water (28 mL) and stirred at rt for 5 min. The mixture was heated at 90 °C over night. The suspension was diluted with some water. The solid in the mixture was filtered off. The filter cake was washed with water twice and dried in a vacuum drying oven to provide the title compound: 3.27 g
LC-MS (Method 1): Rt = 0.85 min; MS (ESIpos): m/z = 245 $[M+H]^+$

## Intermediate 113

2-bromo-3-(4-fluorophenyl)-5-methyl-1,5,6,7-tetrahydro-4H-pyrrolo[3,2-c]pyridin-4-one

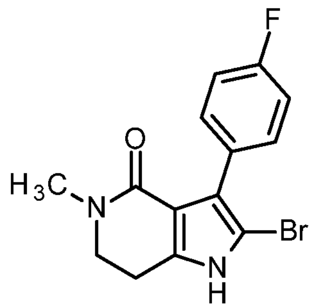

3-(4-Fluorophenyl)-5-methyl-1,5,6,7-tetrahydro-4H-pyrrolo[3,2-c]pyridin-4-one (see Intermediate 112, 3.35 g) was suspended in DMF (32 mL). N-bromosuccinimide (CAS-RN:[128-08-5], 2.93 g, 16.5 mmol) was added and the mixture was stirred under $N_2$ at room temperature for 15 min. The reaction mixture was concentrated under reduced pressure and the residue was diluted with methanol. The resulting precipitate was filtered off. The filter cake was washed with methanol twice to provide the title compound as a grey solid: 3.18 g

LC-MS (Method 1): Rt = 0.95 min; MS (ESIpos): m/z = 323 $[M+H]^+$

$^1$H-NMR (400 MHz, DMSO-$d_6$): δ [ppm] = 12.03 (br s, 1H), 7.43-7.30 (m, 2H), 7.21-7.03 (m, 2H), 3.52 (t, 2H), 2.87-2.79 (m, 5H).

**Intermediate 114**

2-(2-aminopyridin-4-yl)-3-(4-fluorophenyl)-5-methyl-1,5,6,7-tetrahydro-4H-pyrrolo[3,2-c]pyridin-4-one

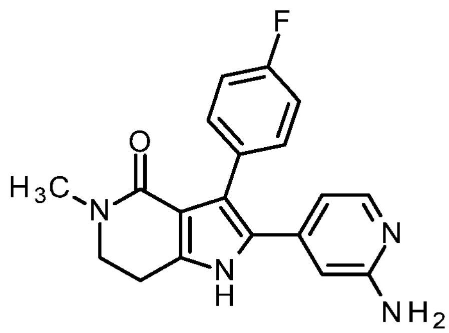

2-Bromo-3-(4-fluorophenyl)-5-methyl-1,5,6,7-tetrahydro-4H-pyrrolo[3,2-c]pyridin-4-one (see Intermediate 113, 1.92 g), 4-(4,4,5,5-tetramethyl-1,3,2-dioxaborolan-2-yl)pyridin-2-amine (CAS-RN:[1195995-72-2], 1.96 g, 8.89 mmol), cesium carbonate (5.80 g, 17.8 mmol) and XPhos Pd G3 (CAS-RN:[1445085-55-1], 502 mg, 593 μmol) were suspended in a degassed mixture of 1 ,4-dioxane (25 mL) and water (2.7 mL) and stirred at 80 °C overnight. The reaction mixture was diluted with ethyl acetate and water. The layers were separated and the aqueous layer was extracted with ethyl acetate once. The combined organic layers were dried using a waterresistant filter and concentrated under reduced pressure. The crude product was diluted with dichloromethane. A solid precipitated, it was filtered off and washed with acetone to to provide the title compound: 1.50 g.

LC-MS (Method 1): $R_t$ = 0.63 min; MS (ESIpos): m/z = 337 $[M+H]^+$

$^1$H-NMR (400 MHz, DMSO-$d_6$): δ [ppm] = 11.66 (s, 1H), 7.70 (d, 1H), 7.24-7.18 (m, 2H), 7.11-7.03 (m, 2H), 6.22 (d, 1H), 6.13 (dd, 1H), 5.78 (s, 2H), 3.58-3.51 (m, 2H), 2.90 (t, 2H), 2.84 (s, 3H)

**Intermediate 115**

methyl 4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]benzoate

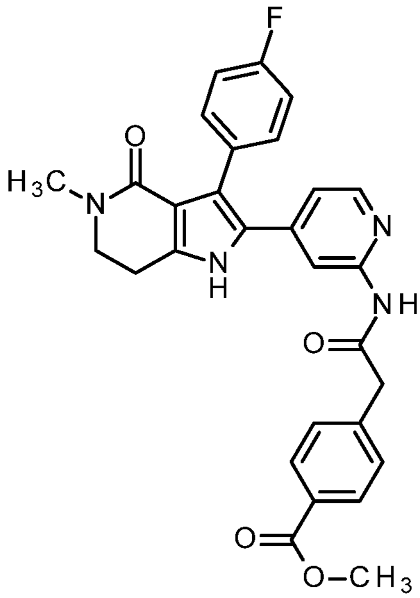

To a solution of 2-(2-aminopyridin-4-yl)-3-(4-fluorophenyl)-5-methyl-1,5,6,7-tetrahydro-4H-pyrrolo[3,2-c]pyridin-4-one (see Intermediate 114, 795 mg) in N,N-dimethylacetamide (40 mL), N,N-diisopropylethylamine (2.5 mL, 14 mmol), HATU (CAS-RN:[148893-10-1], 4.49 g, 11.8 mmol) and [4-(methoxycarbonyl)phenyl]acetic acid (CAS-RN:[22744-12-3], 918 mg, 4.73 mmol) were added and stirred at 60 °C for 18 hours. Aqueous saturated sodium hydrogencarbonate solution was added to the reaction mixture. It was diluted with ethyl acetate and the aqueous layer was extracted with ethyl acetate three times. The combined organic layers were dried over anhydrous sodium sulfate, filtered and concentrated under reduced pressure. The crude product was purified by chromatography (50 g column, silica ULTRA, gradient: ethyl acetate / methanol 0%-20% methanol) to provide the title compound: 859 mg.

LC-MS (Method 1): Rt = 0.99 min; MS (ESIpos): m/z = 513 $[M+H]^+$

$^1$H-NMR (400 MHz, DMSO-$d_6$): δ [ppm] = 11.85 (s, 1H), 10.70 (s, 1H), 8.06 (d, 1H), 8.00 (s, 1H), 7.96-7.91 (m, 2H), 7.46 (d, 2H), 7.23-7.16 (m, 2H), 7.10-7.03 (m, 2H), 6.60 (dd, 1H), 3.85 (s, 3H), 3.78 (s, 2H), 3.54 (t, 2H), 2.96-2.88 (m, 2H), 2.84 (s, 3H)

**Intermediate 116**

4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]benzoic acid

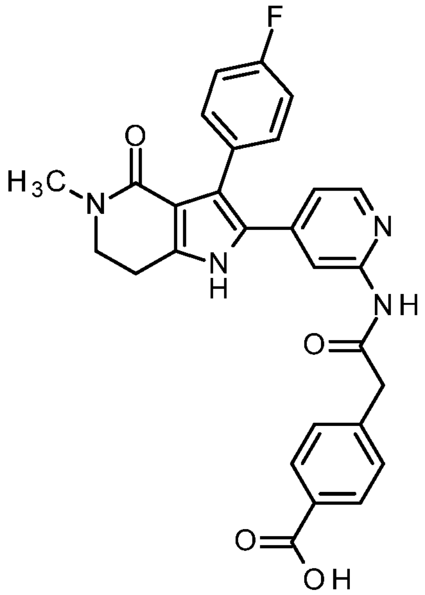

To a solution of methyl 4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]benzoate (see Intermediate 115, 696 mg) in methanol (5.0 mL) /THF (10 mL), lithium hydroxide monohydrate (285 mg, 6.79 mmol) in water (5.0 mL) was added. The reaction mixture was stirred for 2 hours at room temperature. The reaction mixture was diluted with water, pH was set to 4 with 1N HCL and the solution was extracted with chloroform/ethanol 9:1 and water. Precipitate in the aqueous phase was filtered off and dried to provide 397 mg of the title compound:

LC-MS (Method 1): Rt = 0.86 min; MS (ESIpos): m/z = 499 $[M+H]^+$

$^1$H-NMR (400 MHz, DMSO-$d_6$): $\delta$ [ppm] = 13.09-12.78 (m, 1H), 11.85 (s, 1H), 10.69 (s, 1H), 8.05 (d, 1H), 8.01 (s, 1H), 7.93-7.87 (m, 2H), 7.43 (d, 2H), 7.23-7.15 (m, 2H), 7.11-7.03 (m, 2H), 6.59 (dd, 1H), 3.77 (s, 2H), 3.54 (t, 2H), 2.92 (t, 2H), 2.84 (s, 3H)

**Intermediate 117**

tert-butyl 4-{4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]benzoyl}piperazine-1-carboxylate

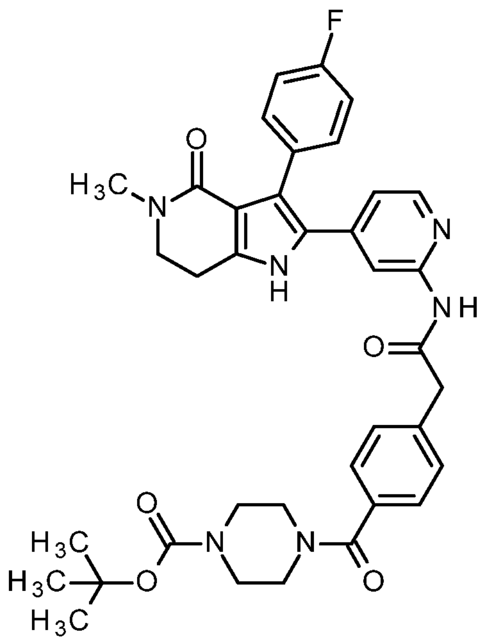

To a solution of 4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]benzoic acid (see Intermediate 116, 352 mg, 705 μmol) in DMF (9.8 mL), potassium carbonate (585 mg, 4.23 mmol), HATU (CAS-RN:[148893-10-1], 670 mg, 1.76 mmol) and tert-butyl piperazine-1-carboxylate (CAS-RN:[57260-71-6], 171 mg, 917 μmol) were added and stirred at room temperature for 16 h. Aqueous saturated sodium hydrogencarbonate solution was added to the reaction mixture. It was diluted with ethyl acetate and the aqueous layer was extracted with ethyl acetate three times. The combined organic layers were washed with brine, dried over anhydrous sodium sulfate, filtered and concentrated under reduced pressure. The crude product was purified by chromatography (25 g column, silica ULTRA, gradient: ethyl acetate / methanol 2%-20% methanol) to provide the title compound:

LC-MS (Method 1): Rt = 1.09 min; MS (ESIneg): m/z = 665 [M-H]-

$^1$H-NMR (400 MHz, DMSO-$d_6$) δ [ppm]: 1.172 (0.57), 1.401 (13.34), 1.988 (1.03), 2.084 (2.45), 2.522 (1.14), 2.687 (16.00), 2.728 (4.91), 2.837 (5.98), 2.888 (6.18), 2.903 (0.64), 2.921 (1.32), 2.938 (0.69), 3.384 (0.72), 3.526 (0.88), 3.543 (1.67), 3.560 (0.89), 3.728 (2.14), 6.588 (0.75), 6.592 (0.76), 6.601 (0.73), 6.605 (0.77), 7.046 (0.75), 7.069 (1.69), 7.091 (1.07), 7.177 (1.01), 7.183 (0.44), 7.191 (1.18), 7.199 (0.96), 7.214 (0.76), 7.355 (0.48), 7.376 (3.61), 7.382 (3.52), 7.403 (0.46), 7.950 (0.78), 8.017 (0.96), 8.049 (1.07), 8.063 (1.02), 10.681 (1.22), 11.847 (0.89).

**Intermediate 118**

2-bromo-1-(2-chloropyridin-4-yl)ethan-1-one

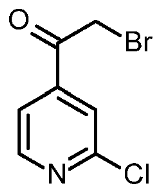

To a solution of 1-(2-chloropyridin-4-yl)ethan-1-one (CAS-RN: [23794-15-2], 25.0 g, 161 mmol) in acetic acid (500 mL) were added hydrobromic acid in acetic acid (26 mL, 33% purity, 160 mmol) and bromine (9.1 mL, 180 mmol) at room temperature. The mixture was stirred at room temperature for 16 hours. The reaction mixture was filtered. The filter cake was diluted with saturated sodium bicarbonate and extracted with ethyl acetate. The organic phase was washed with brine, dried over anhydrous sodium sulfate and filtered. The filtrate was concentrated and purified by column

chromatography on silica gel (200-300 mesh, petroleum ether: ethyl acetate = 5: 1) to give 32.0 g of the title compound as a yellow solid.

LC-MS (Method 6): Rt = 0.717 min; MS (ESIpos): m/z = 234.0 / 236.0 $[M+H]^+$.

$^1$H NMR (400 MHz, DMSO-$d_6$): δ [ppm] = 8.67 (d, 1H), 8.00 (s, 1H), 7.86 (dd, 1.2 Hz, 1H), 5.03 (s, 2H).

**Intermediate 119**

2-(2-chloropyridin-4-yl)-5-methyl-1,5,6,7-tetrahydro-4H-pyrrolo[3,2-c]pyridin-4-one

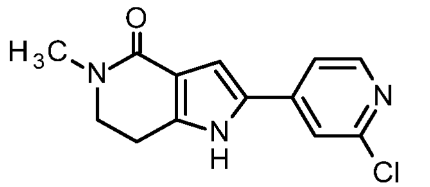

To a solution of 2-bromo-1-(2-chloropyridin-4-yl)ethan-1-one (see Intermediate 118, 32.0 g) in ethanol (320 mL) were added ammonium acetate (42.1 g, 546 mmol) and 1-methylpiperidine-2,4-dione (CAS-RN:[118263-97-1], 19.1 g, 150 mmol) in one portion. The mixture was stirred at 25 °C for 16 hours. The reaction mixture was diluted with water (1280 mL) and filtered. The filter cake was dried to give 21.0 g of the title compound as a yellow solid.

LC-MS (Method 6): Rt = 0.747 min; MS (ESIpos): m/z = 262.2 $[M+H]^+$.

$^1$H NMR (400 MHz, DMSO-$d_6$): δ [ppm] = 12.00 (s, 1H) 8.29 (d, 1H), 7.74 (d, 1H), 7.64 (dd, 1.6 Hz, 1H), 7.15 (d, 1H), 3.56 (t, 2H), 3.95 (t, 2H), 2.92 (s, 3H).

**Intermediate 120**

2-(2-chloropyridin-4-yl)-5-methyl-1,5-dihydro-4H-pyrrolo[3,2-c]pyridin-4-one

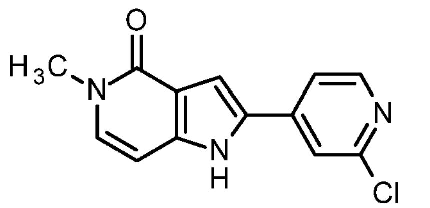

To a mixture of 2-(2-chloropyridin-4-yl)-5-methyl-1,5,6,7-tetrahydro-4H-pyrrolo[3,2-c]pyridin-4-one (see Intermediate 119, 5.00 g) in 1,4-dioxane (1800 mL) was added bis(oxido)manganese (16.6 g, 191 mmol) in one portion. The reaction mixture was stirred at 100 °C for 16 hours. The reaction mixture (combined with another batch starting from 10 g) was filtered and the filtrate was concentrated to give crude product. The crude product was triturated with methanol (130 mL) to give 8.20 g of the title compound as a yellow solid.

LC-MS (Method 6): Rt = 0.768 min; MS (ESIpos): m/z = 260.1 $[M+H]^+$.

$^1$H NMR (400 MHz, DMSO-$d_6$): δ [ppm] = 12.12 (s, 1H) 8.37 (d, 1H), 7.88 (d, 1H), 7.77 (dd, 1H), 7.40 (d, 1H), 7.35 (d, 1H), 6.47 (d, 1H), 3.50 (s, 3H).

**Intermediate 121**

2-(4-fluorophenyl)-N-[4-(5-methyl-4-oxo-4,5-dihydro-1H-pyrrolo[3,2-c]pyridin-2-yl)pyridin-2-yl]acetamide

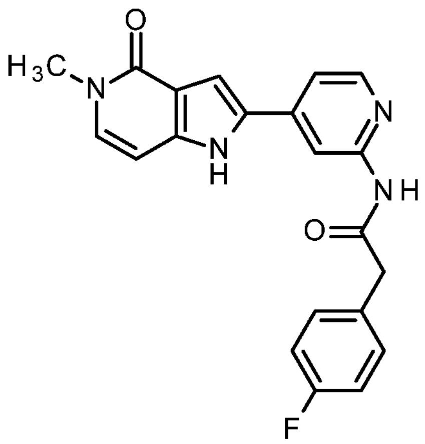

To a mixture of 2-(2-chloropyridin-4-yl)-5-methyl-1,5-dihydro-4H-pyrrolo[3,2-c]pyridin-4-one (see Intermediate 120, 2.20 g) and 2-(4-fluorophenyl)acetamide (CAS-RN:[332-29-6], 2.59 g, 16.9 mmol,) in tert-amyl alcohol (330 mL) were added (9,9-dimethyl-9H-xanthene-4,5-diyl)bis(diphenylphosphine) (490 mg, 847 μmol), palladium(II) acetate (190 mg, 847 μmol) and cesium carbonate (6.90 g, 21.2 mmol) in one portion at room temperature. The reaction mixture was stirred at 110 °C for 16 hours under nitrogen atmosphere. The reaction mixture was concentrated in reduced pressure to give a residue. The residue was diluted with water and extracted with ethyl acetate. The organic phase was washed with brine, dried over anhydrous sodium sulfate and filtered. The filtrate was concentrated and purified by column chromatography on silica gel (200-300 mesh, petroleum ether: ethyl acetate = 1: 1) to give 1.10 g of the title compound as a yellow solid.

LC-MS (Method 6): Rt = 0.786 min; MS (ESIpos): m/z = 377.2 $[M+H]^+$.

**Intermediate 122**

N-[4-(3-bromo-5-methyl-4-oxo-4,5-dihydro-1H-pyrrolo[3,2-c]pyridin-2-yl)pyridin-2-yl]-2-(4-fluorophenyl)acetamide

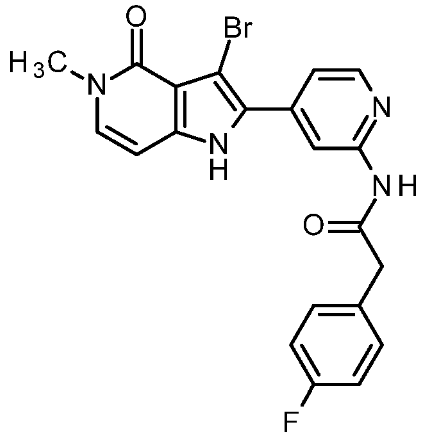

To a solution of 2-(4-fluorophenyl)-N-[4-(5-methyl-4-oxo-4,5-dihydro-1H-pyrrolo[3,2-c]pyridin-2-yl)pyridin-2-yl]acetamide (see Intermediate 121, 800 mg) in N,N-dimethylformamide (40 mL) was added N-bromosuccinimide (416 mg, 2.34 mmol) in one portion at -30 °C. The reaction mixture was stirred at -30 °C for 5 hours. The reaction mixture was concentrated and purified by column chromatography on silica gel (200-300 mesh, petroleum ether: ethyl acetate

= 1: 2) to give 210 mg of the title compound as a yellow solid.
LC-MS (Method 6): Rt = 0.817 min; MS (ESIpos): m/z = 455.2 / 457.2 $[M+H]^+$.

**Intermediate 123**

(rac)-4,4-difluoro-2-(4-fluorophenyl)butanamide (Racemate)

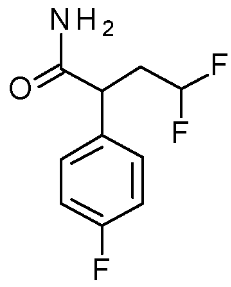

(rac)-4,4-Difluoro-2-(4-fluorophenyl)butanoic acid (Racemate) (2.00 g) was dissolved in dichloromethane and cooled down with an ice bath. Oxalyl chloride (960 μL, 11 mmol) was added, followed by 50 μL DMF. It was stirred at rt for 1 hour under argon atmosphere. The mixture was cooled down again and treated with aqueous ammonia (20 mL, 33 % purity, 120 mmol) drop wise. To the reaction mixture was water added and extracted with dichloromethane three times. Between both layers a white precipitate was formed. It was filtered off, washed with dichloromethane and water and dried at 50°C under vacuo. The layers of the filtrate were separated and the organic layer was washed with water and brine, filtered through a silicone coated filter and concentrated under reduced pressure to provide the analytically pure target compound: 1.12 g.

LC-MS (Method 2): Rt = 0.87 min; MS (ESIpos): m/z = 218 $[M+H]^+$

$^1$H-NMR (400 MHz, DMSO-d6) δ [ppm] = 1.97 - 2.21 (m, 1 H), 2.53 - 2.65 (m, 1 H), 3.69 (dd, 1 H), 5.65 - 6.14 (m, 1 H), 6.99 (br s, 1 H), 7.08 - 7.22 (m, 2 H), 7.29 - 7.43 (m, 2 H), 7.58 (br s, 1 H). - contains DMF.

**Intermediate 124**

ethyl-2-cyano-4,4-difluorobutanoate (Racemate)

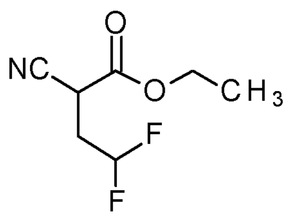

To a solution of ethyl cyanoacetate (CAS-RN:[105-56-6], 52.8 g, 467 mmol) in tetrahydrofuran (1000 mL) was drop-wise added lithium bis(trimethylsilyl)amide (467 mL, 1.0 M, 467mmol) at -70 °C. The mixture was stirred at -70 °C for 1 hour, a solution of 2,2-difluoroethyl trifluoromethanesulfonate ((CAS-RN:[74427-22-8], 100 g, 467 mmol) in tetrahydrofuran (500 mL) was drop-wise added and and the resulting mixture was stirred at room temperature for 16 hours. Aqueous hydrochloric acid solution (1 M) was added and extracted with ethyl acetate. The combined organic phase was dried over anhydrous sodium sulfate, filtered and concentrated to give a residue. The residue (combined with two other batches starting from 21.1 g and 52.8 g) was purified by silica gel chromatography (100-200 mesh, petroleum ether: ethyl acetate = 1: 0 then 100: 1) to give 107 g ethyl-2-cyano-4,4-difluorobutanoate (Racemate) as yellow oil.

$^1$H-NMR (400 MHz, $CDCl_3$): δ [ppm] = 6.22-5.93 (m, 1H), 4.37-4.27 (m, 2H), 3.73 (t, 1H), 2.63-2.40 (m, 2H), 1.35 (t, 3H).

**Intermediate 125**

ethyl-2-(aminomethyl)-4,4-difluorobutanoate hydrogen chloride salt (Racemate)

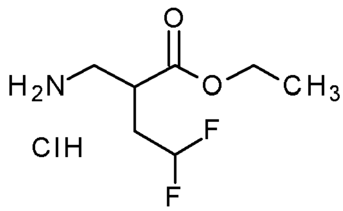

A mixture of ethyl-2-cyano-4,4-difluorobutanoate (Racemate) (see Intermediate 124, 20.0 g) and bis(oxido)platinum (2.56 g, 11.3 mmol) in methanol (400 mL) and hydrochloric acid (11 mL, 12 M, 140 mmol) was stirred at room temperature for 16 hours under hydrogen (15 psi). The combined mixture (combined with four other batches starting each from 20 g) was filtered through a pad of celite. The filtrate was concentrated and the combined product (100 g) was used to next step directely.

**Intermediate 126**

ethyl-2-{[(tert-butoxycarbonyl)amino]methyl}-4,4-difluorobutanoate (Racemate)

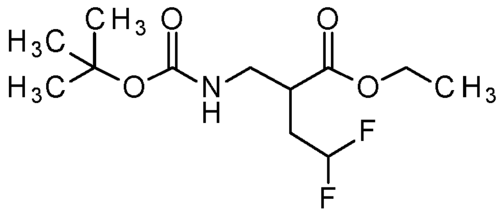

A mixture of ethyl-2-(aminomethyl)-4,4-difluorobutanoate hydrogen chloride salt (Racemate) (see Intermediate 125, 52.5 g), di-tert-butyl dicarbonate (63.2 g, 289 mmol) and N,N-diisopropylethylamine (130 mL, 720 mmol) in dichloromethane (520 mL) was stirred at room temperature for 2 hours. Water was added and the mixture was extracted with dichloromethane. The organic phase was washed with hydrochloric acid solution (1M) twice. The organic phase was dried over anhydrous sodium sulfate, filtered and concentrated to give a residue. The residue was purified by silica gel chromatography (100-200 mesh, petroleum ether: ethyl acetate = 1:0 then 100:1) to give 75.0 g ethyl-2-{[(tert-butoxycarbonyl)amino]methyl}-4,4-difluorobutanoate (Racemate) as yellow oil.

$^1$H-NMR (400 MHz, $CDCl_3$): $\delta$ [ppm] = 6.11-5.83 (m, 1H), 4.88 (s, 1H), 4.22-4.17 (m, 2H), 3.42-3.36 (m, 2H), 2.84-2.80 (m, 1H), 2.28-2.20 (m, 1H), 2.01-2.00 (m, 1H), 1.44 (s, 9H), 1.30-1.26 (m, 3H).

**Intermediate 127**

ethyl-2-{[(tert-butoxycarbonyl)(methyl)amino]methyl}-4,4-difluorobutanoate (Racemate)

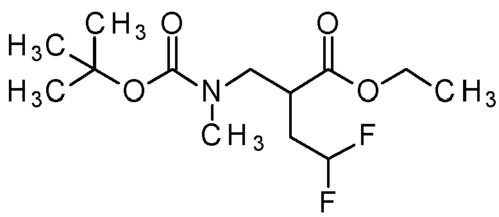

To a solution of ethyl-2-{[(tert-butoxycarbonyl)amino]methyl}-4,4-difluorobutanoate (Racemate) (see Intermediate 126, 73.0 g) in tetrahydrofuran (500 mL) was add drop-wise to a solution of sodium hydride (12.5 g, 60 % purity, 311 mmol, contained mineral oil) in tetrahydrofuran (500 mL) at 0 °C. The mixture was stirred at 0 °C for 1 hour. Iodomethane (73.7 g, 519 mmol) was drop-wise added and the resulting mixture was stirred at 0 °C for 0.5 hour. Then the mixture was stirred at 40 °C for 3 hours. Saturated aqueous ammonium chloride solution was added and the mixture was extracted with ethyl acetate. The organic phase was washed with brine, dried over anhydrous sodium sulfate, filtered and concentrated to give a residue. The residue (combined with another batch starting from 5 g) was purified by silica gel chromatography (200-300 mesh, petroleum ether: ethyl acetate = 100:1 then 10:1) to give 40 g ethyl-2-{[(tert-butoxycarbonyl)(methyl)amino]methyl}-4,4-difluorobutanoate (Racemate) as yellow oil.

$^1$H-NMR (400 MHz, $CDCl_3$): δ [ppm] = 6.09-5.77 (m, 1H), 4.20-4.16 (m, 2H), 3.50-3.36 (m, 2H), 2.98-2.94 (m, 0.5H), 2.86 (s, 3H), 2.80-2.73 (m, 0.5H), 2.34-2.17 (m, 1H), 2.00-1.82 (m, 1H), 1.46 (s, 9H), 1.30-1.26 (m, 3H).

**Intermediate 128**

ethyl-4,4-difluoro-2-[(methylamino)methyl]butanoate hydrogen chloride salt (Racemate)

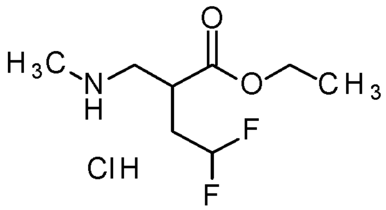

To a solution of ethyl-2-{[(tert-butoxycarbonyl)(methyl)amino]methyl}-4,4-difluorobutanoate (Racemate) (see Intermediate 127, 35.0 g) in 1,4-dioxane (150 mL) was added hydrochloric acid (150 mL, 4 M in dioxane) at room temperature. The reaction mixture was stirred at room temperature for 2 hours. The mixture was cocnentrated by evaporation in reduced pressure to give 27 g ethyl-4,4-difluoro-2-[(methylamino)methyl]butanoate hydrochloride salt (Racemate) as a yellow oil.

$^1$H-NMR (400 MHz, DMSO-$d_6$): δ [ppm] = 9.26 (s, 1H), 9.07 (s, 1H), 6.35-6.05 (m, 1H), 4.17-4.10 (m, 2H), 3.41 (s, 3H), 3.21-3.17 (m, 1H), 3.09-3.04 (m, 2H), 2.31-2.22 (m, 2H), 1.21(t, 3H).

**Intermediate 129**

ethyl-2-{[(3-ethoxy-3-oxopropanoyl)(methyl)amino]methyl}-4,4-difluorobutanoate (Racemate)

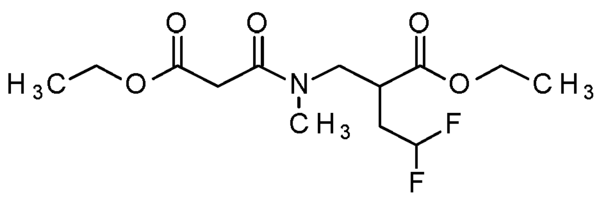

N,N-diisopropylethylamine (41 mL, 230 mmol) was added at 0°C to a solution of 4,4-difluoro-2-[(methylamino)methyl]butanoate hydrogen chloride salt (Racemate) (see Intermediate 128, 27.0 g) in dichloromethane (277 mL). And ethyl-3-chloro-3-oxopropanoate (17.5 g, 117 mmol) was added to the mixture slowly at below 15°C. The mixture was stirred at room temperature for 16 hours. The mixture (combined with another batch starting from 3.9 g) was adjusted to pH~3 with aqueous hydrochloric acid solution (2 M). Then the mixture was extracted with dichloromethane and the combined organic layer was washed with brine, dried over anhydrous sodium sulfate, filtered and concentrated by evaporation in vacuum. The residue was purified by column chromatography (100-200 mesh, petroleum ether: ethyl acetate = 20:1 then 5:1) to give 30 g ethyl-2-{[(3-ethoxy-3-oxopropanoyl)(methyl)amino]methyl}-4,4-difluorobutanoate (Racemate) as yellow oil.

**Intermediate 130**

5-(2,2-difluoroethyl)-1-methylpiperidine-2,4-dione (Racemate)

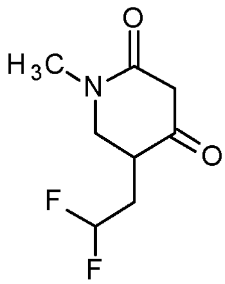

To a solution of ethyl-2-{[(3-ethoxy-3-oxopropanoyl)(methyl)amino]methyl}-4,4-difluorobutanoate (Racemate) (see Intermediate 129, 30.0 g) in ethanol (300 mL) was added sodium ethoxide (CAS-RN:[141-52-6], 7.92 g) at 0-5 °C. Then the mixture was stirred at room temperature for 4 hours. The mixture was treated with aqueous hydrogen chloride solution (2 M) until pH~3, the aqueous phase was extracted with ethyl acetate. The combined organic phase was washed with brine, dried over anhydrous sodium sulfate, filtered and concentrated to give a residue. The residue was dissolved in acetonitrile (300 mL) and water (15 mL) at room temperature, and the resulting mixture was heated at reflux for 16 hours. The mixture was concentrated to dryness. The residue was purified by flash column chromatography (petroleum ether: ethyl acetate = 30:1 to 1:1) to give 4.0 g 5-(2,2-difluoroethyl)-1-methylpiperidine-2,4-dione (Racemate) as yellow oil.

LC-MS (Method: Instrument: SHIMADZU LCMS-2020 SingleQuad; Column: Chromolith@Flash RP-18E 25-2 MM; eluent A: water + 0.0375 vol % trifluoroacetic acid, eluent B: acetonitrile + 0.01875 vol % trifluoroacetic acid; gradient: 0-0.8 min 0-60% B, 0.8-1.2 min 60% B; flow 1.5 mL/min; temperature: 50 °C; PDA: 220 nm & 254 nm.): Rt = 0.627 min; MS (ESIpos): m/z = 192.1 $[M+H]^+$.

$^1$H-NMR (400 MHz, $CDCl_3$): $\delta$ [ppm] = 6.20-5.91 (m, 1H), 3.63-3.58 (m, 1H), 3.44-3.29 (m, 3H), 3.07 (s, 3H), 2.99-2.93 (m, 1H), 2.51-2.43 (m, 1H), 1.89-1.77 (m, 1H).

**Intermediate 131**

(rac)-2-(2-chloropyridin-4-yl)-7-(2,2-difluoroethyl)-5-methyl-1,5,6,7-tetrahydro-4H-pyrrolo[3,2-c]pyridin-4-one (Racemate)

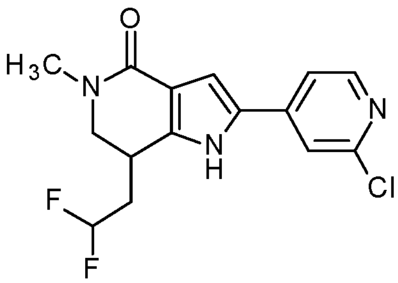

(rac)-5-(2,2-Difluoroethyl)-1-methylpiperidine-2,4-dione (see Intermediate 130, 1.00 g), 2-bromo-1-(2-chloropyridin-4-yl)ethan-1-one (1.23 g, 5.23 mmol) and ammonium acetate (1.21 g, 15.7 mmol) were dissolved in 39 mL 1-propanol and stirred at 120°C for 4 hours under nitrogen atmosphere. Most of the 1-propanol was removed under reduced pressure. The residue was diluted with ethyl acetate and 2 M aqueous sodiumhydroxide solution. The layers were separated and the aquoeus layer was extracted with ethyl acetate once. The combined organic layers were dried using a waterresistant filter and the clear filtrate was concentrated under reduced pressure. The residue was diluted with dichloromethane. A yellow solid precipitated and was filtered off under vacuo to provide batch 1 of the desired product in 95% purity: 593 mg. The clear filtrate was concentrated under reduced pressure and purified by flash chromatography (25g ultra column, gradient dichloromethane/ethanol 0-10) to provide batch 2 of the target compound in 88% purity: 340 mg. Batch 1 and 2 were combined and used for the following reaction: 932 mg in 90% purity.

LC-MS (Method 2): $R_t$ = 0.88 min; MS (ESIpos): m/z = 326 $[M+H]^+$

$^1$H-NMR (400 MHz, DMSO-d6) δ [ppm]: 2.10 - 2.28 (m, 1 H) 2.32 - 2.46 (m, 1 H) 2.93 (s, 3 H) 3.32 (br dd, 1 H) 3.43 - 3.52 (m, 1 H) 3.72 (dd, 1 H) 6.11 - 6.56 (m, 1 H) 7.17 (d, 1 H) 7.69 (dd, 1 H) 7.80 (d, 1 H) 8.30 (d, 1 H) 11.87 (br s, 1 H).

## Intermediate 132

N-{4-[7-(2,2-difluoroethyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (Mixture of stereoisomers)

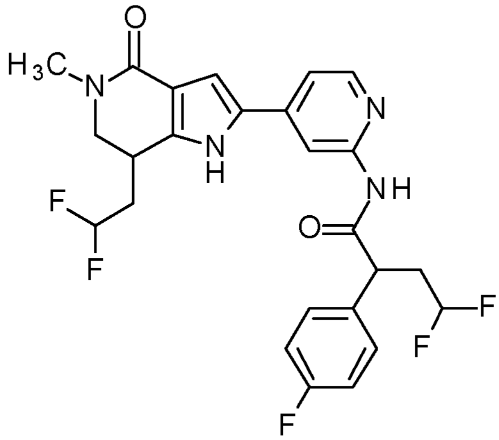

(rac)-2-(2-Chloropyridin-4-yl)-7-(2,2-difluoroethyl)-5-methyl-1,5,6,7-tetrahydro-4H-pyrrolo[3,2-c]pyridin-4-one (Racemate) (see Intermediate 131, 410 mg), (rac)-4,4-difluoro-2-(4-fluorophenyl)butanamide (Racemate) (see Intermediate 123, 410 mg), cesium carbonate (1.23 g, 3.78 mmol), di-tert-butyl(2',4',6'-triisopropyl-3,6-dimethoxy-[1,1'-biphenyl]-2-yl)phosphine (122 mg, 252 µmol; CAS-RN:[1160861-53-9]) and [(2-di-tert-butylphosphino-3,6-dimethoxy-2',4', 6'-triisopropyl-1,1'-biphenyl)-2-(2'-amino-1,1'-biphenyl)]palladium(II) methanesulfonate (108 mg, 126 µmol; CAS-RN:[1536473-72-9]) were dissolved in 11 mL dioxane and stirred at 100°C for 1 hour under nitrogen atmosphere. This reaction mixture was combined with the same reaction starting from (rac)-2-(2-Chloropyridin-4-yl)-7-(2,2-difluoroethyl)-5-methyl-1,5,6,7-tetrahydro-4H-pyrrolo[3,2-c]pyridin-4-one (Racemate) (see Intermediate 131, 50 mg) and was diluted with ethyl acetate and water. The layers were separated and the aqueous layer was extracted with ethyl acetate once. The combined organic layers were dried using a waterresistant filter and the clear filtrate was concentrated under reduced pressure. The crude product was purified by flash chromatography (25g silica ultra column, gradient dichloromethane/ethanol 0-10%) to provide the target compound in 91% purity: 773 mg.

LC-MS (Method 2): Rt = 1.13 min; MS (ESIpos): m/z = 507 $[M+H]^+$

$^1$H-NMR (400 MHz, DMSO-d6) δ [ppm]: 0.812 (1.01), 0.829 (1.07), 0.856 (2.76), 0.873 (2.89), 0.883 (0.80), 0.901 (1.72), 0.920 (0.89), 0.928 (0.67), 0.945 (0.72), 1.035 (0.80), 1.053 (1.91), 1.071 (0.86), 1.104 (3.44), 1.117 (1.05), 1.139 (3.62), 1.249 (1.97), 1.266 (1.90), 2.065 (2.17), 2.081 (0.56), 2.091 (0.50), 2.094 (0.53), 2.106 (0.48), 2.120 (0.96), 2.132 (0.74), 2.144 (0.49), 2.171 (0.47), 2.181 (0.49), 2.221 (0.41), 2.229 (0.45), 2.323 (0.48), 2.327 (0.58), 2.419 (0.83), 2.436 (0.70), 2.518 (2.07), 2.523 (1.36), 2.542 (0.53), 2.553 (0.51), 2.565 (0.48), 2.578 (0.42), 2.669 (0.49), 2.673 (0.43), 2.752 (0.45), 2.922 (16.00), 3.283 (0.56), 3.295 (0.77), 3.308 (0.87), 3.320 (0.72), 3.402 (1.03), 3.416 (0.85), 3.423 (0.57), 3.433 (1.26), 3.440 (0.68), 3.447 (1.00), 3.452 (0.61), 3.470 (1.45), 3.565 (9.51), 3.665 (1.29), 3.669 (0.99), 3.682 (1.67), 3.692 (1.43), 3.706 (1.53), 3.727 (0.67), 3.741 (0.57), 3.777 (1.27), 4.192 (0.64), 4.206 (0.75), 4.214 (0.75), 4.228 (0.60), 4.356 (0.56), 5.773 (0.70), 5.896 (0.48), 5.903 (0.86), 5.914 (1.43), 5.926 (0.73), 6.025 (0.43), 6.036 (0.88), 6.044 (0.70), 6.055 (0.71), 6.176 (0.43), 6.276 (0.61), 6.282 (0.60), 6.835 (0.84), 6.860 (2.89), 6.866 (2.92), 6.988 (1.25), 7.131 (2.79), 7.136 (0.94), 7.148 (1.07), 7.154 (5.85), 7.159 (1.09), 7.176 (4.02), 7.184 (0.58), 7.195 (4.08), 7.218 (2.19), 7.354 (2.81), 7.359 (1.21), 7.367 (4.23), 7.376 (2.92), 7.380 (1.86), 7.384 (2.40), 7.389 (2.40), 7.487 (2.00), 7.492 (0.96), 7.501 (2.19), 7.509 (2.07), 7.523 (1.78), 7.581 (1.23), 8.215 (2.62), 8.228 (2.59), 8.244 (1.97), 10.794 (2.54), 11.870 (1.43).

**Intermediate 133**

N-{4-[3-bromo-7-(2,2-difluoroethyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (Mixture of stereoisomers)

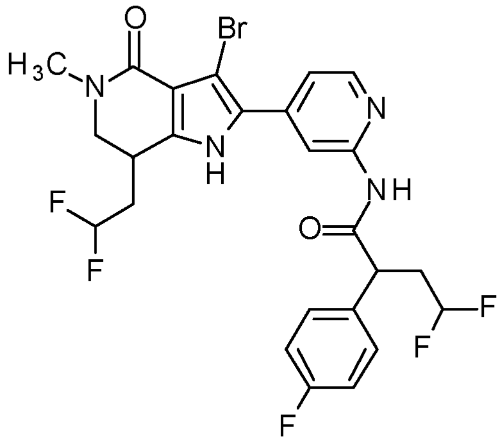

N-{4-[7-(2,2-Difluoroethyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (mixture of stereoisomers) (see Intermediate 132, 1.44 g) was dissolved in 26 mL DMF and cooled down with an ice bath. N-bromosuccinimide (504 mg, 2.83 mmol) was added and the mixture was stirred at 0°C under nitrogen atmosphere for 1 hour. To the reaction mixture saturated aqueous sodium hydrogencarbonate solution and saturated aqueous sodiumthiosulfate solution was added. This mixture was extracted with dichloromethane/isopropanole (7:3) twice. The combined organic layers were dried using a waterresistant filter. The clear filtrate was concentrated under reduced pressure. The crude product was purified by flash chromatography (50g silica ultra column, gradient dichloromethane/ethanol 0-10%) and HPLC to provide the target compound in 98% purity: 0.66 g.

LC-MS (Method 1): Rt = 1.15 min; MS (ESIpos): m/z = 585 $[M+H]^+$

$^1$H-NMR (400 MHz, DMSO-d6) $\delta$ [ppm]= 2.05 - 2.41 (m, 3 H), 2.76 (ddd, 1 H), 2.92 (s, 3 H), 3.29 (br dd, 1 H), 3.39 - 3.47 (m, 1 H), 3.67 - 3.79 (m, 1 H), 4.22 (br dd, 1 H), 5.85 - 6.48 (m, 2 H), 7.19 (td, 2 H), 7.41 (dd, 1 H), 7.46 - 7.58 (m, 2 H), 8.34 (d, 1 H), 8.48 (d, 1 H), 10.89 (s, 1 H), 12.13 (s, 1 H).

**Intermediate 134**

(rac)-N-{4-[3-bromo-7-(2,2-difluoroethyl)-5-methyl-4-oxo-4,5-dihydro-1H-pyrrolo[3,2-c]pyridin-2-yl]pyridin-2-yl}-4,4-difluoro-2-(4-fluorophenyl)butanamide (Racemate)

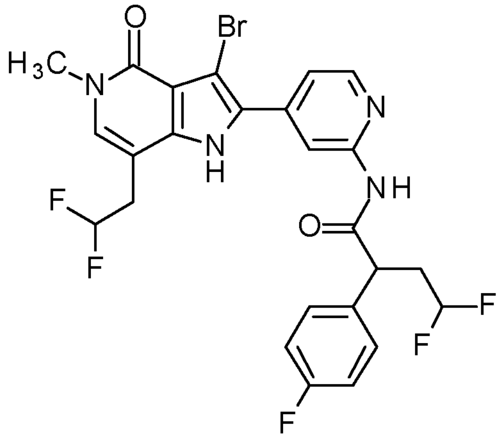

N-{4-[3-Bromo-7-(2,2-difluoroethyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (mixture of stereoisomers, see Intermediate 133, 655 mg) was dissolved in 48 mL 1,4-dioxane and 2,3-dichloro-5,6-dicyano-1,4-benzoquinone (762 mg, 3.36 mmol) was added. The mixture was stirred at 80 °C overnight. The reaction mixture was diluted with ethyl acetate and treated with 1 M aqueous sodium hydroxide solution. The layers were separated and the aqueous layer was extracted with ethyl acetate twice. The combined organic layers were dried using a waterresistant filter and concentrated under reduced pressure to provide a mixture of starting material and product. This mixture was therefore used for the same reaction again. A mixture of N-{4-[3-Bromo-7-(2,2-difluoroethyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (mixture of stereoisomers, see Intermediate 133, 655 mg) and (rac)-N-{4-[3-bromo-7-(2,2-difluoroethyl)-5-methyl-4-oxo-4,5-dihydro-1H-pyrrolo[3,2-c]pyridin-2-yl]pyridin-2-yl}-4,4-difluoro-2-(4-fluorophenyl)butanamide (racemate) (see Intermediate 134) (627 mg) was dissolved in 46 mL 1,4-dioxane and 2,3-dichloro-5,6-dicyano-1,4-benzoquinone (729 mg, 3.21 mmol) was added. The mixture was stirred at 80 °C for 2 h. The reaction mixture was diluted with ethyl acetate and treated with 1 M aqueous sodium hydroxide solution. The layers were separated and the aqueous layer was extracted with ethyl acetate twice. The combined organic layers were dried using a waterresistant filter and concentrated under reduced pressure. The crude product was purified by flash chromatography (25 g silica ultra column, gradient dichloromethane/ethanol 1-25%) to provide a mixture of the desired compound and starting material: 127 mg, which was used without further purification.
LC-MS (Method 1): Rt = 1.14 min; MS (ESIpos): m/z = 583/585 $[M+H]^+$

## Intermediate 135

benzyl 2,2-dimethyl-3-(methylamino)propanoate

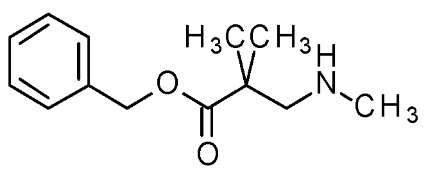

Benzyl 2,2-dimethyl-3-oxopropanoate (5.00 g, 24.2 mmol; CAS-RN:[97518-80-4]) and methanamine (13 ml, 2.0 M in methanol, 27 mmol; CAS-RN:[74-89-5]) were dissolved in 79 mL methanol and stirred for 1h at 60°C under argon atmosphere. The mixture was cooled down and 2.8 mL acetic acid and sodium cyanoborohydride (3.05 g, 48.5 mmol; CAS-RN:[25895-60-7]) were added. The reaction mixture was stirred at rt overnight. The reaction mixture was concentrated under reduced pressure. The residue was diluted with ethyl acetate and aqueous saturated sodium hydrogencarbonate solution. The layers were separated and the aqueous layer extracted with ethyl acetate twice. The combined organic layers were washed with brine, filtered through a water resistant filter and concentrated under reduced pressure. The crude product was purified by chromatography (KP-NH-column 110g; gradient: dichloromethane/ethanol 0-10%) to provide two batches of the target compound: 2.47 g (72% purity) and 0.40 g (83% purity). These batches were combined and used in the following reaction.

batch 1:

LC-MS (Method 2): Rt = 1.13 min; MS (ESIpos): m/z = 223 $[M+H]^+$
$^1$H-NMR (400 MHz, DMSO-d6) δ [ppm]: 1.084 (0.68), 1.114 (16.00), 2.240 (7.31), 2.552 (3.34), 3.568 (0.43), 5.079 (3.61), 7.330 (0.43), 7.340 (0.42), 7.356 (3.56), 7.361 (1.18), 7.369 (0.88), 7.373 (1.03).

batch 2:

LC-MS (Method 2): Rt = 1.13 min; MS (ESIpos): m/z = 223 $[M+H]^+$
$^1$H-NMR (400 MHz, DMSO-d6) δ [ppm]: .11 (s, 6 H) 2.24 (s, 3 H) 2.55 (s, 2 H) 5.08 (s, 2 H) 7.23 - 7.49 (m, 5 H).

**Intermediate 136**

benzyl 3-[(3-ethoxy-3-oxopropanoyl)(methyl)amino]-2,2-dimethylpropanoate

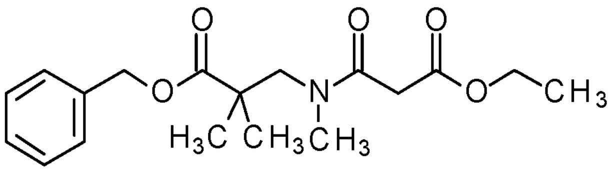

Benzyl 2,2-dimethyl-3-(methylamino)propanoate (see Intermediate 135, 2.87 g, 80 % purity) was dissolved in 9.9 mL dichloromethane and cooled down to 0°C. Then N,N-diisopropylethylamine (2.0 mL, 11 mmol) and 4-dimethylaminopyridine (127 mg, 1.04 mmol) were added. Ethyl 3-chloro-3-oxopropanoate (1.5 mL, 11 mmol) was added dropwise at t<15°C. The reaction mixture was stirred under nitrogen atmosphere for 1.5 h at 0° C and then over night at rt. The reaction mixture was diluted with half saturated ammonium chloride solution. The aqueous layer was extracted with dichloromethane three times. The combined organic layers were washed with brine, filtered through a water resistant filter and concentrated under reduced pressure. The resulting crude product was combined with a batch of the same reaction starting from benzyl 2,2-dimethyl-3-(methylamino)propanoate (see Intermediate 135, 1.38 g, 47% purity) and purified by chromatography (KP silica ULTRA column 50g; gradient: dichloromethane/ ethanol 0-10%) to provide target compound containing fractions: 3.1 g and 1.4 g both in 33% purtiy.

LC-MS (Method 1): $R_t$ = 1.11 min; MS (ESIpos): m/z = 337 $[M+H]^+$

$^1$H-NMR (400 MHz, DMSO-d6) δ [ppm]: 1.04 - 1.24 (m, 9 H) 2.85 (s, 3 H) 3.50 (d, 4 H) 3.96 - 4.18 (m, 2 H) 5.07 (s, 2 H) 7.13 - 7.48 (m, 5 H).

**Intermediate 137**

3-[(3-ethoxy-3-oxopropanoyl)(methyl)amino]-2,2-dimethylpropanoic acid

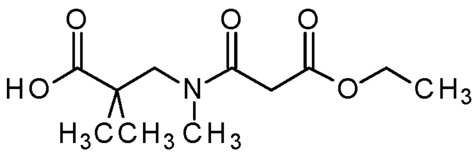

Benzyl 3-[(3-ethoxy-3-oxopropanoyl)(methyl)amino]-2,2-dimethylpropanoate (see Intermediate 136, 3.71 g) was dissolved in 190 mL ethanol and palladium/carbon (3.24 g, 10 % purity, 3.04 mmol) was added under argon atmosphere. The mixture was stirred for 5 min at room temperature. The atmosphere was changed to hydrogen by four alternating evacuation/refill cycles. The reaction mixture was stirred for 2 h at room temperature. The reaction mixture was filtered through celite and the resulting filter cake was washed with ethanol twice. After partial evaporation of the solvent the residual solution was filtered through a 0,20 μm syringe filter to get rid of the remaining catalyst. The filtrate

was concentrated under reduced pressure. The crude product was used for the following reaction withour further prurifications: 3.27 g, 83% purtiy.
LC-MS (Method 1): $R_t$ = 0.84 min; MS (ESIpos): m/z = 246 $[M+H]^+$

## Intermediate 138

ethyl 3-[(3-ethoxy-3-oxopropanoyl)(methyl)amino]-2,2-dimethylpropanoate

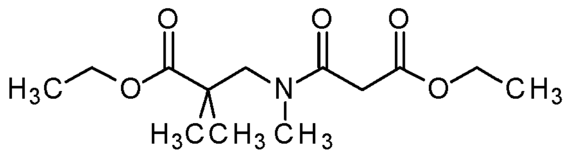

3-[(3-Ethoxy-3-oxopropanoyl)(methyl)amino]-2,2-dimethylpropanoic acid (see Intermediate 137, 3.27 g, 83 % purity) was suspended in 45 mL ethanol. Thionyl chloride (1.4 mL, 19 mmol) was added drop-wise and the mixture was stirred for 2h under reflux. The reaction mixture was combined with a batch of the same reaction starting from 3-[(3-ethoxy-3-oxopropanoyl)(methyl)amino]-2,2-dimethylpropanoic acid (see Intermediate 137, 190 mg) and concentrated under reduced pressure. The residue was diluted with aqueous saturated sodium hydrogencarbonate solution and extracted with ethyl acetate three times. The combined organic layers were washed with water, filtered through a water resistant filter and concentrated under reduced pressure. The crude product was purified by chromatography (KP silica ULTRA column 25g; gradient: hexane/ethyl acetate 0-50%) to provide the target compound in 90% purity: 2.1 g.

LC-MS (Method 1): $R_t$ = 0.97 min; MS (ESIpos): m/z = 275 $[M+H]^+$

$^1$H-NMR (400 MHz, DMSO-d6) $\delta$ [ppm] = 1.09 (s, 6 H) 1.15 - 1.25 (m, 6 H) 2.89 (s, 3 H) 3.43 - 3.53 (m, 4 H) 3.92 - 4.15 (m, 4 H).

## Intermediate 139

ethyl (rac)-1,5,5-trimethyl-2,4-dioxopiperidine-3-carboxylate (Racemate)

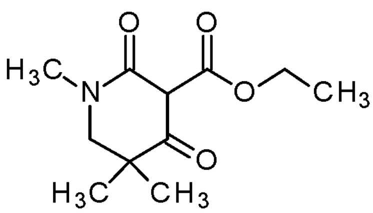

Sodium ethoxide (2.0 mL, 21 % purity in ethanol, 8.4 mmol) was cooled down with an ice bath. Ethyl 3-[(3-ethoxy-3-oxopropanoyl)(methyl)amino]-2,2-dimethylpropanoate (see Intermediate 138, 2.09 g) was dissolved in 11 mL ethanol and added drop wise (t<5°C) to the sodium ethoxide solution. The mixture was stirred at approx. 0°C for 30 min and at room temperature over night under nitrogen atmosphere. By the addition of hydrochloric acid (4 M) the pH of the reaction mixture was adjusted to 1. The aqueous mixture was extracted with dichloromethane twice. The combined organic layers were filtered through a water resistant filter and concentrated under reduced pressure to provide the target compound in 95% purity: 1.5 g.

LC-MS (Method 1): Rt = 0.91 min; MS (ESIpos): m/z = 229 $[M+H]^+$

$^1$H-NMR (400 MHz, DMSO-d6) $\delta$ [ppm] = 1.08 (s, 6 H) 1.23 (t, 3 H) 2.93 (s, 3 H) 3.20 (s, 2 H) 4.06 - 4.16 (m, 1 H) 4.22 (q, 2 H). - contains impurities.

**Intermediate 140**

1,5,5-trimethylpiperidine-2,4-dione

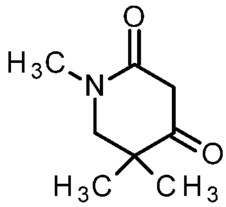

Ethyl (rac)-1,5,5-trimethyl-2,4-dioxopiperidine-3-carboxylate (Racemate) (see Intermediate 139, 1.49 g) was dissolved in 17 mL acetonitrile and 1.8 mL water and was treated with hydrochloric acid (12 ml, 2.0 M, 25 mmol). It was stirred for 2 h under reflux. The reaction mixture was filtered through a water resistant filter and concentrated under reduced pressure to provide the crude product which was used without further purification: 1.03 g (54% purity).

LC-MS (Method 1): Rt = 0.58 min; MS (ESIpos): m/z = 156 $[M+H]^+$

$^1$H-NMR (400 MHz, DMSO-d6) $\delta$ [ppm] = 11.02 (s, 6 H) 2.92 (s, 3 H) 3.33 (s, 2 H) 3.37 (s, 2 H). - contains impurities.

**Intermediate 141**

2-(2-chloropyridin-4-yl)-5,7,7-trimethyl-1,5,6,7-tetrahydro-4H-pyrrolo[3,2-c]pyridin-4-one

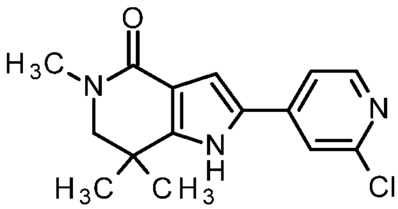

1,5,5-Trimethylpiperidine-2,4-dione (see Intermediate 140, 1.03 g), 2-bromo-1-(2-chloropyridin-4-yl)ethan-1-one (1.56 g, 6.64 mmol) and ammonium acetate (1.53 g, 19.9 mmol) were dissolved in 49 mL propanol stirred at 120 °C for 4 hours under argon atmosphere. The reaction mixture was concentrated under reduced pressure and treated with dichloromethane and water. Between both layers a yellow precipitate was formed. It was filtered off, washed with water and dichloromethane and dried at 50°C under vacuo to provide batch 1 of the target compound in 98% purity: 697 mg. The filtrate was extracted with dichloromethane three times, the combined organic layers were washed with water and brine, filtered through a silicone coated filter and concentrated under reduced pressure. The crude product was treated with dichloromethane and sonicated. The undissolved precipitate was filtered off, washed with water and dichloromethane and dried at 50°C under vacuo to provide batch 2 of the target compound in 98% purity: 184 mg. Filtrate II was suspended in ~1 mL dichloromethane and diluted with MTBE. The formed precipitate was filtered off, washed with water and dichloromethane and dried at 50°C under vacuo to provide batch 3 of the target compound in 76% purity: 73 mg. The batches were combined and used for the following reaction.

batch 1:

LC-MS (Method 2): Rt = 0.90 min; MS (ESIpos): m/z = 290 $[M+H]^+$
$^1$H-NMR (400 MHz, DMSO-d6) $\delta$ [ppm] = 1.32 (s, 6 H), 2.93 (s, 3 H), 3.31 (s, 2 H), 7.13 (d, 1 H), 7.71 (dd, 1 H), 7.84 (d, 1 H), 8.29 (d, 1 H), 11.58 (br s, 1 H).

**Intermediate 142**

(rac)-4,4-difluoro-2-(4-fluorophenyl)-N-[4-(5,7,7-trimethyl-4-oxo-4,5,6,7-tetrahydro-1H-pyrrolo[3,2-c]pyridin-2-yl)pyridin-2-yl]butanamide (Racemate)

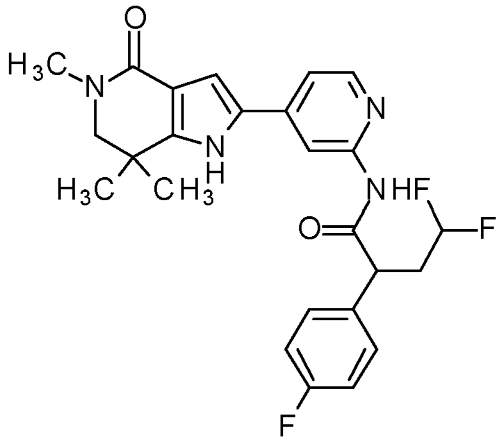

2-(2-Chloropyridin-4-yl)-5,7,7-trimethyl-1,5,6,7-tetrahydro-4H-pyrrolo[3,2-c]pyridin-4-one (see Intermediate 141, 954 mg), (rac)-4,4-difluoro-2-(4-fluorophenyl)butanamide (Racemate) (see Intermediate 123, 1.07 g), di-tert-butyl(2',4',6'-triisopropyl-3,6-dimethoxy-[1,1'-biphenyl]-2-yl)phosphine (319 mg, 658 µmol; CAS-RN:[1160861-53-9]), [(2-di-tert-butylphosphino-3,6-dimethoxy-2',4', 6'-triisopropyl-1,1'-biphenyl)-2-(2'-amino-1,1'-biphenyl)]palladium(II) methanesulfonate (281 mg, 329 µmol; CAS-RN:[1536473-72-9]) and cesium carbonate (3.22 g, 9.88 mmol) were dissolved in 28 mL 1,4-dioxane and stirred at 100 °C over night under argon atmosphere. The reaction mixture was diluted with dichloromethane and water. It was extracted with dichloromethane three times, the combined organic layers layer were washed with water and brine, filtered through a silicone coated filter and concentrated under reduced pressure. The crude product was purified by flash chromatography (25g column, silica ULTRA; dichloromethane / ethanol 0%-10%) to provide the target compound in 97% purity, batch 1: 512 mg. Further target compound containing fractions were further purified by HPLC to provide the target compound in 97% purity, batch 2: 238 mg. The batches were combined in used in the following reation.

batch 1:

LC-MS (Method 2): Rt = 1.14 min; MS (ESIpos): m/z = 471 $[M+H]^+$
$^1$H-NMR (400 MHz, DMSO-d6) δ [ppm] = 1.32 (d, 6 H), 2.12 - 2.31 (m, 1 H), 2.70 - 2.83 (m, 1 H), 2.92 (s, 3 H), 3.29 (s, 2 H), 4.21 (dd, 1 H), 5.83 - 6.23 (m, 1 H), 6.80 (d, 1 H), 7.13 - 7.26 (m, 2 H), 7.40 (dd, 1 H), 7.47 - 7.55 (m, 2 H), 8.18 - 8.30 (m, 2 H), 10.78 (s, 1 H), 11.61 (br d, 1 H). - contains ethanol and impurities in the aromatic range.

batch 2:

$^1$H-NMR (400 MHz, DMSO-d6) δ [ppm] = 1.32 (d, 6 H), 2.12 - 2.31 (m, 1 H), 2.70 - 2.83 (m, 1 H), 2.92 (s, 3 H), 3.29 (s, 2 H), 4.21 (dd, 1 H), 5.83 - 6.23 (m, 1 H), 6.80 (d, 1 H), 7.13 - 7.26 (m, 2 H), 7.40 (dd, 1 H), 7.47 - 7.55 (m, 2 H), 8.18 - 8.30 (m, 2 H), 10.78 (s, 1 H), 11.61 (br d, 1 H).

**Intermediate 143**

(rac)-N-[4-(3-bromo-5,7,7-trimethyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate)

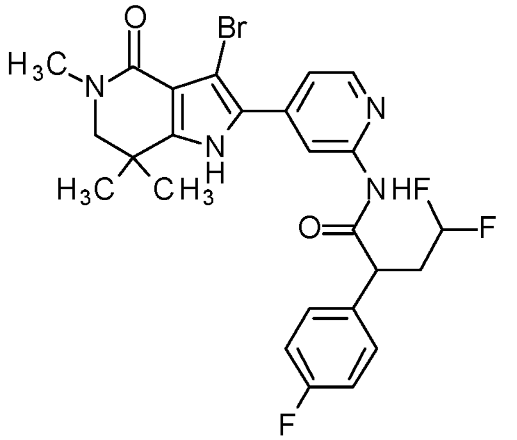

(rac)-4,4-Difluoro-2-(4-fluorophenyl)-N-[4-(5,7,7-trimethyl-4-oxo-4,5,6,7-tetrahydro-1H-pyrrolo[3,2-c]pyridin-2-yl)pyridin-2-yl]butanamide (Racemate) (see Intermediate 142, 1.29 g) was dissolved in 25 mL DMF and cooled down with an ice bath. N-bromo succinimide (734 mg, 4.13 mmol) was added and it was stirred at 0°C under argon atmosphere for 1 hour. To the reaction mixture aqueous saturated sodium hydrogencarbonate solution and aqueous saturated sodium thiosulfate solution were added. It was stirred for 1 hour, the formed precipitate was filtered off, washed with water and dried at 50°C under vacuo to provide the crude product which was purified by HPLC: 73 mg, analytically pure.

LC-MS (Method 2): Rt = 1.16 min; MS (ESIpos): m/z = 549 $[M+H]^+$

$^1$H-NMR (400 MHz, DMSO-d6) δ [ppm] = .29 (d, 6 H), 2.15 - 2.31 (m, 1 H), 2.70 - 2.87 (m, 1 H), 2.92 (s, 3 H), 3.29 (s, 2 H), 4.22 (dd, 1 H), 5.85 - 6.21 (m, 1 H), 7.13 - 7.29 (m, 2 H), 7.38 (dd, 1 H), 7.48 - 7.55 (m, 2 H), 8.34 (dd, 1 H), 8.44 (s, 1 H), 10.88 (s, 1 H), 11.90 (s, 1 H). - contains dichloromethane.

## Intermediate 144

N-(4-bromopyridin-2-yl)acetamide

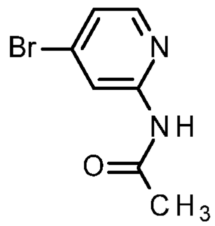

4-Bromopyridine-2-amine (200 g, 1.16 mol, 1 eq, CAS-RN: [84249-14-9]) was dissolved in 1500 mL of dichloromethane. Acetic anhydride (163.9 mL, 1.73 mol, 1.5 eq, CAS-RN: [108-24-7]) was added in one portion. The reaction mixture was allowed to stir at 25 °C for 12 h. The reaction mixture was washed with water (500 mL). The organic layer was separated and concentrated under reduced pressure to give the crude product as a white solid, which was used without further purification: 250 g, 1.16 mmol, quantitative yield.
$^1$H-NMR (400 MHz, DMSO-d6) δ [ppm] = 10.72 (br s, 1H), 8.32 (d, J = 1.6 Hz, 1H), 8.21 (d, J = 5.4 Hz, 1H), 7.35-7.34 (m, 1H), 2.11 (s, 3H)

## Intermediate 145

N-[4-(1-ethoxyvinyl)pyridin-2-yl]acetamide

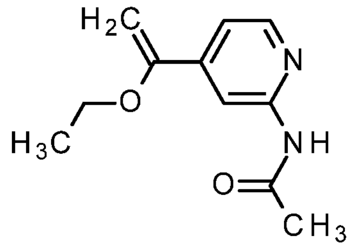

N-(4-bromopyridin-2-yl)acetamide (100 g, 465 mmol, 1 eq) was dissolved in 1000 mL of DMF. To the resulting mixture was added tributyl(1-ethoxyvinyl)stannane (184 g, 511 mmol, 1.1 eq, CAS-RN: [97674-02-7]) in one portion. Bis(triphenylphosphine)palladium(II) dichloride (6.53 g, 9.30 mmol, 2 mol %, CAS-RN: [13965-03-2]) was then added in one portion. The resulting mixture was degassed with nitrogen by three sequential evacuation and backfill cycles and then stirred at 70 °C for 12 h. On completion, the reaction was poured into 1000 mL of an aqueous saturated solution of potassium fluoride. The resulting solution was extracted with ethyl acetate (1000 mL x 3). The combined organic layers were dried (sodium sulfate), filtered, and concentrated in vacuo to provide the crude residue. The residue was purified by flash normal phase column chromatography ($SiO_2$, petroleum ether/ethyl acetate=100/1 to 1/1) to provide the title compound in 90% purity: 50 g, 218 mmol, 46.92 % yield.

$^1$H-NMR (400 MHz, $CDCl_3$) $\delta$ [ppm] = 8.70 (br s, 1H), 8.46 (br s, 1H), 8.23 (d, J = 5.2 Hz, 1H), 7.31- 7.28 (m, 1H), 4.91 (d, J = 3.2 Hz, 1H), 4.39 (d, J = 3.2 Hz, 1H), 3.94 (q, J = 7.2 Hz, 2H), 2.23 (s, 3H), 1.45 (t, J = 7.2 Hz, 3H).

## Intermediate 146

N-[4-(bromoacetyl)pyridin-2-yl]acetamide

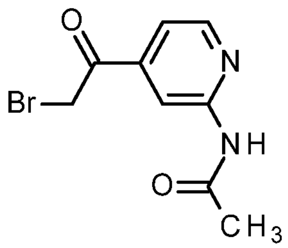

N-[4-(1-ethoxyvinyl)-2-pyridyl]acetamide (41 g, 198 mmol, 1 eq) was dissolved in 400 mL of THF and 40 mL of water and cooled to 0 °C. *N*-Bromosuccinimide (38.9 g, 218 mmol, 1.1 eq, CAS-RN: [128-08-5]) was added in portions at 0 °C. Once all NBS was added, the mixture was allowed to warm to 25 °C and stirred for 1 h. The mixture was then washed with 20 mL of water, and extracted with ethyl acetate (400 mL x 2). The combined organic layers were concentrated *in vacuo* to provide a crude residue. The crude residue was purified by normal phase flash column chromatography ($SiO_2$, petroleum ether/ethyl acetate=100/1 to 1/1). The material was still impure, and was used as a crude product: 55 g, impure.

$^1$H-NMR (400 MHz, DMSO-d6) $\delta$ [ppm] = 11.06 (br s, 1H), 8.55-8.51 (m, 1H), 8.51 (s, 1H), 7.60-7.59 (m, 1H), 4.94 (s, 2H), 2.13 (s, 3H).

## Intermediate 147

N-[4-(5-cyclopropyl-4-oxo-4,5,6,7-tetrahydro-1H-pyrrolo[3,2-c]pyridin-2-yl)pyridin-2-yl]acetamide

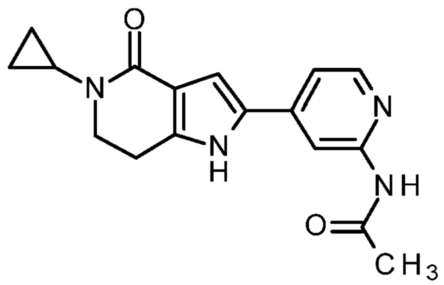

1-Cyclopropylpiperidine-2,4-dione (34.0 g, 222 mmol, 1.1 eq, CAS-RN: [1501330-86-4]) and N-[4-(bromoacetyl)pyridin-2-yl]acetamide (52 g, 202.27 mmol, 1 eq) were dissolved in 350 mL of ethanol. Ammonium acetate (62.3g, 809 mmol, 4 eq, CAS-RN: [631-61-8]) was added in one portion, and the resulting mixture was stirred at 25 °C for 2 h. The mixture was then filtered, and the precipitated solid was washed with 50 mL of EtOH. The filtrate was collected and dried under reduced pressure to provide the target compound in 95% purity: 40 g, 122 mmol, 60.53 % yield.

$^1$H NMR (400 MHz, DMSO-d6) $\delta$ = 12.18 - 11.62 (m, 1H), 10.42 (s, 1H), 8.29 - 8.15 (m, 2H), 7.32-7.31 (m, 1H), 6.85 (s, 1H), 3.54-3.51 (m, 2H), 2.88-2.86 (m, 2H), 2.65 - 2.55 (m, 1H), 2.11 (s, 3H), 0.79 - 0.67 (m, 2H), 0.62 - 0.50 (m, 2H).

## Intermediate 148

N-[4-(5-cyclopropyl-3-iodo-4-oxo-4,5,6,7-tetrahydro-1H-pyrrolo[3,2-c]pyridin-2-yl)pyridin-2-yl]acetamide

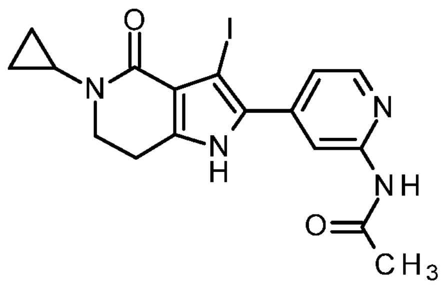

N-[4-(5-cyclopropyl-4-oxo-4,5,6,7-tetrahydro-1H-pyrrolo[3,2-c]pyridin-2-yl)pyridin-2-yl] acetamide (30 g, 96.6 mmol, 1 eq) was dissolved in 150 mL of DMF. A solution of *N*-Iodosuccinimide (23.9 g, 106.33 mmol, 1.1 eq, CAS-RN: [516-12-1]) in 150 mL of DMF was added by dropwise addition. The resulting mixture was stirred at 25° C for 2 h. The mixture was washed with 150 mL of water, and the aqueous solution extracted with ethyl acetate (500 mL x 2). The combined organic layers were concentrated *in vacuo* to provide 50 g of the target compound as a crude residue, which was used without further purification.

$^1$H NMR (400 MHz, DMSO-d6) $\delta$ = 12.21 (s, 1H), 10.53 (s, 1H), 8.48 (s, 1H), 8.32 (d, J = 6.4 Hz, 1H), 7.38-7.37 (m, 1H), 3.52-3.50(m, 2H), 2.89 - 2.84 (m, 2H), 2.64 - 2.56 (m, 1H), 2.12 (s, 3H), 0.77 - 0.67 (m, 2H), 0.61 - 0.53 (m, 2H).

## Intermediate 149

N-[4-(5-cyclopropyl-3-iodo-4-oxo-4,5-dihydro-1H-pyrrolo[3,2-c]pyridin-2-yl)pyridin-2-yl]acetamide

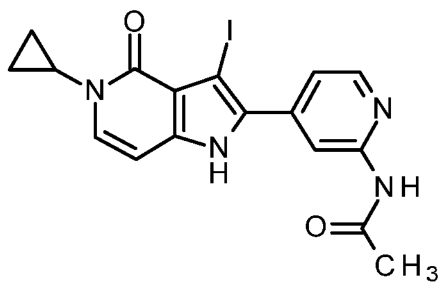

N-[4-(5-cyclopropyl-3-iodo-4-oxo-4,5,6,7-tetrahydro-1H-pyrrolo[3,2-c]pyridin-2-yl)pyridin-2-yl] acetamide (50 g, 114 mmol, 1 eq) was dissolved in 500 mL of 1,4-dioxane. 2,3-Dichloro-5,6-dicyano-p-benzoquinone (39.0 g, 171 mmol, 1.5 eq, CAS-RN: [84-58-2]) was added in one portion, and the resulting mixture was stirred at 60 °C for 12 h. The resulting mixture was quenched by the addition of 500 mL of a saturated aqueous solution of NaHCOs. The resulting mixture was extracted with ethyl acetate (500 mL x 3). The combined organic layers were concentrated *in vacuo* to give 16 g of the target compound in 60% purity, containing 15% recovered intermediate 148, which was used without further purification.

$^1$H NMR (400 MHz, DMSO-d6) δ = 12.37 - 12.24 (m, 1H), 10.64 (s, 1H), 8.54 (s, 1H), 8.40 (d, J = 5.2 Hz, 1H), 7.47 - 7.45 (m, 1H), 7.28 (d, J = 7.6 Hz, 1H), 6.39 (d, J = 7.6 Hz, 1H), 3.28 - 3.22 (m, 1H), 2.14 - 2.12 (m, 3H), 1.07 - 0.94 (m, 2H), 0.85 - 0.76 (m, 2H).

## Intermediate 150

N-[4-(5-cyclopropyl-4-oxo-3-phenyl-4,5-dihydro-1H-pyrrolo[3,2-c]pyridin-2-yl)pyridin-2-yl]acetamide

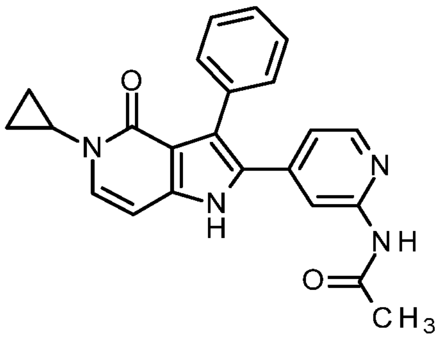

N-[4-(5-cyclopropyl-3-iodo-4-oxo-4,5-dihydro-1H-pyrrolo[3,2-c]pyridin-2-yl)pyridin-2-yl] acetamide (2.0 g, 4.6 mmol, 1 *eq*, Intermediate 149, 60% purity, containing 15% intermediate 148) was dissolved in 4 mL of water and 20 mL of 1,4-dioxane. Phenylboronic acid (672 mg, 5.5 mmol, 1.2 eq, CAS-RN: [98-80-6]), potassium carbonate (1.91 g, 10.4 mmol, 2.25 *eq*, CAS-RN: [584-08-7]), and [1,1'-Bis(diphenylphosphino) ferrocene]dichloropalladium(II) (337 mg, 0.46 mmol, 0.1 *eq*, CAS-RN: [72287-26-4]) were added in sequence. The resulting mixture was stirred at 80 °C for 12 h under a nitrogen atmosphere. Upon completion, the mixture was cooled to 25° C and quenched by the addition of 40 mL of water. The resulting mixture was extracted with ethyl acetate (50 mL x 3), and the combined organic layers were washed with brine (50 mL x 3), dried (sodium sulfate), and concentrated *in vacuo.* The resulting residue was purified by preparative HPLC (column: YMC-Triart Prep C18 250 mm*50 mm*10 mm; mobile phase: [water (0.225% FA)-ACN]; B%: 15%-40%, 20 min) to provide the target compound as a yellow solid: 1.2 g, 3.12 mmol.

$^1$H NMR (400 MHz, DMSO-$d_6$) δ = 12.01 (br s, 1H), 10.43 (s, 1H), 8.12 (s, 1H), 8.07 (d, *J* = 5.2 Hz, 1H), 7.33 - 7.22 (m, 7H), 6.67 (dd, *J* = 1.2, 5.2 Hz, 1H), 6.41 (d, *J* = 7.2 Hz, 1H), 3.19 (dt, *J* = 3.6, 7.2 Hz, 1H), 2.06 (s, 3H), 1.01 - 0.88 (m, 2H), 0.83 - 0.72 (m, 2H).

## Intermediate 151

N-[4-(5-cyclopropyl-4-oxo-3-phenyl-4,5,6,7-tetrahydro-1H-pyrrolo[3,2-c]pyridin-2-yl)pyridin-2-yl]acetamide

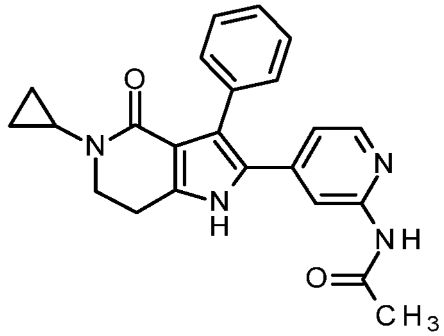

N-[4-(5-cyclopropyl-3-iodo-4-oxo-4,5-dihydro-1H-pyrrolo[3,2-c]pyridin-2-yl)pyridin-2-yl] acetamide (2.0 g, 4.6 mmol, 1 *eq*, Intermediate 149, 60% purity, containing 15% intermediate 148) was dissolved in 4 mL of water and 20 mL of 1,4-dioxane. Phenylboronic acid (672 mg, 5.5 mmol, 1.2 *eq,* CAS-RN: [98-80-6]), potassium carbonate (1.91 g, 10.4 mmol, 2.25 *eq,* CAS-RN: [584-08-7]), and [1,1'-Bis(diphenylphosphino) ferrocene]dichloropalladium(II) (337 mg, 0.46 mmol, 0.1 *eq,* CAS-RN: [72287-26-4]) were added in sequence. The resulting mixture was stirred at 80 °C for 12 h under a nitrogen atmosphere. Upon completion, the mixture was cooled to 25° C and quenched by the addition of 40 mL of water. The resulting mixture was extracted with ethyl acetate (50 mL x 3), and the combined organic layers were washed with brine (50 mL x 3), dried (sodium sulfate), and concentrated *in vacuo.* The resulting residue was purified by preparative HPLC (column: YMC-Triart Prep C18 250 mm*50 mm*10 mm; mobile phase: [water (0.225% FA)-ACN]; B%: 15%-40%, 20 min) to provide the target compound, N-[4-(5-cyclopropyl-4-oxo-3-phenyl-4,5,6,7-tetrahydro-1H-pyrrolo[3,2-c]pyridin-2-yl)pyridin-2-yl]acetamide as a yellow solid: 300 mg, 0.77 mmol.
LC-MS (Method 7): Rt = 0.442 min; MS (ESIpos): m/z = 387.2 $[M+H]^+$

<u>**Intermediate 152**</u>

2-(2-aminopyridin-4-yl)-5-cyclopropyl-3-phenyl-1,5-dihydro-4H-pyrrolo[3,2-c]pyridin-4-one

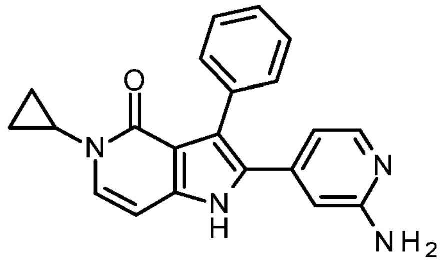

N-[4-(5-cyclopropyl-4-oxo-3-phenyl-4,5-dihydro-1H-pyrrolo[3,2-c]pyridin-2-yl)pyridin-2-yl]acetamide (1.2 g, 3.12 mmol, 1 *eq*) was dissolved in 20 mL of methanol. Sodium hydroxide (249 mg, 6.24 mmol, 2 *eq,* CAS-RN: [1310-73-2]) was added in one portion. The resulting mixture was stirred at 80 °C for 2 h. On completion, the mixture was cooled to 25 °C, concentrated *in vacuo,* and diluted with 20 mL of water. The solution was extracted with ethyl acetate (20 mL x 3). The combined organic layers were washed with brine (20 mL x 3), dried (sodium sulfate), filtered, and concentrated *in vacuo* to provide the target compound, which was used without further purification: 1.2 g, crude material.

$^1$H NMR (400 MHz, DMSO-d6) $\delta$ = 11.85 (br d, J = 4.0 Hz, 1H), 7.72 (d, J = 5.2 Hz, 1H), 7.29 (s, 5H), 7.23 (d, J = 7.2 Hz, 1H), 6.40 - 6.33 (m, 2H), 6.18 (d, J = 5.2 Hz, 1H), 5.85 (s, 2H), 3.23 - 3.12 (m, 1H), 1.00 - 0.85 (m, 2H), 0.83 - 0.69 (m, 2H).

<u>**Intermediate 153**</u>

2-(2-aminopyridin-4-yl)-5-cyclopropyl-3-phenyl-1,5,6,7-tetrahydro-4H-pyrrolo[3,2-c]pyridin-4-one

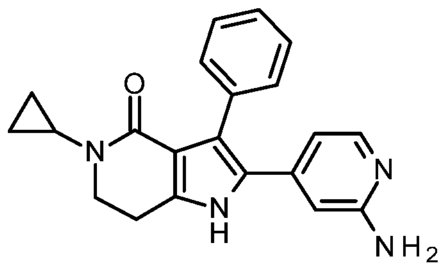

N-[4-(5-cyclopropyl-4-oxo-3-phenyl-4,5,6,7-tetrahydro-1H-pyrrolo[3,2-c]pyridin-2-yl)pyridin-2-yl]acetamide (300 mg, 0.77 mmol, 1 eq) was dissolved in 4 mL of methanol. Sodium hydroxide (62 mg, 1.55 mmol, 2 eq, CAS-RN: [1310-73-2]) was added. The resulting mixture was stirred at 80 °C for 2 h. On completion, the mixture was cooled to 25 °C, concentrated *in vacuo,* and diluted with 20 mL of water. The solution was extracted with ethyl acetate (20 mL x 3). The combined organic layers were washed with brine (20 mL x 3), dried (sodium sulfate), filtered, and concentrated *in vacuo* to provide the target compound, which was used without further purification: 150 mg, crude material.
LC-MS (Method 7): Rt = 0.995 min; MS (ESIpos): m/z = 345.1 $[M+H]^+$

## Intermediate 154

N-{4-[5-cyclopropyl-3-(4-fluorophenyl)-4-oxo-4,5,6,7-tetrahydro-1H-pyrrolo[3,2-c]pyridin-2-yl]pyridin-2-yl}acetamide

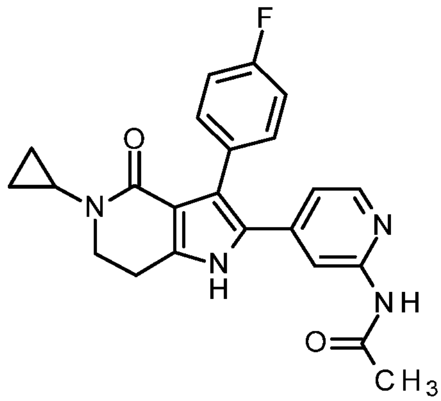

N-[4-(5-cyclopropyl-3-iodo-4-oxo-4,5,6,7-tetrahydro-1H-pyrrolo[3,2-c]pyridin-2-yl)pyridin-2-yl]acetamide (1.51 g, 3.45 mmol, 1 eq, see Intermediate 148) was dissolved in 15 mL of 1,4-dioxane and 3 mL of water. (4-Fluorophenyl)boronic acid (580 mg, 4.15 mmol, 1.2 eq, CAS-RN: [1765-93-1]), potassium carbonate (1.43 g, 10.3 mmol, 3 eq, CAS-RN: [584-08-7]), and [1,1'-Bis(diphenylphosphino)ferrocene]dichloropalladium(II) (252 mg, 0.345 mmol, 0.1 eq, CAS-RN: [72287-26-4]) were added in sequence. The resulting mixture was stirred at 80 °C for 16 h under a nitrogen atmosphere. On completion, the mixture was quenched with 50 mL of water and extracted with ethyl acetate (50 mL x 3). The combined organic layers were washed with brine (50 mL x 3), dried (sodium sulfate), filtered, and concentrated *in vacuo.* The crude residue was purified by silica gel column chromatography (petroleum ether/ethyl acetate=3/1-1/1) to give the target compound as a yellow solid: 3.2 g, 7.91 mmol, 76% yield.

$^1$H NMR (400 MHz, DMSO-$d_6$) $\delta$ = 11.84 (s, 1H), 10.34 (s, 1H), 8.10 - 7.95 (m, 2H), 7.24 - 7.17 (m, 2H), 7.15 - 7.03 (m, 2H), 6.64 (dd, *J* = 1.6, 5.2 Hz, 1H), 3.54 (t, *J* = 6.4 Hz, 2H), 2.88 (t, *J* = 6.4 Hz, 2H), 2.04 (s, 3H), 0.72 - 0.64 (m, 2H), 0.58 - 0.50 (m, 2H).

## Intermediate 155

N-{4-[5-cyclopropyl-3-(4-fluorophenyl)-4-oxo-4,5-dihydro-1H-pyrrolo[3,2-c]pyridin-2-yl]pyridin-2-yl}acetamide

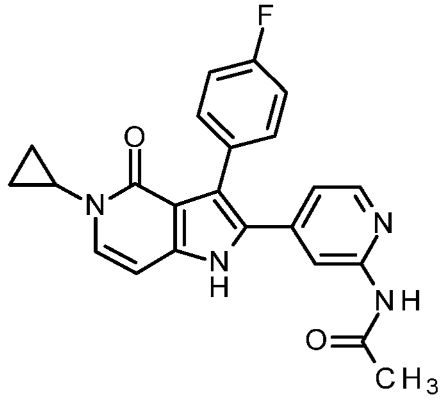

and

**Intermediate 156**

N-{4-[7-chloro-5-cyclopropyl-3-(4-fluorophenyl)-4-oxo-4,5-dihydro-1H-pyrrolo[3,2-c]pyridin-2-yl]pyridin-2-yl}acetamide

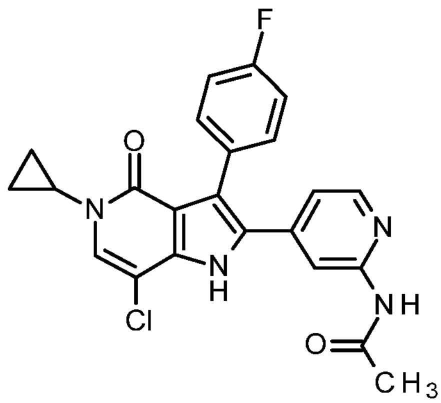

N-{4-[5-cyclopropyl-3-(4-fluorophenyl)-4-oxo-4,5,6,7-tetrahydro-1H-pyrrolo[3,2-c]pyridin-2-yl]pyridin-2-yl}acetamide (1 g, 2.47 mmol, 1 eq) was dissolved in 15 mL of 1,4-dioxane. 2,3-dichloro-5,6-dicyano-p-benzoquinone (841 mg, 3.71 mmol, 1.5 eq, CAS-RN: [84-58-2]) was added in one portion, and the resulting mixture was stirred at 60 °C for 16 h. On completion, the mixture was quenched by the addition of 20 mL of a saturated aqueous solution of $NaHCO_3$, and the resulting mixture extracted with ethyl acetate (20 mL x 3). The combined organic layers were washed with brine (20 mL x 3), dried (sodium sulfate), filtered, and concentrated *in vacuo.* The crude residue was purified by reverse phase preparative HPLC (column: YMC-Triart Prep C18 250mm * 50mm * 10um; mobile phase: [water (0.225% FA)-ACN]; B%: 13%-43%, 20 min) to provide

Intermediate 155:

300 mg (745.5 µmol, 30% yield) of target compound N-{4-[5-cyclopropyl-3-(4-fluorophenyl)-4-oxo-4,5-dihydro-1H-pyrrolo[3,2-c]pyridin-2-yl]pyridin-2-yl}acetamide (Intermediate 155)

LC-MS (Method 7): Rt = 1.137 min; MS (ESIpos): m/z = 403.2 $[M+H]^+$

and

Intermediate 156:

500 mg (1.14 mmol, 46% yield) of target compound N-{4-[7-chloro-5-cyclopropyl-3-(4-fluorophenyl)-4-oxo-4,5-dihydro-1H-pyrrolo[3,2-c]pyridin-2-yl]pyridin-2-yl}acetamide (Intermediate 156) as a brown solid:

$^1H$ NMR (400 MHz, DMSO-$d_6$) δ = 12.33 (s, 1H), 10.53 (s, 1H), 8.17 (d, *J* = 5.2 Hz, 1H), 8.04 (s, 1H), 7.49 (s, 1H), 7.27 (br dd, *J* = 5.6, 8.4 Hz, 2H), 7.12 (br t, *J* = 8.8 Hz, 2H), 6.89 - 6.84 (m, 1H), 3.24 - 3.18 (m, 1H), 2.06 (s, 3H),

1.01 - 0.89 (m, 2H), 0.88 - 0.80 (m, 2H)

**Intermediate 157**

2-(2-aminopyridin-4-yl)-7-chloro-5-cyclopropyl-3-(4-fluorophenyl)-1,5-dihydro-4H-pyrrolo[3,2-c]pyridin-4-one

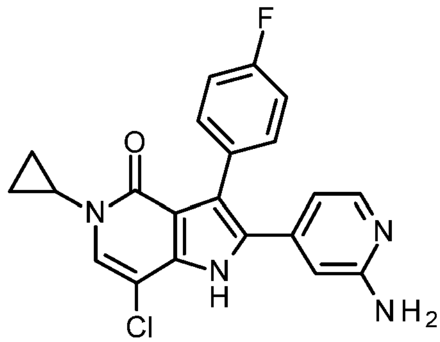

N-{4-[7-chloro-5-cyclopropyl-3-(4-fluorophenyl)-4-oxo-4,5-dihydro-1H-pyrrolo[3,2-c]pyridin-2-yl]pyridin-2-yl}acetamide (200 mg, 457 umol, 1 *eq*) was dissolved in 2 mL MeOH. Sodium hydroxide (36.6 mg, 915 μmol, 2 *eq*, CAS-RN: [1310-73-2]) was added in one portion. The resulting mixture was stirred at 80 °C for 2 h. On completion, the mixture was concentrated *in vacuo* and suspended in 2 mL of water. The resulting mixture was extracted with ethyl acetate (2 mL x 3). The combined organic layers were washed with brine (2 mL x 3), dried (sodium sulfate), filtered, and concentrated *in vacuo* to give 100 mg of the target compound as a yellow solid, which was used without further purification.

**Intermediate 158**

N-{4-[5-cyclopropyl-4-oxo-3-(pyridin-2-yl)-4,5,6,7-tetrahydro-1H-pyrrolo[3,2-c]pyridin-2-yl]pyridin-2-yl}acetamide

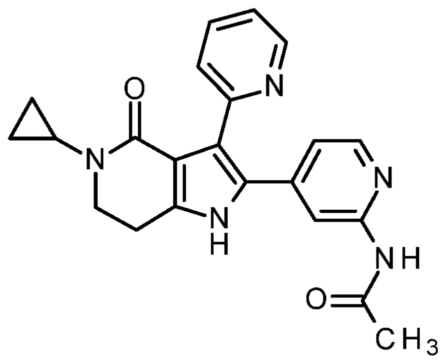

N-[4-(5-cyclopropyl-3-iodo-4-oxo-4,5,6,7-tetrahydro-1H-pyrrolo[3,2-c]pyridin-2-yl)pyridin-2-yl]acetamide (1 g, 2.29 mmol, 1 eq, see Intermediate 148) was dissolved in 20 mL of DMF. Tributyl(2-pyridyl)stannane (1.69 g, 4.58 mmol, 2 eq, CAS-RN: [17997-47-6]), tetrakis(triphenylphosphine)-palladium(0) (264 mg, 229 μmol, 0.1 eq, CAS-RN: [14221-01-3]), and lithium chloride (106 mg, 2.52 mmol, 1.1 eq, CAS-RN: [7447-41-8]) were added in sequence. The resulting mixture was stirred at 100 °C for 12 h under a nitrogen atmosphere. Upon completion, the mixture was quenched by addition of 80 mL of water, and the resulting mixture was extracted with ethyl acetate (80 mL x 3). The combined organic layers were washed with brine (80 mL x 3), dried (sodium sulfate), filtered, and concentrated *in vacuo.* The residue was purified by reverse phase preparative HPLC (column: Phenomenex luna C18 250mm * 50mm * 15um; mobile phase: [water (0.225% FA)-ACN]; B%: 1%-20%, 23 min) to provide the target compound as a yellow solid in 95% purity: 680 mg, 1.67 mmol, 18% yield.

$^1H$ NMR (400 MHz, DMSO-$d_6$) $\delta$ = 11.93 (br s, 1H), 10.41 (s, 1H), 8.30 - 8.16 (m, 2H), 7.32 (dd, *J* = 1.2, 5.2 Hz, 1H), 6.84 (d, *J* = 2.0 Hz, 1H), 3.53 (t, *J* = 6.8 Hz, 2H), 2.87 (t, *J* = 6.8 Hz, 2H), 2.61 (td, *J* = 3.2, 7.2 Hz, 1H), 2.11 (s,

4H), 0.77 - 0.68 (m, 2H), 0.63 - 0.54 (m, 2H).

**Intermediate 159**

N-{4-[5-cyclopropyl-4-oxo-3-(pyridin-2-yl)-4,5-dihydro-1H-pyrrolo[3,2-c]pyridin-2-yl]pyridin-2-yl}acetamide

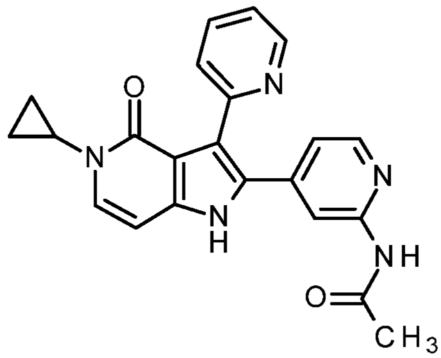

N-{4-[5-cyclopropyl-4-oxo-3-(pyridin-2-yl)-4,5,6,7-tetrahydro-1H-pyrrolo[3,2-c]pyridin-2-yl]pyridin-2-yl}acetamide (680 mg, 1.67 mmol, 1 eq) was dissolved in 10 mL of 1,4-dioxane. 2,3-Dichloro-5,6-dicyano-p-benzoquinone (1.14 g, 5.00 mmol, 3 eq, CAS-RN: [84-58-2]) was added in one portion, and the resulting mixture was stirred at 60 °C for 16 h. Upon completion, the mixture was quenched by the addition of 10 mL of a saturated aqueous solution of $NaHCO_3$. The resulting mixture was extracted with ethyl acetate (20 mL x 3). The combined organic layers were washed with brine (20 mL x 3), dried (sodium sulfate), filtered, and concentrated *in vacuo* to provide a crude residue. The crude residue was purified by reverse phase preparative HPLC (column: YMC-Triart Prep C18 150mm * 40mm * 7um; mobile phase: [water (0.1% TFA)-ACN]; B%: 13%-33%, 10 min) to provide the target compound as a brown solid: 60 mg, 155 umol, 9.3% yield.
LC-MS (Method 7): Rt = 1.293 min; MS (ESIpos): m/z = 386.0 $[M+H]^+$

**Intermediate 160**

2-(2-aminopyridin-4-yl)-5-cyclopropyl-3-(pyridin-2-yl)-1,5-dihydro-4H-pyrrolo[3,2-c]pyridin-4-one

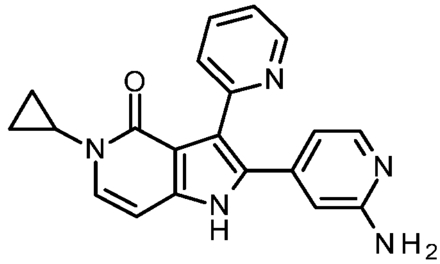

N-{4-[5-cyclopropyl-4-oxo-3-(pyridin-2-yl)-4,5-dihydro-1H-pyrrolo[3,2-c]pyridin-2-yl]pyridin-2-yl}acetamide (50 mg, 129 μmol, 1 eq) was dissolved in 2 mL of MeOH. Sodium hydroxide (25.9 mg, 648 μmol, 5 eq, CAS-RN: [1310-73-2]) was added in one portion. The resulting mixture was stirred at 80 °C for 2 h. Upon completion, the mixture was concentrated *in vacuo,* and the crude residue resuspended in 2 mL of water. The resulting mixture was extracted with ethyl acetate (2 mL x 3). The combined organic layers were washed with brine (2 mL x 3), dried (sodium sulfate), filtere, and concentrated *in vacuo* to provide the target compound as a yellow solid, which was used without further purification: 50 mg, crude.
LC-MS (Method 7): $R_t$ = 0.657 min; MS (ESIpos): m/z = 344.0 $[M+H]^+$

**Intermediate 161**

2-(2-chloropyridin-4-yl)-5-methyl-7-(2,2,2-trifluoroethyl)-1,5,6,7-tetrahydro-4H-pyrrolo[3,2-c]pyridin-4-one

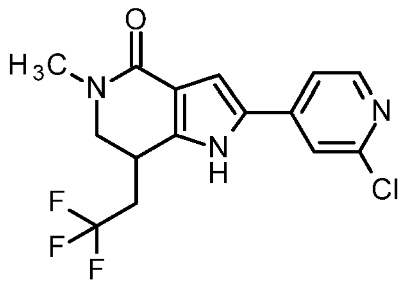

To a stirred solution of 1-methyl-5-(2,2,2-trifluoroethyl)piperidine-2,4-dione (960 mg, 4.59 mmol, see Intermediate 13) in 1-propanol (17 ml) in a microwave tube was added 2-bromo-1-(2-chloropyridin-4-yl)ethan-1-one (1.08 g, 4.59 mmol, see Intermediate 118) and ammonium acetate (1.06 g, 13.8 mmol; CAS-RN:[631-61-8]) and the mixture was stirred at 120° C for 4 h. The mixture was concentrated in vacuum and the residue was heated to 120° C for 4 h. Dichloromethane and an aqueous sodium hydroxide solution (10%, w/v) were added and the mixture was stirred resulting in the precipitation of a solid. The solid was collected by filtration and dried in vacuum to give 1.46 g (92 % yield) of the title compound as a crude product that was used without further purification.

LC-MS (Method 1): Rt = 0.92 min; MS (ESIpos): m/z = 344 $[M+H]^+$

$^1$H-NMR (400 MHz, DMSO-d6) δ [ppm]: 2.518 (2.24), 2.523 (1.55), 2.669 (0.50), 2.673 (0.53), 2.841 (0.42), 2.871 (0.56), 2.934 (16.00), 3.424 (0.52), 3.440 (1.15), 3.454 (0.71), 3.462 (1.47), 3.468 (1.47), 3.477 (0.83), 3.720 (0.73), 3.740 (0.70), 7.189 (4.92), 7.681 (1.56), 7.685 (1.70), 7.695 (1.58), 7.698 (1.80), 7.800 (2.55), 7.803 (2.47), 8.309 (2.29), 8.323 (2.23).

**Intermediate 162**

2-(2-chloropyridin-4-yl)-5-methyl-7-(2,2,2-trifluoroethyl)-1,5-dihydro-4H-pyrrolo[3,2-c]pyridin-4-one

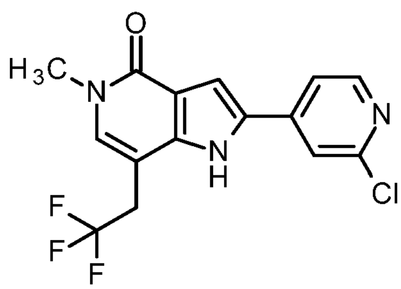

To a solution of 2-(2-chloropyridin-4-yl)-5-methyl-7-(2,2,2-trifluoroethyl)-1,5,6,7-tetrahydro-4H-pyrrolo[3,2-c]pyridin-4-one (1.69 g, 4.92 mmol) in 1,4-dioxane (180 mL) was added 4,5-dichloro-3,6-dioxocyclohexa-1,4-diene-1,2-dicarbonitrile (1.45 g, 6.39 mmol) and the reaction mixture was stirred at 100° C for 7 hours. The reaction mixture was directly purified by chromatography (340 g column, silica ULTRA, gradient: dichloromethane/ethanol 0-10% ethanol) to give a solid that was triturated with dichloromethane to give 1.23 g (73 % yield) of the title compound.

LC-MS (Method 1): Rt = 0.92 min; MS (ESIpos): m/z = 342 $[M+H]^+$

$^1$H-NMR (400 MHz, DMSO-d6) δ [ppm]: 1.053 (0.56), 2.323 (0.46), 2.327 (0.65), 2.332 (0.45), 2.518 (2.17), 2.523 (1.45), 2.665 (0.45), 2.669 (0.64), 2.674 (0.45), 3.480 (16.00), 3.705 (0.54), 3.733 (1.53), 3.760 (1.44), 3.788 (0.45), 7.483 (3.65), 7.510 (3.11), 7.516 (3.01), 7.868 (1.66), 7.872 (1.79), 7.881 (1.72), 7.885 (1.88), 7.979 (2.71), 7.981

(2.60), 8.395 (2.48), 8.409 (2.37), 11.817 (1.13).

**Intermediate 163**

(2RS)-4,4-difluoro-2-(4-fluorophenyl)-N-{4-[5-methyl-4-oxo-7-(2,2,2-trifluoroethyl)-4,5-dihydro-1H-pyrrolo[3,2-c]pyridin-2-yl]pyridin-2-yl}butanamide

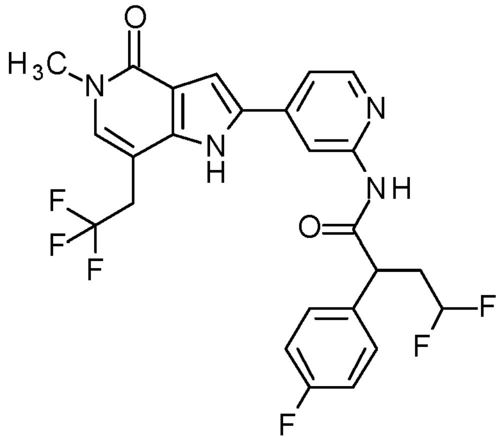

To a stirred solution of 2-(2-chloropyridin-4-yl)-5-methyl-7-(2,2,2-trifluoroethyl)-1,5-dihydro-4H-pyrrolo[3,2-c]pyridin-4-one (400 mg, 1.17 mmol) in 1,4-dioxane (10 mL) in a sealed tube was added (2RS)-4,4-difluoro-2-(4-fluorophenyl)butanamide (381 mg, 1.76 mmol), cesium carbonate (1.14 g, 3.51 mmol; CAS-RN:[534-17-8]), *t*-BuBrettPhos-Pd G3 (100 mg, 117 μmol) and *t*-BuBrett Phos (113 mg, 234 μmol), the vial was degassed and backfilled with argon and the mixture was heated to 100°C for 16 h. Ethyl acetate and methanol (10:1) were added and the mixture was washed with half-saturated sodium chloride solution, dried (sodium sulfate), filtered and the solvent was removed in vacuum. Silicagel chromatography (gradient: dichloromethane / ethanol 0-10%) gave 442 mg (72 % yield) of the title compound.

LC-MS (Method 2): Rt = 1.14 min; MS (ESIpos): m/z = 523 $[M+H]^+$

$^1$H-NMR (400 MHz, DMSO-d6) δ [ppm]: 0.811 (0.70), 0.828 (1.58), 0.833 (1.07), 0.837 (0.57), 0.849 (1.30), 0.855 (1.56), 0.866 (0.49), 0.871 (0.44), 0.933 (0.70), 0.950 (0.73), 1.035 (1.06), 1.052 (1.96), 1.070 (1.00), 1.234 (0.90), 1.392 (0.65), 2.081 (0.41), 2.116 (0.41), 2.131 (0.43), 2.326 (0.44), 2.518 (2.43), 2.522 (1.64), 2.541 (0.72), 2.553 (0.63), 2.565 (0.51), 2.577 (0.46), 2.771 (0.44), 3.422 (0.43), 3.435 (0.45), 3.440 (0.46), 3.452 (0.52), 3.476 (16.00), 3.564 (2.31), 3.669 (0.80), 3.683 (0.92), 3.691 (0.91), 3.706 (0.80), 3.743 (0.47), 3.771 (1.25), 3.800 (1.20), 3.827 (0.44), 4.214 (0.61), 4.228 (0.75), 4.236 (0.74), 4.250 (0.60), 4.358 (0.56), 5.758 (2.99), 5.773 (0.61), 5.903 (1.12), 5.914 (1.43), 5.926 (0.61), 6.033 (0.52), 6.044 (1.38), 6.055 (1.07), 6.184 (0.50), 6.989 (1.10), 7.131 (2.12), 7.136 (0.76), 7.150 (4.02), 7.153 (6.33), 7.170 (1.08), 7.176 (3.39), 7.183 (1.07), 7.200 (4.41), 7.217 (0.84), 7.222 (2.39), 7.353 (2.33), 7.359 (0.99), 7.367 (2.57), 7.376 (2.20), 7.384 (0.89), 7.389 (1.94), 7.435 (3.97), 7.503 (2.07), 7.508 (1.02), 7.516 (2.40), 7.526 (3.56), 7.530 (2.38), 7.539 (3.68), 7.543 (2.20), 7.583 (1.11), 8.316 (2.48), 8.318 (2.55), 8.332 (2.38), 8.387 (2.27), 10.888 (2.60), 11.872 (1.62), 11.877 (1.59).

**Intermediate 164**

(2RS)-N-{4-[3-bromo-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide

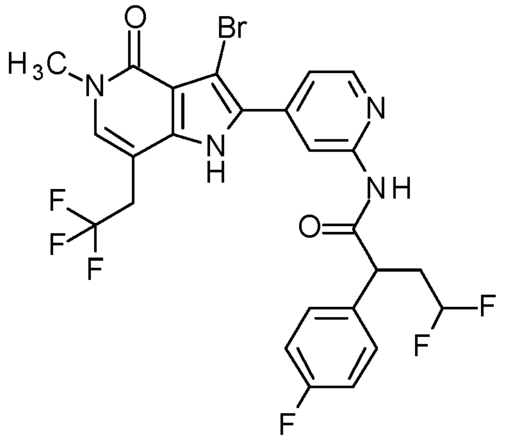

To a stirred solution of (2RS)-4,4-difluoro-2-(4-fluorophenyl)-N-{4-[5-methyl-4-oxo-7-(2,2,2-trifluoroethyl)-4,5-dihydro-1H-pyrrolo[3,2-c]pyridin-2-yl]pyridin-2-yl}butanamide (438 mg, 838 µmol) in DMF (8 mL) was added NBS (224 mg, 1.26 mmol; CAS-RN:[128-08-5]) and the mixture was stirred at 0° C for 1 h. An aqueous solution of sodium bicarbonate and an aqueous solution of disodium sulfurothioate was added and the mixture was stirred for 30 minutes. The mixture was extracted with ethyl acetate. The organic phase was washed with saturated sodium chloride solution, dried (sodium sulfate), filtered and the solvent was removed in vacuum. Silicagel chromatography (Gradient: dichloromethane / ethanol 0-10%) followed by aminophase silicagel chromatography (Gradient: hexane / ethyl acetate 5-100%) gave 105 mg (21 % yield) of the title compound.

LC-MS (Method 2): Rt = 1.19 min; MS (ESIpos): m/z = 601 $[M+H]^+$

$^1$H-NMR (400 MHz, DMSO-d6) δ [ppm]: 1.154 (2.38), 1.172 (5.14), 1.190 (2.62), 1.987 (9.34), 2.267 (0.41), 2.518 (2.43), 2.523 (1.55), 2.769 (0.43), 3.448 (16.00), 3.680 (0.49), 3.708 (1.41), 3.736 (1.30), 3.762 (0.41), 3.999 (0.67), 4.017 (2.00), 4.034 (1.92), 4.053 (0.62), 4.223 (0.64), 4.237 (0.75), 4.245 (0.75), 4.259 (0.59), 5.758 (1.99), 5.887 (0.56), 6.016 (0.53), 6.027 (1.12), 6.038 (0.56), 6.167 (0.52), 7.176 (2.29), 7.181 (0.77), 7.193 (0.89), 7.199 (4.77), 7.204 (0.91), 7.216 (0.78), 7.221 (2.51), 7.433 (1.49), 7.437 (1.52), 7.445 (1.50), 7.450 (1.53), 7.490 (2.84), 7.496 (2.77), 7.502 (1.18), 7.510 (2.51), 7.518 (2.32), 7.527 (0.87), 7.532 (2.06), 8.420 (2.12), 8.433 (2.07), 8.542 (2.29), 10.979 (2.17), 12.209 (1.80).

## Intermediate 165

(2 RS)-N-{ 4-[3-bromo-5-methyl-4-oxo-7 -(2,2 ,2-trifluoroethyl)-1-{[2-(trimethylsilyl)ethoxy]methyl}-4,5-dihydro-1H-pyrrolo[3,2-c]pyridin-2-yl]pyridin-2-yl}-4,4-difluoro-2-(4-fluorophenyl)butanamide

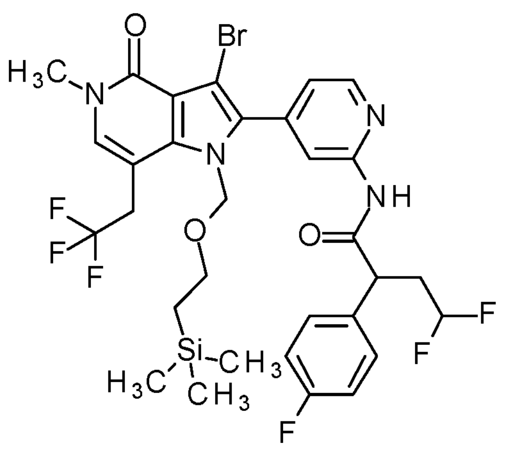

To a solution of (2RS)-N-{4-[3-bromo-5-methyl-4-oxo-7-(2,2,2-trifluoroethyl)-4,5-dihydro-1H-pyrrolo[3,2-c]pyridin-2-yl]pyridin-2-yl}-4,4-difluoro-2-(4-fluorophenyl)butanamide (133 mg, 75 % purity, 166 μmol) in N,N-dimethylformamide (2.0 ml) was added sodium hydride (9.95 mg, 60 % purity, in oil, 249 μmol; CAS-RN:[7646-69-7]) at r.t. After stirring for 10 minutes, [2-(chloromethoxy)ethyl](trimethyl)silane (44 μl, 250 μmol) was added and the reaction mixture was stirred at room temperature for 2.5 h. A half-saturated sodium chloride solution was added and the mixture was extracted with ethyl acetate. The organic phase was dried (sodium sulfate), filtered and the solvent was removed in vacuum. Silicagel chromatography (Gradient: hexane / ethyl acetate 30-100%) gave 25.0 mg of the title compound as a crude product that was used without further purification.

LC-MS (Method 2): Rt = 1.45 min; MS (ESIpos): m/z = 733 $[M+H]^+$

$^1$H-NMR (400 MHz, DMSO-d6) δ [ppm]: -0.047 (0.72), -0.038 (1.17), -0.034 (0.65), -0.030 (0.64), -0.019 (4.36), -0.008 (1.00), 0.000 (9.67), 0.006 (1.18), 0.017 (2.15), 0.020 (1.14), 0.056 (1.43), 0.069 (0.56), 0.077 (1.17), 0.876 (0.63), 0.926 (0.46), 0.935 (0.50), 0.943 (0.54), 1.230 (4.31), 1.247 (8.39), 1.265 (3.96), 1.308 (0.85), 2.063 (16.00), 2.594 (7.72), 2.598 (5.35), 3.523 (2.88), 3.531 (2.73), 3.567 (0.44), 3.629 (0.82), 3.649 (0.88), 3.669 (0.41), 3.793 (0.46), 3.821 (0.55), 4.075 (1.11), 4.093 (3.42), 4.111 (3.46), 4.128 (1.16), 5.682 (0.50), 5.691 (0.50), 7.168 (0.63), 7.191 (0.80), 7.213 (0.44), 7.462 (0.42), 7.475 (0.41), 7.483 (0.41), 7.605 (1.00).

**Intermediate 166**

(2 RS)-4, 4-difl uoro-2 -(4- fl uorophenyl)- N-{ 4- [5-methyl-4-oxo-3-(pyridi n-2 -yl)- 7-(2,2,2-trifluoroethyl)-1-{[2-(trimethylsilyl)ethoxy]methyl}-4,5-dihydro-1H-pyrrolo[3,2-c]pyridin-2-yl]pyridin-2-yl}butanamide

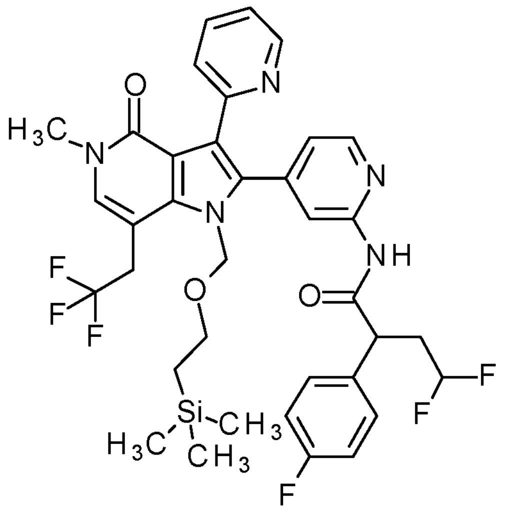

To a stirred solution of (2RS)-N-{4-[3-bromo-5-methyl-4-oxo-7-(2,2,2-trifluoroethyl)-1-{[2-(trimethylsilyl)ethoxy]methyl}-4,5-dihydro-1H-pyrrolo[3,2-c]pyridin-2-yl]pyridin-2-yl}-4,4-difluoro-2-(4-fluorophenyl)butanamide (24.0 mg, 32.8 μmol) and 2-(tributylstannyl)pyridine (24.2 mg, 65.6 μmol) in 1,4-dioxane (500 μL) in a sealed tube was added XPhos Pd G3 (4.17 mg, 4.92 μmol) and the tube was twice degassed and backfilled with argon. The mixture was heated to 80° C for 16 h. The reaction mixture was directly purified by silicagel chromatography (Gradient: dichloromethane / methanol 0-30%) to give 10 mg of a mixture of the title compound and of (2RS)-4,4-difluoro-2-(4-fluorophenyl)-N-{4-[5-methyl-4-oxo-3-(pyridin-2-yl)-7-(2,2,2-trifluoroethyl)-4,5-dihydro-1H-pyrrolo[3,2-c]pyridin-2-yl]pyridin-2-yl}butanamide that was used as a crude product without further purification.
LC-MS (Method 2): Rt = 1.47 min; MS (ESIpos): m/z = 730 $[M+H]^+$

## EXPERIMENTAL SECTION -EXAMPLES

### Example 1

2-(4-fluorophenyl)-N-[4-(5-methyl-4-oxo-3-phenyl-4,5,6,7-tetrahydro-1H-pyrrolo[3,2-c]pyridin-2-yl)pyridin-2-yl]acetamide

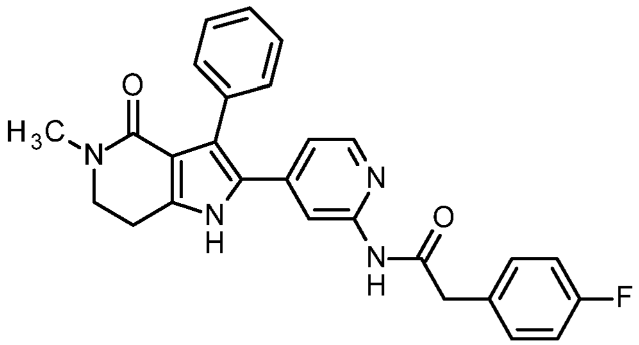

2-(2-Aminopyridin-4-yl)-5-methyl-3-phenyl-1,5,6,7-tetrahydro-4H-pyrrolo[3,2-c]pyridin-4-one (see Intermediate 3, 375 mg), (4-fluorophenyl)acetic acid (CAS-RN:[405-50-5], 363 mg, 2.36 mmol), PyBOP (CAS-RN:[128625-52-5], 3.06 g, 5.89 mmol) and N,N-diisopropylethylamine (1.2 mL, 7.1 mmol) were dissolved in 11 mL N,N-dimethylacetamide and were stirred at room temperature over night under nitrogen atmosphere. Aqueous saturated sodium hydrogencarbonate solution was added to the reaction mixture. It was diluted with dichloromethane and the aqueous layer was extracted with dichloromethane two times. The combined organic layers were dried using a waterresistant filter and concentrated under reduced pressure. The crude product was purified by preparative reverse phase HPLC (eluent: acetonitrile / water with ammonia as additive) to provide the title compound: 260 mg.

LC-MS (Method 2): Rt = 1.09 min; MS (ESIpos): m/z = 455 $[M+H]^+$

$^1$H-NMR (400MHz, DMSO-$d_6$): $\delta$ [ppm]= 2.84 (s, 3H), 2.92 (t, 2H), 3.54 (t, 2H), 3.68 (s, 2H), 6.50 (dd, 1H), 7.11 - 7.20 (m, 4H), 7.21 - 7.28 (m, 3H), 7.33 - 7.39 (m, 2H), 7.98 (d, 1H), 8.05 (s, 1H), 10.63 (s, 1H), 11.82 (s, 1H)

### Example 2

4,4-difluoro-2-(4-fluorophenyl)-N-[4-(5-methyl-4-oxo-3-phenyl-4,5,6,7-tetrahydro-1H-pyrrolo[3,2-c]pyridin-2-yl)pyridin-2-yl]butanamide (Racemate)

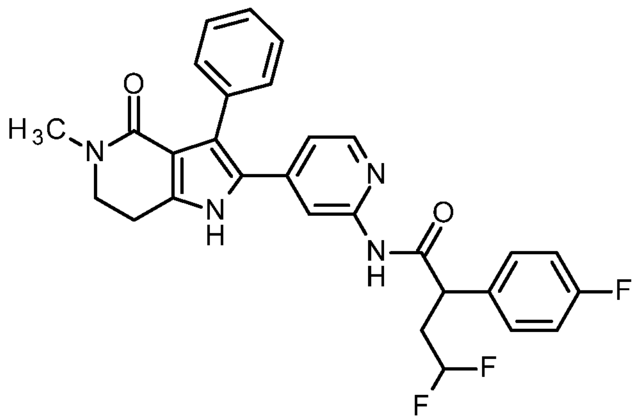

2-(2-Aminopyridin-4-yl)-5-methyl-3-phenyl-1,5,6,7-tetrahydro-4H-pyrrolo[3,2-c]pyridin-4-one (see Intermediate 3, 375 mg), 4,4-difluoro-2-(4-fluorophenyl)butanoic acid (Racemate) (CAS-RN:[1538957-14-0], 514 mg, 2.36 mmol), PyBOP (CAS-RN:[128625-52-5], 3.06 g, 5.89 mmol) and N,N-diisopropylethylamine (1.2 mL, 7.1 mmol) were dissolved in 11 mL N,N-dimethylacetamide and were stirred at room temperature over night under nitrogen atmosphere. Aqueous saturated sodium hydrogencarbonate solution was added to the reaction mixture. It was diluted with dichloromethane and the aqueous layer was extracted with dichloromethane two times. The combined organic layers were dried using a waterresistant filter and concentrated under reduced pressure. The crude product was purified by preparative reverse phase HPLC (eluent: acetonitrile / water with ammonia as additive) to provide the title compound: 414 mg.

LC-MS (Method 2): Rt = 1.19 min; MS (ESIpos): m/z = 519 $[M+H]^+$

$^1$H-NMR (400MHz, DMSO-$d_6$): δ [ppm]= 2.11 - 2.26 (m, 1H), 2.60 - 2.77 (m, 1H), 2.84 (s, 3H), 2.93 - 3.00 (m, 2H), 3.55 (t, 2H), 4.15 (dd, 1H), 5.80 - 6.15 (m, 1H), 6.52 (dd, 1H), 7.09 - 7.30 (m, 7H), 7.42 - 7.52 (m, 2H), 7.95 - 8.07 (m, 2H), 10.71 (s, 1H), 11.84 (s, 1H)

## Example 3

4,4-difluoro-2-(4-fluorophenyl)-N-[4-(5-methyl-4-oxo-3-phenyl-4,5,6,7-tetrahydro-1H-pyrrolo[3,2-c]pyridin-2-yl)pyridin-2-yl]butanamide (Enantiomer 1)

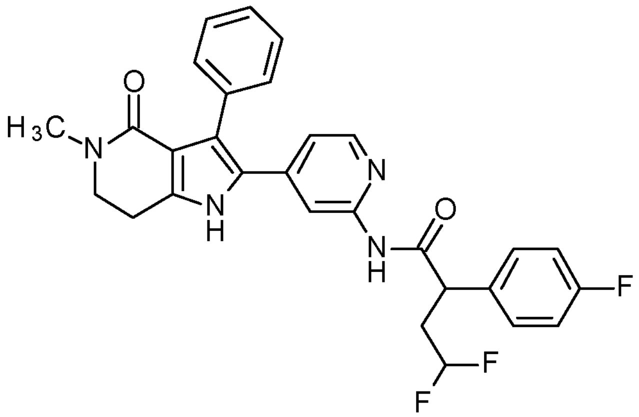

The racemic compound (see Example 2, 100 mg) was separated into enantiomers by preparative chiral HPLC to give enantiomer 1 (35 mg, >99 %ee, see Example 3) and enantiomer 2 (30 mg, >99 % ee, see Example 4).

Preparative chiral HPLC method:
Instrument: PrepCon Labomatic HPLC-2; Column: YMC Cellulose SB 10μ, 250x50; eluent A: hexane + 0.1 vol % diethylamine; eluent B: ethanol; isocratic: 50%A+50%B; flow: 100 mL/min; temperature: 25°C; UV: 254 nm

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 50%A+50%B; flow: 1.4 mL/min; temperature: 25°C; UV: 254 nm

Analytical Chiral HPLC (method Example 3): Rt = 1.08 min.

LC-MS (Method 2): Rt = 1.19 min; MS (ESIneg): m/z = 517 [M-H]-

$^1$H-NMR (400MHz, DMSO-$d_6$): δ [ppm]= 2.10 - 2.28 (m, 1H), 2.61 - 2.77 (m, 1H), 2.84 (s, 3H), 2.94 (t, 2H), 3.55 (t, 2H), 4.15 (dd, 1H), 5.79 - 6.15 (m, 1H), 6.52 (dd, 1H), 7.13 - 7.28 (m, 7H), 7.41 - 7.51 (m, 2H), 7.97 (d, 1H), 8.00 (s, 1H), 10.71 (s, 1H), 11.84 (s, 1H)

**Example 4**

4,4-difluoro-2-(4-fluorophenyl)-N-[4-(5-methyl-4-oxo-3-phenyl-4,5,6,7-tetrahydro-1H-pyrrolo[3,2-c]pyridin-2-yl)pyridin-2-yl]butanamide (Enantiomer 2)

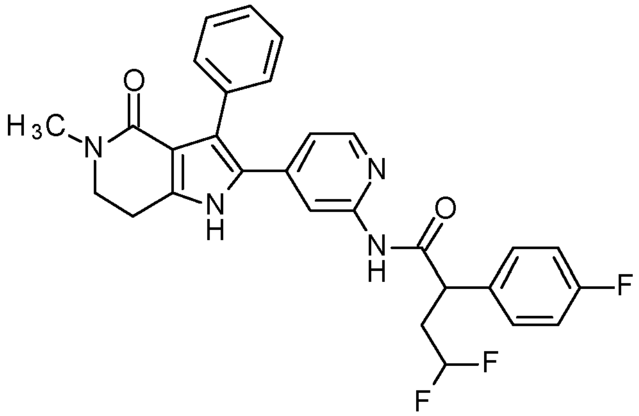

For the preparation of the racemic title compound see Example 2. Separation of enantiomers by preparative chiral HPLC (method see Example 3) gave the title compound (30 mg, >99 %ee).

Analytical Chiral HPLC (method see Example 3): Rt = 1.52 min.

LC-MS (Method 2): $R_t$ = 1.19 min; MS (ESIneg): m/z = 517 [M-H]-

$^1$H-NMR (400MHz, DMSO-$d_6$): δ [ppm]= 2.10 - 2.29 (m, 1H), 2.59 - 2.77 (m, 1H), 2.84 (s, 3H), 2.94 (t, 2H), 3.55 (t, 2H), 4.15 (dd, 1H), 5.79 - 6.19 (m, 1H), 6.52 (dd, 1H), 7.09 - 7.30 (m, 7H), 7.40 - 7.52 (m, 2H), 7.94 - 8.07 (m, 2H), 10.71 (s, 1H), 11.84 (s, 1H).

**Example 5**

2-(4-fluorophenyl)-N-[4-(5-methyl-4-oxo-3-phenyl-4,5-dihydro-1H-pyrrolo[3,2-c]pyridin-2-yl)pyridin-2-yl]acetamide

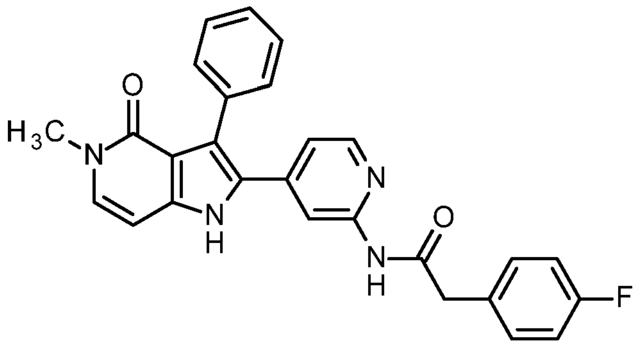

2-(4-Fluorophenyl)-N-[4-(5-methyl-4-oxo-3-phenyl-4,5,6,7-tetrahydro-1H-pyrrolo[3,2-c]pyridin-2-yl)pyridin-2-yl]acetamide (see Example 1, 110 mg) was dissolved in 1,4-dioxane (10 mL) and 2,3-dichloro-5,6-dicyano-1,4-benzochinone (CAS-RN:[84-58-2], 60.4 mg, 266 μmol) was added. The reaction mixture was stirred at 80 °C for 1 hour. The reaction mixture was diluted with ethyl acetate and treated with 1 M aqueous sodiumhydroxyd-solution. The layers were separated and the aqueous layer was extracted with ethyl acetate twice. The combined organic layers were dried using a waterresistant filter and concentrated under reduced pressure. The crude product was purified by chromatography (5 g spheric silica column, gradient: dichloromethane/ethanol 1-10% ethanol) to provide the title compound: 87 mg.

LC-MS (Method 1): Rt = 1.05 min; MS (ESIpos): m/z = 453.8 $[M+H]^+$

$^1$H-NMR (400MHz, DMSO-$d_6$): δ [ppm]= 3.38 (s, 3H), 3.69 (s, 2H), 6.42 (d, 1H), 6.64 (dd, 1H), 7.12 - 7.21 (m, 2H), 7.22 - 7.31 (m, 5H), 7.33 - 7.41 (m, 3H), 8.07 (d, 1H), 8.15 (s, 1H), 10.72 (s, 1H), 12.00 (s, 1H)

## Example 6

4,4-difluoro-2-(4-fluorophenyl)-N-[4-(5-methyl-4-oxo-3-phenyl-4,5-dihydro-1H-pyrrolo[3,2-c]pyridin-2-yl)pyridin-2-yl]butanamide (Racemate)

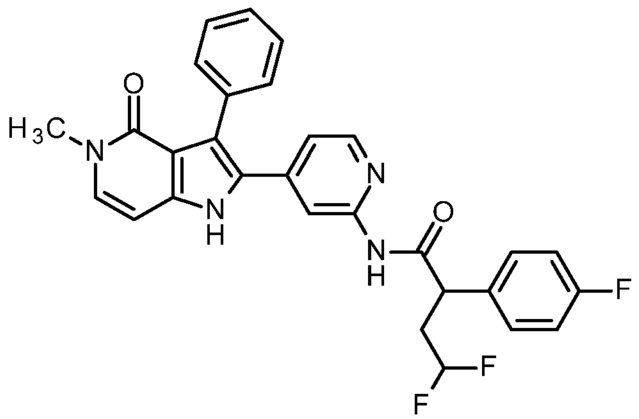

4,4-Difluoro-2-(4-fluorophenyl)-N-[4-(5-methyl-4-oxo-3-phenyl-4,5,6,7-tetrahydro-1H-pyrrolo-[3,2-c]pyridin-2-yl)pyridin-2-yl]butanamide (Racemate) (see Example 2, 100 mg) was dissolved in 1,4-dioxane (8.2 mL) and 2,3-dichloro-5,6-dicyano-1,4-benzochinone (CAS-RN:[84-58-2], 48.2 mg, 212 μmol) was added. The reaction mixture was stirred at 80 °C for 1 hour. The reaction mixture was diluted with ethyl acetate and treated with 1 M aqueous sodiumhydroxyd-solution. The layers were separated and the aqueous layer was extracted with ethyl acetate twice. The combined organic layers were dried using a waterresistant filter and concentrated under reduced pressure. The crude product was purified by chromatography (5 g spheric silica column, gradient: dichloromethane/ethanol 1-10% ethanol) to provide the title compound: 74.4 mg.

LC-MS (Method 1): Rt = 1.17 min; MS (ESIneg): m/z = 515 [M-H]-

$^1$H-NMR (400MHz, DMSO-$d_6$): δ [ppm]= 2.12 - 2.29 (m, 1H), 2.65 - 2.75 (m, 1H), 3.39 (s, 3H), 4.17 (dd, 1H), 5.81 - 6.16 (m, 1H), 6.44 (d, 1H), 6.66 (dd, 1H), 7.15 - 7.31 (m, 7H), 7.39 (d, 1H), 7.42 - 7.50 (m, 2H), 8.05 - 8.09 (m, 1H), 8.12 (s, 1H), 10.81 (s, 1H), 12.02 (s, 1H).

## Example 7

4,4-difluoro-2-(4-fluorophenyl)-N-[4-(5-methyl-4-oxo-3-phenyl-4,5-dihydro-1H-pyrrolo[3,2-c]pyridin-2-yl)pyridin-2-yl]butanamide (Enantiomer 1)

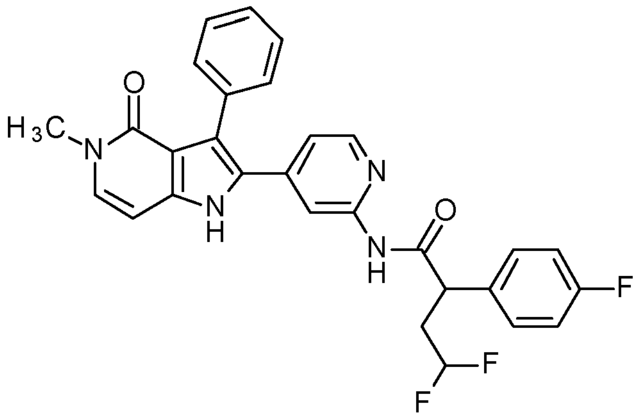

The racemic compound (see Example 6, 65.0 mg) was separated into enantiomers by preparative chiral HPLC to give enantiomer 1 (22 mg, >99 %ee, see Example 7) and enantiomer 2 (20 mg, >99 % ee, see Example 8).

Preparative chiral HPLC method:
Instrument: PrepCon Labomatic HPLC; Column: YMC Cellulose SB 10μ, 250x50; eluent A: hexane + 0.1 vol % diethylamine; eluent B: ethanol + 0.1 vol % diethylamine; isocratic: 70%A+30%B; flow: 100 mL/min; temperature: 25°C; UV: 254 nm.

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 70%A+30%B; flow: 1.4 mL/min; temperature: 25°C; UV: 254 nm

Analytical Chiral HPLC (method Example 7): Rt = 1.43 min.

LC-MS (Method 2): Rt = 1.17 min; MS (ESIneg): m/z = 515 [M-H]-

$^1$H-NMR (400MHz, DMSO-$d_6$): δ [ppm]= 2.11 - 2.29 (m, 1H), 2.61 - 2.79 (m, 1H), 3.39 (s, 3H), 4.17 (br dd, 1H), 5.81 - 6.17 (m, 1H), 6.44 (d, 1H), 6.66 (dd, 1H), 7.14 - 7.33 (m, 7H), 7.39 (d, 1H), 7.42 - 7.54 (m, 2H), 8.06 (d, 1H), 8.12 (s, 1H), 10.81 (s, 1H), 12.02 (s, 1H).

## Example 8

4,4-difluoro-2-(4-fluorophenyl)-N-[4-(5-methyl-4-oxo-3-phenyl-4,5-dihydro-1H-pyrrolo[3,2-c]pyridin-2-yl)pyridin-2-yl]butanamide (Enantiomer 2)

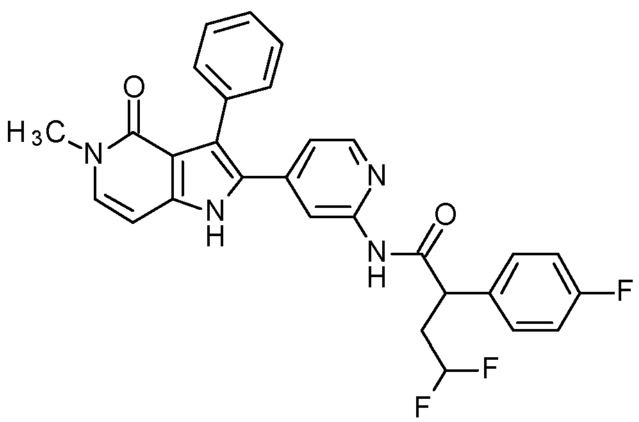

For the preparation of the racemic title compound see Example 6. Separation of enantiomers by preparative

chiral HPLC (method see Example 7) gave the title compound (20 mg, >99 %ee).

Analytical Chiral HPLC (method see Example 7): Rt = 1.76 min.

LC-MS (Method 2): Rt = 1.17 min; MS (ESIneg): m/z = 515 [M-H]-

$^1$H-NMR (400MHz, DMSO-$d_6$): δ [ppm]= 2.10 - 2.30 (m, 1H), 2.61 - 2.79 (m, 1H), 3.39 (s, 3H), 4.17 (dd, 1H), 5.82 - 6.17 (m, 1H), 6.44 (d, 1H), 6.66 (dd, 1H), 7.17 - 7.33 (m, 7H), 7.39 (d, 1H), 7.42 - 7.53 (m, 2H), 8.06 (d, 1H), 8.12 (s, 1H), 10.81 (s, 1H), 12.02 (s, 1H).

## Example 9

4,4-difluoro-2-(4-fluorophenyl)-N-{4-[5-methyl-4-oxo-3-(pyridin-3-yl)-4,5,6,7-tetrahydro-1H-pyrrolo[3,2-c]pyridin-2-yl]pyridin-2-yl}butanamide (Racemate)

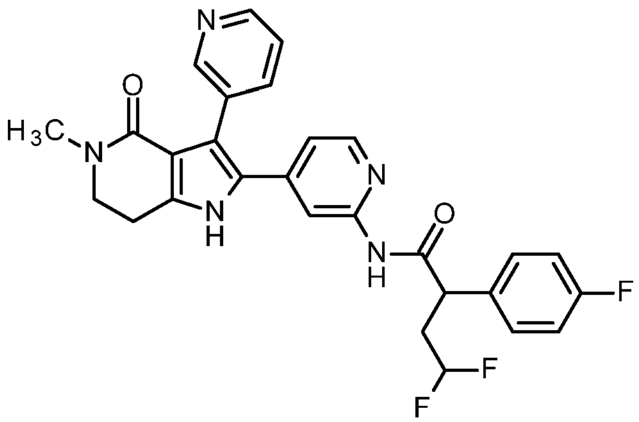

2-(2-Aminopyridin-4-yl)-5-methyl-3-(pyridin-3-yl)-1,5,6,7-tetrahydro-4H-pyrrolo[3,2-c]pyridin-4-one (see Intermediate 6, 150 mg, 470 μmol), 4,4-difluoro-2-(4-fluorophenyl)butanoic acid (Racemate) (CAS-RN:[1538957-14-0] 205 mg, 939 μmol), PyBOP (CAS-RN:[128625-52-5], 1.22 g, 2.35 mmol) and N,N-diisopropylethylamine (490 μL, 2.8 mmol) were dissolved in 4.4 mL N,N-dimethylacetamide and were stirred at room temperature over night under nitrogen atmosphere. Aqueous saturated sodium hydrogencarbonate solution was added to the reaction mixture. It was diluted with dichloromethane and the aqueous layer was extracted with dichloromethane two times. The combined organic layers were dried using a waterresistant filter and concentrated under reduced pressure. The crude product was purified by preparative reverse phase HPLC (eluent: acetonitrile / water with ammonia as additive) to provide the title compound: 19.8 mg.

LC-MS (Method 2): Rt = 1.05 min; MS (ESIpos): m/z = 520 $[M+H]^+$

$^1$H-NMR (400MHz, DMSO-$d_6$): δ [ppm]= 2.18 (br dd, 1H), 2.58 - 2.75 (m, 1H), 2.85 (s, 3H), 2.96 (t, 2H), 3.56 - 3.60 (m, 2H), 4.14 (dd, 1H), 5.79 - 6.15 (m, 1H), 6.65 (dd, 1H), 7.15 - 7.25 (m, 2H), 7.31 - 7.36 (m, 1H), 7.40 - 7.46 (m, 2H), 7.63 - 7.68 (m, 1H), 7.96 (s, 1H), 8.07 (d, 1H), 8.33 (d, 1H), 8.44 (dd, 1H), 10.76 (s, 1H), 12.02 (s, 1H).

## Example 10

4,4-difluoro-2-(4-fluorophenyl)-N-{4-[5-methyl-4-oxo-3-(pyridin-3-yl)-4,5,6,7-tetrahydro-1H-pyrrolo[3,2-c]pyridin-2-yl]pyridin-2-yl}butanamide (Enantiomer 1)

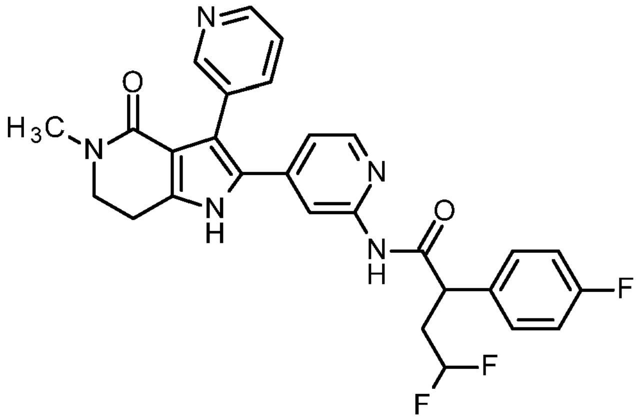

The racemic compound (see Example 9Example 9, 40 mg) was separated into enantiomers by preparative chiral HPLC to give enantiomer 1 (19 mg, >99 %ee, see Example 10) and enantiomer 2 (18 mg, >99 % ee, see Example 11).

Preparative chiral HPLC method:
Instrument: PrepCon Labomatic; Column: YMC Cellulose SB 5μ, 250x30; eluent A: hexane + 0.1 vol % diethylamine; eluent B: ethanol; isocratic: 60%A+40%B; flow: 40 mL/min; temperature: 25°C; UV: 254 nm

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 60%A+40%B; flow: 1.4 mL/min; temperature: 25°C; UV: 254 nm

Analytical Chiral HPLC (method Example 10): $R_t$ = 1.41 min.

LCLC-MS (Method 2): Rt = 1.04 min; MS (ESIpos): m/z = 520 $[M+H]^+$
$^1$H-NMR (400MHz, DMSO-$d_6$): δ [ppm]= 2.08 - 2.28 (m, 1H), 2.58 - 2.76 (m, 1H), 2.85 (s, 3H), 2.95 (t, 2H), 3.57 (t, 2H), 4.14 (dd, 1H), 5.81 - 6.15 (m, 1H), 6.63 (dd, 1H), 7.15 - 7.24 (m, 2H), 7.26 - 7.33 (m, 1H), 7.39 - 7.48 (m, 2H), 7.61 (dt, 1H), 7.97 (s, 1H), 8.06 (d, 1H), 8.30 (dd, 1H), 8.42 (dd, 1H), 10.75 (s, 1H), 12.00 (br s, 1H).

## Example 11

4,4-difluoro-2-(4-fluorophenyl)-N-{4-[5-methyl-4-oxo-3-(pyridin-3-yl)-4,5,6,7-tetrahydro-1H-pyrrolo[3,2-c]pyridin-2-yl]pyridin-2-yl}butanamide (Enantiomer 2)

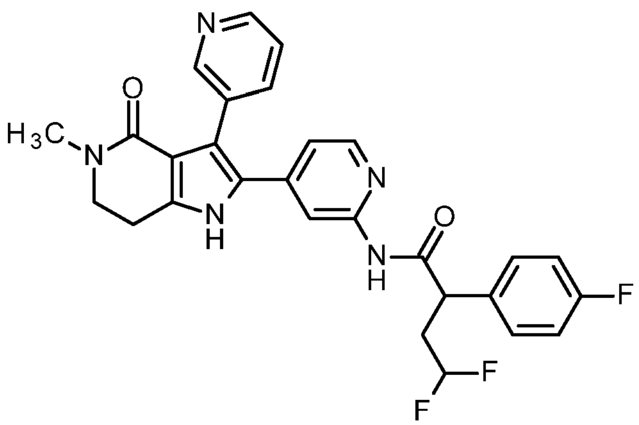

For the preparation of the racemic title compound see Example 9. Separation of enantiomers by preparative chiral HPLC (method see Example 10) gave the title compound (18 mg, >99 %ee).

Analytical Chiral HPLC (method see Example 10): Rt = 2.19 min.

LC-MS (Method 2): Rt = 1.04 min; MS (ESIpos): m/z = 520 $[M+H]^+$

$^1$H-NMR (400MHz, DMSO-$d_6$): δ [ppm]= 2.08 - 2.28 (m, 1H), 2.58 - 2.76 (m, 1H), 2.85 (s, 3H), 2.95 (t, 2H), 3.57 (t, 2H), 4.14 (dd, 1H), 5.81 - 6.15 (m, 1H), 6.63 (dd, 1H), 7.15 - 7.24 (m, 2H), 7.26 - 7.33 (m, 1H), 7.39 - 7.48 (m, 2H), 7.61 (dt, 1H), 7.97 (s, 1H), 8.06 (d, 1H), 8.30 (dd, 1H), 8.42 (dd, 1H), 10.75 (s, 1H), 12.00 (br s, 1H).

**Example 12**

2-(4-fluorophenyl)-N-{4-[5-methyl-4-oxo-3-(pyridin-3-yl)-4,5,6,7-tetrahydro-1H-pyrrolo[3,2-c]pyridin-2-yl]pyridin-2-yl}acetamide

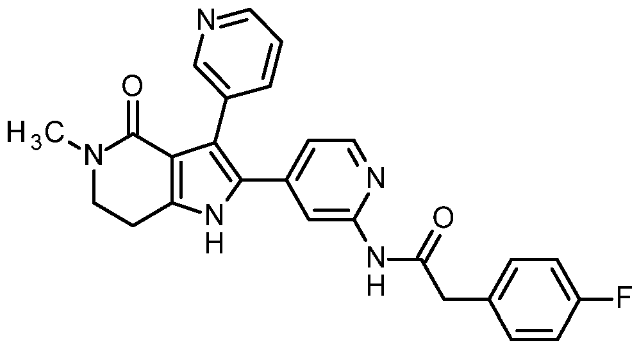

2-(2-Aminopyridin-4-yl)-5-methyl-3-(pyridin-3-yl)-1,5,6,7-tetrahydro-4H-pyrrolo[3,2-c]pyridin-4-one (see Intermediate 6, 100 mg), (4-fluorophenyl)acetic acid (CAS-RN:[405-50-5], 72.4 mg, 470 µmol), PyBOP (CAS-RN:[128625-52-5], 611 mg, 1.17 mmol) and N,N-diisopropylethylamine (250 µL, 1.4 mmol) were dissolved in 2.2 mL N,N-dimethylacetamide and were stirred at at 80 °C over night under nitrogen atmosphere. Aqueous saturated sodium hydrogencarbonate solution was added to the reaction mixture. It was diluted with dichloromethane and the aqueous layer was extracted with dichloromethane two times. The combined organic layers were dried using a waterresistant filter and concentrated under reduced pressure. The crude product was purified by preparative reverse phase HPLC (eluent: acetonitrile / water with ammonia as additive). The product was dissolved in ethyl acetate and the organic layer was extracted with aqueous 1 M HCl-solution once. The layers were separated and the pH value of the aqueous layer was adjusted to pH 10 using aqueous 1 M NaOH-solution. It was extracted with EtOAc twice. The combined organic layers were dried using a waterresistant filter. The clear filtrate was concentrated under reduced pressure. The extract was treated with MTBE and the formed precipitate was filtered off, washed with MTBE and dried at 50°C under vacuum to provide the title compound: 5 mg.

LC-MS (Method 2): Rt = 0.93 min; MS (ESIpos): m/z = 456.7 $[M+H]^+$

$^1$H-NMR (400MHz, DMSO-$d_6$): δ [ppm]= 2.85 (s, 3H), 2.94 (t, 2H), 3.56 (t, 2H), 3.67 (s, 2H), 6.61 (dd, 1H), 7.09 - 7.20 (m, 2H), 7.24 - 7.38 (m, 3H), 7.62 (dt, 1H), 8.00 (s, 1H), 8.08 (d, 1H), 8.30 (d, 1H), 8.41 (dd, 1H), 10.66 (s, 1H), 11.97 (s, 1H).

**Example 13**

2-(4-fluorophenyl)-N-{4-[5-methyl-4-oxo-3-phenyl-7-(2,2,2-trifluoroethyl)-4,5,6,7-tetrahydro-1H-pyrrolo[3,2-c]pyridin-2-yl]pyridin-2-yl}propanamide (Mixture of 4 stereoisomers)

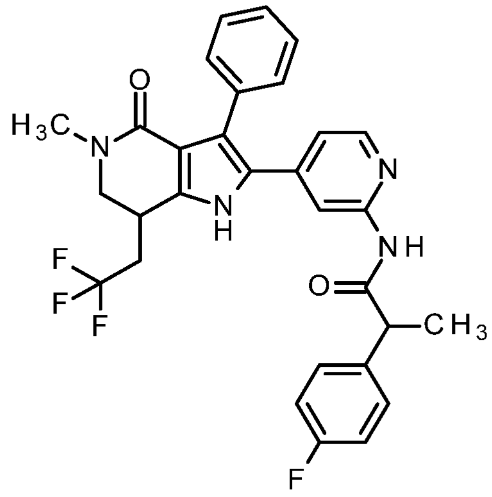

2-(2-Aminopyridin-4-yl)-5-methyl-3-phenyl-7-(2,2,2-trifluoroethyl)-1,5,6,7-tetrahydro-4H-pyrrolo-[3,2-c]pyridin-4-one (Racemate) (see Intermediate 16, 300 mg), 2-(4-fluorophenyl)propanoic acid (Racemate) (CAS-RN:[75908-73-5], 252 mg, 1.50 mmol), PyBOP (CAS-RN:[128625-52-5], 1.17 g, 2.25 mmol) and N,N-diisopropylethylamine (780 µL, 4.5 mmol) were dissolved in 4.4 mL N,N-dimethylacetamide and were stirred at room temperature for two days under argon atmosphere. Aqueous saturated sodium hydrogencarbonate solution and water were added to the reaction mixture. It was diluted with dichloromethane and the aqueous layer was extracted with dichloromethane. The organic layer was dried using a waterresistant filter and concentrated under reduced pressure. The crude product was purified by chromatography (10 g column, silica ULTRA, gradient: hexane / ethyl acetate 25%-75% ethyl acetate) to provide the title compound: 345 mg.

LC-MS (Method 2): Rt = 1.30 min; MS (ESIpos): m/z = 551.5 $[M+H]^+$

$^1$H-NMR (500MHz, DMSO-$d_6$): δ [ppm]= 1.38 (d, 3H), 2.65 - 2.75 (m, 1H), 2.83 - 2.93 (m, 4H), 3.41 - 3.50 (m, 2H), 3.71 - 3.80 (m, 1H), 3.96 - 4.02 (m, 1H), 6.49 (dd, 1H), 7.16 - 7.21 (m, 4H), 7.22 - 7.27 (m, 3H), 7.40 - 7.44 (m, 2H), 7.99 (d, 1H), 8.10 (s, 1H), 10.61 (s, 1H), 11.84 (d, 1H).

**Example 14**

2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}propanamide (Racemate)

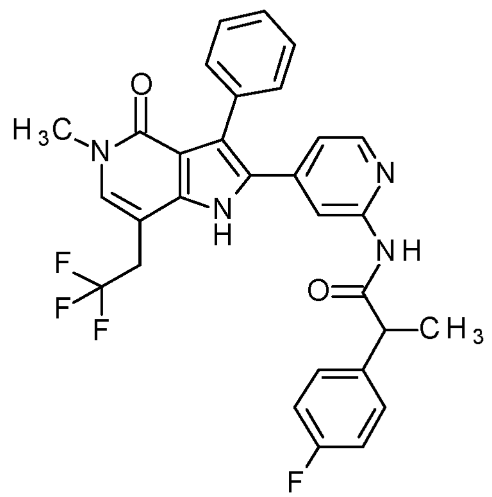

2-(4-Fluorophenyl)-N-{4-[5-methyl-4-oxo-3-phenyl-7-(2,2,2-trifluoroethyl)-4,5,6,7-tetrahydro-1H-pyrrolo[3,2-c]pyridin-2-yl]pyridin-2-yl}propanamide (Mixture of 4 stereoisomers) (see Example 13, 99.0 mg) was dissolved in 1,4-dioxane (7.7 mL) and 2,3-dichloro-5,6-dicyano-1,4-benzochinone (CAS-RN:[84-58-2], 46.9 mg, 207 μmol) was added. The reaction mixture was stirred at 80 °C for 1 hour. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, dried using a waterresistant filter and concentrated under reduced pressure. The crude product was purified by chromatography (10g column, silica ULTRA, gradient: ethyl acetate /ethanol 0-10% ethanol) to provide the title compound: 80.0 mg.

LC-MS (Method 2): Rt = 1.27 min; MS (ESIpos): m/z = 549.5 $[M+H]^+$

$^1$H-NMR (400MHz, DMSO-$d_6$): δ [ppm]= 1.38 (d, 3H), 3.40 (s, 3H), 3.78 (q, 2H), 4.02 (q, 1H), 6.65 (dd, 1H), 7.10 - 7.20 (m, 2H), 7.21 - 7.29 (m, 5H), 7.37 - 7.44 (m, 2H), 7.46 (s, 1H), 8.03 - 8.12 (m, 1H), 8.18 (d, 1H), 10.69 (s, 1H), 11.94 (s, 1H).

## Example 15

(+)-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}propanamide (Enantiomer 1)

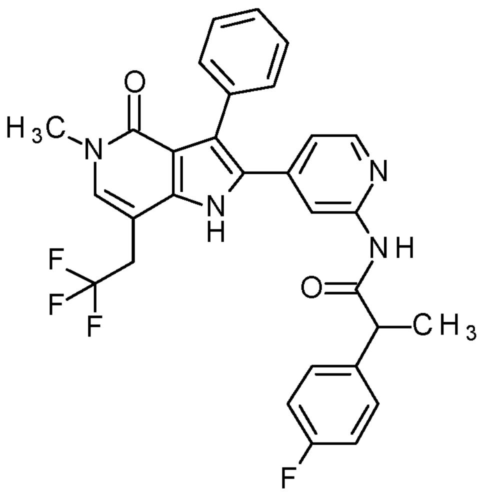

The racemic compound (see Example 14, 73.0 mg) was separated into enantiomers by preparative chiral HPLC to give enantiomer 1 (20 mg, >99 %ee, see Example 15) and enantiomer 2 (29 mg, 82.3% ee, see Example 16).

Preparative chiral HPLC method:
Instrument: Sepiatec: Prep SFC100; Column: Chiralpak IA 5μ 250x30mm; eluent A: CO2; eluent B: ethanol + 0.2 vol % aqueous ammonia (32%); isocratic: 10%B; flow: 100 mL/min; temperature: 40°C; BPR: 150bar; UV: 254 nm
Analytical chiral HPLC method:
Instrument: Agilent: 1260, Aurora SFC-Modul; Column: Chiralpak IA 5μ 100x4.6mm; eluent A: CO2; eluent B: ethanol + 0.2 vol % aqueous ammonia (32%); isocratic: 10%B; flow: 4 mL/min; temperature: 37.5°C; BPR: 100bar; UV: 254 nm

Analytical Chiral HPLC (method Example 15): Rt = 6.29 min.

LC-MS (Method 1): Rt = 1.26 min; MS (ESIpos): m/z = 549 $[M+H]^+$
$[\alpha]_D$ = +150,8° (from solution in DMSO, c = 6.4 mg/mL)
$^1$H-NMR (400MHz, DMSO-$d_6$): δ [ppm]= 1.38 (d, 3H), 3.40 (s, 3H), 3.78 (q, 2H), 4.02 (q, 1H), 6.65 (dd, 1H), 7.11 - 7.20 (m, 2H), 7.21 - 7.30 (m, 5H), 7.37 - 7.44 (m, 2H), 7.46 (s, 1H), 8.09 (d, 1H), 8.18 (s, 1H), 10.69 (s, 1H), 11.94 (s, 1H).

**Example 16**

(-)-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}propanamide (Enantiomer 2)

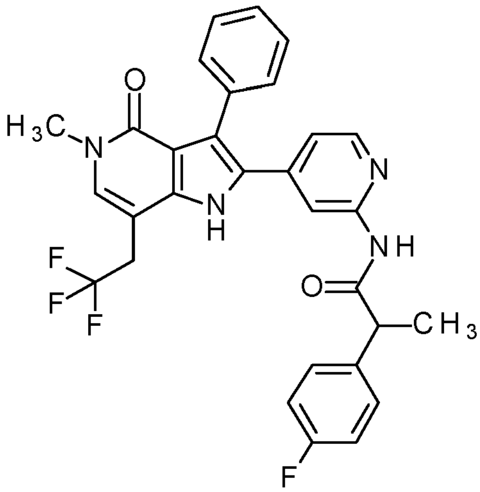

For the preparation of the racemic title compound see Example 14. Separation of enantiomers by preparative chiral HPLC (method see Example 15) gave the title compound (29 mg, 82.3 %ee).
Analytical Chiral HPLC (method see Example 15): Rt = 7.85 min.

LC-MS (Method 1): Rt= 1.26 min; MS (ESIpos): m/z = 549 $[M+H]^+$

$[\alpha]_D$ = -113.5° (from solution in DMSO, c = 7.0 mg/mL)

$^1$H-NMR (400MHz, DMSO-$d_6$): δ [ppm]= 1.38 (d, 3H), 3.40 (s, 3H), 3.70 - 3.84 (m, 2H), 4.02 (q, 1H), 6.65 (dd, 1H), 7.10 - 7.19 (m, 2H), 7.20 - 7.32 (m, 5H), 7.36 - 7.52 (m, 3H), 8.09 (d, 1H), 8.18 (s, 1H), 10.69 (s, 1H), 11.94 (s, 1H).

**Example 17**

4,4-difluoro-2-(4-fluorophenyl)-N-{4-[5-methyl-4-oxo-3-phenyl-7-(2,2,2-trifluoroethyl)-4,5,6,7-tetrahydro-1H-pyrrolo[3,2-c]pyridin-2-yl]pyridin-2-yl}butanamide (Mixture of 4 stereoisomers)

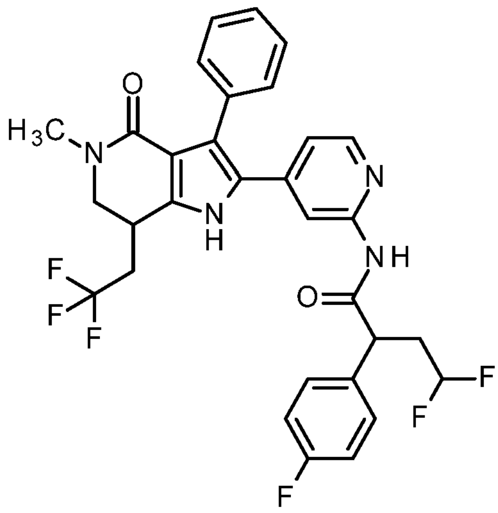

2-(2-Aminopyridin-4-yl)-5-methyl-3-phenyl-7-(2,2,2-trifluoroethyl)-1,5,6,7-tetrahydro-4H-pyrrolo-[3,2-c]pyridin-4-one (Racemate) (see Intermediate 16, 300 mg), 4,4-difluoro-2-(4-fluorophenyl)butanoic acid (Racemate) (CAS-RN:[1538957-14-0], 327 mg, 1.50 mmol), PyBOP (CAS-RN:[128625-52-5], 1.17 g, 2.25 mmol) and N,N-diisopropylethylamine (780 μL, 4.5 mmol) were dissolved in 4.4 mL N,N-dimethylacetamide and were stirred at room temperature under argon atmosphere for 2 days. Aqueous saturated sodium hydrogencarbonate solution and water were added to the reaction mixture. It was diluted with dichloromethane and the aqueous layer was extracted with dichloromethane once. The organic layer was filtered through a silicone coated filter and concentrated under reduced pressure. The crude product was purified by chromatography (10 g column, silica ULTRA, gradient: hexane / ethyl acetate 25-75% ethyl acetate) to provide the title compound: 377 mg.

LC-MS (Method 2): $R_t$ = 1.32 min; MS (ESIpos): m/z = 601 $[M+H]^+$

$^1$H-NMR (500MHz, DMSO-$d_6$): δ [ppm]= 2.12 - 2.29 (m, 1H), 2.66 - 2.77 (m, 2H), 2.82 - 2.97 (m, 4H), 3.40 - 3.51 (m, 2H), 3.75 (br dd, 1H), 4.16 (dd, 1H), 5.82 - 6.14 (m, 1H), 6.53 (dd, 1H), 7.12 - 7.27 (m, 7H), 7.43 - 7.49 (m, 2H), 8.00 (d, 1H), 8.04 (br s, 1H), 10.76 (s, 1H), 11.85 (d, 1H).

## Example 18

4,4-difluoro-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}butanamide (Racemate)

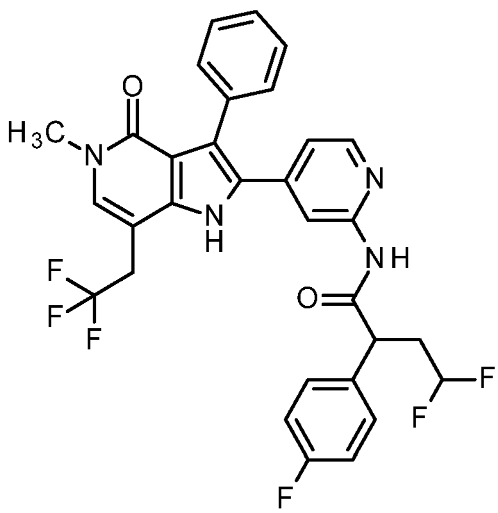

4,4-Difluoro-2-(4-fluorophenyl)-N-{4-[5-methyl-4-oxo-3-phenyl-7-(2,2,2-trifluoroethyl)-4,5,6,7-tetrahydro-1H-pyrrolo[3,2-c]pyridin-2-yl]pyridin-2-yl}butanamide (Mixture of 4 stereoisomers) (see Example 17, 100 mg) was dissolved in 1,4-dioxane (7.1 mL) and 2,3-dichloro-5,6-dicyano-1,4-benzochinone (CAS-RN:[84-58-2], 43.5 mg, 191 μmol) was added. The reaction mixture was stirred at 80 °C for 1 hour. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, filtered through a silicone coated filter and concentrated under reduced pressure. The crude product was purified by chromatography (10 g column, silica ULTRA, gradient: ethyl acetate /ethanol 0-10% ethanol) to provide the title compound: 77 mg.

LC-MS (Method 2): Rt = 1.28 min; MS (ESIpos): m/z = 599.5 $[M+H]^+$

$^1H$-NMR (400MHz, DMSO-$d_6$): δ [ppm]= 2.12 - 2.29 (m, 1H), 2.62 - 2.79 (m, 1H), 3.40 (s, 3H), 3.77 (q, 2H), 4.17 (dd, 1H), 5.97 (s, 1H), 6.67 - 6.78 (m, 1H), 7.15 - 7.32 (m, 7H), 7.41 - 7.51 (m, 3H), 8.07 - 8.17 (m, 2H), 10.83 (s, 1H), 11.95 (s, 1H).

## Example 19

(-)-4,4-difluoro-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}butanamide (Enantiomer 1)

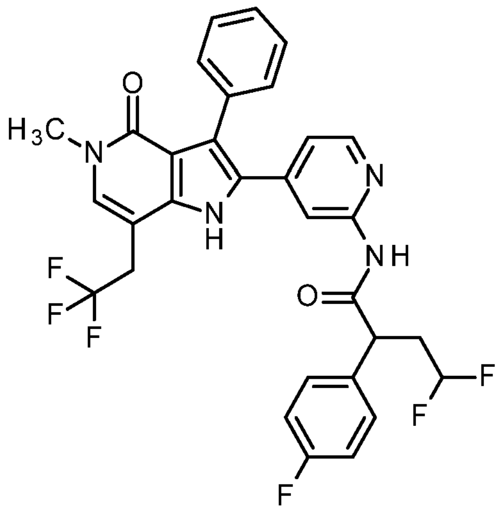

The racemic compound (see Example 18, 73.0 mg) was separated into enantiomers by preparative chiral HPLC to give enantiomer 1 (24 mg, >99 %ee, see Example 19) and enantiomer 2 (19 mg, 94.9% ee, see Example 20).

Preparative chiral HPLC method:
Instrument: PrepCon Labomatic HPLC; Column: YMC Cellulose SB 10μ, 250x50; eluent A: hexane + 0.1 vol % diethylamine; eluent B: ethanol + 0.1 vol % diethylamine; isocratic: 90%A+10%B; flow: 100 mL/min; temperature: 25°C; UV: 254 nm

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 90%A+10%B; flow: 1.4 mL/min; temperature: 25°C; UV: 254 nm

Analytical Chiral HPLC (method Example 19): Rt = 3.98 min.

LC-MS (Method 2): Rt = 1.28 min; MS (ESIpos): m/z = 599 $[M+H]^+$

$[\alpha]_D$ = -145.1° (from solution in DMSO, c = 5.3 mg/mL)

$^1$H-NMR (400MHz, DMSO-$d_6$): δ [ppm]= 2.10 - 2.30 (m, 1H), 2.63 - 2.77 (m, 1H), 3.40 (s, 3H), 3.70 - 3.85 (m, 2H), 4.17 (br dd, 1H), 5.81 - 6.18 (m, 1H), 6.67 - 6.76 (m, 1H), 7.14 - 7.31 (m, 7H), 7.42 - 7.51 (m, 3H), 8.05 - 8.20 (m, 2H), 10.82 (s, 1H), 11.95 (s, 1H).

**Example 20**

(+)-4,4-difluoro-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}butanamide (Enantiomer 2)

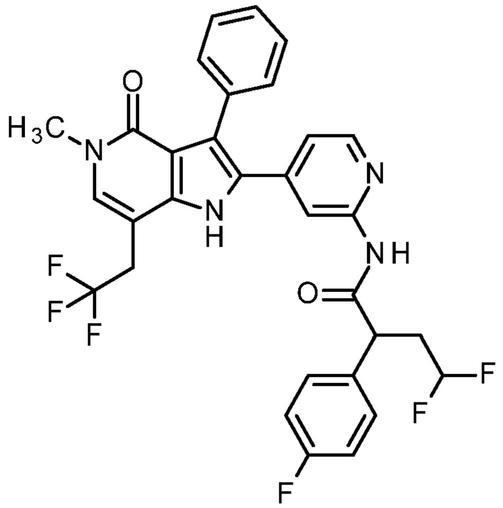

For the preparation of the racemic title compound see Example 18. Separation of enantiomers by preparative chiral HPLC (method see Example 19) gave the title compound (19 mg, 94.9 %ee).

Analytical Chiral HPLC (method see Example 19): Rt = 4.48 min.

LC-MS (Method 2): Rt = 1.28 min; MS (ESIpos): m/z = 599 $[M+H]^+$

$[\alpha]_D$ = +143.0° (from solution in DMSO, c = 4.9 mg/mL)

$^1$H-NMR (400MHz, DMSO-$d_6$): δ [ppm]= 2.11 - 2.30 (m, 1H), 2.63 - 2.79 (m, 1H), 3.40 (s, 3H), 3.77 (q, 2H), 4.17 (br dd, 1H), 5.80 - 6.17 (m, 1H), 6.66 - 6.78 (m, 1H), 7.14 - 7.33 (m, 7H), 7.41 - 7.53 (m, 3H), 8.03 - 8.18 (m, 2H), 10.82 (s, 1H), 11.95 (s, 1H).

**Example 21**

2-(4-fluorophenyl)-N-{4-[3-(4-fluorophenyl)-5-methyl-4-oxo-7-(2,2,2-trifluoroethyl)-4,5,6,7-tetrahydro-1H-pyrrolo[3,2-c]pyridin-2-yl]pyridin-2-yl}propanamide (mixture of 4 stereoisomers)

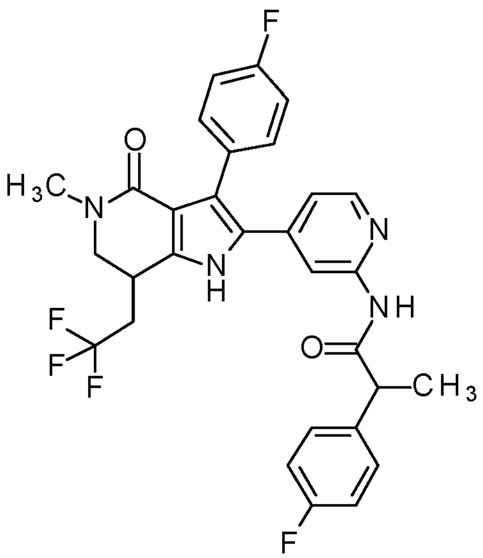

2-(2-Aminopyridin-4-yl)-3-(4-fluorophenyl)-5-methyl-7-(2,2,2-trifluoroethyl)-1,5,6,7-tetrahydro-4H-pyrrolo[3,2-c]pyridin-4-one (Racemate) (see Intermediate 19, 300 mg), 2-(4-fluorophenyl)propanoic acid (Racemate) (CAS-RN:[75908-73-5], 241 mg, 1.43 mmol), PyBOP (CAS-RN:[128625-52-5], 1.12 g, 2.15 mmol) and N,N-diisopropylethylamine (750 μL, 4.3 mmol) were dissolved in 4.2 mL N,N-dimethylacetamide and were stirred at room temperature for two days under argon atmosphere. Aqueous saturated sodium hydrogencarbonate solution was added to the reaction mixture. It was diluted with dichloromethane and the aqueous layer was extracted with dichloromethane once. The organic layer was filtered through a silicone coated filter and concentrated under reduced pressure. The crude product was purified by chromatography (10 g column, silica ULTRA, gradient: hexane / ethyl acetate 25-75% ethyl acetate) and by preparative reverse phase HPLC (eluent: acetonitrile / water with ammonia as additive) to provide the title compound: 208 mg.

LC-MS (Method 2): Rt = 1.30 min; MS (ESIpos): m/z = 569.5 $[M+H]^+$

$^1$H-NMR (400 MHz, DMSO-$d_6$) δ [ppm]: 1.035 (0.58), 1.053 (1.23), 1.070 (0.58), 1.358 (6.15), 1.375 (6.39), 2.522 (3.30), 2.665 (0.53), 2.669 (0.71), 2.673 (0.64), 2.678 (0.52), 2.706 (0.47), 2.870 (16.00), 2.892 (0.98), 2.923 (0.46), 3.418 (0.59), 3.423 (0.62), 3.435 (1.68), 3.440 (1.14), 3.445 (0.93), 3.457 (1.97), 3.740 (0.70), 3.761 (0.68), 3.990 (1.22), 4.008 (1.23), 4.025 (0.41), 6.573 (1.75), 6.577 (1.82), 6.587 (1.45), 6.590 (1.90), 7.045 (1.58), 7.067 (3.50), 7.089 (2.28), 7.132 (2.27), 7.138 (0.90), 7.150 (1.00), 7.155 (4.92), 7.161 (1.41), 7.170 (2.60), 7.177 (3.63), 7.184 (2.96), 7.190 (2.38), 7.205 (1.76), 7.384 (2.24), 7.390 (1.11), 7.398 (2.57), 7.407 (2.45), 7.415 (1.02), 7.421 (2.11), 8.045 (2.59), 8.050 (1.78), 8.058 (4.96), 10.609 (2.75), 11.861 (1.24), 11.871 (1.32).

## Example 22

2-(4-fl uorophenyl)- N-{4- [3-(4-fl uorophenyl)-5-methyl-4-oxo-7-(2,2,2-trifl uoroethyl)-4,5-d i hyd ro-1H-pyrrolo[3,2-c]pyridin-2-yl]pyridin-2-yl}propanamide (Racemate)

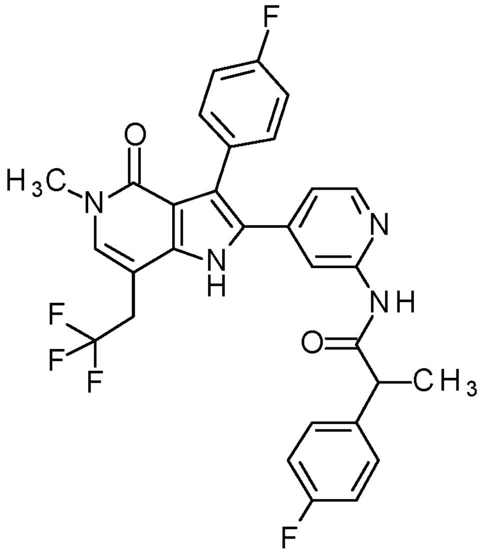

2-(4-Fluorophenyl)-N-{4-[3-(4-fluorophenyl)-5-methyl-4-oxo-7-(2,2,2-trifluoroethyl)-4,5,6,7-tetrahydro-1H-pyrrolo[3,2-c]pyridin-2-yl]pyridin-2-yl}propanamide (Mixture of 4 stereoisomers) (see Example 21, 100 mg, 176 μmol) was dissolved in 1,4-dioxane (7.5 mL) and 2,3-dichloro-5,6-dicyano-1,4-benzochinone (CAS-RN:[84-58-2], 45.9 mg, 202 μmol) was added. The reaction mixture was stirred at 80 °C for 1 hour. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, filtered through a silicone coated filter and concentrated under reduced pressure. The crude product was purified by chromatography (10 g column, silica ULTRA, gradient: ethyl acetate /ethanol 0-10% ethanol) to provide the title compound: 101 mg.

LC-MS (Method 2): Rt = 1.28 min; MS (ESIpos): m/z = 567.5 $[M+H]^+$

$^1$H-NMR (400MHz, DMSO-$d_6$): δ [ppm]= 1.37 (d, 3H), 3.41 (s, 3H), 3.77 (q, 2H), 3.95 - 4.07 (m, 1H), 6.70 - 6.82 (m, 1H), 7.05 - 7.20 (m, 4H), 7.22 - 7.30 (m, 2H), 7.35 - 7.43 (m, 2H), 7.46 (s, 1H), 8.10 - 8.20 (m, 2H), 10.69 (s, 1H), 11.95 (s, 1H).

## Example 23

(-)-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}propanamide (Enantiomer 1)

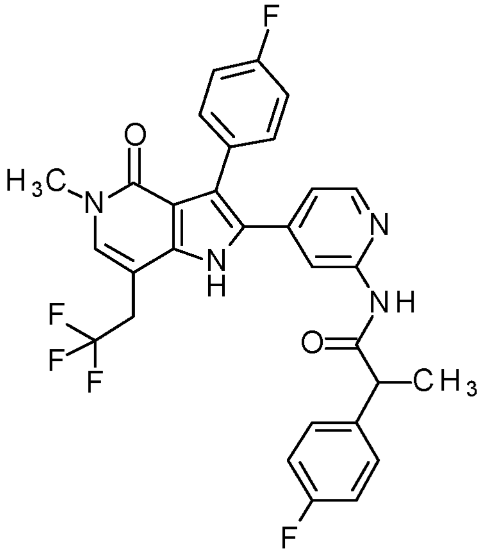

The racemic compound (see Example 22, 85.0 mg) was separated into enantiomers by preparative chiral HPLC to give enantiomer 1 (34 mg, >99 %ee, see Example 23) and enantiomer 2 (34 mg, 96.9% ee, see Example 24).

Preparative chiral HPLC method:
Instrument: PrepCon Labomatic HPLC; Column: YMC Cellulose SB 10μ, 250x50; eluent A: methyl tert-butyl ether + 0.1 vol % diethylamine; eluent B: acetonitrile; isocratic: 98%A+2%B; flow: 100 mL/min; temperature: 25°C; UV: 254 nm

Analytical chiral HPLC method:
Instrument: Waters Alliance 2695; Column: YMC Cellulose SB 3μ, 100x4.6; eluent A: methyl tert-butyl ether + 0.1 vol % diethylamine; eluent B: acetonitrile; isocratic: 98%A+2%B; flow: 1.4 mL/min; temperature: 25°C; UV: 254 nm

Analytical Chiral HPLC (method Example 23): $R_t$ = 2.63 min.

$[\alpha]_D$ = -140.6° (from solution in DMSO, c = 7.0 mg/mL)

$^1$H-NMR (400 MHz, DMSO-$d_6$) δ [ppm]: 1.183 (0.91), 1.231 (0.78), 1.361 (6.16), 1.379 (6.29), 2.518 (4.10), 2.523 (2.79), 3.407 (16.00), 3.732 (0.53), 3.761 (1.47), 3.787 (1.41), 3.815 (0.48), 4.004 (1.35), 4.022 (1.34), 6.727 (2.19), 6.731 (1.73), 6.740 (1.73), 6.744 (2.21), 7.070 (2.08), 7.075 (0.80), 7.093 (4.44), 7.098 (1.11), 7.110 (0.94), 7.115 (2.72), 7.136 (2.34), 7.141 (0.86), 7.158 (4.91), 7.176 (0.93), 7.181 (2.73), 7.237 (2.56), 7.243 (1.13), 7.251 (2.89), 7.259 (2.55), 7.267 (0.96), 7.273 (2.20), 7.390 (2.40), 7.396 (1.12), 7.404 (2.70), 7.412 (2.46), 7.421 (0.98), 7.426 (2.13), 7.465 (4.33), 8.141 (5.34), 8.147 (3.01), 8.153 (3.29), 10.690 (3.16), 11.956 (2.70).

**Example 24**

(+)-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}propanamide (Enantiomer 2)

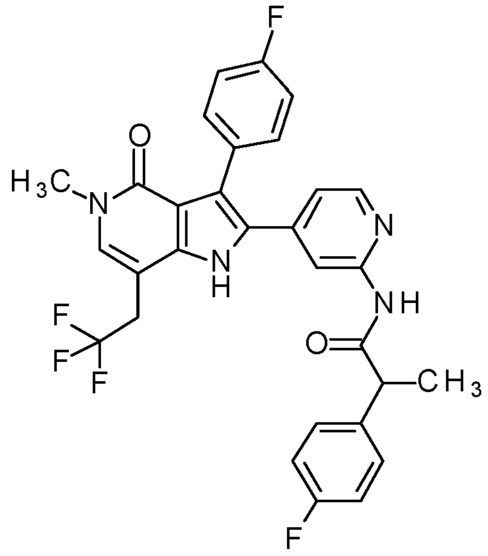

For the preparation of the racemic title compound see Example 22. Separation of enantiomers by preparative chiral HPLC (method see Example 23) gave the title compound (34 mg, 96.9 %ee).

Analytical Chiral HPLC (method see Example 23): Rt = 3.45 min.

$[\alpha]_D$ = +134.8° (from solution in DMSO, c = 4.4 mg/mL)

$^1$H-NMR (400 MHz, DMSO-$d_6$) $\delta$ [ppm]: 0.783 (0.51), 1.103 (0.53), 1.174 (1.18), 1.183 (1.82), 1.231 (0.88), 1.255 (0.46), 1.361 (6.31), 1.379 (6.33), 2.518 (2.86), 2.523 (1.96), 2.539 (0.76), 3.407 (16.00), 3.732 (0.54), 3.760 (1.50), 3.788 (1.43), 3.815 (0.48), 3.987 (0.40), 4.004 (1.37), 4.022 (1.35), 6.727 (2.18), 6.731 (1.83), 6.741 (1.78), 6.744 (2.18), 7.070 (2.10), 7.075 (0.82), 7.092 (4.42), 7.110 (0.93), 7.115 (2.72), 7.136 (2.32), 7.141 (0.86), 7.158 (4.90), 7.176 (0.93), 7.181 (2.72), 7.237 (2.53), 7.243 (1.17), 7.252 (2.93), 7.259 (2.60), 7.268 (0.97), 7.273 (2.14), 7.391 (2.41), 7.396 (1.12), 7.404 (2.70), 7.412 (2.49), 7.421 (1.00), 7.426 (2.13), 7.464 (4.28), 8.141 (4.79), 8.148 (3.06), 8.153 (3.36), 10.690 (3.18), 11.956 (2.71).

## Example 25

4,4-difluoro-2-(4-fluorophenyl)-N-{4-[3-(4-fluorophenyl)-5-methyl-4-oxo-7-(2,2,2-trifluoroethyl)-4,5,6,7-tetrahydro-1H-pyrrolo[3,2-c]pyridin-2-yl]pyridin-2-yl}butanamide (mixture of 4 stereoisomers)

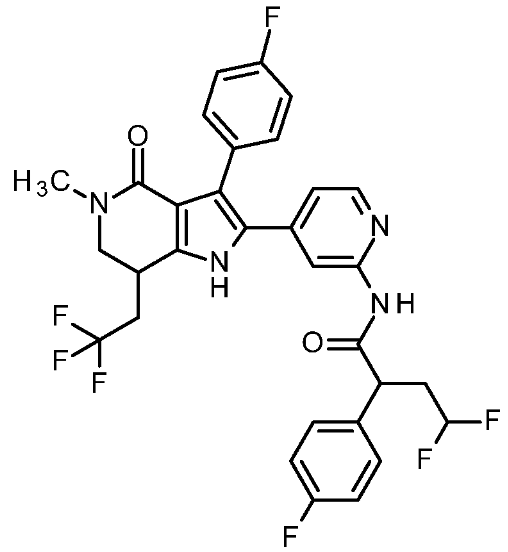

2-(2-Aminopyridin-4-yl)-3-(4-fluorophenyl)-5-methyl-7-(2,2,2-trifluoroethyl)-1,5,6,7-tetrahydro-4H-pyrrolo[3,2-c]pyridin-4-one (Racemate) (see Intermediate 19, 300 mg), 4,4-difluoro-2-(4-fluorophenyl)butanoic acid (Racemate) (CAS-RN:[1538957-14-0], 313 mg, 1.43 mmol), PyBOP (CAS-RN:[128625-52-5], 1.12 g, 2.15 mmol) and N,N-diisopropylethylamine (750 μL, 4.3 mmol) were dissolved in 4.2 mL N,N-dimethylacetamide and were stirred at room temperature under argon atmosphere for 2 days. Aqueous saturated sodium hydrogencarbonate solution and water were added to the reaction mixture. It was diluted with dichloromethane and the aqueous layer was extracted with dichloromethane once. The organic layer was filtered through a silicone coated filter and concentrated under reduced pressure. The crude product was purified by chromatography (10 g column, silica ULTRA, gradient: hexane / ethyl acetate 25-75% ethyl acetate) to provide the title compound: 420 mg.

LC-MS (Method 2): Rt = 1.34 min; MS (ESIpos): m/z = 619.5 $[M+H]^+$

$^1$H-NMR (500MHz, DMSO-$d_6$): δ [ppm]= 2.12 - 2.28 (m, 1H), 2.60 - 2.77 (m, 2H), 2.81 - 2.96 (m, 4H), 3.40 - 3.49 (m, 2H), 3.71 - 3.80 (m, 1H), 4.15 (dd, 1H), 5.80 - 6.15 (m, 1H), 6.63 (dd, 1H), 7.06 (td, 2H), 7.13 - 7.22 (m, 4H), 7.41 - 7.47 (m, 2H), 7.99 (br s, 1H), 8.07 (d, 1H), 10.75 (s, 1H), 11.87 (d, 1H).

**Example 26**

4,4-difluoro-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}butanamide (Racemate)

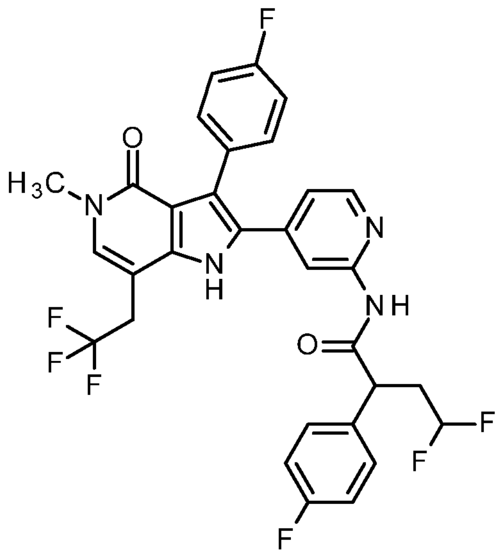

4,4-Difluoro-2-(4-fluorophenyl)-N-{4-[3-(4-fluorophenyl)-5-methyl-4-oxo-7-(2,2,2-trifluoroethyl)-4,5,6,7-tetrahydro-1H-pyrrolo[3,2-c]pyridin-2-yl]pyridin-2-yl}butanamide (Mixture of 4 stereoisomers) (see Example 25, 100 mg) was dissolved in 1,4-dioxane (6.9 mL) and 2,3-dichloro-5,6-dicyano-1,4-benzochinone (CAS-RN:[84-58-2], 40.4 mg, 178 μmol) was added. The reaction mixture was stirred at 80 °C for 1 hour under argon atmosphere. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, filtered through a silicone coated filter and concentrated under reduced pressure. The crude product was purified by chromatography (10 g column, silica ULTRA, gradient: ethyl acetate /ethanol 0-10% ethanol) to provide the title compound: 83 mg.

LC-MS (Method 2): Rt = 1.30 min; MS (ESIneg): m/z = 617.5 $[M+H]^-$

$^1$H-NMR (400MHz, DMSO-$d_6$): δ [ppm]= 2.19 (br dd, 1H), 2.62 - 2.80 (m, 1H), 3.41 (s, 3H), 3.77 (q, 2H), 4.16 (br dd, 1H), 5.74 - 6.22 (m, 1H), 6.80 (dd, 1H), 7.05 - 7.14 (m, 2H), 7.15 - 7.33 (m, 4H), 7.40 - 7.55 (m, 3H), 8.07 (s, 1H), 8.17 (d, 1H), 10.82 (s, 1H), 11.97 (s, 1H).

**Example 27**

4,4-difluoro-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}butanamide (Enantiomer 1)

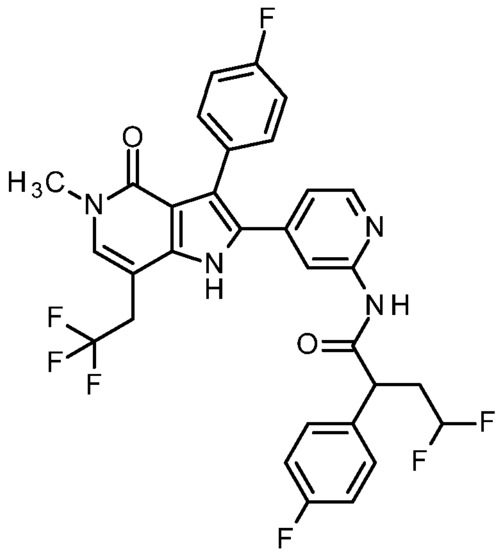

The racemic compound (see Example 26, 67.0 mg) was separated into enantiomers by preparative chiral HPLC to give enantiomer 1 (20 mg, 87.0 %ee, see Example 27) and enantiomer 2 (19 mg, >99% ee, see Example 28).

Preparative chiral HPLC method:
Instrument: PrepCon Labomatic HPLC-2; Column: YMC Cellulose SB 10μ, 250x50; eluent A: methyl tert-butyl ether + 0.1 vol % diethylamine; eluent B: acetonitrile; isocratic: 98%A+2%B; flow: 100 mL/min; temperature: 25°C; UV: 280 nm
Analytical chiral HPLC method:
Instrument: Waters Alliance 2695; Column: YMC Cellulose SB 3μ, 100x4.6; eluent A: methyl tert-butyl ether + 0.1 vol % diethylamine; eluent B: acetonitrile; isocratic: 98%A+2%B; flow: 1.4 mL/min; temperature: 25°C; UV: 280 nm

Analytical Chiral HPLC (method Example 27): Rt = 1.44 min.
$^1$H-NMR (400MHz, DMSO-$d_6$): δ [ppm]= 2.14 - 2.30 (m, 1H), 2.60 - 2.80 (m, 1H), 3.41 (s, 3H), 3.77 (q, 2H), 4.16 (dd, 1H), 5.80 - 6.16 (m, 1H), 6.80 (dd, 1H), 7.05 - 7.13 (m, 2H), 7.15 - 7.28 (m, 4H), 7.40 - 7.53 (m, 3H), 8.07 (s, 1H), 8.14 - 8.21 (m, 1H), 10.82 (s, 1H), 11.96 (s, 1H).

**Example 28**

4,4-difluoro-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}butanamide (Enantiomer 2)

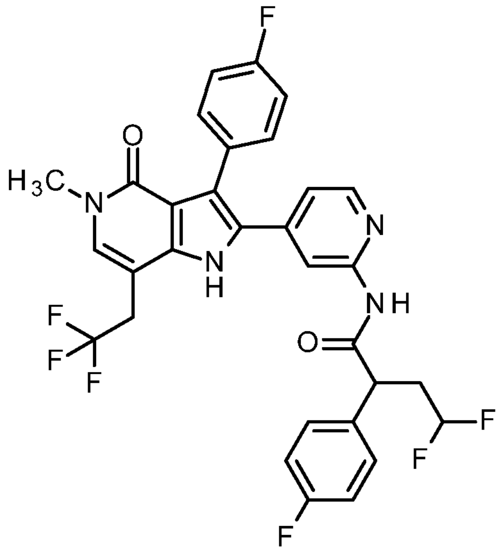

For the preparation of the racemic title compound see Example 26. Separation of enantiomers by preparative chiral HPLC (method see Example 27) gave the title compound (19 mg, >99 %ee).

Analytical Chiral HPLC (method see Example 27): Rt = 2.45 min.

$^1$H-NMR (400MHz, DMSO-$d_6$): δ [ppm]= 2.10 - 2.29 (m, 1H), 2.60 - 2.79 (m, 1H), 3.41 (s, 3H), 3.68 - 3.84 (m, 2H), 4.16 (dd, 1H), 5.76 - 6.16 (m, 1H), 6.80 (dd, 1H), 7.05 - 7.14 (m, 2H), 7.15 - 7.30 (m, 4H), 7.41 - 7.51 (m, 3H), 8.07 (s, 1H), 8.17 (d, 1H), 10.82 (s, 1H), 11.97 (s, 1H).

## Example 29

2-(4-fluorophenyl)-N-{4-[3-(4-fluorophenyl)-5,7-dimethyl-4-oxo-4,5,6,7-tetrahydro-1H-pyrrolo[3,2-c]pyridin-2-yl]pyridin-2-yl}propanamide (mixture of 4 stereoisomers)

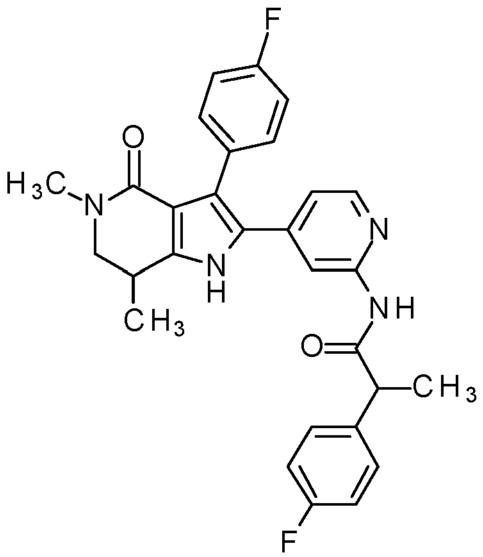

2-(2-Aminopyridin-4-yl)-3-(4-fluorophenyl)-5,7-dimethyl-1,5,6,7-tetrahydro-4H-pyrrolo[3,2-c]pyridin-4-one (Racemate) (see Intermediate 22, 200 mg), 2-(4-fluorophenyl)propanoic acid (Racemate) (CAS-RN:[ 75908-73-5], 192 mg, 1.14 mmol), PyBOP (CAS-RN:[128625-52-5], 891 mg, 1.71 mmol) and N,N-diisopropylethylamine (600 µL, 3.4 mmol) were dissolved in 3.3 mL N,N-dimethylacetamide and were stirred at room temperature under argon atmosphere for 2 days. Aqueous saturated sodium hydrogencarbonate solution and water were added to the reaction mixture. It was diluted with dichloromethane and the aqueous layer was extracted with dichloromethane once. The organic layer was

filtered through a silicone coated filter and concentrated under reduced pressure. The crude product was purified by chromatography (10 g column, silica ULTRA, gradient: dichloromethane / ethanol 0-3% ethanol) to provide the title compound: 209 mg.

LC-MS (Method 2): $R_t$ = 1.22 min; MS (ESIpos): m/z = 501.5 $[M+H]^+$

$^1$H-NMR (400MHz, DMSO-$d_6$): δ [ppm]= 1.31 (dd, 3H), 1.35 - 1.40 (m, 3H), 2.85 (s, 3H), 3.14 - 3.29 (m, 2H), 3.62 (ddd, 1H), 3.99 (q, 1H), 6.63 (dd, 1H), 7.02 - 7.10 (m, 2H), 7.12 - 7.23 (m, 4H), 7.36 - 7.45 (m, 2H), 7.96 (s, 1H), 8.04 (d, 1H), 10.67 (br s, 1H), 11.78 (br d, 1H).

**Example 30**

2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}propanamide (Racemate)

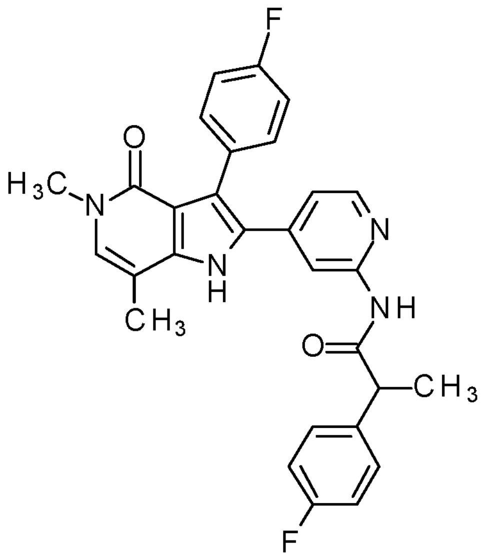

2-(4-Fluorophenyl)-N-{4-[3-(4-fluorophenyl)-5,7-dimethyl-4-oxo-4,5,6,7-tetrahydro-1H-pyrrolo[3,2-c]pyridin-2-yl]pyridin-2-yl}propanamide (mixture of 4 stereoisomers) (see Example 29, 200 mg) was dissolved in 1,4-dioxane (17 mL) and 2,3-dichloro-5,6-dicyano-1,4-benzochinone (CAS-RN:[84-58-2], 104 mg, 459 μmol) was added. The reaction mixture was stirred at 80 °C for 1 hour under argon atmosphere. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, filtered through a silicone coated filter and concentrated under reduced pressure. The crude product was purified by preparative reverse phase HPLC (eluent: acetonitrile / water with ammonia as additive) to provide the title compound: 37 mg.

LC-MS (Method 2): Rt = 1.23 min; MS (ESIpos): m/z = 499 $[M+H]^+$

$^1$H-NMR (400 MHz, DMSO-$d_6$) δ [ppm]: 1.359 (6.45), 1.376 (6.72), 2.235 (9.40), 2.237 (10.03), 2.518 (7.94), 2.523 (5.52), 2.848 (0.47), 3.159 (1.92), 3.172 (2.04), 3.356 (16.00), 3.533 (5.58), 4.000 (1.46), 4.018 (1.40), 4.035 (0.47), 4.097 (0.46), 4.110 (0.44), 6.740 (0.86), 6.744 (0.99), 6.748 (2.18), 6.753 (2.54), 6.757 (1.16), 6.761 (2.00), 6.765 (2.09), 7.058 (1.99), 7.080 (4.90), 7.103 (4.08), 7.125 (1.18), 7.136 (3.04), 7.158 (6.35), 7.181 (3.71), 7.192 (3.38), 7.195 (3.36), 7.215 (1.40), 7.222 (2.90), 7.228 (2.16), 7.236 (3.75), 7.244 (2.78), 7.251 (1.40), 7.258 (2.20), 7.389 (2.43), 7.396 (1.70), 7.403 (3.01), 7.411 (3.33), 7.419 (1.78), 7.425 (2.29), 7.432 (1.11), 8.108 (1.05), 8.118 (3.20), 8.131 (4.09), 8.149 (1.22), 8.452 (2.04), 9.755 (2.56), 10.655 (3.08), 10.673 (1.19), 11.830 (2.57), 11.988 (0.61).

**Example 31**

(-)-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}propanamide (Enantiomer 1)

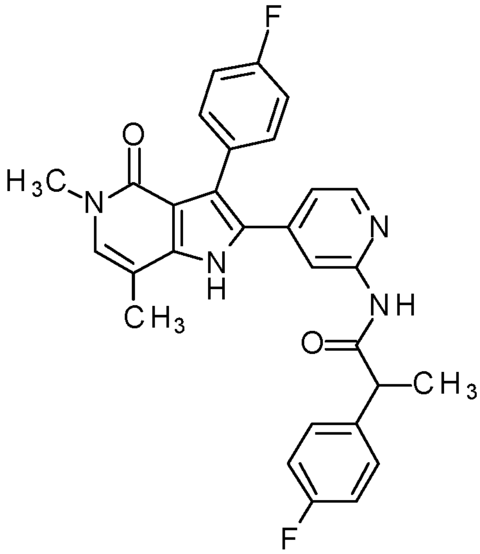

The racemic compound (see Example 30, 17.0 mg) was separated into enantiomers by preparative chiral HPLC to give enantiomer 1 (10 mg, >99% ee, see Example 31) and enantiomer 2 (7 mg, >99% ee, see Example 32).

Preparative chiral HPLC method:
Instrument: PrepCon Labomatic HPLC; Column: YMC Cellulose SB 10μ, 250x50; eluent A: hexane + 0.1 vol % diethylamine; eluent B: 2-propanol; isocratic: 90%A+10%B; flow: 140 mL/min; temperature: 25°C; UV: 254 nm

Analytical chiral HPLC method:
Instrument: Thermo Fisher UltiMate 3000; Column: YMC Cellulose SB 3μ, 100x4.6; eluent A: hexane + 0.1 vol % diethylamine; eluent B: 2-propanol; isocratic: 90%A+10%B; flow: 1.4 mL/min; temperature: 25°C; UV: 254 nm

Analytical Chiral HPLC (method Example 31): Rt = 4.48 min.

LC-MS (Method 2): Rt = 1.23 min; MS (ESIpos): m/z = 499 $[M+H]^+$

$[\alpha]_D$ = -95.2° (from solution in DMSO, c = 10.0 mg/mL)

$^1$H NMR (400 MHz, DMSO-$d_6$) δ ppm 1.37 (d, 3 H) 2.24 (d, 3 H) 3.36 (s, 3 H) 4.01 (q, 1 H) 6.76 (dd, 1 H) 7.00 - 7.12 (m, 2 H) 7.12 - 7.21 (m, 3 H) 7.22 - 7.31 (m, 2 H) 7.37 - 7.44 (m, 2 H) 8.08 - 8.20 (m, 2 H) 10.66 (s, 1 H) 11.83 (s, 1 H).

**Example 32**

(+)-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}propanamide (Enantiomer 2)

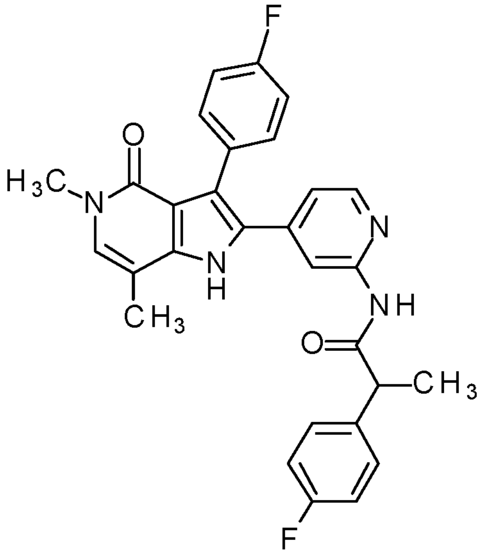

For the preparation of the racemic title compound see Example 30. Separation of enantiomers by preparative chiral HPLC (method see Example 31) gave the title compound (7 mg, >99 %ee).

Analytical Chiral HPLC (method see Example 31): Rt = 7.36 min.

LC-MS (Method 2): Rt = 1.23 min; MS (ESIpos): m/z = 499 $[M+H]^+$

$[\alpha]_D$ = +121.9° (from solution in DMSO, c = 7.0 mg/mL)

$^1$H NMR (400 MHz, DMSO-$d_6$) $\delta$ ppm 1.37 (d, 3 H) 2.24 (d, 3 H) 3.36 (s, 3 H) 4.01 (q, 1 H) 6.76 (dd, 1 H) 7.01 - 7.11 (m, 2 H) 7.12 - 7.20 (m, 3 H) 7.21 - 7.30 (m, 2 H) 7.37 - 7.47 (m, 2 H) 8.04 - 8.20 (m, 2 H) 10.66 (s, 1 H) 11.83 (s, 1 H).

## Example 33

4,4-difluoro-2-(4-fluorophenyl)-N-{4-[3-(4-fluorophenyl)-5,7-dimethyl-4-oxo-4,5,6,7-tetrahydro-1H-pyrrolo[3,2-c]pyridin-2-yl]pyridin-2-yl}butanamide (mixture of 4 stereoisomers)

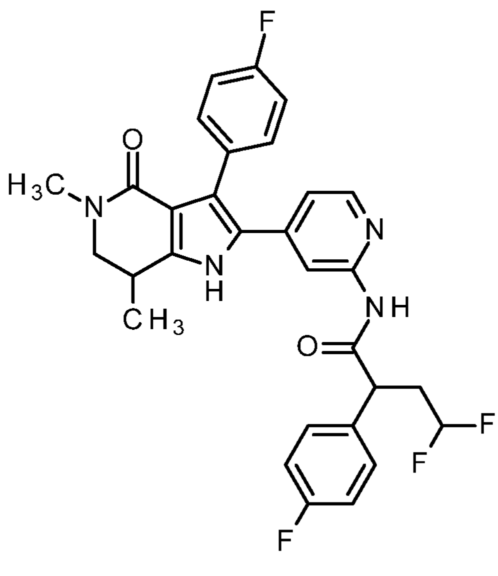

2-(2-Aminopyridin-4-yl)-3-(4-fluorophenyl)-5,7-dimethyl-1,5,6,7-tetrahydro-4H-pyrrolo[3,2-c]pyridin-4-one (Racemate) (see Intermediate 22, 200 mg), 4,4-difluoro-2-(4-fluorophenyl)butanoic acid (Racemate) (CAS-RN:[1538957-14-0], 249 mg, 1.14 mmol), PyBOP (CAS-RN:[128625-52-5], 891 mg, 1.71 mmol) and N,N-diisopropylethylamine (600 μL, 3.4 mmol) were dissolved in 3.3 mL N,N-dimethylacetamide and were stirred at room temperature under argon atmosphere for 2 days. Aqueous saturated sodium hydrogencarbonate solution and water were added to the reaction mixture. It was diluted with dichloromethane and the aqueous layer was extracted with dichloromethane once. The organic layer was filtered through a silicone coated filter and concentrated under reduced pressure. The crude product was purified by chromatography (10 g column, silica ULTRA, gradient: dichloromethane / ethanol 0-3% ethanol) and and by preparative reverse phase HPLC (eluent: acetonitrile / water with ammonia as additive) to provide the title compound: 161 mg.

LC-MS (Method 1): Rt = 1.21 min; MS (ESIpos): m/z = 551.8 $[M+H]^+$

$^1$H-NMR (400MHz, DMSO-$d_6$): δ [ppm]= 1.31 (dd, 3H), 2.08 - 2.28 (m, 1H), 2.59 - 2.77 (m, 1H), 2.85 (s, 3H), 3.18 - 3.28 (m, 2H), 3.61 (ddd, 1H), 4.14 (dd, 1H), 5.81 - 6.14 (m, 1H), 6.65 (dd, 1H), 7.01 - 7.09 (m, 2H), 7.14 - 7.27 (m, 4H), 7.40 - 7.49 (m, 2H), 7.95 (s, 1H), 8.05 (d, 1H), 10.71 (s, 1H), 11.75 (d, 1H).

## Example 34

4,4-difluoro-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}butanamide (Racemate)

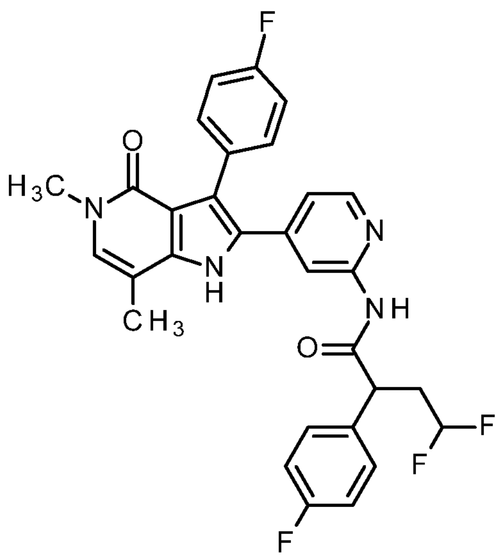

4,4-Difluoro-2-(4-fluorophenyl)-N-{4-[3-(4-fluorophenyl)-5,7-dimethyl-4-oxo-4,5,6,7-tetrahydro-1H-pyrrolo[3,2-c]pyridin-2-yl]pyridin-2-yl}butanamide (mixture of 4 stereoisomers) (see Example 33, 150 mg) was dissolved in 1,4-dioxane (12 mL) and 2,3-dichloro-5,6-dicyano-1,4-benzochinone (CAS-RN:[84-58-2], 71.1 mg, 313 μmol) was added. The reaction mixture was stirred at 80 °C for 2 hours under argon atmosphere. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, filtered through a silicone coated filter and concentrated under reduced pressure. The crude product was purified by chromatography (10 g column, silica ULTRA, gradient: ethyl acetate / ethanol 0-3% ethanol) to provide the title compound: 121 mg.

LC-MS (Method 2): Rt = 1.22 min; MS (ESIpos): m/z = 549 $[M+H]^+$

$^1$H-NMR (400MHz, DMSO-$d_6$): δ [ppm]= 2.24 (d, 4H), 2.60 - 2.80 (m, 1H), 3.36 (s, 3H), 4.16 (br dd, 1H), 5.78 - 6.15 (m, 1H), 6.82 (dd, 1H), 7.04 - 7.13 (m, 2H), 7.15 - 7.28 (m, 5H), 7.40 - 7.50 (m, 2H), 8.07 (s, 1H), 8.13 - 8.17 (m, 1H), 10.79 (s, 1H), 11.84 (s, 1H).

**Example 35**

(-)-4,4-difluoro-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}butanamide (Enantiomer 1)

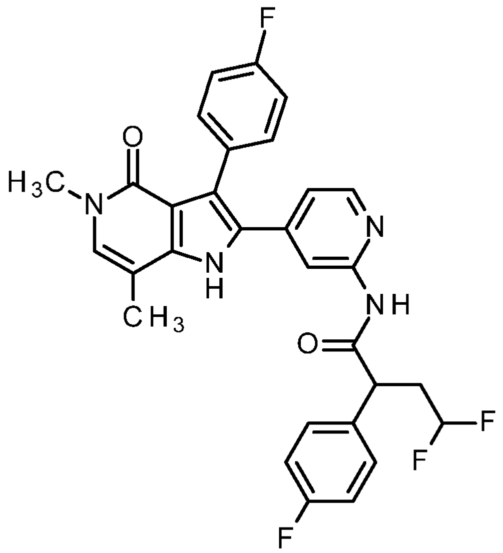

The racemic compound (see Example 34, 110 mg) was separated into enantiomers by preparative chiral HPLC to give enantiomer 1 (38 mg, >99% ee, see Example 35) and enantiomer 2 (35 mg, 95.9% ee, see Example 36).

Preparative chiral HPLC method:
Instrument: PrepCon Labomatic HPLC; Column: YMC Cellulose SB 10μ, 250x50; eluent A: hexane + 0.1 vol % diethylamine; eluent B: ethanol + 0.1 vol % diethylamine; gradient: 0-50 min 5-15% B; flow: 100 mL/min; temperature: 25°C; UV: 254 nm
Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 94%A+6%B; flow: 1.4 mL/min; temperature: 25°C; UV: 254 nm

Analytical Chiral HPLC (method Example 35): Rt = 8.44 min.

$[\alpha]_D$ = -164.2° (from solution in DMSO, c = 4.5 mg/mL)
$^1$H NMR (400 MHz, DMSO-$d_6$) δ ppm 2.09 - 2.27 (m, 4 H) 2.61 - 2.79 (m, 1 H) 3.35 - 3.37 (m, 3 H) 4.16 (br dd, 1 H) 5.81 - 6.18 (m, 1 H) 6.82 (dd, 1 H) 7.08 (t, 2 H) 7.14 - 7.31 (m, 5 H) 7.45 (dd, 2 H) 8.07 (s, 1 H) 8.14 (d, 1 H) 10.79 (s, 1 H) 11.84 (s, 1 H).

**Example 36**

(+)-4,4-difluoro-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}butanamide (Enantiomer 2)

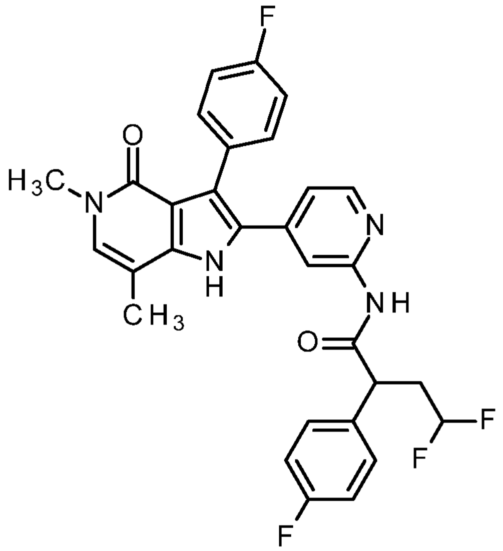

For the preparation of the racemic title compound see Example 34. Separation of enantiomers by preparative chiral HPLC (method see Example 35) gave the title compound (35 mg, 95.9 %ee).

Analytical Chiral HPLC (method see Example 35): Rt = 9.34 min.

$[\alpha]_D$ = +141.1° (from solution in DMSO, c = 4.5 mg/mL)

$^1$H NMR (400 MHz, DMSO-$d_6$) $\delta$ ppm 2.12 - 2.29 (m, 4 H) 2.67 (br s, 1 H) 3.35 (br s, 3 H) 4.11 - 4.22 (m, 1 H) 5.81 - 6.19 (m, 1 H) 6.82 (br d, 1 H) 7.01 - 7.15 (m, 2 H) 7.15 - 7.30 (m, 5 H) 7.39 - 7.50 (m, 2 H) 8.07 (s, 1 H) 8.14 (br d, 1 H) 10.79 (s, 1 H) 11.84 (s, 1 H).

## Example 37

N-{4-[5,7-dimethyl-4-oxo-3-phenyl-4,5,6,7-tetrahydro-1H-pyrrolo[3,2-c]pyridin-2-yl]pyridin-2-yl}-2-(4-fluorophenyl)propanamide (mixture of 4 stereoisomers)

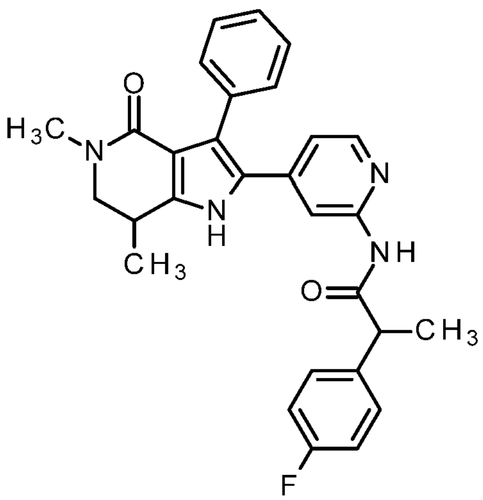

2-(2-Aminopyridin-4-yl)-5,7-dimethyl-3-phenyl-1,5,6,7-tetrahydro-4H-pyrrolo[3,2-c]pyridin-4-one (Racemate) (see Intermediate 25, 200 mg), 2-(4-fluorophenyl)propanoic acid (Racemate) (CAS-RN:[75908-73-5], 202 mg, 1.20 mmol), PyBOP (CAS-RN:[128625-52-5], 939 mg, 1.81 mmol) and N,N-diisopropylethylamine (630 $\mu$L, 3.6 mmol) were dissolved in 3.5 mL N,N-dimethylacetamide and were stirred at room temperature under argon atmosphere for 2 days. Aqueous saturated sodium hydrogencarbonate solution and water were added to the reaction mixture. It was diluted

with dichloromethane and the aqueous layer was extracted with dichloromethane once. The organic layer was filtered through a silicone coated filter and concentrated under reduced pressure. The crude product was purified by chromatography (10 g column, silica ULTRA, gradient: dichloromethane / ethanol 0-3% ethanol) and by preparative reverse phase HPLC (eluent: acetonitrile / water with ammonia as additive) to provide the title compound: 63 mg.

LC-MS (Method 1): Rt = 1.13 min; MS (ESIneg): m/z = 481 [M-H]-

$^1$H-NMR (400MHz, DMSO-$d_6$): δ [ppm]= 1.31 (dd, 3H), 1.37 (d, 3H), 2.85 (s, 3H), 3.12 - 3.29 (m, 2H), 3.62 (ddd, 1H), 4.00 (q, 1H), 6.49 (dd, 1H), 7.10 - 7.19 (m, 4H), 7.21 - 7.26 (m, 3H), 7.39 - 7.47 (m, 2H), 7.97 (d, 1H), 8.07 (s, 1H), 10.58 (s, 1H), 11.72 (d, 1H).

**Example 38**

N-[4-(5,7-dimethyl-4-oxo-3-phenyl-4,5-dihydro-1H-pyrrolo[3,2-c]pyridin-2-yl)pyridin-2-yl]-2-(4-fluorophenyl)propanamide (Racemate)

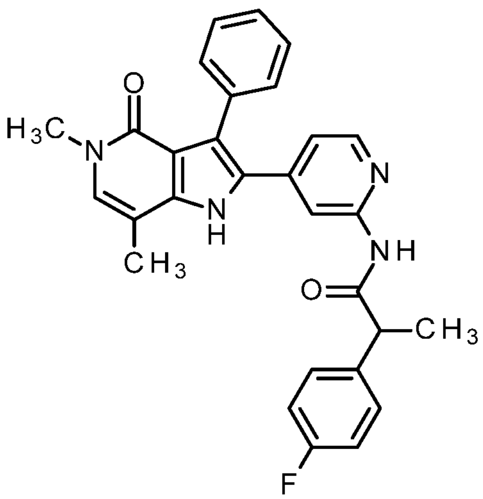

N-{4-[5,7-Dimethyl-4-oxo-3-phenyl-4,5,6,7-tetrahydro-1H-pyrrolo[3,2-c]pyridin-2-yl]pyridin-2-yl}-2-(4-fluorophenyl)propanamide (mixture of 4 stereoisomers) (see Example 37, 55.0 mg) was dissolved in 1,4-dioxane (4.9 mL) and 2,3-dichloro-5,6-dicyano-1,4-benzochinone (CAS-RN:[84-58-2], 29.8 mg, 131 µmol) was added. The reaction mixture was stirred at 80 °C for 2 hours under argon atmosphere. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, filtered through a silicone coated filter and concentrated under reduced pressure. The crude product was purified by chromatography (10 g column, silica ULTRA, gradient: ethyl acetate / ethanol 0-3% ethanol) to provide the title compound: 31 mg.

LC-MS (Method 2): Rt = 1.19 min; MS (ESIpos): m/z = 481.5 $[M+H]^+$

$^1$H-NMR (400MHz, DMSO-$d_6$): δ [ppm]= 1.38 (d, 3H), 2.24 (d, 3H), 3.35 (s, 3H), 3.91 - 4.15 (m, 1H), 6.58 - 6.81 (m, 1H), 7.11 - 7.32 (m, 8H), 7.37 - 7.49 (m, 2H), 8.00 - 8.10 (m, 1H), 8.18 (s, 1H), 10.66 (s, 1H), 11.81 (s, 1H).

**Example 39**

N-{4-[5,7-dimethyl-4-oxo-3-phenyl-4,5,6,7-tetrahydro-1H-pyrrolo[3,2-c]pyridin-2-yl]pyridin-2-yl}-4,4-difluoro-2-(4-fluorophenyl)butanamide (mixture of 4 stereoisomers)

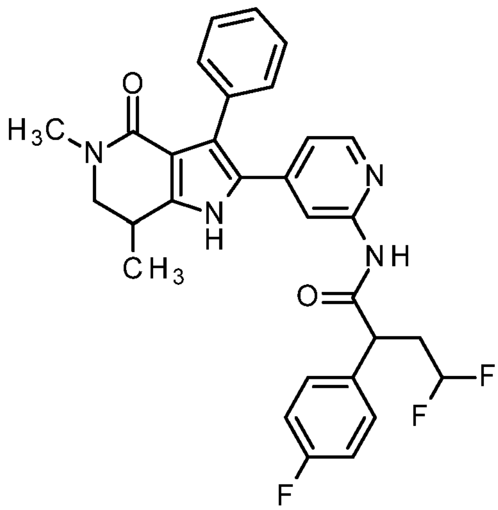

2-(2-Aminopyridin-4-yl)-5,7-dimethyl-3-phenyl-1,5,6,7-tetrahydro-4H-pyrrolo[3,2-c]pyridin-4-one (Racemate) (see Intermediate 25, 200 mg), 4,4-difluoro-2-(4-fluorophenyl)butanoic acid (Racemate) (CAS-RN:[1538957-14-0], 263 mg, 1.20 mmol), PyBOP (CAS-RN:[128625-52-5], 939 mg, 1.81 mmol) and N,N-diisopropylethylamine (630 µL, 3.6 mmol) were dissolved in 3.5 mL N,N-dimethylacetamide and were stirred at room temperature under argon atmosphere for 2 days. Aqueous saturated sodium hydrogencarbonate solution and water were added to the reaction mixture. It was diluted with dichloromethane and the aqueous layer was extracted with dichloromethane once. The organic layer was filtered through a silicone coated filter and concentrated under reduced pressure. The crude product was purified by chromatography (10 g column, silica ULTRA, gradient: dichloromethane / ethanol 0-3% ethanol) and and by preparative reverse phase HPLC (eluent: acetonitrile / water with ammonia as additive) to provide the title compound: 65 mg.

LC-MS (Method 1): Rt = 1.19 min; MS (ESIneg): m/z = 531 [M-H]-

$^1$H-NMR (400MHz, DMSO-$d_6$): δ [ppm]= 1.32 (dd, 3H), 2.10 - 2.28 (m, 1H), 2.60 - 2.78 (m, 1H), 2.85 (s, 3H), 3.11 - 3.28 (m, 2H), 3.57 - 3.67 (m, 1H), 4.15 (br dd, 1H), 5.80 - 6.15 (m, 1H), 6.55 (dd, 1H), 7.12 - 7.27 (m, 7H), 7.40 - 7.52 (m, 2H), 7.96 - 8.05 (m, 2H), 10.72 (s, 1H), 11.73 (d, 1H).

## Example 40

N-[4-(5,7-dimethyl-4-oxo-3-phenyl-4,5-dihydro-1H-pyrrolo[3,2-c]pyridin-2-yl)pyridin-2-yl]-4,4-difluoro-2-(4-fluorophenyl)butanamide (Racemate)

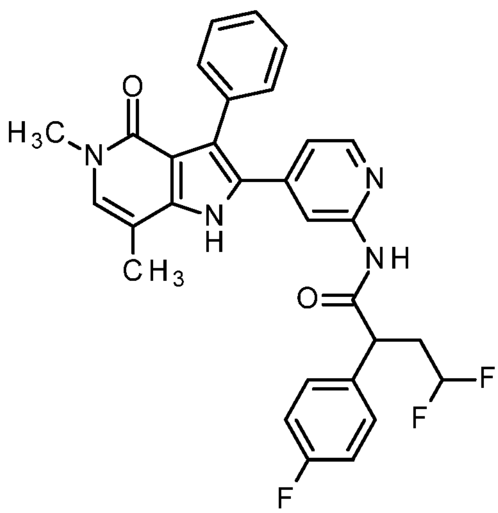

N-{4-[5,7-Dimethyl-4-oxo-3-phenyl-4,5,6,7-tetrahydro-1H-pyrrolo[3,2-c]pyridin-2-yl]pyridin-2-yl}-4,4-difluoro-2-(4-fluorophenyl)butanamide (mixture of 4 stereoisomers) (see Example 39, 55.0 mg) was dissolved in 1,4-dioxane (4.4 mL) and 2,3-dichloro-5,6-dicyano-1,4-benzochinone (CAS-RN:[84-58-2], 27.0 mg, 119 μmol) was added. The reaction mixture was stirred at 80 °C for 2 hours under argon atmosphere. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, filtered through a silicone coated filter and concentrated under reduced pressure. The crude product was purified by chromatography (10 g column, silica ULTRA, gradient: ethyl acetate / ethanol 0-3% ethanol) to provide the title compound: 38 mg.

LC-MS (Method 2): $R_t$ = 1.21 min; MS (ESIpos): m/z = 531 $[M+H]^+$

$^1$H-NMR (400MHz, DMSO-$d_6$): δ [ppm]= 2.24 (d, 4H), 2.59 - 2.79 (m, 1H), 3.35 (s, 3H), 4.17 (br dd, 1H), 5.79 - 6.20 (m, 1H), 6.69 - 6.77 (m, 1H), 7.15 - 7.31 (m, 8H), 7.41 - 7.51 (m, 2H), 8.02 - 8.18 (m, 2H), 10.80 (s, 1H), 11.82 (s, 1H).

## Example 41

4,4-difluoro-2-(4-fluorophenyl)-N-{4-[5,6,7-trimethyl-4-oxo-3-phenyl-4,5,6,7-tetrahydro-1H-pyrrolo[3,2-c]pyridin-2-yl]pyridin-2-yl}butanamide (mixture of 8 stereoisomers)

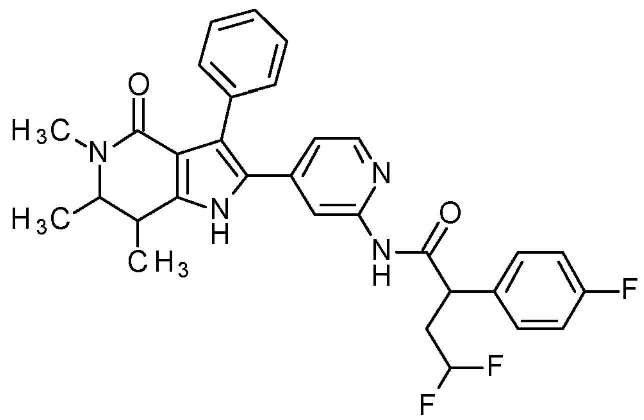

2-(2-Aminopyridin-4-yl)-5,6,7-trimethyl-3-phenyl-1,5,6,7-tetrahydro-4H-pyrrolo[3,2-c]pyridin-4-one (mixture of 4 stereoisomers) (see Intermediate 34, 46.5 mg), 4,4-difluoro-2-(4-fluorophenyl)butanoic acid (Racemate) (CAS-RN:[1538957-14-0], 58.6 mg, 268 μmol), PyBOP (CAS-RN:[128625-52-5], 349 mg, 671 μmol) and N,N-diisopropylethylamine (140 μL, 810 μmol) were dissolved in 1.2 mL N,N-dimethylacetamide and were stirred at room temperature under nitrogen atmosphere overnight. Aqueous saturated sodium hydrogencarbonate solution was added to the reaction mixture. It was diluted with dichloromethane and the aqueous layer was extracted with dichloromethane once. The organic layer was dried using a waterresistant filter and concentrated under reduced pressure. The crude product was purified by preparative reverse phase HPLC (eluent: acetonitrile / water with ammonia as additive) to provide 36.1 mg of the title compound.

LC-MS (Method 2): $R_t$ = 1.25 min; MS (ESIneg): m/z = 545 [M-H]-

$^1$H-NMR (400MHz, DMSO-$d_6$): δ [ppm]= 1.00 - 1.35 (m, 7H), 2.12 - 2.27 (m, 1H), 2.59 - 2.76 (m, 1H), 2.82 - 2.90 (m, 3H), 3.40 - 3.69 (m, 1H), 4.15 (dt, 1H), 5.81 - 6.17 (m, 1H), 6.47 - 6.62 (m, 1H), 7.09 - 7.26 (m, 7H), 7.42 - 7.50 (m, 2H), 7.94 - 8.05 (m, 2H), 10.67 - 10.77 (m, 1H), 11.54 - 11.86 (m, 1H).

## Example 42

N-[4-(7,7-dimethyl-4-oxo-3-phenyl-4,5,6,7-tetrahydro-1H-pyrrolo[3,2-c]pyridin-2-yl)pyridin-2-yl]-2-(4-fluorophenyl)acetamide

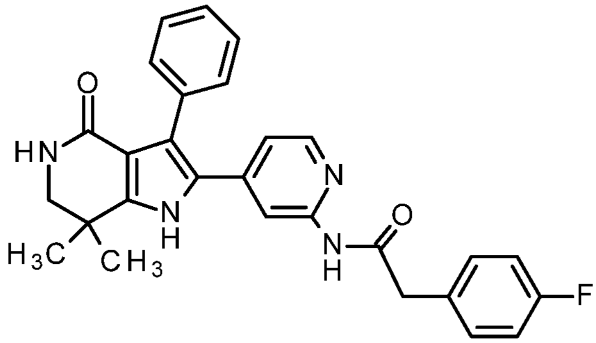

2-(2-Aminopyridin-4-yl)-7,7-dimethyl-3-phenyl-1,5,6,7-tetrahydro-4H-pyrrolo[3,2-c]pyridin-4-one (see Intermediate 37, 100 mg, 301 μmol), (4-fluorophenyl)acetic acid (CAS-RN:[405-50-5], 92.7 mg, 602 μmol), PyBOP (CAS-RN:[128625-52-5], 783 mg, 1.50 mmol) and N,N-diisopropylethylamine (310 μL, 1.8 mmol) were dissolved in 2.8 mL N,N-dimethylacetamide and were stirred at room temperature under argon atmosphere for 3 days. Aqueous saturated sodium hydrogencarbonate solution was added to the reaction mixture, it was diluted with dichloromethane and the aqueous layer was extracted with dichloromethane twice. The organic layer was filtered through a silicone coated filter and concentrated under reduced pressure. The crude product was purified by preparative reverse phase HPLC (eluent: acetonitrile / water with ammonia as additive), by flash chromatography (28 g column, amino phase, gradient: ethyl acetate / ethanol 0-10% ethanol) and by preparative reverse phase HPLC (eluent: acetonitrile / water with ammonia as additive) again to provide the title compound: 26 mg.

LC-MS (Method 2): $R_t$ = 1.09 min; MS (ESIpos): m/z = 469 $[M+H]^+$

$^1$H-NMR (400MHz, DMSO-$d_6$): δ [ppm]= 1.32 (s, 6H), 3.13 (d, 2H), 3.68 (s, 2H), 6.54 (dd, 1H), 7.02 (br s, 1H), 7.11 - 7.28 (m, 7H), 7.32 - 7.42 (m, 2H), 7.94 - 8.09 (m, 2H), 10.65 (s, 1H), 11.55 (s, 1H).

## Example 43

N-[4-(7,7-dimethyl-4-oxo-3-phenyl-4,5,6,7-tetrahydro-1H-pyrrolo[3,2-c]pyridin-2-yl)pyridin-2-yl]-4,4-difluoro-2-(4-fluorophenyl)butanamide (Racemate)

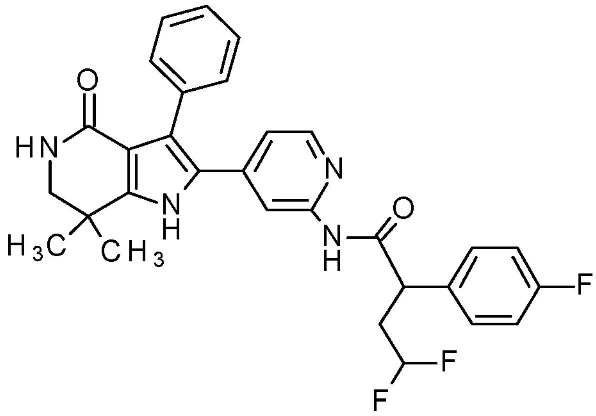

2-(2-Aminopyridin-4-yl)-7,7-dimethyl-3-phenyl-1,5,6,7-tetrahydro-4H-pyrrolo[3,2-c]pyridin-4-one (see Intermediate 37, 150 mg), 4,4-difluoro-2-(4-fluorophenyl)butanoic acid (Racemate) (CAS-RN:[1538957-14-0], 197 mg, 903 μmol), PyBOP (CAS-RN:[128625-52-5], 1.17 g, 2.26 mmol) and N,N-diisopropylethylamine (470 μL, 2.7 mmol) were dissolved in 4.2 mL N,N-dimethylacetamide and were stirred at room temperature under nitrogen atmosphere for 3 days. Aqueous saturated sodium hydrogencarbonate solution was added to the reaction mixture. It was diluted with dichloromethane and the aqueous layer was extracted with dichloromethane twice. The organic layer was dried using a silicon coated filter and concentrated under reduced pressure. The crude product was purified by flash chromatography (55 g column, amino phase, gradient: ethyl acetate / ethanol 0-10% ethanol) and by preparative reverse phase HPLC (eluent:

acetonitrile / water with ammonia as additive) to provide 64 mg of the title compound.

LC-MS (Method 2): Rt = 1.18 min; MS (ESIpos): m/z = 533 $[M+H]^+$

$^1$H-NMR (400MHz, DMSO-$d_6$): δ [ppm]= 1.33 (d, 6H), 2.11 - 2.28 (m, 1H), 2.61 - 2.80 (m, 1H), 3.14 (d, 2H), 4.16 (dd, 1H), 5.76 - 6.17 (m, 1H), 6.53 - 6.63 (m, 1H), 7.03 (s, 1H), 7.12 - 7.29 (m, 7H), 7.40 - 7.50 (m, 2H), 7.95 - 8.05 (m, 2H), 10.73 (s, 1H), 11.58 (s, 1H).

**Example 44**

N-[4-(7,7-dimethyl-4-oxo-3-phenyl-4,5,6,7-tetrahydro-1H-pyrrolo[3,2-c]pyridin-2-yl)pyridin-2-yl]-4,4-difluoro-2-(4-fluorophenyl)butanamide (Enantiomer 1)

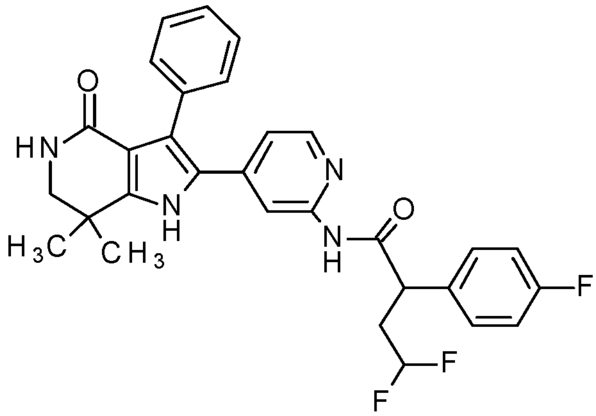

The racemic compound (see Example 43, 60 mg) was separated into enantiomers by preparative chiral HPLC to give enantiomer 1 (27 mg, >99% ee, see Example 44) and enantiomer 2 (25 mg, 96.9% ee, see Example 45).

Preparative chiral HPLC method:
Instrument: Sepiatec: Prep SFC100; Column: Chiralpak IA 5μ 250x30mm; eluent A: CO2; eluent B: 2-propanol + 0.4 vol % diethylamin; isocratic: 25%B; flow: 100 mL/min; temperature: 40°C; BPR: 150bar; UV: 254 nm

Analytical chiral HPLC method:
Instrument: Agilent: 1260, Aurora SFC-Modul; Column: Chiralpak IA 5μ 100x4.6mm; eluent A: CO2; eluent B: 2-propanol + 0.4 vol % diethylamin; isocratic: 25%B; flow: 4 mL/min; temperature: 37.5°C; BPR: 100bar; UV: 254 nm

Analytical Chiral HPLC (method Example 44): Rt = 2.45 min.

LC-MS (Method 2): Rt = 1.17 min; MS (ESIpos): m/z = 533 $[M+H]^+$
$^1$H NMR (400 MHz, DMSO-$d_6$) δ ppm 1.33 (d, 6 H) 2.11 - 2.30 (m, 1 H) 2.58 - 2.78 (m, 1 H) 3.14 (d, 2 H) 4.16 (dd, 1 H) 5.77 - 6.16 (m, 1 H) 6.54 - 6.63 (m, 1 H) 7.03 (s, 1 H) 7.11 - 7.29 (m, 7 H) 7.37 - 7.51 (m, 2 H) 7.92 - 8.09 (m, 2 H) 10.73 (s, 1 H) 11.58 (s, 1 H).

**Example 45**

N-[4-(7,7-dimethyl-4-oxo-3-phenyl-4,5,6,7-tetrahydro-1H-pyrrolo[3,2-c]pyridin-2-yl)pyridin-2-yl]-4,4-difluoro-2-(4-fluorophenyl)butanamide (Enantiomer 2)

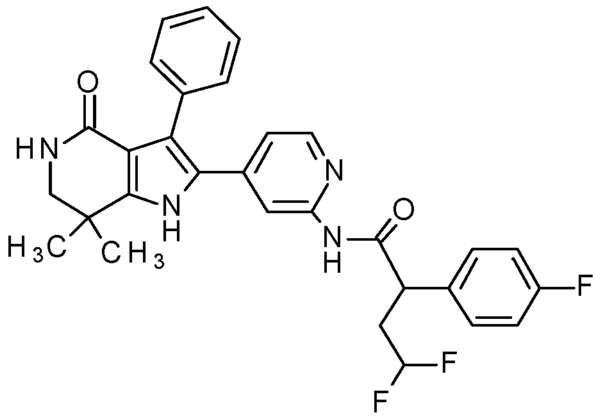

For the preparation of the racemic title compound see Example 43. Separation of enantiomers by preparative chiral HPLC (method see Example 44) gave the title compound (25 mg, 96.9 %ee).

Analytical Chiral HPLC (method see Example 44): Rt = 4.41 min.

LC-MS (Method 2): Rt = 1.17 min; MS (ESIpos): m/z = 533 $[M+H]^+$

$^1$H NMR (400 MHz, DMSO-$d_6$) δ ppm 1.33 (d, 6 H) 2.11 - 2.29 (m, 1 H) 2.67 (dt, 1 H) 3.14 (d, 2 H) 4.16 (dd, 1 H) 5.77 - 6.16 (m, 1 H) 6.50 - 6.65 (m, 1 H) 7.03 (s, 1 H) 7.13 - 7.26 (m, 7 H) 7.39 - 7.51 (m, 2 H) 7.95 - 8.06 (m, 2 H) 10.73 (s, 1 H) 11.58 (s, 1 H).

## Example 46

N-[4-(7,7-dimethyl-4-oxo-3-phenyl-4,5,6,7-tetrahydro-1H-pyrrolo[3,2-c]pyridin-2-yl)pyridin-2-yl]-2-(4-fluorophenyl)propanamide (Racemate)

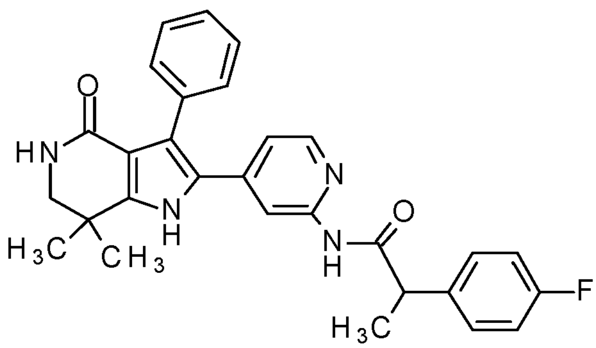

2-(2-Aminopyridin-4-yl)-7,7-dimethyl-3-phenyl-1,5,6,7-tetrahydro-4H-pyrrolo[3,2-c]pyridin-4-one (see Intermediate 37, 150 mg), 2-(4-fluorophenyl)propanoic acid (Racemate) (CAS-RN:[75908-73-5], 152 mg, 903 μmol), PyBOP (CAS-RN:[128625-52-5], 1.17 g, 2.26 mmol) and N,N-diisopropylethylamine (470 μL, 2.7 mmol) were dissolved in 4.2 mL N,N-dimethylacetamide and were stirred at room temperature under argon atmosphere for 3 days. Aqueous saturated sodium hydrogencarbonate solution was added to the reaction mixture, it was diluted with dichloromethane and the aqueous layer was extracted with dichloromethane twice. The combined organic layers were dried using a waterresistant filter and concentrated under reduced pressure. The crude product was purified by chromatography (55 g column, amino phase, gradient: ethyl acetate / ethanol 0-10% ethanol) and by preparative reverse phase HPLC (eluent: acetonitrile / water with ammonia as additive) to provide 59 mg of the title compound.

LC-MS (Method 2): $R_t$ = 1.16 min; MS (ESIpos): m/z = 483 $[M+H]^+$

$^1$H-NMR (400MHz, DMSO-$d_6$): δ [ppm]= 1.33 (d, 6H), 1.38 (d, 3H), 3.14 (d, 2H), 4.01 (q, 1H), 6.52 (dd, 1H), 7.03 (s, 1H), 7.12 - 7.26 (m, 7H), 7.37 - 7.46 (m, 2H), 7.99 (d, 1H), 8.06 (d, 1H), 10.59 (s, 1H), 11.57 (s, 1H)

**Example 47**

N-[4-(7,7-dimethyl-4-oxo-3-phenyl-4,5,6,7-tetrahydro-1H-pyrrolo[3,2-c]pyridin-2-yl)pyridin-2-yl]-2-(4-fluorophenyl)propanamide (Enantiomer 1)

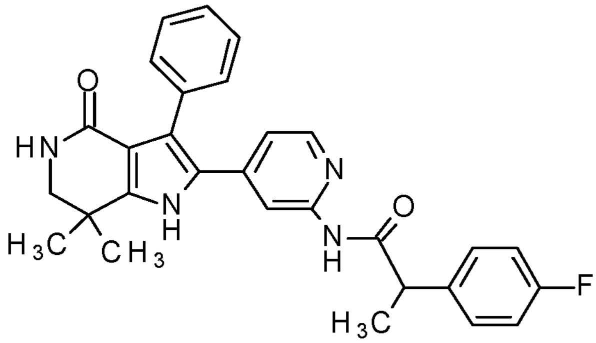

The racemic compound (see Example 46, 55 mg) was separated into enantiomers by preparative chiral HPLC to give enantiomer 1 (24 mg, >99% ee, see Example 47) and enantiomer 2 (24 mg, >99% ee, see Example 48).

Preparative chiral HPLC method:
Instrument: PrepCon Labomatic HPLC; Column: YMC Cellulose SB 5μ, 250x30; eluent A: hexane + 0.1 vol % diethylamine; eluent B: ethanol; isocratic: 90%A+10%B; flow: 40 mL/min; temperature: 25°C; UV: 254 nm

Analytical chiral HPLC method:
Instrument: Thermo Fisher UltiMate 3000; Column: YMC Cellulose SB 3μ, 100x4.6; eluent A: hexane + 0.1 vol % diethylamine; eluent B: ethanol; isocratic: 90%B+10%B; flow: 1.4 mL/min; temperature: 25°C; UV: 254 nm

Analytical Chiral HPLC (method Example 47): Rt = 3.69 min.

LC-MS (Method 2): Rt = 1.18 min; MS (ESIpos): m/z = 483 $[M+H]^+$

$^1$H-NMR (400MHz, DMSO-$d_6$): δ [ppm]= 1.33 (s, 6H), 1.38 (d, 3H), 3.14 (d, 2H), 4.01 (q, 1H), 6.51 (dd, 1H), 7.03 (s, 1H), 7.13 - 7.28 (m, 7H), 7.38 - 7.47 (m, 2H), 7.99 (d, 1H), 8.07 (s, 1H), 10.59 (s, 1H), 11.57 (s, 1H).

**Example 48**

N-[4-(7,7-dimethyl-4-oxo-3-phenyl-4,5,6,7-tetrahydro-1H-pyrrolo[3,2-c]pyridin-2-yl)pyridin-2-yl]-2-(4-fluorophenyl)propanamide (Enantiomer 2)

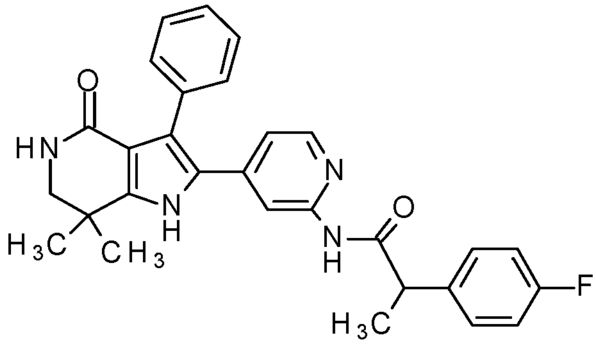

For the preparation of the racemic title compound see Example 46. Separation of enantiomers by preparative

chiral HPLC (method see Example 47) gave the title compound (24 mg, >99%ee).

Analytical Chiral HPLC (method see Example 47): Rt = 4.68 min.

LC-MS (Method 2): Rt = 1.18 min; MS (ESIpos): m/z = 483 $[M+H]^+$

$^1$H-NMR (400MHz, DMSO-$d_6$): δ [ppm]= 1.33 (s, 6H), 1.38 (d, 3H), 3.14 (d, 2H), 4.01 (q, 1H), 6.51 (dd, 1H), 7.03 (s, 1H), 7.12 - 7.26 (m, 7H), 7.35 - 7.48 (m, 2H), 7.99 (d, 1H), 8.06 (s, 1H), 10.59 (s, 1H), 11.57 (s, 1H).

**Example 49**

4,4-difluoro-2-(4-fluorophenyl)-N-[4-(5'-methyl-4'-oxo-3'-phenyl-1',4',5',7'-tetrahydrospiro[cyclobutane-1,6'-pyrrolo[3,2-c]pyridin]-2'-yl)pyridin-2-yl]butanamide (Racemate)

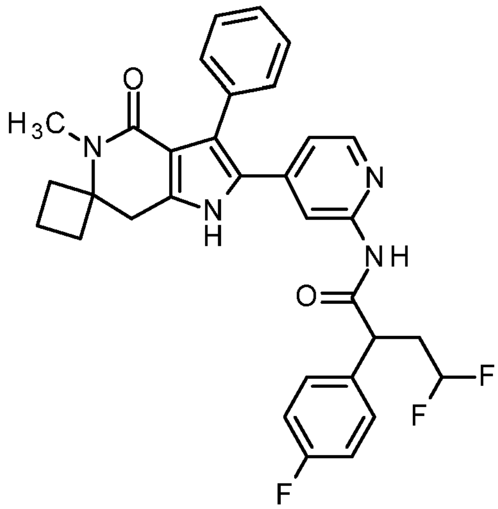

2'-(2-Aminopyridin-4-yl)-5'-methyl-3'-phenyl-1',7'-dihydrospiro[cyclobutane-1,6'-pyrrolo[3,2-c]pyridin]-4'(5'H)-one (see Intermediate 44, 160 mg), 4,4-difluoro-2-(4-fluorophenyl)butanoic acid (Racemate) (CAS-RN:[1538957-14-0], 195 mg, 893 μmol), PyBOP (CAS-RN:[128625-52-5], 697 mg, 1.34 mmol) and N,N-diisopropylethylamine (470 μL, 2.7 mmol) were dissolved in 2.6 mL N,N-dimethylacetamide and were stirred at room temperature under argon atmosphere for 2 days. Aqueous saturated sodium hydrogencarbonate solution and water were added to the reaction mixture. It was diluted with dichloromethane and the aqueous layer was extracted with dichloromethane once. The organic layer was dried using a silicon coated filter and concentrated under reduced pressure. The crude product was purified by flash chromatography (10 g column, silica ULTRA, gradient: dichloromethane / ethanol 0-5% ethanol) and by preparative reverse phase HPLC (eluent: acetonitrile / water with ammonia as additive) to provide 114 mg of the title compound.

LC-MS (Method 1): Rt = 1.26 min; MS (ESIneg): m/z = 557 [M-H]-

$^1$H-NMR (400MHz, DMSO-$d_6$): δ [ppm]= 1.72 - 1.85 (m, 2H), 1.96 (dt, 2H), 2.12 - 2.28 (m, 1H), 2.40 - 2.49 (m, 2H), 2.60 - 2.71 (m, 1H), 2.94 (s, 3H), 3.14 (s, 2H), 4.16 (dd, 1H), 5.79 - 6.16 (m, 1H), 6.53 (dd, 1H), 7.11 - 7.29 (m, 7H), 7.40 - 7.49 (m, 2H), 7.98 (d, 1H), 8.02 (s, 1H), 10.73 (s, 1H), 11.89 (s, 1H).

**Example 50**

(-)-4,4-difluoro-2-(4-fluorophenyl)-N-[4-(5'-methyl-4'-oxo-3'-phenyl-1',4',5',7'-tetrahydrospiro-[cyclobutane-1,6'-pyrrolo[3,2-c]pyridin]-2'-yl)pyridin-2-yl]butanamide (Enantiomer 1)

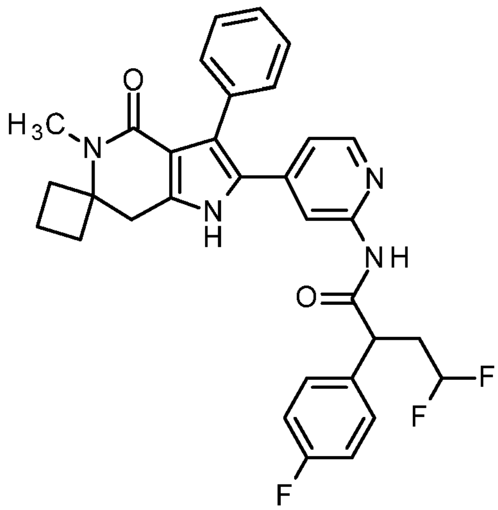

The racemic compound (see Example 49, 100 mg) was separated into enantiomers by preparative chiral HPLC to give enantiomer 1 (31 mg, >99% ee, see Example 50) and enantiomer 2 (31 mg, 95.8% ee, see Example 51).

Preparative chiral HPLC method:
Instrument: PrepCon Labomatic HPLC; Column: YMC Cellulose SB 5μ, 250x30; eluent A: hexane + 0.1 vol % diethylamine; eluent B: ethanol; isocratic: 80%A+20%B; flow: 40 mL/min; temperature: 25°C; UV: 254 nm.

Analytical chiral HPLC method:
Instrument: Thermo Fisher UltiMate 3000; Column: YMC Cellulose SB 3μ, 100x4.6; eluent A: hexane + 0.1 vol % diethylamine; eluent B: ethanol; isocratic: 80%B+20%B; flow: 1.4 mL/min; temperature: 25°C; UV: 254 nm.

Analytical Chiral HPLC (method Example 50): $R_t$ = 1.67 min.

$[\alpha]_D$ = -152.0° (from solution in DMSO, c = 6.6 mg/mL)
$^1$H-NMR (400MHz, DMSO-$d_6$): δ [ppm]= 1.73 - 1.86 (m, 2H), 1.90 - 2.03 (m, 2H), 2.10 - 2.28 (m, 1H), 2.41 - 2.48 (m, 2H), 2.62 - 2.77 (m, 1H), 2.94 (s, 3H), 3.14 (s, 2H), 4.15 (dd, 1H), 5.80 - 6.18 (m, 1H), 6.53 (dd, 1H), 7.10 - 7.29 (m, 7H), 7.42 - 7.52 (m, 2H), 7.91 - 8.06 (m, 2H), 10.72 (s, 1H), 11.88 (s, 1H).

**Example 51**

(+)-4,4-difluoro-2-(4-fluorophenyl)-N-[4-(5'-methyl-4'-oxo-3'-phenyl-1',4',5',7'-tetrahydrospiro-[cyclobutane-1,6'-pyrrolo[3,2-c]pyridin]-2'-yl)pyridin-2-yl]butanamide (Enantiomer 2)

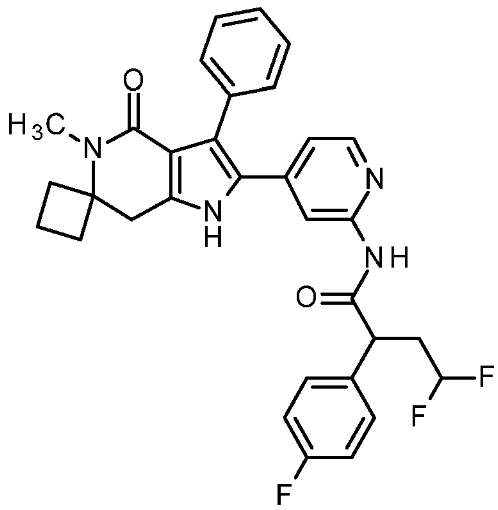

For the preparation of the racemic title compound see Example 49. Separation of enantiomers by preparative chiral HPLC (method see Example 50) gave the title compound (31 mg, 95.8%ee).

Analytical Chiral HPLC (method see Example 50): Rt = 2.40 min.

$[\alpha]_D$ = +180.2° (from solution in DMSO, c = 5.6 mg/mL)

$^1$H-NMR (400MHz, DMSO-$d_6$): δ [ppm]= 1.72 - 1.85 (m, 2H), 1.90 - 2.02 (m, 2H), 2.10 - 2.28 (m, 1H), 2.42 - 2.48 (m, 2H), 2.61 - 2.78 (m, 1H), 2.94 (s, 3H), 3.14 (s, 2H), 4.15 (dd, 1H), 5.81 - 6.19 (m, 1H), 6.53 (dd, 1H), 7.09 - 7.31 (m, 7H), 7.39 - 7.52 (m, 2H), 7.94 - 8.06 (m, 2H), 10.72 (s, 1H), 11.88 (s, 1H).

**Example 52**

2-(4-fluorophenyl)-N-[4-(5'-methyl-4'-oxo-3'-phenyl-1',4',5',7'-tetrahydrospiro[cyclobutane-1,6'-pyrrolo[3,2-c]pyridin]-2'-yl)pyridin-2-yl]propanamide (Racemate)

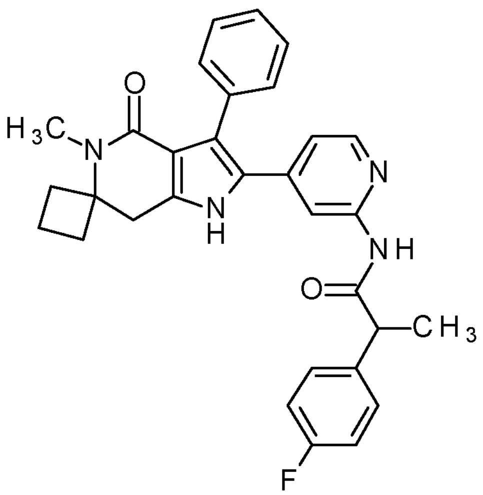

2'-(2-Aminopyridin-4-yl)-5'-methyl-3'-phenyl-1',7'-dihydrospiro[cyclobutane-1,6'-pyrrolo[3,2-c]pyridin]-4'(5'H)-one (see Intermediate 44, 160 mg), 2-(4-fluorophenyl)propanoic acid (Racemate) (CAS-RN:[75908-73-5], 150 mg, 893 μmol), PyBOP (CAS-RN:[128625-52-5], 697 mg, 1.34 mmol) and N,N-diisopropylethylamine (470 μL, 2.7 mmol) were dissolved in 2.6 mL N,N-dimethylacetamide and were stirred at room temperature under argon atmosphere for 2 days. Aqueous saturated sodium hydrogencarbonate solution and water were added to the reaction mixture. It was diluted with dichloromethane and the aqueous layer was extracted with dichloromethane once. The organic layer was

dried using a silicon coated filter and concentrated under reduced pressure. The crude product was purified by preparative reverse phase HPLC (eluent: acetonitrile / water with ammonia as additive) to provide 91 mg of the title compound.

LC-MS (Method 2): Rt = 1.30 min; MS (ESIpos): m/z = 509.5 $[M+H]^+$

$^1$H-NMR (400MHz, DMSO-$d_6$): δ [ppm]= 1.38 (d, 3H), 1.74 - 1.86 (m, 2H), 1.95 (dt, 2H), 2.43 - 2.48 (m, 2H), 2.94 (s, 3H), 3.14 (s, 2H), 4.01 (q, 1H), 6.49 (dd, 1H), 7.13 - 7.21 (m, 4H), 7.21 - 7.27 (m, 3H), 7.37 - 7.46 (m, 2H), 7.96 (d, 1H), 8.08 (d, 1H), 10.58 (s, 1H), 11.88 (s, 1H).

## Example 53

(-)-2-(4-fluorophenyl)-N-[4-(5'-methyl-4'-oxo-3'-phenyl-1',4',5',7'-tetrahydrospiro[cyclobutane-1,6'-pyrrolo[3,2-c]pyridin]-2'-yl)pyridin-2-yl]propanamide (Enantiomer 1)

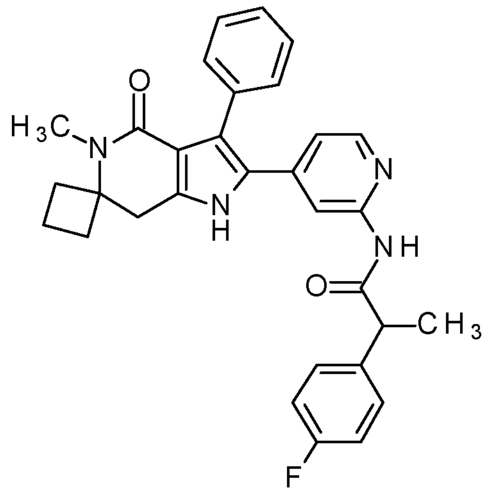

The racemic compound (see Example 52, 80 mg) was separated into enantiomers by preparative chiral HPLC to give enantiomer 1 (30 mg, > 99% ee, see Example 53) and enantiomer 2 (25 mg, > 99% ee, see Example 54).

Preparative chiral HPLC method:
Instrument: PrepCon Labomatic HPLC; Column: YMC Cellulose SB 10μ, 250x50; eluent A: hexane + 0.1 vol % diethylamine; eluent B: ethanol + 0.1 vol % diethylamine; isocratic: 90%A+10%B; flow: 100 mL/min; temperature: 25°C; UV: 254 nm.

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 90%A+10%B; flow: 1.4 mL/min; temperature: 25°C; UV: 254 nm.

Analytical Chiral HPLC (method Example 53): Rt = 2.86 min.

$[\alpha]_D$ = -159.7° (from solution in DMSO, c = 4.0 mg/mL)

$^1$H NMR (400 MHz, DMSO-$d_6$) δ ppm 1.38 (d, 3 H) 1.72 - 1.86 (m, 2 H) 1.90 - 2.01 (m, 2 H) 2.41 - 2.49 (m, 2 H) 2.94 (s, 3 H) 3.14 (s, 2 H) 4.01 (q, 1 H) 6.49 (dd, 1 H) 7.11 - 7.20 (m, 4 H) 7.20 - 7.30 (m, 3 H) 7.37 - 7.46 (m, 2 H) 7.97 (d, 1 H) 8.08 (s, 1 H) 10.58 (s, 1 H) 11.88 (s, 1 H).

**Example 54**

(+)-2-(4-fluorophenyl)-N-[4-(5'-methyl-4'-oxo-3'-phenyl-1',4',5',7'-tetrahydrospiro[cyclobutane-1,6'-pyrrolo[3,2-c]pyridin]-2'-yl)pyridin-2-yl]propanamide (Enantiomer 2)

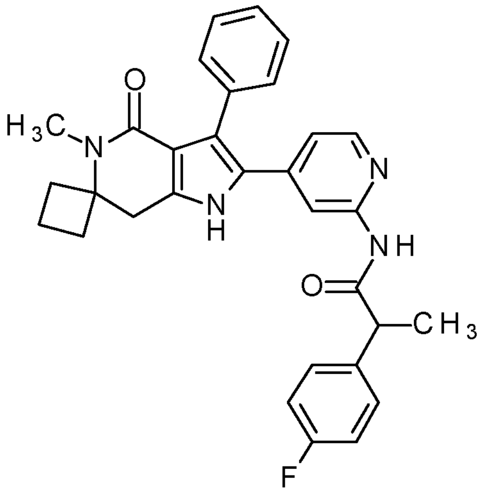

For the preparation of the racemic title compound see Example 52. Separation of enantiomers by preparative chiral HPLC (method see Example 53) gave the title compound (25 mg, > 99% ee).

Analytical Chiral HPLC (method see Example 53): $R_t$ = 8.59 min.

$[\alpha]_D$ = +141.7° (from solution in DMSO, c = 4.3 mg/mL)

$^1$H NMR (400 MHz, DMSO-$d_6$) $\delta$ ppm 1.38 (d, 3 H) 1.72 - 1.84 (m, 2 H) 1.87 - 2.03 (m, 2 H) 2.41 - 2.49 (m, 2 H) 2.94 (s, 3 H) 3.14 (s, 2 H) 4.01 (q, 1 H) 6.49 (dd, 1 H) 7.09 - 7.31 (m, 7 H) 7.37 - 7.45 (m, 2 H) 7.97 (d, 1 H) 8.08 (s, 1 H) 10.58 (s, 1 H) 11.88 (s, 1 H).

**Example 55**

N-{4-[7-(cyclopropylmethyl)-5-methyl-4-oxo-3-phenyl-4,5,6,7-tetrahydro-1H-pyrrolo[3,2-c]pyridin-2-yl]pyridin-2-yl}-2-(4-fluorophenyl)acetamide (Racemate)

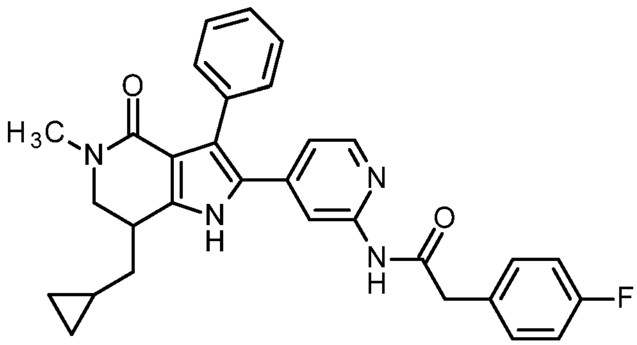

2-(2-Aminopyridin-4-yl)-7-(cyclopropylmethyl)-5-methyl-3-phenyl-1,5,6,7-tetrahydro-4H-pyrrolo-[3,2-c]pyridin-4-one (Racemate) (see Intermediate 54, 110 mg), (4-fluorophenyl)acetic acid (CAS-RN:[405-50-5], 91.0 mg, 591 µmol), PyBOP (CAS-RN:[128625-52-5], 461 mg, 886 µmol) and N,N-diisopropylethylamine (310 µL, 1.8 mmol) were dissolved in 2.7 mL N,N-dimethylacetamide and were stirred at room temperature under argon atmosphere overnight. Aqueous saturated sodium hydrogencarbonate solution was added to the reaction mixture, it was diluted with dichloromethane

and the aqueous layer was extracted with dichloromethane once. The organic layer was washed with water twice, dried using a silicon coated filter and concentrated under reduced pressure. The crude product was purified by preparative reverse phase HPLC (eluent: acetonitrile / water with ammonia as additive). The product was diluted with MTBE and hexane. The formed precipitate was filtered off to provide the title compound: 13.9 mg.

LC-MS (Method 2): $R_t$ = 1.27 min; MS (ESIneg): m/z = 507.6 [M-H]-

$^1$H-NMR (400MHz, DMSO-$d_6$): δ [ppm]= -0.02 - 0.13 (m, 2H), 0.37 - 0.56 (m, 2H), 0.76 - 0.90 (m, 1H), 1.53 - 1.63 (m, 2H), 2.86 (s, 3H), 3.04 - 3.15 (m, 1H), 3.46 (dd, 1H), 3.67 (s, 2H), 3.75 (dd, 1H), 6.50 (dd, 1H), 7.09 - 7.26 (m, 7H), 7.31 - 7.40 (m, 2H), 7.99 (d, 1H), 8.04 (s, 1H), 10.63 (s, 1H), 11.68 (s, 1H).

**Example 56**

N-{4-[7-(cyclopropylmethyl)-5-methyl-4-oxo-3-phenyl-4,5-dihydro-1H-pyrrolo[3,2-c]pyridin-2-yl]pyridin-2-yl}-2-(4-fluorophenyl)acetamide

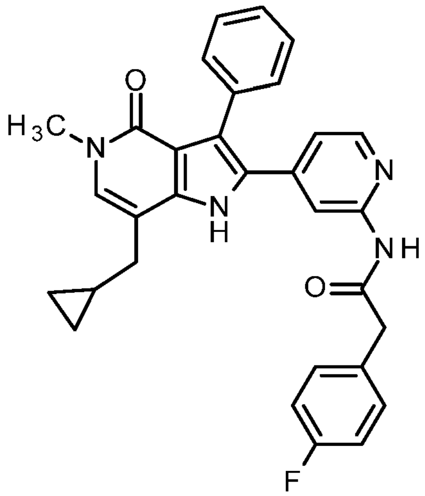

N-{4-[7-(Cyclopropylmethyl)-5-methyl-4-oxo-3-phenyl-4,5,6,7-tetrahydro-1H-pyrrolo[3,2-c]-pyridin-2-yl]pyridin-2-yl}-2-(4-fluorophenyl)acetamide (Racemate) (see Example 55, 25.0 mg) was dissolved in 1,4-dioxane (2.1 mL) and 2,3-dichloro-5,6-dicyano-1,4-benzochinone (CAS-RN:[84-58-2], 12.8 mg, 56.5 μmol) was added. The reaction mixture was stirred at 80 °C for 2 hours under argon atmosphere. The reaction mixture was diluted with ethyl acetate and treated with 1 M aqueous sodiumhydroxyd-solution. The layers were separated and the aqueous layer was extracted with ethyl acetate twice. The combined organic layers were dried using a silicon coated filter and concentrated under reduced pressure. The crude product was purified by preparative reverse phase HPLC (eluent: acetonitrile / water with formic acid as additive) to provide 2 mg of the title compound.
$^1$H NMR (400 MHz, DMSO-$d_6$) δ ppm 0.18 - 0.30 (m, 2 H) 0.43 - 0.58 (m, 2 H) 1.03 - 1.15 (m, 1 H) 2.56 (d, 2 H) 3.38 (s, 3 H) 3.68 (s, 2 H) 6.69 (dd, 1 H) 7.07 - 7.30 (m, 8 H) 7.30 - 7.41 (m, 2 H) 8.04 - 8.22 (m, 2 H) 10.71 (s, 1 H) 11.76 (s, 1 H).

**Example 57**

N-{4-[7-(cyclopropylmethyl)-5-methyl-4-oxo-3-phenyl-4,5,6,7-tetrahydro-1H-pyrrolo[3,2-c]pyridin-2-yl]pyridin-2-yl}-2-(4-fluorophenyl)propanamide (mixture of 4 stereoisomers)

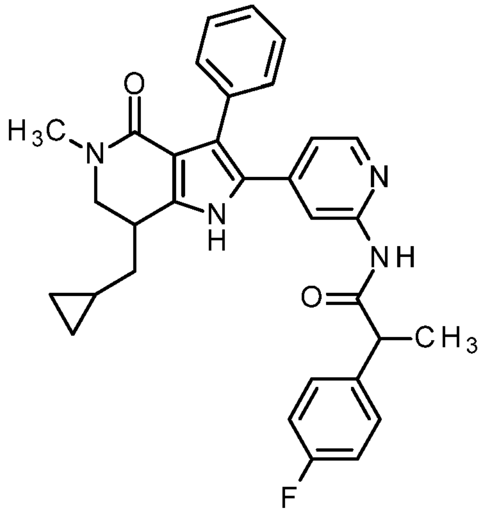

2-(2-Aminopyridin-4-yl)-7-(cyclopropylmethyl)-5-methyl-3-phenyl-1,5,6,7-tetrahydro-4H-pyrrolo-[3,2-c]pyridin-4-one (Racemate) (see Intermediate 54, 170 mg), 2-(4-fluorophenyl)propanoic acid (Racemate) (CAS-RN:[75908-73-5], 154 mg, 913 μmol), PyBOP (CAS-RN:[128625-52-5], 713 mg, 1.37 mmol) and N,N-diisopropylethylamine (480 μL, 2.7 mmol) were dissolved in 4.2 mL N,N-dimethylacetamide and were stirred at room temperature under nitrogen atmosphere overnight. Aqueous saturated sodium hydrogencarbonate solution was added to the reaction mixture, it was diluted with dichloromethane and the aqueous layer was extracted with dichloromethane once. The organic layer was washed with water twice, dried using a silicon coated filter and concentrated under reduced pressure. The crude product was purified by preparative reverse phase HPLC (eluent: acetonitrile / water with ammonia as additive) to provide 163 mg of the title compound.

LC-MS (Method 2): Rt = 1.34 min; MS (ESIpos): m/z = 523.5 $[M+H]^+$

$^1$H-NMR (400MHz, DMSO-$d_6$): δ [ppm]= -0.01 - 0.16 (m, 2H), 0.45 (br dd, 1H), 0.48 - 0.58 (m, 1H), 0.82 (br d, 1H), 1.37 (d, 3H), 1.55 - 1.65 (m, 2H), 2.87 (s, 3H), 3.04 - 3.14 (m, 1H), 3.47 (dd, 1H), 3.76 (dd, 1H), 4.00 (q, 1H), 6.48 (dd, 1H), 7.11 - 7.19 (m, 4H), 7.20 - 7.28 (m, 3H), 7.41 (dd, 2H), 7.96 (d, 1H), 8.06 (s, 1H), 10.58 (s, 1H), 11.70 (d, 1H).

## Example 58

N-{4-[7-(cyclopropylmethyl)-5-methyl-4-oxo-3-phenyl-4,5-dihydro-1H-pyrrolo[3,2-c]pyridin-2-yl]pyridin-2-yl}-2-(4-fluorophenyl)propanamide (Racemate)

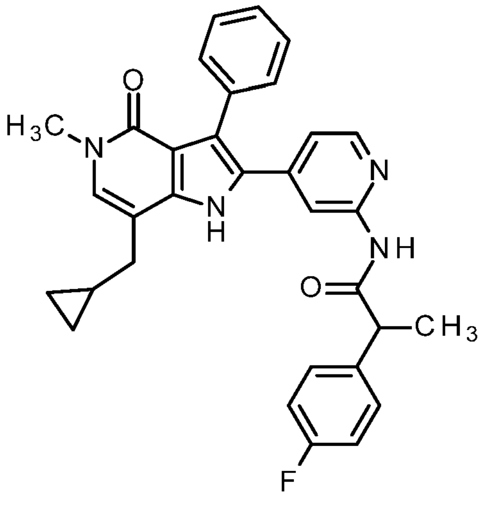

N-{4-[7-(Cyclopropylmethyl)-5-methyl-4-oxo-3-phenyl-4,5,6,7-tetrahydro-1H-pyrrolo[3,2-c]pyridin-2-yl]pyridin-2-yl}-2-(4-fluorophenyl)propanamide (mixture of 4 stereoisomers) (see Example 57, 155 mg) was dissolved in 1,4-dioxane (13 mL) and 2,3-dichloro-5,6-dicyano-1,4-benzochinone (CAS-RN:[84-58-2], 77.4 mg, 341 μmol) was added. The reaction mixture was stirred at 80 °C for 2 hours under argon atmosphere. The reaction mixture was diluted with ethyl acetate and treated with 1 M aqueous sodiumhydroxyd-solution. The layers were separated and the aqueous layer was extracted with ethyl acetate twice. The combined organic layers were dried using a silicon coated filter and concentrated under reduced pressure. The crude product was dissolved in 1,4-dioxane (13 mL) again and 2,3-dichloro-5,6-dicyano-1,4-benzochinone (CAS-RN:[84-58-2], 77.4 mg, 341 μmol) was added. The reaction mixture was stirred at 80 °C for 2 hours under argon atmosphere. The reaction mixture was diluted with ethyl acetate and treated with 1 M aqueous sodiumhydroxyd-solution again. The layers were separated and the aqueous layer was extracted with ethyl acetate twice. The combined organic layers were dried using a silicon coated filter and concentrated under reduced pressure. The crude product was purified by preparative reverse phase HPLC (eluent: acetonitrile / water with formic acid as additive) to provide 18.3 mg of the title compound.

$^1$H NMR (400 MHz, DMSO-$d_6$) δ ppm 0.19 - 0.29 (m, 2 H) 0.46 - 0.56 (m, 2 H) 1.05 - 1.16 (m, 1 H) 1.38 (d, 3 H) 2.57 (d, 2 H) 3.38 (s, 3 H) 4.02 (q, 1 H) 6.66 (dd, 1 H) 7.10 - 7.30 (m, 8 H) 7.37 - 7.49 (m, 2 H) 8.06 (dd, 1 H) 8.16 (s, 1 H) 10.65 (s, 1 H) 11.78 (s, 1 H).

## Example 59

N-{4-[7-(cyclopropylmethyl)-5-methyl-4-oxo-3-phenyl-4,5,6,7-tetrahydro-1H-pyrrolo[3,2-c]pyridin-2-yl]pyridin-2-yl}-4,4-difluoro-2-(4-fluorophenyl)butanamide (mixture of 4 stereoisomers)

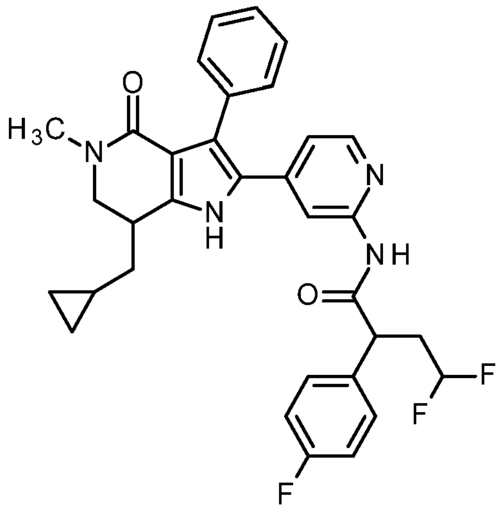

2-(2-Aminopyridin-4-yl)-7-(cyclopropylmethyl)-5-methyl-3-phenyl-1,5,6,7-tetrahydro-4H-pyrrolo-[3,2-c]pyridin-4-one (Racemate) (see Intermediate 54, 170 mg), 4,4-difluoro-2-(4-fluorophenyl)butanoic acid (Racemate) (CAS-RN:[1538957-14-0], 199 mg, 913 μmol), PyBOP (CAS-RN:[128625-52-5], 713 mg, 1.37 mmol) and N,N-diisopropylethylamine (480 μL, 2.7 mmol) were dissolved in 4.2 mL N,N-dimethylacetamide and stirred at room temperature under nitrogen atmosphere overnight. Aqueous saturated sodium hydrogencarbonate solution was added to the reaction mixture. It was diluted with dichloromethane and the aqueous layer was extracted with dichloromethane once. The organic layer was washed with water twice, dried using a silicon coated filter and concentrated under reduced pressure. The crude product was purified by flash chromatography (25 g column, silica ULTRA, gradient: ethyl acetate / ethanol 0-10% ethanol) and by preparative reverse phase HPLC (eluent: acetonitrile / water with ammonia as additive) to provide 48.6 mg of the title compound.

LC-MS (Method 2): Rt = 1.35 min; MS (ESIpos): m/z = 573.6 $[M+H]^+$

$^1$H-NMR (400MHz, DMSO-$d_6$): δ [ppm]= 0.01 - 0.17 (m, 2H), 0.39 - 0.58 (m, 2H), 0.82 (dt, 1H), 1.59 (br t, 2H), 2.19 (br dd, 1H), 2.62 - 2.78 (m, 1H), 2.87 (s, 3H), 3.05 - 3.16 (m, 1H), 3.43 - 3.52 (m, 1H), 3.76 (ddd, 1H), 4.15 (dd, 1H), 5.81 - 6.16 (m, 1H), 6.53 (dt, 1H), 7.09 - 7.18 (m, 2H), 7.19 - 7.28 (m, 5H), 7.45 (dd, 2H), 7.94 - 8.04 (m, 2H), 10.72

(s, 1H), 11.71 (d, 1H).

**Example 60**

N-{4-[7-(cyclopropylmethyl)-5-methyl-4-oxo-3-phenyl-4,5-dihydro-1H-pyrrolo[3,2-c]pyridin-2-yl]pyridin-2-yl}-4,4-difluoro-2-(4-fluorophenyl)butanamide (Racemate)

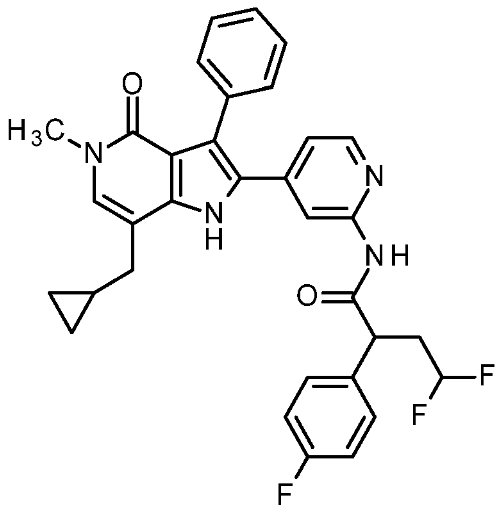

N-{4-[7-(Cyclopropylmethyl)-5-methyl-4-oxo-3-phenyl-4,5,6,7-tetrahydro-1H-pyrrolo[3,2-c]-pyridin-2-yl]pyridin-2-yl}-4,4-difluoro-2-(4-fluorophenyl)butanamide (mixture of 4 stereoisomers) (see Example 59, 149 mg) was dissolved in 1,4-dioxane (11 mL) and 2,3-dichloro-5,6-dicyano-1,4-benzochinone (CAS-RN:[84-58-2], 67.9 mg, 299 μmol) was added. The reaction mixture was stirred at 80 °C for 2 hours under argon atmosphere. The reaction mixture was diluted with ethyl acetate and treated with 1 M aqueous sodiumhydroxyd-solution. The layers were separated and the aqueous layer was extracted with ethyl acetate twice. The combined organic layers were dried using a silicon coated filter and concentrated under reduced pressure. The crude product was dissolved in 1,4-dioxane (11 mL) again and 2,3-dichloro-5,6-dicyano-1,4-benzochinone (CAS-RN:[84-58-2], 67.9 mg, 299 μmol) was added. The reaction mixture was stirred at 80 °C for 2 hours under argon atmosphere. The reaction mixture was diluted with ethyl acetate and treated with 1 M aqueous sodiumhydroxyd-solution again. The layers were separated and the aqueous layer was extracted with ethyl acetate twice. The combined organic layers were dried using a silicon coated filter and concentrated under reduced pressure. The crude product was purified by preparative reverse phase HPLC (eluent: acetonitrile / water with formic acid as additive) to provide 8 mg of the title compound.

$^1$H NMR (400 MHz, DMSO-$d_6$) δ ppm 0.18 - 0.33 (m, 2 H) 0.44 - 0.56 (m, 2 H) 1.04 - 1.17 (m, 1 H) 2.08 - 2.30 (m, 1 H) 2.57 (d, 2 H) 2.61 - 2.78 (m, 1 H) 3.38 (s, 3 H) 4.17 (br dd, 1 H) 5.78 - 6.17 (m, 1 H) 6.66 - 6.78 (m, 1 H) 7.16 - 7.29 (m, 8 H) 7.40 - 7.50 (m, 2 H) 8.02 - 8.15 (m, 2 H) 10.79 (s, 1 H) 11.79 (s, 1 H).

**Example 61**

N-{4-[7-(cyclopropylmethyl)-3-(4-fluorophenyl)-5-methyl-4-oxo-4,5,6,7-tetrahydro-1H-pyrrolo[3,2-c]pyridin-2-yl]pyridin-2-yl}-2-(4-fluorophenyl)acetamide (Racemate)

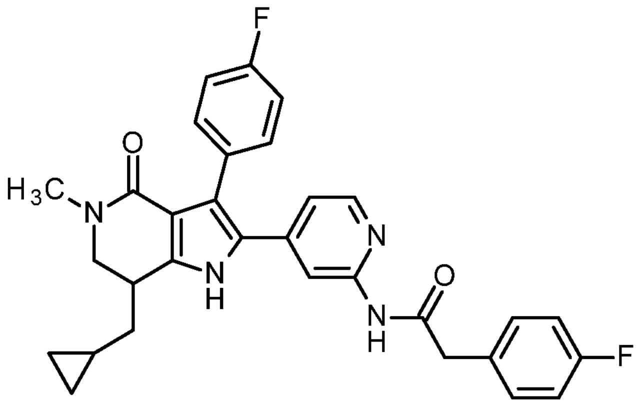

2-(2-Aminopyridin-4-yl)-7-(cyclopropylmethyl)-3-(4-fluorophenyl)-5-methyl-1,5,6,7-tetrahydro-4H-pyrrolo[3,2-c]pyridin-4-one (Racemate) (see Intermediate 57, 127 mg), (4-fluorophenyl)aceticacid (CAS-RN:[405-50-5], 100 mg, 651 µmol), PyBOP (CAS-RN:[128625-52-5], 508 mg, 976 µmol) and N,N-diisopropylethylamine (340 µL, 2.0 mmol) were dissolved in 3.0 mL N,N-dimethylacetamide and were stirred at room temperature under nitrogen atmosphere overnight. Aqueous saturated sodium hydrogencarbonate solution was added to the reaction mixture, it was diluted with dichloromethane and the aqueous layer was extracted with dichloromethane once. The organic layer was washed with water twice, dried using a silicon coated filter and concentrated under reduced pressure. The crude product was purified by preparative reverse phase HPLC (eluent: acetonitrile / water with ammonia as additive). The product was diluted with MTBE and hexane. The formed precipitate was filtered off to provide 37.3 mg of the title compound.

LC-MS (Method 2): $R_t$ = 1.28 min; MS (ESIpos): m/z = 527 $[M+H]^+$

$^1$H-NMR (400MHz, DMSO-$d_6$): δ [ppm]= -0.02 - 0.12 (m, 2H), 0.37 - 0.60 (m, 2H), 0.74 - 0.88 (m, 1H), 1.52 - 1.65 (m, 2H), 2.86 (s, 3H), 3.03 - 3.13 (m, 1H), 3.41 - 3.51 (m, 1H), 3.67 (s, 2H), 3.75 (dd, 1H), 6.59 (dd, 1H), 7.01 - 7.09 (m, 2H), 7.11 - 7.26 (m, 4H), 7.31 - 7.40 (m, 2H), 8.00 (s, 1H), 8.05 (d, 1H), 10.63 (s, 1H), 11.71 (s, 1H).

## Example 62

N-{4-[7-(cyclopropylmethyl)-3-(4-fluorophenyl)-5-methyl-4-oxo-4,5,6,7-tetrahydro-1H-pyrrolo[3,2-c]pyridin-2-yl]pyridin-2-yl}-2-(4-fluorophenyl)propanamide (mixture of 4 stereoisomers)

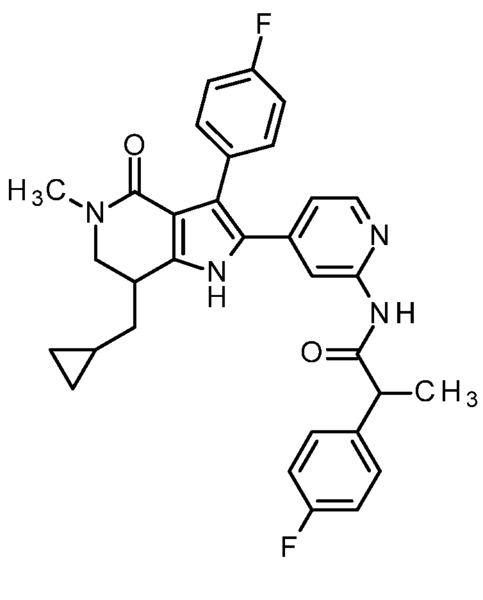

2-(2-Aminopyridin-4-yl)-7-(cyclopropylmethyl)-3-(4-fluorophenyl)-5-methyl-1,5,6,7-tetrahydro-4H-pyrrolo[3,2-c]pyridin-4-one (Racemate) (see Intermediate 57, 190 mg), 2-(4-fluorophenyl)propanoic acid (Racemate) (CAS-RN:[75908-73-5], 164 mg, 973 μmol), PyBOP (CAS-RN:[128625-52-5], 760 mg, 1.46 mmol) and N,N-diisopropylethylamine (510 μL, 2.9 mmol) were dissolved in 4.5 mL N,N-dimethylacetamide and were stirred at room temperature under nitrogen atmosphere overnight. Aqueous saturated sodium hydrogencarbonate solution was added to the reaction mixture, it was diluted with dichloromethane and the aqueous layer was extracted with dichloromethane once. The organic layer was washed with water twice, dried using a silicon coated filter and concentrated under reduced pressure. The crude product was purified by preparative reverse phase HPLC (eluent: acetonitrile / water with ammonia as additive) to provide 111 mg of the title compound.

LC-MS (Method 2): Rt = 1.35 min; MS (ESIpos): m/z = 541.5 $[M+H]^+$

$^1$H-NMR (400MHz, DMSO-$d_6$): δ [ppm]= -0.02 - 0.15 (m, 2H), 0.39 - 0.48 (m, 1H), 0.48 - 0.58 (m, 1H), 0.75 - 0.90 (m, 1H), 1.36 (d, 3H), 1.59 (br t, 2H), 2.87 (s, 3H), 3.10 (br t, 1H), 3.44 - 3.53 (m, 1H), 3.76 (ddd, 1H), 3.99 (q, 1H), 6.54 - 6.61 (m, 1H), 7.01 - 7.09 (m, 2H), 7.12 - 7.25 (m, 4H), 7.40 (dd, 2H), 7.97 - 8.07 (m, 2H), 10.57 (s, 1H), 11.72 (d, 1H).

**Example 63**

N-{4-[7-(cyclopropylmethyl)-3-(4-fluorophenyl)-5-methyl-4-oxo-4,5-dihydro-1H-pyrrolo[3,2-c]pyridin-2-yl]pyridin-2-yl}-2-(4-fluorophenyl)propanamide (Racemate)

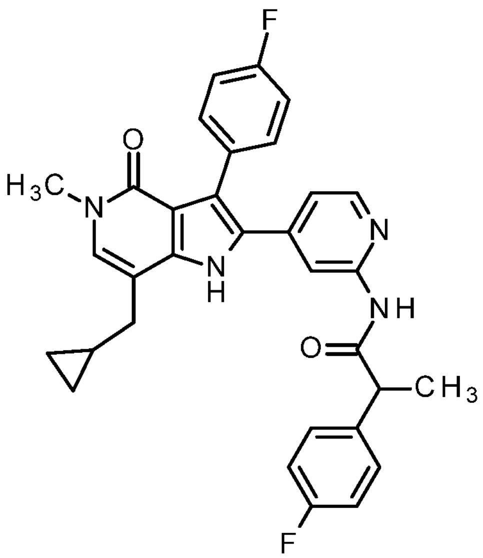

N-{4-[7-(Cyclopropylmethyl)-3-(4-fluorophenyl)-5-methyl-4-oxo-4,5,6,7-tetrahydro-1H-pyrrolo-[3,2-c]pyridin-2-yl]pyridin-2-yl}-2-(4-fluorophenyl)propanamide (mixture of 4 stereoisomers) (see Example 62, 104 mg) was dissolved in 1,4-dioxane (8.2 mL) and 2,3-dichloro-5,6-dicyano-1,4-benzochinone (CAS-RN:[84-58-2], 50.2 mg, 221 μmol) was added. The reaction mixture was stirred at 80 °C for one hour under argon atmosphere. The reaction mixture was diluted with ethyl acetate and treated with 1 M aqueous sodiumhydroxyd-solution. The layers were separated and the aqueous layer was extracted with ethyl acetate twice. The combined organic layers were dried using a silicon coated filter and concentrated under reduced pressure. The crude product was dissolved in 1,4-dioxane (8.2 mL) again and 2,3-dichloro-5,6-dicyano-1,4-benzochinone (CAS-RN:[84-58-2], 50.2 mg, 221 μmol) was added. The reaction mixture was stirred at 80 °C for one hour under argon atmosphere. The reaction mixture was diluted with ethyl acetate and treated with 1 M aqueous sodiumhydroxyd-solution again. The layers were separated and the aqueous layer was extracted with ethyl acetate twice. The combined organic layers were dried using a silicon coated filter and concentrated under reduced pressure. The crude product was purified by flash chromatography (10 g column, silica ULTRA, gradient: ethyl acetate / ethanol 0-10% ethanol) and by preparative reverse phase HPLC (eluent: acetonitrile / water with formic acid as additive) to provide 9 mg of the title compound.

$^1$H NMR (400 MHz, DMSO-$d_6$) δ ppm 0.19 - 0.29 (m, 2 H) 0.42 - 0.55 (m, 2 H) 1.05 - 1.15 (m, 1 H) 1.37 (d, 3 H) 2.57 (d, 2 H) 3.38 (s, 3 H) 4.01 (q, 1 H) 6.72 - 6.80 (m, 1 H) 7.04 - 7.12 (m, 2 H) 7.13 - 7.20 (m, 2 H) 7.21 - 7.30 (m, 3 H) 7.36 - 7.47 (m, 2 H) 8.00 - 8.24 (m, 2 H) 10.65 (s, 1 H) 11.80 (s, 1 H).

**Example 64**

N-{4-[7-(cyclopropylmethyl)-3-(4-fluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (mixture of 4 stereoisomers)

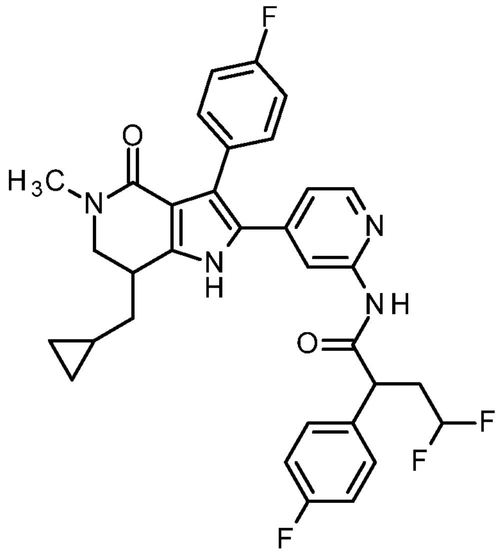

2-(2-Aminopyridin-4-yl)-7-(cyclopropylmethyl)-3-(4-fluorophenyl)-5-methyl-1,5,6,7-tetrahydro-4H-pyrrolo[3,2-c]pyridin-4-one (Racemate) (see Intermediate 57, 190 mg), 4,4-difluoro-2-(4-fluorophenyl)butanoic acid (Racemate) (CAS-RN:[1538957-14-0], 212 mg, 973 μmol), PyBOP (CAS-RN:[128625-52-5], 760 mg, 1.46 mmol) and N,N-diisopropylethylamine (510 μL, 2.9 mmol) were dissolved in 4.5 mL N,N-dimethylacetamide and stirred at room temperature under nitrogen atmosphere overnight. Aqueous saturated sodium hydrogencarbonate solution was added to the reaction mixture. It was diluted with dichloromethane and the aqueous layer was extracted with dichloromethane once. The organic layer was washed with water twice, dried using a silicon coated filter and concentrated under reduced pressure. The crude product was purified by flash chromatography (28 g column, amino phase, gradient: hexane/ethyl acetate 0-60% ethyl acetate, ethyl acetate / ethanol 0-10% ethanol) and by preparative reverse phase HPLC (eluent: acetonitrile / water with ammonia as additive) to provide 186 mg of the title compound.

LC-MS (Method 2): Rt = 1.35 min; MS (ESIpos): m/z = 591.5 $[M+H]^+$

$^1$H-NMR (400MHz, DMSO-$d_6$): δ [ppm]= 0.01 - 0.14 (m, 2H), 0.39 - 0.47 (m, 1H), 0.48 - 0.57 (m, 1H), 0.75 - 0.91 (m, 1H), 1.54 - 1.63 (m, 2H), 2.10 - 2.28 (m, 1H), 2.58 - 2.75 (m, 1H), 2.87 (s, 3H), 3.09 (td, 1H), 3.44 - 3.53 (m, 1H), 3.76 (ddd, 1H), 4.14 (dd, 1H), 5.76 - 6.14 (m, 1H), 6.63 (dt, 1H), 6.98 - 7.09 (m, 2H), 7.14 - 7.23 (m, 4H), 7.39 - 7.48 (m, 2H), 7.94 (s, 1H), 8.04 (d, 1H), 10.71 (s, 1H), 11.73 (d, 1H).

**Example 65**

N-{4-[7-(cyclopropylmethyl)-3-(4-fluorophenyl)-5-methyl-4-oxo-4,5-dihydro-1H-pyrrolo[3,2-c]pyridin-2-yl]pyridin-2-yl}-4,4-difluoro-2-(4-fluorophenyl)butanamide (Racemate)

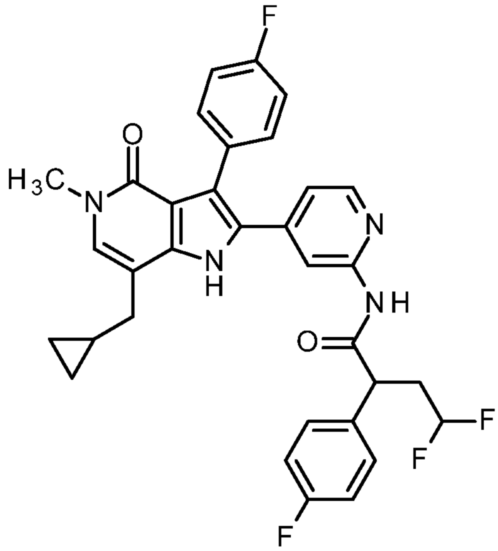

N-{4-[7-(Cyclopropylmethyl)-3-(4-fluorophenyl)-5-methyl-4-oxo-4,5,6,7-tetrahydro-1H-pyrrolo-[3,2-c]pyridin-2-yl]pyridin-2-yl}-4,4-difluoro-2-(4-fluorophenyl)butanamide (mixture of 4 stereoisomers) (see Example 64, 179 mg) was dissolved in 1,4-dioxane (13 mL) and 2,3-dichloro-5,6-dicyano-1,4-benzochinone (CAS-RN:[84-58-2], 79.1 mg, 349 μmol) was added. The reaction mixture was stirred at 80 °C for one hour under argon atmosphere. The reaction mixture was diluted with ethyl acetate and treated with 1 M aqueous sodiumhydroxyd-solution. The layers were separated and the aqueous layer was extracted with ethyl acetate twice. The combined organic layers were dried using a silicon coated filter and concentrated under reduced pressure. The crude product was dissolved in 1,4-dioxane (13 mL) again and 2,3-dichloro-5,6-dicyano-1,4-benzochinone (CAS-RN:[84-58-2], 79.1 mg, 349 μmol) was added. The reaction mixture was stirred at 80 °C for one hour under argon atmosphere. The reaction mixture was diluted with ethyl acetate and treated with 1 M aqueous sodiumhydroxyd-solution again. The layers were separated and the aqueous layer was extracted with ethyl acetate twice. The combined organic layers were dried using a silicon coated filter and concentrated under reduced pressure.The crude product was purified by flash chromatography and by preparative reverse phase HPLC (eluent: acetonitrile / water with formic acid as additive) to provide 25.3 mg of the title compound.

$^{1}H$ NMR (400 MHz, DMSO-d6) δ ppm 0.19 - 0.27 (m, 2 H) 0.46 - 0.58 (m, 2 H) 1.05 - 1.17 (m, 1 H) 2.11 - 2.28 (m, 1 H) 2.57 (d, 2 H) 2.67 (dt, 1 H) 3.38 (s, 3 H) 4.16 (dd, 1 H) 5.78 - 6.14 (m, 1 H) 6.83 (dd, 1 H) 7.04 - 7.12 (m, 2 H) 7.16 - 7.31 (m, 5 H) 7.41 - 7.50 (m, 2 H) 8.05 (s, 1 H) 8.14 (dd, 1 H) 10.78 (s, 1 H) 11.80 (s, 1 H).

## Example 66

N-{4-[3-(2,4-difluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (Racemate)

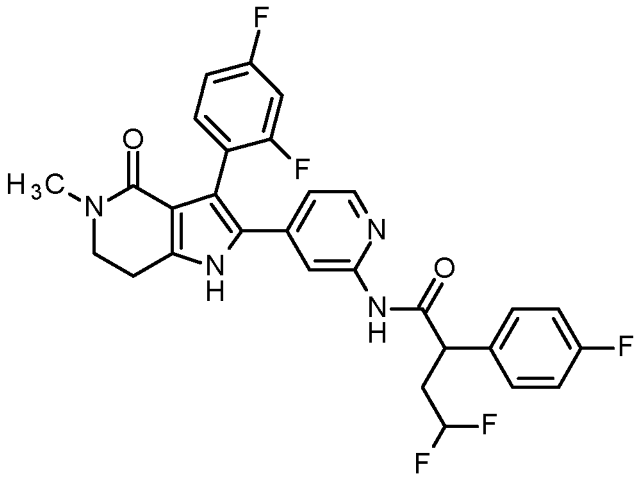

2-(2-Aminopyridin-4-yl)-3-(2,4-difluorophenyl)-5-methyl-1,5,6,7-tetrahydro-4H-pyrrolo[3,2-c]-pyridin-4-one (see Intermediate 60, 138 mg), 4,4-difluoro-2-(4-fluorophenyl)butanoic acid (Racemate) (CAS-RN:[1538957-14-0], 170 mg, 779 μmol), PyBOP (CAS-RN:[128625-52-5], 608 mg, 1.17 mmol) and N,N-diisopropylethylamine (410 μL, 2.3 mmol) were dissolved in 3.6 mL N,N-dimethylacetamide and stirred at room temperature under nitrogen atmosphere overnight. Aqueous saturated sodium hydrogencarbonate solution was added to the reaction mixture. It was diluted with dichloromethane and the aqueous layer was extracted with dichloromethane once. The organic layer was dried using a waterresistant filter and concentrated under reduced pressure. The crude product was purified by preparative reverse phase HPLC (eluent: acetonitrile / water with ammonia as additive) and afterwards it was dissolved in some dichloromethane and diluted slowly with hexanes. The precipitated solid was filtered off and dried to provide 48 mg of the title compound.

LC-MS (Method 2): Rt = 1.21 min; MS (ESIpos): m/z = 555.5 $[M+H]^+$

$^1$H-NMR (400MHz, DMSO-$d_6$): δ [ppm]= 2.08 - 2.27 (m, 1H), 2.58 - 2.74 (m, 1H), 2.83 (s, 3H), 2.96 (t, 2H), 3.56 (t, 2H), 4.12 (dd, 1H), 5.78 - 6.13 (m, 1H), 6.75 (dd, 1H), 6.93 - 7.02 (m, 1H), 7.10 - 7.24 (m, 4H), 7.37 - 7.47 (m, 2H), 7.92 (s, 1H), 8.09 (d, 1H), 10.69 (s, 1H), 11.99 (s, 1H)

**Example 67**

N-{4-[3-(2,4-difluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (Enantiomer 1)

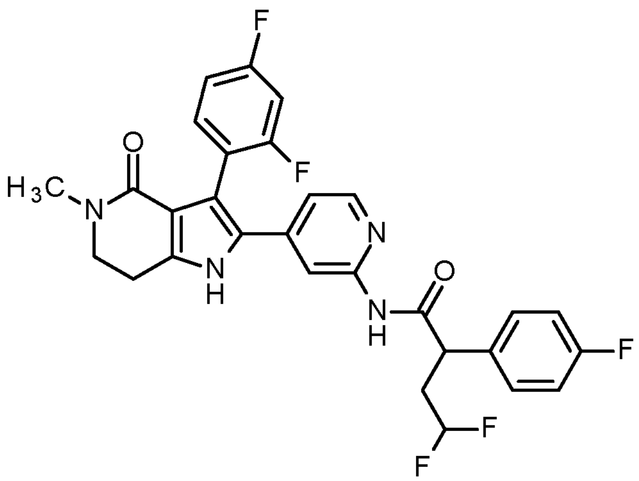

The racemic compound (see Example 66, 43 mg) was separated into enantiomers by preparative chiral HPLC to give enantiomer 1 (18 mg, > 99% ee, see Example 67) and enantiomer 2 (21 mg, > 99% ee, see Example 68).

Preparative chiral HPLC method:
Instrument: PrepCon Labomatic HPLC; Column: YMC Cellulose SB 10μ, 250x50; eluent A: hexane + 0.1 vol % diethylamine; eluent B: ethanol + 0.1 vol % diethylamine; isocratic: 80%A+20%B; flow: 80 mL/min; temperature: 25°C; UV: 254 nm
Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 80%A+20%B; flow: 1.4 mL/min; temperature: 25°C; UV: 254 nm

Analytical Chiral HPLC (method Example 67): Rt = 1.59 min.

LC-MS (Method 2): Rt = 1.20 min; MS (ESIpos): m/z = 555 $[M+H]^+$
$^1H$ NMR (400 MHz, DMSO-$d_6$) δ ppm 2.09 - 2.26 (m, 1 H) 2.59 - 2.76 (m, 1 H) 2.83 (s, 3 H) 2.96 (t, 2 H) 3.56 (t, 2 H) 4.12 (dd, 1 H) 5.79 - 6.19 (m, 1 H) 6.75 (dd, 1 H) 6.98 (br t, 1 H) 7.11 - 7.27 (m, 4 H) 7.36 - 7.50 (m, 2 H) 7.92 (s, 1 H) 8.09 (d, 1 H) 10.69 (s, 1 H) 12.00 (s, 1 H).

**Example 68**

N-{4-[3-(2,4-difluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (Enantiomer 2)

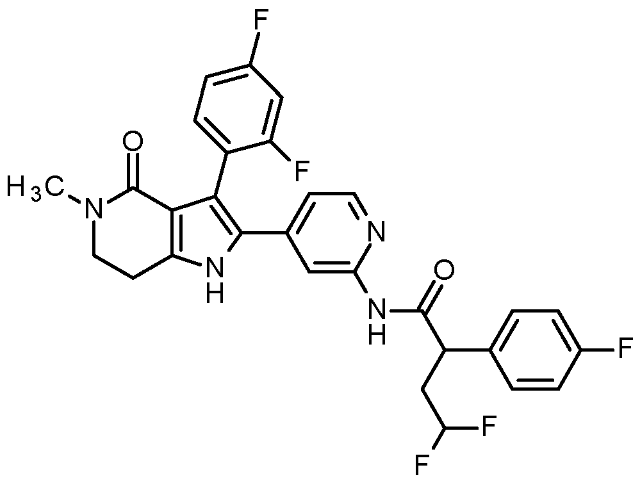

For the preparation of the racemic title compound see Example 66. Separation of enantiomers by preparative chiral HPLC (method see Example 67) gave the title compound (21 mg, > 99% ee).
Analytical Chiral HPLC (method see Example 67): $R_t$ = 5.56 min.

LC-MS (Method 2): $R_t$ = 1.20 min; MS (ESIpos): m/z = 555 $[M+H]^+$

$^1H$ NMR (400 MHz, DMSO-$d_6$) δ ppm 2.09 - 2.26 (m, 1 H) 2.57 - 2.77 (m, 1 H) 2.83 (s, 3 H) 2.96 (t, 2 H) 3.56 (t, 2 H) 4.12 (dd, 1 H) 5.75-6.16 (m, 1 H) 6.75 (dd, 1 H) 6.98 (br t, 1 H) 7.10 - 7.23 (m, 4 H) 7.38 - 7.46 (m, 2 H) 7.92 (s, 1 H) 8.09 (d, 1 H) 10.69 (s, 1 H) 12.00 (s, 1 H).

**Example 69**

N-{4-[3-(2,4-difluorophenyl)-5-methyl-4-oxo-4,5,6,7-tetrahydro-1H-pyrrolo[3,2-c]pyridin-2-yl]pyridin-2-yl}-2-(4-fluorophenyl)propanamide (Racemate)

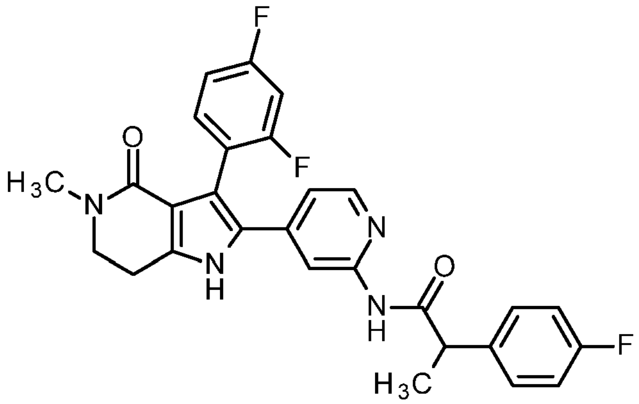

2-(2-Aminopyridin-4-yl)-3-(2,4-difluorophenyl)-5-methyl-1,5,6,7-tetrahydro-4H-pyrrolo[3,2-c]-pyridin-4-one (see Intermediate 60, 138 mg), 2-(4-fluorophenyl)propanoic acid (Racemate) (CAS-RN:[75908-73-5], 131 mg, 779 μmol), PyBOP (CAS-RN:[128625-52-5], 608 mg, 1.17 mmol) and N,N-diisopropylethylamine (410 μL, 2.3 mmol) were dissolved in 3.6 mL N,N-dimethylacetamide and stirred at room temperature under nitrogen atmosphere overnight. Aqueous saturated sodium hydrogencarbonate solution was added to the reaction mixture. It was diluted with dichloromethane and the aqueous layer was extracted with dichloromethane once. The organic layer was dried using a waterresistant filter and concentrated under reduced pressure. The crude product was purified by preparative reverse phase HPLC (eluent: acetonitrile / water with ammonia as additive) and afterwards it was dissolved in some dichloromethane and diluted slowly with hexanes. The precipitated solid was filtered off and dried to provide 59.5 mg of the title compound.

LC-MS (Method 2): Rt = 1.19 min; MS (ESIpos): m/z = 505 $[M+H]^+$

$^1$H-NMR (400MHz, DMSO-$d_6$): δ [ppm]= 1.35 (d, 3H), 2.83 (s, 3H), 2.95 (t, 2H), 3.56 (t, 2H), 3.97 (q, 1H), 6.70 (dd, 1H), 6.98 (br t, 1H), 7.09 - 7.27 (m, 4H), 7.34 - 7.44 (m, 2H), 7.98 (s, 1H), 8.08 (d, 1H), 10.55 (s, 1H), 11.99 (s, 1H).

## Example 70

N-{4-[3-(2,4-difluorophenyl)-5-methyl-4-oxo-4,5,6,7-tetrahydro-1H-pyrrolo[3,2-c]pyridin-2-yl]pyridin-2-yl}-2-(4-fluorophenyl)propanamide (Enantiomer 1)

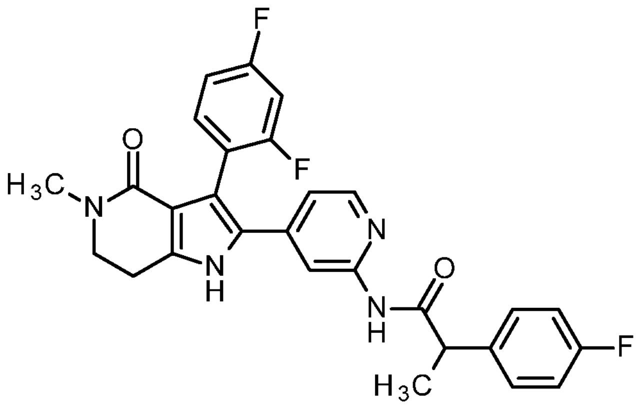

The racemic compound (see Example 69, 55 mg) was separated into enantiomers by preparative chiral HPLC to give enantiomer 1 (20 mg, > 99% ee, see Example 70) and enantiomer 2 (21 mg, > 99% ee, see Example 71).

Preparative chiral HPLC method:
Instrument: PrepCon Labomatic HPLC; Column: YMC Cellulose SB 10μ, 250x50; eluent A: hexane + 0.1 vol % diethylamine; eluent B: ethanol + 0.1 vol % diethylamine; isocratic: 70%A+30%B; flow: 80 mL/min; temperature: 25°C; UV: 254 nm

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 70%A+30%B; flow: 1.4 mL/min; temperature: 25°C; UV: 254 nm

Analytical Chiral HPLC (method Example 70): Rt = 1.42 min.

LC-MS (Method 2): Rt = 1.17 min; MS (ESIpos): m/z = 505 $[M+H]^+$

$^1$H NMR (400 MHz, DMSO-$d_6$) δ ppm 1.35 (d, 3 H) 2.83 (s, 3 H) 2.95 (t, 2 H) 3.56 (t, 2 H) 3.97 (q, 1 H) 6.70 (dd, 1 H) 6.92 - 7.06 (m, 1 H) 7.08 - 7.23 (m, 4 H) 7.34 - 7.46 (m, 2 H) 7.98 (s, 1 H) 8.06 - 8.12 (m, 1 H) 10.55 (s, 1 H) 11.99 (s, 1 H).

**Example 71**

N-{4-[3-(2,4-difluorophenyl)-5-methyl-4-oxo-4,5,6,7-tetrahydro-1H-pyrrolo[3,2-c]pyridin-2-yl]pyridin-2-yl}-2-(4-fluorophenyl)propanamide (Enantiomer 2)

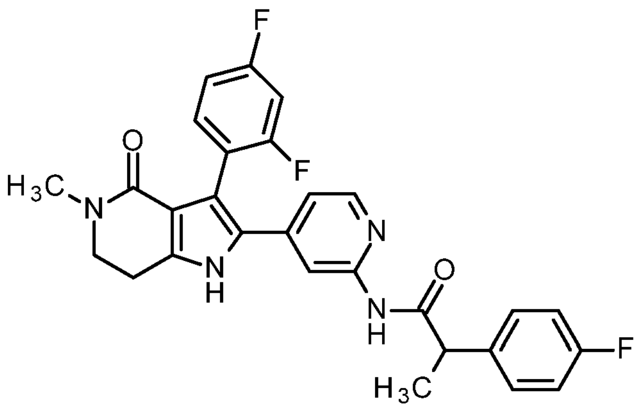

For the preparation of the racemic title compound see Example 69. Separation of enantiomers by preparative chiral HPLC (method see Example 70) gave the title compound (21 mg, > 99% ee).
Analytical Chiral HPLC (method see Example 70): $R_t$ = 6.35 min.

LC-MS (Method 2): $R_t$ = 1.17 min; MS (ESIpos): m/z = 505 $[M+H]^+$

$^1$H NMR (400 MHz, DMSO-$d_6$) δ ppm 1.35 (d, 3 H) 2.83 (s, 3 H) 2.95 (t, 2 H) 3.56 (t, 2 H) 3.97 (q, 1 H) 6.70 (dd, 1 H) 6.95 - 7.05 (m, 1 H) 7.10 - 7.26 (m, 4 H) 7.32 - 7.43 (m, 2 H) 7.98 (s, 1 H) 8.08 (d, 1 H) 10.55 (s, 1 H) 11.99 (s, 1 H).

**Example 72**

2-(4-fluorophenyl)-N-[4-(4-oxo-3-phenyl-4,5,6,7-tetrahydro-1H-pyrrolo[3,2-c]pyridin-2-yl)pyridin-2-yl]acetamide

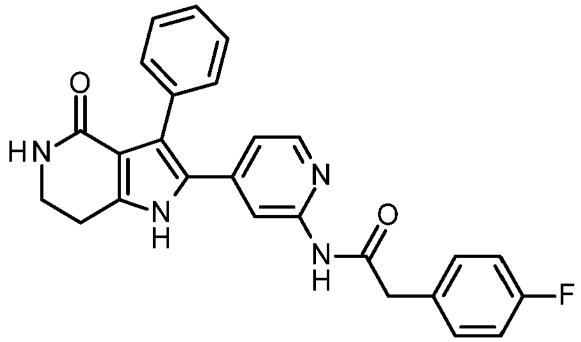

2-(2-Aminopyridin-4-yl)-3-phenyl-1,5,6,7-tetrahydro-4H-pyrrolo[3,2-c]pyridin-4-one (see Intermediate 63, 310 mg), (4-fluorophenyl)acetic acid (CAS-RN:[405-50-5], 314 mg, 2.04 mmol), PyBOP (CAS-RN:[128625-52-5], 1.59 g, 3.06 mmol) and N,N-diisopropylethylamine (1.1 mL, 6.1 mmol) were dissolved in 9.5 mL N,N-dimethylacetamide and were stirred at room temperature under nitrogen atmosphere for three days. Aqueous saturated sodium hydrogencarbonate solution was added to the reaction mixture, it was diluted with dichloromethane and the aqueous layer was extracted with dichloromethane twice. The combined organic layers were washed with water once, dried using a silicon coated filter and concentrated under reduced pressure. The crude product was purified by flash chromatography (25 g column, silica ULTRA, gradient: ethyl acetate / ethanol 0-30% ethanol) and HPLC chromatography (eluent: acetonitrile / water with ammonia as additive) to provide 60.5 mg of the title compound.

LC-MS (Method 2): $R_t$ = 0.99 min; MS (ESIpos): m/z = 441 $[M+H]^+$

$^1$H-NMR (400MHz, DMSO-$d_6$): δ[ppm]= 2.83 (t, 2H), 3.38 (td, 2H), 3.68 (s, 2H), 6.51 (dd, 1H), 6.95 (br s, 1H), 7.10 - 7.29 (m, 7H), 7.34 - 7.40 (m, 2H), 7.99 (d, 1H), 8.05 (s, 1H), 10.64 (s, 1H), 11.84 (s, 1H).

## Example 73

2-(4-fluorophenyl)-N-[4-(4-oxo-3-phenyl-4,5-dihydro-1H-pyrrolo[3,2-c]pyridin-2-yl)pyridin-2-yl]acetamide

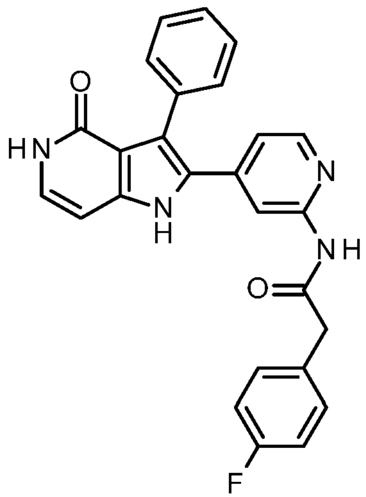

2-(4-Fluorophenyl)-N-[4-(4-oxo-3-phenyl-4,5,6,7-tetrahydro-1H-pyrrolo[3,2-c]pyridin-2-yl)-pyridin-2-yl]acetamide (see Example 72, 81.0 mg) was dissolved in 1,4-dioxane (7.9 mL) and 2,3-dichloro-5,6-dicyano-1,4-benzochinone (CAS-RN:[84-58-2], 48.0 mg, 211 μmol) was added. The reaction mixture was stirred at 80 °C for one hour under argon atmosphere. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, dried using a water resistant filter and concentrated under reduced pressure. The crude product was dissolved in 1,4-dioxane (7.9 mL) again and 2,3-dichloro-5,6-dicyano-1,4-benzochinone (CAS-RN:[84-58-2], 48.0 mg, 211 μmol) was added. The reaction mixture was stirred at 80 °C for one hour under argon

atmosphere. The reaction mixture was diluted with ethyl acetate and treated with 1 M aqueous sodiumhydroxyd-solution again. The layers were separated and the aqueous layer was extracted with ethyl acetate three times. The combined organic layers were washed once with water and brine, dried using a water resistant filter and concentrated under reduced pressure.The crude product was purified by flash chromatography (10 g column, silica ULTRA, gradient: ethyl acetate / ethanol 0-10% ethanol and 10%-100% ethanol) to provide 3.8 mg of the title compound.

LC-MS (Method 2): $R_t$ = 1.02 min; MS (ESIpos): m/z = 439 $[M+H]^+$

$^1$H-NMR (400MHz, DMSO-$d_6$): δ [ppm]= 3.70 (s, 2H), 6.39 (dd, 1H), 6.64 (dd, 1H), 7.04 (dd, 1H), 7.11 - 7.21 (m, 2H), 7.25 - 7.31 (m, 5H), 7.33 - 7.41 (m, 2H), 8.07 (d, 1H), 8.15 (s, 1H), 10.72 (s, 2H), 12.00 (s, 1H).

**Example 74**

2-(4-fluorophenyl)-N-[4-(4-oxo-3-phenyl-4,5,6,7-tetrahydro-1H-pyrrolo[3,2-c]pyridin-2-yl)pyridin-2-yl]propanamide (Racemate)

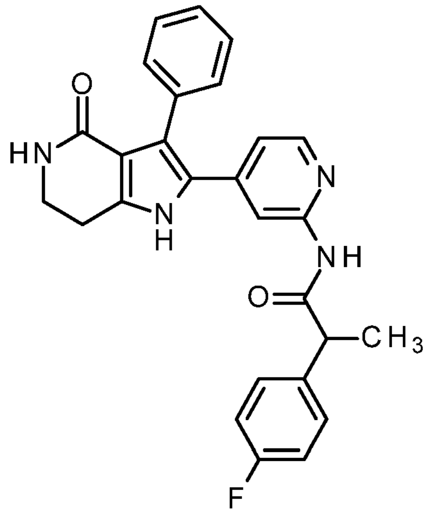

2-(2-Aminopyridin-4-yl)-3-phenyl-1,5,6,7-tetrahydro-4H-pyrrolo[3,2-c]pyridin-4-one (see Intermediate 63, 310 mg), 2-(4-fluorophenyl)propanoic acid (Racemate) (CAS-RN:[75908-73-5], 343 mg, 2.04 mmol), PyBOP (CAS-RN:[128625-52-5], 1.59 g, 3.06 mmol) and N,N diisopropylethylamine (1.1 mL, 6.1 mmol) were dissolved in 9.5 mL N,N-dimethylacetamide and stirred at room temperature under nitrogen atmosphere overnight. Aqueous saturated sodium hydrogencarbonate solution was added to the reaction mixture. It was diluted with dichloromethane and the aqueous layer was extracted with dichloromethane twice. The combined organic layers were washed with water once, dried using a silicone coated filter and concentrated under reduced pressure. The crude product was purified by flash chromatography (25 g column, silica ULTRA, gradient: ethyl acetate / ethanol 0-30% ethanol) and by preparative reverse phase HPLC (eluent: acetonitrile / water with ammonia as additive) to provide 16 mg of the title compound.

LC-MS (Method 2): Rt = 1.10 min; MS (ESIpos): m/z = 455 $[M+H]^+$

$^1$H-NMR (400MHz, DMSO-$d_6$): δ [ppm]= 1.38 (d, 3H), 2.84 (t, 2H), 3.39 (td, 2H), 4.00 (q, 1H), 6.49 (dd, 1H), 6.96 (br s, 1H), 7.12 - 7.30 (m, 7H), 7.37 - 7.45 (m, 2H), 7.97 (d, 1H), 8.08 (s, 1H), 10.58 (s, 1H), 11.85 (s, 1H).

Example **75**

4,4-difluoro-2-(4-fluorophenyl)-N-[4-(4-oxo-3-phenyl-4,5,6,7-tetrahydro-1H-pyrrolo[3,2-c]pyridin-2-yl)pyridin-2-yl]butanamide (Racemate)

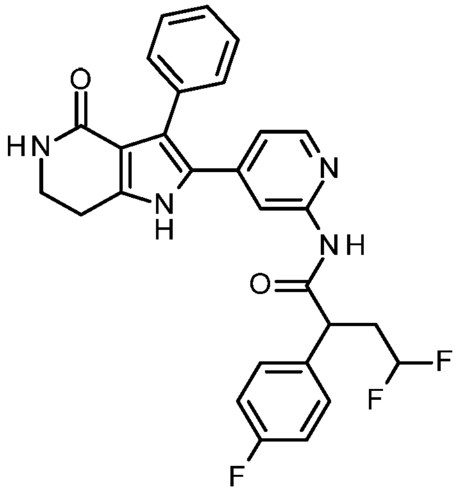

2-(2-Aminopyridin-4-yl)-3-phenyl-1,5,6,7-tetrahydro-4H-pyrrolo[3,2-c]pyridin-4-one (see Intermediate 63, 310 mg), 4,4-difluoro-2-(4-fluorophenyl)butanoic acid (Racemate) (CAS-RN:[1538957-14-0], 444 mg, 2.04 mmol), PyBOP (CAS-RN:[128625-52-5], 1.59 g, 3.06 mmol) and N,N-diisopropylethylamine (1.1 mL, 6.1 mmol) were dissolved in 9.5 mL N,N-dimethylacetamide and stirred at room temperature under nitrogen atmosphere overnight. Aqueous saturated sodium hydrogencarbonate solution was added to the reaction mixture. It was diluted with dichloromethane and the aqueous layer was extracted with dichloromethane twice. The combined organic layers were washed with water once, dried using a silicone coated filter and concentrated under reduced pressure. The crude product was diluted with dichloromethane and the precipitated solid was filtered off. Afterwards it was purified by preparative reverse phase HPLC (eluent: acetonitrile / water with ammonia as additive) to provide 121 mg of the title compound.

LC-MS (Method 2): $R_t$ = 1.13 min; MS (ESIpos): m/z = 505 $[M+H]^+$

$^1$H NMR (400 MHz, DMSO-$d_6$) $\delta$ ppm 0.19 - 0.27 (m, 2 H) 0.46 - 0.58 (m, 2 H) 1.05 - 1.17 (m, 1 H) 2.11 - 2.28 (m, 1 H) 2.57 (d, 2 H) 2.67 (dt, 1 H) 3.38 (s, 3 H) 4.16 (dd, 1 H) 5.78 - 6.14 (m, 1 H) 6.83 (dd, 1 H) 7.04 - 7.12 (m, 2 H) 7.16 - 7.31 (m, 5 H) 7.41 - 7.50 (m, 2 H) 8.05 (s, 1 H) 8.14 (dd, 1 H) 10.78 (s, 1 H) 11.80 (s, 1 H).

## Example 76

4,4-difluoro-2-(4-fluorophenyl)-N-[4-(4-oxo-3-phenyl-4,5-dihydro-1 H-pyrrolo[3,2-c]pyridin-2-yl)pyridin-2-yl]butanamide (Racemate)

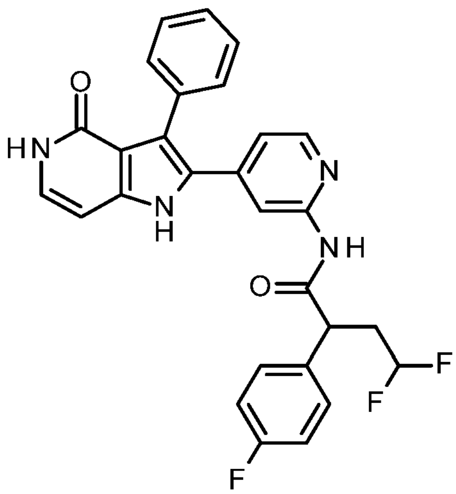

4,4-Difluoro-2-(4-fluorophenyl)-N-[4-(4-oxo-3-phenyl-4,5,6,7-tetrahydro-1 H-pyrrolo[3,2-c]-pyridin-2-yl)pyridin-2-yl]butanamide (Racemate) (see Example 75, 140 mg) was dissolved in 1,4-dioxane (12 mL) and 2,3-dichloro-5,6-dicyano-1,4-benzochinone (CAS-RN:[84-58-2], 72.4 mg, 319 μmol) was added. The reaction mixture was stirred at 80 °C for 2 hours under argon atmosphere. The reaction mixture was diluted with ethyl acetate and treated with 1 M aqueous sodiumhydroxyd-solution. The layers were separated and the aqueous layer was extracted with ethyl acetate three times. The combined organic layers were washed once with water and brine, dried using a water resistant filter and concentrated under reduced pressure. The crude product was dissolved in 1,4-dioxane (12 mL) again and 2,3-dichloro-5,6-dicyano-1,4-benzochinone (CAS-RN:[84-58-2], 72.4 mg, 319 pmol) was added. The reaction mixture was stirred at 80 °C for 2 hours under argon atmosphere. The reaction mixture was diluted with ethyl acetate and treated with 1 M aqueous sodiumhydroxyd-solution again. The layers were separated and the aqueous layer was extracted with ethyl acetate three times. The combined organic layers were washed once with water and brine, dried using a water resistant filter and concentrated under reduced pressure. The crude product was purified by preparative reverse phase HPLC (eluent: acetonitrile / water with ammonia as additive) to provide 31.4 mg of the title compound.

LC-MS (Method 2): $R_t$ = 1.13 min; MS (ESIpos): m/z = 503 $[M+H]^+$

$^1$H-NMR (400MHz, DMSO-$d_6$): δ [ppm]= 2.11 - 2.29 (m, 1H), 2.61 - 2.82 (m, 1H), 4.17 (dd, 1H), 5.80 - 6.21 (m, 1H), 6.41 (d, 1H), 6.65 (dd, 1H), 7.01 - 7.09 (m, 1H), 7.20 (t, 2H), 7.27 (s, 5H), 7.43 - 7.51 (m, 2H), 8.06 (d, 1H), 8.12 (s, 1H), 10.73 (br d, 1H), 10.81 (s, 1H), 12.02 (s, 1H).

## Example 77

(-)-4,4-difluoro-2-(4-fluorophenyl)-N-[4-(4-oxo-3-phenyl-4,5-dihydro-1 H-pyrrolo[3,2-c]pyridin-2-yl)pyridin-2-yl]butanamide (Enantiomer 1)

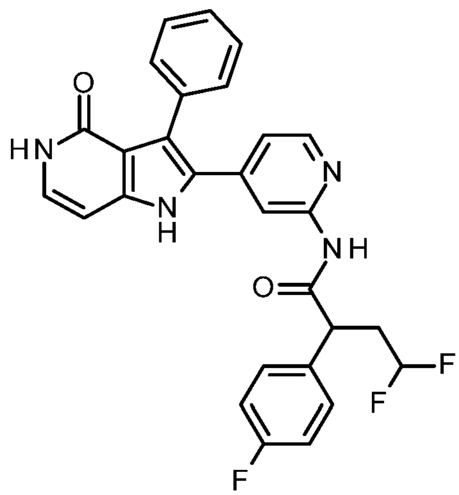

The racemic compound (see Example 76, 22 mg) was separated into enantiomers by preparative chiral HPLC to give enantiomer 1 (6.5 mg, > 99% ee, see Example 77) and enantiomer 2 (5.9 mg, > 99% ee, see Example 78).

Preparative chiral HPLC method:
Instrument: PrepCon Labomatic HPLC; Column: YMC Cellulose SB 5μ, 250x30; eluent A: hexane + 0.1 vol % diethylamine; eluent B: ethanol; isocratic: 90%A+10%B; flow: 85 mL/min; temperature: 25°C; UV: 254 nm;

Analytical chiral HPLC method:
Instrument: Thermo Fisher UltiMate 3000; Column: YMC Cellulose SB 3μ, 100x4.6; eluent A: hexane + 0.1 vol % diethylamine; eluent B: ethanol; isocratic: 90%A+10%B; flow: 1.4 mL/min; temperature: 25°C; UV: 254 nm;

Analytical Chiral HPLC (method Example 77): Rt = 5.87 min.

$[\alpha]_D$ = -158.1° (from solution in DMSO, c = 3.2 mg/mL)

$^1$H NMR (400 MHz, DMSO-$d_6$) δ ppm 2.08 - 2.28 (m, 1 H), 2.61 - 2.81 (m, 1 H), 4.17 (br dd, 1 H), 5.76 - 6.21 (m, 1 H), 6.36 - 6.49 (m, 1 H), 6.66 (dd, 1 H), 7.01 - 7.11 (m, 1 H), 7.16 - 7.33 (m, 7 H), 7.40 - 7.52 (m, 2 H), 8.06 (d, 1

H), 8.12 (s, 1 H), 10.74 (br d, 1 H), 10.81 (s, 1 H), 12.03 (s, 1 H).

**Example 78**

(+)-4,4-difluoro-2-(4-fluorophenyl)-N-[4-(4-oxo-3-phenyl-4,5-dihydro-1 H-pyrrolo[3,2-c]pyridin-2-yl)pyridin-2-yl]butanamide (Enantiomer 2)

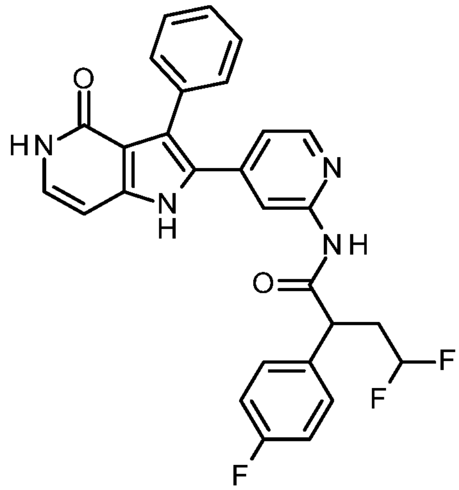

For the preparation of the racemic title compound see Example 76. Separation of enantiomers by preparative chiral HPLC (method see Example 77) gave the title compound (5.9 mg, > 99% ee).

Analytical Chiral HPLC (method see Example 77): $R_t$ = 6.76 min.

$[\alpha]_D$ = +166.3° (from solution in DMSO, c = 2.95 mg/mL)

$^1$H NMR (400 MHz, DMSO-$d_6$) $\delta$ ppm 2.06 - 2.30 (m, 1 H), 2.62 - 2.82 (m, 1 H), 4.17 (br dd, 1 H), 5.76 - 6.21 (m, 1 H), 6.35 - 6.50 (m, 1 H), 6.66 (dd, 1 H), 7.00 - 7.12 (m, 1 H), 7.14 - 7.35 (m, 7 H), 7.37 - 7.55 (m, 2 H), 8.00 - 8.22 (m, 2 H), 10.74 (br d, 1 H), 10.81 (s, 1 H), 12.02 (s, 1 H).

**Example 79**

N-[4-(7-chloro-5-methyl-4-oxo-3-phenyl-4,5-dihydro-1H-pyrrolo[3,2-c]pyridin-2-yl)pyridin-2-yl]-2-(4-fluorophenyl)acetamide

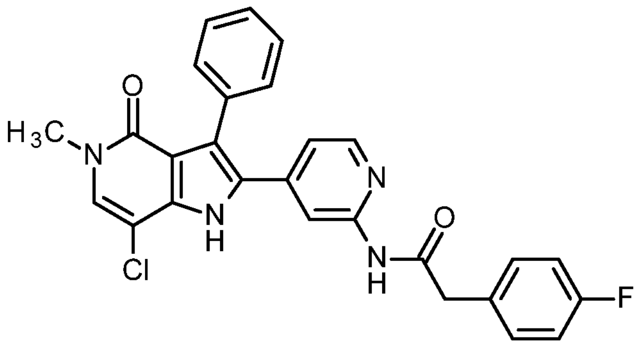

2-(4-Fluorophenyl)-N-[4-(5-methyl-4-oxo-3-phenyl-4,5-dihydro-1 H-pyrrolo[3,2-c]pyridin-2-yl)pyridin-2-yl]acetamide (see Example 5, 20.0 mg) was suspended in DMF (100 $\mu$L), N-chlorosuccinimide (CAS-RN:[128-09-6], 5.90 mg, 44.2 $\mu$mol) was added and the mixture was stirred at room temperature for one day under nitrogen atmosphere.

Then further N-chlorosuccinimide (CAS-RN:[128-09-6], 2.95 mg, 22.1 μmol) was added and the mixture was stirred at rt overnight. The mixture was directly purified by preparative HPLC chromatography (eluent: acetonitrile / water with ammonia as additive) to provide 6.4 mg of the title compound.

LC-MS (Method 2): Rt = 1.13 min; MS (ESIpos): m/z = 487 $[M+H]^+$

$^1$H-NMR (400MHz, DMSO-$d_6$): δ [ppm]= 3.39 (s, 3H), 3.69 (s, 2H), 6.71 (dd, 1H), 7.09 - 7.19 (m, 2H), 7.20 - 7.31 (m, 5H), 7.33 - 7.42 (m, 2H), 7.73 (s, 1H), 8.11 (d, 1H), 8.16 (s, 1H), 10.73 (s, 1H), 12.31 (s, 1H).

## Example 80

4,4-difluoro-2-(4-fluorophenyl)-N-[4-(5'-methyl-4'-oxo-3'-phenyl-1',4',5',7'-tetrahydrospiro-[cyclopropane-1,6'-pyrrolo[3,2-c]pyridin]-2'-yl)pyridin-2-yl]butanamide (Racemate)

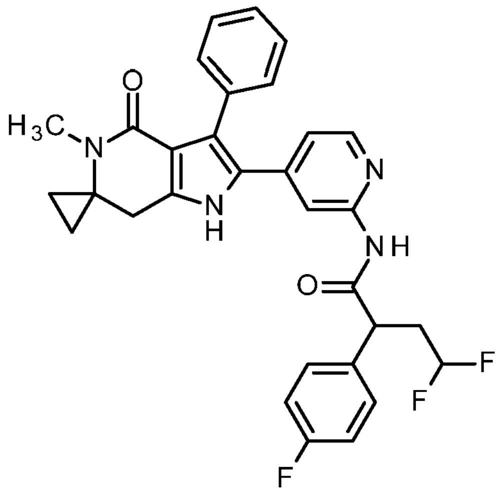

2'-(2-Aminopyridin-4-yl)-5'-methyl-3'-phenyl-1',7'-dihydrospiro[cyclopropane-1,6'-pyrrolo[3,2-c]-pyridin]-4'(5'H)-one (see Intermediate 69, 79.0 mg), 4,4-difluoro-2-(4-fluorophenyl)butanoic acid (Racemate) (CAS-RN:[1538957-14-0], 75.1 mg, 344 μmol), PyBOP (CAS-RN:[128625-52-5], 358 mg, 688 μmol) and N,N-diisopropylethylamine (240 μL, 1.4 mmol) were dissolved in 1.3 mL N,N-dimethylacetamide and stirred at room temperature under nitrogen atmosphere for 2 days. Aqueous saturated sodium hydrogencarbonate solution was added to the reaction mixture. It was diluted with dichloromethane and water. The aqueous layer was extracted with dichloromethane once, the organic layer was dried using a silicone coated filter and concentrated under reduced pressure. The crude product was purified by preparative reverse phase HPLC (eluent: acetonitrile / water with formic acid as additive) to provide 6.4 mg of the title compound.

LC-MS (Method 2): Rt = 1.26 min; MS (ESIpos): m/z = 545.5 $[M+H]^+$
$^1$H NMR (400 MHz, DMSO-$d_6$) δ ppm 0.70 - 0.82 (m, 2 H) 1.03 - 1.13 (m, 2 H) 2.11 - 2.28 (m, 1 H) 2.59 - 2.77 (m, 4 H) 2.78 - 2.92 (m, 2 H) 4.15 (dd, 1 H) 5.76 - 6.16 (m, 1 H) 6.54 (dd, 1 H) 7.14 - 7.30 (m, 7 H) 7.40 - 7.50 (m, 2 H) 7.98 (d, 1 H) 8.01 (s, 1 H) 10.72 (s, 1 H) 11.85 (s, 1 H).

## Example 81

4,4-difluoro-2-(4-fluorophenyl)-N-{4-[3'-(4-fluorophenyl)-5'-methyl-4'-oxo-1',4',5',7'-tetrahydro-spiro[cyclopropane-1,6'-pyrrolo[3,2-c]pyridin]-2'-yl]pyridin-2-yl}butanamide (Racemate)

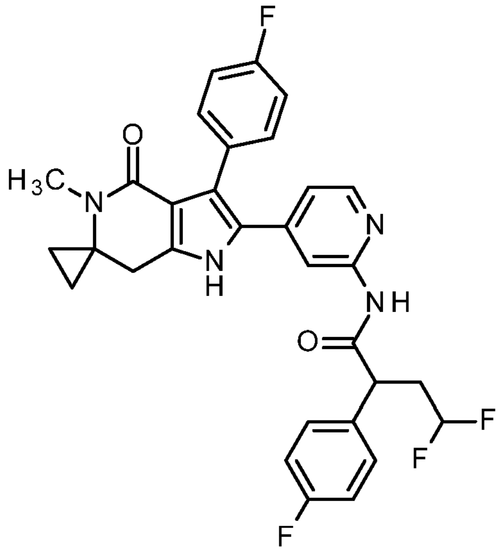

2'-(2-Aminopyridin-4-yl)-3'-(4-fluorophenyl)-5'-methyl-1',7'-dihydrospiro[cyclopropane-1,6'-pyrrolo[3,2-c]pyridin]-4'(5'H)-one (see Intermediate 72, 172 mg), 4,4-difluoro-2-(4-fluorophenyl)-butanoic acid (Racemate) (CAS-RN:[1538957-14-0], 155 mg, 712 μmol), PyBOP (CAS-RN:[128625-52-5], 741 mg, 1.42 mmol) and N,N-diisopropylethylamine (500 μL, 2.8 mmol) were dissolved in 2.8 mL N,N-dimethylacetamide and stirred at room temperature under nitrogen atmosphere for 2 days. Aqueous saturated sodium hydrogencarbonate solution was added to the reaction mixture. It was diluted with dichloromethane and water. The aqueous layer was extracted with dichloromethane once, the organic layer was dried using a silicone coated filter and concentrated under reduced pressure. The crude product was purified by preparative reverse phase HPLC (eluent: acetonitrile / water with ammonia as additive) to provide 38 mg of the title compound.

LC-MS (Method 2): $R_t$ = 1.26 min; MS (ESIpos): m/z = 563.5 $[M+H]^+$

$^1$H-NMR (400MHz, DMSO-$d_6$): δ [ppm]= 0.69 - 0.79 (m, 2H), 1.00 - 1.14 (m, 2H), 2.09 - 2.28 (m, 1H), 2.58 - 2.74 (m, 4H), 2.84 (d, 2H), 4.14 (dd, 1H), 5.79 - 6.15 (m, 1H), 6.64 (dd, 1H), 7.03 - 7.11 (m, 2H), 7.14 - 7.26 (m, 4H), 7.38 - 7.49 (m, 2H), 7.97 (s, 1H), 8.05 (d, 1H), 10.72 (s, 1H), 11.87 (s, 1H).

## Example 82

N-[4-(6,6-dimethyl-4-oxo-3-phenyl-4,5,6,7-tetrahydro-1 H-pyrrolo[3,2-c]pyridin-2-yl)pyridin-2-yl]-2-(4-fluorophenyl)acetamide

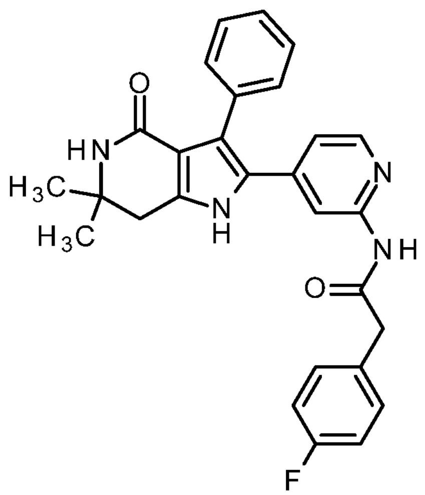

To a screw cap vial was added 2-(2-aminopyridin-4-yl)-6,6-dimethyl-3-phenyl-1,5,6,7-tetrahydro-4H-pyrrolo[3,2-c]pyridin-4-one formic acid salt (see Intermediate 75, 12.0 mg), 2-(4-fluorophenyl)acetic acid (8.26 mg, 53.6 μmol) and N-(3-Dimethylaminopropyl)-N'-ethylcarbodiimide hydrochloride (10.2 mg, 53.6 μmol) followed by 360 μL of N,N-dimethylformamide. To the reaction mixture was added diisopropylethylamine (18.7 μL 107 μmol). The reaction was stirred at rt for 16 hours under nitrogen, followed by another addition of N-(3-Dimethylaminopropyl)-N'-ethylcarbodiimide hydrochloride (10.2 mg, 53.6 μmol), 2-(4-fluorophenyl)acetic acid (8.26 mg, 53.6 μmol) and diisopropylethylamine (18.7 μL 107 μmol). After 48 hours, the reaction was concentrated, and the crude product was purified by preparative reverse phase HPLC (eluent: acetonitrile / water 5 to 70 % + 0.1 % formic acid) The title compound containing fractions were concentrated under reduced pressure to provide 2.7 mg of the title compound.

LC-MS (Method 4): Rt= 2.29 min; MS (ESIpos): m/z = 469 $[M+H]^+$

$^1$H-NMR (400 MHz, DMSO-$d_6$) δ [ppm] = 11.79 (s, 1H), 10.63 (s, 1H), 8.04 (d, J = 1.5 Hz, 1H), 8.01 - 7.96 (m, 1H), 7.40 - 7.31 (m, 2H), 7.29 - 7.19 (m, 5H), 7.19 - 7.11 (m, 2H), 6.84 (s, 1H), 6.51 (dd, J = 5.3, 1.7 Hz, 1H), 3.68 (s, 2H), 2.79 (s, 2H), 1.27 (s, 6H).

**Example 83**

2-(4-fluorophenyl)-N-{4-[3-(4-fluorophenyl)-6,6-dimethyl-4-oxo-4,5,6,7-tetrahydro-1H-pyrrolo[3,2-c]pyridin-2-yl]pyridin-2-yl}acetamide

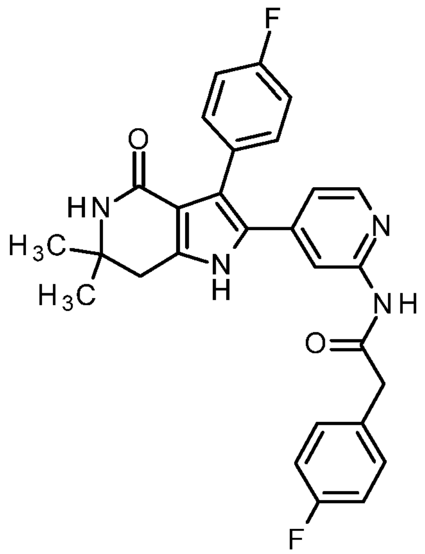

To a solution of 2-(2-aminopyridin-4-yl)-3-(4-fluorophenyl)-6,6-dimethyl-1,5,6,7-tetrahydro-4H-pyrrolo[3,2-c]pyridin-4-one formic acid salt (see Intermediate 78, 27.5 mg), 2-(4-fluorophenyl)acetic acid (18.2 mg, 118 μmol) and PyBop (53.1 mg, 102 μmol) in 780 μL of *N,N*-dimethylformamide was added diisopropylethylamine (41.0 μL, 235 μmol).The reaction was stirred at 50 °C for 10 hours. To the reaction was added more PyBop (53.1 mg, 102 μmol) and diisopropylethylamine (41.0 μL, 235 μmol). The reaction was heated at 65 °C for 60 hours, the reaction was concentrated and the crude product was purified by preparative reverse phase HPLC (eluent: acetonitrile / water 15 to 50 % + 0.1 % formic acid) The title compound containing fractions were concentrated under reduced pressure to provide the title compound: 5.30 mg.

LC-MS (Method 5): Rt = 4.30 min; MS (ESIpos): m/z = 487 $[M+H]^+$

$^1$H-NMR (400 MHz, DMSO-$d_6$) δ [ppm] = 11.81 (s, 1H), 10.62 (s, 1H), 8.05 (d, J = 5.3 Hz, 1H), 8.00 (d, J = 1.6 Hz, 1H), 7.35 (dd, J = 8.5, 5.7 Hz, 2H), 7.23 (dd, J = 8.6, 5.8 Hz, 2H), 7.19 - 7.10 (m, 2H), 7.07 (t, J = 8.9 Hz, 2H), 6.87 (s, 1H), 6.60 (dd, J = 5.3, 1.6 Hz, 1H), 3.67 (s, 2H), 2.79 (s, 2H), 1.27 (s, 6H).

**Example 84**

2-(4-fluorophenyl)-N-{4-[3-(2-fluorophenyl)-6,6-dimethyl-4-oxo-4,5,6,7-tetrahydro-1H-pyrrolo[3,2-c]pyridin-2-yl]pyridin-2-yl}acetamide

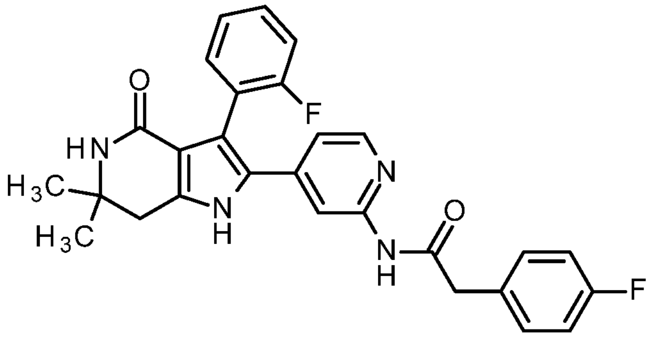

A screw cap vial was charged with 2-(2-aminopyridin-4-yl)-3-(2-fluorophenyl)-6,6-dimethyl-1,5,6,7-tetrahydro-4H-pyrrolo[3,2-c]pyridin-4-one formic acid salt (see Intermediate 84, 74.0 mg), 2-(4-fluorophenyl)acetic acid (39.1 mg, 253 μmol), COMU (CAS-RN:[1075198-30-9], 136 mg, 317 μmol). The vial was filled with 1.06 mL of *N,N*-dimethylacetamide followed by the addition of diisopropylethylamine (73.6 μL, 422 μmol).The reaction was stirred at 50 °C for 16 h. The reaction was cooled down and COMU (CAS-RN:[1075198-30-9], 136 mg, 317 μmol), 2-(4-fluorophenyl)acetic acid (39.1 mg, 253 μmol) and diisopropylethylamine (73.6 μL, 422 μmol) were added to the reaction mixture. The reaction mixture was stirred further 24 h at 50 °C and was then concentrated under vacuum. The crude product was purified by preparative reverse phase HPLC (eluent: acetonitrile / water 5 to 50 % + 0.1 % formic acid). The title compound containing fractions were concentrated under reduced pressure to provide 32.0 mg of the title compound.

LC-MS (Method 4): $R_t$ = 2.34 min; MS (ESIpos): m/z = 487 $[M+H]^+$

$^1$H-NMR (400 MHz, DMSO-$d_6$) δ [ppm] = 11.92 (s, 1H), 10.62 (s, 1H), 8.09 - 7.94 (m, 2H), 7.36 (dd, 3H), 7.24 - 7.04 (m, 5H), 6.86 (s, 1H), 6.61 (dd, 1H), 3.68 (s, 2H), 2.82 (s, 2H), 1.28 (s, 6H).

**Example 85**

2-(4-fluorophenyl)-N-{4-[3-(3-fluorophenyl)-6,6-dimethyl-4-oxo-4,5,6,7-tetrahydro-1H-pyrrolo[3,2-c]pyridin-2-yl]pyridin-2-yl}acetamide

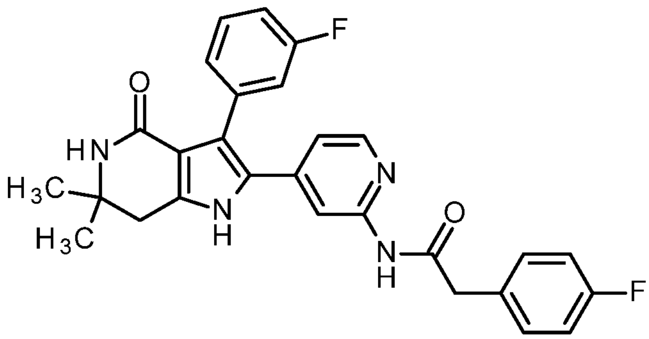

To a solution of 2-(2-aminopyridin-4-yl)-3-(3-fluorophenyl)-6,6-dimethyl-1,5,6,7-tetrahydro-4H-pyrrolo[3,2-c]pyridin-4-one formic acid salt (see Intermediate 81, 47.0 mg), 2-(4-fluorophenyl)acetic acid (41.4 mg, 268 μmol) and PyBop (140 mg, 268 μmol) in 1.34 mL of N,N-dimethylformamide was added diisopropylethylamine (70.2 μL, 402 μmol). The reaction was stirred at 50 °C for 36 h. To the reaction was 2-(4-fluorophenyl)acetyl chloride (120 mg, 695 μmol and diisopropylethylamine (46.8 μL, 268 μmol). The reaction was stirred another 24 h at rt. The reaction mixture was concentrated, and the crude product was purified by preparative reverse phase HPLC (eluent: acetonitrile / water 5 to 48 % + 0.1 % formic acid) The title compound containing fractions were concentrated under reduced pressure to provide 29.0 mg of the title compound.

LC-MS (Method 4): Rt = 2.37 min; MS (ESIpos): m/z = 487 [M+H]+

$^1$H-NMR (400 MHz, DMSO-$d_6$) δ [ppm] = 11.87 (s, 1H), 10.65 (s, 1H), 8.06 (d, 1H), 8.01 (s, 1H), 7.40 - 7.32 (m, 2H), 7.31 - 7.12 (m, 4H), 7.09 - 7.02 (m, 2H), 6.99 (d, 1H), 6.90 (s, 1H), 6.61 (dd, 1H), 3.67 (s, 2H), 2.80 (s, 2H), 1.27 (s, 6H).

**Example 86**

N-{4-[6,6-dimethyl-3-(3-methylphenyl)-4-oxo-4,5,6,7-tetrahydro-1H-pyrrolo[3,2-c]pyridin-2-yl]pyridin-2-yl}-2-(4-fluorophenyl)acetamide

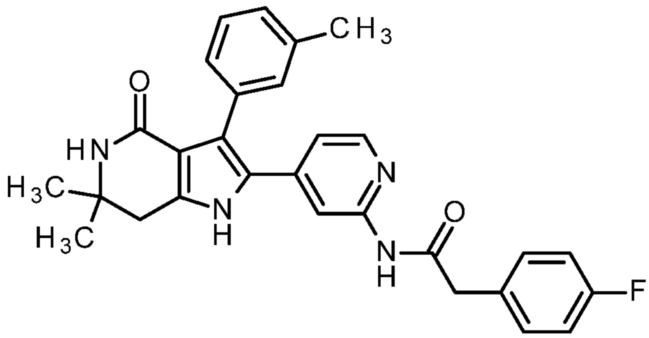

2-(2-Aminopyridin-4-yl)-6,6-dimethyl-3-(3-methylphenyl)-1,5,6,7-tetrahydro-4H-pyrrolo[3,2-c]-pyridin-4-one formic acid salt (see Intermediate 87, 70.0 mg) and diisopropylethylamine (176 µL, 1.01 mmol,) were dissolved in 2.02 mL of *N,N*-dimethylformamide. The mixture was cooled down to 0 °C, followed by the addition of 2-(4-fluorophenyl)acetyl chloride (139 mg, 808 µmol). The mixture was allowed to warm up to rt and stirred under nitrogen atmosphere for 24 h. The reaction mixture was concentrated, and the crude product was purified by flash chromatography on silica gel (dichloromethane / methanol 10% to 50%). The title compound containing fractions were concentrated under reduced pressure and purified by preparative reverse phase HPLC (eluent: acetonitrile / water 10 to 50% + 0.1% formic acid). The title compound containing fractions were concentrated under reduced pressure to provide 19.5 mg of the title compound.

LC-MS (Method 4): Rt = 2.45 min; MS (ESIpos): m/z = 483 [M+H]$^+$

$^1$H-NMR (400 MHz, DMSO-$d_6$) δ [ppm] = 11.77 (s, 1H), 10.62 (s, 1H), 8.05 (d, 1H), 7.99 (d, 1H), 7.36 (dd, 2H), 7.20 - 7.09 (m, 3H), 7.08 - 7.01 (m, 2H), 6.98 (d, 1H), 6.81 (s, 1H), 6.51 (dd, 1H), 3.68 (s, 2H), 2.79 (s, 2H), 2.23 (s, 3H), 1.27 (s, 6H).

**Example 87**

2-(4-fluorophenyl)-N-{4-[3-(2-fluorophenyl)-6,6-dimethyl-4-oxo-4,5,6,7-tetrahydro-1H-pyrrolo[3,2-c]pyridin-2-yl]pyridin-2-yl}propanamide

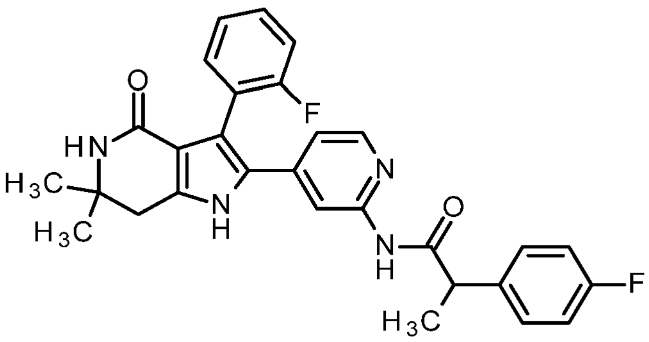

A screw cap vial was charged with 2-(2-aminopyridin-4-yl)-3-(2-fluorophenyl)-6,6-dimethyl-1,5,6,7-tetrahydro-4H-pyrrolo[3,2-c]pyridin-4-one formic acid salt (see Intermediate 84, 44.5 mg), 2-(4-fluorophenyl)acetic acid (64.1 mg, 381 µmol), PyBop (198 mg, 381 µmol). The vial was filled with 1.30 mL of *N,N*-dimethylacetamide followed by the addition of diisopropylethylamine (111 µL, 635 µmol).The reaction was stirred at 70 °C for 36 h. The reaction was cooled down concentrated under vacuum. The crude product was purified by preparative reverse phase HPLC (eluent: acetonitrile / water 20 to 50 % + 0.1 % formic acid). The title compound containing fractions were concentrated under reduced pressure to provide 11.0 mg of the title compound.

LC-MS (Method 4): $R_t$ = 2.49 min; MS (ESIpos): m/z = 501 $[M+H]^+$

$^1$H-NMR (400 MHz, DMSO-$d_6$) δ [ppm] = 11.93 (s, 1H), 10.56 (s, 1H), 8.06 - 7.87 (m, 2H), 7.40 (dd, 2H), 7.36 - 7.28 (m, 1H), 7.25 - 7.06 (m, 5H), 6.87 (s, 1H), 6.58 (dd, 1H), 3.99 (q, 1H), 2.82 (s, 2H), 1.36 (d, 3H), 1.27 (s, 6H).

## Example 88

N-{4-[6,6-dimethyl-3-(3-methylphenyl)-4-oxo-4,5,6,7-tetrahydro-1H-pyrrolo[3,2-c]pyridin-2-yl]pyridin-2-yl}-2-(4-fluorophenyl)propanamide (Racemate)

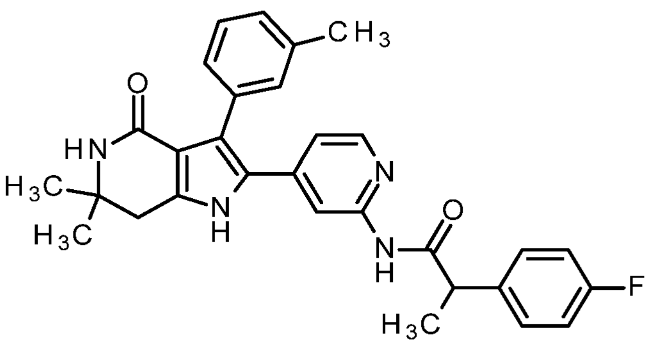

To a solution 2-(2-aminopyridin-4-yl)-6,6-dimethyl-3-(3-methylphenyl)-1,5,6,7-tetrahydro-4H-pyrrolo[3,2-c]pyridin-4-one formic acid salt (see Intermediate 87, 75.1 mg), 2-(4-fluorophenyl)propanoic acid (109 mg, 651 µmol) and PyBop (339 mg, 651 µmol) in 2.17 mL of *N,N*-dimethylacetamide was added diisopropylethylamine (189 µL, 1.08 mmol).The reaction was stirred at 70 °C for 36 h. The reaction was cooled down to rt concentrated under reduced pressure and the crude product was purified by preparative reverse phase HPLC (eluent: (acetonitrile / water 20 to 50 % + 0.1% formic acid) The title compound containing fractions were concentrated under reduced pressure to provide 45.0 mg of the title compound.

LC-MS (Method 4): Rt = 2.56 min; MS (ESIpos): m/z = 497 $[M+H]^+$

$^1$H-NMR (400 MHz, DMSO-$d_6$) δ [ppm] = 11.77 (s, 1H), 10.57 (s, 1H), 8.07 (d, 1H), 7.96 (d, 1H), 7.52 - 7.34 (m, 2H), 7.23 - 7.09 (m, 3H), 7.07 - 7.00 (m, 2H), 6.98 (d, 1H), 6.81 (s, 1H), 6.50 (dd, 1H), 4.01 (q, 1H), 2.80 (s, 2H), 2.23 (s, 3H), 1.38 (d, 3H), 1.27 (s, 6H).

**Example 89**

N-{4-[3-(4-chlorophenyl)-6,6-dimethyl-4-oxo-4,5,6,7-tetrahydro-1H-pyrrolo[3,2-c]pyridin-2-yl]pyridin-2-yl}-2-(4-fluorophenyl)propanamide (Racemate)

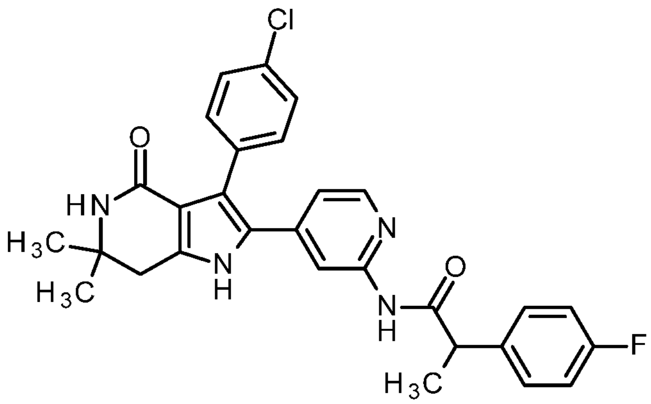

2-(2-Aminopyridin-4-yl)-3-(4-chlorophenyl)-6,6-dimethyl-1,5,6,7-tetrahydro-4H-pyrrolo[3,2-c]-pyridin-4-one formic acid salt (see Intermediate 90, 60.0 mg) and diisopropylethylamine (85.5 µL, 491 µmol) were dissolved in 1.68 mL of *N,N*-dimethylformamide. The mixture was cooled down to 0 °C, followed by the addition of 2-(4-fluorophenyl)propanoyl chloride (Racemate) (61.0 mg, 327 µmol). The mixture was allowed to warm up to rt and stirred under nitrogen atmosphere for 72 h. The reaction mixture was concentrated, and the crude product was purified by flash chromatography on silica gel (dichloromethane / methanol 2% to 15%). The title compound containing fractions were concentrated under reduced pressure. The product was purified by preparative reverse phase HPLC (eluent: acetonitrile / water 50 to 60 % + 0.1% formic acid). The title compound containing fractions were concentrated under reduced pressure to provide 8.91 mg of the title compound.

LC-MS (Method 4): Rt = 2.69 min; MS (ESIpos): m/z = 517 $[M+H]^+$

$^1$H-NMR (400 MHz, DMSO-$d_6$) δ [ppm] = 11.85 (s, 1H), 10.56 (s, 1H), 8.11 -7.96 (m, 2H), 7.41 (t, 2H), 7.34 - 7.27 (m, 2H), 7.25 - 7.21 (m, 2H), 7.16 (t, 2H), 6.89 (s, 1H), 6.62 (d, 1H), 4.07 - 3.95 (m, 1H), 2.81 (s, 2H), 1.37 (d, 3H), 1.28 (s, 6H).

**Example 90**

N-{4-[3-(4-chlorophenyl)-6,6-dimethyl-4-oxo-4,5,6,7-tetrahydro-1H-pyrrolo[3,2-c]pyridin-2-yl]pyridin-2-yl}-2-(4-fluorophenyl)acetamide

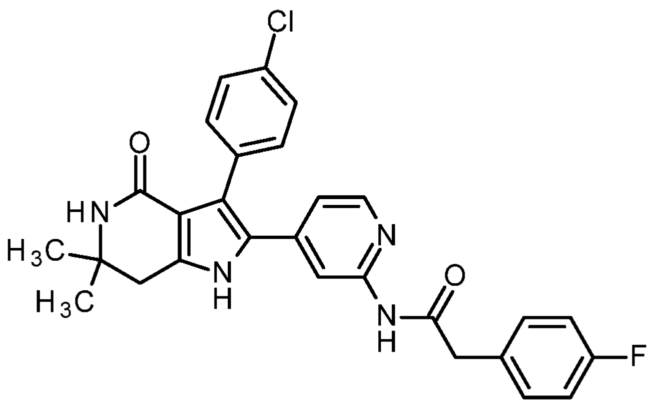

2-(2-Aminopyridin-4-yl)-3-(4-chlorophenyl)-6,6-dimethyl-1,5,6,7-tetrahydro-4H-pyrrolo[3,2-c]-pyridin-4-one formic acid salt (see Intermediate 90, 94.0 mg) and diisopropylethylamine (134 μL, 769 μmol) were dissolved in 2.63 mL of *N,N*-dimethylformamide. The mixture was cooled down to 0 °C, followed by the addition of 2-(4-fluorophenyl)acetyl chloride (88.4 mg, 512 μmol). The mixture was allowed to warm up to rt and stirred under nitrogen atmosphere for 72 h. The reaction mixture was concentrated, and the crude product was purified by flash chromatography on silica gel (dichloromethane / methanol 2% to 20%). The title compound containing fractions were concentrated under reduced pressure. The product was purified by preparative reverse phase HPLC (eluent: acetonitrile / water 50 to 60 % + 0.1% formic acid). The title compound containing fractions were concentrated under reduced pressure to provide 3.9 mg of the title compound.

LC-MS (Method 4): $R_t$ = 2.55 min; MS (ESIpos): m/z = 503 $[M+H]^+$

$^1$H-NMR (400 MHz, DMSO-$d_6$) δ [ppm] = 11.84 (s, 1H), 10.62 (s, 1H), 8.07 (d, 1H), 8.02 (s, 1H), 7.35 (dd, 2H), 7.30 (d, 2H), 7.22 (d, 2H), 7.15 (t, 2H), 6.87 (s, 1H), 6.61 (dd, 1H), 3.68 (s, 2H), 2.79 (s, 2H), 1.27 (s, 6H).

## Example 91

N-{4-[6,6-dimethyl-3-(4-methylphenyl)-4-oxo-4,5,6,7-tetrahydro-1H-pyrrolo[3,2-c]pyridin-2-yl]pyridin-2-yl}-2-(4-fluorophenyl)propanamide (Racemate)

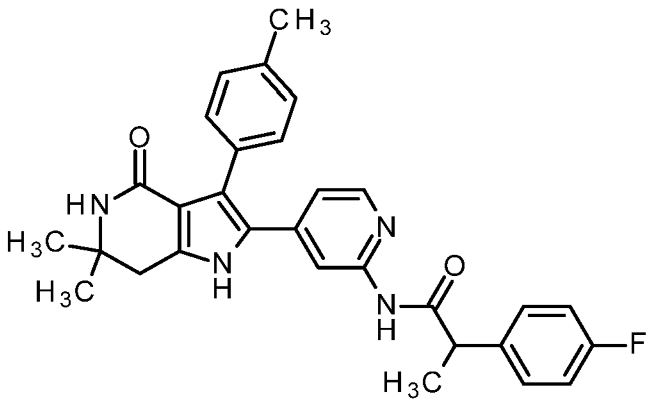

2-(2-Aminopyridin-4-yl)-6,6-dimethyl-3-(4-methylphenyl)-1,5,6,7-tetrahydro-4H-pyrrolo[3,2-c]-pyridin-4-one formic acid salt (see Intermediate 93, 65.0 mg) and diisopropylethylamine (98.1 μL, 563 μmol) were dissolved in 1.82 mL of *N,N*-dimethylformamide. The mixture was cooled down to 0 °C, followed by the addition of 2-(4-fluorophenyl)propanoyl chloride (Racemate) (70.0 mg, 375 μmol). The mixture was allowed to warm up to rt and stirred under nitrogen atmosphere for 72 h. The reaction mixture was concentrated and the crude product was purified by flash chromatography on silica gel (dichloromethane / methanol 2% to 15%).The title compound containing fractions were concentrated under reduced pressure and purified by preparative reverse phase HPLC (eluent: acetonitrile /water 50 to 60% + 0.1% formic acid). The title compound containing fractions were concentrated under reduced pressure to provide 16.0 mg of the title compound.

LC-MS (Method 4): Rt = 2.60 min; MS (ESIpos): m/z = 497 $[M+H]^+$

$^1$H-NMR (400 MHz, DMSO-$d_6$) δ [ppm] = 11.75 (s, 1H), 10.56 (s, 1H), 8.08 (s, 1H), 7.98 (d, 1H), 7.43 (dd, 2H), 7.20 - 7.01 (m, 6H), 6.81 (s, 1H), 6.53 (dd, 1H), 4.02 (q, 1H), 2.80 (s, 2H), 2.31 (s, 3H), 1.39 (d, 3H), 1.28 (s, 6H)

## Example 92

N-{4-[6,6-dimethyl-3-(4-methylphenyl)-4-oxo-4,5,6,7-tetrahydro-1H-pyrrolo[3,2-c]pyridin-2-yl]pyridin-2-yl}-2-(4-fluorophenyl)acetamide

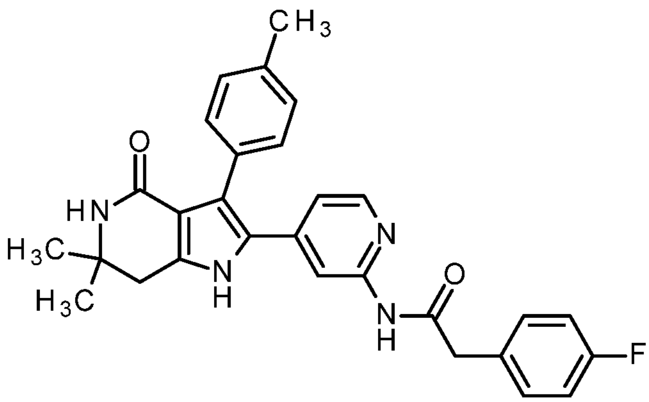

2-(2-Aminopyridin-4-yl)-6,6-dimethyl-3-(4-methylphenyl)-1,5,6,7-tetrahydro-4H-pyrrolo[3,2-c]-pyridin-4-one (see Intermediate 93, 79.0 mg) formic acid salt and diisopropylethylamine (120 µL, 687 µmol) were dissolved in 2.35 mL of N,N-dimethylformamide. The mixture was cooled down to 0 °C, followed by the addition of 2-(4-fluorophenyl)acetyl chloride (79.3 mg, 458 µmol). The mixture was allowed to warm up to rt and stirred under nitrogen atmosphere for 72 h. The reaction mixture was concentrated and the crude product was purified by flash chromatography on silica gel (High performance, RediSep Tf gold, dichloromethane / methanol 2% to 20%). The title compound containing fractions were concentrated under reduced pressure and purified by preparative reverse phase HPLC (eluent: acetonitrile / water 50 to 60% + 0.1% Formic acid). The title compound containing fractions were concentrated under reduced pressure to provide 6.90 mg of the title compound.

LC-MS (Method 4): $R_t$ = 2.45 min; MS (ESIpos): m/z = 483 $[M+H]^+$

$^1$H-NMR (400 MHz, DMSO-$d_6$) δ [ppm] = 11.73 (s, 1H), 10.60 (s, 1H), 8.05 (s, 1H), 7.99 (d, 1H), 7.36 (dd, 2H), 7.15 (t, 2H), 7.12 - 7.01 (m, 4H), 6.80 (s, 1H), 6.53 (d, 1H), 3.69 (s, 2H), 2.78 (s, 2H), 2.30 (s, 3H), 1.26 (s, 6H).

## Example 93

N-{4-[6,6-dimethyl-4-oxo-3-(pyridin-3-yl)-4,5,6,7-tetrahydro-1H-pyrrolo[3,2-c]pyridin-2-yl]pyridin-2-yl}-2-(4-fluorophenyl)acetamide

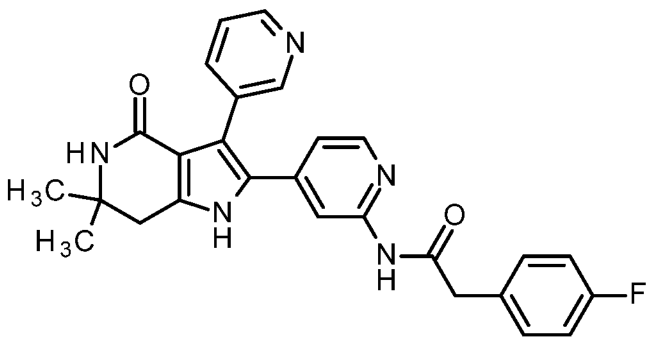

A screw cap vial was charged with N-[4-(3-bromo-6,6-dimethyl-4-oxo-4,5,6,7-tetrahydro-1H-pyrrolo[3,2-c]pyridin-2-yl)pyridin-2-yl]-2-(4-fluorophenyl)acetamide (see Intermediate 96, 30.0 mg), 3-pyridylboronic acid (14.9 mg, 121 µmol), XPhos Pd G3 (CAS RN: [1445085-55-1], 5.12 mg, 6.05 µmol), and $K_3PO_4$ (32.1 mg, 151 µmol). The vial was degassed with argon and charged with 300 µL of 1,4-dioxane / water 4:1. The reaction was heated at 80 °C. After 20 h the reaction was cooled down and concentrated under vacuum. The residue was purified by preparative reverse phase HPLC (eluent: acetonitrile / water 10 to 100% + 0.1% formic acid). The title compound containing fractions were concentrated under reduced pressure to provide 20.5 mg of the title compound.

LC-MS (Method 4): Rt = 1.72 min; MS (ESIpos): m/z = 470 $[M+H]^+$
$^1$H-NMR (400 MHz, DMSO-$d_6$) δ [ppm] = 11.93 (s, 1H), 10.64 (s, 1H), 8.40 (dd, 1H), 8.35 (d, 1H), 8.08 (d, 1H), 8.00

(s, 1H), 7.65 (d, 1H), 7.34 (dd, 2H), 7.29 (dd, 1H), 7.15 (t, 2H), 6.93 (s, 1H), 6.63 (dd, 1H), 3.67 (s, 2H), 2.81 (s, 2H), 1.28 (s, 6H).

**Example 94**

2-(4-fluorophenyl)-N-{4-[3-(4-fluorophenyl)-6,6-dimethyl-4-oxo-4,5,6,7-tetrahydro-1H-pyrrolo[3,2-c]pyridin-2-yl]pyridin-2-yl}propanamide (Racemate)

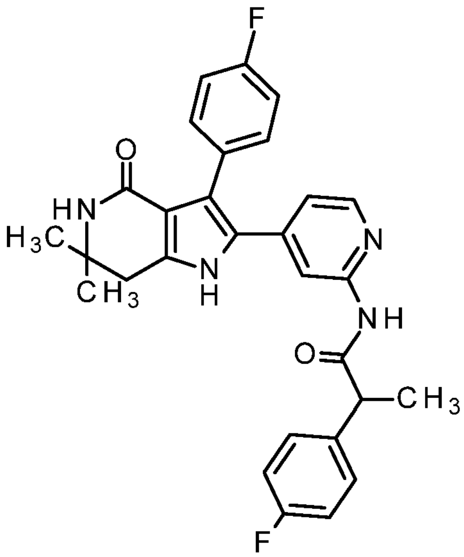

A screw cap vial was charged with N-[4-(3-bromo-6,6-dimethyl-4-oxo-4,5,6,7-tetrahydro-1H-pyrrolo[3,2-c]pyridin-2-yl)pyridin-2-yl]-2-(4-fluorophenyl)propanamide (Racemate) (see Intermediate 98, 60.0 mg), (4-fluorophenyl)boronic acid (34.6 mg, 247 μmol), XPhos Pd G3 (CAS-RN:[1445085-55-1], 10.5 mg, 12.4 μmol), and $K_3PO_4$ (65.5 mg, 309 μmol). The vial was degassed with argon and charged with 640 μL of 1,4-dioxane / water 4:1. The reaction was heated at 80 °C. After 16 h the reaction was cooled down and concentrated under vacuum. The residue was purified by preparative reverse phase HPLC (eluent: acetonitrile / water 40 to 50% + 0.1% formic acid). The title compound containing fractions were concentrated under reduced pressure to provide 42.0 mg of the title compound.

LC-MS (Method 4): Rt = 2.55 min; MS (ESIpos): m/z = 501 $[M+H]^+$

$^1$H-NMR (400 MHz, DMSO-$d_6$) δ [ppm] = 11.81 (s, 1H), 10.54 (s, 1H), 8.06 - 7.99 (m, 2H), 7.40 (dd, 2H), 7.26 - 7.20 (m, 2H), 7.15 (t, 2H), 7.07 (t, 2H), 6.85 (s, 1H), 6.59 (dd, 1.6 Hz, 1H), 3.99 (q, 1H), 2.80 (s, 2H), 1.36 (d, 3H), 1.28 (s, 6H).

**Example 95**

2-(4-fluorophenyl)-N-{4-[3-(4-fluorophenyl)-6,6-dimethyl-4-oxo-4,5,6,7-tetrahydro-1H-pyrrolo[3,2-c]pyridin-2-yl]pyridin-2-yl}propanamide (Enantiomer 1)

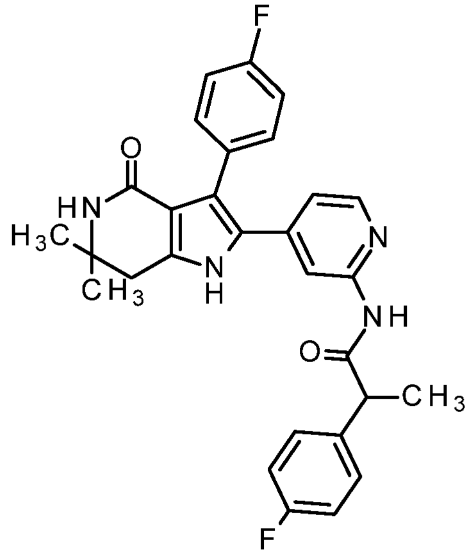

The racemic compound (see Example 94, 82 mg) was separated into enantiomers by preparative chiral HPLC to give enantiomer 1 (27 mg, >99 %ee, see Example 95) and enantiomer 2 (22 mg, >99 %ee, see Example 96).

Preparative chiral HPLC method:
Instrument: PrepCon Labomatic HPLC-3; Column: YMC Cellulose SB 5μ, 250x30; eluent A: hexane + 0.1 vol % diethylamine; eluent B: ethanol; isocratic: 70%A+30%B; flow: 40 mL/min; temperature: 25°C; UV: 254 nm

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 70%A+30%B; flow: 1.4 mL/min; temperature: 25°C; UV: 254 nm

Analytical Chiral HPLC (method Example 95): $R_t$ = 1.21 min.

LC-MS (Method 1): $R_t$ = 1.11 min; MS (ESIneg): m/z = 499 [M-H]-

$^1$H-NMR (400 MHz, DMSO-$d_6$) δ [ppm]: 0.830 (0.69), 0.840 (0.49), 0.852 (0.50), 0.858 (1.21), 0.953 (0.42), 1.237 (0.93), 1.272 (16.00), 1.353 (4.97), 1.371 (4.99), 1.394 (0.49), 2.522 (2.39), 2.539 (3.52), 2.803 (4.99), 3.984 (1.07), 4.002 (1.07), 6.570 (1.57), 6.575 (1.50), 6.584 (1.46), 6.588 (1.59), 6.889 (2.63), 7.049 (1.61), 7.071 (3.45), 7.094 (2.10), 7.132 (1.78), 7.155 (3.75), 7.177 (2.08), 7.207 (1.98), 7.212 (0.92), 7.221 (2.32), 7.228 (2.02), 7.237 (0.77), 7.243 (1.64), 7.381 (1.90), 7.386 (0.89), 7.395 (2.14), 7.402 (1.95), 7.417 (1.65), 8.019 (4.75), 8.033 (2.22), 10.574 (2.46), 11.826 (1.74).

**Example 96**

2-(4-fluorophenyl)-N-{4-[3-(4-fluorophenyl)-6,6-dimethyl-4-oxo-4,5,6,7-tetrahydro-1H-pyrrolo[3,2-c]pyridin-2-yl]pyridin-2-yl}propanamide (Enantiomer 2)

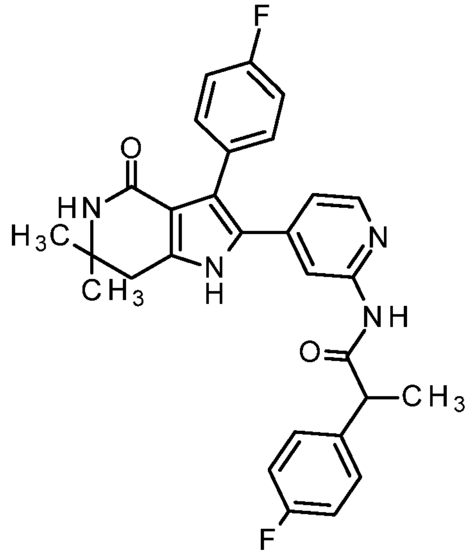

For the preparation of the racemic title compound see Example 94. Separation of enantiomers by preparative chiral HPLC (method see Example 95) gave the title compound (22 mg, >99 %ee).

Analytical Chiral HPLC (method see Example 95): Rt = 2.72 min.

LC-MS (Method 1): Rt = 1.10 min; MS (ESIneg): m/z = 499 [M-H]-

$^1$H-NMR (400 MHz, DMSO-$d_6$) δ [ppm]: 0.814 (0.50), 0.831 (1.10), 0.836 (0.57), 0.841 (0.72), 0.853 (0.81), 0.858 (1.69), 0.874 (0.57), 0.936 (0.41), 0.953 (0.44), 1.244 (1.34), 1.273 (16.00), 1.354 (4.90), 1.371 (4.84), 1.395 (0.45), 2.803 (5.00), 3.984 (1.09), 4.002 (1.06), 6.571 (1.53), 6.575 (1.41), 6.584 (1.40), 6.588 (1.48), 6.887 (2.65), 7.049 (1.53), 7.071 (3.32), 7.093 (1.94), 7.133 (1.68), 7.155 (3.52), 7.177 (1.98), 7.207 (1.96), 7.212 (0.98), 7.221 (2.25), 7.228 (1.93), 7.243 (1.52), 7.381 (1.88), 7.386 (0.95), 7.395 (2.13), 7.402 (1.91), 7.416 (1.57), 8.019 (4.62), 8.033 (2.15), 10.572 (2.46), 11.825 (1.83).

## Example 97

N-{4-[6,6-dimethyl-4-oxo-3-(pyridin-3-yl)-4,5,6,7-tetrahydro-1H-pyrrolo[3,2-c]pyridin-2-yl]pyridin-2-yl}-2-(4-fluorophenyl)propanamide (Racemate)

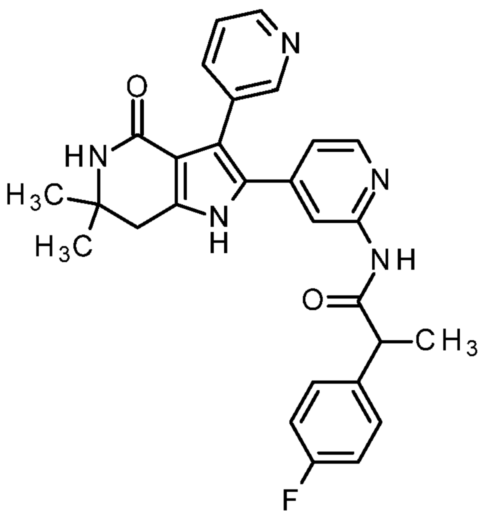

A screw cap vial was charged with N-[4-(3-bromo-6,6-dimethyl-4-oxo-4,5,6,7-tetrahydro-1H-pyrrolo[3,2-c]pyridin-2-yl)pyridin-2-yl]-2-(4-fluorophenyl)propanamide (Racemate) (see Intermediate 98, 60.0 mg, 124 µmol), 3-pyridylboronic acid (30.4 mg, 247 µmol), XPhos Pd G3 (CAS-RN:[1445085-55-1], 10.5 mg, 12.4 µmol), and $K_3PO_4$ (65.5 mg, 309 µmol). The vial was degassed with argon and charged with 640 µL of 1,4-dioxane / water 4:1. The reaction was heated at 80 °C. After 16 h the reaction was cooled down and concentrated under vacuum. The residue was purified by flash chromatography on silica gel (dichloromethane / methanol 0% to 15%). The title compound containing fractions were concentrated under reduced pressure to provide 32.1 mg of the title compound.

LC-MS (Method 4): Rt = 1.20 min; MS (ESIpos): m/z = 484 $[M+H]^+$

$^1$H-NMR (400 MHz, DMSO-$d_6$) δ [ppm] = 11.93 (s, 1H), 10.59 (s, 1H), 8.42 (dd, 1H), 8.35 (d, 1H), 8.08 - 8.00 (m, 2H), 7.67 - 7.61 (m, 1H), 7.44 - 7.35 (m, 2H), 7.29 (dd, 1H), 7.16 (t, 2H), 6.93 (s, 1H), 6.60 (dd, 1H), 4.00 (q, 1H), 2.82 (s, 2H), 1.36 (d, 3H), 1.29 (s, 6H).

**Example 98**

N-{4-[6,6-dimethyl-4-oxo-3-(pyridin-3-yl)-4,5,6,7-tetrahydro-1H-pyrrolo[3,2-c]pyridin-2-yl]pyridin-2-yl}-2-(4-fluorophenyl)propanamide (Enantiomer 1)

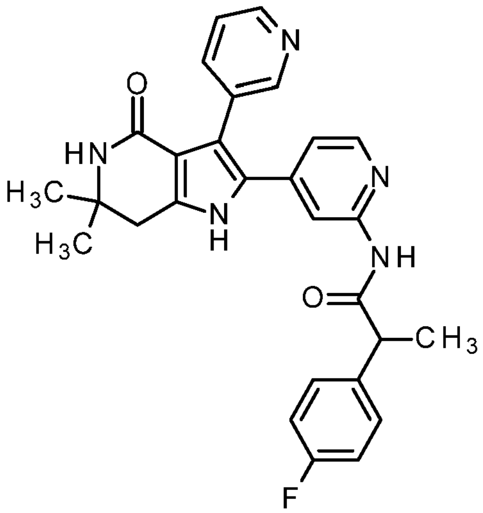

The racemic compound (see Example 97, 48 mg) was separated into enantiomers by preparative chiral HPLC to give enantiomer 1 (9.5 mg, >99 %ee, see Example 98) and enantiomer 2 (9.0 mg, >99 % ee, see Example 99).

Preparative chiral HPLC method:
Instrument: PrepCon Labomatic HPLC; Column: YMC Cellulose SB 5µ, 250x30; eluent A: hexane + 0.1 vol % diethylamine; eluent B: ethanol; isocratic: 60%A+40%B; flow: 40 mL/min; temperature: 25°C; UV: 254 nm

Analytical chiral HPLC method:
Instrument: Waters Alliance 2695; Column: YMC Cellulose SB 3µ, 100x4.6; eluent A: hexane + 0.1 vol % diethylamine; eluent B: ethanol; isocratic: 60%A+40%B; flow: 1.4 mL/min; temperature: 25°C; UV: 254 nm

Analytical Chiral HPLC (method Example 98): Rt = 1.49min.

LC-MS (Method 2): Rt = 1.03 min; MS (ESIneg): m/z = 482 [M-H]-

$^1$H-NMR (400 MHz, DMSO-$d_6$) δ [ppm]: 1.237 (0.43), 1.282 (16.00), 1.352 (5.20), 1.370 (5.16), 2.518 (2.33), 2.522 (1.49), 2.673 (0.40), 2.822 (5.15), 3.988 (1.10), 4.006 (1.08), 6.586 (1.73), 6.590 (1.62), 6.599 (1.63), 6.603 (1.66), 6.965 (2.68), 7.142 (1.91), 7.147 (0.67), 7.158 (0.82), 7.164 (4.05), 7.169 (0.85), 7.181 (0.72), 7.186 (2.19), 7.271 (0.98), 7.273 (1.01), 7.283 (1.02), 7.285 (1.05), 7.291 (1.08), 7.293 (1.06), 7.303 (1.09), 7.305 (1.08), 7.379 (1.98), 7.384 (0.89), 7.393 (2.19), 7.400 (1.98), 7.409 (0.76), 7.414 (1.70), 7.627 (0.97), 7.631 (1.44), 7.636 (1.08), 7.646

(0.86), 7.651 (1.30), 7.656 (0.91), 8.036 (2.34), 8.047 (2.55), 8.060 (2.22), 8.342 (1.99), 8.347 (2.18), 8.349 (2.02), 8.408 (1.68), 8.412 (1.76), 8.420 (1.69), 8.424 (1.62), 10.615 (2.53), 11.954 (1.73).

## Example 99

N-{4-[6,6-dimethyl-4-oxo-3-(pyridin-3-yl)-4,5,6,7-tetrahydro-1H-pyrrolo[3,2-c]pyridin-2-yl]pyridin-2-yl}-2-(4-fluorophenyl)propanamide (Enantiomer 2)

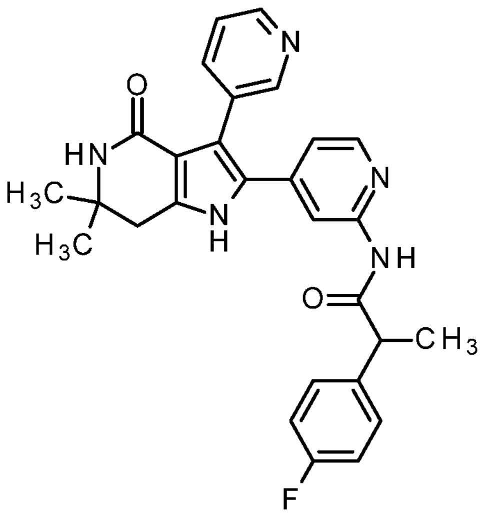

For the preparation of the racemic title compound see Example 97. Separation of enantiomers by preparative chiral HPLC (method see Example 98) gave the title compound (9 mg, > 99 %ee).

Analytical Chiral HPLC (method see Example 98): $R_t$ = 2.01 min.

LC-MS (Method 2): $R_t$ = 1.03 min; MS (ESIneg): m/z = 482 [M-H]-

$^1$H-NMR (400 MHz, DMSO-$d_6$) δ [ppm]: 0.831 (0.52), 0.854 (0.43), 0.858 (0.55), 1.238 (0.65), 1.283 (16.00), 1.353 (5.27), 1.371 (5.11), 1.395 (0.57), 2.332 (0.95), 2.336 (0.43), 2.518 (5.84), 2.523 (3.82), 2.673 (0.95), 2.678 (0.44), 2.822 (5.06), 3.989 (1.08), 4.006 (1.06), 5.759 (0.55), 6.586 (1.76), 6.590 (1.71), 6.599 (1.62), 6.603 (1.73), 6.964 (2.63), 7.142 (1.96), 7.147 (0.66), 7.159 (0.78), 7.164 (4.11), 7.170 (0.79), 7.181 (0.71), 7.186 (2.23), 7.271 (0.96), 7.274 (1.00), 7.284 (0.98), 7.286 (1.03), 7.291 (1.08), 7.293 (1.07), 7.303 (1.08), 7.305 (1.06), 7.379 (2.00), 7.385 (0.88), 7.393 (2.22), 7.401 (1.96), 7.410 (0.73), 7.415 (1.73), 7.627 (1.02), 7.631 (1.47), 7.636 (1.09), 7.646 (0.89), 7.651 (1.29), 7.656 (0.93), 8.036 (2.29), 8.048 (2.43), 8.061 (2.16), 8.342 (1.98), 8.343 (2.08), 8.347 (2.12), 8.349 (1.96), 8.408 (1.70), 8.413 (1.72), 8.420 (1.70), 8.425 (1.57), 10.615 (2.49), 11.953 (1.80).

## Example 100

2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}propanamide (Racemate)

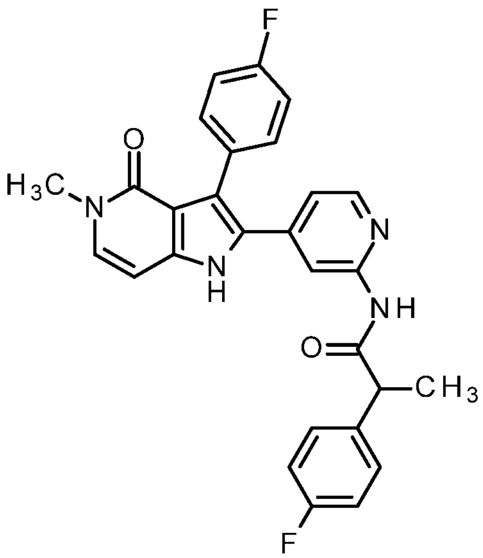

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 95.0 mg), (4-fluoro-phenyl)boronic acid (56.7 mg, 405 μmol), XPhos Pd G3 (CAS-RN:[1445085-55-1], 17.1 mg, 20.2 μmol), and $K_3PO_4$ (128 mg, 607 μmol). The vial was degassed with argon and charged with 1.01 mL of 1,4-dioxane / water 4:1. The reaction was heated at 90 °C. After 20 min the reaction was cooled down and concentrated under vacuum. The residue was purified by preparative reverse phase HPLC (eluent: acetonitrile / water 60 to 80% + 0.1% TFA). The title compound containing fractions were concentrated under reduced pressure to provide 96.1 mg of the title compound.

LC-MS (Method 4): Rt = 2.59 min; MS (ESIpos): m/z = 485 $[M+H]^+$

$^1$H-NMR (400 MHz, DMSO-$d_6$) δ [ppm] = 12.02 (s, 1H), 10.65 (s, 1H), 8.17 - 8.02 (m, 2H), 7.44 - 7.34 (m, 3H), 7.28 (dd, 2H), 7.13 (dt, 4H), 6.72 (dd, 1H), 6.43 (d, 1H), 4.01 (q, 1H), 3.39 (s, 3H), 1.38 (d, 3H).

**Example 101**

(-)-2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}propanamide (Enantiomer 1)

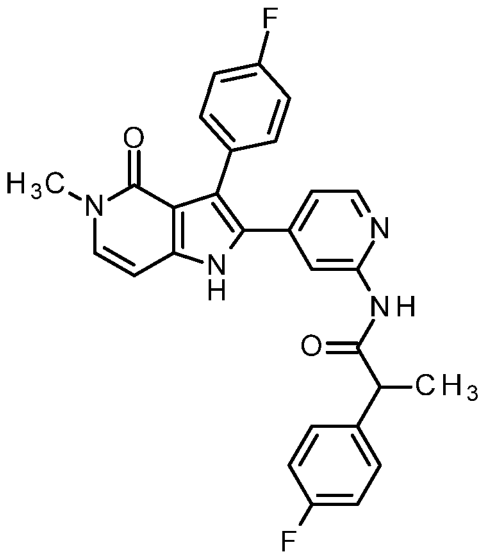

The racemic compound (see Example 100, 90 mg) was separated into enantiomers by preparative chiral HPLC to give enantiomer 1 (40.1 mg, >99 %ee, see Example 101) and enantiomer 2 (18.2 mg, >99 % ee, see Example 102).

Preparative chiral HPLC method:
Instrument: PrepCon Labomatic HPLC; Column: YMC Cellulose SB 10μ, 250x50; eluent A: methyl tert-butyl ether + 0.1 vol % diethylamine; eluent B: acetonitrile; isocratic: 90%A+10%B; flow: 140 mL/min; temperature: 25°C; UV: 254 nm
Analytical chiral HPLC method:
Instrument: Waters Alliance 2695; Column: YMC Cellulose SB 3μ, 100x4.6; eluent A: methyl tert-butyl ether + 0.1 vol % diethylamine; eluent B: acetonitrile; isocratic: 90%A+10%B; flow: 1.4 mL/min; temperature: 25°C; UV: 254 nm

Analytical Chiral HPLC (method Example 101): Rt = 2.08 min.

LC-MS (Method 2): $R_t$ = 1.15 min; MS (ESIpos): m/z = 485 $[M+H]^+$
$[\alpha]_D$ = -152,6° (from solution in DMSO, c = 2,43 mg/mL)
$^1$H-NMR (400 MHz, DMSO-$d_6$): δ [ppm] = 12.04 (s, 1H), 10.66 (s, 1H), 8.14 (d, 1H), 8.12-8.09 (m, 1H), 7.44-7.37 (m, 3H), 7.27 (dd, 2H), 7.19-7.06 (m, 4H), 6.71 (dd, 1H), 6.43 (d, 1H), 4.01 (q, 1H), 3.39 (s, 3H), 1.37 (d, 3H)

**Example 102**

(+)-2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}propanamide (Enantiomer 2)

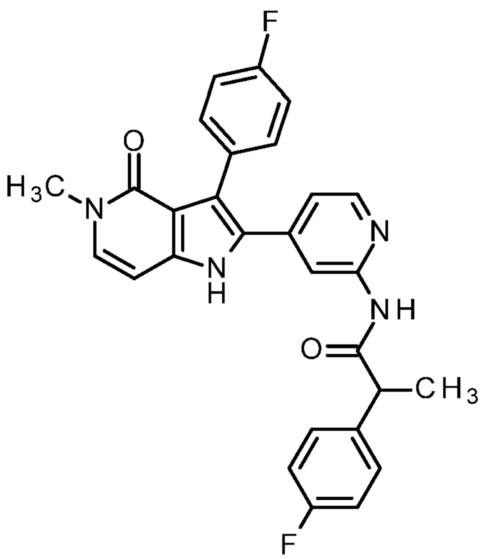

For the preparation of the racemic title compound see Example 100. Separation of enantiomers by preparative chiral HPLC (method see Example 101) gave the title compound (18.2 mg, >99 %ee).
Analytical Chiral HPLC (method see Example 101): Rt = 5.35 min.

LC-MS (Method 2): Rt = 1.15 min; MS (ESIpos): m/z = 485 $[M+H]^+$

$[\alpha]_D$ = +182,5 ° (from solution in DMSO, c = 2,49 mg/mL)

$^1$H-NMR (400 MHz, DMSO-$d_6$): δ [ppm] = 12.04 (s, 1H), 10.66 (s, 1H), 8.14 (d, 1H), 8.12-8.09 (m, 1H), 7.45-7.37 (m, 3H), 7.31-7.24 (m, 2H), 7.20-7.06 (m, 4H), 6.71 (dd, 1H), 6.43 (d, 1H), 4.01 (q, 1H), 3.39 (s, 3H), 1.37 (d, 3H)

**Example 103**

2-(4-fluorophenyl)-N-[4-(5-methyl-4-oxo-3-phenyl-4,5-dihydro-1H-pyrrolo[3,2-c]pyridin-2-yl)pyridin-2-yl]propanamide (Racemate)

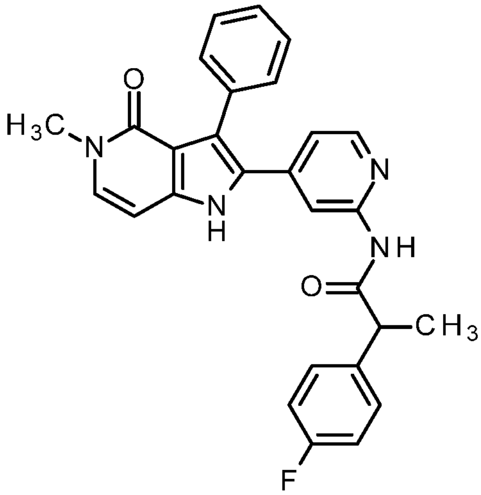

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 50.0 mg), phenylboronic acid (26.0 mg, 213 μmol), XPhos Pd G3 (CAS-RN:[1445085-55-1], 9.0 mg, 10.7 μmol), and $K_3PO_4$ (67.8 mg, 320 μmol). The vial was degassed with argon and charged with 530 μL of 1,4-dioxane / water 4:1. The reaction was heated at 90 °C. After 16 h the reaction was cooled down and concentrated under vacuum. The residue was purified by preparative reverse phase HPLC (eluent: acetonitrile / water 40 to 60% + 0.1% TFA). The title compound containing fractions were concentrated under reduced pressure to provide 45.0 mg of the title compound.

LC-MS (Method 4): Rt = 2.54 min; MS (ESIpos): m/z = 467 $[M+H]^+$

$^1$H-NMR (400 MHz, DMSO-$d_6$) δ [ppm] = 12.01 (s, 1H), 10.66 (s, 1H), 8.17 (s, 1H), 8.06 (d, 1H), 7.47 - 7.36 (m, 3H), 7.32 - 7.24 (m, 5H), 7.18 (t, 2H), 6.65 (dd, 1H), 6.44 (d, 1H), 4.03 (q, 1H), 3.40 (s, 3H), 1.40 (d, 3H).

**Example 104**

2-(4-fluorophenyl)-N-[4-(5-methyl-4-oxo-3-phenyl-4,5-dihydro-1H-pyrrolo[3,2-c]pyridin-2-yl)pyridin-2-yl]propanamide (Enantiomer 1)

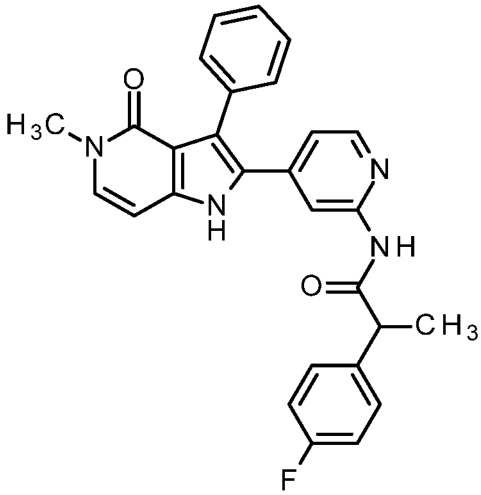

The racemic compound (see Example 103, 38 mg) was separated into enantiomers by preparative chiral HPLC to give enantiomer 1 (7.9 mg, >99 %ee, see Example 104) and enantiomer 2 (5.8 mg, >99 % ee, see Example 105).

Preparative chiral HPLC method:
Instrument: PrepCon Labomatic HPLC; Column: YMC Cellulose SB 10μ, 250x50; eluent A: hexane + 0.1 vol % diethylamine; eluent B: ethanol + 0.1 vol % diethylamine; isocratic: 80%A+20%B; flow: 120 mL/min; temperature: 25°C; UV: 254 nm

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 80%A+20%B; flow: 1.4 mL/min; temperature: 25°C; UV: 254 nm

Analytical Chiral HPLC (method Example 104): Rt = 1.89 min.

LC-MS (Method 2): Rt = 1.13 min; MS (ESIpos): m/z = 467 $[M+H]^+$

$^1$H-NMR (400 MHz, DMSO-$d_6$): δ [ppm] = 12.01 (s, 1H), 10.66 (s, 1H), 8.18 (d, 1H), 8.04 (d, 1H), 7.46-7.36 (m, 3H), 7.32-7.23 (m, 5H), 7.21-7.13 (m, 2H), 6.62 (dd, 1H), 6.43 (d, 1H), 4.02 (q, 1H), 3.39 (s, 3H), 1.38 (d, 3H)

**Example 105**

2-(4-fluorophenyl)-N-[4-(5-methyl-4-oxo-3-phenyl-4,5-dihydro-1H-pyrrolo[3,2-c]pyridin-2-yl)pyridin-2-yl]propanamide (Enantiomer 2)

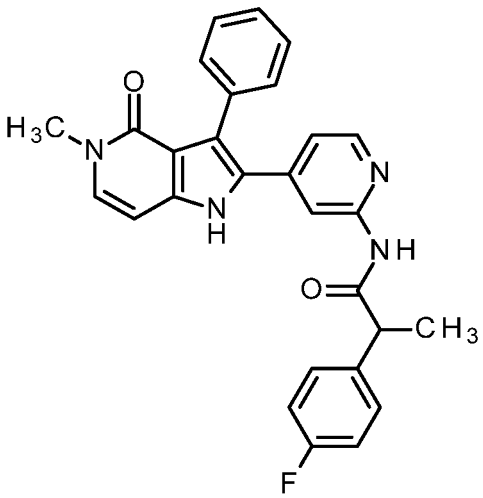

For the preparation of the racemic title compound see Example 103. Separation of enantiomers by preparative chiral HPLC (method see Example 104) gave the title compound (5.8 mg, >99 %ee).

Analytical Chiral HPLC (method see Example 104): Rt = 3.35 min.

LC-MS (Method 2): Rt = 1.13 min; MS (ESIpos): m/z = 467 $[M+H]^+$

$^1$H-NMR (400 MHz, DMSO-$d_6$): $\delta$ [ppm] = 12.02 (s, 1H), 10.66 (s, 1H), 8.18 (d, 1H), 8.04 (d, 1H), 7.47-7.35 (m, 3H), 7.31-7.22 (m, 5H), 7.21-7.13 (m, 2H), 6.62 (dd, 1H), 6.43 (d, 1H), 4.02 (q, 1H), 3.39 (s, 3H), 1.38 (d, 3H)

## Example 106

N-{4-[3-(3-chlorophenyl)-5-methyl-4-oxo-4,5-dihydro-1H-pyrrolo[3,2-c]pyridin-2-yl]pyridin-2-yl}-2-(4-fluorophenyl)propanamide (Racemate)

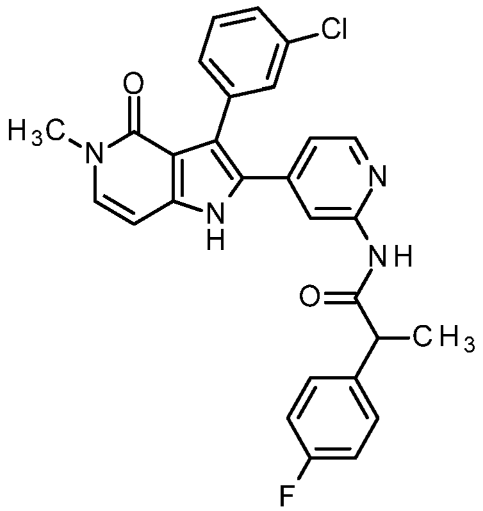

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 20.0 mg), (3-chlorophenyl)boronic acid (8.7 mg, 55.4 μmol), XPhos Pd G3 (CAS-RN:[1445085-55-1], 3.6 mg, 4.26 μmol) and $K_3PO_4$ (27.1 mg, 128 μmol). The vial was degassed with argon and charged with 210 μL of 1,4-dioxane / water 4:1. The reaction was heated at 80 °C. After 1 h the reaction was cooled down and concentrated under vacuum. The residue was purified by preparative reverse phase HPLC (eluent: acetonitrile / water 50 to 70% + 0.1% TFA). The title compound containing fractions were concentrated under reduced pressure to provide 10.4 mg of the title compound.

LC-MS (Method 4): Rt = 2.72 min; MS (ESIpos): m/z = 501 $[M+H]^+$

$^1$H-NMR (400 MHz, DMSO-$d_6$) δ [ppm] = 12.09 (s, 1H), 10.67 (s, 1H), 8.16 (d, 1H), 8.12 (d, 1H), 7.45 - 7.37 (m, 3H), 7.37 - 7.31 (m, 2H), 7.31 - 7.25 (m, 1H), 7.21 - 7.13 (m, 3H), 6.71 (dd, 1H), 6.44 (d, 1H), 4.02 (q, 1H), 3.40 (s, 3H), 1.39 (d, 3H).

**Example 107**

4,4-difluoro-2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}butanamide (Racemate)

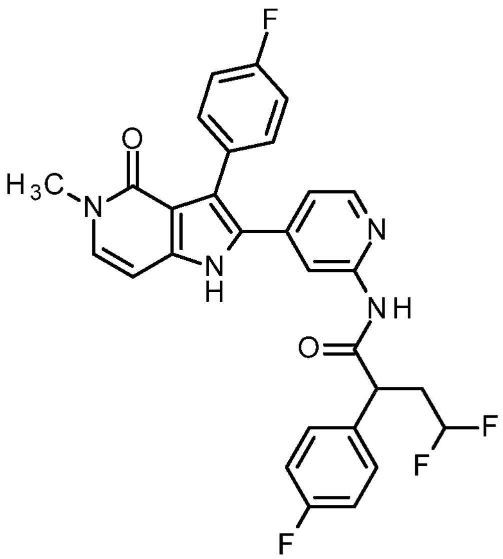

A screw cap vial was charged with N-[4-(3-bromo-5-methyl-4-oxo-4,5-dihydro-1H-pyrrolo[3,2-c]pyridin-2-yl)pyridin-2-yl]-4,4-difluoro-2-(4-fluorophenyl)butanamide (Racemate) (see Intermediate 105, 100 mg), (4-fluorophenyl)boronic acid (53.9 mg, 385 μmol), XPhos Pd G3 (CAS-RN:[1445085-55-1], 16.2 mg, 19.3 μmol) and $K_3PO_4$ (122 mg, 578 μmol). The vial was degassed with argon and charged with 960 μL of 1,4-dioxane / water 4:1. The reaction was heated at 90 °C. After 1 h the reaction was cooled down and concentrated under vacuum. The residue was purified by preparative reverse phase HPLC (eluent: acetonitrile / water 40 to 65% + 0.1% TFA). The title compound containing fractions were concentrated under reduced pressure to provide 73.0 mg of the title compound.

LC-MS (Method 4): Rt = 2.66 min; MS (ESIpos): m/z = 535 $[M+H]^+$

$^1$H-NMR (400 MHz, DMSO-$d_6$) δ [ppm] = 11.75 (s, 1H), 10.50 (s, 1H), 7.85 (d, 1H), 7.80 (s, 1H), 7.17 (dd, 2H), 7.12 (d, 1H), 7.00 (dd, 2H), 6.92 (t, 2H), 6.83 (t, 2H), 6.50 (dd, 1H), 6.17 (d, 1H), 5.71 (tt, 1H), 3.89 (dd, 1H), 3.12 (s, 3H), 2.52 - 2.31 (m, 1H), 2.02 - 1.78 (m, 1H).

**Example 108**

(-)-4,4-difluoro-2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}butanamide (Enantiomer 1)

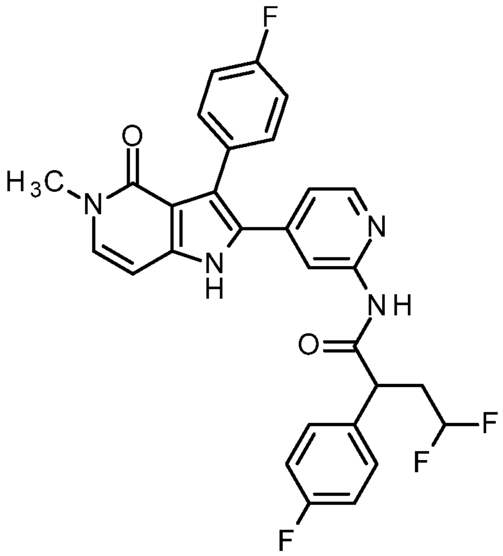

The racemic compound (see Example 107, 170 mg) was separated into enantiomers by preparative chiral HPLC to give enantiomer 1 (75.0 mg, >99 %ee, see Example 108) and enantiomer 2 (72.0 mg, >99 % ee, see Example 109).

Preparative chiral HPLC method:
Instrument: PrepCon Labomatic HPLC; Column: YMC Cellulose SB 10μ, 250x50; eluent A: hexane + 0.1 vol % diethylamine; eluent B: ethanol + 0.1 vol % diethylamine; isocratic: 70%A+30%B; flow: 120 mL/min; temperature: 25°C; UV: 254 nm

Analytical chiral HPLC method:
Instrument: Waters Alliance 2695; Column: YMC Cellulose SB 3μ, 100x4.6; eluent A: hexane + 0.1 vol % diethylamine; eluent B: ethanol; isocratic: 70%A+30%B; flow: 1.4 mL/min; temperature: 25°C; UV: 254 nm

Analytical Chiral HPLC (method Example 108): Rt = 1.41 min.

$[\alpha]_D$ = -206.3° (from solution in DMSO, c = 1.5 mg/mL)

$^1$H-NMR (400 MHz, DMSO-$d_6$) δ [ppm]: 0.798 (0.72), 0.815 (0.82), 0.822 (0.79), 0.840 (0.46), 0.886 (0.41), 0.905 (0.84), 0.922 (0.42), 1.137 (0.76), 1.154 (1.25), 1.173 (0.64), 1.235 (0.60), 1.259 (0.60), 2.169 (0.40), 2.202 (0.41), 2.323 (0.50), 2.327 (0.70), 2.332 (0.48), 2.467 (0.47), 2.472 (0.56), 2.518 (2.69), 2.523 (1.71), 2.531 (0.44), 2.536 (0.42), 2.665 (0.77), 2.669 (0.89), 2.674 (0.73), 2.688 (0.41), 3.347 (0.55), 3.368 (0.49), 3.391 (16.00), 4.140 (0.66), 4.153 (0.77), 4.162 (0.77), 4.176 (0.62), 5.841 (0.60), 5.971 (0.55), 5.981 (1.20), 5.993 (0.56), 6.122 (0.54), 6.428 (4.35), 6.445 (4.03), 6.746 (2.16), 6.750 (2.05), 6.760 (2.03), 6.763 (2.08), 7.080 (2.13), 7.085 (0.82), 7.097 (0.98), 7.102 (4.55), 7.108 (1.00), 7.119 (0.85), 7.124 (2.63), 7.168 (2.14), 7.173 (0.76), 7.185 (0.98), 7.190 (4.60), 7.207 (0.81), 7.212 (2.54), 7.249 (2.58), 7.255 (1.05), 7.263 (2.85), 7.271 (2.53), 7.280 (0.85), 7.285 (2.15), 7.386 (3.76), 7.404 (3.60), 7.425 (2.24), 7.430 (1.01), 7.439 (2.45), 7.447 (2.27), 7.456 (0.86), 7.461 (1.95), 8.076 (2.46), 8.114 (2.70), 8.128 (2.55), 10.796 (2.94), 12.044 (2.61).

**Example 109**

(+)-4,4-difluoro-2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}butanamide (Enantiomer 2)

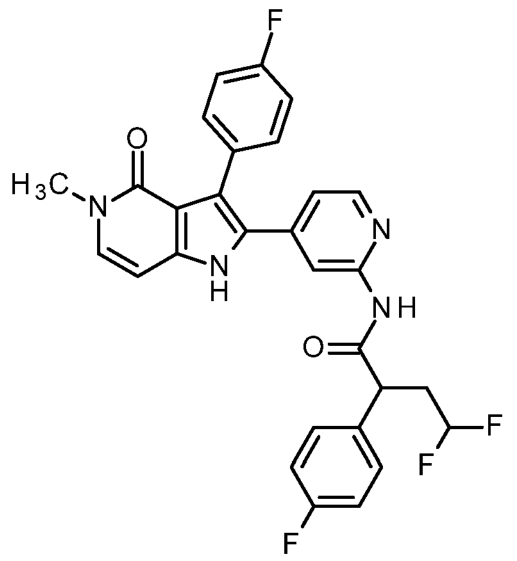

For the preparation of the racemic title compound see Example 107. Separation of enantiomers by preparative chiral HPLC (method see Example 108) gave the title compound (72.0 mg, >99 %ee).

Analytical Chiral HPLC (method see Example 108): Rt = 2.25 min.

$[\alpha]_D$ = +200.7° (from solution in DMSO, c = 1.8 mg/mL)

$^1$H-NMR (400 MHz, DMSO-$d_6$) δ [ppm]: 0.795 (0.46), 0.798 (0.74), 0.815 (0.86), 0.822 (0.81), 0.841 (0.47), 0.886 (0.42), 0.905 (0.86), 0.923 (0.44), 1.085 (0.61), 1.138 (0.47), 1.232 (0.69), 1.259 (0.95), 2.171 (0.41), 2.202 (0.42), 2.323 (0.73), 2.327 (1.01), 2.332 (0.71), 2.518 (4.11), 2.523 (2.62), 2.665 (1.01), 2.669 (1.23), 2.674 (0.96), 2.688 (0.42), 3.391 (16.00), 4.140 (0.68), 4.153 (0.74), 4.162 (0.74), 4.176 (0.63), 5.841 (0.59), 5.971 (0.54), 5.981 (1.18), 5.993 (0.54), 6.122 (0.52), 6.427 (3.97), 6.445 (4.19), 6.746 (2.15), 6.750 (2.00), 6.759 (2.03), 6.763 (2.00), 7.080 (2.13), 7.085 (0.79), 7.097 (1.00), 7.102 (4.52), 7.108 (0.98), 7.119 (0.83), 7.125 (2.60), 7.168 (2.16), 7.173 (0.76), 7.190 (4.60), 7.207 (0.79), 7.212 (2.50), 7.249 (2.57), 7.255 (1.05), 7.263 (2.88), 7.271 (2.45), 7.280 (0.83), 7.285 (2.11), 7.386 (3.65), 7.404 (3.57), 7.425 (2.23), 7.430 (1.01), 7.438 (2.45), 7.447 (2.22), 7.456 (0.86), 7.461 (1.93), 8.076 (2.47), 8.114 (2.67), 8.128 (2.52), 10.796 (2.94), 12.043 (2.62).

## Example 110

2 -(4- fl uorophenyl)- N-{ 4- [3-(2- fl uoropyridi n-3-yl)-5-methyl-4-oxo-4, 5-di hydro-1 H -pyrrolo[3, 2-c]pyridin-2-yl]pyridin-2-yl}propanamide (Racemate)

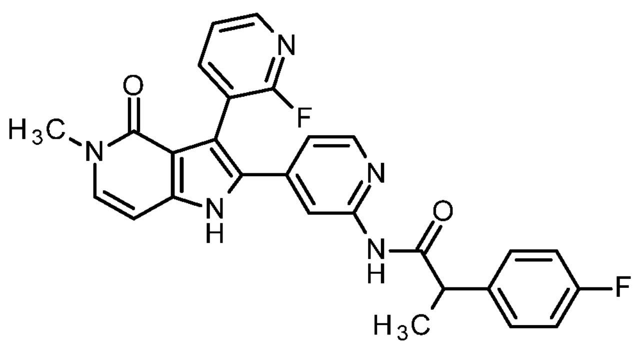

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (2-fluoro-3-pyridyl)boronic acid (11.3 mg, 79.9 µmol), XPhos Pd G3 (CAS-RN:[1445085-55-1], 2.69 mg, 3.20 µmol) and $K_3PO_4$ (20.3 mg, 95.9 µmol). The vial was degassed with argon and charged with 160 µL of 1,4-dioxane / water 4:1. The reaction was heated at 100

°C. After 1 h the reaction was cooled down and concentrated under vacuum. The residue was purified by preparative reverse phase HPLC (eluent: acetonitrile / water 40 to 65% + 0.1% TFA). The title compound containing fractions were concentrated under reduced pressure to provide 8.60 mg of the title compound.

LC-MS (Method 4): Rt = 2.29 min; MS (ESIpos): m/z = 486 $[M+H]^+$

$^1$H-NMR (400 MHz, DMSO-$d_6$) δ [ppm] = 12.24 (s, 1H), 10.65 (s, 1H), 8.21 (dd, 1H), 8.18 (d, 1H), 8.12 (d, 1H), 7.83 (ddd, 1H), 7.44 - 7.37 (m, 3H), 7.33 (ddd, 1H), 7.25 - 7.09 (m, 2H), 6.83 (dd, 1H), 6.47 (d, 1H), 4.01 (q, 1H), 3.40 (s, 3H), 1.37 (d, 3H).

**Example 111**

2-(4-fluorophenyl)-N-{4-[5-methyl-3-(2-methylphenyl)-4-oxo-4,5-dihydro-1H-pyrrolo[3,2-c]pyridin-2-yl]pyridin-2-yl}propanamide (Racemate)

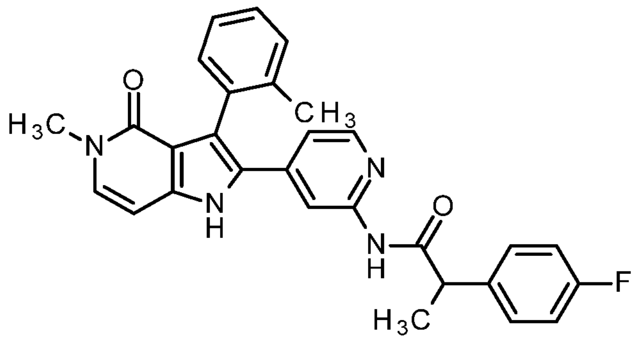

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), o-tolylboronic acid (8.69 mg, 79.9 μmol), XPhos Pd G3 (CAS-RN:[1445085-55-1], 2.69 mg, 3.20 μmol) and $K_3PO_4$ (20.3 mg, 95.9 μmol). The vial was degassed with argon and charged with 160 μL of 1,4-dioxane / water 4:1. The reaction was heated at 100 °C. After 1 h the reaction was cooled down and concentrated under vacuum. The residue was purified by preparative reverse phase HPLC (eluent: acetonitrile / water 45 to 70% + 0.1% TFA). The title compound containing fractions were concentrated under reduced pressure to provide 10.4 mg of the title compound in a 1:1 mixture of atropisomers.

LC-MS (Method 4): Rt = 2.61 min; MS (ESIpos): m/z = 481 $[M+H]^+$

$^1$H-NMR (400 MHz, DMSO-$d_6$) δ [ppm] = 12.00 (d, 2H), 10.58 (d, 2H), 8.16 (d, 1H), 8.11 (s, 1H), 7.98 (t, 2H), 7.42 (ddd, 4H), 7.37 (d, 2H), 7.25 - 7.20 (m, 4H), 7.16 (td, 4H), 7.09 (td, 2H), 7.02 (d, 1H), 6.97 (d, 1H), 6.51 (dd, 1H), 6.49 (dd, 1H), 6.45 (d, 1H), 6.43 (d, 1H), 4.01 (q, 2H), 3.36 (s, 6H), 2.03 (s, 3H), 1.99 (s, 3H), 1.40 (d, 3H), 1.38 (d, 3H).

**Example 112**

N-{4-[3-(2-chlorophenyl)-5-methyl-4-oxo-4,5-dihydro-1H-pyrrolo[3,2-c]pyridin-2-yl]pyridin-2-yl}-2-(4-fluorophenyl)propanamide (Racemate)

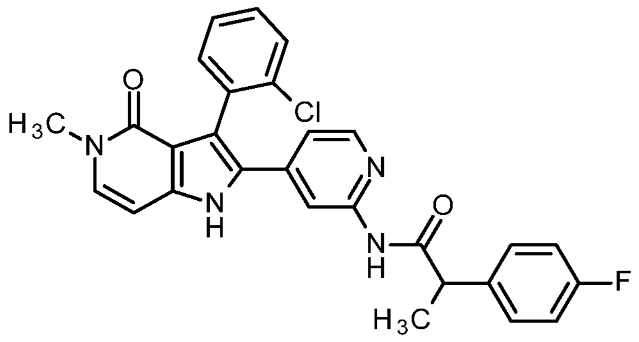

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 20.0 mg), 2-chlorophenyl)boronic acid (13.3 mg, 85.2 μmol), XPhos Pd G3 (CAS-RN:[1445085-55-1], 3.59 mg, 4.26 μmol) and $K_3PO_4$ (27.1 mg, 128 μmol). The vial was degassed with argon and charged with 210 μL of 1,4-dioxane / water 4:1. The reaction was heated at 75 °C. After 16 h the reaction was cooled down and concentrated under vacuum. The residue was purified by preparative reverse phase HPLC (eluent: acetonitrile / water 45 to 70% + 0.1% TFA). The title compound containing fractions were concentrated under reduced pressure to provide 4.20 mg of the title compound in a 1:1 mixture of atropisomers.

LC-MS (Method 4): $R_t$ = 2.57 min; MS (ESIpos): m/z = 501 $[M+H]^+$

$^1$H-NMR (400 MHz, DMSO-$d_6$) δ [ppm] = 12.10 (d, 2H), 10.60 (d, 2H), 8.14 (d, 2H), 8.07 (dd, 2H), 7.52 - 7.33 (m, 10H), 7.33 - 7.22 (m, 4H), 7.17 (t, 4H), 6.63 (td, 2H), 6.45 (dd, 2H), 4.01 (t, 2H), 3.37 (s, 6H), 1.39 (d, 6H).

## Example 113

2-(4-fluorophenyl)-N-{4-[3-(3-hydroxyphenyl)-5-methyl-4-oxo-4,5-dihydro-1H-pyrrolo[3,2-c]pyridin-2-yl]pyridin-2-yl}propanamide (Racemate)

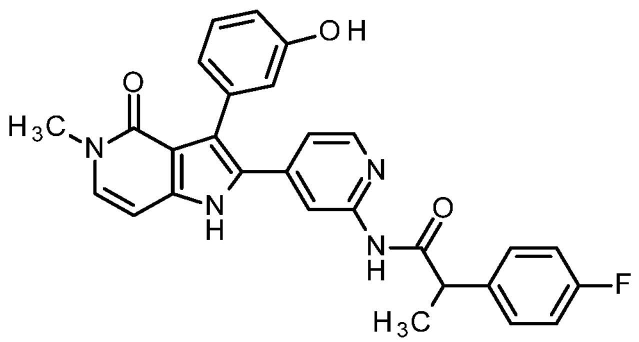

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (3-hydroxyphenyl)boronic acid (8.82 mg, 639 μmol), XPhos Pd G3 (CAS-RN:[1445085-55-1], 2.69 mg, 3.20 μmol) and $K_3PO_4$ (20.3 mg, 95.9 μmol). The vial was degassed with argon and charged with 160 μL of 1,4-dioxane / water 4:1. The reaction was heated at 75 °C. After 16 h the reaction was cooled down and concentrated under vacuum. The residue was purified by preparative reverse phase HPLC (eluent: acetonitrile / water 25 to 55% + 0.1% TFA). The title compound containing fractions were concentrated under reduced pressure to provide 11.7 mg of the title compound.

LC-MS (Method 4): Rt = 2.29 min; MS (ESIpos): m/z = 483 $[M+H]^+$

$^1$H-NMR (400 MHz, DMSO-$d_6$) δ [ppm] = 11.95 (s, 1H), 10.64 (s, 1H), 9.16 (s, 1H), 8.18 (s, 1H), 8.05 (d, 1H), 7.43 (dd, 2H), 7.37 (d, 1H), 7.16 (t, 2H), 7.06 (t, 1H), 6.68 (s, 1H), 6.65 (dq, 3H), 6.42 (d, 1H), 4.03 (q, 1H), 3.39 (s, 3H), 1.40 (d, 3H).

**Example 114**

N-{4-[3-(3,4-difluorophenyl)-5-methyl-4-oxo-4,5-dihydro-1H-pyrrolo[3,2-c]pyridin-2-yl]pyridin-2-yl}-2-(4-fluorophenyl)propanamide (Racemate)

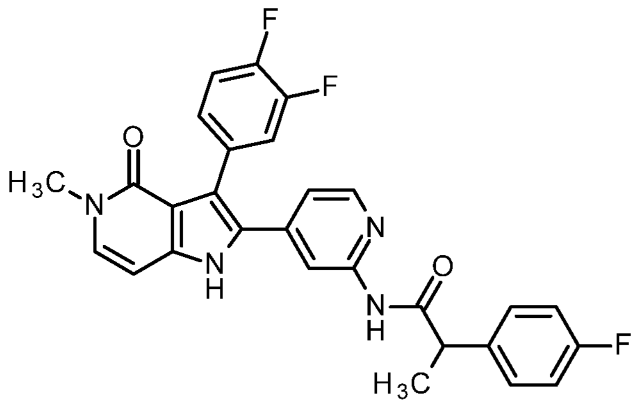

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (3,4-difluorophenyl)boronic acid (10.1 mg, 63.9 μmol), XPhos Pd G3 (CAS-RN:[1445085-55-1], 2.69 mg, 3.2 μmol) and $K_3PO_4$ (20.3 mg, 95.9 μmol). The vial was degassed with argon and charged with 160 μL of 1 ,4-dioxane / water 4:1. The reaction was heated at 90 °C. After 16 h the reaction was cooled down and concentrated under vacuum. The residue was purified by preparative reverse phase HPLC (eluent: acetonitrile / water 25 to 60% + 0.1% TFA). The title compound containing fractions were concentrated under reduced pressure to provide 8.91 mg of the title compound.

LC-MS (Method 4): $R_t$ = 2.66 min; MS (ESIpos): m/z = 503 $[M+H]^+$

$^1$H-NMR (400 MHz, DMSO-$d_6$) δ [ppm] = 12.09 (s, 1H), 10.64 (s, 1H), 8.16 (d, 1H), 8.10 (d, 1H), 7.45 - 7.37 (m, 3H), 7.37 - 7.25 (m, 2H), 7.15 (t, 2H), 7.07 - 6.98 (m, 1H), 6.82 (dd, 1H), 6.44 (d, 1H), 4.00 (q, 1H), 3.40 (s, 3H), 1.37 (d, 3H).

**Example 115**

2-(4-fluorophenyl)-N-{4-[3-(3-furyl)-5-methyl-4-oxo-4,5-dihydro-1H-pyrrolo[3,2-c]pyridin-2-yl]pyridin-2-yl}propanamide (Racemate)

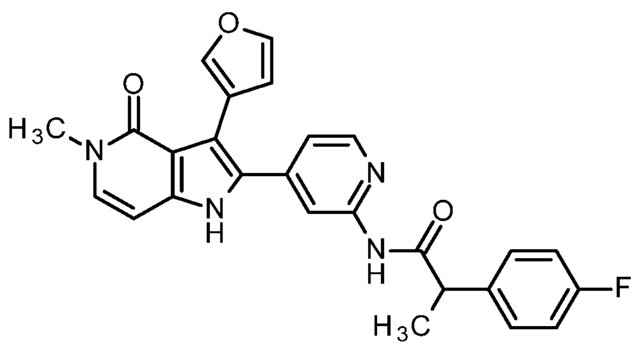

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 20.0 mg), (3-furylboronic acid (9.54 mg, 85.2 μmol), XPhos Pd G3 (CAS-RN:[1445085-55-1], 3.59 mg, 4.26 μmol) and $K_3PO_4$ (27.1 mg, 128 μmol). The vial was degassed with argon and charged with 210 μL of 1,4-dioxane / water 4:1. The reaction was heated at 90 °C. After

16 h the reaction was cooled down and concentrated under vacuum. The residue was purified by preparative reverse phase HPLC (eluent: acetonitrile / water 25 to 55% + 0.1% TFA). The title compound containing fractions were concentrated under reduced pressure to provide 9.60 mg of the title compound.

LC-MS (Method 4): $R_t$ = 2.39 min; MS (ESIpos): m/z = 457 $[M+H]^+$
$^1$H-NMR (400 MHz, DMSO-$d_6$) $\delta$ [ppm] = 11.95 (s, 1H), 10.71 (s, 1H), 8.25 (s, 1H), 8.22 (d, 1H), 7.77 (d, 1H), 7.60 (t, 1H), 7.48 - 7.42 (m, 2H), 7.39 (d, 1H), 7.24 - 7.11 (m, 2H), 7.03 (dd, 1H), 6.41 (d, 1H), 6.36 (d, 1H), 4.06 (q, 1H), 3.43 (s, 3H), 1.41 (d, 3H).

**Example 116**

2-(4-fluorophenyl)-N-{4-[3-(2-furyl)-5-methyl-4-oxo-4,5-dihydro-1H-pyrrolo[3,2-c]pyridin-2-yl]pyridin-2-yl}propanamide (Racemate)

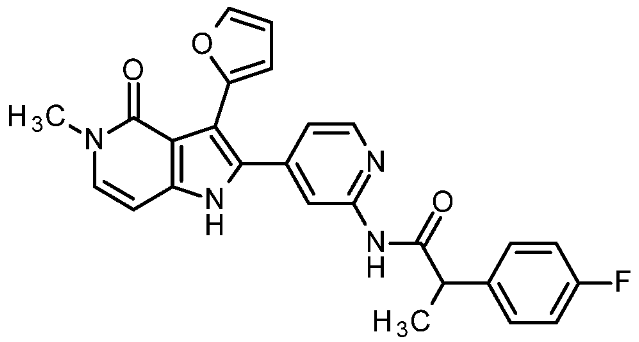

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 20.0 mg), furylboronic acid (14.3 mg, 128 µmol), XPhos Pd G3 (CAS-RN:[1445085-55-1], 3.59 mg, 4.30 µmol) and $K_3PO_4$ (27.1 mg, 128 µmol). The vial was degassed with argon and charged with 210 µL of 1,4-dioxane / water 4:1. The reaction was heated at 75 °C. After 24 h the reaction was cooled down and concentrated under vacuum. The residue was purified by preparative reverse phase HPLC (eluent: acetonitrile / water 30 to 60% + 0.1% TFA). The title compound containing fractions were concentrated under reduced pressure to provide 9.20 mg of the title compound as a 1:1 mixture of atropisomers.

LC-MS (Method 4): Rt = 2.40 min; MS (ESIpos): m/z = 457 $[M+H]^+$

$^1$H-NMR (400 MHz, DMSO-$d_6$) $\delta$ [ppm] = 12.11 (s, 1H), 11.95 (s, 1H), 10.70 (d, 2H), 8.27 - 8.12 (m, 4H), 7.76 (d, 1H), 7.60 (t, 1H), 7.51 (d, 1H), 7.46 - 7.33 (m, 6H), 7.16 (t, 4H), 7.03 (dd, 1H), 6.86 - 6.76 (m, 2H), 6.51 (dd, 1H), 6.41 (t, 2H), 6.35 (d, 1H), 4.04 (q, 2H), 3.42 (d, 6H), 1.40 (d, 6H).

**Example 117**

2-(4-fluorophenyl)-N-{4-[5-methyl-4-oxo-3-(3,4,5-trifluorophenyl)-4,5-dihydro-1H-pyrrolo[3,2-c]pyridin-2-yl]pyridin-2-yl}propanamide (Racemate)

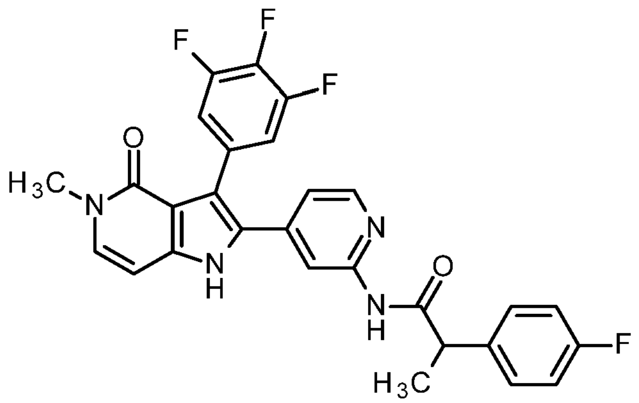

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 20.0 mg), (3,4,5-trifluorophenyl)boronic acid (15.0 mg, 85.2μmol), XPhos Pd G3 (CAS-RN:[1445085-55-1], 3.60 mg, 4.30 μmol) and $K_3PO_4$ (27.1 mg, 128 μmol). The vial was degassed with argon and charged with 210 μL of 1,4-dioxane / water 4:1. The reaction was heated at 75 °C. After 24 h the reaction was cooled down and concentrated under vacuum. The residue was purified by preparative reverse phase HPLC (eluent: acetonitrile / water 25 to 55% + 0.1% TFA). The title compound containing fractions were concentrated under reduced pressure to provide 11.6 mg of the title compound.

LC-MS (Method 4): Rt = 2.76 min; MS (ESIpos): m/z = 521 $[M+H]^+$

$^1H$-NMR (400 MHz, DMSO-$d_6$) δ [ppm] = 12.16 (s, 1H), 10.64 (s, 1H), 8.21 (d, 1H), 8.06 (d, 1H), 7.51 - 7.32 (m, 3H), 7.20 - 7.08 (m, 4H), 6.94 (dd, 1H), 6.45 (d, 1H), 4.00 (q, 1H), 3.41 (s, 3H), 1.36 (d, 3H).

**Example 118**

4,4-difluoro-2-(4-fluorophenyl)-N-{4-[5-methyl-4-oxo-3-(pyridin-3-yl)-4,5-dihydro-1H-pyrrolo[3,2-c]pyridin-2-yl]pyridin-2-yl}butanamide (Racemate)

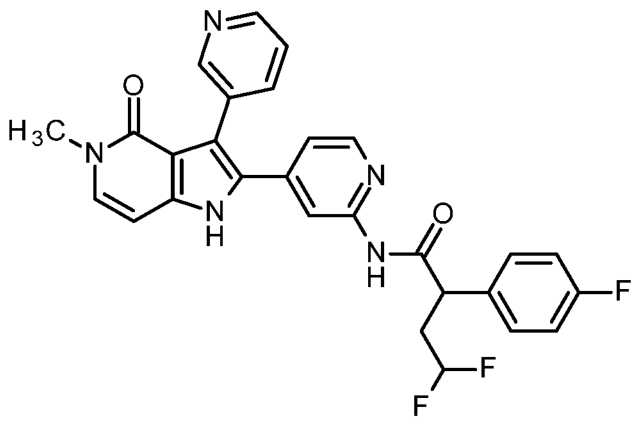

A screw cap vial was charged with N-[4-(3-bromo-5-methyl-4-oxo-4,5-dihydro-1H-pyrrolo[3,2-c]pyridin-2-yl)pyridin-2-yl]-4,4-difluoro-2-(4-fluorophenyl)butanamide (Racemate) (see Intermediate 105, 50.0 mg), 3-pyridylboronic acid (23.7 mg, 193 μmol), XPhos Pd G3 (CAS-RN:[1445085-55-1], 8.12 mg, 9.63 μmol) and $K_3PO_4$ (61.2 mg, 289 μmol). The vial was degassed with argon and charged with 480 μL of 1,4-dioxane / water 4:1. The reaction was heated at 75 °C. After 24 h the reaction was cooled down and concentrated under vacuum. The residue was purified by preparative reverse phase HPLC (eluent: acetonitrile / water 30 to 60% + 0.1% TFA). The title compound containing fractions were concentrated under reduced pressure to provide 12.3 mg of the title compound.

LC-MS (Method 4): Rt = 2.01 min; MS (ESIpos): m/z = 518 [M+H]+

$^1$H-NMR (400 MHz, DMSO-$d_6$) δ [ppm] = 12.38 (s, 1H), 10.86 (s, 1H), 8.85 - 8.61 (m, 2H), 8.21 (t, 2H), 8.04 (s, 1H), 7.76 (t, 1H), 7.52 - 7.34 (m, 3H), 7.20 (t, 2H), 6.95 (dd, 1H), 6.51 (d, 1H), 5.98 (tt, 1H), 4.16 (dd, 1H), 3.43 (s, 3H), 2.83 - 2.60 (m, 1H), 2.27 - 2.08 (m, 1H).

## Example 119

2-(4-fluorophenyl)-N-{4-[3-(4-hydroxyphenyl)-5-methyl-4-oxo-4,5-dihydro-1H-pyrrolo[3,2-c]pyridin-2-yl]pyridin-2-yl}propanamide (Racemate)

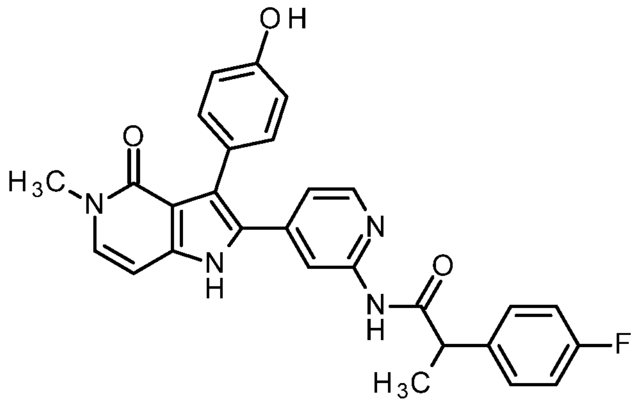

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), 4-hydroxyphenyl)boronic acid (8.82 mg, 63.9 μmol), XPhos Pd G3 (CAS-RN:[1445085-55-1], 2.69 mg, 3.20 μmol) and $K_3PO_4$ (20.3 mg, 95.9 μmol). The vial was degassed with argon and charged with 160 μL of 1,4-dioxane / water 4:1. The reaction was heated at 75 °C. After 16 h the reaction was cooled down and concentrated under vacuum. The residue was purified by preparative reverse phase HPLC (eluent: acetonitrile / water 30 to 60% + 0.1% TFA). The title compound containing fractions were concentrated under reduced pressure to provide 8.70 mg of the title compound.

LC-MS (Method 4): Rt = 2.24 min; MS (ESIpos): m/z = 483 $[M+H]^+$

$^1$H-NMR (400 MHz, DMSO-$d_6$) δ [ppm] = 11.88 (s, 1H), 10.65 (s, 1H), 9.28 (s, 1H), 8.17 (s, 1H), 8.05 (d, 1H), 7.42 (dd, 2H), 7.36 (d, 1H), 7.17 (t, 2H), 7.05 (d, 2H), 6.74 - 6.60 (m, 3H), 6.41 (d, 1H), 4.02 (t, 1H), 3.38 (s, 3H), 1.40 (d, 3H).

## Example 120

N-{4-[3-(2,5-difluorophenyl)-5-methyl-4-oxo-4,5-dihydro-1 H-pyrrolo[3,2-c]pyridin-2-yl]pyridin-2-yl}-2-(4-fluorophenyl)propanamide (Racemate)

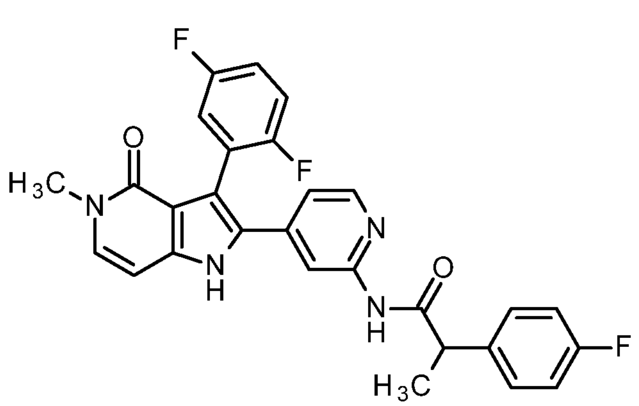

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (2,5-difluorophenyl)-boronic acid (10.1 mg, 63.9 μmol), XPhos Pd G3 (CAS-RN:[1445085-55-1], 2.69 mg, 3.20 μmol) and $K_3PO_4$ (20.3 mg, 95.9 μmol). The vial was degassed with argon and charged with 160 μL of 1,4-dioxane / water 4:1. The reaction was heated at 75 °C. After 16 h the reaction was cooled down and concentrated under vacuum. The residue was purified by preparative reverse phase HPLC (eluent: acetonitrile / water 25 to 55% + 0.1% TFA). The title compound containing fractions were concentrated under reduced pressure to provide 6.20 mg of the title compound.

LC-MS (Method 4): Rt = 2.57 min; MS (ESIpos): m/z = 503 $[M+H]^+$

$^1$H-NMR (400 MHz, DMSO-$d_6$) δ [ppm] = 12.20 (s, 1H), 10.63 (s, 1H), 8.17 (d, 1H), 8.14 (s, 1H), 7.44 - 7.35 (m, 3H), 7.29 - 7.08 (m, 5H), 6.84 (dd, 1H), 6.46 (d, 1H), 4.01 (q, 1H), 3.40 (s, 3H), 1.38 (d, 3H).

**Example 121**

N-{4-[3-(3,5-difluorophenyl)-5-methyl-4-oxo-4,5-dihydro-1H-pyrrolo[3,2-c]pyridin-2-yl]pyridin-2-yl}-2-(4-fluorophenyl)propanamide (Racemate)

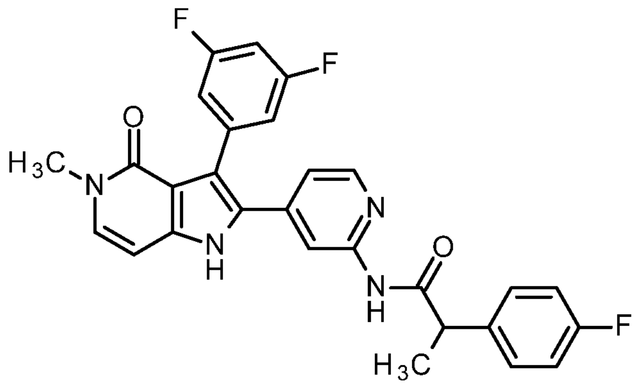

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (3,5-difluorophenyl)boronic acid (10.1 mg, 63.9 μmol), XPhos Pd G3 (CAS-RN:[1445085-55-1], 2.69 mg, 3.20 μmol) and $K_3PO_4$ (20.3 mg, 95.9 μmol). The vial was degassed with argon and charged with 160 μL of 1,4-dioxane / water 4:1. The reaction was heated at 75 °C. After 16 h the reaction was cooled down and concentrated under vacuum. The residue was purified by preparative reverse phase HPLC (eluent: acetonitrile / water 30 to 60% + 0.1% TFA). The title compound containing fractions were concentrated under reduced pressure to provide 7.32 mg of the title compound.

LC-MS (Method 4): $R_t$ = 2.67 min; MS (ESIpos): m/z = 503 $[M+H]^+$

$^1$H-NMR (400 MHz, DMSO-$d_6$) δ [ppm] = 12.16 (s, 1H), 10.69 (s, 1H), 8.18 (d, 1H), 8.12 (d, 1H), 7.41 (dd, 3H), 7.15 (q, 3H), 7.03 - 6.92 (m, 2H), 6.83 (dd, 1H), 6.45 (d, 1H), 4.01 (q, 1H), 3.41 (s, 3H), 1.38 (d, 3H).

**Example 122**

N-{4-[3-(5-chloro-2-thienyl)-5-methyl-4-oxo-4,5-dihydro-1H-pyrrolo[3,2-c]pyridin-2-yl]pyridin-2-yl}-2-(4-fluorophenyl)propanamide (Racemate)

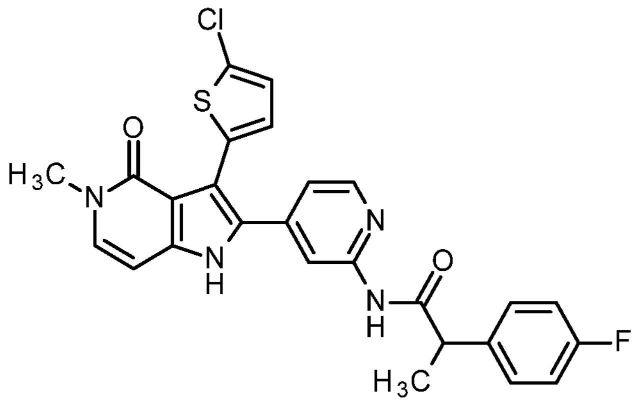

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (5-chloro-2-thienyl)boronic acid (10.4 mg, 63.9 μmol), XPhos Pd G3 (CAS-RN:[1445085-55-1], 2.69 mg, 3.20 μmol) and $K_3PO_4$ (20.3 mg, 95.9 μmol). The vial was degassed with argon and charged with 160 μL of 1,4-dioxane / water 4:1. The reaction was heated at 75 °C. After 2 h the reaction was cooled down and concentrated under vacuum. The residue was purified by preparative reverse phase HPLC (eluent: acetonitrile / water 30 to 70% + 0.1% TFA). The title compound containing fractions were concentrated under reduced pressure to provide 3.56 mg of the title compound.

LC-MS (Method 4): Rt = 2.73 min; MS (ESIpos): m/z = 507 $[M+H]^+$

$^1$H-NMR (400 MHz, DMSO-$d_6$) δ [ppm] = 12.20 (s, 1H), 10.70 (s, 1H), 8.23 (d, 2H), 7.50-7.36 (m, 3H), 7.17 (t, 2H), 7.00 (d, 1H), 6.96 (d, 1H), 6.82 (d, 1H), 6.43 (d, 1H), 4.05 (t, 1H), 3.42 (s, 3H), 1.40 (d, 3H).

## Example 123

N-{ 4-[3-(2 -cyanophenyl)-5- methyl-4-oxo-4, 5-di hydro-1 H-pyrrolo[3,2 -c] pyri di n-2 -yl] pyrid i n-2- yl}-2-(4-fluorophenyl)propanamide (Racemate)

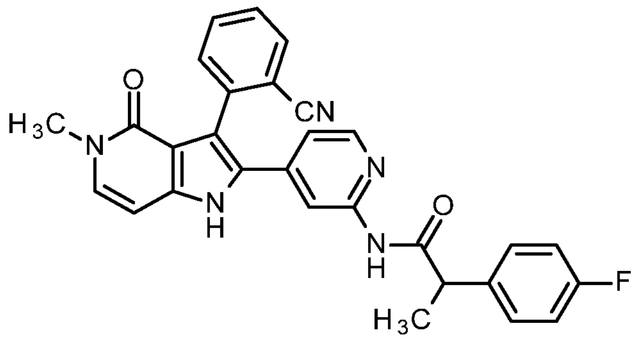

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (2-cyanophenyl)boronic acid (9.39 mg ,63.9 pmol), XPhos Pd G3 (CAS-RN:[1445085-55-1], 2.69 mg, 3.20 μmol) and $K_3PO_4$ (20.3 mg, 959 μmol). The vial was degassed with argon and charged with 160 μL of 1,4-dioxane / water 4:1. The reaction was heated at 75 °C. After 16 h the reaction was cooled down and concentrated under vacuum. The residue was purified by preparative reverse phase HPLC (eluent: acetonitrile / water 25 to 55% + 0.1% TFA). The title compound containing fractions were concentrated under reduced pressure to provide 5.80 mg of the title compound as a 1:1 mixture of atropisomers.

LC-MS (Method 4): Rt = 2.40 min; MS (ESIpos): m/z = 492 $[M+H]^+$

$^1$H-NMR (400 MHz, DMSO-$d_6$) δ [ppm] = 12.25 (d, 2H), 10.62 (d, 2H), 8.12 (t, 2H), 8.08 (s, 1H), 8.05 (s, 1H), 7.84 (d, 1H), 7.79 (d, 1H), 7.66 (t, 1H), 7.61 (t, 1H), 7.52 (t, 2H), 7.47 - 7.33 (m, 10H), 7.17 (t, 4H), 6.70 (dd, 1H), 6.66 (dd, 1H), 6.48 (dd, 2H), 4.03 - 3.95 (m, 2H), 3.39 (s, 6H), 1.37 (d, 2H), 1.35 (d, 2H).

**Example 124**

2-(4-fluorophenyl)-N-{4-[3-(2-hydroxyphenyl)-5-methyl-4-oxo-4,5-dihydro-1H-pyrrolo[3,2-c]pyridin-2-yl]pyridin-2-yl}propanamide (Racemate)

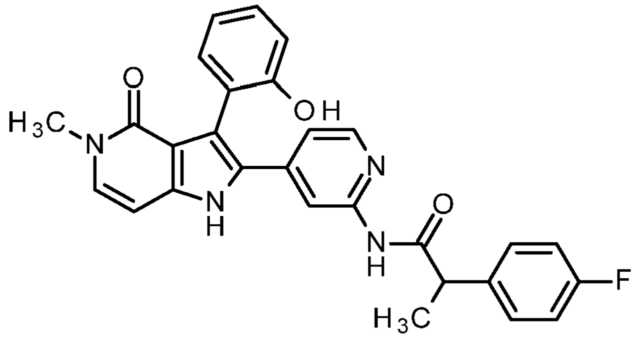

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (2-hydroxyphenyl)boronic acid (8.82 mg,63.9 μmol), XPhos Pd G3 (CAS-RN:[1445085-55-1], 2.69 mg, 3.20 μmol) and $K_3PO_4$ (20.3 mg, 959 μmol). The vial was degassed with argon and charged with 160 μL of 1,4-dioxane / water 4:1. The reaction was heated at 75 °C. After 20 h the reaction was cooled down and concentrated under vacuum. The residue was purified by preparative reverse phase HPLC (eluent: acetonitrile / water 30 to 60% + 0.1% TFA). The title compound containing fractions were concentrated under reduced pressure to provide 9.28 mg of the title compound.

LC-MS (Method 4): $R_t$ = 2.41 min; MS (ESIpos): m/z = 483 $[M+H]^+$

$^1$H-NMR (400 MHz, DMSO-$d_6$) δ [ppm] = 11.99 (s, 1H), 10.64 (s, 1H), 8.16 (s, 1H), 8.03 (d, 1H), 7.43 (dd, 2H), 7.39 (d, 1H), 7.24 - 7.08 (m, 3H), 6.98 (s, 1H), 6.84 (d, 1H), 6.78 - 6.67 (m, 2H), 6.48 (d, 1H), 4.07 - 3.98 (m, 1H), 3.40 (s, 3H), 1.41 (d, 3H).

**Example 125**

2-(4-fluorophenyl)-N-{4-[5-methyl-3-(5-methyl-2-thienyl)-4-oxo-4,5-dihydro-1H-pyrrolo[3,2-c]pyridin-2-yl]pyridin-2-yl}propanamide (Racemate)

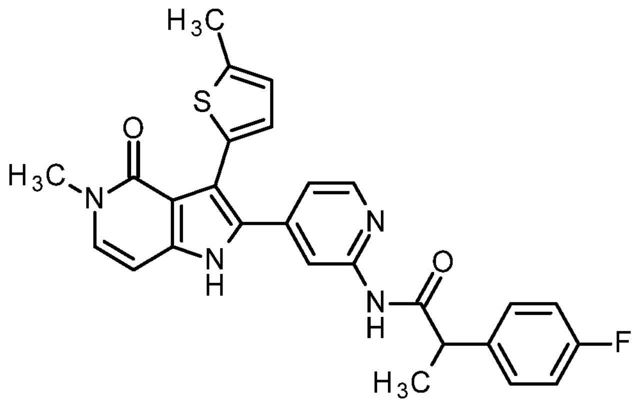

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (5-methyl-2-thienyl)boronic acid (9.08 mg, 63.9 μmol), XPhos Pd G3 (CAS-RN:[1445085-55-1], 2.69 mg, 3.20 μmol) and $K_3PO_4$ (20.3 mg, 95.9 μmol). The vial was degassed with argon and charged with 160 μL of 1,4-dioxane / water 4:1. The reaction was heated at 75

°C. After 20 h the reaction was cooled down and concentrated under vacuum. The residue was purified by preparative reverse phase HPLC (eluent: acetonitrile / water 25 to 55% + 0.1% TFA). The title compound containing fractions were concentrated under reduced pressure to provide 5.20 mg of the title compound.

LC-MS (Method 4): Rt = 2.61 min; MS (ESIpos): m/z = 487 $[M+H]^+$

$^1$H-NMR (400 MHz, DMSO-$d_6$) δ [ppm] = 12.06 (s, 1H), 10.68 (s, 1H), 8.19 (d, 1H), 8.15 (d, 1H), 7.48 - 7.40 (m, 2H), 7.38 (d, 1H), 7.23 - 7.07 (m, 2H), 6.89 (dd, 1H), 6.74 (d, 1H), 6.68 (d, 1H), 6.40 (d, 1H), 4.03 (q, 1H), 3.39 (s, 3H), 2.42 (s, 3H), 1.40 (d, 3H).

## Example 126

N-{4-[3-(2-aminopyridin-4-yl)-5-methyl-4-oxo-4,5-dihydro-1H-pyrrolo[3,2-c]pyridin-2-yl]pyridin-2-yl}-2-(4-fluorophenyl)propanamide (Racemate)

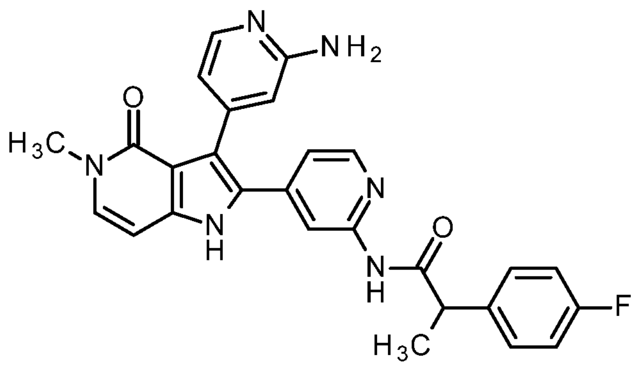

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 17.0 mg), 4-(4,4,5,5-tetramethyl-1,3,2-dioxaborolan-2-yl)pyridin-2-amine (15.9 mg, 72.4 μmol), XPhos Pd G3 (CAS-RN:[1445085-55-1], 3.05 mg, 3.62 μmol) and $K_3PO_4$ (23.0 mg, 109 μmol). The vial was degassed with argon and charged with 180 μL of 1,4-dioxane / water 4:1. The reaction was heated at 75 °C. After 1 h the reaction was cooled down and concentrated under vacuum. The residue was purified by preparative reverse phase HPLC (eluent: acetonitrile / water 10 to 60% + 0.1% TFA). The title compound containing fractions were concentrated under reduced pressure to provide 14.2 mg of the title compound.

LC-MS (Method 4): Rt = 1.88 min; MS (ESIpos): m/z = 483 $[M+H]^+$

$^1$H-NMR (400 MHz, DMSO-$d_6$) δ [ppm] = 12.24 (s, 1H), 10.68 (s, 1H), 8.27 (d, 1H), 8.17 (d, 1H), 8.04 (s, 2H), 7.86 (d, 1H), 7.81 (dd, 1H), 7.44 (d, 1H), 7.42 - 7.36 (m, 2H), 7.25 - 7.14 (m, 2H), 7.08 (dd, 1H), 6.98 (d, 1H), 6.47 (d, 1H), 4.00 (q, 1H), 3.43 (s, 3H), 1.36 (d, 3H).

## Example 127

N-{4-[3-(2-cyano-5-fluorophenyl)-5-methyl-4-oxo-4,5-dihydro-1H-pyrrolo[3,2-c]pyridin-2-yl]pyridin-2-yl}-2-(4-fluorophenyl)propanamide (Racemate)

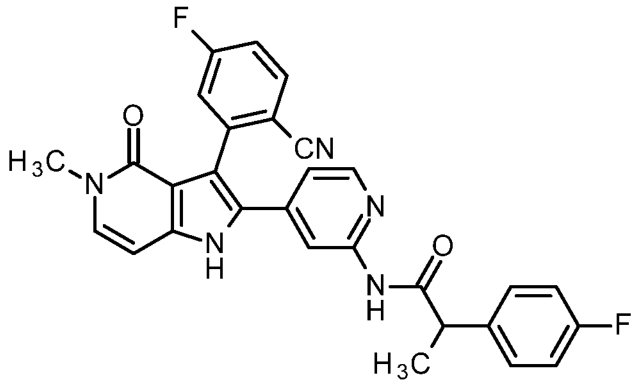

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 20.0 mg), 4-fluoro-2-(4,4,5,5-tetramethyl-1,3,2-dioxaborolan-2-yl)benzonitrile (42.1 mg, 170 μmol), XPhos Pd G3 (CAS-RN:[1445085-55-1], 3.59 mg, 4.26 μmol) and $K_3PO_4$ (27.1 mg, 128 μmol). The vial was degassed with argon and charged with 210 μL of 1,4-dioxane / water 4:1. The reaction was heated at 75 °C. After 1 h the reaction was cooled down and concentrated under vacuum. The residue was purified by preparative reverse phase HPLC (eluent: acetonitrile / water 25 to 60% + 0.1% TFA). The title compound containing fractions were concentrated under reduced pressure to provide 6.10 mg of the title compound as a 1:1 mixture of atropisomers.

LC-MS (Method 4): Rt = 2.48 min; MS (ESIpos): m/z = 510 $[M+H]^+$

$^1$H-NMR (400 MHz, DMSO-$d_6$) δ [ppm] = 12.32 (d, 2H), 10.63 (d, 2H), 8.19 (t, 2H), 8.03 (dd, 2H), 7.95 (dd, 1H), 7.88 (dd, 1H), 7.49 - 7.35 (m, 9H), 7.29 (dd, 1H), 7.16 (q, 4H), 6.84 (dd, 1H), 6.80 (dd, 1H), 6.49 (d, 2H), 4.02 - 3.95 (m, 1H), 3.40 (s, 6H), 1.36 (d, 3H), 1.35 (d, 3H).

Example **128**

N-{4-[3-(2-cyano-5-fluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide (Racemate)

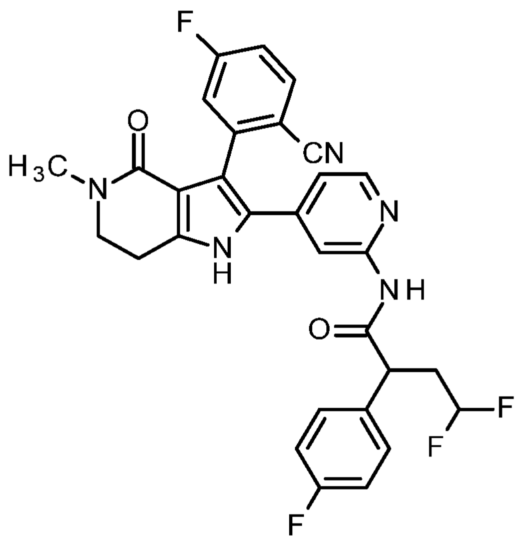

A screw cap vial was charged with N-[4-(3-bromo-5-methyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate) (see Intermediate 104, 60.0 mg,), 4-fluoro-2-(4,4,5,5-tetramethyl-1,3,2-dioxaborolan-2-yl)benzonitrile (114 mg, 462 μmol), XPhos Pd G3 (CAS-RN:[1445085-55-1],

9.74 mg, 11.6 μmol) and $K_3PO_4$ (73.5 mg, 347 pmol). The vial was degassed with argon and charged with 580 μL of 1,4-dioxane / water 4:1. The reaction was heated at 75 °C. After 1 h the reaction was cooled down and concentrated under vacuum. The residue was purified by preparative reverse phase HPLC (eluent: acetonitrile / water 25 to 60% + 0.1% TFA). The title compound containing fractions were concentrated under reduced pressure to provide 33.6 mg of the title compound as a 1:1 mixture of atropisomers.

LC-MS (Method 4): Rt = 2.57 min; MS (ESIpos): m/z = 560 $[M+H]^+$

$^1$H-NMR (400 MHz, DMSO-$d_6$) δ [ppm] = 12.32 (s, 2H), 10.77 (d, 2H), 8.21 (dd, 3H), 8.01 - 7.92 (m, 3H), 7.87 (dd, 1H), 7.50 - 7.36 (m, 8H), 7.27 (dd, 1H), 7.25 - 7.14 (m, 4H), 6.88 (ddd, 2H), 6.49 (dd, 2H), 5.96 (tt, 2H), 4.12 (d, 2H), 3.40 (s, 6H), 2.71 - 2.57 (m, 2H), 2.27 - 2.01 (m, 2H).

Example **129**

4,4-difluoro-2-(4-fluorophenyl)-N-{4-[5-methyl-3-(2-methyl-1,3-thiazol-5-yl)-4-oxo-4,5,6,7-tetrahydro-1H-pyrrolo[3,2-c]pyridin-2-yl]pyridin-2-yl}butanamide (Racemate)

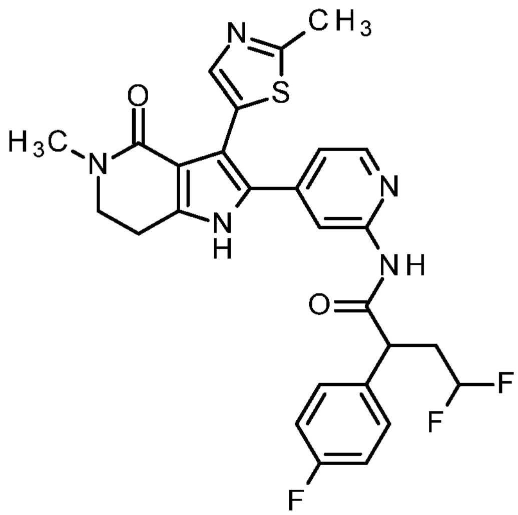

A microwave vial was charged with N-[4-(3-bromo-5-methyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate) (see Intermediate 104, 20.0 mg), 2-methyl-5-(4,4,5,5-tetramethyl-1,3,2-dioxaborolan-2-yl)thiazole (34.7 mg, 154 μmol), XPhos Pd G3 (CAS-RN:[1445085-55-1], 6.47 mg, 7.67 μmol) and $K_3PO_4$ (32.7 mg, 154 μmol). The vial was sealed, degassed with argon and charged with 500 μL of 1,4-dioxane / water 4:1. The reaction was heated in a microwave at 105 °C. After 30 min the reaction was cooled down and concentrated under vacuum. The residue was purified by preparative reverse phase HPLC (eluent: acetonitrile / water 20 to 60% + 0.1% TFA). The title compound containing fractions were concentrated under reduced pressure and purified by preparative reverse phase HPLC (eluent: acetonitrile / water 5 to 45% + 0.1% TFA). The title compound containing fractions were cocentrated under reduces pressure to provide 13.4 mg of the title compound.

LC-MS (Method 4): Rt = 2.37 min; MS (ESIpos): m/z = 540 $[M+H]^+$

$^1$H-NMR (400 MHz, DMSO-$d_6$) δ [ppm] = 12.02 (s, 1H), 10.75 (s, 1H), 8.14 (d, 1H), 8.02 (s, 1H), 7.46 (dd, 2H), 7.30 (s, 1H), 7.23 - 7.13 (m, 2H), 6.86 (dd, 1H), 5.99 (tt, 1H), 4.16 (t, 1H), 3.55 (t, 2H), 2.97 - 2.89 (m, 2H), 2.86 (s, 3H), 2.75 - 2.67 (m, 1H), 2.62 (s, 3H), 2.20 (d, 1H).

**Example 130**

2-(4-fluorophenyl)-N-{4-[5-methyl-4-oxo-3-(2-thienyl)-4,5-dihydro-1H-pyrrolo[3,2-c]pyridin-2-yl]pyridin-2-yl}propanamide (Racemate)

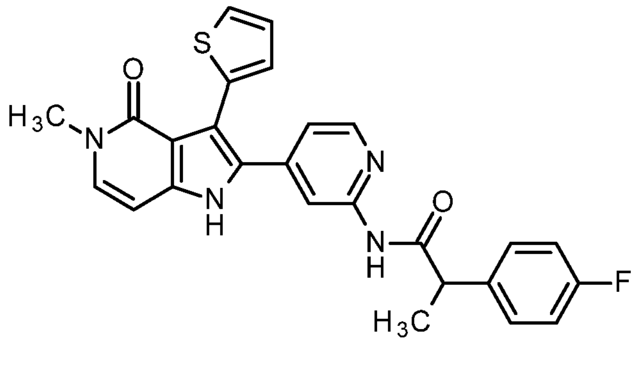

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), 2-thienylboronic acid (8.18 mg, 63.9 µmol), XPhos Pd G3 (CAS-RN:[1445085-55-1], 2.69 mg, 3.20 µmol) and $K_3PO_4$ (20.3 mg, 95.9 µmol). The vial was degassed with argon and charged with 160 µL of 1,4-dioxane / water 4:1. The reaction was heated at 75 °C. After 2 h the reaction was cooled down and concentrated under vacuum. The residue was purified by preparative reverse phase HPLC (eluent: acetonitrile / water 25 to 55% + 0.1% TFA). The title compound containing fractions were concentrated under reduced pressure to provide 6.90 mg of the title compound.

LC-MS (Method 4): $R_t$ = 2.50 min; MS (ESIpos): m/z = 473 $[M+H]^+$

$^1$H-NMR (400 MHz, DMSO-$d_6$) δ [ppm] = 12.36 (s, 1H), 10.94 (s, 1H), 8.43 (s, 1H), 8.38 (d, 1H), 7.74 (d, 1H), 7.71 - 7.62 (m, 3H), 7.41 (t, 2H), 7.26 (dt, 2H), 7.07 (dd, 1H), 6.67 (d, 1H), 4.28 (q, 1H), 3.65 (s, 3H), 1.65 (d, 3H).

**Example 131**

2-(4-fluorophenyl)-N-{4-[5-methyl-4-oxo-3-(3-thienyl)-4,5-dihydro-1H-pyrrolo[3,2-c]pyridin-2-yl]pyridin-2-yl}propanamide (Racemate)

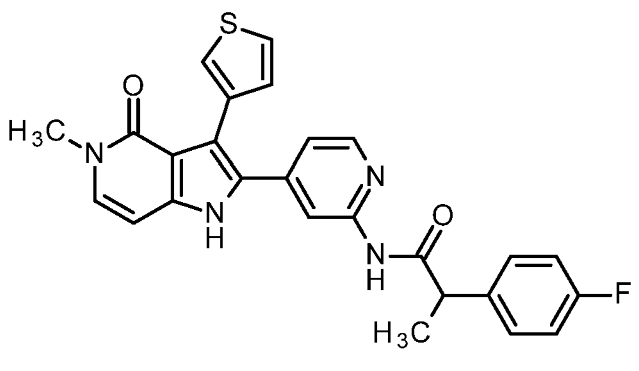

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), potassium trifluoro(3-thienyl)borate(1-) (CAS-RN:[192863-37-9], 12.1 mg, 63.9 µmol), XPhos Pd G3 (CAS-RN:[1445085-55-1], 2.69 mg, 3.20 µmol) and $K_3PO_4$ (20.3 mg, 95.9 µmol).

The vial was degassed with argon and charged with 160 µL of 1,4-dioxane / water 4:1. The reaction was heated at 75 °C. After 1 h the reaction was cooled down and concentrated under vacuum. The residue was purified by preparative

reverse phase HPLC (eluent: acetonitrile / water 20 to 60% + 0.1% TFA). The title compound containing fractions were concentrated under reduced pressure to provide 9.70 mg of the title compound.

LC-MS (Method 4): Rt = 2.49 min; MS (ESIpos): m/z = 473 $[M+H]^+$

$^1$H-NMR (400 MHz, DMSO-$d_6$) δ [ppm] = 11.97 (s, 1H), 10.67 (s, 1H), 8.17 (s, 1H), 8.12 (d, 1H), 7.47 - 7.40 (m, 3H), 7.38 (d, 1H), 7.34 - 7.31 (m, 1H), 7.16 (t, 2H), 7.00 (dd, 1H), 6.77 (dd, 1H), 6.41 (d, 1H), 4.03 (q, 1H), 3.40 (s, 3H), 1.39 (d, 3H).

**Example 132**

N-[4-(3,5-dimethyl-4-oxo-4,5-dihydro-1H-pyrrolo[3,2-c]pyridin-2-yl)pyridin-2-yl]-2-(4-fluorophenyl)propanamide (Racemate)

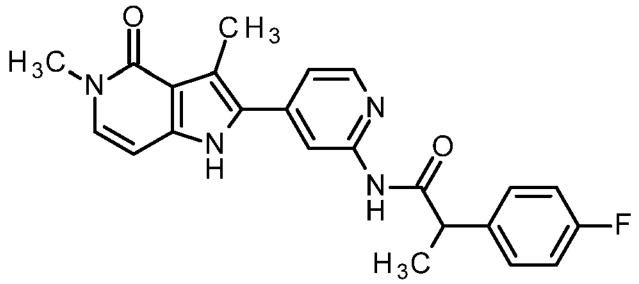

A microwave vial vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), 2,4,6-trimethyl-1,3,5,2,4,6-trioxatriborinane (18.0 µL, 63.9 µmol), XPhos Pd G3 (CAS-RN:[1445085-55-1], 2.69 mg, 3.20 µmol) and $K_3PO_4$ (20.3 mg, 95.9 µmol). The vial was sealed, degassed with argon and charged with 410 µL of 1,4-dioxane / water 4:1. The reaction was heated at 75 °C in a microwave. After 1 h the reaction was cooled down and concentrated under vacuum. The residue was purified by preparative reverse phase HPLC (eluent: acetonitrile / water 20 to 60% + 0.1% TFA). The title compound containing fractions were concentrated and purified by preparative reverse phase HPLC (eluent: acetonitrile / water 30 to 45% + 0.1% TFA). The title compound containing fractions were concentrated under reduced pressure to provide 4.60 mg of the title compound.

LC-MS (Method 4): $R_t$ = 2.21 min; MS (ESIpos): m/z = 405 $[M+H]^+$

$^1$H-NMR (400 MHz, DMSO-$d_6$) δ [ppm] = 11.67 (s, 1H), 10.72 (s, 1H), 8.33 (d, 2H), 7.46 (dd, 2H), 7.31 (d, 1H), 7.28 (dd, 1H), 7.16 (t, 2H), 6.35 (d, 1H), 4.07 (q, 1H), 3.43 (s, 3H), 2.62 (s, 3H), 1.43 (d, 3H).

**Example 133**

N-{4-[3-(5-chloro-2-thienyl)-5-methyl-4-oxo-4,5-dihydro-1H-pyrrolo[3,2-c]pyridin-2-yl]pyridin-2-yl}-4,4-difluoro-2-(4-fluorophenyl)butanamide (Racemate)

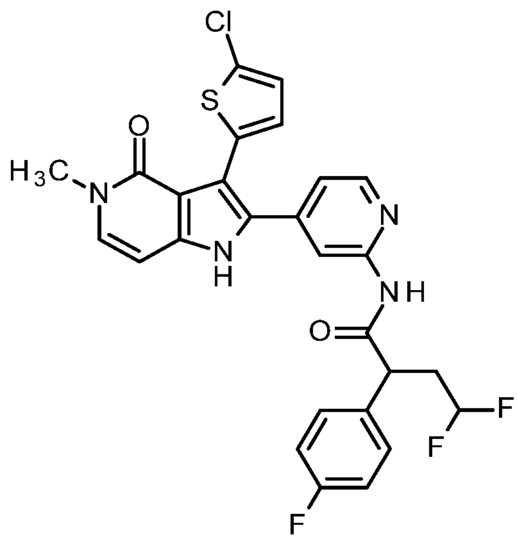

A screw cap vial was charged with N-[4-(3-bromo-5-methyl-4-oxo-4,5-dihydro-1H-pyrrolo[3,2-c]pyridin-2-yl)pyridin-2-yl]-4,4-difluoro-2-(4-fluorophenyl)butanamide (Racemate) (see Intermediate 105, 66.0 mg), (5-chloro-2-thienyl)boronic acid (82.6 mg, 508 μmol), XPhos Pd G3 (CAS-RN:[1445085-55-1], 10.7 mg, 12.7 μmol) and $K_3PO_4$ (80.8 mg, 381 μmol). The vial was degassed with argon and charged with 640 μL of 1,4-dioxane / water 4:1. The reaction was heated at 95 °C. After 16 h the reaction was cooled down and concentrated under vacuum. The residue was purified by preparative reverse phase HPLC (eluent: acetonitrile / water 30 to 70% + 0.1% TFA). The title compound containing fractions were concentrated under reduced pressure to provide 43.6 mg of the title compound.

LC-MS (Method 4): Rt = 2.78 min; MS (ESIpos): m/z = 557 $[M+H]^+$

$^1$H-NMR (400 MHz, DMSO-$d_6$) δ [ppm] = 12.19 (s, 1H), 10.84 (s, 1H), 8.23 (d, 1H), 8.17 (s, 1H), 7.47 (dd, 2H), 7.41 (d, 1H), 7.19 (t, 2H), 7.01 - 6.93 (m, 2H), 6.80 (d, 1H), 6.43 (d, 1H), 6.00 (tt, 1H), 4.19 (dd, 1H), 3.41 (s, 3H), 2.80 - 2.58 (m, 1H), 2.29 - 2.05 (m, 1H).

**Example 134**

N-{4-[5-cyclopropyl-3-(4-fluorophenyl)-4-oxo-4,5-dihydro-1H-pyrrolo[3,2-c]pyridin-2-yl]pyridin-2-yl}-4,4-difluoro-2-(4-fluorophenyl)butanamide (Racemate)

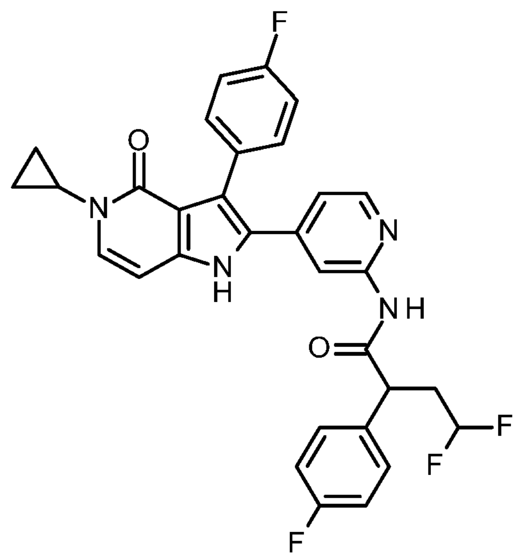

A screw cap vial was charged with N-[4-(3-bromo-5-cyclopropyl-4-oxo-4,5-dihydro-1H-pyrrolo[3,2-c]pyridin-2-yl)pyridin-2-yl]-4,4-difluoro-2-(4-fluorophenyl)butanamide (Racemate) (see Intermediate 111, 70.0 mg), (4-fluorophenyl)boronic acid (35.9 mg, 257 μmol), XPhos Pd G3 (CAS-RN:[1445085-55-1], 10.8 mg, 12.8 pmol) and $K_3PO_4$ (81.6 mg, 385 pmol). The vial was degassed with argon and charged with 640 μL of 1,4-dioxane / water 4:1. The reaction was heated at 80 °C. After 3 h the reaction was cooled down and concentrated under vacuum. The residue was purified by preparative reverse phase HPLC (eluent: acetonitrile / water 30 to 70% + 0.1% TFA). The title compound containing fractions were concentrated under reduced pressure to provide 44.0 mg of the title compound.

LC-MS (Method 4): Rt = 2.83 min; MS (ESIpos): m/z = 561 $[M+H]^+$

$^1$H-NMR (400 MHz, DMSO-$d_6$) δ [ppm] = 12.05 (s, 1H), 10.83 (s, 1H), 8.13 (d, 1H), 8.07 (s, 1H), 7.45 (dd, 2H), 7.29 (dd, 3H), 7.20 (t, 2H), 7.16 - 7.04 (m, 2H), 6.76 (dd, 1H), 6.41 (d, 1H), 5.99 (tt, 1H), 4.26 - 4.12 (m, 1H), 3.19 (tt, 1H), 2.70 (ddt, 1H), 2.31 - 2.01 (m, 1H), 1.04 - 0.85 (m, 2H), 0.78 (dd, 2H).

## Example 135

(-)-N-{4-[5-cyclopropyl-3-(4-fluorophenyl)-4-oxo-4,5-dihydro-1H-pyrrolo[3,2-c]pyridin-2-yl]pyridin-2-yl}-4,4-difluoro-2-(4-fluorophenyl)butanamide (Enatiomer 1)

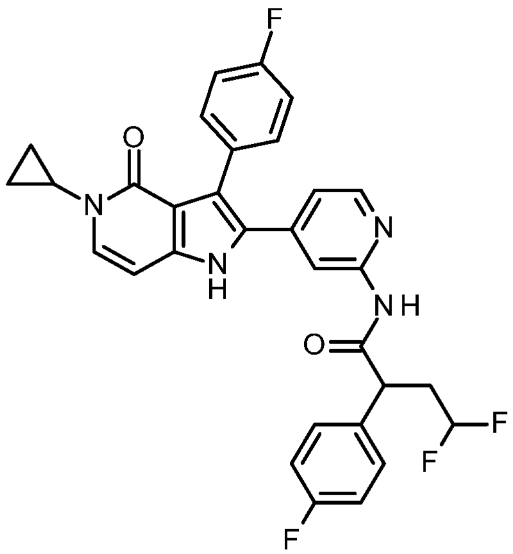

The racemic compound (see Example 134, 36 mg) was separated into enantiomers by preparative chiral HPLC to give enantiomer 1 (16 mg, >99 %ee, see Example 135) and enantiomer 2 (14 mg, >99 % ee, see Example 136).

Preparative chiral HPLC method:
Instrument: PrepCon Labomatic HPLC; Column: YMC Cellulose SB 5μ, 250x30; eluent A: methyl tert-butyl ether + 0.1 vol % diethylamine; eluent B: acetonitrile; isocratic: 90%A+10%B; flow: 60 mL/min; temperature: 25°C; UV: 254 nm

Analytical chiral HPLC method:
Instrument: Thermo Fisher UltiMate 3000; Column: YMC Cellulose SB 3μ, 100x4.6; eluent A: methyl tert-butyl ether + 0.1 vol % diethylamine; eluent B: acetonitrile; isocratic: 90%A+10%B; flow: 1.4 mL/min; temperature: 25°C; UV: 254 nm

Analytical Chiral HPLC (method Example 135): Rt = 2.53 min.

$[\alpha]_D$ = -151.3° (from solution in DMSO, c = 16 mg/mL)
$^1$H NMR (400 MHz, DMSO-$d_6$) δ ppm 0.74 - 0.80 (m, 2 H), 0.90 - 0.98 (m, 2 H), 2.06 - 2.28 (m, 1 H), 2.59 - 2.80 (m, 1 H), 3.15-3.21 (m, 1H), 4.16 (dd, 1 H), 5.74 - 6.17 (m, 1 H), 6.40 (d, 1 H), 6.75 (dd, 1 H), 7.01 - 7.36 (m, 7 H), 7.39

- 7.55 (m, 2 H), 8.00 - 8.16 (m, 2 H), 10.80 (s, 1 H), 12.05 (s, 1 H).

**Example 136**

(+)-N-{4-[5-cyclopropyl-3-(4-fluorophenyl)-4-oxo-4,5-dihydro-1 H-pyrrolo[3,2-c]pyridin-2-yl]pyridin-2-yl}-4,4-difluoro-2-(4-fluorophenyl)butanamide (Enatiomer 2)

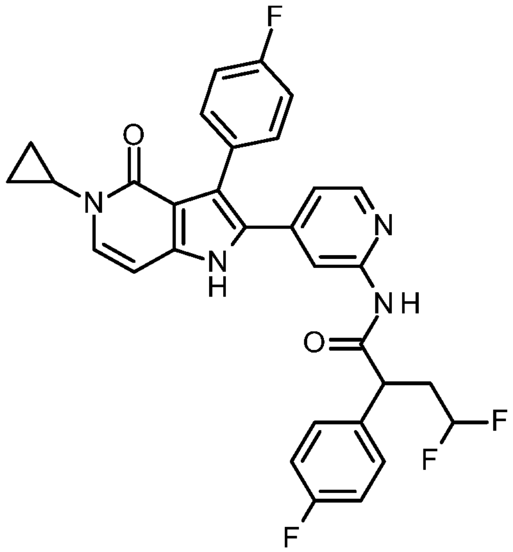

For the preparation of the racemic title compound see Example 134. Separation of enantiomers by preparative chiral HPLC (method see Example 135) gave the title compound (14 mg, >99 %ee).

Analytical Chiral HPLC (method see Example 135): Rt = 3.30 min.

$[\alpha]_D$ = +179.4° (from solution in DMSO, c = 14 mg/mL)

$^1$H NMR (400 MHz, DMSO-$d_6$) $\delta$ ppm 0.71 - 0.81 (m, 2 H), 0.89 - 1.00 (m, 2 H), 2.11 - 2.30 (m, 1 H), 2.60 - 2.77 (m, 1 H), 3.16 - 3.24 (m, 1 H), 4.16 (dd, 1 H), 5.80 - 6.18 (m, 1 H), 6.40 (d, 1 H), 6.74 (dd, 1 H), 7.07 - 7.15 (m, 2 H), 7.15 - 7.23 (m, 2 H), 7.24 - 7.32 (m, 3 H), 7.40 - 7.49 (m, 2 H), 8.08 (s, 1 H), 8.12 (d, 1 H), 10.80 (s, 1 H), 12.04 (s, 1 H).

**Example 137**

N-[4-(5-cyclopropyl-4-oxo-3-phenyl-4,5-dihydro-1H-pyrrolo[3,2-c]pyridin-2-yl)pyridin-2-yl]-4,4-difluoro-2-(4-fluorophenyl)butanamide (Racemate)

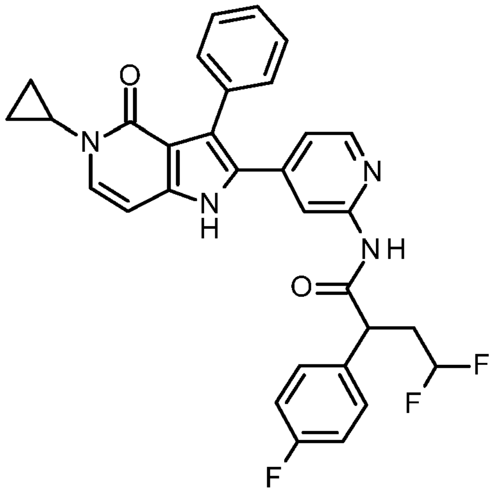

A screw cap vial was charged with a stir bar, N-[4-(3-bromo-5-cyclopropyl-4-oxo-4,5-dihydro-1H-pyrrolo[3,2-c]pyridin-2-yl)pyridin-2-yl]-4,4-difluoro-2-(4-fluorophenyl)butanamide (Racemate) (see Intermediate 111, 100 mg), XPhos Pd G3 (CAS-RN:[1445085-55-1], 15 mg, 0.0183 mmol), phenylboronic acid (45 mg, 0.367 mmol) and potassium phosphate (117 mg, 0.550 mmol). The vial was purged with nitrogen and charged with a mixture of argon-degassed 4:1 1,4-dioxane:water (0.92 mL). The vessel was sealed and placed on an aluminum block preheated at 80 °C. After stirring for 2.5 h, the reaction was cooled to room temperature, filtered over celite, and concentrated. The crude residue was purified by preparative reverse phase HPLC (eluent: 10-100% acetonitrile/water gradient buffered with 0.1% trifluoroacetic acid) to obtain 52 mg of the title compound.

LC-MS (Method 4): $R_t$ = 2.77 min; MS (ESIpos): m/z = 543 $[M+H]^+$

$^1$H NMR (400 MHz, DMSO-$d_6$) $\delta$ 12.02 (s, 1H), 10.83 (s, 1H), 8.11 (s, 1H), 8.06 (d, 1H), 7.52 - 7.42 (m, 2H), 7.31 - 7.24 (m, 6H), 7.23 - 7.18 (m, 2H), 6.66 (dd, 1H), 6.40 (d, 1H), 5.99 (tt, 1H), 4.17 (dd, 1H), 3.18 (tt, 1H), 2.83 - 2.60 (m, 1H), 2.20 (dtt, 1H), 0.99 - 0.88 (m, 2H), 0.84 - 0.70 (m, 2H).

**Example 138**

4,4-difluoro-2-(4-fluorophenyl)-N-{4-[5-methyl-4-oxo-3-(1,3-thiazol-4-yl)-4,5,6,7-tetrahydro-1H-pyrrolo[3,2-c]pyridin-2-yl]pyridin-2-yl}butanamide (Racemate)

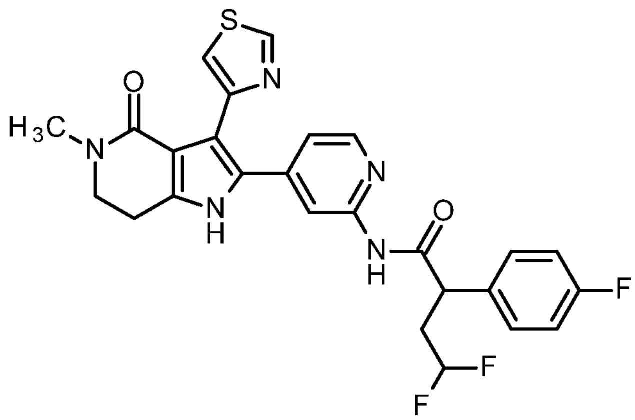

A screw cap vial was charged with N-[4-(3-bromo-5-methyl-4-oxo-4,5,6,7-tetrahydro-1H-pyrrolo[3,2-c]pyridin-2-yl)pyridin-2-yl]-4,4-difluoro-2-(4-fluorophenyl)butanamide (Racemate) (see Intermediate 104, 20.0 mg), 4-(4,4,5,5-tetramethyl-1,3,2-dioxaborolan-2-yl)thiazole (32.4 mg, 153 µmol), XPhos Pd G3 (CAS-RN:[1445085-55-1], 6.47 mg, 7.67 µmol) and $K_3PO_4$ (24.4 mg, 115 µmol). The vial was degassed with argon and charged with 190 µL of 1,4-dioxane

/ water 4:1. The reaction was heated at 110 °C. After 3 h the reaction was cooled down and concentrated under vacuum. The residue was purified by preparative reverse phase HPLC (eluent: acetonitrile / water 20 to 60% + 0.1% TFA). The title compound containing fractions were concentrated under reduced pressure to provide 3.16 mg of the title compound.

LC-MS (Method 4): Rt = 2.26 min; MS (ESIpos): m/z = 526 $[M+H]^+$

$^1$H-NMR (400 MHz, DMSO-$d_6$) δ [ppm] = 11.90 (s, 1H), 10.68 (s, 1H), 8.95 (d, 1H), 8.08 (d, 1H), 7.88 (s, 1H), 7.65 (d, 1H), 7.51 - 7.40 (m, 2H), 7.20 (t, 2H), 6.73 (dd, 1H), 6.19 - 5.76 (m, 1H), 4.15 (t, 1H), 3.56 (t, 2H), 2.94 (t, 2H), 2.87 (s, 3H), 2.76 - 2.58 (m, 1H), 2.28 - 2.15 (m, 1H).

**Example 139**

4,4-difluoro-2-(4-fluorophenyl)-N-{4-[3-(4-fluorophenyl)-5-methyl-4-oxo-4,5,6,7-tetrahydro-1H-pyrrolo[3,2-c]pyridin-2-yl]pyridin-2-yl}butanamide (Racemate)

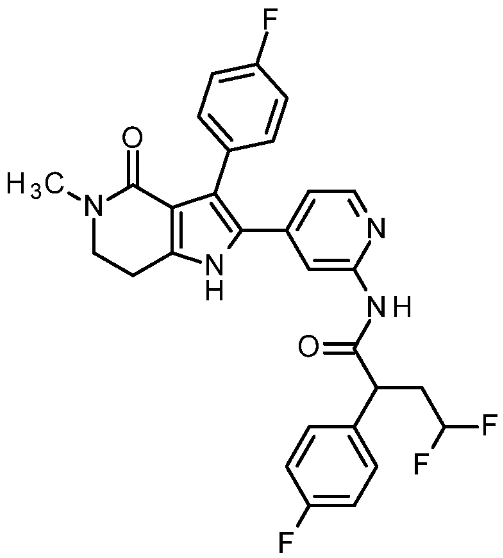

2-(2-Aminopyridin-4-yl)-3-(4-fluorophenyl)-5-methyl-1,5,6,7-tetrahydro-4H-pyrrolo[3,2-c]pyridin-4-one (see Intermediate 114, 250 mg, 743 μmol), 4,4-difluoro-2-(4-fluorophenyl)butanoic acid (Racemate) (CAS-RN:[1538957-14-0], 211 mg, 966 μmol), PyBOP (CAS-RN:[128625-52-5], 967 mg, 1.86 mmol) and N,N-diisopropylethylamine (780 μL, 4.5 mmol) were dissolved in 12 mL N,N-dimethylacetamide and were stirred at room temperature over night under nitrogen atmosphere. Additional Pybop (502.8 mg) was added and stirring continued at rt for 16 h. Aqueous saturated sodium hydrogencarbonate solution was added to the reaction mixture. It was diluted with ethyl acetate and the aqueous layer was extracted with ethyl acetate three times. The combined organic layers were dried over anhydrous sodium sulfate, filtered and concentrated under reduced pressure. The crude product was purified by chromatography (50 g column, silica ULTRA, gradient: hexane / ethyl acetate 25-100% ethyl acetate) to provide the title compound: 220 mg.

LC-MS (Method 1): Rt = 1.17 min; MS (ESIneg): m/z = 535 $[M-H]^-$

$^1$H-NMR (400 MHz, DMSO-$d_6$) δ [ppm]: 1.138 (0.89), 1.154 (1.13), 1.172 (2.29), 1.190 (1.11), 1.987 (3.80), 2.084 (14.92), 2.116 (0.59), 2.326 (0.49), 2.518 (2.08), 2.522 (1.34), 2.664 (0.67), 2.669 (0.77), 2.673 (0.73), 2.728 (1.73), 2.843 (16.00), 2.888 (2.11), 2.901 (0.65), 2.917 (1.47), 2.935 (3.11), 2.952 (1.72), 3.535 (1.92), 3.552 (4.07), 3.569 (1.71), 4.017 (0.88), 4.035 (0.89), 4.119 (0.65), 4.133 (0.77), 4.143 (0.76), 4.156 (0.64), 5.829 (0.61), 5.959 (0.53), 5.970 (1.23), 5.981 (0.58), 6.110 (0.54), 6.617 (2.17), 6.622 (2.01), 6.630 (2.04), 6.635 (2.01), 7.039 (2.00), 7.045 (0.81), 7.056 (0.99), 7.062 (4.61), 7.067 (1.08), 7.079 (0.89), 7.084 (2.85), 7.155 (0.54), 7.162 (4.60), 7.168 (1.70), 7.177 (3.66), 7.185 (7.04), 7.199 (2.48), 7.207 (2.78), 7.415 (2.29), 7.421 (1.01), 7.429 (2.51), 7.437 (2.32), 7.445 (0.89), 7.451 (1.98), 7.951 (2.69), 8.032 (2.82), 8.045 (2.66), 10.704 (2.96), 11.863 (2.22).

## Example 140

4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]-N-(2-methoxyethyl)benzamide

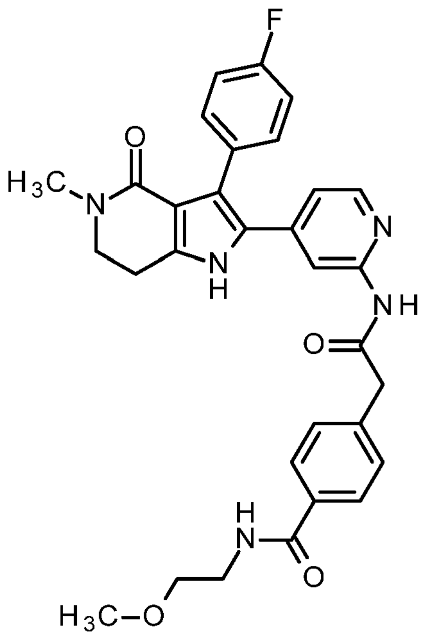

To a solution of 4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]benzoic acid (see Intermediate 116, 170 mg) in DMF (4.7 mL), potassium carbonate (170 mg, 1.23 mmol), HATU (CAS-RN:[148893-10-1], 169 mg, 443 μmol) and 2-methoxyethan-1-amine (CAS-RN:[38256-93-8], 51 μL, 96 % purity, 550 μmol) were added and stirred at room temperature for 16h. Aqueous saturated sodium hydrogencarbonate solution was added to the reaction mixture. It was diluted with ethyl acetate and the aqueous layer was extracted with ethyl acetate three times. The combined organic layers were dried over anhydrous sodium sulfate, filtered and concentrated under reduced pressure. The crude product was diluted with methanol. A solid precipitated, it was filtered off, washed with MeOH and dried to provide the title compound: 37 mg.

LC-MS (Method 1): Rt = 0.87 min; MS (ESIpos): m/z = 556 $[M-H]^+$

$^1$H-NMR (400 MHz, DMSO-$d_6$): δ [ppm] = 11.84 (s, 1H), 10.66 (s, 1H), 8.48 (t, 1H), 8.08-8.03 (m, 1H), 8.01 (s, 1H), 7.84-7.77 (m, 2H), 7.39 (d, 2H), 7.23-7.16 (m, 2H), 7.11-7.03 (m, 2H), 6.59 (dd, 1H), 3.74 (s, 2H), 3.54 (t, 2H), 3.48-3.38 (m, 4H), 3.26 (s, 3H), 2.95-2.89 (m, 2H), 2.84 (s, 3H)

## Example 141

4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]-N-(2-methoxyethyl)-N-methylbenzamide

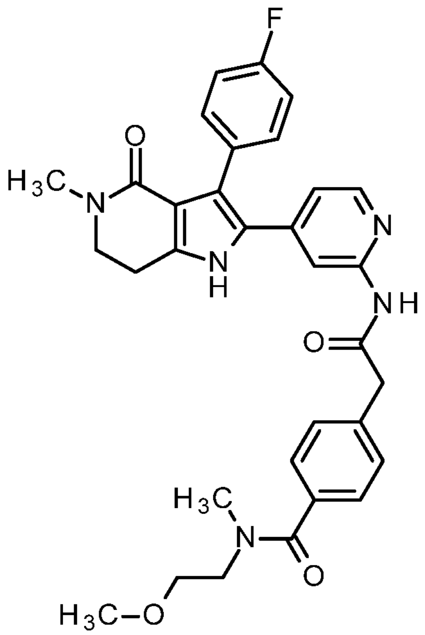

To a solution of 4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]benzoic acid (see Intermediate 116, 170 mg) in DMF (4.7 mL), potassium carbonate (170 mg, 1.23 mmol), HATU (CAS-RN:[148893-10-1], 169 mg, 443 μmol) and 2-methoxy-N-methylethan-1-amine (CAS-RN:[109-85-3], 57 μL, 98 % purity, 550 μmol) were added and stirred at room temperature for 16h. Aqueous saturated sodium hydrogencarbonate solution was added to the reaction mixture. It was diluted with ethyl acetate and the aqueous layer was extracted with ethyl acetate three times. The combined organic layers were dried over anhydrous sodium sulfate, filtered and concentrated under reduced pressure. The crude product was purified by preparative reverse phase HPLC and followed by extraction with ethyl acetate and aqueous saturated sodium hydrogencarbonate solution to provide 90.1 mg of the the title compound.

LC-MS (Method 1): Rt = 0.90 min; MS (ESIpos): m/z = 570 $[M+H]^+$

$^1$H-NMR (400 MHz, DMSO-$d_6$): δ [ppm] = 11.84 (s, 1H), 10.67 (s, 1H), 8.05 (d, 1H), 8.02 (s, 1H), 7.41-7.30 (m, 4H), 7.23-7.16 (m, 2H), 7.11-7.03 (m, 2H), 6.59 (dd, 1H), 3.72 (s, 2H), 3.64-3.49 (m, 4H), 3.46-3.35 (m, 2H), 3.31-3.14 (m, 3H), 2.98-2.88 (m, 5H), 2.84 (s, 3H).

**Example 142**

4-[2-({4-[3-(4-fluorophenyl)-5-methyl-4-oxo-4,5-dihydro-1H-pyrrolo[3,2-c]pyridin-2-yl]pyridin-2-yl}amino)-2-oxoethyl]-N-(2-methoxyethyl)benzamide

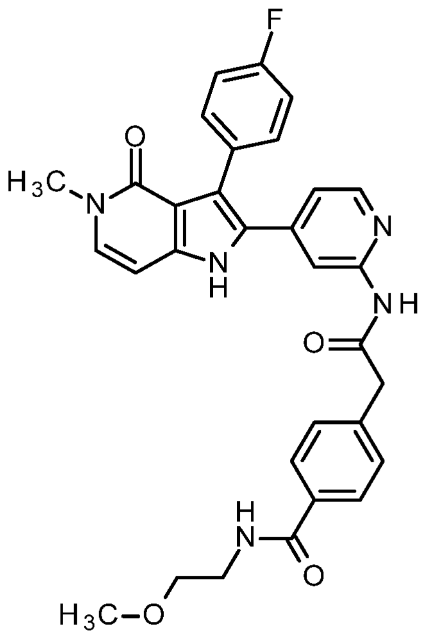

To 4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]-N-(2-methoxyethyl)benzamide (see Example 140, 55.0 mg) in 1,4-dioxane (3.2 mL) 4,5-dichloro-3,6-dioxocyclohexa-1,4-diene-1,2-dicarbonitrile (22.5 mg, 99.0 μmol) was added. The reaction mixture was stirred at 80°C for 16 hours. Then further 4,5-dichloro-3,6-dioxocyclohexa-1,4-diene-1,2-dicarbonitrile (22.5 mg) was added and it was stirred at 80°C for 16 hours again. Afterwards, further 4,5-dichloro-3,6-dioxocyclohexa-1,4-diene-1,2-dicarbonitrile (22.5 mg) was added and it was stirred at 80°C for further 16 hours. Aqueous saturated sodium hydrogencarbonate solution was added to the reaction mixture. It was diluted with ethyl acetate and the aqueous layer was extracted with ethyl acetate three times. The combined organic layers were dried over anhydrous sodium sulfate, filtered and concentrated under reduced pressure. The crude product was purified by chromatography (10 g column, silica ULTRA, gradient: dichloromethane/ethanol 2-12% ethanol) and preparative thin layer chromatography (dichloromethane/methanol 10% methanol) to provide the title compound: 3.1 mg.

LC-MS (Method 1): Rt = 0.90 min; MS (ESIpos): m/z = 554 $[M+H]^+$

$^1$H-NMR (400 MHz, DMSO-$d_6$): δ [ppm] = 12.05 (s, 1H), 10.75 (s, 1H), 8.48 (t, 1H), 8.16-8.10 (m, 2H), 7.81 (d, 2H), 7.39 (dd, 3H), 7.31-7.25 (m, 2H), 7.14-7.08 (m, 2H), 6.73 (dd, 1H), 6.42 (d, 1H), 3.79-3.73 (m, 2H), 3.45-3.40 (m, 4H), 3.38 (s, 3H), 3.26 (s, 3H).

**Example 143**

4-[2-({4-[3-(4-fluorophenyl)-5-methyl-4-oxo-4,5-dihydro-1H-pyrrolo[3,2-c]pyridin-2-yl]pyridin-2-yl}amino)-2-oxoethyl]-N-(2-methoxyethyl)-N-methylbenzamide

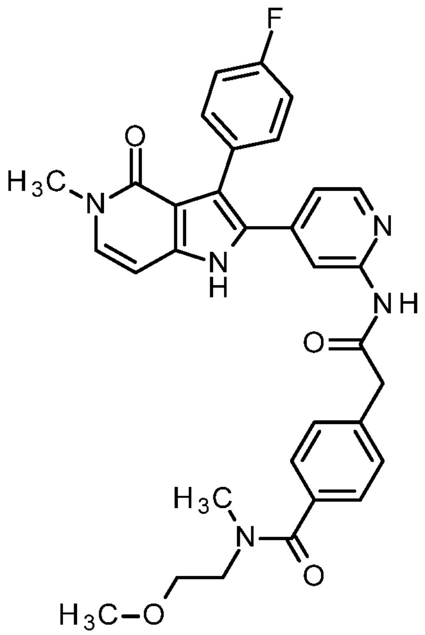

To 4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]-N-(2-methoxyethyl)-N-methylbenzamide (see Example 141, 38.0 mg, 66.7 μmol) in 1,4-dioxane (2.2 mL) 4,5-dichloro-3,6-dioxocyclohexa-1,4-diene-1,2-dicarbonitrile (15.1 mg, 66.7 μmol) was added. The reaction mixture was stirred at 80°C for 16 hours. Then further 4,5-dichloro-3,6-dioxocyclohexa-1,4-diene-1,2-dicarbonitrile (15.1 mg) was added and it was stirred at 80°C for 16 hours again. Afterwards, further 4,5-dichloro-3,6-dioxocyclohexa-1,4-diene-1,2-dicarbonitrile (15.1 mg) was added and it was stirred at 80°C for further 16 hours. Aqueous saturated sodium hydrogencarbonate solution was added to the reaction mixture. It was diluted with ethyl acetate and the aqueous layer was extracted with ethyl acetate three times. The combined organic layers were dried over anhydrous sodium sulfate, filtered and concentrated under reduced pressure. The crude product was purified by preparative reverse phase HPLC (eluent: acetonitrile / water with formic acid as additive) to provide 8.2 mg of the title compound as formic acid -salt

LC-MS (Method 1): $R_t$ = 0.93 min; MS (ESIpos): m/z = 568 $[M+H]^+$

$^1$H-NMR (400 MHz, DMSO-$d_6$) δ [ppm]: 0.851 (0.47), 1.137 (7.02), 1.232 (2.44), 2.115 (3.12), 2.323 (1.00), 2.326 (1.33), 2.331 (1.00), 2.539 (1.14), 2.664 (1.00), 2.669 (1.35), 2.673 (1.00), 2.686 (0.60), 2.888 (0.44), 2.954 (2.60), 3.178 (2.05), 3.384 (16.00), 3.556 (0.95), 3.737 (4.60), 6.410 (2.95), 6.428 (2.98), 6.717 (1.53), 6.720 (1.44), 6.733 (1.53), 7.086 (1.81), 7.109 (3.79), 7.131 (2.23), 7.263 (2.28), 7.278 (2.72), 7.285 (2.40), 7.299 (1.95), 7.324 (1.53), 7.344 (4.35), 7.361 (2.70), 7.375 (4.07), 7.393 (3.02), 8.130 (3.16), 8.142 (3.72), 8.215 (1.44), 10.763 (2.74), 12.033 (1.14).

## Example 144

N-{4-[3-(4-fluorophenyl)-5-methyl-4-oxo-4,5,6,7-tetrahydro-1H-pyrrolo[3,2-c]pyridin-2-yl]pyridin-2-yl}-2-[4-(piperazine-1-carbonyl)phenyl]acetamide

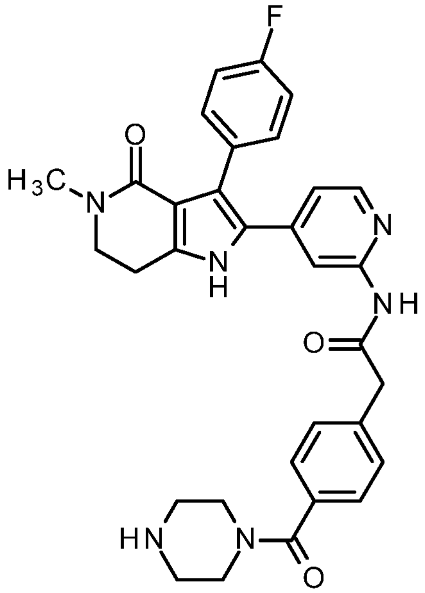

To a solution of tert-butyl 4-{4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]benzoyl}piperazine-1-carboxylate (see Intermediate 117, 473 mg) in dichloromethane (25 mL), HCl in dioxane (8.9 mL, 4.0 M, 36 mmol) was added. The mixture was stirred for 3 hours at room temperature. The reaction mixture was concentrated under reduced pressure, suspended in dichloromethane, filtered and washed with dichloromethane to provide 394 mg of the title compound as an HCl salt.

LC-MS (Method 1): Rt = 0.67 min; MS (ESIpos): m/z = 567 $[M+H]^+$

$^1$H-NMR (400 MHz, DMSO-$d_6$) $\delta$ [ppm]: 1.232 (0.45), 2.323 (0.71), 2.327 (0.94), 2.331 (0.73), 2.669 (1.01), 2.841 (16.00), 2.923 (1.72), 2.940 (3.68), 2.957 (1.95), 3.142 (2.67), 3.385 (0.62), 3.535 (2.30), 3.552 (4.41), 3.570 (2.29), 4.414 (1.25), 5.760 (12.77), 6.729 (1.57), 6.740 (1.56), 7.065 (2.06), 7.087 (4.57), 7.110 (2.89), 7.194 (2.76), 7.208 (3.18), 7.215 (2.63), 7.230 (2.04), 7.395 (1.46), 7.417 (8.98), 7.424 (10.15), 7.445 (1.54), 7.868 (2.42), 8.083 (2.90), 8.097 (2.77), 9.230 (1.54), 11.028 (0.81), 12.019 (1.78).

## Example 145

2-(4-fluorophenyl)-N-{4-[3-(6-fluoropyridin-3-yl)-5-methyl-4-oxo-4,5-dihydro-1H-pyrrolo[3,2-c]pyridin-2-yl]pyridin-2-yl}propanamide (Racemate)

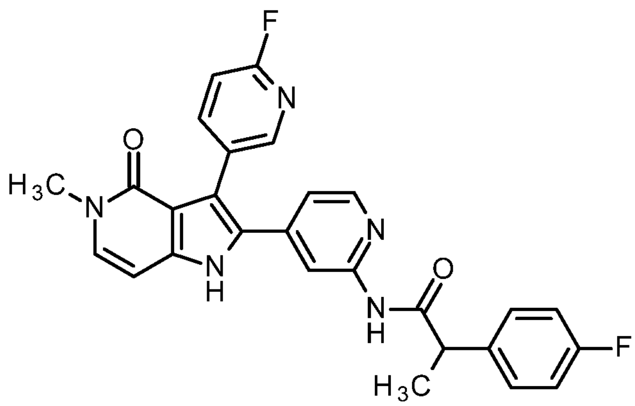

A screw cap vial was charged with N-[4-(3-bromo-5-methyl-4-oxo-4,5-dihydro-1H-pyrrolo[3,2-c]pyridin-2-yl)pyridin-2-yl]-2-(4-fluorophenyl)propanamide (Racemate) (see Intermediate 102, 15.0 mg), (6-fluoro-3-pyridyl)boronic acid (9.01 mg, 63.9 µmol), XPhos Pd G3 (CAS-RN:[1445085-55-1], (2.69 mg, 3.20 µmol) and $K_3PO_4$ (20.3 mg, 95.9 µmol).

The vial was degassed with argon and charged with 160 μL of 1,4-dioxane / water 4:1. The reaction was heated at 75 °C. After 16 h the reaction was cooled down and concentrated under vacuum. The residue was purified by preparative reverse phase HPLC (eluent: acetonitrile / water 25 to 55% + 0.1% TFA). The target compound containing fractions were concentrated under reduced pressure to provide 7.90 mg of the title compound.

LC-MS (Method 4): Rt = 2.36 min; MS (ESIpos): m/z = 486 $[M+H]^+$

$^1$H-NMR (400 MHz, DMSO-$d_6$) δ [ppm] = 12.18 (s, 1H), 10.65 (s, 1H), 8.18 (d, 1H), 8.11 (s, 1H), 8.03 (d, 1H), 7.88 (td, 1H), 7.46 - 7.34 (m, 3H), 7.20 - 7.06 (m, 3H), 6.85 (dd, 1H), 6.46 (d, 1H), 4.00 (q, 1H), 3.41 (s, 3H), 1.36 (d, 3H).

## Example 146

2-(4-fluorophenyl)-N-{4-[5-methyl-4-oxo-3-(pyridin-2-yl)-4,5-dihydro-1H-pyrrolo[3,2-c]pyridin-2-yl]pyridin-2-yl}acetamide

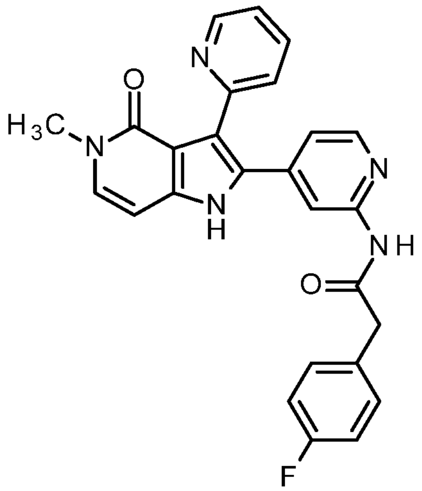

To a solution of N-[4-(3-bromo-5-methyl-4-oxo-4,5-dihydro-1H-pyrrolo[3,2-c]pyridin-2-yl)pyridin-2-yl]-2-(4-fluorophenyl)acetamide (see Intermediate 122, 60.0 mg) and 2-(tributylstannyl)-pyridine (97.0 mg, 264 μmol) in 1,4-dioxane (6.0 mL) were added tetrakis(triphenylphosphine)-palladium(0) (15.2 mg, 13.2 μmol) and lithium chloride (16.8 mg, 395 μmol) in one portion at room temperature. The reaction mixture was stirred at 100 °C for 16 hours under nitrogen atmosphere. The reaction mixture (combined with two other batches starting from 30 mg and 60 mg) was concentrated in reduced pressure to give a residue. The residue was diluted with water and extracted with ethyl acetate. The organic phase was washed with brine, dried over anhydrous sodium sulfate and filtered. The filtrate was concentrated and purified by preparative-TLC (ethyl acetate: methanol = 10: 1) to give crude product. The crude product was purified by preparative HPLC (Instrument: Gilson-281; Column: Phenomenex Synergi C18 150*25*10 μm; eluent A: water (0.225% formic acid), eluent B: acetonitrile; gradient: 0-10 min 18-48% B; flow 25 mL/min; temperature: room temperature; Detector: UV 220/254 nm) to give 29.3 mg of the title compound as formic acid salt as a yellow solid.

LC-MS (Method 6): Rt = 0.794 min; MS (ESIpos): m/z = 454.3 $[M+H]^+$.

$^1$H NMR (400 MHz, DMSO-$d_6$): δ [ppm] = 12.09 (s, 1H), 10.67 (s, 1H), 8.42 (d, 1H), 8.12 (d, 1H), 8.07 (s, 1H), 7.75 (dt, 1.6 Hz, 1H), 7.53 (d, 1H), 7.40 (d, 1H), 7.38-7.33 (m, 2H), 7.29-7.24 (m, 1H), 7.19-7.13 (m, 2H), 6.75 (dd, 1.6 Hz, 1H), 6.45 (d, 1H), 3.68 (s, 2H), 3.40 (s, 3H).

## Example 147

(rac)-N-{4-[7-(2,2-difluoroethyl)-5-methyl-4-oxo-3-phenyl-4,5-dihydro-1H-pyrrolo[3,2-c]pyridin-2-yl]pyridin-2-yl}-4,4-difluoro-2-(4-fluorophenyl)butanamide (Racemate)

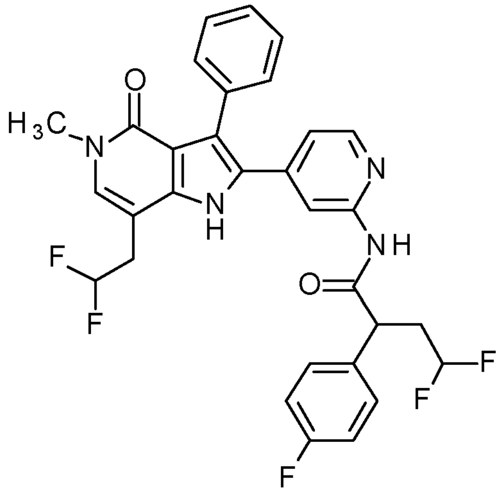

(rac)-N-{4-[3-Bromo-7-(2,2-difluoroethyl)-5-methyl-4-oxo-4,5-dihydro-1H-pyrrolo[3,2-c]pyridin-2-yl]pyridin-2-yl}-4,4-difluoro-2-(4-fluorophenyl)butanamide (Racemate) (see Intermediate 134, 120 mg), phenylboronic acid (37.6 mg, 309 μmol), potassium phosphate (131 mg, 617 μmol) and Methanesulfonato(2-dicyclohexylphosphino-2',4',6'-tri-i-propyl-1,1'-biphenyl)(2'-amino-1,1'-biphenyl-2-yl)palladium(II) (17.4 mg, 20.6 μmol; CAS-RN:[1445085-55-1]) were suspended in a degassed mixture of 0.97 mL 1,4-dioxane and 0.24 mL water and heated at 75 °C for 1.5 hours. The reaction mixture was diluted with a mixture of dichloromethane/isopropanol (7:3) and water. The layers were separated and the aqueous layer was extracted with dichloromethane/isopropanol once. The combined organic layers were dried using a waterresistant filter and concentrated under reduced pressure. The crude product was purified by flash chromatography (10 g spheric silica column, gradient dichloromethane/ethanol 1-25%) to provide the target compound in 97% purity: 77 mg.

$^1$H-NMR (400 MHz, DMSO-d6) δ [ppm] = 2.12 - 2.29 (m, 1 H), 2.61 - 2.77 (m, 1 H), 3.21 - 3.32 (m, 2 H), 3.39 (s, 3 H), 4.17 (dd, 1 H), 5.79 - 6.44 (m, 2 H), 6.70 - 6.76 (m, 1 H), 7.17 - 7.27 (m, 7 H), 7.36 (s, 1 H), 7.43 - 7.50 (m, 2 H), 8.06 - 8.17 (m, 2 H), 10.81 (s, 1 H), 11.94 (s, 1 H).

## Example 148

N-{4-[7-(2,2-difluoroethyl)-5-methyl-4-oxo-3-phenyl-4,5-dihydro-1H-pyrrolo[3,2-c]pyridin-2-yl]pyridin-2-yl}-4,4-difluoro-2-(4-fluorophenyl)butanamide (Enantiomer 1)

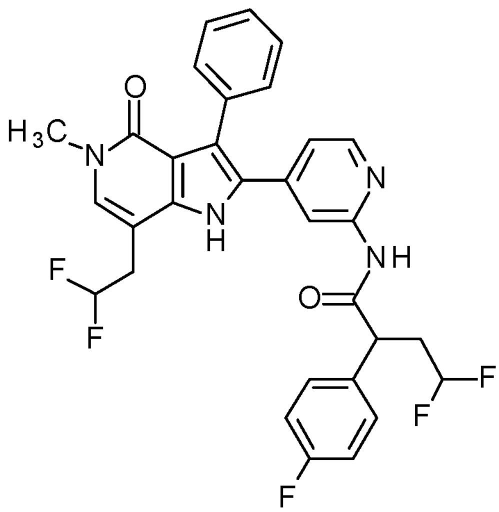

The racemic compound (see Example 147, 66 mg) was separated into the enantiomers by preparative chiral HPLC to give enantiomer 1 (19 mg, 96.3 % ee, see Example 148) and enantiomer 2 (20 mg, 92.3 % ee, see Example-

Example 149149).

Preparative chiral HPLC method:
Instrument: PrepCon Labomatic HPLC-3; Column: Chiralpak IG 5 μ, 250x30; eluent A: methyl tert-butyl ether + 0.1 vol % diethylamine; eluent B: acetonitrile; isocratic: 95%A+5%B; flow: 80 mL/min; temperature: 25 °C; UV: 254 nm;

Analytical chiral HPLC method:
Instrument: Thermo Fisher UltiMate 3000; Column: Chiralpak IG 3 μ, 100x4.6; eluent A: methyl tert-butyl ether + 0.1 vol % diethylamine; eluent B: acetonitrile; isocratic: 95%A+5%B; flow: 1.4 mL/min; temperature: 25°C; UV: 254 nm;

Analytical Chiral HPLC (method see Example 148): Rt = 1.72 min.

LC-MS (Method 2): Rt = 1.22 min; MS (ESIpos): m/z = 581 $[M+H]^+$

$^1$H-NMR (400 MHz, DMSO-$d_6$) δ [ppm] = 2.11 - 2.27 (m, 1 H) 2.67 - 2.78 (m, 1 H) 3.22 - 3.32 (m, 2 H) 3.37 - 3.42 (m, 3 H) 4.07 - 4.23 (m, 1 H) 5.78 - 6.12 (m, 1 H) 6.12 - 6.45 (m, 1 H) 6.67 - 6.80 (m, 1 H) 7.16 - 7.31 (m, 7 H) 7.33 - 7.39 (m, 1 H) 7.41 - 7.51 (m, 2 H) 8.05 - 8.16 (m, 2 H) 10.63 - 10.88 (m, 1 H) 11.86 - 12.01 (m, 1 H).

## Example 149

N-{4-[7-(2,2-difluoroethyl)-5-methyl-4-oxo-3-phenyl-4,5-dihydro-1H-pyrrolo[3,2-c]pyridin-2-yl]pyridin-2-yl}-4,4-difluoro-2-(4-fluorophenyl)butanamide (Enantiomer 2)

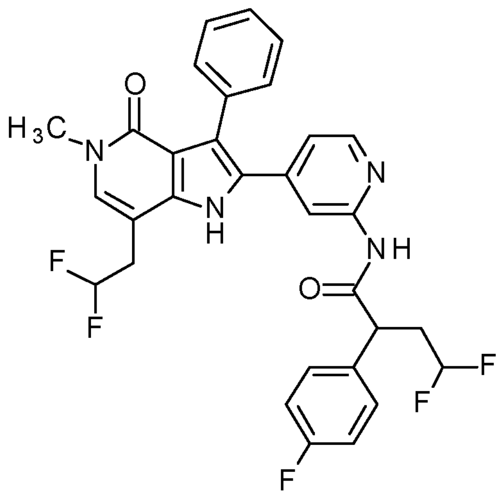

For the preparation of the racemic title compound see Example 147. Separation of the enantiomers by preparative chiral HPLC (method see Example 148Example 148) gave the title compound (20 mg).
Analytical Chiral HPLC (method see Example 148): Rt = 2.38 min.

LC-MS (Method 2): Rt = 1.22 min; MS (ESIpos): m/z = 581 $[M+H]^+$

$^1$H-NMR (400 MHz, DMSO-$d_6$) δ [ppm] = 2.11 - 2.27 (m, 1 H) 2.67 - 2.78 (m, 1 H) 3.22 - 3.32 (m, 2 H) 3.37 - 3.42 (m, 3 H) 4.07 - 4.23 (m, 1 H) 5.78 - 6.12 (m, 1 H) 6.12 - 6.45 (m, 1 H) 6.67 - 6.80 (m, 1 H) 7.16 - 7.31 (m, 7 H) 7.33 - 7.39 (m, 1 H) 7.41 - 7.51 (m, 2 H) 8.05 - 8.16 (m, 2 H) 10.63 - 10.88 (m, 1 H) 11.86 - 12.01 (m, 1 H).

**Example 150**

(rac)-4,4-difluoro-2-(4-fluorophenyl)-N-[4-(5,7,7-trimethyl-4-oxo-3-phenyl-4,5,6,7-tetrahydro-1 H-pyrrolo[3,2-c]pyridin-2-yl)pyridin-2-yl]butanamide (Racemate)

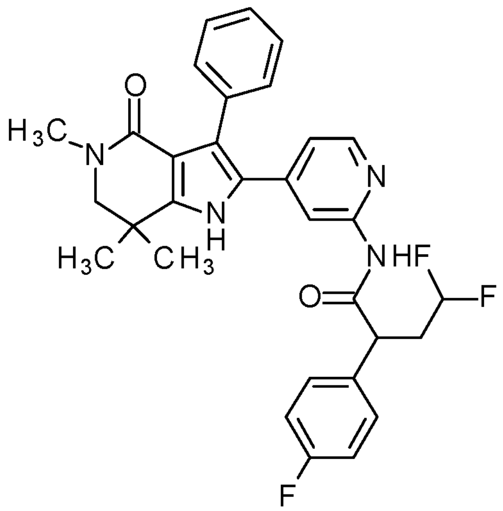

(rac)-N-[4-(3-Bromo-5,7,7-trimethyl-4-oxo-4,5,6,7-tetrahydro-1H-pyrrolo[3,2-c]pyridin-2-yl)pyridin-2-yl]-4,4-difluoro-2-(4-fluorophenyl)butanamide (see Intermediate 143, 70.0 mg), phenylboronic acid (23.3 mg, 191 µmol), potsassium phosphate (81.1 mg, 382 µmol) and methanesulfonato(2-dicyclohexylphosphino-2',4',6'-tri-i-propyl-1,1'-biphenyl)(2'-amino-1,1'-biphenyl-2-yl)palladium(II) (10.8 mg, 12.7 µmol; CAS-RN:[1445085-55-1]) were dissolved in 600 µL 1,4-dioxane (600 µl) and 150 µL water (150 µl) and stirred at 75 °C for 5 hours under nitrogen atmosphere. The reaction mixture was diluted with dichloromethane and water, it was stirred for 5 minutes, filtered through a silicone coated filter and concentrated under reduced pressure. The crude product was purified by HPLC to provide the target compound in 99% purity: 36 mg.

LC-MS (Method 2): Rt = 1.23 min; MS (ESIpos): m/z = 547 $[M+H]^+$

$^1$H-NMR (400 MHz, DMSO-d6) δ [ppm] = 1.34 (d, 6 H), 2.12 - 2.26 (m, 1 H), 2.62 - 2.75 (m, 1 H), 2.86 (s, 3 H), 3.31 (s, 2 H), 4.15 (dd, 1 H), 5.80 - 6.16 (m, 1 H), 6.54 - 6.61 (m, 1 H), 7.11 - 7.26 (m, 7H), 7.42 - 7.51 (m, 2 H), 7.95 - 8.04 (m, 2 H), 10.72 (s, 1 H), 11.59 (s, 1 H).

**Example 151**

(-)-4,4-difluoro-2-(4-fluorophenyl)-N-[4-(5,7,7-trimethyl-4-oxo-3-phenyl-4,5,6,7-tetrahydro-1H-pyrrolo[3,2-c]pyridin-2-yl)pyridin-2-yl]butanamide (Enantiomer 1)

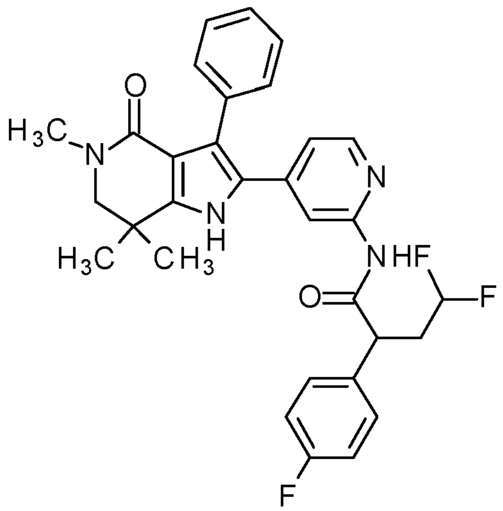

The racemic compound (see Example 150, 23 mg) was separated into the enantiomers by preparative chiral HPLC to give enantiomer 1 (10 mg, >99.9 % ee, see Example 151) and enantiomer 2 (10 mg, >99.9 % ee, see Example 152).

Preparative chiral HPLC method:
Instrument: Instrument: PrepCon Labomatic HPLC-4; Column: YMC Cellulose SB 10 μ, 250x50; eluent A: hexane + 0.1 vol % diethylamine; eluent B: ethanol; isocratic: 90%A+10%B; flow: 100 mL/min; temperature: 25°C; UV: 254 nm;
Analytical chiral HPLC method:
Instrument: Thermo Fisher UltiMate 3000; Column: YMC Cellulose SB 3 μ, 100x4.6; eluent A: hexane + 0.1 vol % diethylamine; eluent B: ethanol; isocratic: 90%A+10%B; flow: 1.4 mL/min; temperature: 25°C; UV: 254 nm;

Analytical Chiral HPLC (method see Example 151): Rt = 2.56 min.

$[a]_D$ = -150,34° (from solution in DMSO, c = 2.8 mg/mL)
$^1$H-NMR (400 MHz, DMSO-d6) δ [ppm] = 1.34 (d, 6 H), 2.08 - 2.29 (m, 1 H), 2.59 - 2.77 (m, 1 H), 2.86 (s, 3 H), 3.31 (s, 2 H), 4.15 (br dd, 1 H), 5.75 - 6.18 (m, 1 H), 6.52 - 6.61 (m, 1 H), 7.09 - 7.27 (m, 7 H), 7.40 - 7.51 (m, 2 H), 8.00 (d, 2 H), 10.72 (s, 1 H), 11.60 (s, 1 H).

## Example 152

(+)-4,4-difluoro-2-(4-fluorophenyl)-N-[4-(5,7,7-trimethyl-4-oxo-3-phenyl-4,5,6,7-tetrahydro-1H-pyrrolo[3,2-c]pyridin-2-yl)pyridin-2-yl]butanamide (Enantiomer 2)

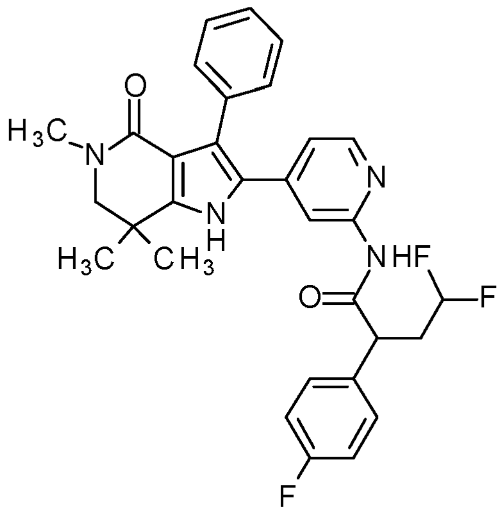

For the preparation of the racemic title compound see Example 150. Separation of the enantiomers by preparative chiral HPLC (method see Example 151) gave the title compound (10 mg).

Analytical Chiral HPLC (method see Example 151): Rt = 3.39 min.

$[a]_D$ = 167,11° (from solution in DMSO, c = 2.8 mg/mL)

$^1$H-NMR (400 MHz, DMSO-d6) δ [ppm] = 1.34 (d, 6 H), 2.12 - 2.28 (m, 1 H), 2.60 - 2.79 (m, 1 H), 2.86 (s, 3 H), 3.31 (s, 2 H), 4.15 (br dd, 1 H), 5.79 - 6.17 (m, 1 H), 6.52 - 6.61 (m, 1 H), 7.09 - 7.28 (m, 7 H), 7.40 - 7.50 (m, 2 H), 8.00 (d, 2 H), 10.72 (s, 1 H), 11.60 (s, 1 H).

## Example 153

(Rac)-N-[4-(5-methyl-4-oxo-3-phenyl-4,5,6,7-tetrahydro-1H-pyrrolo[3,2-c]pyridin-2-yl)pyridin-2-yl]-2-(pyridin-4-yl)propanamide (Racemate)

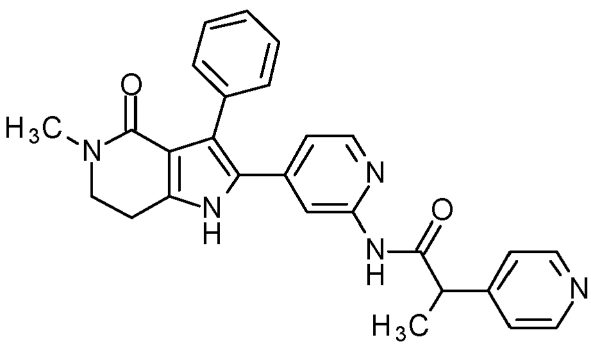

2-(2-aminopyridin-4-yl)-5-methyl-3-phenyl-1,5,6,7-tetrahydro-4H-pyrrolo[3,2-c]pyridin-4-one (see Intermediate 3, 194 mg), (rac)-sodium 2-(pyridin-4-yl)propanoate (Racemate) (211 mg, 1.22 mmol), N,N-diisopropylethylamine (640 μL, 3.7 mmol) and Pybop (951 mg, 1.83 mmol) were dissolved in 5.7 mL DMA and stirred at rt under nitrogen atmosphere overnight. To the reaction mixture saturated aqueous sodium hydrogencarbonate solution and dichloromethane were added. The layers were separated and the aqueous layer was extracted with dichloromethane once. The combined organic layers were dried using a waterresistant filter. The clear filtrate was concentrated under reduced pressure. The crude product was purified by preparative HPLC under basic conditions, flash chromatography (10g silica ultra column, gradient dichloromethane/ethanol 0-20%) and precipitation from dichloromethane to provide the target compound in 94% purity: 132 mg.

LC-MS (Method 2): Rt = 0.92 min; MS (ESIpos): m/z = 453 $[M+H]^+$

$^1$H-NMR (400 MHz, DMSO-d6) δ [ppm] = 1.40 (d, 3H), 2.84 (s, 3H), 2.93 (t, 2H), 3.55 (t, 2H), 4.02 (q, 1H), 6.51 (dd, 1H), 7.10 - 7.20 (m, 2H), 7.21 - 7.31 (m, 3H), 7.36 (d, 2H), 7.98 (d, 1H), 8.04 (s, 1H), 8.45 - 8.58 (m, 2H), 10.68 (s, 1H), 11.84 (s, 1H). - contains dichloromethane

**Example 154**

N-[4-(5-methyl-4-oxo-3-phenyl-4,5,6,7-tetrahydro-1H-pyrrolo[3,2-c]pyridin-2-yl)pyridin-2-yl]-2-(pyridin-4-yl)propanamide (Entantiomer 1)

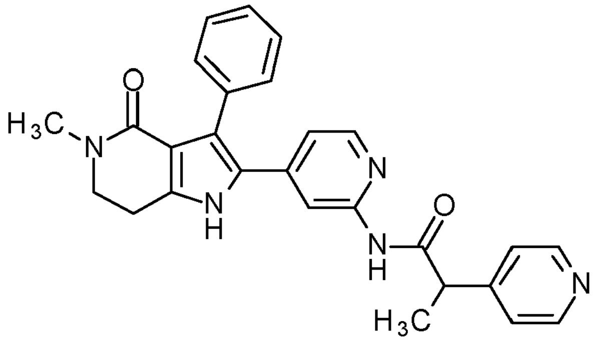

The racemic compound (see Example 153, 131 mg) was separated into the enantiomers by preparative chiral HPLC to give enantiomer 1 (55 mg, 100 % ee, see 154) and enantiomer 2 (57 mg, 99.1 % ee, see 155).

Preparative chiral HPLC method:
Instrument: PrepCon Labomatic HPLC-3; Column: YMC Cellulose SB 10 μ, 250x50; eluent A: hexane + 0.1 vol % diethylamine; eluent B: ethanol + 0.1 vol % diethylamine; isocratic: 60%A+40%B; flow: 80 mL/min; temperature: 25°C; UV: 254 nm;

Analytical chiral HPLC method:
Instrument: : Waters Alliance 2695; Column: YMC Cellulose SB 3 μ, 100x4.6; eluent A: hexane + 0.1 vol % diethylamine; eluent B: ethanol; isocratic: 60%A+40%B; flow: 1.4 mL/min; temperature: 25°C; UV: 254 nm;

Analytical Chiral HPLC: $R_t$ = 1.33 min.

$[a]_D$ = -152,4° (from solution in DMSO, c = 5.1 mg/mL)
$^1$H-NMR (400 MHz, DMSO-d6) δ [ppm] = 1.40 (d, 3 H) 2.84 (s, 3 H) 2.93 (t, 2 H) 3.55 (t, 2 H) 4.02 (q, 1 H) 6.51 (dd, 1 H) 7.13 - 7.21 (m, 2 H) 7.21 - 7.28 (m, 3 H) 7.34 - 7.41 (m, 2 H) 7.98 (d, 1 H) 8.04 (d, 1 H) 8.50 - 8.57 (m, 2 H) 10.68 (s, 1 H) 11.84 (s, 1 H). - slight impurities in the aliphatic range.

**Example 155**

N-[4-(5-methyl-4-oxo-3-phenyl-4,5,6,7-tetrahydro-1H-pyrrolo[3,2-c]pyridin-2-yl)pyridin-2-yl]-2-(pyridin-4-yl)propanamide (Enantiomer 2)

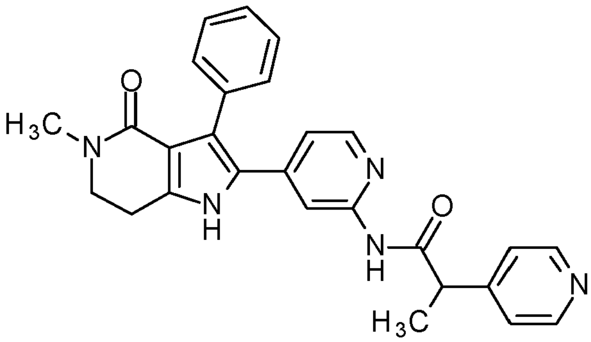

For the preparation of the racemic title compound see Example 153. Separation of the enantiomers by preparative chiral HPLC (method see Example 154) gave the title compound (57 mg).

Analytical Chiral HPLC (method see Example 154): Rt = 2.99 min.

$[a]_D$ = 156,7° (from solution in DMSO, c = 4.9 mg/mL)

$^1$H-NMR (400 MHz, DMSO-$d_6$) δ [ppm] = .40 (d, 3 H) 2.84 (s, 3 H) 2.93 (t, 2 H) 3.55 (t, 2 H) 4.02 (q, 1 H) 6.51 (dd, 1 H) 7.13 - 7.20 (m, 2 H) 7.22 - 7.28 (m, 3 H) 7.30 - 7.42 (m, 2 H) 7.98 (d, 1 H) 8.04 (s, 1 H) 8.49 - 8.58 (m, 2 H) 10.68 (s, 1 H) 11.84 (s, 1 H).

## Example 156

(2RS)-N-(4-(5-cyclopropyl-4-oxo-3-phenyl-4,5,6,7-tetrahydro-1H-pyrrolo[3,2-c]pyridin-2-yl)pyridin-2-yl)-4,4-difluoro-2-(4-fluorophenyl)butanamide

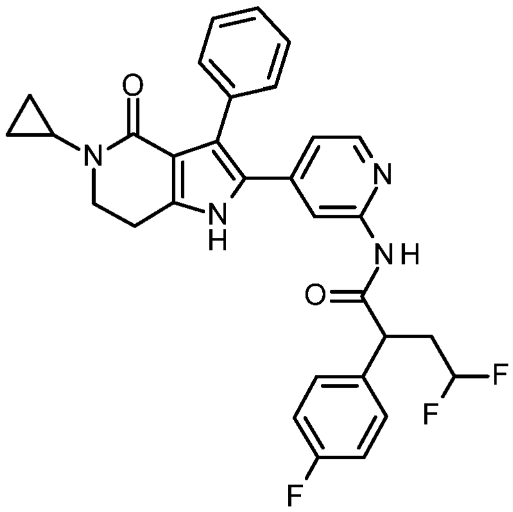

2-(2-aminopyridin-4-yl)-5-cyclopropyl-3-phenyl-1,5,6,7-tetrahydro-4H-pyrrolo[3,2-c]pyridin-4-one (150 mg, 438 μmol, 1 eq, see Intermediate 153) was dissolved in 2 mL of pyridine. 4,4-Difluoro-2-(4-fluorophenyl)butanoic acid (114 mg, 525 μmol, 1.2 eq) and a solution of T3P (836 mg, 1.31 mmol, 50% in DMF, 3 *eq*, CAS-RN: [68957-94-8]) were added in one portion. The reaction mixture was stirred at 25 °C for 16 h. The reaction was quenched by the addition of 2 mL of water, and extracted with ethyl acetate (2 mL × 3). The combined organic layers were washed with brine (2 mL × 3), dried (sodium sulfate), filtered, and concentrated *in vacuo* to provide a crude residue. The crude residue was purified by reverse phase preparative HPLC (column: Phenomenex luna C18 150mm* 25mm* 10μm; mobile phase: [water (0.225% FA)-ACN]; B%: 45%-75%, 11 min) to provide the title compound in 96% purity: 50 mg, 88.1 μmol, 20 % yield.

LC-MS (Method 7): Rt = 1.650 min; MS (ESIpos): m/z = 545.4 $[M+H]^+$

$^1$H NMR (400 MHz, DMSO-$d_6$) δ = 11.83 (s, 1H), 10.72 (s, 1H), 8.01 - 7.94 (m, 2H), 7.44 (dd, *J* = 5.6, 8.4 Hz, 2H), 7.25 - 7.15 (m, 7H), 6.53 (dd, *J*= 1.2, 5.6 Hz, 1H), 6.14 - 5.82 (m, 1H), 4.22 - 4.10 (m, 1H), 3.53 (br t, *J* = 6.8 Hz, 2H), 3.22 - 3.15 (m, 1H), 2.87 (br t, *J* = 6.4 Hz, 2H), 2.79 - 2.69 (m, 1H), 2.25 - 2.16 (m, 1H), 0.71 - 0.63 (m, 2H), 0.56 - 0.49 (m, 2H).

## Example 157

(2R or S)-N-[4-(5-cyclopropyl-4-oxo-3-phenyl-4,5-dihydro-1H-pyrrolo[3,2-c]pyridin-2-yl)pyridin-2-yl]-4,4-difluoro-2-(4-fluorophenyl)butanamide (Enantiomer 1)

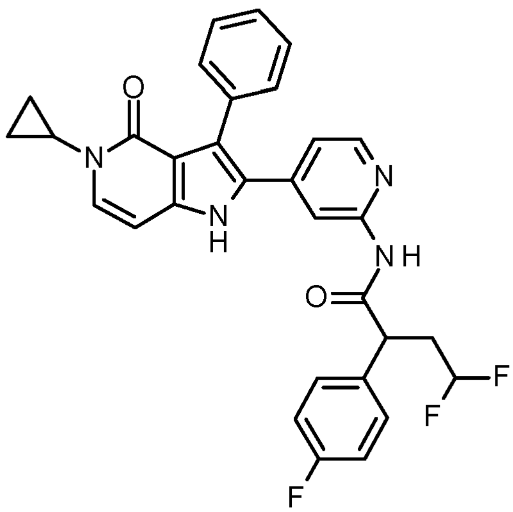

The racemic compound (see Example 137, 1.1 g), 2.03 mmol was separated into the enantiomers by preparative chiral preparatice SFC to give enantiomer 1 (470 mg, > 99 % ee, see example 157) and enantiomer 2 (440 mg, > 99 % ee, see example 158).

Preparative chiral SFC / HPLC method:
Chiral-SFC: column: DAICEL CHIRALPAK AD (250mm* 30mm* 10um); mobile phase: [0.1% $NH_3H_2O$ in EtOH]; B%: 25%-25%, 4.5; 90 min.
Analytical chiral SFC / HPLC method:
Column: Chiralpak AD-3 50×4.6mm I.D., 3um; Mobile phase: Phase A for $CO_2$, and Phase B for EtOH(0.05%DEA); Gradient elution:EtOH (0.05% DEA) in $CO_2$ from 5% to 40%; flow rate: 3mL/min; Detector: PDA; Column Temp: 35°C;Back Pressure: 100Bar"

Analytical Chiral HPLC: Rt = 1.574 min.

LC-MS (Method 7): Rt = 1.775 min; MS (ESIpos): m/z = 543.4 $[M+H]^+$
$^1$H NMR (400 MHz, DMSO-$d_6$) δ = 12.00 (s, 1H), 10.79 (s, 1H), 8.11 (s, 1H), 8.08 - 8.03 (m, 1H), 7.50 - 7.41 (m, 2H), 7.34 - 7.15 (m, 8H), 6.65 (dd, *J* = 1.6, 5.2 Hz, 1H), 6.40 (d, *J*= 7.2 Hz, 1H), 6.15 - 5.80 (m, 1H), 4.17 (dd, *J* = 5.6, 9.2 Hz, 1H), 3.23 - 3.13 (m, 1H), 2.77 - 2.61 (m, 1H), 2.28 - 2.14 (m, 1H), 0.97 - 0.89 (m, 2H), 0.82 - 0.72 (m, 2H).

## Example 158

(2R or S)-N-[4-(5-cyclopropyl-4-oxo-3-phenyl-4,5-dihydro-1H-pyrrolo[3,2-c]pyridin-2-yl)pyridin-2-yl]-4,4-difluoro-2-(4-fluorophenyl)butanamide (Enantiomer 2)

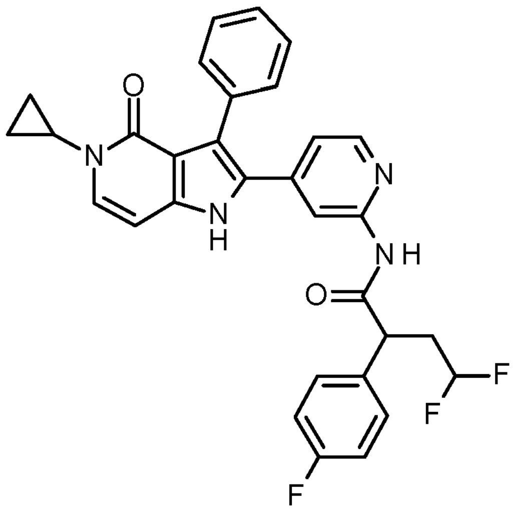

For the preparation of the racemic title compound see Example 137. Separation of the enantiomers by preparative chiral SFC / HPLC (method see Example 157) gave the title compound (440 mg, > 99% e.e.).

Analytical Chiral HPLC (method see Example 157): Rt = 1.759 min..

LC-MS (Method 7): Rt = 1.767 min; MS (ESIpos): m/z = 543.4 $[M+H]^+$

$^1$H NMR (400 MHz, DMSO-$d_6$) $\delta$ = 12.00 (s, 1H), 10.79 (s, 1H), 8.11 (s, 1H), 8.05 (d, J = 5.2 Hz, 1H), 7.48 - 7.43 (m, 2H), 7.30 - 7.24 (m, 6H), 7.23 - 7.17 (m, 2H), 6.65 (dd, J = 1.6, 5.2 Hz, 1H), 6.40 (d, J = 7.2 Hz, 1H), 6.16 - 5.82 (m, 1H), 4.17 (dd, J = 5.2, 9.2 Hz, 1H), 3.25 - 3.14 (m, 1H), 2.84 - 2.59 (m, 1H), 2.29 - 2.07 (m, 1H), 1.01 - 0.88 (m, 2H), 0.84 - 0.71 (m, 2H).

## Example 159

N-[4-(5-cyclopropyl-4-oxo-3-phenyl-4,5-dihydro-1H-pyrrolo[3,2-c]pyridin-2-yl)pyridin-2-yl]-2-(4-fluorophenyl)acetamide

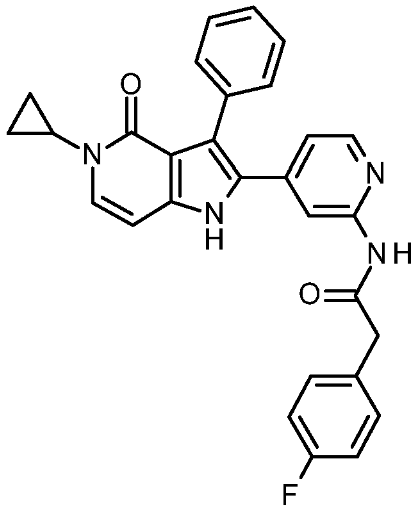

2-(2-Aminopyridin-4-yl)-5-cyclopropyl-3-phenyl-1,5-dihydro-4H-pyrrolo[3,2-c]pyridin-4-one (90.0 mg, 262 µmol, 1 *eq*, see Intermediate 152) was dissolved in 1 mL of pyridine. 2-(4-fluorophenyl)acetic acid (40.5 mg, 262 µmol, 1 *eq,* CAS-RN: [405-50-5]) and T3P (501 mg, 788 umol, 3 *eq,* 50% purity in DMF, CAS-RN: [68957-94-8]) were added in one portion, and the resulting mixture was stirred at 25 °C for 12 h. On completion, the reaction mixture was quenched by the addition of 3 mL of saturated aqueous ammonium chloride solution. The resulting mixture was extracted with

ethyl acetate (5 mL × 3). The combined organic layers were washed with brine (5 mL × 3), dried (sodium sulfate), filtered, and concentrated under reduced pressure to give a crude residue. The crude residue was purified by preparative HPLC (column: Phenomenex luna C18 150 * 25mm * 10um; mobile phase: [water (0.225% FA)-ACN]; B%: 30%-60%, 11.5min) to give the target compound: 48.2 mg, 100.7 μmol, 37.8% yield.

LC-MS (Method 7): $R_t$ = 1.53 min; MS (ESIpos): m/z = 479.3 $[M+H]^+$

$^1$H NMR (400 MHz, METHANOL-$d_4$) δ = 8.08 (s, 1H), 8.03 (d, *J* = 5.6 Hz, 1H), 7.37 - 7.29 (m, 8H), 7.07 (t, *J*= 8.8 Hz, 2H), 6.78 (dd, *J* = 1.6, 5.6 Hz, 1H), 6.56 (d, *J*= 7.2 Hz, 1H), 3.71 (s, 2H), 3.23 - 3.17 (m, 1H), 1.06 (d, *J*= 6.4 Hz, 2H), 0.86 (dd, *J* = 1.6, 3.6 Hz, 2H)

**Example 160**

(2RS)-N-{4-[5-cyclopropyl-4-oxo-3-(pyridin-2-yl)-4,5-dihydro-1H-pyrrolo[3,2-c]pyridin-2-yl]pyridin-2-yl}-4,4-difluoro-2-(4-fluorophenyl)butanamide

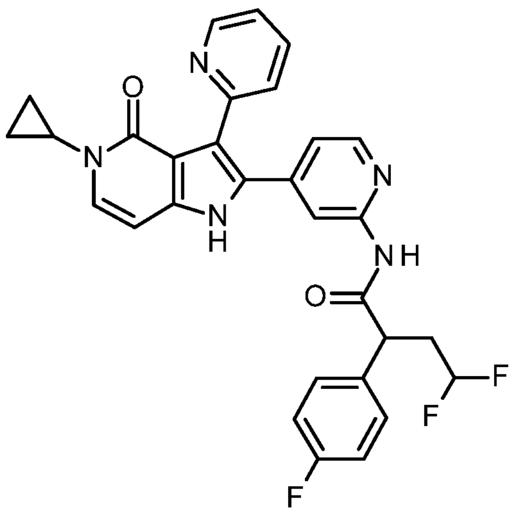

2-(2-Aminopyridin-4-yl)-5-cyclopropyl-3-(pyridin-2-yl)-1,5-dihydro-4H-pyrrolo[3,2-c]pyridin-4-one (50 mg, 145 μmol, 1 eq, see Intermediate 160) was dissolved in 1 mL of pyridine. 4,4-difluoro-2-(4-fluorophenyl)butanoic acid (31.7 mg, 145 μmol, 1 eq) and a solution of T3P (277 mg, 436 mmol, 50% in DMF, 3 eq, CAS-RN: [68957-94-8]) were added in sequence. The resulting mixture was stirred at 25 °C for 2 h. Upon completion, the mixture was concentrated in vacuo to provide a crude residue. The crude residue was purified by reverse phase preparative HPLC (column: Phenomenex luna C18 150mm * 25mm * 10um; mobile phase: [water (0.225% FA)-ACN]; B%: 18%-48%, 11.5 min) to provide the target compound as a yellow solid in 96% purity: 10 mg, 17.7 umol, 12.2% yield.

LC-MS (Method 7): Rt = 1.458 min; MS (ESIpos): m/z = 544.0 $[M+H]^+$

$^1$H NMR (400 MHz, DMSO-$d_6$) δ = 12.10 (s, 1H), 10.76 (s, 1H), 8.41 (d, J = 4.4 Hz, 1H), 8.14 - 8.09 (m, 1H), 8.03 (s, 1H), 7.78 (dt, J = 1.6, 7.8 Hz, 1H), 7.55 (d, J = 8.0 Hz, 1H), 7.45 (dd, J = 5.6, 8.4 Hz, 2H), 7.32 - 7.27 (m, 2H), 7.21 (t, J = 8.8 Hz, 2H), 6.76 (dd, J = 1.2, 5.2 Hz, 1H), 6.43 (d, J = 7.2 Hz, 1H), 6.17 - 5.83 (m, 1H), 4.16 (dd, J = 5.6, 8.8 Hz, 1H), 3.21 (br d, J = 4.0 Hz, 1H), 2.79 - 2.68 (m, 1H), 2.20 (td, J = 3.9, 18.0 Hz, 1H), 0.99 - 0.91 (m, 3H), 0.81 - 0.75 (m, 2H).

**Example 161**

(2RS)-N-{4-[7-chloro-5-cyclopropyl-3-(4-fluorophenyl)-4-oxo-4,5-dihydro-1H-pyrrolo[3,2-c]pyridin-2-yl]pyridin-2-yl}-4,4-difluoro-2-(4-fluorophenyl)butanamide

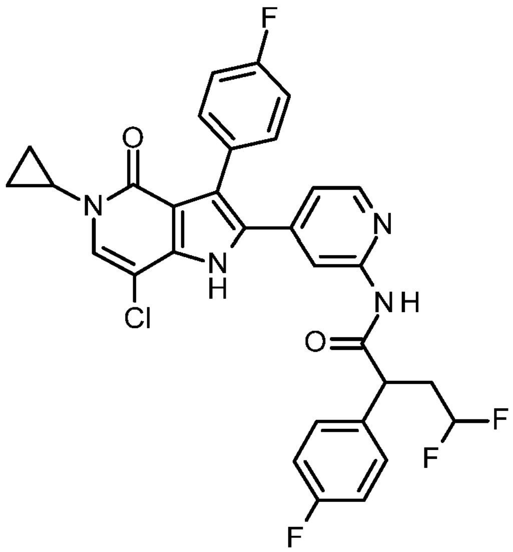

2-(2-Aminopyridin-4-yl)-7-chloro-5-cyclopropyl-3-(4-fluorophenyl)-1,5-dihydro-4H-pyrrolo[3,2-c]pyridin-4-one (100 mg, 253 μmol, 1 eq, see Intermediate 157) was dissolved in 2 mL of pyridine. 4,4-difluoro-2-(4-fluorophenyl)butanoic acid (55.2 mg, 253 μmol, 1 *eq*) and a solution of T3P (483 mg, 759 mmol, 50% in DMF, 3 *eq*, CAS-RN: [68957-94-8]) were added in one portion. The resulting mixture was stirred at 25 °C for 16 h. On completion, the mixture was concentrated *in vacuo* to provide the crude residue. The crude residue was twice purified by reverse phase preparative HPLC (column: Phenomenex Gemini 150mm * 25mm * 10um; mobile phase: [water (0.05% NH3 in H2O)-ACN]; B%: 42%-72%, 10 min) to provide the target compound in 99.7% purity: 30 mg, 50.2 μmol, 19.8% yield.

LC-MS (Method 7): $R_t$ = 1.963 min; MS (ESIpos): m/z = 595.1 $[M+H]^+$

$^1$H NMR (400 MHz, DMSO-$d_6$) δ = 12.32 (br s, 1H), 10.79 (s, 1H), 8.14 (d, *J*= 5.2 Hz, 1H), 8.07 (s, 1H), 7.52 - 7.40 (m, 3H), 7.28 - 7.15 (m, 4H), 7.14 - 7.05 (m, 2H), 6.83 (dd, *J* = 1.6, 5.2 Hz, 1H), 6.18 - 5.78 (m, 1H), 4.16 (br dd, *J* = 5.6, 8.8 Hz, 1H), 3.25 - 3.16 (m, 1H), 2.77 - 2.60 (m, 1H), 2.28 - 2.09 (m, 1H), 0.99 - 0.89 (m, 2H), 0.88 - 0.80 (m, 2H).

## Example 162

(2RS)-4,4-difluoro-2-(4-fluorophenyl)-N-{4-[5-methyl-4-oxo-3-(pyridin-2-yl)-7-(2,2,2-trifluoroethyl)-4,5-dihydro-1H-pyrrolo[3,2-c]pyridin-2-yl]pyridin-2-yl}butanamide

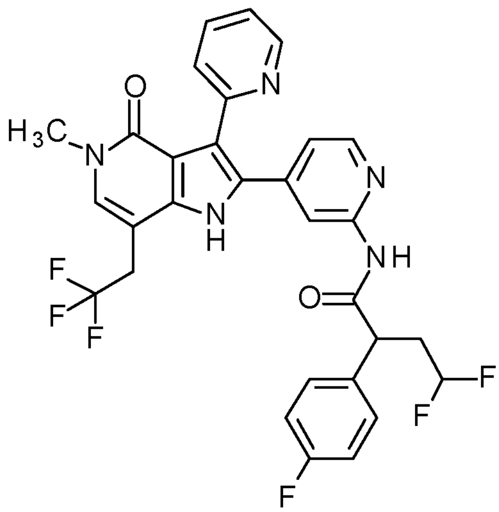

To a solution of (2RS)-4,4-difluoro-2-(4-fluorophenyl)-N-{4-[5-methyl-4-oxo-3-(pyridin-2-yl)-7-(2,2,2-trifluoroethyl)-1-{[2-(trimethylsilyl)ethoxy]methyl}-4,5-dihydro-1H-pyrrolo[3,2-c]pyridin-2-yl]pyridin-2-yl}butanamide containig some (2RS)-4,4-difluoro-2-(4-fluorophenyl)-N-{4-[5-methyl-4-oxo-3-(pyridin-2-yl)-7-(2,2,2-trifluoroethyl)-4,5-dihydro-1H-pyrrolo[3,2-c]pyridin-2-yl]pyridin-2-yl}butanamide (9.00 mg, 12.3 μmol, see Intermediate 166) in acetonitrile (170 μl) was added boron trifluoride diethyl etherate (7.6 μl, 62 μmol) and the reaction mixture was stirred at rt for 16 h under nitrogen atmosphere. Aqueous ammonia (110 μl, 33 % purity; CAS-RN:[7664-41-7]) and water (230 μl) were added. The reaction mixture was stirred at rt for 16 h. The mixture was directly purified by silicagel chromatography (Gradient: dichloromethane / ethanol 0-12%) resulting in 1.30 mg (approx. 17 % yield) of the title compound.

LC-MS (Method 2): Rt = 1.14 min; MS (ESIpos): m/z = 600 $[M+H]^+$

$^1$H-NMR (400 MHz, DMSO-$d_6$): δ [ppm] = 12.03 (s, 1H), 10.79 (s, 1H), 8.46-8.32 (m, 1H), 8.15 (dd, 1H), 8.03 (s, 1H), 7.75 (td, 1H), 7.52 (dt, 1H), 7.48 (s, 1H), 7.47-7.42 (m, 2H), 7.26 (ddd, 1H), 7.23-7.17 (m, 2H), 6.82 (dd, 1H), 6.15-5.79 (m, 1H), 4.16 (dd, 1H), 3.87-3.72 (m, 2H), 3.42 (s, 3H), 2.79-2.60 (m, 1H), 2.27-2.09 (m, 1H).

## EXPERIMENTAL SECTION - BIOLOGICAL ASSAYS

The following assays can be used to illustrate the commercial utility of the compounds according to the present invention.

Examples were tested in selected biological assays one or more times. When tested more than once, data are reported as either average values or as median values or single individual measurements, wherein

- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.
- Individual measurements are shown when median or average values cannot be computed.

Examples were synthesized one or more times. When synthesized more than once, data from biological assays represent average values calculated utilizing data sets obtained from testing of one or more synthetic batch.

The ***in vitro* activity** of the compounds of the present invention can be demonstrated in the following assays:

### CSNK1A1 assay 1

CSNK1A1-inhibitory activity of compounds of the present invention in presence of 1 μM adenosine-tri-phosphate (ATP) was quantified employing the CSNK1A1 assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows : In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent"). Recombinant fusion protein of N-terminal Glutathion-S-Transferase (GST) and full-length human CSNK1A1, expressed by baculovirus infected insect cells and purified via Glutathion affinity chromatography, was purchased from Life Technologies (product no. PV4174) and used as enzyme. As substrate for the kinase reaction the biotinylated peptide Btn-Ahx-SGSEGDSESGEEEG (C-terminus in amide form) was used which can be purchased e.g. from the company Biosyntan (Berlin-Buch, Germany).

For the assay 50 nl of a 100fold concentrated solution of the test compound in DMSO was pipetted into a white 1536-well microtiter plate (Greiner Bio-One, Frickenhausen, Germany), 2 μL of a solution of CSNK1A1 in aqueous assay buffer [50 mM HEPES pH 7.5, 10% (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 μL of a solution of ATP (1.67 μM => final conc. in the 5 μL assay volume is 1 μM) and peptide substrate (50 μM => final conc. in the 5 μL assay volume is 30 μM) in assay buffer and the resulting mixture was incubated for a reaction time of 30 min at 22°C. The concentration of CSNK1A1 was adjusted depending of the activity of the enzyme lot and was chosen appropriate to have the assay in the linear range, a typical concentration is 0.15 ng/μL. The reaction was stopped by the addition of 2.5 μL of "ADP-Glo-reagent" (1:1.5fold diluted with water) and the resulting mixture was incubated at 22°C for 1 h to convert the ATP not consumed in the kinase reaction completely to

cAMP. Subsequently 2.5 μL of the "kinase detection reagent" (1.2 fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1A1.

The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**CSNK1A1 assay 2**

CSNK1A1-inhibitory activity of compounds of the present invention in presence of 1 μM adenosine-tri-phosphate (ATP) was quantified employing the CSNK1A1 assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows: In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent").

Recombinant fusion protein of N-terminal Glutathion-S-Transferase (GST) and full-length human CSNK1A1, expressed by baculovirus infected insect cells and purified via Glutathion affinity chromatography, was purchased from Life Technologies (product no. PV4174) and used as enzyme. As substrate for the kinase reaction the biotinylated peptide biotin-Ahx-KRRRAL-pS-VASLPGL (C-terminus in amide form) was used which can be purchased e.g. from the company Biosyntan (Berlin-Buch, Germany).

For the assay 50 nl of a 100fold concentrated solution of the test compound in DMSO was pipetted into a white 1536-well microtiter plate (Greiner Bio-One, Frickenhausen, Germany), 2 μL of a solution of CSNK1A1 in aqueous assay buffer [50 mM HEPES pH 7.5, 10% (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 μL of a solution of ATP (1.67 μM => final conc. in the 5 μL assay volume is 1 μM) and peptide substrate (50 μM => final conc. in the 5 μL assay volume is 30 μM) in assay buffer and the resulting mixture was incubated for a reaction time of 30 min at 22°C. The concentration of CSNK1A1 was adjusted depending of the activity of the enzyme lot and was chosen appropriate to have the assay in the linear range, a typical concentrations are about 0.0375 ng/μL. The reaction was stopped by the addition of 2.5 μL of "ADP-Glo-reagent" (1:1.5fold diluted with water) and the resulting mixture was incubated at 22°C for 1 h to convert the ATP not consumed in the kinase reaction completely to cAMP. Subsequently 2.5 μL of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1A1.

The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**CSNK1A1 high ATP assay**

CSNK1A1-inhibitory activity of compounds of the present invention in presence of 1 mM adenosine-tri-phosphate (ATP) was quantified employing the CSNK1A1-high-ATP-assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows : In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent").

Recombinant fusion protein of N-terminal Glutathion-S-Transferase (GST) and full-length human CSNK1A1, expressed by baculovirus infected insect cells and purified via Glutathion affinity chromatography, was purchased from Life Technologies (product no. PV4174) and used as enzyme. As substrate for the kinase reaction the biotinylated peptide biotin-Ahx-KRRRAL-pS-VASLPGL (C-terminus in amide form) was used which can be purchased e.g. from the company Biosyntan (Berlin-Buch, Germany).

For the assay 50 nl of a 100fold concentrated solution of the test compound in DMSO was pipetted into a white low volume 384-well microtiter plate (Greiner Bio-One, Frickenhausen, Germany), 2 μL of a solution of CSNK1A1 in aqueous assay buffer [50 mM HEPES pH 7.5, 10 % (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 μL of a solution of ATP (1.67 mM => final conc. in the 5 μL assay volume is 1 mM) and peptide substrate (167 μM => final conc. in the 5 μL assay volume is 100 μM) in assay buffer and the resulting mixture was incubated for a reaction time of 30 min at 22°C. The concentration of CSNK1A1 was adjusted depending of the activity of the enzyme lot and was chosen appropriate to have the assay in the linear range, a typical concentration is about 0.4 ng/μL. The reaction was stopped by the addition of 2.5 μL of "ADP-Glo-reagent" (1:1.5fold diluted with water) and the resulting mixture was incubated at 22°C for 1 h to convert the ATP not consumed in the kinase reaction completely to cAMP. Subsequently 2.5 μL of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1A1.

The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**CSNK1D assay**

CSNK1D-inhibitory activity of compounds of the present invention in presence of 1 μM adenosine-tri-phosphate (ATP) was quantified employing the CSNK1D assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows : In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent"). Recombinant fusion protein of N-terminal Glutathion-S-Transferase (GST) and full-length human CSNK1D, expressed by baculovirus infected insect cells and purified via Glutathion affinity chromatography, was purchased from Life Technologies (product no. PV3665) and used as enzyme. As substrate for the kinase reaction the biotinylated peptide Btn-Ahx-SGSEGDSESGEEEG (C-terminus in amide form) was used which can be purchased e.g. from the company Biosyntan (Berlin-Buch, Germany).

For the assay 50 nl of a 100fold concentrated solution of the test compound in DMSO was pipetted into a white 1536-well microtiter plate (Greiner Bio-One, Frickenhausen, Germany), 2 μL of a solution of CSNK1D in aqueous assay buffer [50 mM HEPES pH 7.5, 10 % (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at 22°C to allow pre-binding of the test compounds to the enzyme before the start of the kinase reaction. Then the kinase reaction was started by the addition of 3 μL of a solution of ATP (1.67 μM => final conc. in the 5 μL assay volume is 1 μM) and peptide substrate (50 μM => final conc. in the 5 μL assay volume is 30 μM) in assay buffer and the resulting mixture was incubated for a reaction time of 30 min at 22°C. The concentration of CSNK1D was adjusted depending of the activity of the enzyme lot and was chosen appropriate to have the assay in the linear range, a typical concentration is about 0.5 ng/μL. The reaction was stopped by the addition of 2.5 μL of "ADP-Glo-reagent" (1:1.5fold diluted with water) and the resulting mixture was incubated at 22°C for 1 h to convert the ATP not consumed in the kinase reaction completely to cAMP. Subsequently 2.5 μL of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1D.

The data were normalised (enzyme reaction without inhibitor = 0 % inhibition, all other assay components but no enzyme = 100 % inhibition). Usually the test compounds were tested on the same microtiterplate in 11 different

concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**CSNK1G3 assay**

CSNK1G3-inhibitory activity of compounds of the present invention in presence of 1 μM adenosine-tri-phosphate (ATP) was quantified employing the CSNK1G3 assay as described in the following paragraphs. In essence, the enzyme activity is measured by quantification of the adenosine-di-phosphate (ADP), which is generated as a co-product of the enzyme reaction, via the "ADP-Glo™ Kinase Assay" kit from the company Promega. This detection system works as follows : In a first step the ATP not consumed in the kinase reaction is quantitatively converted to cAMP employing an adenylate cyclase ("ADP-Glo-reagent"), then the adenylate cyclase is stopped and the ADP generated in the kinase reaction converted to ATP which generates in a luciferase-based reaction a glow-luminescence signal ("Kinase Detection Reagent"). Recombinant fusion protein of N-terminal Glutathion-S-Transferase (GST) and full-length human CSNK1G3, expressed by baculovirus infected insect cells and purified via Glutathion affinity chromatography, was purchased from Life Technologies (product no. PV3838) and used as enzyme. As substrate for the kinase reaction the biotinylated peptide biotin-Ahx-KRRRAL-pS-VASLPGL (C-terminus in amide form) was used which can be purchased e.g. from the company Biosyntan (Berlin-Buch, Germany).

For the assay 50 nl of a 100fold concentrated solution of the test compound in DMSO was pipetted into a white 1536-well microtiter plate (Greiner Bio-One, Frickenhausen, Germany), 2 μL of a solution of CSNK1G3 in aqueous assay buffer [50 mM HEPES pH 7.5, 10 % (v/v) glycerol, 10 mM $MgCl_2$, 50 mM NaCl, 1 mM dithiothreitol, 0.01 % (w/v) bovine serum albumin, 0.01 % (v/v) Triton X-100] were added and the mixture was incubated for 15 min at22°C to allow pre-binding of the test compounds to the enzyme before the start of the kinase reaction. Then the kinase reaction was started by the addition of 3 μL of a solution of ATP (1.67 μM => final conc. in the 5 μL assay volume is 1 μM) and peptide substrate (50 μM => final conc. in the 5 μL assay volume is 30 μM) in assay buffer and the resulting mixture was incubated for a reaction time of 30 min at 22°C. The concentration of CSNK1G3 was adjusted depending of the activity of the enzyme lot and was chosen appropriate to have the assay in the linear range, a typical concentration is about 0.06 ng/μL. The reaction was stopped by the addition of 2.5 μL of "ADP-Glo-reagent" (1:1.5fold diluted with water) and the resulting mixture was incubated at 22°C for 1 h to convert the ATP not consumed in the kinase reaction completely to cAMP. Subsequently 2.5 μL of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the CSNK1G3.

The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**WT-EGFR kinase assay**

Inhibitory activity of compounds of the present invention against wild-type Epidermal Growth Factor Receptor (EGFR) was quantified employing the TR-FRET based EGFR assay as described in the following paragraphs.

Recombinant fusion protein of N-terminal Glutathion-S-Transferase (GST) and a fragment of human EGFR (amino acids R669 to A1210), expressed in Sf9 insect cells and purified via affinity chromatography using Glutathion Sepharose as described above, was used as kinase. As substrate for the kinase reaction the biotinylated peptide biotin-Ahx-AEEEEYFELVAKKK (C-terminus in amide form) was used which can be purchased e.g. form the company Biosynthan GmbH (Berlin-Buch, Germany).

For the assay 50 nl of a 100fold concentrated solution of the test compound in DMSO was pipetted into either a black low volume 384well microtiter plate or a black 1536well microtiter plate (both Greiner Bio-One, Frickenhausen, Germany), 2 μL of a solution of EGFR in aqueous assay buffer [50 mM Hepes pH 7.0, 10 mM $MgCl_2$, 1mM dithiothreitol, 0.5 mM EGTA, 0.3 mM activated sodium ortho-vanadate, 0.005 % (w/v) bovine serum albumin, 0.005% (v/v) Tween-20] were added and the mixture was incubated for 15 min at 22°C to allow pre-binding of the test compounds to the enzyme before the start of the kinase reaction. Then the kinase reaction was started by the addition of 3 μL of a solution of adenosine-tri-phosphate (ATP, 3.33 mM => final conc. in the 5 μL assay volume is 2 mM) and substrate (1.67 μM => final conc. in the 5 μL assay volume is 1 μM) in assay buffer and the resulting mixture was incubated for a reaction

time of 30 min at 22°C. The concentration of EGFR was adjusted depending of the activity of the enzyme lot and was chosen appropriate to have the assay in the linear range, typical concentration was 7.6 pg/μL. The reaction was stopped by the addition of 3 μL of a solution of HTRF detection reagents (83.3 nM streptavidine-XL665 [Cisbio Bioassays, Codolet, France] and 1.67 nM PT66-Tb-Cryptate, a terbium-cryptate labelled anti-phospho-tyrosine antibody from Cisbio Bioassays [instead of the PT66-Tb-cryptate PT66-Eu-Chelate from Perkin Elmer can also be used]) in an aqueous EDTA-solution (133.3 mM EDTA, 0.2 % (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC50 values were calculated using Genedata Screener™ software.

**Bub1 kinase assay**

Bub1-inhibitory activity of compounds of the present invention was quantified employing the Bub1 TR-FRET assay as described in the following paragraphs.

N-terminally $His_6$-tagged recombinant catalytic domain of human Bub1 (amino acids 704-1085), expressed in insect cells (Hi5) and purified by Ni-NTA affinity chromatography and subsequent size exclusion chromatography, was used as enzyme. As substrate for the kinase reaction the biotinylated peptide biotin-Ahx-VLLPKKSFAEPG (C-terminus in amid form) was used which can be purchased e.g. form the company Biosyntan (Berlin, Germany).

For the assay 50 nl of a 100 fold concentrated solution of the test compound in DMSO was pipetted into either a black low volume 384well microtiter plate or a black 1536well microtiter plate (both Greiner Bio-One, Frickenhausen, Germany), 2 μL of a solution of Bub1 in aqueous assay buffer [50 mM Tris/HCl pH 7.5, 10 mM magnesium chloride ($MgCl_2$), 200 mM potassium chloride (KCl), 1.0 mM dithiothreitol (DTT), 0.1 mM sodium ortho-vanadate, 1% (v/v) glycerol, 0.01 % (w/v) bovine serum albumine (BSA), 0.005% (v/v) Trition X-100 (Sigma), 1x *Complete EDTA-free* protease inhibitor mixture (Roche)] were added and the mixture was incubated for 15 min at 22°C to allow pre-binding of the test compounds to the enzyme before the start of the kinase reaction. Then the kinase reaction was started by the addition of 3 μL of a solution of adenosine-tri-phosphate (ATP, 16.7 μM => final conc. in the 5 μL assay volume is 10 μM) and substrate (1.67 μM => final conc. in the 5 μL assay volume is 1 μM) in assay buffer and the resulting mixture was incubated for a reaction time of 60 min at 22°C. The concentration of Bub1 was adjusted depending of the activity of the enzyme lot and was chosen appropriate to have the assay in the linear range, typical concentrations were in the range of 200 ng/mL. The reaction was stopped by the addition of 5 μL of a solution of TR-FRET detection reagents (0.2 μM streptavidine-XL665 [Cisbio Bioassays, Codolet, France] and 1 nM anti-phosho-Serine antibody [Merck Millipore, cat. # 35-002] and 0.4 nM LANCE EU-W1024 labeled anti-mouse IgG antibody [Perkin-Elmer, product no. AD0077, as an alternative a Terbium-cryptate-labeled anti-mouse IgG antibody from Cisbio Bioassays can be used]) in an aqueous EDTA-solution (50 mM EDTA, 0.2 % (w/v) bovine serum albumin in 100 mM HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar or Pherastar FS (both from BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). The test compounds were tested on the same microtiterplate, usually in 11 different concentrations in the range of 20 μM to 0.1 nM (20 μM, 5.9 μM, 1.7 μM, 0.51 μM, 0.15 μM, 44 nM, 13 nM, 3.8 nM, 1.1 nM, 0.33 nM and 0.1 nM, the dilution series prepared separately before the assay on the level of the 100fold concentrated solutions in DMSO by serial 1:3.4 dilutions, the exact concentrations and the number of tested concentrations may vary depending on the liquid handling instrumentation used for test sample preparation) in duplicate values for each concentration and $IC_{50}$ values were calculated by a 4 parameter fit.

### Bub1 high ATP kinase assay

Bub1-inhibitory activity of compounds of the present invention at a high ATP concentration was quantified employing the Bub1 TR-FRET high ATP kinase assay as described in the following paragraphs.

N-terminally $His_6$-tagged recombinant catalytic domain of human Bub1 (amino acids 704-1085), expressed in insect cells (Hi5) and purified by Ni-NTA affinity chromatography and subsequent size exclusion chromatography, was used as enzyme. As substrate for the kinase reaction the biotinylated peptide biotin-Ahx-VLLPKKSFAEPG (C-terminus in amid form) was used which can be purchased e.g. form the company Biosyntan (Berlin, Germany).

For the assay 50 nl of a 100fold concentrated solution of the test compound in DMSO was pipetted into either a black low volume 384well microtiter plate or a black 1536well microtiter plate (both Greiner Bio-One, Frickenhausen, Germany), 3 μL of a solution of adenosine-tri-phosphate (ATP, 3.33 mM => final conc. in the 5 μL assay volume is 2 mM) and substrate (1.67 μM => final conc. in the 5 μL assay volume is 1 μM) in aqueous assay buffer [50 mM Tris/HCl pH 7.5, 10 mM magnesium chloride ($MgCl_2$), 200 mM potassium chloride (KCl), 1.0 mM dithiothreitol (DTT), 0.1 mM sodium ortho-vanadate, 1% (v/v) glycerol, 0.01 % (w/v) bovine serum albumine (BSA), 0.005% (v/v) Trition X-100 (Sigma), 1x *Complete EDTA-free* protease inhibitor mixture (Roche)] were added. Then the kinase reaction was started by the addition of 2 μL of a solution of Bub1 in assay buffer and the resulting mixture was incubated for a reaction time of 60 min at 22°C. The concentration of Bub1 was adjusted depending of the activity of the enzyme lot and was chosen appropriate to have the assay in the linear range, a typical concentration is about 200 ng/mL. The reaction was stopped by the addition of 3 μL of a solution of TR-FRET detection reagents (0.167 μM streptavidine-XL665 [Cisbio Bioassays, Codolet, France] and 1.67 nM anti-phosho-Serine antibody [Merck Millipore, cat. # 35-002] and 0.67 nM LANCE EU-W1024 labeled anti-mouse IgG antibody [Perkin-Elmer, product no. AD0077, as an alternative a Terbium-cryptate-labeled anti-mouse IgG antibody from Cisbio Bioassays can be used]) in an aqueous EDTA-solution (83.3 mM EDTA, 0.2 % (w/v) bovine serum albumin in 100 mM HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar or Pherastar FS (both from BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.0.7 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC50 values were calculated by a 4 parameter fit.

### Cellular Mechanistic assays

### P-RPS6 (Ser244/247) Ribosomal protein S6

The kinase CSNK1A phosphorylates Ribosomal protein S6 at Ser247. In-cell Western assay simultaneously detects two targets at 700 and 800 nm using two spectrally distinct near-infrared dyes. With a specific antibody, Ser244/247-phosphorylated RPS6 (Thermo Fisher 44-923) can be quantified and the samples can be normalized with cell stains Draq5 (Cell signaling, 4084L) and Sapphire700 (LiCor, 928-40022) in parallel.

2000 HCT 116 cells were seeded in growth medium (DMEM / Ham's F12, 10% FCS) in 96well plate (Falcon 353075) over night at 37°C. Cells were treated with varying concentrations of test compounds at 37°C for4h. Cells were fixed with 4% paraformaldehyde, washed (Sigma-Aldrich, AB351787, Tween 20, 1%) and blocked with buffer (Odyssey blocking buffer, LiCor, 927-40000) before incubating with the primary antibody (Ser244/247-phosphorylated RPS6 (Thermo Fisher 44-923) overnight at 2-8°C. After washing, secondary IRDye-labeled antibody mix with cell stains was added for 1h and washed again. Plates were scanned with LiCor Odyssey Infrared Imager CLX at 800 nm for pRPS6 and at 700 nm for cell stains Draq5/Sapphire. The quotient of 800 nm and 700 nm for standard compound-treated cells was set as 0% and the quotient of 800 nm and 700 nm of DMSO treated cells was set as 100%. The results given as % reflecting the inhibition of Casein kinase activity compared to control and normalized according to cell number.

The $IC_{50}$ values were determined by means of a 4 parameter fit.

### P-β-Catenin (Ser45)

50,000 DLD-1 cells were seeded in 96well plates (nunc #161093) in RPMI 1640 (Biochrom; # FG 1215, 10% FCS,

2mM L-Glutamine). After 24h, cells were treated with varying concentrations of test compounds at 37°C for 30 min. Cells were washed twice with ice-cold PBS buffer, treated with lysis buffer and all next steps were performed to the supplier's manual (β-Catenin pS45 ELISA Kit; ab205703). The content of pS45 was measured with ELISA at 450 nm, calculated with calibration curve and normalized to protein content. The normalized quotient of control compound-treated cells treated cells was set as 0% and the normalized quotient of untreated cells was set as 100%.The results given as % reflecting the content of β-Catenin pS45 compared to control. The $IC_{50}$ values were determined by means of a 4 parameter fit.

**Proliferation Assays**

**HCT 116**

400 HCT 116 cells/30 μL/well were plated in growth medium (DMEM / Ham's F12, 10% FCS) in a 384-well plate (CORNING #3571) at day 1. Reference plate was seeded for time zero determination. All plates were incubated overnight 37°C. Day 2: test compound was added in 7-step dilution and incubate at 37°C for 96h. Day 2: time zero plate: 30 μL/well CTG solution (Promega Cell Titer Glo solution; catalog # G755B and G756B) were added, incubated for 30 minutes, read luminescence on PheraStar. Day 6: compound treated plates: 30 μL/well CTG solution (Promega Cell Titer Glo solution; catalog # G755B and G756B) were added, incubated for 30 minutes, luminescence was read on PheraStar. Proliferation was calculated after subtracting time zero luminescence values from day 6 values and comparing to untreated wells. The $IC_{50}$ values were determined using the four parameter fit.

**A549**

400 A549 cells/30 μL/well were plated in growth medium (DMEM/Ham's F12, 10% FCS) in a 384-well plate (CORNING #3571) at day 1. Reference plate was seeded for time zero determination. All plates were incubated overnight 37°C. Day 2: test compound was added in 7-step dilution and incubated at 37°C for 96h. Day 2: time zero plate: 30 μL/well CTG solution were added (Promega Cell Titer Glo solution; catalog # G755B and G756B), incubated for 30 minutes, luminescence was read on PheraStar. Day 6: compound treated plates: 30 μL/well CTG solution (Promega Cell Titer Glo solution; catalog # G755B and G756B) were added, incubated for 30 minutes, luminescence was read on PheraStar. Proliferation was calculated after subtracting time zero luminescence values from day 6 values and comparing to untreated wells.

The $IC_{50}$ values were determined using the four parameter fit.

**TMD8**

400 TMD8 cells/30 μL/well were plated in growth medium (RPM11640, 20% FCS) in a 384-well plate (CORNING #3571) at day 1. Reference plate was seeded for time zero determination. All plates were incubated overnight 37°C. Day 2: test compound was added in 7-step dilution and incubated at 37°C for 96h. Day 2: time zero plate: 30 μL/well CTG solution (Promega Cell Titer Glo solution; catalog # G755B and G756B) were added, incubated 30 minutes, luminescence was read on PheraStar. Day 6: compound treated plates: 30 μL/well CTG solution (Promega Cell Titer Glo solution; catalog # G755B and G756B) were added, incubated 30 minutes, luminescence was read on PheraStar. Proliferation was calculated after subtracting time zero luminescence values from day 6 values and comparing to untreated wells.

The $IC_{50}$ values were determined using the four parameter fit.

**Results:**

Table 2 shows the results of the inhibition in the CSNK1A1 and CSNK1D biochemical assays.

**Table 2**

| Example No | CSNK1A1 Assay 2 $IC_{50}$ [mol/l] (median) | CSNK1D $IC_{50}$ [mol/l] (median) |
|---|---|---|
| **1** | 1.3 E-9 | 3.7 E-9 |
| **2** | 1.7 E-9 | 3.3 E-9 |
| **3** | 4.7 E-9 | 5.9 E-9 |

(continued)

| Example No | CSNK1A1 Assay 2 $IC_{50}$ [mol/l] (median) | CSNK1D $IC_{50}$ [mol/l] (median) |
|---|---|---|
| **4** | 1.1 E-9 | 2.3 E-9 |
| **5** | 8.6 E-10 | 2.5 E-9 |
| **6** | 2.5 E-9 | 3.7 E-9 |
| **7** | 7.3 E-9 | 7.0 E-9 |
| **8** | 9.4 E-10 | 2.4 E-9 |
| **9** | 7.8 E-9 | 9.2 E-9 |
| **10** | 1.1 E-7 | 6.4 E-8 |
| **11** | 4.3 E-9 | 4.7 E-9 |
| **12** | 1.0 E-8 | 1.3 E-8 |
| **13** | 3.5 E-9 | 3.9 E-9 |
| **14** | 4.7 E-9 | 2.7 E-9 |
| **15** | 2.3 E-9 | 2.8 E-9 |
| **16** | 7.7 E-9 | 4.4 E-9 |
| **17** | 4.7 E-9 | 4.3 E-9 |
| **18** | 5.9 E-9 | 3.1 E-9 |
| **19** | 2.2 E-8 | 1.4 E-8 |
| **20** | 1.4 E-8 | 1.2 E-8 |
| **21** | 8.6 E-9 | 6.6 E-9 |
| **22** | 6.2 E-9 | 3.2 E-9 |
| **23** | 8.5 E-8 | 3.7 E-8 |
| **24** | 4.5 E-9 | 4.0 E-9 |
| **25** | 6.4 E-9 | 5.0 E-9 |
| **26** | 1.1 E-8 | 7.1 E-9 |
| **27** | 1.2 E-8 | 5.8 E-9 |
| **28** | 6.1 E-9 | 5.0 E-9 |
| **29** | 2.9 E-9 | 4.1 E-9 |
| **30** | 5.7 E-9 | 3.8 E-9 |
| **31** | 5.3 E-8 | 1.4 E-8 |
| **32** | 4.4 E-9 | 3.6 E-9 |
| **33** | 4.3 E-9 | 4.3 E-9 |
| **34** | 5.8 E-9 | 5.3 E-9 |
| **35** | 6.6 E-8 | 2.2 E-8 |
| **36** | 3.5 E-9 | 3.5 E-9 |
| **37** | 3.1 E-9 | 4.1 E-9 |
| **38** | 7.8 E-9 | 7.1 E-9 |
| **39** | 3.0 E-9 | 3.7 E-9 |
| **40** | 4.0 E-9 | 4.0 E-9 |

(continued)

| Example No | CSNK1A1 Assay 2 $IC_{50}$ [mol/l] (median) | CSNK1D $IC_{50}$ [mol/l] (median) |
|---|---|---|
| 41 | 5.7 E-9 | 6.1 E-9 |
| 42 | 1.2 E-9 | 2.1 E-9 |
| 43 | 2.6 E-9 | 2.7 E-9 |
| 44 | 1.1 E-8 | 1.2 E-8 |
| 45 | 2.0 E-9 | 2.7 E-9 |
| 46 | 2.7 E-9 | 3.3 E-9 |
| 47 | 1.3 E-8 | 9.6 E-9 |
| 48 | 1.8 E-9 | 2.9 E-9 |
| 49 | 4.6 E-9 | 4.5 E-9 |
| 50 | 1.2 E-8 | 9.8 E-9 |
| 51 | 1.8 E-9 | 2.4 E-9 |
| 52 | 3.4 E-9 | 3.8 E-9 |
| 53 | 2.1 E-8 | 1.6 E-8 |
| 54 | 1.6 E-9 | 3.6 E-9 |
| 55 | 2.0 E-9 | 2.2 E-9 |
| 56 | 4.4 E-9 | 4.8 E-9 |
| 57 | 4.5 E-9 | 2.9 E-9 |
| 58 | 5.2 E-9 | 4.0 E-9 |
| 59 | 4.9 E-9 | 3.6 E-9 |
| 60 | 1.5 E-8 | 9.5 E-9 |
| 61 | 2.0 E-9 | 1.8 E-9 |
| 62 | 7.6 E-9 | 4.0 E-9 |
| 63 | 1.2 E-8 | 8.0 E-9 |
| 64 | 8.5 E-9 | 4.8 E-9 |
| 65 | 1.1 E-8 | 6.6 E-9 |
| 66 | 4.4 E-9 | 3.2 E-9 |
| 67 | 1.6 E-8 | 8.6 E-9 |
| 68 | 4.1 E-9 | 5.6 E-9 |
| 69 | 3.1 E-9 | 2.8 E-9 |
| 70 | 1.6 E-8 | 7.6 E-9 |
| 71 | 1.7 E-9 | 2.1 E-9 |
| 72 | 6.6 E-10 | 1.3 E-9 |
| 73 | 1.9 E-9 | 5.4 E-9 |
| 74 | 1.6 E-9 | 3.0 E-9 |
| 75 | 2.7 E-9 | 4.5 E-9 |
| 76 | 2.0 E-9 | 3.1 E-9 |
| 79 | 5.1 E-9 | 4.8 E-9 |

(continued)

| Example No | CSNK1A1 Assay 2 $IC_{50}$ [mol/l] (median) | CSNK1D $IC_{50}$ [mol/l] (median) |
|---|---|---|
| **80** | 4.7 E-9 | 5.1 E-9 |
| **81** | 4.6 E-9 | 4.3 E-9 |
| **82** | 1.3 E-9 | 2.4 E-9 |
| **83** | 1.9 E-9 | 3.5 E-9 |
| **84** | 3.4 E-9 | 4.8 E-9 |
| **85** | 1.9 E-9 | 3.9 E-9 |
| **86** | 4.0 E-9 | 5.2 E-9 |
| **87** | 1.2 E-8 | 1.3 E-8 |
| **88** | 8.6 E-9 | 1.1 E-8 |
| **89** | 6.8 E-9 | 4.3 E-9 |
| **90** | 3.2 E-9 | 4.1 E-9 |
| **91** | 7.7 E-9 | 7.6 E-9 |
| **92** | 4.5 E-9 | 5.7 E-9 |
| **93** | 4.0 E-9 | 9.3 E-9 |
| **94** | 3.0 E-9 | 3.2 E-9 |
| **95** | 1.4 E-8 | 4.6 E-9 |
| **96** | 1.3 E-9 | 2.0 E-9 |
| **97** | 8.1 E-9 | 1.4 E-8 |
| **98** | 7.1 E-8 | 1.9 E-8 |
| **99** | 4.3 E-9 | 5.5 E-9 |
| **100** | 5.9 E-9 | 8.6 E-9 |
| **101** | 1.2 E-8 | 5.6 E-9 |
| **102** | 1.0 E-9 | 2.0 E-9 |
| **103** | 5.0 E-9 | 1.1 E-8 |
| **104** | 7.0 E-9 | 5.1 E-9 |
| **105** | 7.1 E-10 | 1.5 E-9 |
| **106** | 9.2 E-9 | 1.1 E-8 |
| **107** | 3.5 E-9 | 4.4 E-9 |
| **108** | 1.5 E-8 | 9.4 E-9 |
| **109** | 2.1 E-9 | 3.7 E-9 |
| **110** | 3.0 E-8 | 2.1 E-8 |
| **111** | 3.7 E-8 | 2.2 E-8 |
| **112** | 2.3 E-8 | 1.4 E-8 |
| **113** | 3.2 E-8 | 3.3 E-8 |
| **114** | 4.8 E-9 | 6.8 E-9 |
| **115** | 2.2 E-9 | 5.6 E-9 |
| **116** | 3.3 E-9 | 7.8 E-9 |

(continued)

| Example No | CSNK1A1 Assay 2 $IC_{50}$ [mol/l] (median) | CSNK1D $IC_{50}$ [mol/l] (median) |
|---|---|---|
| **117** | 4.7 E-9 | 7.4 E-9 |
| **118** | 8.6 E-9 | 1.2 E-8 |
| **119** | 1.5 E-8 | 1.1 E-8 |
| **120** | 6.5 E-9 | 8.1 E-9 |
| **121** | 7.0 E-9 | 1.3 E-8 |
| **122** | 3.6 E-9 | 9.0 E-9 |
| **123** | 2.5 E-8 | 2.2 E-8 |
| **124** | 5.6 E-9 | 1.1 E-8 |
| **125** | 3.3 E-9 | 5.9 E-9 |
| **126** | 5.5 E-8 | 6.3 E-8 |
| **127** | 3.3 E-8 | 3.3 E-8 |
| **128** | 1.4 E-8 | 1.2 E-8 |
| **129** | 1.5 E-8 | 1.3 E-8 |
| **130** | 2.4 E-9 | 3.9 E-9 |
| **131** | 7.0 E-9 | 1.0 E-8 |
| **132** | 6.3 E-9 | 1.1 E-8 |
| **133** | 2.3 E-9 | 4.8 E-9 |
| **134** | 5.5 E-9 | 7.0 E-9 |
| **135** | 7.8 E-9 | 5.4 E-9 |
| **136** | 3.1 E-9 | 2.1 E-9 |
| **137** | 2.6 E-9 | 3.0 E-9 |
| **138** | 8.9 E-9 | 8.8 E-9 |
| **139** | 4.2 E-9 | 5.5 E-9 |
| **140** | 1.6 E-9 | 1.8 E-9 |
| **141** | 3.6 E-9 | 2.7 E-9 |
| **142** | 2.7 E-9 | 3.8 E-9 |
| **143** | 4.0 E-9 | 2.7 E-9 |
| **144** | 2.0 E-9 | 3.0 E-9 |
| **145** | 9.7 E-9 | 1.0 E-8 |
| **146** | 3.3 E-9 | 4.4 E-9 |
| **147** | 4.3 E-9 | 2.9 E-9 |
| **148** | 1.2 E-8 | 6.8 E-9 |
| **149** | 3.7 E-9 | 3.8 E-9 |
| **150** | 4.1 E-9 | 3.8 E-9 |
| **151** | 1.7 E-8 | 1.9 E-8 |
| **152** | 4.3 E-9 | 4.5 E-9 |
| **153** | 2.0 E-9 | 2.4 E-9 |

(continued)

| Example No | CSNK1A1 Assay 2 $IC_{50}$ [mol/l] (median) | CSNK1D $IC_{50}$ [mol/l] (median) |
|---|---|---|
| **154** | 5.1 E-9 | 6.0 E-9 |
| **155** | 1.3 E-9 | 2.0 E-9 |
| **156** | 2.3 E-9 | 3.1 E-9 |
| **157** | 2.0 E-9 | 2.3 E-9 |
| **158** | 3.2 E-9 | 5.1 E-9 |
| **159** | 9.6 E-10 | 1.6 E-9 |
| **160** | 8.7 E-9 | 1.0 E-8 |
| **161** | 2.0 E-8 | 1.1 E-8 |
| **162** | 1.4 E-8 | 1.2 E-8 |

Table 3 shows the results of the inhibition in the WT-EGFR assay.

**Table 3**

| Example No | EGFR Wildtyp 2mM ATP $IC_{50}$ [mol/l] (median) |
|---|---|
| 1 | > 2.00 E-5 |
| 002 | > 2.00 E-5 |
| 003 | 1.08 E-7<br>8.69 E-8<br>> 2.00 E-5<br>> 2.00 E-5 |
| 004 | 3.01 E-7<br>2.92 E-7<br>> 2.00 E-5<br>> 2.00 E-5 |
| 005 | > 2.00 E-5 |
| 006 | > 2.00 E-5 |
| 007 | 1.18 E-5<br>1.34 E-5 |
| 008 | 7.82 E-6<br>7.83 E-6<br>> 2.00 E-5<br>> 2.00 E-5 |
| 009 | > 2.00 E-5 |
| 010 | > 2.00 E-5 |
| 011 | > 2.00 E-5 |
| 012 | > 2.00 E-5 |
| 013 | > 2.00 E-5 |
| 014 | > 2.00 E-5 |
| 015 | 1.91 E-6 |

(continued)

| Example No | EGFR Wildtyp 2mM ATP $IC_{50}$ [mol/l] (median) |
|---|---|
| 016 | > 2.00 E-5 |
| 017 | > 2.00 E-5 |
| 018 | > 2.00 E-5 |
| 019 | > 2.00 E-5 |
| 020 | > 2.00 E-5 |
| 021 | > 2.00 E-5 |
| 022 | > 2.00 E-5 |
| 023 | > 2.00 E-5 |
| 024 | > 2.00 E-5 |
| 025 | > 2.00 E-5 |
| 026 | > 2.00 E-5 |
| 027 | > 2.00 E-5 |
| 028 | > 5.71 E-6<br>> 5.71 E-6 |
| 029 | > 2.00 E-5 |
| 030 | > 2.00 E-5 |
| 031 | > 2.00 E-5 |
| 032 | > 2.00 E-5 |
| 033 | > 2.00 E-5 |
| 034 | > 2.00 E-5 |
| 035 | > 2.00 E-5 |
| 036 | > 2.00 E-5 |
| 037 | > 2.00 E-5 |
| 038 | > 2.00 E-5 |
| 039 | > 2.00 E-5 |
| 040 | > 2.00 E-5 |
| 041 | > 2.00 E-5 |
| 042 | 5.60 E-6 |
| 043 | > 2.00 E-5 |
| 044 | > 2.00 E-5 |
| 045 | > 2.00 E-5 |
| 046 | > 2.00 E-5 |
| 047 | > 2.00 E-5 |
| 048 | > 2.00 E-5 |
| 049 | > 2.00 E-5 |
| 050 | 1.19 E-5 |
| 051 | 3.20 E-6 |

(continued)

| Example No | EGFR Wildtyp 2mM ATP $IC_{50}$ [mol/l] (median) |
|---|---|
| 052 | > 2.00 E-5 |
| 053 | > 2.00 E-5 |
| 054 | > 2.00 E-5 |
| 055 | 3.43 E-6 |
| 056 | > 2.00 E-5 |
| 057 | > 2.00 E-5 |
| 058 | > 2.00 E-5 |
| 059 | > 2.00 E-5 |
| 060 | > 2.00 E-5 |
| 061 | 8.18 E-7 |
| 062 | > 5.71 E-6<br>> 5.71 E-6 |
| 063 | > 2.00 E-5 |
| 064 | > 2.00 E-5 |
| 065 | > 2.00 E-5 |
| 066 | > 2.00 E-5 |
| 067 | > 2.00 E-5 |
| 068 | > 2.00 E-5 |
| 069 | > 2.00 E-5 |
| 070 | > 2.00 E-5 |
| 071 | > 2.00 E-5 |
| 072 | > 2.00 E-5 |
| 073 | > 2.00 E-5 |
| 074 | > 2.00 E-5 |
| 075 | > 2.00 E-5 |
| 076 | > 2.00 E-5 |
| 079 | > 2.00 E-5 |
| 080 | > 2.00 E-5 |
| 081 | > 2.00 E-5 |
| 082 | > 2.00 E-5 |
| 083 | > 2.00 E-5 |
| 084 | 1.60 E-5<br>> 2.00 E-5 |
| 085 | > 5.71 E-6<br>> 5.71 E-6 |
| 086 | > 2.00 E-5 |
| 087 | > 2.00 E-5 |

(continued)

| Example No | EGFR Wildtyp 2mM ATP $IC_{50}$ [mol/l] (median) |
|---|---|
| 088 | > 2.00 E-5 |
| 089 | > 2.00 E-5 |
| 090 | > 2.00 E-5 |
| 091 | > 2.00 E-5 |
| 092 | > 2.00 E-5 |
| 093 | > 2.00 E-5 |
| 094 | > 2.00 E-5 |
| 095 | > 2.00 E-5 |
| 096 | > 2.00 E-5 |
| 097 | > 2.00 E-5 |
| 098 | > 2.00 E-5 |
| 099 | > 2.00 E-5 |
| 100 | > 2.00 E-5 |
| 101 | 3.24 E-8 |
| 102 | 8.75 E-7 |
| 103 | > 2.00 E-5 |
| 104 | > 2.00 E-5 |
| 105 | 8.22 E-6 |
| 106 | > 2.00 E-5 |
| 107 | > 2.00 E-5 |
| 108 | 6.63 E-7 |
| 109 | 3.85 E-6 |
| 110 | > 2.00 E-5 |
| 111 | > 2.00 E-5 |
| 112 | > 2.00 E-5 |
| 113 | > 2.00 E-5 |
| 114 | > 2.00 E-5 |
| 115 | > 2.00 E-5 |
| 116 | > 5.71 E-6<br>> 5.71 E-6 |
| 117 | > 2.00 E-5 |
| 118 | > 2.00 E-5 |
| 119 | > 2.00 E-5 |
| 120 | > 2.00 E-5 |
| 121 | > 5.71 E-6<br>> 5.71 E-6 |

(continued)

| Example No | EGFR Wildtyp 2mM ATP $IC_{50}$ [mol/l] (median) |
|---|---|
| 122 | > 5.71 E-6<br>> 5.71 E-6 |
| 123 | > 2.00 E-5 |
| 124 | > 2.00 E-5 |
| 125 | > 1.63 E-6<br>> 1.63 E-6 |
| 126 | > 2.00 E-5 |
| 127 | > 2.00 E-5 |
| 128 | > 2.00 E-5 |
| 129 | > 2.00 E-5 |
| 130 | > 2.00 E-5 |
| 131 | > 2.00 E-5 |
| 132 | > 2.00 E-5 |
| 133 | > 2.00 E-5 |
| 134 | > 2.00 E-5 |
| 135 | > 2.00 E-5 |
| 136 | > 2.00 E-5 |
| 137 | > 2.00 E-5 |
| 138 | > 2.00 E-5 |
| 139 | > 2.00 E-5<br>> 5.71 E-6 |
| 140 | > 2.00 E-5 |
| 141 | > 2.00 E-5 |
| 142 | > 2.00 E-5 |
| 143 | > 2.00 E-5 |
| 144 | 3.62 E-6 |
| 145 | > 2.00 E-5 |
| 146 | > 2.00 E-5 |
| 147 | > 2.00 E-5 |
| 148 | 8.75 E-6 |
| 149 | > 2.00 E-5 |
| 150 | > 2.00 E-5 |
| 151 | > 2.00 E-5 |
| 152 | > 2.00 E-5 |
| 153 | > 2.00 E-5 |
| 154 | > 2.00 E-5 |
| 155 | > 2.00 E-5 |

(continued)

| Example No | EGFR Wildtyp 2mM ATP $IC_{50}$ [mol/l] (median) |
|---|---|
| 156 | > 2.00 E-5 |
| 157 | > 2.00 E-5 |
| 158 | > 2.00 E-5 |
| 159 | > 2.00 E-5 |
| 160 | > 2.00 E-5 |
| 161 | > 2.00 E-5 |
| 162 | > 2.00 E-5 |

In particular, compounds of the present invention have surprisingly been found to effectively inhibit CSNK1A1. In certain embodiments, compounds of the present invention display an $IC_{50}$ of below 100 nM in a CSNK1A1 kinase assay in the presence of 1 μM ATP. Furthermore, in certain embodiments, compounds of the present invention additionally show low inhibition of wild type-EGFR kinase. In certain embodiments, compounds of the present invention are less potent than 600 nM in a wild type-EGFR kinase assay in the presence of 2 mM ATP. In certain embodiments, compounds of the present invention display an $IC_{50}$ below 100 nM in a CSNK1A1 kinase assay in the presence of 1 mM ATP and are less potent than 100 nM in a wild type-EGFR kinase assay in the presence of 2 mM ATP.

In contrast to the claimed compounds of this invention, compounds claimed in WO 2016/120196 do not show the advantageous combined properties described above. This can be seen in Table 4.

**Table 4**

| WO 2016/120196 **Example No.** | **CSNK1A1 Assay 1 $IC_{50}$ [mol/l] (median)** | **CSNK1A1 Assay 2 $IC_{50}$ [mol/l] (median)** | **EGFR Wildtyp-2mM ATP $IC_{50}$ [mol/l] (median)** |
|---|---|---|---|
| **14** | 2.03E-8 | | 1.04E-7 |
| **16** | 4.03E-8 | | 3.03E-7 |
| **23** | | 1.14E-8 | 1.50E-7 |
| **24** | < 3.43E-9<br>9.32E-9<br>1.01E-8<br>5.57E-9<br>4.38E-9 | 4.51E-9 | 4.26E-8 |
| **25** | 6.25E-8 | | 3.21E-7 |
| **40** | 2.41E-8 | | 8.55E-8 |

## Claims

**1.** A compound of formula (I)

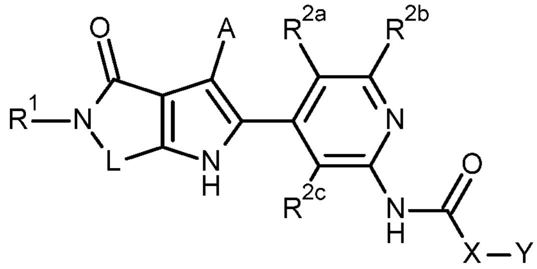

(I)

in which:

$R^1$ represents hydrogen or a group selected from $C_1$-$C_4$-alkyl, $C_3$-$C_6$-cycloalkyl, $C_3$-$C_6$-cycloalkyl-$C_1$-$C_4$-alkyl-, $C_1$-$C_3$-alkoxy-$C_1$-$C_3$-alkyl-, and $C_1$-$C_5$-hydroxyalkyl, wherein said groups are each independently optionally substituted, one or more times, with halogen;
$R^{2a}$ represents hydrogen, hydroxy, halogen, cyano, -$NH_2$, -NHMe, -$NMe_2$, $C_1$-$C_2$-alkoxy, $C_1$-$C_2$-haloalkyl, or $C_1$-$C_2$-haloalkoxy;
$R^{2b}$ represents hydrogen, hydroxy, halogen, cyano, -$NH_2$, -$NHR^9$, or -$NMe_2$;
$R^{2c}$ represents hydrogen, halogen, cyano, -$NMe_2$, $C_1$-$C_2$-alkoxy, or $C_1$-$C_2$-haloalkoxy;
L represents

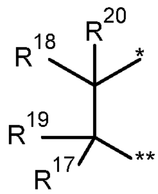

or

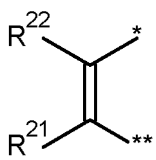

wherein * represents the point of direct attachment to the adjacent N-atom and ** represents the point of direct attachment to the adjacent C-atom;
$R^{17}$ represents hydrogen, $C_1$-$C_3$-alkyl, $C_1$-$C_3$-alkoxy, $C_1$-$C_3$-haloalkyl, or $C_3$-$C_6$-cycloalkyl-$C_1$-$C_4$-alkyl-;
$R^{18}$ represents hydrogen, $C_1$-$C_3$-alkyl, $C_1$-$C_3$-haloalkyl, or $C_3$-$C_6$-cycloalkyl-$C_1$-$C_4$-alkyl-, or
$R^{17}$ and $R^{18}$, together with the carbon atoms to which they are attached, form a $C_3$-$C_6$-cycloalkyl ring;
$R^{19}$ represents hydrogen, $C_1$-$C_3$-alkyl, $C_1$-$C_3$-haloalkyl, or $C_3$-$C_6$-cycloalkyl-$C_1$-$C_4$-alkyl-;
$R^{20}$ represents hydrogen, $C_1$-$C_3$-alkyl, $C_1$-$C_3$-alkoxy, $C_1$-$C_3$-haloalkyl, or $C_3$-$C_6$-cycloalkyl-$C_1$-$C_4$-alkyl-, or
$R^{18}$ and $R^{20}$, together with the carbon atom to which they are attached, form a $C_3$-$C_6$-cycloalkyl ring;
$R^{21}$ represents hydrogen, halogen, $C_1$-$C_3$-alkyl, $C_1$-$C_3$-haloalkyl, or $C_3$-$C_6$-cycloalkyl-$C_1$-$C_4$-alkyl-;
$R^{22}$ represents hydrogen, $C_1$-$C_3$-alkyl, $C_1$-$C_3$-haloalkyl, or $C_3$-$C_6$-cycloalkyl-$C_1$-$C_4$-alkyl-;
X represents methylene or ethylene,
wherein said methylene and ethylene groups are each independently optionally substituted, one or more times, with $R^3$;
Y represents phenyl or a heteroaryl group,
wherein said phenyl and heteroaryl groups are each independently optionally substituted, one or more times, with $R^4$;
$R^3$ represents hydroxy, halogen, cyano, $C_1$-$C_3$-alkyl, $C_1$-$C_3$-alkoxy, $C_1$-$C_3$-haloalkyl, $C_1$-$C_2$-haloalkoxy, $C_3$-$C_4$-cycloalkyl, -$NHR^6$, $R^5O$-$C_1$-$C_3$-alkyl, $R^6R^7N$-$C_1$-$C_5$-alkyl, or $R^6R^7N$-X'-$C(R^{10})(R^{11})$-$C_1$-$C_2$-alkyl-;

X' represents methylene or ethylene;
$R^4$ represents hydroxy, halogen, cyano, $C_1$-$C_4$-alkyl, $C_1$-$C_4$-alkoxy, $C_1$-$C_4$-haloalkyl, $C_1$-$C_4$-haloalkoxy, -$NMe_2$, -$CO_2R^{14}$, -$CONR^{15}R^{16}$, or -$SO_2Me$;
$R^5$ represents hydrogen, $C_1$-$C_2$-alkyl, $C_1$-$C_2$-haloalkyl, or $C_3$-$C_4$-cycloalkyl;
$R^6$ represents hydrogen, $C_1$-$C_2$-alkyl, $C_1$-$C_2$-haloalkyl, or $C_3$-$C_4$-cycloalkyl;
$R^7$ represents hydrogen, $C_1$-$C_2$-alkyl, $C_1$-$C_2$-haloalkyl, $C_3$-$C_4$-cycloalkyl, or -$CO_2$-$C_1$-$C_4$-alkyl, or
$R^6$ and $R^7$, together with the nitrogen atom to which they are attached, represent a 4- to 7-membered heterocyclic ring, wherein said heterocyclic ring optionally contains one or more further heteroatoms selected from N, O, S, or groups selected from C(=O), S(=O), and $SO_2$, and is independently optionally substituted, one or more times, with halogen, $C_1$-$C_3$-alkyl, $C_3$-cycloalkyl-$C_1$-$C_2$-alkyl-, -$NH_2$, -$NHR^9$, -$NR^9R^{9a}$, or hydroxy;
A represents a group selected from:

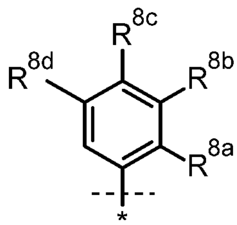
,
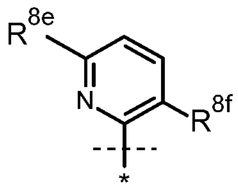
,
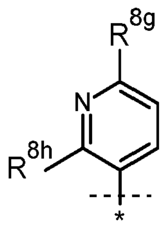
,
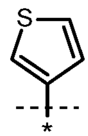
,

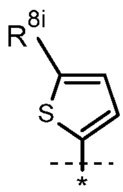
,
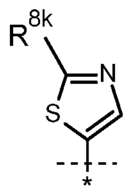
,
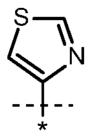
,
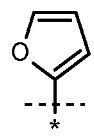
,
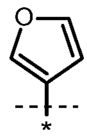
, and
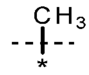
,

wherein * indicates the point of attachment of said group to the rest of the formula (I);
$R^{8a}$ represents hydrogen, hydroxy, halogen, cyano, $C_1$-$C_2$-alkyl, or $C_1$-$C_2$-haloalkyl;
$R^{8b}$ represents hydrogen, hydroxy, halogen, cyano, $C_1$-$C_2$-alkyl, or $C_1$-$C_2$-haloalkyl;
$R^{8c}$ represents hydrogen, hydroxy, halogen, $C_1$-$C_2$-alkyl, or $C_1$-$C_2$-haloalkyl;
$R^{8d}$ represents hydrogen, hydroxy, halogen, cyano, $C_1$-$C_2$-alkyl, or $C_1$-$C_2$-haloalkyl;
$R^{8e}$ represents hydrogen, hydroxy, halogen, cyano, $C_1$-$C_2$-alkyl, or $C_1$-$C_2$-haloalkyl;
$R^{8f}$ represents hydrogen, hydroxy, halogen, cyano, $C_1$-$C_2$-alkyl, or $C_1$-$C_2$-haloalkyl;
$R^{8g}$ represents hydrogen, hydroxy, amino, halogen, cyano, $C_1$-$C_2$-alkyl, or $C_1$-$C_2$-haloalkyl;
$R^{8h}$ represents hydrogen, hydroxy, halogen, cyano, $C_1$-$C_2$-alkyl, or $C_1$-$C_2$-haloalkyl;
$R^{8i}$ represents hydrogen, hydroxy, halogen, cyano, $C_1$-$C_2$-alkyl, or $C_1$-$C_2$-haloalkyl;
$R^{8k}$ represents hydrogen, hydroxy, halogen, cyano, $C_1$-$C_2$-alkyl, or $C_1$-$C_2$-haloalkyl;
$R^9$ represents $C_1$-$C_3$-alkyl or cyclopropyl;
$R^{9a}$ represents $C_1$-$C_3$-alkyl or cyclopropyl;
$R^{10}$ represents methyl, halogen, or hydroxy;
$R^{11}$ represents methyl, halogen, or hydroxy;
$R^{14}$ represents hydrogen, methyl, or ethyl;
$R^{15}$ represents hydrogen, methyl, or ethyl;
$R^{16}$ represents hydrogen, methyl, ethyl, methoxyethyl, or dimethylaminoethyl, or
$R^{15}$ and $R^{16}$, together with the nitrogen atom to which they are attached, represent a 5- to 6-membered heterocyclic ring, wherein said heterocyclic ring optionally contains one or two further heteroatoms selected from N, O, S, or groups selected from C(=O), S(=O), and $SO_2$;
or an N-oxide, a salt, a tautomer or a stereoisomer of said compound, or a salt of said N-oxide, tautomer or stereoisomer.

**2.** The compound of formula (I) according to claim 1, wherein:

$R^1$ represents hydrogen or a group selected from $C_1$-$C_2$-alkyl and $C_3$-$C_4$-cycloalkyl, wherein said groups are

each independently optionally substituted, one or more times, with halogen;
$R^{2a}$ represents hydrogen or halogen;
$R^{2b}$ represents hydrogen, halogen, $-NH_2$, or $-NHR^9$;
$R^{2c}$ represents hydrogen or halogen;
L represents

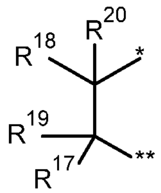

or

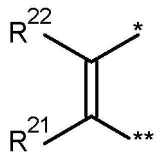

wherein * represents the point of direct attachment to the adjacent N-atom and ** represents the point of direct attachment to the adjacent C-atom;
$R^{17}$ represents hydrogen, $C_1$-$C_3$-alkyl, $C_1$-$C_3$-haloalkyl, or $C_3$-$C_6$-cycloalkyl-$C_1$-$C_4$-alkyl-;
$R^{18}$ represents hydrogen, $C_1$-$C_3$-alkyl, $C_1$-$C_3$-haloalkyl, or $C_3$-cycloalkyl-$C_1$-alkyl-, or
$R^{17}$ and $R^{18}$, together with the carbon atoms to which they are attached, form a $C_3$-$C_5$-cycloalkyl ring;
$R^{19}$ represents hydrogen, $C_1$-$C_3$-alkyl, $C_1$-$C_3$-haloalkyl, or $C_3$-$C_6$-cycloalkyl-$C_1$-$C_4$-alkyl-;
$R^{20}$ represents hydrogen or $C_1$-$C_3$-alkyl, or
$R^{18}$ and $R^{20}$, together with the carbon atom to which they are attached, form a $C_3$-$C_6$-cycloalkyl ring;
$R^{21}$ represents hydrogen, halogen, $C_1$-$C_3$-alkyl, $C_1$-$C_3$-haloalkyl, or $C_3$-$C_6$-cycloalkyl-$C_1$-$C_4$-alkyl-;
$R^{22}$ represents hydrogen or $C_1$-$C_3$-alkyl;
X represents methylene,
wherein said methylene group is optionally substituted, one or more times, with $R^3$;
Y represents phenyl or a heteroaryl group,
wherein said phenyl and heteroaryl groups are each independently optionally substituted, one or more times, with $R^4$;
$R^3$ represents $C_1$-$C_3$-alkyl, $C_1$-$C_3$-haloalkyl, $-NHR^6$, $R^5O$-$C_1$-$C_3$-alkyl, or $R^6R^7N$-$C_1$-$C_4$-alkyl;
$R^4$ represents, halogen, $C_1$-$C_4$-alkyl, $C_1$-$C_4$-haloalkyl, $C_1$-$C_4$-haloalkoxy, $-CO_2R^{14}$, $-CONR^{15}R^{16}$, or $-SO_2Me$;
$R^5$ represents hydrogen, $C_1$-$C_2$-alkyl, or $C_1$-$C_2$-haloalkyl;
$R^6$ represents hydrogen, $C_1$-$C_2$-alkyl, $C_1$-$C_2$-haloalkyl, or $C_3$-$C_4$-cycloalkyl;
$R^7$ represents hydrogen, $C_1$-$C_2$-alkyl, $C_1$-$C_2$-haloalkyl, $C_3$-$C_4$-cycloalkyl, or $-CO_2$-$C_1$-$C_4$-alkyl, or
$R^6$ and $R^7$, together with the nitrogen atom to which they are attached, represent a 4- to 7-membered heterocyclic ring, wherein said heterocyclic ring optionally contains one or more further heteroatoms selected from N, O, S, and is independently optionally substituted, one or more times, with halogen, $C_1$-$C_3$-alkyl, $C_3$-cycloalkyl-$C_1$-$C_2$-alkyl-, $-NH_2$, $-NHR^9$, $-NR^9R^{9a}$, or hydroxy;
A represents a group selected from:

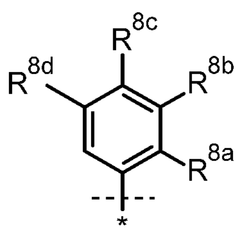
,
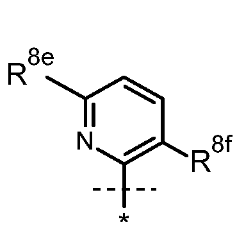
,
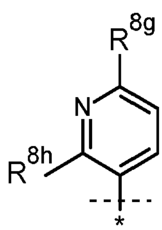
,
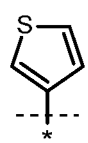
,

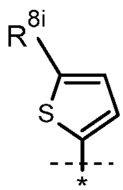 , 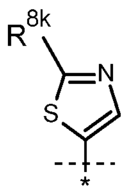 , 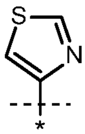 , 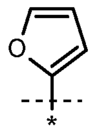 , 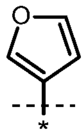 , and 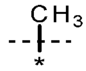 ,

wherein * indicates the point of attachment of said group to the rest of the formula (I);
$R^{8a}$ represents hydrogen, hydroxy, halogen, cyano, or $C_1$-alkyl;
$R^{8b}$ represents hydrogen, hydroxy, halogen, or $C_1$-alkyl;
$R^{8c}$ represents hydrogen, hydroxy, halogen, or $C_1$-alkyl;
$R^{8d}$ represents hydrogen, hydroxy, or halogen;
$R^{8e}$ represents hydrogen;
$R^{8f}$ represents hydrogen;
$R^{8g}$ represents hydrogen, amino, or halogen;
$R^{8h}$ represents hydrogen or halogen;
$R^{8i}$ represents hydrogen, halogen, or $C_1$-$C_2$-alkyl;
$R^{8k}$ represents hydrogen or $C_1$-alkyl;
$R^9$ represents $C_1$-$C_3$-alkyl or cyclopropyl;
$R^{9a}$ represents $C_1$-$C_3$-alkyl or cyclopropyl;
$R^{14}$ represents hydrogen, methyl, or ethyl;
$R^{15}$ represents hydrogen, methyl, or ethyl;
$R^{16}$ represents hydrogen, methyl, ethyl, methoxyethyl, or dimethylaminoethyl, or
$R^{15}$ and $R^{16}$, together with the nitrogen atom to which they are attached, represent a 5- to 6-membered heterocyclic ring, wherein said heterocyclic ring optionally contains one or two further heteroatoms selected from N, and O;

or an N-oxide, a salt, a tautomer or a stereoisomer of said compound, or a salt of said N-oxide, tautomer or stereoisomer.

**3.** The compound of formula (I) according to claim 1 or 2, wherein:

$R^1$ represents hydrogen or a group selected from $C_1$-$C_2$-alkyl and $C_3$-$C_4$-cycloalkyl;
$R^{2a}$ represents hydrogen or halogen;
$R^{2b}$ represents hydrogen;
$R^{2c}$ represents hydrogen or halogen;
L represents

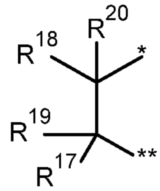

or

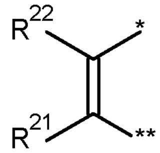

wherein * represents the point of direct attachment to the adjacent N-atom and ** represents the point of direct attachment to the adjacent C-atom;

$R^{17}$ represents hydrogen, $C_1$-$C_3$-alkyl, $C_1$-$C_3$-haloalkyl, or $C_3$-$C_4$-cycloalkyl-$C_1$-alkyl-;
$R^{18}$ represents hydrogen or $C_1$-$C_3$-alkyl;
$R^{19}$ represents hydrogen, $C_1$-$C_3$-alkyl, $C_1$-$C_3$-haloalkyl, or $C_3$-$C_4$-cycloalkyl-$C_1$-alkyl-;
$R^{20}$ represents hydrogen or $C_1$-$C_3$-alkyl, or
$R^{18}$ and $R^{20}$, together with the carbon atom to which they are attached, form a $C_3$-$C_6$-cycloalkyl ring;
$R^{21}$ represents hydrogen, halogen, $C_1$-$C_3$-alkyl, $C_1$-$C_3$-haloalkyl, or $C_3$-$C_4$-cycloalkyl-$C_1$-alkyl-;
$R^{22}$ represents hydrogen;
X represents methylene,
wherein said methylene group is optionally substituted, one or two times, with $R^3$;
Y represents phenyl,
wherein said phenyl is optionally substituted, one or more times, with $R^4$;
$R^3$ represents $C_1$-$C_3$-alkyl, $C_1$-$C_3$-haloalkyl, or $R^5O$-$C_1$-$C_3$-alkyl;
$R^4$ represents, halogen, $C_1$-$C_4$-alkyl, $C_1$-$C_4$-haloalkyl, -$CONR^{15}R^{16}$, or -$SO_2Me$;
$R^5$ represents hydrogen;
A represents a group selected from:

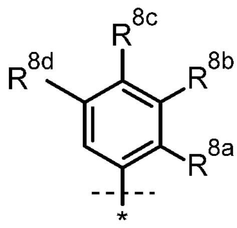 , 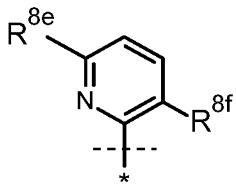 , 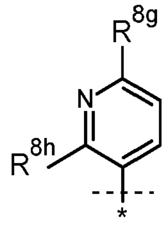 , 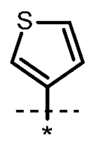 ,

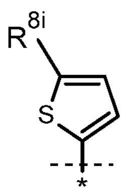 , 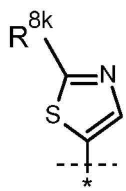 , 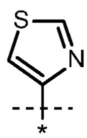 , 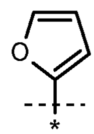 , 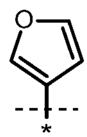 , and 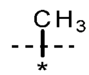 ,

wherein * indicates the point of attachment of said group to the rest of the formula (I);
$R^{8a}$ represents hydrogen, hydroxy, halogen, or cyano;
$R^{8b}$ represents hydrogen, halogen, or $C_1$-alkyl;
$R^{8c}$ represents hydrogen, halogen, or $C_1$-alkyl;
$R^{8d}$ represents hydrogen or halogen;
$R^{8e}$ represents hydrogen;
$R^{8f}$ represents hydrogen;
$R^{8g}$ represents hydrogen or halogen;
$R^{8h}$ represents hydrogen;
$R^{8i}$ represents hydrogen, halogen, or $C_1$-$C_2$-alkyl;
$R^{8k}$ represents hydrogen or $C_1$-alkyl;
$R^{15}$ represents hydrogen or methyl;
$R^{16}$ represents methyl or methoxyethyl, or
$R^{15}$ and $R^{16}$, together with the nitrogen atom to which they are attached, represent a 5- to 6-membered heterocyclic ring, wherein said heterocyclic ring optionally contains one further heteroatoms selected from N, and O;

or an N-oxide, a salt, a tautomer or a stereoisomer of said compound, or a salt of said N-oxide, tautomer or stereoisomer.

**4.** The compound of formula (I) according to claim 1, 2 or 3, wherein:

$R^1$ represents hydrogen or a group selected from $C_1$-alkyl and $C_3$-cycloalkyl;
$R^{2a}$ represents hydrogen;

$R^{2b}$ represents hydrogen;
$R^{2c}$ represents hydrogen;
L represents

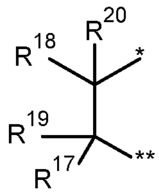

or

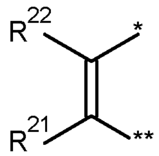

wherein * represents the point of direct attachment to the adjacent N-atom and ** represents the point of direct attachment to the adjacent C-atom;
$R^{17}$ represents hydrogen or $C_1$-alkyl;
$R^{18}$ represents hydrogen or $C_1$-alkyl;
$R^{19}$ represents hydrogen, $C_1$-$C_3$-alkyl, $C_1$-$C_3$-haloalkyl, or $C_3$--cycloalkyl-$C_1$-alkyl-;
$R^{20}$ represents hydrogen or $C_1$-alkyl, or
$R^{18}$ and $R^{20}$, together with the carbon atom to which they are attached, form a $C_3$-$C_4$-cycloalkyl ring;
$R^{21}$ represents hydrogen, $C_1$-$C_3$-alkyl, $C_1$-$C_3$-haloalkyl, or $C_3$-cycloalkyl-$C_1$-alkyl-;
$R^{22}$ represents hydrogen;
X represents methylene,
wherein said methylene group is optionally substituted once with $R^3$;
Y represents phenyl,
wherein said phenyl is optionally substituted once with $R^4$;
$R^3$ represents $C_1$-$C_3$-alkyl or $C_1$-$C_2$-haloalkyl;
$R^4$ represents halogen or -CONR$^{15}$R$^{16}$,
A represents a group selected from:

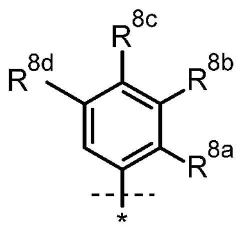 , 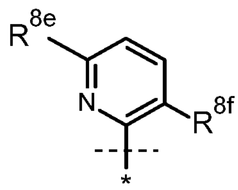 ,

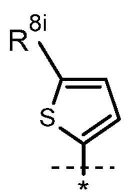 , 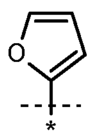 and 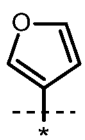 ,

wherein * indicates the point of attachment of said group to the rest of the formula (I);
$R^{8a}$ represents hydrogen or fluoro;

$R^{8b}$ represents hydrogen or fluoro;
$R^{8c}$ represents hydrogen, fluoro, or chloro;
$R^{8d}$ represents hydrogen;
$R^{8e}$ represents hydrogen;
$R^{8f}$ represents hydrogen;
$R^{8i}$ represents hydrogen, chloro, or $C_1$-alkyl;
$R^{15}$ represents hydrogen;
$R^{16}$ represents methoxyethyl, or
$R^{15}$ and $R^{16}$, together with the nitrogen atom to which they are attached, represent a 5- to 6-membered heterocyclic ring, wherein said heterocyclic ring optionally contains one further heteroatom N;

or an N-oxide, a salt, a tautomer or a stereoisomer of said compound, or a salt of said N-oxide, tautomer or stereoisomer.

**5.** The compound of formula (I) according to any of claims 1 to 4, which is selected from:

2-(4-fluorophenyl)-N-[4-(5-methyl-4-oxo-3-phenyl-4,5,6,7-tetrahydro-1H-pyrrolo[3,2-c]pyridin-2-yl)pyridin-2-yl]acetamide;
4,4-difluoro-2-(4-fluorophenyl)-N-[4-(5-methyl-4-oxo-3-phenyl-4,5,6,7-tetrahydro-1H-pyrrolo[3,2-c]pyridin-2-yl)pyridin-2-yl]butanamide;
(2R)-4,4-difluoro-2-(4-fluorophenyl)-N-[4-(5-methyl-4-oxo-3-phenyl-4,5,6,7-tetrahydro-1H-pyrrolo[3,2-c]pyridin-2-yl)pyridin-2-yl]butanamide;
(2S)-4,4-difluoro-2-(4-fluorophenyl)-N-[4-(5-methyl-4-oxo-3-phenyl-4, 5,6,7-tetrahydro-1H-pyrrolo[3,2-c]pyridin-2-yl)pyridin-2-yl]butanamide;
2-(4-fluorophenyl)-N-[4-(5-methyl-4-oxo-3-phenyl-4,5-dihydro-1H-pyrrolo[3,2-c]pyridin-2-yl)pyridin-2-yl]acetamide;
4,4-difluoro-2-(4-fluorophenyl)-N- [4-(5 -methyl-4-oxo-3 -phenyl-4,5-dihydro-1H-pyrrolo[3,2-c]pyridin-2-yl)pyridin-2-yl]butanamide;
(2R)-4,4-difluoro-2-(4-fluorophenyl)-N-[4-(5-methyl-4-oxo-3-phenyl-4,5-dihydro-1H-pyrrolo[3,2-c]pyridin-2-yl)pyridin-2-yl]butanamide;
(2S)-4,4-difluoro-2-(4-fluorophenyl)-N-[4-(5-methyl-4-oxo-3-phenyl-4,5-dihydro-1H-pyrrolo[3,2-c]pyridin-2-yl)pyridin-2-yl]butanamide;
4,4-difluoro-2-(4-fluorophenyl)-N-{4-[5-methyl-4-oxo-3-(pyridin-3-yl)-4,5,6,7-tetrahydro-1H-pyrrolo[3,2-c]pyridin-2-yl]pyridin-2-yl}butanamide;
(2R)-4,4-difluoro-2-(4-fluorophenyl)-N-{4-[5-methyl-4-oxo-3-(pyridin-3-yl)-4,5,6,7-tetrahydro-1H-pyrrolo[3,2-c]pyridin-2-yl]pyridin-2-yl}butanamide;
(2S)-4,4-difluoro-2-(4-fluorophenyl)-N-{4-[5-methyl-4-oxo-3-(pyridin-3-yl)-4,5,6,7-tetrahydro-1H-pyrrolo[3,2-c]pyridin-2-yl]pyridin-2-yl}butanamide;
2-(4-fluorophenyl)-N-{4-[5-methyl-4-oxo-3-(pyridin-3-yl)-4,5,6,7-tetrahydro-1H-pyrrolo[3,2-c]pyridin-2-yl]pyridin-2-yl}acetamide;
2-(4-fluorophenyl)-N-{4-[5-methyl-4-oxo-3-phenyl-7-(2,2,2-trifluoroethyl)-4,5,6,7-tetrahydro-1H-pyrrolo[3,2-c]pyridin-2-yl]pyridin-2-yl}propanamide;
2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}propanamide;
(2R)-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}propanamide;
(2S)-2-(4-fluorophenyl)-N- {4-[5-methyl-4-oxo-3-phenyl-7-(2,2,2-trifluoroethyl)-4,5-dihydro-1H-pyrrolo[3,2-c]pyridin-2-yl]pyridin-2-yl}propanamide;
4,4-difluoro-2-(4-fluorophenyl)-N-{4-[5-methyl-4-oxo-3-phenyl-7-(2,2,2-trifluoroethyl)-4,5,6,7-tetrahydro-1H-pyrrolo[3,2-c]pyridin-2-yl]pyridin-2-yl} butanamide;
4,4-difluoro-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl} butanamide;
(2R)-4,4-difluoro-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}butanamide;
(2S)-4,4-difluoro-2-(4-fluorophenyl)-N-{4-[5-methyl-4-oxo-3-phenyl-7-(2,2,2-trifluoroethyl)-4,5-dihydro-1H-pyrrolo[3,2-c]pyridin-2-yl]pyridin-2-yl}butanamide;
2-(4-fluorophenyl)-N- {4-[3-(4-fluorophenyl)-5-methyl-4-oxo-7-(2,2,2-trifluoroethyl)-4,5,6,7-tetrahydro-1H-pyrrolo[3,2-c]pyridin-2-yl]pyridin-2-yl}propanamide;

2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}propanamide;
(2R)-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}propanamide;
(2S)-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}propanamide;;
4,4-difluoro-2-(4-fluorophenyl)-N-{4-[3-(4-fluorophenyl)-5-methyl-4-oxo-7-(2,2,2-trifluoroethyl)-4,5,6,7-tetrahydro-1H-pyrrolo[3,2-c]pyridin-2-yl]pyridin-2-yl} butanamide;
4,4-difluoro-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}butanamide;
(2R)-4,4-difluoro-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}butanamide;
(2S)-4,4-difluoro-2-(4-fluorophenyl)-N-{4-[3-(4-fluorophenyl)-5-methyl-4-oxo-7-(2,2,2-trifluoroethyl)-4,5-dihydro-1H-pyrrolo[3,2-c]pyridin-2-yl]pyridin-2-yl}butanamide;
2-(4-fluorophenyl)-N-{4-[3-(4-fluorophenyl)-5,7-dimethyl-4-oxo-4,5,6,7-tetrahydro-1H-pyrrolo[3,2-c]pyridin-2-yl]pyridin-2-yl}propanamide;
2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}propanamide;
(2R)-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}propanamide;
(2S)-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}propanamide;
4,4-difluoro-2-(4-fluorophenyl)-N- {4-[3-(4-fluorophenyl)-5, 7-dimethyl-4-oxo-4, 5,6,7-tetrahydro-1H-pyrrolo[3,2-c]pyridin-2-yl]pyridin-2-yl} butanamide;
4,4-difluoro-2-(4-fluorophenyl)-N- {4-[3-(4-fluorophenyl)-5, 7-dimethyl-4-oxo-4, 5-dihydro-1H-pyrrolo[3,2-c]pyridin-2-yl]pyridin-2-yl}butanamide;
(2R)-4,4-difluoro-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}butanamide;
(2S)-4,4-difluoro-2-(4-fluorophenyl)-N-{4-[3-(4-fluorophenyl)-5,7-dimethyl-4-oxo-4,5-dihydro-1H-pyrrolo[3,2-c]pyridin-2-yl]pyridin-2-yl}butanamide;
N-{4-[5,7-dimethyl-4-oxo-3-phenyl-4,5,6,7-tetrahydro-1H-pyrrolo[3,2-c]pyridin-2-yl]pyridin-2-yl} -2-(4-fluorophenyl)propanamide;
N-[4-(5,7-dimethyl-4-oxo-3-phenyl-4,5-dihydro-1H-pyrrolo[3,2-c]pyridin-2-yl)pyridin-2-yl]-2-(4-fluorophenyl)propanamide;
N-{4-[5,7-dimethyl-4-oxo-3-phenyl-4,5,6,7-tetrahydro-1H-pyrrolo[3,2-c]pyridin-2-yl]pyridin-2-yl}-4,4-difluoro-2-(4-fluorophenyl)butanamide;
N-[4-(5,7-dimethyl-4-oxo-3-phenyl-4,5-dihydro-1H-pyrrolo[3,2-c]pyridin-2-yl)pyridin-2-yl]-4,4-difluoro-2-(4-fluorophenyl)butanamide;
4,4-difluoro-2-(4-fluorophenyl)-N-{4-[5,6,7-trimethyl-4-oxo-3-phenyl-4,5,6,7-tetrahydro-1H-pyrrolo[3,2-c]pyridin-2-yl]pyridin-2-yl}butanamide;
N-[4-(7,7-dimethyl-4-oxo-3-phenyl-4,5,6,7-tetrahydro-1H-pyrrolo[3,2-c]pyridin-2-yl)pyridin-2-yl]-2-(4-fluorophenyl)acetamide;
N-[4-(7,7-dimethyl-4-oxo-3-phenyl-4,5,6,7-tetrahydro-1H-pyrrolo[3,2-c]pyridin-2-yl)pyridin-2-yl]-4,4-difluoro-2-(4-fluorophenyl)butanamide;
(2R)-N-[4-(7,7-dimethyl-4-oxo-3-phenyl-4,5,6,7-tetrahydro-1H-pyrrolo[3,2-c]pyridin-2-yl)pyridin-2-yl]-4,4-difluoro-2-(4-fluorophenyl)butanamide;
(2S)-N-[4-(7,7-dimethyl-4-oxo-3-phenyl-4,5,6,7-tetrahydro-1H-pyrrolo[3,2-c]pyridin-2-yl)pyridin-2-yl]-4,4-difluoro-2-(4-fluorophenyl)butanamide;
N-[4-(7,7-dimethyl-4-oxo-3-phenyl-4,5,6,7-tetrahydro-1H-pyrrolo[3,2-c]pyridin-2-yl)pyridin-2-yl]-2-(4-fluorophenyl)propanamide;
(2R)-N-[4-(7,7-dimethyl-4-oxo-3-phenyl-4,5,6,7-tetrahydro-1H-pyrrolo[3,2-c]pyridin-2-yl)pyridin-2-yl]-2-(4-fluorophenyl)propanamide;
(2S)-N-[4-(7,7-dimethyl-4-oxo-3-phenyl-4,5,6,7-tetrahydro-1H-pyrrolo[3,2-c]pyridin-2-yl)pyridin-2-yl]-2-(4-fluorophenyl)propanamide;
4,4-difluoro-2-(4-fluorophenyl)-N-[4-(5'-methyl-4'-oxo-3'-phenyl-1',4',5',7'-tetrahydrospiro[cyclobutane-1,6'-pyrrolo[3,2-c]pyridin]-2'-yl)pyridin-2-yl]butanamide;
(2R)-4,4-difluoro-2-(4-fluorophenyl)-N-[4-(5'-methyl-4'-oxo-3'-phenyl-1',4',5',7'-tetrahydrospiro-[cyclobutane-1,6'-pyrrolo[3,2-c]pyridin]-2'-yl)pyridin-2-yl]butanamide;

(2S)-4,4-difluoro-2-(4-fluorophenyl)-N-[4-(5'-methyl-4'-oxo-3'-phenyl-1',4',5',7'-tetrahydrospiro-[cyclobutane-1,6'-pyrrolo[3,2-c]pyridin]-2'-yl)pyridin-2-yl]butanamide;
2-(4-fluorophenyl)-N-[4-(5'-methyl-4'-oxo-3'-phenyl-1',4',5',7'-tetrahydrospiro[cyclobutane-1,6'-pyrrolo[3,2-c]pyridin]-2'-yl)pyridin-2-yl]propanamide;
(2R)-2-(4-fluorophenyl)-N-[4-(5'-methyl-4'-oxo-3'-phenyl-1',4',5',7'-tetrahydrospiro[cyclobutane-1,6'-pyrrolo[3,2-c]pyridin]-2'-yl)pyridin-2-yl]propanamide;
(2S)-2-(4-fluorophenyl)-N-[4-(5'-methyl-4'-oxo-3'-phenyl-1',4',5',7'-tetrahydrospiro[cyclobutane-1,6'-pyrrolo[3,2-c]pyridin]-2'-yl)pyridin-2-yl]propanamide;
N-{4-[7-(cyclopropylmethyl)-5-methyl-4-oxo-3-phenyl-4,5,6,7-tetrahydro-1H-pyrrolo[3,2-c]pyridin-2-yl]pyridin-2-yl}-2-(4-fluorophenyl)acetamide;
N-{4-[7-(cyclopropylmethyl)-5-methyl-4-oxo-3-phenyl-4,5-dihydro-1H-pyrrolo[3,2-c]pyridin-2-yl]pyridin-2-yl}-2-(4-fluorophenyl)acetamide;
N-{4-[7-(cyclopropylmethyl)-5-methyl-4-oxo-3-phenyl-4,5,6,7-tetrahydro-1H-pyrrolo[3,2-c]pyridin-2-yl]pyridin-2-yl}-2-(4-fluorophenyl)propanamide;
N-{4-[7-(cyclopropylmethyl)-5-methyl-4-oxo-3-phenyl-4,5-dihydro-1H-pyrrolo[3,2-c]pyridin-2-yl]pyridin-2-yl}-2-(4-fluorophenyl)propanamide;
N-{4-[7-(cyclopropylmethyl)-5-methyl-4-oxo-3-phenyl-4,5,6,7-tetrahydro-1H-pyrrolo[3,2-c]pyridin-2-yl]pyridin-2-yl}-4,4-difluoro-2-(4-fluorophenyl)butanamide;
N-{4-[7-(cyclopropylmethyl)-5-methyl-4-oxo-3-phenyl-4,5-dihydro-1H-pyrrolo[3,2-c]pyridin-2-yl]pyridin-2-yl}-4,4-difluoro-2-(4-fluorophenyl)butanamide;
N-{4-[7-(cyclopropylmethyl)-3-(4-fluorophenyl)-5-methyl-4-oxo-4,5,6,7-tetrahydro-1H-pyrrolo[3,2-c]pyridin-2-yl]pyridin-2-yl}-2-(4-fluorophenyl)acetamide;
N-{4-[7-(cyclopropylmethyl)-3-(4-fluorophenyl)-5-methyl-4-oxo-4,5,6,7-tetrahydro-1H-pyrrolo[3,2-c]pyridin-2-yl]pyridin-2-yl}-2-(4-fluorophenyl)propanamide;
N-{4-[7-(cyclopropylmethyl)-3-(4-fluorophenyl)-5-methyl-4-oxo-4,5-dihydro-1H-pyrrolo[3,2-c]pyridin-2-yl]pyridin-2-yl}-2-(4-fluorophenyl)propanamide;
N-{4-[7-(cyclopropylmethyl)-3-(4-fluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide;
N-{4-[7-(cyclopropylmethyl)-3-(4-fluorophenyl)-5-methyl-4-oxo-4,5-dihydro-1H-pyrrolo[3,2-c]pyridin-2-yl]pyridin-2-yl}-4,4-difluoro-2-(4-fluorophenyl)butanamide;
N-{4-[3-(2,4-difluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide;
(2R)-N-{4-[3-(2,4-difluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide;
(2S)-N-{4-[3-(2,4-difluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide;
N-{4-[3-(2,4-difluorophenyl)-5-methyl-4-oxo-4,5,6,7-tetrahydro-1H-pyrrolo[3,2-c]pyridin-2-yl]pyridin-2-yl} -2-(4-fluorophenyl)propanamide;
(2R)-N-{4-[3-(2,4-difluorophenyl)-5-methyl-4-oxo-4,5,6,7-tetrahydro-1H-pyrrolo[3,2-c]pyridin-2-yl]pyridin-2-yl}-2-(4-fluorophenyl)propanamide;
(2S)-N-{4-[3-(2,4-difluorophenyl)-5-methyl-4-oxo-4,5,6,7-tetrahydro-1H-pyrrolo[3,2-c]pyridin-2-yl]pyridin-2-yl}-2-(4-fluorophenyl)propanamide;
2-(4-fluorophenyl)-N-[4-(4-oxo-3-phenyl-4,5,6,7-tetrahydro-1H-pyrrolo[3,2-c]pyridin-2-yl)pyridin-2-yl]acetamide;
2-(4-fluorophenyl)-N-[4-(4-oxo-3-phenyl-4,5-dihydro-1H-pyrrolo[3,2-c]pyridin-2-yl)pyridin-2-yl]acetamide;
2-(4-fluorophenyl)-N-[4-(4-oxo-3-phenyl-4,5,6,7-tetrahydro-1H-pyrrolo[3,2-c]pyridin-2-yl)pyridin-2-yl]propanamide;
4,4-difluoro-2-(4-fluorophenyl)-N-[4-(4-oxo-3-phenyl-4, 5,6, 7-tetrahydro-1H-pyrrolo[3,2-c]pyridin-2-yl)pyridin-2-yl]butanamide;
4,4-difluoro-2-(4-fluorophenyl)-N-[4-(4-oxo-3-phenyl-4,5-dihydro-1H-pyrrolo[3,2-c]pyridin-2-yl)pyridin-2-yl]butanamide;
(2R)-4,4-difluoro-2-(4-fluorophenyl)-N-[4-(4-oxo-3-phenyl-4,5-dihydro-1H-pyrrolo[3,2-c]pyridin-2-yl)pyridin-2-yl]butanamide;
(2S)-4,4-difluoro-2-(4-fluorophenyl)-N-[4-(4-oxo-3-phenyl-4,5-dihydro-1H-pyrrolo[3,2-c]pyridin-2-yl)pyridin-2-yl]butanamide;
N-[4-(7-chloro-5-methyl-4-oxo-3-phenyl-4,5-dihydro-1H-pyrrolo[3,2-c]pyridin-2-yl)pyridin-2-yl]-2-(4-fluorophenyl)acetamide;
4,4-difluoro-2-(4-fluorophenyl)-N-[4-(5'-methyl-4'-oxo-3'-phenyl-1',4',5',7'-tetrahydrospiro-[cyclopropane-1,6'-pyrrolo[3,2-c]pyridin]-2'-yl)pyridin-2-yl]butanamide;
4,4-difluoro-2-(4-fluorophenyl)-N-{4-[3'-(4-fluorophenyl)-5'-methyl-4'-oxo-1',4',5',7'-tetrahydro-spiro[cyclopropane-1,6'-pyrrolo[3,2-c]pyridin]-2'-yl]pyridin-2-yl}butanamide;
N-[4-(6,6-dimethyl-4-oxo-3-phenyl-4,5,6,7-tetrahydro-1H-pyrrolo[3,2-c]pyridin-2-yl)pyridin-2-yl]-2-(4-fluorophenyl)acetamide;
2-(4-fluorophenyl)-N-{4-[3-(4-fluorophenyl)-6,6-dimethyl-4-oxo-4,5,6,7-tetrahydro-1H-pyrrolo[3,2-c]pyridin-2-yl]pyridin-2-yl}acetamide;
2-(4-fluorophenyl)-N-{4-[3-(2-fluorophenyl)-6,6-dimethyl-4-oxo-4,5,6,7-tetrahydro-1H-pyrrolo[3,2-c]pyridin-2-yl]pyridin-2-yl}acetamide;
2-(4-fluorophenyl)-N-{4-[3-(3-fluorophenyl)-6,6-dimethyl-4-oxo-4,5,6,7-tetrahydro-1H-pyrrolo[3,2-c]pyridin-2-yl]pyridin-2-yl}acetamide;
N-{4-[6,6-dimethyl-3-(3-methylphenyl)-4-oxo-4,5,6,7-tetrahydro-1H-pyrrolo[3,2-c]pyridin-2-yl]pyridin-2-yl}-2-(4-fluorophenyl)acetamide;
2-(4-fluorophenyl)-N-{4-[3-(2-fluorophenyl)-6,6-dimethyl-4-oxo-4,5,6,7-tetrahydro-1H-pyrrolo[3,2-c]pyridin-2-yl]pyridin-2-yl}propanamide;
N-{4-[6,6-dimethyl-3-(3-methylphenyl)-4-oxo-4,5,6,7-tetrahydro-1H-pyrrolo[3,2-c]pyridin-2-yl]pyridin-2-yl}-2-(4-fluorophenyl)propanamide;
N-{4-[3-(4-chlorophenyl)-6,6-dimethyl-4-oxo-4,5,6,7-tetrahydro-1H-pyrrolo[3,2-c]pyridin-2-yl]pyridin-2-yl}-2-(4-fluorophenyl)propanamide;
N-{4-[3-(4-chlorophenyl)-6,6-dimethyl-4-oxo-4,5,6,7-tetrahydro-1H-pyrrolo[3,2-c]pyridin-2-yl]pyridin-2-yl}-2-(4-fluorophenyl)acetamide;
N-{4-[6,6-dimethyl-3-(4-methylphenyl)-4-oxo-4,5,6,7-tetrahydro-1H-pyrrolo[3,2-c]pyridin-2-yl]pyridin-2-yl}-2-(4-fluorophenyl)propanamide;
N-{4-[6,6-dimethyl-3-(4-methylphenyl)-4-oxo-4,5,6,7-tetrahydro-1H-pyrrolo[3,2-c]pyridin-2-yl]pyridin-2-yl}-2-(4-fluorophenyl)acetamide;
N-{4-[6,6-dimethyl-4-oxo-3-(pyridin-3-yl)-4,5,6,7-tetrahydro-1H-pyrrolo[3,2-c]pyridin-2-yl]pyridin-2-yl}-2-(4-fluorophenyl)acetamide;
2-(4-fluorophenyl)-N-{4-[3-(4-fluorophenyl)-6,6-dimethyl-4-oxo-4,5,6,7-tetrahydro-1H-pyrrolo[3,2-c]pyridin-2-yl]pyridin-2-yl}propanamide;
(2R)-2-(4-fluorophenyl)-N-{4-[3-(4-fluorophenyl)-6,6-dimethyl-4-oxo-4,5,6,7-tetrahydro-1H-pyrrolo[3,2-c]pyridin-2-yl]pyridin-2-yl}propanamide;
(2S)-2-(4-fluorophenyl)-N-{4-[3-(4-fluorophenyl)-6,6-dimethyl-4-oxo-4,5,6,7-tetrahydro-1H-pyrrolo[3,2-c]pyridin-2-yl]pyridin-2-yl}propanamide;
N-{4-[6,6-dimethyl-4-oxo-3-(pyridin-3-yl)-4,5,6,7-tetrahydro-1H-pyrrolo[3,2-c]pyridin-2-yl]pyridin-2-yl}-2-(4-fluorophenyl)propanamide;
(2R)-N-{4-[6,6-dimethyl-4-oxo-3-(pyridin-3-yl)-4,5,6,7-tetrahydro-1H-pyrrolo[3,2-c]pyridin-2-yl]pyridin-2-yl}-2-(4-fluorophenyl)propanamide;
(2S)-N-{4-[6,6-dimethyl-4-oxo-3-(pyridin-3-yl)-4,5,6,7-tetrahydro-1H-pyrrolo[3,2-c]pyridin-2-yl]pyridin-2-yl}-2-(4-fluorophenyl)propanamide;
2-(4-fluorophenyl)-N- {4-[3-(4-fluorophenyl)-5-methyl-4-oxo-4,5-dihydro-1H-pyrrolo[3,2-c]pyridin-2-yl]pyridin-2-yl}propanamide;
(2R)-2-(4-fluorophenyl)-N- {4-[3-(4-fluorophenyl)-5-methyl-4-oxo-4,5-dihydro-1H-pyrrolo[3,2-c]pyridin-2-yl]pyridin-2-yl}propanamide;
(2S)-2-(4-fluorophenyl)-N- {4-[3-(4-fluorophenyl)-5-methyl-4-oxo-4,5-dihydro-1H-pyrrolo[3,2-c]pyridin-2-yl]pyridin-2-yl}propanamide;
2-(4-fluorophenyl)-N-[4-(5-methyl-4-oxo-3-phenyl-4,5-dihydro-1H-pyrrolo[3,2-c]pyridin-2-yl)pyridin-2-yl]propanamide;
(2R)-2-(4-fluorophenyl)-N- [4-(5 -methyl-4-oxo-3 -phenyl-4,5-dihydro-1H-pyrrolo [3,2-c]pyridin-2-yl)pyridin-2-yl]propanamide;
(2S)-2-(4-fluorophenyl)-N-[4-(5-methyl-4-oxo-3-phenyl-4,5-dihydro-1H-pyrrolo[3,2-c]pyridin-2-yl)pyridin-2-yl]propanamide;
N-{4-[3-(3-chlorophenyl)-5-methyl-4-oxo-4,5-dihydro-1H-pyrrolo[3,2-c]pyridin-2-yl]pyridin-2-yl} -2-(4-fluorophenyl)propanamide;
4,4-difluoro-2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}butanamide;
(2R)-4,4-difluoro-2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}butanamide;
(2S)-4,4-difluoro-2-(4-fluorophenyl)-N-{4-[3-(4-fluorophenyl)-5-methyl-4-oxo-4,5-dihydro-1H-pyrrolo[3,2-c]pyridin-2-yl]pyridin-2-yl}butanamide;
2-(4-fluorophenyl)-N-{4-[3-(2-fluoropyridin-3-yl)-5-methyl-4-oxo-4,5-dihydro-1H-pyrrolo[3,2-c]pyridin-2-yl]pyridin-2-yl}propanamide;
2-(4-fluorophenyl)-N-{4-[5-methyl-3-(2-methylphenyl)-4-oxo-4,5-dihydro-1H-pyrrolo[3,2-c]pyridin-2-yl]pyridin-2-yl}propanamide;
N-{4-[3-(2-chlorophenyl)-5-methyl-4-oxo-4,5-dihydro-1H-pyrrolo[3,2-c]pyridin-2-yl]pyridin-2-yl}-2-(4-fluorophenyl)propanamide;
2-(4-fluorophenyl)-N-{4-[3-(3-hydroxyphenyl)-5-methyl-4-oxo-4,5-dihydro-1H-pyrrolo[3,2-c]pyridin-2-yl]pyridin-2-yl}propanamide;
N-{4-[3-(3,4-difluorophenyl)-5-methyl-4-oxo-4,5-dihydro-1H-pyrrolo[3,2-c]pyridin-2-yl]pyridin-2-yl}-2-(4-fluorophenyl)propanamide;
2-(4-fluorophenyl)-N-{4-[3-(3-furyl)-5-methyl-4-oxo-4,5-dihydro-1H-pyrrolo[3,2-c]pyridin-2-yl]pyridin-2-yl}propanamide;
2-(4-fluorophenyl)-N-{4-[3-(2-furyl)-5-methyl-4-oxo-4,5-dihydro-1H-pyrrolo[3,2-c]pyridin-2-yl]pyridin-2-yl}propanamide;
2-(4-fluorophenyl)-N-{4-[5-methyl-4-oxo-3-(3,4,5-trifluorophenyl)-4,5-dihydro-1H-pyrrolo[3,2-c]pyridin-2-yl]pyridin-2-yl}propanamide;
4,4-difluoro-2-(4-fluorophenyl)-N-{4-[5-methyl-4-oxo-3-(pyridin-3-yl)-4,5-dihydro-1H-pyrrolo[3,2-c]pyridin-2-yl]pyridin-2-yl}butanamide;
2-(4-fluorophenyl)-N-{4-[3-(4-hydroxyphenyl)-5-methyl-4-oxo-4,5-dihydro-1H-pyrrolo[3,2-c]pyridin-2-yl]pyridin-2-yl}propanamide;
N-{4-[3-(2,5-difluorophenyl)-5-methyl-4-oxo-4,5-dihydro-1H-pyrrolo[3,2-c]pyridin-2-yl]pyridin-2-yl}-2-(4-fluorophenyl)propanamide;
N-{4-[3-(3,5-difluorophenyl)-5-methyl-4-oxo-4,5-dihydro-1H-pyrrolo[3,2-c]pyridin-2-yl]pyridin-2-yl}-2-(4-fluorophenyl)propanamide;
N-{4-[3-(5-chloro-2-thienyl)-5-methyl-4-oxo-4,5-dihydro-1H-pyrrolo[3,2-c]pyridin-2-yl]pyridin-2-yl} -2-(4-fluorophenyl)propanamide;
N-{4-[3-(2-cyanophenyl)-5-methyl-4-oxo-4,5-dihydro-1H-pyrrolo[3,2-c]pyridin-2-yl]pyridin-2-yl} -2-(4-fluorophenyl)propanamide;
2-(4-fluorophenyl)-N-{4-[3-(2-hydroxyphenyl)-5-methyl-4-oxo-4,5-dihydro-1H-pyrrolo[3,2-c]pyridin-2-yl]pyridin-2-yl}propanamide;
2-(4-fluorophenyl)-N-{4-[5-methyl-3-(5-methyl-2-thienyl)-4-oxo-4,5-dihydro-1H-pyrrolo[3,2-c]pyridin-2-yl]pyridin-2-yl}propanamide;
N-{4-[3-(2-aminopyridin-4-yl)-5-methyl-4-oxo-4,5-dihydro-1H-pyrrolo[3,2-c]pyridin-2-yl]pyridin-2-yl} -2-(4-fluorophenyl)propanamide;
N-{4-[3-(2-cyano-5-fluorophenyl)-5-methyl-4-oxo-4,5-dihydro-1H-pyrrolo[3,2-c]pyridin-2-yl]pyridin-2-yl}-2-(4-fluorophenyl)propanamide;
N-{4-[3-(2-cyano-5-fluorophenyl)-5-methyl-4-oxo-4,5,6,7-tetrahydro-1H-pyrrolo[3,2-c]pyridin-2-yl]pyridin-2-yl}-4,4-difluoro-2-(4-fluorophenyl)butanamide;
4,4-difluoro-2-(4-fluorophenyl)-N-{4-[5-methyl-3-(2-methyl-1,3-thiazol-5-yl)-4-oxo-4,5,6,7-tetrahydro-1H-pyrrolo[3,2-c]pyridin-2-yl]pyridin-2-yl} butanamide;
2-(4-fluorophenyl)-N-{4-[5-methyl-4-oxo-3-(2-thienyl)-4,5-dihydro-1H-pyrrolo[3,2-c]pyridin-2-yl] pyridin-2-yl} propanamide;
2-(4-fluorophenyl)-N-{4-[5-methyl-4-oxo-3-(3-thienyl)-4,5-dihydro-1H-pyrrolo[3,2-c]pyridin-2-yl] pyridin-2-yl} propanamide;
N-[4-(3,5-dimethyl-4-oxo-4,5-dihydro-1H-pyrrolo[3,2-c]pyridin-2-yl)pyridin-2-yl]-2-(4-fluorophenyl)propanamide;
N-{4-[3-(5-chloro-2-thienyl)-5-methyl-4-oxo-4,5-dihydro-1H-pyrrolo[3,2-c]pyridin-2-yl]pyridin-2-yl}-4,4-difluoro-2-(4-fluorophenyl)butanamide;
N- {4-[5-cyclopropyl-3-(4-fluorophenyl)-4-oxo-4, 5-dihydro-1H-pyrrolo [3,2-c]pyridin-2-yl]pyridin-2-yl}-4,4-difluoro-2-(4-fluorophenyl)butanamide;
(2R)-N-{4-[5-cyclopropyl-3-(4-fluorophenyl)-4-oxo-4,5-dihydro-1H-pyrrolo[3,2-c]pyridin-2-yl]pyridin-2-yl}-4,4-difluoro-2-(4-fluorophenyl)butanamide;
(2S)-N-{4-[5-cyclopropyl-3-(4-fluorophenyl)-4-oxo-4,5-dihydro-1H-pyrrolo[3,2-c]pyridin-2-yl]pyridin-2-yl}-4,4-difluoro-2-(4-fluorophenyl)butanamide;
N-[4-(5-cyclopropyl-4-oxo-3-phenyl-4,5-dihydro-1H-pyrrolo[3,2-c]pyridin-2-yl)pyridin-2-yl]-4,4-difluoro-2-(4-fluorophenyl)butanamide;
4,4-difluoro-2-(4-fluorophenyl)-N-{4-[5-methyl-4-oxo-3-(1,3-thiazol-4-yl)-4,5,6,7-tetrahydro-1H-pyrrolo[3,2-c]pyridin-2-yl]pyridin-2-yl}butanamide;
4,4-difluoro-2-(4-fluorophenyl)-N-{4-[3-(4-fluorophenyl)-5-methyl-4-oxo-4,5,6,7-tetrahydro-1H-pyrrolo[3,2-c]pyridin-2-yl]pyridin-2-yl} butanamide;
4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]-N-(2-methoxyethyl)benzamide;
4-[2-({4-[3-(4-fluorophenyl)-5-methyl-4-oxo-4,5,6,7-tetrahydro-1H-pyrrolo[3,2-c]pyridin-2-yl]pyridin-2-yl}amino)-2-oxoethyl]-N-(2-methoxyethyl)-N-methylbenzamide;
4-[2-({4-[3-(4-fluorophenyl)-5-methyl-4-oxo-4,5-dihydro-1H-pyrrolo[3,2-c]pyridin-2-yl]pyridin-2-yl}amino)-2-oxoethyl]-N-(2-methoxyethyl)benzamide;
4-[2-({4-[3-(4-fluorophenyl)-5-methyl-4-oxo-4,5-dihydro-1H-pyrrolo[3,2-c]pyridin-2-yl]pyridin-2-yl}amino)-2-oxoethyl]-N-(2-methoxyethyl)-N-methylbenzamide;
N-{4-[3-(4-fluorophenyl)-5-methyl-4-oxo-4,5,6,7-tetrahydro-1H-pyrrolo[3,2-c]pyridin-2-yl]pyridin-2-yl}-2-[4-(piperazine-1-carbonyl)phenyl]acetamide;
2-(4-fluorophenyl)-N-{4-[3-(6-fluoropyridin-3-yl)-5-methyl-4-oxo-4,5-dihydro-1H-pyrrolo[3,2-c]pyridin-2-yl]pyridin-2-yl}propanamide;
2-(4-fluorophenyl)-N-{4-[5-methyl-4-oxo-3-(pyridin-2-yl)-4,5-dihydro-1H-pyrrolo[3,2-c]pyridin-2-yl]pyridin-2-yl}acetamide;
(2RS)-N-{4-[7-(2,2-difluoroethyl)-5-methyl-4-oxo-3-phenyl-4,5-dihydro-1H-pyrrolo[3,2-c]pyridin-2-yl]pyridin-2-yl}-4,4-difluoro-2-(4-fluorophenyl)butanamide;
(2R)-N-{4-[7-(2,2-difluoroethyl)-5-methyl-4-oxo-3-phenyl-4,5-dihydro-1H-pyrrolo[3,2-c]pyridin-2-yl]pyridin-2-yl}-4,4-difluoro-2-(4-fluorophenyl)butanamide;
(2S)-N-{4-[7-(2,2-difluoroethyl)-5-methyl-4-oxo-3-phenyl-4,5-dihydro-1H-pyrrolo[3,2-c]pyridin-2-yl]pyridin-2-yl}-4,4-difluoro-2-(4-fluorophenyl)butanamide;
(2RS)-4,4-difluoro-2-(4-fluorophenyl)-N-[4-(5,7,7-trimethyl-4-oxo-3-phenyl-4,5,6,7-tetrahydro-1H-pyrrolo[3,2-c]pyridin-2-yl)pyridin-2-yl]butanamide;
(2R)-4,4-difluoro-2-(4-fluorophenyl)-N-[4-(5,7,7-trimethyl-4-oxo-3-phenyl-4,5,6,7-tetrahydro-1H-pyrrolo[3,2-c]pyridin-2-yl)pyridin-2-yl]butanamide;
(2S)-4,4-difluoro-2-(4-fluorophenyl)-N-[4-(5,7,7-trimethyl-4-oxo-3-phenyl-4,5,6,7-tetrahydro-1H-pyrrolo[3,2-c]pyridin-2-yl)pyridin-2-yl]butanamide;
(2RS)-N-[4-(5-methyl-4-oxo-3-phenyl-4,5,6,7-tetrahydro-1H-pyrrolo[3,2-c]pyridin-2-yl)pyridin-2-yl]-2-(pyridin-4-yl)propanamide;
(2R)-N-[4-(5-methyl-4-oxo-3-phenyl-4,5,6,7-tetrahydro-1H-pyrrolo[3,2-c]pyridin-2-yl)pyridin-2-yl]-2-(pyridin-4-yl)propenamide;
(2S)-N-[4-(5-methyl-4-oxo-3-phenyl-4,5,6,7-tetrahydro-1H-pyrrolo[3,2-c]pyridin-2-yl)pyridin-2-yl]-2-(pyridin-4-yl)propanamide;
(2RS)-N-(4-(5-cyclopropyl-4-oxo-3-phenyl-4,5,6,7-tetrahydro-1H-pyrrolo[3,2-c]pyridin-2-yl)pyridin-2-yl)-4,4-difluoro-2-(4-fluorophenyl)butanamide;
(2R)-N-[4-(5-cyclopropyl-4-oxo-3-phenyl-4,5-dihydro-1H-pyrrolo[3,2-c]pyridin-2-yl)pyridin-2-yl]-4,4-difluoro-2-(4-fluorophenyl)butanamide;
(2S)-N-[4-(5-cyclopropyl-4-oxo-3-phenyl-4,5-dihydro-1H-pyrrolo[3,2-c]pyridin-2-yl)pyridin-2-yl]-4,4-difluoro-2-(4-fluorophenyl)butanamide;
N-[4-(5-cyclopropyl-4-oxo-3-phenyl-4,5-dihydro-1H-pyrrolo[3,2-c]pyridin-2-yl)pyridin-2-yl]-2-(4-fluorophenyl)acetamide;
(2RS)-N-{4-[5-cyclopropyl-4-oxo-3-(pyridin-2-yl)-4,5-dihydro-1H-pyrrolo[3,2-c]pyridin-2-yl]pyridin-2-yl}-4,4-difluoro-2-(4-fluorophenyl)butanamide;
(2RS)-N-{4-[7-chloro-5-cyclopropyl-3-(4-fluorophenyl)-4-oxo-4,5-dihydro-1H-pyrrolo[3,2-c]pyridin-2-yl]pyridin-2-yl}-4,4-difluoro-2-(4-fluorophenyl)butanamide;
(2RS)-4,4-difluoro-2-(4-fluorophenyl)-N-{4-[5-methyl-4-oxo-3-(pyridin-2-yl)-7-(2,2,2-trifluoroethyl)-4,5-dihydro-1H-pyrrolo[3,2-c]pyridin-2-yl]pyridin-2-yl}butanamide;
or an N-oxide, a salt, a tautomer or a stereoisomer of said compound, or a salt of said N-oxide, tautomer or stereoisomer.

**6.** A method of preparing a compound of general formula (I) according to any one of claims 1 to 5, said method comprising reacting an intermediate compound of general formula (1-3):

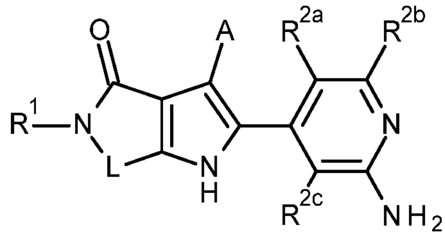

1-3,

in which $R^1$, $R^{2a}$, $R^{2b}$, $R^{2c}$, A, and L are as defined for the compound of general formula (I) according to any one of claims 1 to 4,
with an acylating reagent selected from:

a) a carboxylic acid of formula

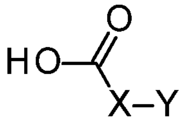

b) an acyl halide of formula

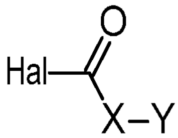

,

wherein Hal represents F, Cl or Br, or
c) an anhydride of formula

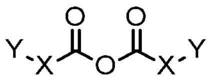

or

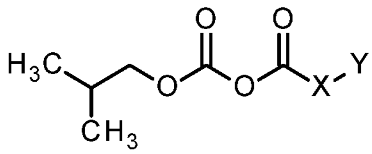

in which X and Y are as defined for the compound of general formula (I) according to any one of claims 1 to 4,

to give a compound of general formula (I):

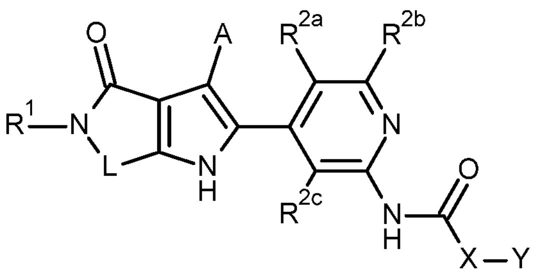

(I),

in which $R^1$, $R^{2a}$, $R^{2b}$, $R^{2c}$, A, L, X, and Y are as defined for the compound of general formula (I) according to any one of claims 1 to 4.

7. The compound of general formula (I) according to any of claims 1 to 5 for use in the treatment or prophylaxis of a hyperproliferative disease and/or a disorder responsive to induction of cell death.

8. The compound of general formula (I) for use according to claim 7, wherein the hyperproliferative disease and/or disorder responsive to induction of cell death is a haematological tumour, solid tumour and/or metastases thereof.

9. The compound of formula (I) for use according to claim 8, wherein the haematological tumour is a lymphoma and/or metastases thereof, preferably wherein the lymphoma is diffuse large B-cell lymphoma and/or metastases thereof; the solid tumour is a cervical tumour, a lung tumour, a colon tumour and/or metastases thereof, preferably wherein the lung tumour is a lung carcinoma and/or metastases thereof and the colon tumour is a colorectal carcinoma and/or metastases thereof.

10. A pharmaceutical composition comprising at least one compound of general formula (I) according to any of claims 1 to 5, together with at least one pharmaceutically acceptable excipient.

11. A combination comprising one or more first active ingredients selected from a compound of general formula (I) according to any of claims 1 to 5, and one or more second active ingredients selected from chemotherapeutic anti-cancer agents and target-specific anti-cancer agents.

12. An intermediate compound of formula 1-3, Ia, or 1-9, or a salt thereof:

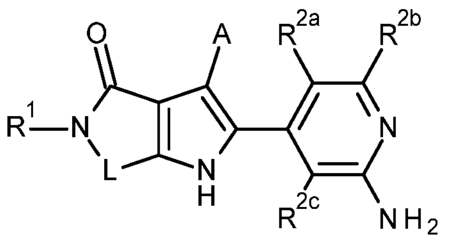
1-3,

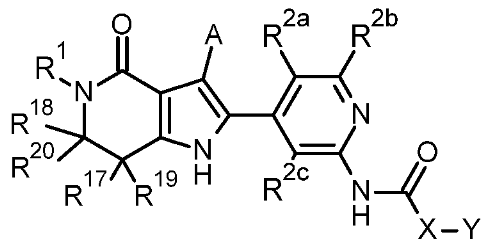
**(Ia)**

,

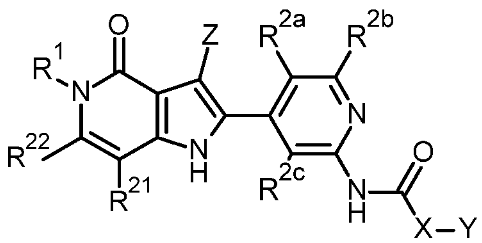

**1-9**

wherein $R^1$, $R^{2a}$, $R^{2b}$, $R^{2c}$, $R^{17}$, $R^{18}$, $R^{19}$, $R^{20}$, $R^{21}$, $R^{22}$, A, X, Y, and L are as defined for the compound of general formula (I) according to any one of claims 1 to 4, and Z represents chloro, bromo, or iodo.

13. Use of a compound of formula 1-3, or a salt thereof:

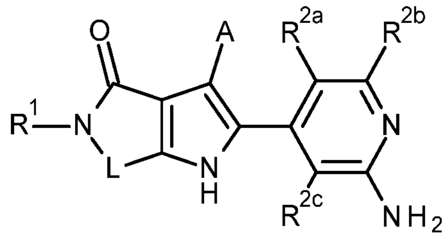

1-3,

in which $R^1$, $R^{2a}$, $R^{2b}$, $R^{2c}$, A, and L are as defined for the compound of general formula (I) according to any one of claims 1 to 4, or
of a compound of general formula (Ia) or a salt thereof:

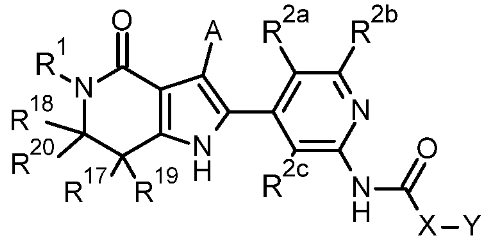

**(Ia)**

,

in which $R^1$, $R^{2a}$, $R^{2b}$, $R^{2c}$, $R^{17}$, $R^{18}$, $R^{19}$, $R^{20}$, A, X, and Y are as defined for the compound of general formula (I) according to any one of claims 1 to 4, or
of a compound of general formula 1-9 or a salt thereof:

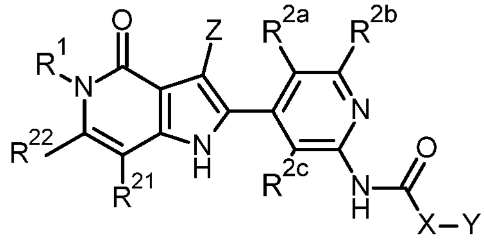

**1-9**

,

in which $R^1$, $R^{2a}$, $R^{2b}$, $R^{2c}$, $R^{21}$, $R^{22}$, X, and Y are as defined for the compound of general formula (I) according to any one of claims 1 to 4, and Z represents chloro, bromo or iodo, or
of a compound of general formula (1-6) or a salt thereof:

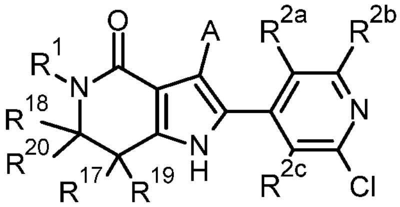

**1-6**

,

in which $R^1$, $R^{2a}$, $R^{2b}$, $R^{2c}$, $R^{17}$, $R^{18}$, $R^{19}$, $R^{20}$ and A are as defined for the compound of general formula (I) according to any one of claims 1 to 4, for the preparation of a compound of formula (I) as defined in any one of claims 1 to 5.

**14.** A method of preparing a compound of general formula (Ib) according to any one of claims 1 to 5, said method comprising reacting an intermediate compound of general formula (Ia):

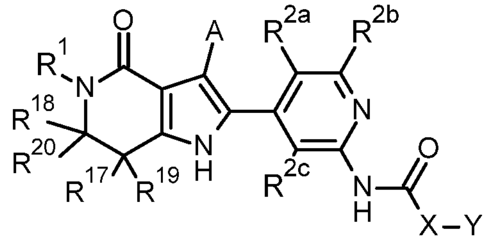

**(Ia)**

,

in which $R^1$, $R^{2a}$, $R^{2b}$, $R^{2c}$, $R^{17}$, $R^{18}$, $R^{19}$, $R^{20}$, A, X, and Y are as defined for the compound of general formula (I) according to any one of claims 1 to 4, with an oxidizing reagent to give a compound of general formula (Ib):

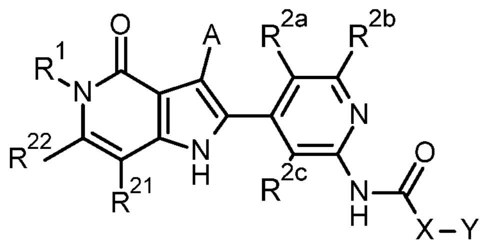

**(Ib)**

,

in which $R^1$, $R^{2a}$, $R^{2b}$, $R^{2c}$, $R^{21}$, $R^{22}$, A, X, and Y are as defined for the compound of general formula (I) according to any one of claims 1 to 4; or said method comprising reacting an intermediate compound of general formula (1-9):

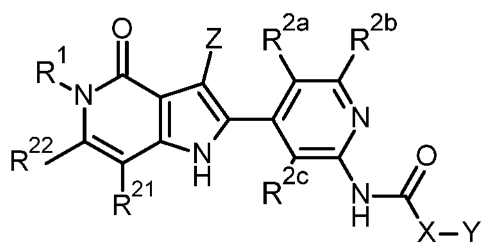

**1-9**

,

in which $R^1$, $R^{2a}$, $R^{2b}$, $R^{2c}$, $R^{21}$, $R^{22}$, X, and Y are as defined for the compound of general formula (I) according to any one of claims 1 to 4, and Z represents chloro, bromo or iodo with a reagent F:

$Z^2$-A **reagent F**

in which A, is as defined for the compound of general formula (I) according to any one of claims 1 to 4 and $Z^2$ represents a stannane such as A-$Sn(nBu)_3$, boronic acid A-$B(OH)_2$ or boronic ester thereof, using a metal-catalyzed reaction, such as, for example, a Suzuki reaction to give a compound of general formula

(Ib):

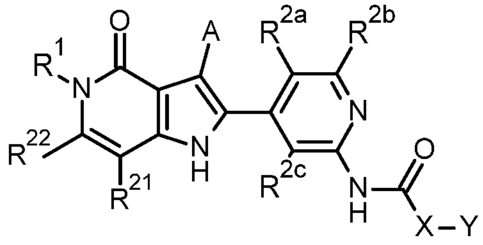

**(Ib)**

,

in which $R^1$, $R^{2a}$, $R^{2b}$, $R^{2c}$, $R^{21}$, $R^{22}$, A, X, and Y are as defined for the compound of general formula (I) according to any one of claims 1 to 4.

**15.** A method of preparing a compound of general formula (Ia) according to any one of claims 1 to 5, said method comprising reacting an intermediate compound of general formula (1-8):

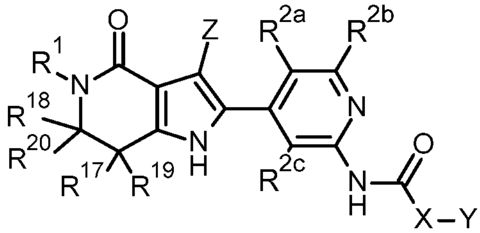

**1-8**

,

in which $R^1$, $R^{2a}$, $R^{2b}$, $R^{2c}$, $R^{17}$, $R^{18}$, $R^{19}$, $R^{20}$, X, and Y are as defined for the compound of general formula (I) according to any one of claims 1 to 4 and Z represents chloro, bromo or iodo, with a reagent F:

$Z^2$-A **reagent F**

in which A, is as defined for the compound of general formula (I) according to any one of claims 1 to 4 and $Z^2$ represents a stannane such as A-Sn(nBu)$_3$, boronic acid A-B(OH)$_2$ or boronic ester thereof using a metal-catalyzed reaction, such as, for example, a Suzuki reaction to give a compound of general formula (Ia):

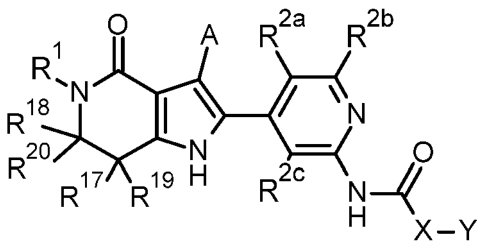

**(Ia)**

in which $R^1$, $R^{2a}$, $R^{2b}$, $R^{2c}$, $R^{17}$, $R^{18}$, $R^{19}$, $R^{20}$, A, X, and Y are as defined for the compound of general formula (I) according to any one of claims 1 to 4, or said method comprising reacting an intermediate compound of general formula (1-6):

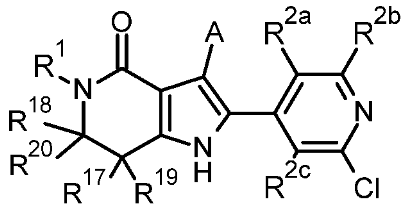

**1-6**

,

in which $R^1$, $R^{2a}$, $R^{2b}$, $R^{2c}$, $R^{17}$, $R^{18}$, $R^{19}$, $R^{20}$ and A are as defined for the compound of general formula (I) according to any one of claims 1 to 4 with a reagent G:

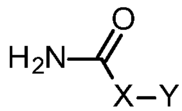

**Reagent G**

wherein X and Y are as defined for the compound of general formula (I) according to any one of claims 1 to 4 using a metal-catalyzed reaction, such as, for example, a palladium catalyzed reaction in the presence of a ligand, such as, for example, Xanthphos or X-Phos, to give a compound of general formula (Ia):

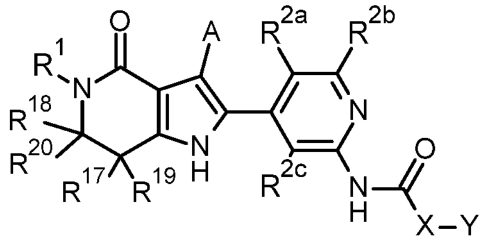

**(Ia)**

,

in which $R^1$, $R^{2a}$, $R^{2b}$, $R^{2c}$, $R^{17}$, $R^{18}$, $R^{19}$, $R^{20}$, A, X, and Y are as defined for the compound of general formula (I) according to any one of claims 1 to 4.

**Patentansprüche**

**1.** Verbindung der Formel (I)

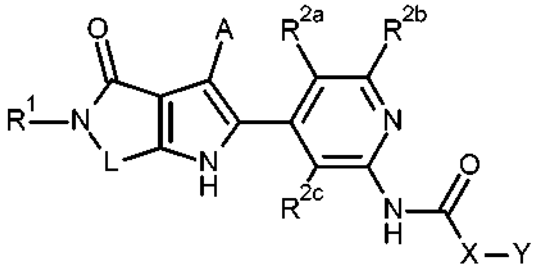

(I)

worin:

$R^1$ Wasserstoff oder eine Gruppe ausgewählt aus $C_1$-$C_4$-Alkyl, $C_3$-$C_6$-Cycloalkyl, $C_3$-$C_6$-Cycloalkyl-$C_1$-$C_4$-alkyl-, $C_1$-$C_3$-Alkoxy-$C_1$-$C_3$-alkyl- und Ci-Cs-Hydroxyalkyl darstellt, wobei die Gruppen jeweils unabhängig optional ein- oder mehrmals mit Halogen substituiert sind;
$R^{2a}$ Wasserstoff, Hydroxy, Halogen, Cyano, -$NH_2$, -NHMe, -$NMe_2$, $C_1$-$C_2$-Alkoxy, $C_1$-$C_2$-Haloalkyl oder $C_1$-$C_2$-Haloalkoxy darstellt;
$R^{2b}$ Wasserstoff, Hydroxy, Halogen, Cyano, -$NH_2$, -$NHR^9$ oder -$NMe_2$ darstellt;
$R^{2c}$ Wasserstoff, Halogen, Cyano, -$NMe_2$, $C_1$-$C_2$-Alkoxy oder $C_1$-$C_2$-Haloalkoxy darstellt;
L

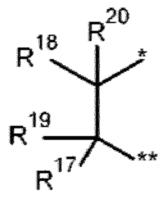

oder

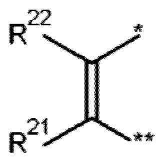

darstellt, wobei * den Punkt der direkten Bindung an das benachbarte N-Atom darstellt und ** den Punkt der direkten Bindung an das benachbarte C-Atom darstellt;
$R^{17}$ Wasserstoff, $C_1$-$C_3$-Alkyl, $C_1$-$C_3$-Alkoxy, $C_1$-$C_3$-Haloalkyl oder $C_3$-$C_6$-Cycloalkyl-$C_1$-$C_4$-alkyl- darstellt;
$R^{18}$ Wasserstoff, $C_1$-$C_3$-Alkyl, $C_1$-$C_3$-Haloalkyl oder $C_3$-$C_6$-Cycloalkyl-$C_1$-$C_4$-alkyl-darstellt oder
$R^{17}$ und $R^{18}$ zusammen mit den Kohlenstoffatomen, an die sie gebunden sind, einen $C_3$-$C_6$-Cycloalkylring bilden;
$R^{19}$ Wasserstoff, $C_1$-$C_3$-Alkyl, $C_1$-$C_3$-Haloalkyl oder $C_3$-$C_6$-Cycloalkyl-$C_1$-$C_4$-alkyl-darstellt;
$R^{20}$ Wasserstoff, $C_1$-$C_3$-Alkyl, $C_1$-$C_3$-Alkoxy, $C_1$-$C_3$-Haloalkyl oder $C_3$-$C_6$-Cycloalkyl-$C_1$-$C_4$-alkyl- darstellt oder
$R^{18}$ und $R^{20}$ zusammen mit dem Kohlenstoffatom, an das sie gebunden sind, einen $C_3$-$C_6$-Cycloalkylring bilden;
$R^{21}$ Wasserstoff, Halogen, $C_1$-$C_3$-Alkyl, $C_1$-$C_3$-Haloalkyl oder $C_3$-$C_6$-Cycloalkyl-$C_1$-$C_4$-alkyl- darstellt;
$R^{22}$ Wasserstoff, $C_1$-$C_3$-Alkyl, $C_1$-$C_3$-Haloalkyl oder $C_3$-$C_6$-Cycloalkyl-$C_1$-$C_4$-alkyl-darstellt;
X Methylen oder Ethylen darstellt,
wobei die Methylen- und Ethylengruppen jeweils unabhängig optional ein- oder mehrmals mit $R^3$ substituiert sind;
Y Phenyl oder eine Heteroarylgruppe darstellt,
wobei die Phenyl- und Heteroarylgruppen jeweils unabhängig optional ein- oder mehrmals mit $R^4$ substituiert sind;
$R^3$ Hydroxy, Halogen, Cyano, $C_1$-$C_3$-Alkyl, $C_1$-$C_3$-Alkoxy, $C_1$-$C_3$-Haloalkyl, $C_1$-$C_2$-Haloalkoxy, $C_3$-$C_4$-Cycloalkyl, -$NHR^6$, $R^5O$-$C_1$-$C_3$-Alkyl, $R^6R^7N$-$C_1$-$C_5$-Alkyl oder $R^6R^7N$-X'-C($R^{10}$)($R^{11}$)-$C_1$-$C_2$-Alkyl- darstellt;
X' Methylen oder Ethylen darstellt;
$R^4$ Hydroxy, Halogen, Cyano, $C_1$-$C_4$-Alkyl, $C_1$-$C_4$-Alkoxy, $C_1$-$C_4$-Haloalkyl, $C_1$-$C_4$-Haloalkoxy, -$NMe_2$, -$CO_2R^{14}$, -$CONR^{15}R^{16}$ oder -$SO_2$Me darstellt;
$R^5$ Wasserstoff, $C_1$-$C_2$-Alkyl, $C_1$-$C_2$-Haloalkyl oder $C_3$-$C_4$-Cycloalkyl darstellt;
$R^6$ Wasserstoff, $C_1$-$C_2$-Alkyl, $C_1$-$C_2$-Haloalkyl oder $C_3$-$C_4$-Cycloalkyl darstellt;
$R^7$ Wasserstoff, $C_1$-$C_2$-Alkyl, $C_1$-$C_2$-Haloalkyl, $C_3$-$C_4$-Cycloalkyl oder -$CO_2$-$C_1$-$C_4$-Alkyl darstellt oder
$R^6$ und $R^7$ zusammen mit dem Stickstoffatom, an das sie gebunden sind, einen 4- bis 7-gliedrigen heterocyclischen Ring darstellen, wobei der heterocyclische Ring optional ein oder mehrere weitere Heteroatome ausgewählt aus N, O, S oder Gruppen ausgewählt aus C(=O), S(=O) und $SO_2$ enthält und unabhängig optional ein- oder mehrmals mit Halogen, $C_1$-$C_3$-Alkyl, $C_3$-Cycloalkyl-$C_1$-$C_2$-alkyl-, -$NH_2$, -$NHR^9$, -$NR^9R^{9a}$ oder Hydroxy substituiert ist;
A eine Gruppe darstellt, ausgewählt aus:

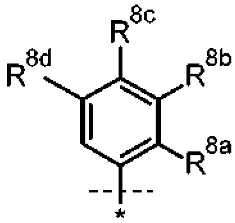 , 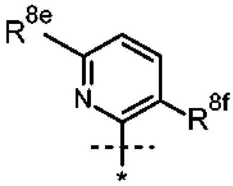 , 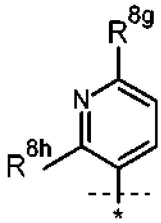 , 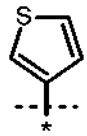 ,

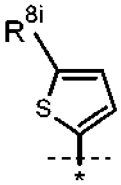 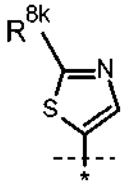 , 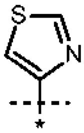 , 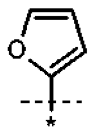 , 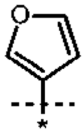 und 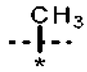 ,

wobei * den Bindungspunkt der Gruppe an den Rest der Formel (I) angibt;
$R^{8a}$ Wasserstoff, Hydroxy, Halogen, Cyano, $C_1$-$C_2$-Alkyl oder $C_1$-$C_2$-Haloalkyl darstellt;
$R^{8b}$ Wasserstoff, Hydroxy, Halogen, Cyano, $C_1$-$C_2$-Alkyl oder $C_1$-$C_2$-Haloalkyl darstellt;
$R^{8c}$ Wasserstoff, Hydroxy, Halogen, $C_1$-$C_2$-Alkyl oder $C_1$-$C_2$-Haloalkyl darstellt;
$R^{8d}$ Wasserstoff, Hydroxy, Halogen, Cyano, $C_1$-$C_2$-Alkyl oder $C_1$-$C_2$-Haloalkyl darstellt;
$R^{8e}$ Wasserstoff, Hydroxy, Halogen, Cyano, $C_1$-$C_2$-Alkyl oder $C_1$-$C_2$-Haloalkyl darstellt;
$R^{8f}$ Wasserstoff, Hydroxy, Halogen, Cyano, $C_1$-$C_2$-Alkyl oder $C_1$-$C_2$-Haloalkyl darstellt;
$R^{8g}$ Wasserstoff, Hydroxy, Amino, Halogen, Cyano, $C_1$-$C_2$-Alkyl oder $C_1$-$C_2$-Haloalkyl darstellt;
$R^{8h}$ Wasserstoff, Hydroxy, Halogen, Cyano, $C_1$-$C_2$-Alkyl oder $C_1$-$C_2$-Haloalkyl darstellt;
$R^{8i}$ Wasserstoff, Hydroxy, Halogen, Cyano, $C_1$-$C_2$-Alkyl oder $C_1$-$C_2$-Haloalkyl darstellt;
$R^{8k}$ Wasserstoff, Hydroxy, Halogen, Cyano, $C_1$-$C_2$-Alkyl oder $C_1$-$C_2$-Haloalkyl darstellt;
$R^9$ $C_1$-$C_3$-Alkyl oder Cyclopropyl darstellt;
$R^{9a}$ $C_1$-$C_3$-Alkyl oder Cyclopropyl darstellt;
$R^{10}$ Methyl, Halogen oder Hydroxy darstellt;
$R^{11}$ Methyl, Halogen oder Hydroxy darstellt;
$R^{14}$ Wasserstoff, Methyl oder Ethyl darstellt;
$R^{15}$ Wasserstoff, Methyl oder Ethyl darstellt;
$R^{16}$ Wasserstoff, Methyl, Ethyl, Methoxyethyl oder Dimethylaminoethyl darstellt oder
$R^{15}$ und $R^{16}$ zusammen mit dem Stickstoffatom, an das sie gebunden sind, einen 5- bis 6-gliedrigen heterocyclischen Ring darstellen, wobei der heterocyclische Ring optional ein oder zwei weitere Heteroatome ausgewählt aus N, O, S oder Gruppen ausgewählt aus C(=O), S(=O) und $SO_2$ enthält;
oder N-Oxid, Salz, Tautomer oder Stereoisomer der Verbindung oder Salz des N-Oxids, Tautomers oder Stereoisomers.

**2.** Verbindung der Formel (I) nach Anspruch 1, wobei:

$R^1$ Wasserstoff oder eine Gruppe ausgewählt aus $C_1$-$C_2$-Alkyl und $C_3$-$C_4$-Cycloalkyl darstellt, wobei die Gruppen jeweils unabhängig optional ein- oder mehrmals mit Halogen substituiert sind;
$R^{2a}$ Wasserstoff oder Halogen darstellt;
$R^{2b}$ Wasserstoff, Halogen, $-NH_2$ oder $-NHR^9$ darstellt;
$R^{2c}$ Wasserstoff oder Halogen darstellt;
L

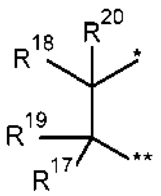

oder

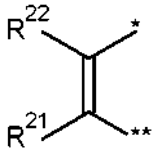

darstellt, wobei * den Punkt der direkten Bindung an das benachbarte N-Atom darstellt und ** den Punkt der direkten Bindung an das benachbarte C-Atom darstellt;
$R^{17}$ Wasserstoff, $C_1$-$C_3$-Alkyl, $C_1$-$C_3$-Haloalkyl oder $C_3$-$C_6$-Cycloalkyl-$C_1$-$C_4$-alkyl-darstellt;
$R^{18}$ Wasserstoff, $C_1$-$C_3$-Alkyl, $C_1$-$C_3$-Haloalkyl oder $C_3$-Cycloalkyl-$C_1$-alkyl- darstellt oder
$R^{17}$ und $R^{18}$ zusammen mit den Kohlenstoffatomen, an die sie gebunden sind, einen $C_3$-$C_5$-Cycloalkylring bilden;
$R^{19}$ Wasserstoff, $C_1$-$C_3$-Alkyl, $C_1$-$C_3$-Haloalkyl oder $C_3$-$C_6$-Cycloalkyl-$C_1$-$C_4$-alkyl-darstellt;
$R^{20}$ Wasserstoff oder $C_1$-$C_3$-Alkyl darstellt oder
$R^{18}$ und $R^{20}$ zusammen mit dem Kohlenstoffatom, an das sie gebunden sind, einen $C_3$-$C_6$-Cycloalkylring bilden;
$R^{21}$ Wasserstoff, Halogen, $C_1$-$C_3$-Alkyl, $C_1$-$C_3$-Haloalkyl oder $C_3$-$C_6$-Cycloalkyl-$C_1$-$C_4$-alkyl- darstellt;
$R^{22}$ Wasserstoff oder $C_1$-$C_3$-Alkyl darstellt;
X Methylen darstellt,
wobei die Methylengruppe optional ein- oder mehrmals mit $R^3$ substituiert ist;
Y Phenyl oder eine Heteroarylgruppe darstellt,
wobei die Phenyl- und Heteroarylgruppen jeweils unabhängig optional ein- oder mehrmals mit $R^4$ substituiert sind;
$R^3$ $C_1$-$C_3$-Alkyl, $C_1$-$C_3$-Haloalkyl, -$NHR^6$, $R^5O$-$C_1$-$C_3$-Alkyl oder $R^6R^7N$-$C_1$-$C_4$-Alkyl darstellt;
$R^4$ Halogen, $C_1$-$C_4$-Alkyl, $C_1$-$C_4$-Haloalkyl, $C_1$-$C_4$-Haloalkoxy, -$CO_2R^{14}$, -$CONR^{15}R^{16}$ oder -$SO_2Me$ darstellt;
$R^5$ Wasserstoff, $C_1$-$C_2$-Alkyl oder $C_1$-$C_2$-Haloalkyl darstellt;
$R^6$ Wasserstoff, $C_1$-$C_2$-Alkyl, $C_1$-$C_2$-Haloalkyl oder $C_3$-$C_4$-Cycloalkyl darstellt;
$R^7$ Wasserstoff, $C_1$-$C_2$-Alkyl, $C_1$-$C_2$-Haloalkyl, $C_3$-$C_4$-Cycloalkyl oder -$CO_2$-$C_1$-$C_4$-Alkyl darstellt oder
$R^6$ und $R^7$ zusammen mit dem Stickstoffatom, an das sie gebunden sind, einen 4- bis 7-gliedrigen heterocyclischen Ring darstellen, wobei der heterocyclische Ring optional ein oder mehrere weitere Heteroatome ausgewählt aus N, O, S enthält und unabhängig optional ein- oder mehrmals mit Halogen, $C_1$-$C_3$-Alkyl, $C_3$-Cycloalkyl-$C_1$-$C_2$-alkyl-, -$NH_2$, -$NHR^9$, -$NR^9R^{9a}$ oder Hydroxy substituiert ist;
A eine Gruppe darstellt, ausgewählt aus:

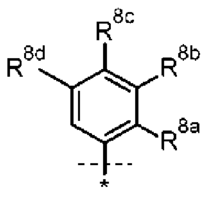
,
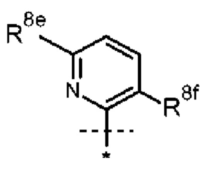
,
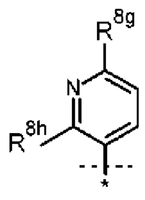
,
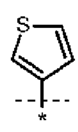
,

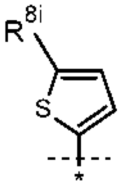
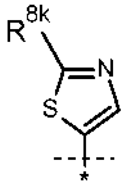
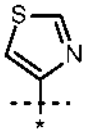
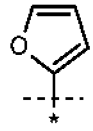
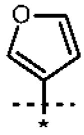
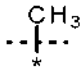

, , , und ,

wobei * den Bindungspunkt der Gruppe an den Rest der Formel (I) angibt;
$R^{8a}$ Wasserstoff, Hydroxy, Halogen, Cyano oder $C_1$-Alkyl darstellt;
$R^{8b}$ Wasserstoff, Hydroxy, Halogen oder $C_1$-Alkyl darstellt;
$R^{8c}$ Wasserstoff, Hydroxy, Halogen oder $C_1$-Alkyl darstellt;
$R^{8d}$ Wasserstoff, Hydroxy oder Halogen darstellt;
$R^{8e}$ Wasserstoff darstellt;
$R^{8f}$ Wasserstoff darstellt;
$R^{8g}$ Wasserstoff, Amino oder Halogen darstellt;
$R^{8h}$ Wasserstoff oder Halogen darstellt;
$R^{8i}$ Wasserstoff, Halogen oder $C_1$-$C_2$-Alkyl darstellt;
$R^{8k}$ Wasserstoff oder $C_1$-Alkyl darstellt;
$R^9$ $C_1$-$C_3$-Alkyl oder Cyclopropyl darstellt;
$R^{9a}$ $C_1$-$C_3$-Alkyl oder Cyclopropyl darstellt;
$R^{14}$ Wasserstoff, Methyl oder Ethyl darstellt;
$R^{15}$ Wasserstoff, Methyl oder Ethyl darstellt;
$R^{16}$ Wasserstoff, Methyl, Ethyl, Methoxyethyl oder Dimethylaminoethyl darstellt oder
$R^{15}$ und $R^{16}$ zusammen mit dem Stickstoffatom, an das sie gebunden sind, einen 5- bis 6-gliedrigen heterocyclischen Ring darstellen, wobei der heterocyclische Ring optional ein oder zwei weitere Heteroatome ausgewählt aus N und O enthält;
oder N-Oxid, Salz, Tautomer oder Stereoisomer der Verbindung oder Salz des N-Oxids, Tautomers oder Stereoisomers.

**3.** Verbindung der Formel (I) nach Anspruch 1 oder 2, wobei:

$R^1$ Wasserstoff oder eine Gruppe ausgewählt aus $C_1$-$C_2$-Alkyl und $C_3$-$C_4$-Cycloalkyl darstellt;
$R^{2a}$ Wasserstoff oder Halogen darstellt;
$R^{2b}$ Wasserstoff darstellt;
$R^{2c}$ Wasserstoff oder Halogen darstellt;
L

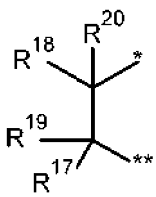

oder

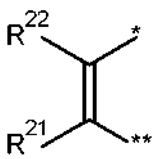

darstellt, wobei * den Punkt der direkten Bindung an das benachbarte N-Atom darstellt und ** den Punkt der direkten Bindung an das benachbarte C-Atom darstellt;
$R^{17}$ Wasserstoff, $C_1$-$C_3$-Alkyl, $C_1$-$C_3$-Haloalkyl oder $C_3$-$C_4$-Cycloalkyl-$C_1$-alkyl- darstellt;

$R^{18}$ Wasserstoff oder $C_1$-$C_3$-Alkyl darstellt;
$R^{19}$ Wasserstoff, $C_1$-$C_3$-Alkyl, $C_1$-$C_3$-Haloalkyl oder $C_3$-$C_4$-Cycloalkyl-$C_1$-alkyl- darstellt;
$R^{20}$ Wasserstoff oder $C_1$-$C_3$-Alkyl darstellt oder
$R^{18}$ und $R^{20}$ zusammen mit dem Kohlenstoffatom, an das sie gebunden sind, einen $C_3$-$C_6$-Cycloalkylring bilden;
$R^{21}$ Wasserstoff, Halogen, $C_1$-$C_3$-Alkyl, $C_1$-$C_3$-Haloalkyl oder $C_3$-$C_4$-Cycloalkyl-$C_1$-alkyl-darstellt;
$R^{22}$ Wasserstoff darstellt;
X Methylen darstellt,
wobei die Methylengruppe optional ein- oder zweimal mit $R^3$ substituiert ist;
Y Phenyl darstellt,
wobei das Phenyl optional ein- oder mehrmals mit $R^4$ substituiert ist;
$R^3$ $C_1$-$C_3$-Alkyl, $C_1$-$C_3$-Haloalkyl oder $R^5O$-$C_1$-$C_3$-Alkyl darstellt;
$R^4$ Halogen, $C_1$-$C_4$-Alkyl, $C_1$-$C_4$-Haloalkyl, -$CONR^{15}R^{16}$ oder -$SO_2Me$ darstellt;
$R^5$ Wasserstoff darstellt;
A eine Gruppe darstellt, ausgewählt aus:

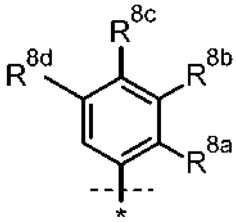 , 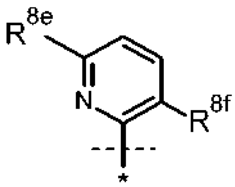 , 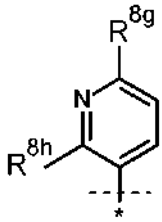 , 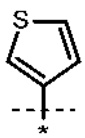 ,

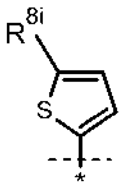 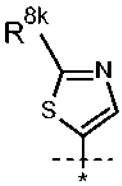 , 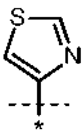 , 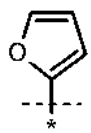 , 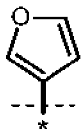 und 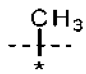 ,

wobei * den Bindungspunkt der Gruppe an den Rest der Formel (I) angibt;
$R^{8a}$ Wasserstoff, Hydroxy, Halogen oder Cyano darstellt;
$R^{8b}$ Wasserstoff, Halogen oder $C_1$-Alkyl darstellt;
$R^{8c}$ Wasserstoff, Halogen oder $C_1$-Alkyl darstellt;
$R^{8d}$ Wasserstoff oder Halogen darstellt;
$R^{8e}$ Wasserstoff darstellt;
$R^{8f}$ Wasserstoff darstellt;
$R^{8g}$ Wasserstoff oder Halogen darstellt;
$R^{8h}$ Wasserstoff darstellt;
$R^{8i}$ Wasserstoff, Halogen oder $C_1$-$C_2$-Alkyl darstellt;
$R^{8k}$ Wasserstoff oder $C_1$-Alkyl darstellt;
$R^{15}$ Wasserstoff oder Methyl darstellt;
$R^{16}$ Methyl oder Methoxyethyl darstellt oder
$R^{15}$ und $R^{16}$ zusammen mit dem Stickstoffatom, an das sie gebunden sind, einen 5- bis 6-gliedrigen heterocyclischen Ring darstellen, wobei der heterocyclische Ring optional ein weiteres Heteroatome ausgewählt aus N und O enthält;
oder N-Oxid, Salz, Tautomer oder Stereoisomer der Verbindung oder Salz des N-Oxids, Tautomers oder Stereoisomers.

**4.** Verbindung der Formel (I) nach Anspruch 1, 2 oder 3, wobei:

$R^1$ Wasserstoff oder eine Gruppe ausgewählt aus $C_1$-Alkyl und $C_3$-Cycloalkyl darstellt;
$R^{2a}$ Wasserstoff darstellt;
$R^{2b}$ Wasserstoff darstellt;

$R^{2c}$ Wasserstoff darstellt;
L

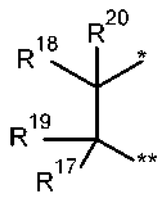

oder

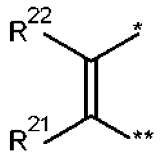

darstellt, wobei * den Punkt der direkten Bindung an das benachbarte N-Atom darstellt und ** den Punkt der direkten Bindung an das benachbarte C-Atom darstellt;
$R^{17}$ Wasserstoff oder $C_1$-Alkyl darstellt;
$R^{18}$ Wasserstoff oder $C_1$-Alkyl darstellt;
$R^{19}$ Wasserstoff, $C_1$-$C_3$-Alkyl, $C_1$-$C_3$-Haloalkyl oder $C_3$--Cycloalkyl-$C_1$-alkyl- darstellt;
$R^{20}$ Wasserstoff oder $C_1$-Alkyl darstellt oder
$R^{18}$ und $R^{20}$ zusammen mit dem Kohlenstoffatom, an das sie gebunden sind, einen $C_3$-$C_4$-Cycloalkylring bilden;
$R^{21}$ Wasserstoff, $C_1$-$C_3$-Alkyl, $C_1$-$C_3$-Haloalkyl oder $C_3$-Cycloalkyl-$C_1$-alkyl- darstellt;
$R^{22}$ Wasserstoff darstellt;
X Methylen darstellt,
wobei die Methylengruppe optional einmal mit $R^3$ substituiert ist;
Y Phenyl darstellt,
wobei das Phenyl optional einmal mit $R^4$ substituiert ist;
$R^3$ $C_1$-$C_3$-Alkyl oder $C_1$-$C_2$-Haloalkyl darstellt;
$R^4$ Halogen oder -CONR$^{15}$R$^{16}$ darstellt;
A eine Gruppe darstellt, ausgewählt aus:

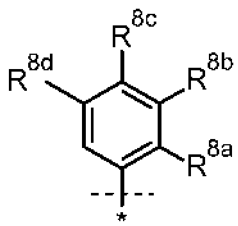
,
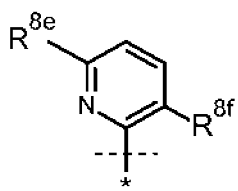
,
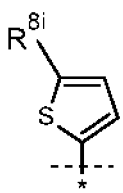
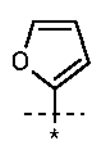
,
und
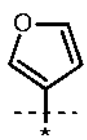
,

wobei * den Bindungspunkt der Gruppe an den Rest der Formel (I) angibt;
$R^{8a}$ Wasserstoff oder Fluor darstellt;
$R^{8b}$ Wasserstoff oder Fluor darstellt;

$R^{8c}$ Wasserstoff, Fluor oder Chlor darstellt;
$R^{8d}$ Wasserstoff darstellt;
$R^{8e}$ Wasserstoff darstellt;
$R^{8f}$ Wasserstoff darstellt;
$R^{8i}$ Wasserstoff, Chlor oder $C_1$-Alkyl darstellt;
$R^{15}$ Wasserstoff darstellt;
$R^{16}$ Methoxyethyl darstellt oder
$R^{15}$ und $R^{16}$ zusammen mit dem Stickstoffatom, an das sie gebunden sind, einen 5- bis 6-gliedrigen heterocyclischen Ring darstellen, wobei der heterocyclische Ring optional ein weiteres Heteroatom N enthält;
oder N-Oxid, Salz, Tautomer oder Stereoisomer der Verbindung oder Salz des N-Oxids, Tautomers oder Stereoisomers.

**5.** Verbindung der Formel (I) nach einem der Ansprüche 1 bis 4, die ausgewählt ist aus:

2-(4-Fluorphenyl)-N-[4-(5-methyl-4-oxo-3-phenyl-4,5,6,7-tetrahydro-1H-pyrrolo[3,2-c]pyridin-2-yl)pyridin-2-yl]acetamid;
4,4-Difluor-2-(4-fluorphenyl)-N-[4-(5-methyl-4-oxo-3-phenyl-4,5,6,7-tetrahydro-1H-pyrrolo[3,2-c]pyridin-2-yl)pyridin-2-yl]butanamid;
(2R)-4,4-Difluor-2-(4-fluorphenyl)-N-[4-(5-methyl-4-oxo-3-phenyl-4,5,6,7-tetrahydro-1H-pyrrolo [3,2-c]pyridin-2-yl)pyridin-2-yl]butanamid;
(2S)-4,4-Difluor-2-(4-fluorphenyl)-N-[4-(5-methyl-4-oxo-3-phenyl-4,5,6,7-tetrahydro-1H-pyrrolo[3,2-c]pyridin-2-yl)pyridin-2-yl]butanamid;
2-(4-Fluorphenyl)-N-[4-(5-methyl-4-oxo-3-phenyl-4,5-dihydro-1H-pyrrolo[3,2-c]pyridin-2-yl)pyridin-2-yl]acetamid;
4,4-Difluor-2-(4-fluorphenyl)-N-[4-(5-methyl-4-oxo-3-phenyl-4,5-dihydro-1H-pyrrolo[3,2-c]pyridin-2-yl)pyridin-2-yl]butanamid;
(2R)-4,4-Difluor-2-(4-fluorphenyl)-N-[4-(5-methyl-4-oxo-3-phenyl-4,5-dihydro-1H-pyrrolo[3,2-c]pyridin-2-yl)pyridin-2-yl]butanamid;
(2S)-4,4-Difluor-2-(4-fluorphenyl)-N-[4-(5-methyl-4-oxo-3-phenyl-4,5-dihydro-1H-pyrrolo[3,2-c]pyridin-2-yl)pyridin-2-yl]butanamid;
4,4-Difluor-2-(4-fluorphenyl)-N- {4-[5-methyl-4-oxo-3-(pyridin-3-yl)-4,5,6,7-tetrahydro-1H-pyrrolo [3,2-c]pyridin-2-yl]pyridin-2-yl} butanamid;
(2R)-4,4-Difluor-2-(4-fluorphenyl)-N-{4-[5-methyl-4-oxo-3-(pyridin-3-yl)-4,5,6,7-tetrahydro-1H-pyrrolo[3,2-c]pyridin-2-yl]pyridin-2-yl}butanamid;
(2S)-4,4-Difluor-2-(4-fluorphenyl)-N- {4-[5-methyl-4-oxo-3-(pyridin-3-yl)-4,5,6,7-tetrahydro-1H-pyrrolo [3,2-c]pyridin-2-yl]pyridin-2-yl}butanamid;
2-(4-Fluorphenyl)-N-{4-[5-methyl-4-oxo-3-(pyridin-3-yl)-4,5,6,7-tetrahydro-1H-pyrrolo[3,2-c]pyridin-2-yl]pyridin-2-yl} acetamid;
2-(4-Fluorphenyl)-N-{4-[5-methyl-4-oxo-3-phenyl-7-(2,2,2-trifluorethyl)-4,5,6,7-tetrahydro-1H-pyrrolo[3,2-c]pyridin-2-yl]pyridin-2-yl}propanamid;
2-(4-Fluorphenyl)-N-{4-[5-methyl-4-oxo-3-phenyl-7-(2,2,2-trifluorethyl)-4,5-dihydro-1H-pyrrolo[3,2-c]pyridin-2-yl]pyridin-2-yl}propanamid;
(2R)-2-(4-Fluorphenyl)-N-{4-[5-methyl-4-oxo-3-phenyl-7-(2,2,2-trifluorethyl)-4,5-dihydro-1H-pyrrolo[3,2-c]pyridin-2-yl]pyridin-2-yl} propanamid;
(2S)-2-(4-Fluorphenyl)-N-{4-[5-methyl-4-oxo-3-phenyl-7-(2,2,2-trifluorethyl)-4,5-dihydro-1H-pyrrolo[3,2-c]pyridin-2-yl]pyridin-2-yl}propanamid;
4,4-Difluor-2-(4-fluorphenyl)-N-{4-[5-methyl-4-oxo-3-phenyl-7-(2,2,2-trifluorethyl)-4,5,6,7-tetrahydro-1H-pyrrolo [3,2-c]pyridin-2-yl]pyridin-2-yl}butanamid;
4,4-Difluor-2-(4-fluorphenyl)-N- {4-[5-methyl-4-oxo-3-phenyl-7-(2,2,2-trifluorethyl)-4,5-dihydro-1H-pyrrolo[3,2-c]pyridin-2-yl]pyridin-2-yl}butanamid;
(2R)-4,4-Difluor-2-(4-fluorphenyl)-N-{4-[5-methyl-4-oxo-3-phenyl-7-(2,2,2-trifluorethyl)-4,5-dihydro-1H-pyrrolo [3,2-c]pyridin-2-yl]pyridin-2-yl} butanamid;
(2S)-4,4-Difluor-2-(4-fluorphenyl)-N-{4-[5-methyl-4-oxo-3-phenyl-7-(2,2,2-trifluorethyl)-4,5-dihydro-1H-pyrrolo [3,2-c]pyridin-2-yl]pyridin-2-yl} butanamid;
2-(4-Fluorphenyl)-N-{4-[3-(4-fluorphenyl)-5-methyl-4-oxo-7-(2,2,2-trifluorethyl)-4,5,6,7-tetrahydro-1H-pyrrolo[3,2-c]pyridin-2-yl]pyridin-2-yl}propanamid;
2-(4-Fluorphenyl)-N-{4-[3-(4-fluorphenyl)-5-methyl-4-oxo-7-(2,2,2-trifluorethyl)-4,5-dihydro-1H-pyrrolo[3,2-c]pyridin-2-yl]pyridin-2-yl} propanamid;

(2R)-2-(4-Fluorphenyl)-N- {4-[3-(4-fluorphenyl)-5-methyl-4-oxo-7-(2,2,2-trifluorethyl)-4,5-dihydro-1H-pyrrolo [3,2-c]pyridin-2-yl]pyridin-2-yl}propanamid;
(2S)-2-(4-Fluorphenyl)-N- {4-[3-(4-fluorphenyl)-5-methyl-4-oxo-7-(2,2,2-trifluorethyl)-4,5-dihydro-1H-pyrrolo [3,2-c]pyridin-2-yl]pyridin-2-yl}propanamid;
4,4-Difluor-2-(4-fluorphenyl)-N- {4-[3-(4-fluorphenyl)-5-methyl-4-oxo-7-(2,2,2-trifluorethyl)-4,5,6,7-tetrahydro-1H-pyrrolo[3,2-c]pyridin-2-yl]pyridin-2-yl}butanamid;
4,4-Difluor-2-(4-fluorphenyl)-N-{4-[3-(4-fluorphenyl)-5-methyl-4-oxo-7-(2,2,2-trifluorethyl)-4,5-dihydro-1H-pyrrolo [3,2-c]pyridin-2-yl]pyridin-2-yl} butanamid;
(2R)-4,4-Difluor-2-(4-fluorphenyl)-N- {4-[3-(4-fluorphenyl)-5-methyl-4-oxo-7-(2,2,2-trifluorethyl)-4,5-dihydro-1H-pyrrolo[3,2-c]pyridin-2-yl]pyridin-2-yl}butanamid;
(2S)-4,4-Difluor-2-(4-fluorphenyl)-N-{4-[3-(4-fluorphenyl)-5-methyl-4-oxo-7-(2,2,2-trifluorethyl)-4,5-dihydro-1H-pyrrolo [3,2-c]pyridin-2-yl]pyridin-2-yl} butanamid;
2-(4-Fluorphenyl)-N-{4-[3-(4-fluorphenyl)-5,7-dimethyl-4-oxo-4,5,6,7-tetrahydro-1H-pyrrolo[3,2-c]pyridin-2-yl]pyridin-2-yl}propanamid;
2-(4-Fluorphenyl)-N-{4-[3-(4-fluorphenyl)-5,7-dimethyl-4-oxo-4,5-dihydro-1H-pyrrolo[3,2-c]pyridin-2-yl]pyridin-2-yl}propanamid;
(2R)-2-(4-Fluorphenyl)-N-{4-[3-(4-fluorphenyl)-5,7-dimethyl-4-oxo-4,5-dihydro-1H-pyrrolo[3,2-c]pyridin-2-yl]pyridin-2-yl}propanamid;
(2S)-2-(4-Fluorphenyl)-N-{4-[3-(4-fluorphenyl)-5,7-dimethyl-4-oxo-4,5-dihydro-1H-pyrrolo[3,2-c]pyridin-2-yl]pyridin-2-yl}propanamid;
4,4-Difluor-2-(4-fluorphenyl)-N- {4-[3-(4-fluorphenyl)-5,7-dimethyl-4-oxo-4,5,6,7-tetrahydro-1H-pyrrolo[3,2-c]pyridin-2-yl]pyridin-2-yl}butanamid;
4,4-Difluor-2-(4-fluorphenyl)-N-{4-[3-(4-fluorphenyl)-5,7-dimethyl-4-oxo-4,5-dihydro-1H-pyrrolo[3,2-c]pyridin-2-yl]pyridin-2-yl}butanamid;
(2R)-4,4-Difluor-2-(4-fluorphenyl)-N- {4-[3-(4-fluorphenyl)-5,7-dimethyl-4-oxo-4,5-dihydro-1H-pyrrolo[3,2-c]pyridin-2-yl]pyridin-2-yl} butanamid;
(2S)-4,4-Difluor-2-(4-fluorphenyl)-N-{4-[3-(4-fluorphenyl)-5,7-dimethyl-4-oxo-4,5-dihydro-1H-pyrrolo[3,2-c]pyridin-2-yl]pyridin-2-yl}butanamid;
N-{4-[5,7-Dimethyl-4-oxo-3-phenyl-4,5,6,7-tetrahydro-1H-pyrrolo[3,2-c]pyridin-2-yl]pyridin-2-yl}-2-(4-fluorphenyl)propanamid;
N- [4-(5,7-Dimethyl-4-oxo-3 -phenyl-4,5-dihydro-1H-pyrrolo [3,2-c]pyridin-2-yl)pyridin-2-yl]-2-(4-fluorphenyl)propanamid;
N-{4-[5,7-Dimethyl-4-oxo-3-phenyl-4,5,6,7-tetrahydro-1H-pyrrolo[3,2-c]pyridin-2-yl]pyridin-2-yl} -4,4-difluor-2-(4-fluorphenyl)butanamid;
N-[4-(5,7-Dimethyl-4-oxo-3-phenyl-4,5-dihydro-1H-pyrrolo[3,2-c]pyridin-2-yl)pyridin-2-yl]-4,4-difluor-2-(4-fluorphenyl)butanamid;
4,4-Difluor-2-(4-fluorphenyl)-N- {4-[5,6,7-trimethyl-4-oxo-3-phenyl-4,5,6,7-tetrahydro-1H-pyrrolo [3,2-c]pyridin-2-yl]pyridin-2-yl} butanamid;
N-[4-(7,7-Dimethyl-4-oxo-3-phenyl-4,5,6,7-tetrahydro-1H-pyrrolo[3,2-c]pyridin-2-yl)pyridin-2-yl]-2-(4-fluorphenyl)acetamid;
N-[4-(7,7-Dimethyl-4-oxo-3-phenyl-4,5,6,7-tetrahydro-1H-pyrrolo[3,2-c]pyridin-2-yl)pyridin-2-yl]-4,4-difluor-2-(4-fluorphenyl)butanamid;
(2R)-N-[4-(7,7-Dimethyl-4-oxo-3-phenyl-4,5,6,7-tetrahydro-1H-pyrrolo[3,2-c]pyridin-2-yl)pyridin-2-yl]-4,4-difluor-2-(4-fluorphenyl)butanamid;
(2S)-N-[4-(7,7-Dimethyl-4-oxo-3-phenyl-4,5,6,7-tetrahydro-1H-pyrrolo[3,2-c]pyridin-2-yl)pyridin-2-yl]-4,4-difluor-2-(4-fluorphenyl)butanamid;
N-[4-(7,7-Dimethyl-4-oxo-3-phenyl-4,5,6,7-tetrahydro-1H-pyrrolo[3,2-c]pyridin-2-yl)pyridin-2-yl]-2-(4-fluorphenyl)propanamid;
(2R)-N-[4-(7,7-Dimethyl-4-oxo-3-phenyl-4,5,6,7-tetrahydro-1H-pyrrolo[3,2-c]pyridin-2-yl)pyridin-2-yl]-2-(4-fluorphenyl)propanamid;
(2S)-N-[4-(7,7-Dimethyl-4-oxo-3-phenyl-4,5,6,7-tetrahydro-1H-pyrrolo[3,2-c]pyridin-2-yl)pyridin-2-yl]-2-(4-fluorphenyl)propanamid;
4,4-Difluor-2-(4-fluorphenyl)-N-[4-(5'-methyl-4'-oxo-3'-phenyl-1',4',5',7'-tetrahydrospiro[cyclobutan-1,6'-pyrrolo[3,2-c]pyridin]-2'-yl)pyridin-2-yl]butanamid;
(2R)-4,4-Difluor-2-(4-fluorphenyl)-N-[4-(5'-methyl-4'-oxo-3'-phenyl-1',4',5',7'-tetrahydrospiro-[cyclobutan-1,6'-pyrrolo[3,2-c]pyridin]-2'-yl)pyridin-2-yl]butanamid;
(2S)-4,4-Difluor-2-(4-fluorphenyl)-N-[4-(5'-methyl-4'-oxo-3'-phenyl-1',4',5',7'-tetrahydrospiro-[cyclobutan-1,6'-pyrrolo[3,2-c]pyridin]-2'-yl)pyridin-2-yl]butanamid;

2-(4-Fluorphenyl)-N-[4-(5'-methyl-4'-oxo-3'-phenyl-1',4',5',7'-tetrahydrospiro[cyclobutan-1,6'-pyrrolo[3,2-c]pyridin]-2'-yl)pyridin-2-yl]propanamid;
(2R)-2-(4-Fluorphenyl)-N-[4-(5'-methyl-4'-oxo-3'-phenyl-1',4',5',7'-tetrahydrospiro[cyclobutan-1,6'-pyrrolo[3,2-c]pyridin]-2'-yl)pyridin-2-yl]propanamid;
(2S)-2-(4-Fluorphenyl)-N-[4-(5'-methyl-4'-oxo-3'-phenyl-1',4',5',7'-tetrahydrospiro[cyclobutan-1,6'-pyrrolo[3,2-c]pyridin]-2'-yl)pyridin-2-yl]propanamid;
N-{4-[7-(Cyclopropylmethyl)-5-methyl-4-oxo-3-phenyl-4,5,6,7-tetrahydro-1H-pyrrolo[3,2-c]pyridin-2-yl]pyridin-2-yl}-2-(4-fluorphenyl)acetamid;
N-{4-[7-(Cyclopropylmethyl)-5-methyl-4-oxo-3-phenyl-4,5-dihydro-1H-pyrrolo[3,2-c]pyridin-2-yl]pyridin-2-yl} -2-(4-fluorphenyl)acetamid;
N-{4-[7-(Cyclopropylmethyl)-5-methyl-4-oxo-3-phenyl-4,5,6,7-tetrahydro-1H-pyrrolo[3,2-c]pyridin-2-yl]pyridin-2-yl}-2-(4-fluorphenyl)propanamid;
N-{4-[7-(Cyclopropylmethyl)-5-methyl-4-oxo-3-phenyl-4,5-dihydro-1H-pyrrolo[3,2-c]pyridin-2-yl]pyridin-2-yl} -2-(4-fluorphenyl)propanamid;
N-{4-[7-(Cyclopropylmethyl)-5-methyl-4-oxo-3-phenyl-4,5,6,7-tetrahydro-1H-pyrrolo[3,2-c]pyridin-2-yl]pyridin-2-yl}-4,4-difluor-2-(4-fluorphenyl)butanamid;
N-{4-[7-(Cyclopropylmethyl)-5-methyl-4-oxo-3-phenyl-4,5-dihydro-1H-pyrrolo[3,2-c]pyridin-2-yl]pyridin-2-yl}-4,4-difluor-2-(4-fluorphenyl)butanamid;
N-{4-[7-(Cyclopropylmethyl)-3-(4-fluorphenyl)-5-methyl-4-oxo-4,5,6,7-tetrahydro-1H-pyrrolo[3,2-c]pyridin-2-yl]pyridin-2-yl} -2-(4-fluorphenyl)acetamid;
N-{4-[7-(Cyclopropylmethyl)-3-(4-fluorphenyl)-5-methyl-4-oxo-4,5,6,7-tetrahydro-1H-pyrrolo[3,2-c]pyridin-2-yl]pyridin-2-yl}-2-(4-fluorphenyl)propanamid;
N-{4-[7-(Cyclopropylmethyl)-3-(4-fluorphenyl)-5-methyl-4-oxo-4,5-dihydro-1H-pyrrolo[3,2-c]pyridin-2-yl]pyridin-2-yl} -2-(4-fluorphenyl)propanamid;
N-{4-[7-(Cyclopropylmethyl)-3-(4-fluorphenyl)-5-methyl-4-oxo-4,5,6,7-tetrahydro-1H-pyrrolo[3,2-c]pyridin-2-yl]pyridin-2-yl}-4,4-difluor-2-(4-fluorphenyl)butanamid;
N-{4-[7-(Cyclopropylmethyl)-3-(4-fluorphenyl)-5-methyl-4-oxo-4,5-dihydro-1H-pyrrolo[3,2-c]pyridin-2-yl]pyridin-2-yl}-4,4-difluor-2-(4-fluorphenyl)butanamid;
N-{4-[3-(2,4-Difluorphenyl)-5-methyl-4-oxo-4,5,6,7-tetrahydro-1H-pyrrolo[3,2-c]pyridin-2-yl]pyridin-2-yl}-4,4-difluor-2-(4-fluorphenyl)butanamid;
(2R)-N-{4-[3-(2,4-Difluorphenyl)-5-methyl-4-oxo-4,5,6,7-tetrahydro-1H-pyrrolo[3,2-c]pyridin-2-yl]pyridin-2-yl}-4,4-difluor-2-(4-fluorphenyl)butanamid;
(2S)-N-{4-[3-(2,4-Difluorphenyl)-5-methyl-4-oxo-4,5,6,7-tetrahydro-1H-pyrrolo[3,2-c]pyridin-2-yl]pyridin-2-yl}-4,4-difluor-2-(4-fluorphenyl)butanamid;
N-{4-[3-(2,4-Difluorphenyl)-5-methyl-4-oxo-4,5,6,7-tetrahydro-1H-pyrrolo[3,2-c]pyridin-2-yl]pyridin-2-yl}-2-(4-fluorphenyl)propanamid;
(2R)-N-{4-[3-(2,4-Difluorphenyl)-5-methyl-4-oxo-4,5,6,7-tetrahydro-lH-pyrrolo[3,2-c]pyridin-2-yl]pyridin-2-yl}-2-(4-fluorphenyl)propanamid;
(2S)-N-{4-[3-(2,4-Difluorphenyl)-5-methyl-4-oxo-4,5,6,7-tetrahydro-1H-pyrrolo[3,2-c]pyridin-2-yl]pyridin-2-yl}-2-(4-fluorphenyl)propanamid;
2-(4-Fluorphenyl)-N-[4-(4-oxo-3-phenyl-4,5,6,7-tetrahydro-1H-pyrrolo[3,2-c]pyridin-2-yl)pyridin-2-yl] acetamid;
2-(4-Fluorphenyl)-N-[4-(4-oxo-3-phenyl-4,5-dihydro-1H-pyrrolo[3,2-c]pyridin-2-yl)pyridin-2-yl] acetamid;
2-(4-Fluorphenyl)-N-[4-(4-oxo-3-phenyl-4,5,6,7-tetrahydro-1H-pyrrolo[3,2-c]pyridin-2-yl)pyridin-2-yl]propanamid;
4,4-Difluor-2-(4-fluorphenyl)-N-[4-(4-oxo-3-phenyl-4,5,6,7-tetrahydro-1H-pyrrolo[3,2-c]pyridin-2-yl)pyridin-2-yl]butanamid;
4,4-Difluor-2-(4-fluorphenyl)-N-[4-(4-oxo-3-phenyl-4,5-dihydro-1H-pyrrolo[3,2-c]pyridin-2-yl)pyridin-2-yl]butanamid;
(2R)-4,4-Difluor-2-(4-fluorphenyl)-N-[4-(4-oxo-3-phenyl-4,5-dihydro-1H-pyrrolo[3,2-c]pyridin-2-yl)pyridin-2-yl]butanamid;
(2S)-4,4-Difluor-2-(4-fluorphenyl)-N-[4-(4-oxo-3-phenyl-4,5-dihydro-1H-pyrrolo[3,2-c]pyridin-2-yl)pyridin-2-yl]butanamid;
N-[4-(7-Chlor-5-methyl-4-oxo-3-phenyl-4,5-dihydro-1H-pyrrolo[3,2-c]pyridin-2-yl)pyridin-2-yl]-2-(4-fluorphenyl)acetamid;
4,4-Difluor-2-(4-fluorphenyl)-N-[4-(5'-methyl-4'-oxo-3'-phenyl-1',4',5',7'-tetrahydrospiro-[cyclopropan-1,6'-pyrrolo[3,2-c]pyridin]-2'-yl)pyridin-2-yl]butanamid;
4,4-Difluor-2-(4-fluorphenyl)-N-{4-[3'-(4-fluorphenyl)-5'-methyl-4'-oxo-1',4',5',7'-tetrahydro-spiro[cyclopropan-1,6'-pyrrolo[3,2-c]pyridin]-2'-yl]pyridin-2-yl}butanamid;

N-[4-(6,6-Dimethyl-4-oxo-3-phenyl-4,5,6,7-tetrahydro-1H-pyrrolo[3,2-c]pyridin-2-yl)pyridin-2-yl]-2-(4-fluorphenyl)acetamid;
2-(4-Fluorphenyl)-N-{4-[3-(4-fluorphenyl)-6,6-dimethyl-4-oxo-4,5,6,7-tetrahydro-1H-pyrrolo[3,2-c]pyridin-2-yl]pyridin-2-yl} acetamid;
2-(4-Fluorphenyl)-N-{4-[3-(2-fluorphenyl)-6,6-dimethyl-4-oxo-4,5,6,7-tetrahydro-1H-pyrrolo[3,2-c]pyridin-2-yl]pyridin-2-yl}acetamid;
2-(4-Fluorphenyl)-N-{4-[3-(3-fluorphenyl)-6,6-dimethyl-4-oxo-4,5,6,7-tetrahydro-1H-pyrrolo[3,2-c]pyridin-2-yl]pyridin-2-yl} acetamid;
N-{4-[6,6-Dimethyl-3-(3-methylphenyl)-4-oxo-4,5,6,7-tetrahydro-1H-pyrrolo[3,2-c]pyridin-2-yl]pyridin-2-yl}-2-(4-fluorphenyl)acetamid;
2-(4-Fluorphenyl)-N-{4-[3-(2-fluorphenyl)-6,6-dimethyl-4-oxo-4,5,6,7-tetrahydro-1H-pyrrolo[3,2-c]pyridin-2-yl]pyridin-2-yl}propanamid;
N-{4-[6,6-Dimethyl-3-(3-methylphenyl)-4-oxo-4,5,6,7-tetrahydro-1H-pyrrolo[3,2-c]pyridin-2-yl]pyridin-2-yl}-2-(4-fluorphenyl)propanamid;
N-{4-[3-(4-Chlorphenyl)-6,6-dimethyl-4-oxo-4,5,6,7-tetrahydro-1H-pyrrolo[3,2-c]pyridin-2-yl]pyridin-2-yl} -2-(4-fluorphenyl)propanamid;
N-{4-[3-(4-Chlorphenyl)-6,6-dimethyl-4-oxo-4,5,6,7-tetrahydro-1H-pyrrolo[3,2-c]pyridin-2-yl]pyridin-2-yl} -2-(4-fluorphenyl)acetamid;
N-{4-[6,6-Dimethyl-3-(4-methylphenyl)-4-oxo-4,5,6,7-tetrahydro-1H-pyrrolo[3,2-c]pyridin-2-yl]pyridin-2-yl} -2-(4-fluorphenyl)propanamid;
N-{4-[6,6-Dimethyl-3-(4-methylphenyl)-4-oxo-4,5,6,7-tetrahydro-1H-pyrrolo[3,2-c]pyridin-2-yl]pyridin-2-yl} -2-(4-fluorphenyl)acetamid;
N-{4-[6,6-Dimethyl-4-oxo-3-(pyridin-3-yl)-4,5,6,7-tetrahydro-1H-pyrrolo[3,2-c]pyridin-2-yl]pyridin-2-yl}-2-(4-fluorphenyl)acetamid;
2-(4-Fluorphenyl)-N-{4-[3-(4-fluorphenyl)-6,6-dimethyl-4-oxo-4,5,6,7-tetrahydro-1H-pyrrolo[3,2-c]pyridin-2-yl]pyridin-2-yl}propanamid;
(2R)-2-(4-Fluorphenyl)-N-{4-[3-(4-fluorphenyl)-6,6-dimethyl-4-oxo-4,5,6,7-tetrahydro-1H-pyrrolo[3,2-c]pyridin-2-yl]pyridin-2-yl}propanamid;
(2S)-2-(4-Fluorphenyl)-N-{4-[3-(4-fluorphenyl)-6,6-dimethyl-4-oxo-4,5,6,7-tetrahydro-1H-pyrrolo [3,2-c]pyridin-2-yl]pyridin-2-yl} propanamid;
N-{4-[6,6-Dimethyl-4-oxo-3-(pyridin-3-yl)-4,5,6,7-tetrahydro-1H-pyrrolo[3,2-c]pyridin-2-yl]pyridin-2-yl}-2-(4-fluorphenyl)propanamid;
(2R)-N-{4-[6,6-Dimethyl-4-oxo-3-(pyridin-3-yl)-4,5,6,7-tetrahydro-1H-pyrrolo[3,2-c]pyridin-2-yl]pyridin-2-yl} -2-(4-fluorphenyl)propanamid;
(2S)-N-{4-[6,6-Dimethyl-4-oxo-3-(pyridin-3-yl)-4,5,6,7-tetrahydro-1H-pyrrolo[3,2-c]pyridin-2-yl]pyridin-2-yl} -2-(4-fluorphenyl)propanamid;
2-(4-Fluorphenyl)-N-{4-[3-(4-fluorphenyl)-5-methyl-4-oxo-4,5-dihydro-1H-pyrrolo[3,2-c]pyridin-2-yl]pyridin-2-yl}propanamid;
(2R)-2-(4-Fluorphenyl)-N-{4-[3-(4-fluorphenyl)-5-methyl-4-oxo-4,5-dihydro-1H-pyrrolo[3,2-c]pyridin-2-yl]pyridin-2-yl}propanamid;
(2S)-2-(4-Fluorphenyl)-N-{4-[3-(4-fluorphenyl)-5-methyl-4-oxo-4,5-dihydro-1H-pyrrolo[3,2-c]pyridin-2-yl]pyridin-2-yl}propanamid;
2-(4-Fluorphenyl)-N-[4-(5-methyl-4-oxo-3-phenyl-4,5-dihydro-1H-pyrrolo[3,2-c]pyridin-2-yl)pyridin-2-yl]propanamid;
(2R)-2-(4-Fluorphenyl)-N-[4-(5-methyl-4-oxo-3-phenyl-4,5-dihydro-1H-pyrrolo[3,2-c]pyridin-2-yl)pyridin-2-yl]propanamid;
(2S)-2-(4-Fluorphenyl)-N-[4-(5-methyl-4-oxo-3-phenyl-4,5-dihydro-1H-pyrrolo[3,2-c]pyridin-2-yl)pyridin-2-yl]propanamid;
N-{4-[3-(3-Chlorphenyl)-5-methyl-4-oxo-4,5-dihydro-1H-pyrrolo[3,2-c]pyridin-2-yl]pyridin-2-yl}-2-(4-fluorphenyl)propanamid;
4,4-Difluor-2-(4-fluorphenyl)-N-{4-[3-(4-fluorphenyl)-5-methyl-4-oxo-4,5-dihydro-1H-pyrrolo[3,2-c]pyridin-2-yl]pyridin-2-yl}butanamid;
(2R)-4,4-Difluor-2-(4-fluorphenyl)-N- {4-[3-(4-fluorphenyl)-5-methyl-4-oxo-4,5-dihydro-1H-pyrrolo[3,2-c]pyridin-2-yl]pyridin-2-yl}butanamid;
(2S)-4,4-Difluor-2-(4-fluorphenyl)-N- {4-[3-(4-fluorphenyl)-5-methyl-4-oxo-4,5-dihydro-1H-pyrrolo[3,2-c]pyridin-2-yl]pyridin-2-yl}butanamid;
2-(4-Fluorphenyl)-N-{4-[3-(2-fluorpyridin-3-yl)-5-methyl-4-oxo-4,5-dihydro-1H-pyrrolo[3,2-c]pyridin-2-yl]pyridin-2-yl}propanamid;

2-(4-Fluorphenyl)-N-{4-[5-methyl-3-(2-methylphenyl)-4-oxo-4,5-dihydro-1H-pyrrolo[3,2-c]pyridin-2-yl]pyridin-2-yl}propanamid;
N-{4-[3-(2-Chlorphenyl)-5-methyl-4-oxo-4,5-dihydro-1H-pyrrolo[3,2-c]pyridin-2-yl]pyridin-2-yl} -2-(4-fluorphenyl)propanamid;
2-(4-Fluorphenyl)-N-{4-[3-(3-hydroxyphenyl)-5-methyl-4-oxo-4,5-dihydro-1H-pyrrolo[3,2-c]pyridin-2-yl]pyridin-2-yl}propanamid;
N-{4-[3-(3,4-Difluorphenyl)-5-methyl-4-oxo-4,5-dihydro-1H-pyrrolo[3,2-c]pyridin-2-yl]pyridin-2-yl}-2-(4-fluorphenyl)propanamid;
2-(4-Fluorphenyl)-N-{4-[3-(3-furyl)-5-methyl-4-oxo-4,5-dihydro-1H-pyrrolo[3,2-c]pyridin-2-yl]pyridin-2-yl}propanamid;
2-(4-Fluorphenyl)-N-{4-[3-(2-furyl)-5-methyl-4-oxo-4,5-dihydro-1H-pyrrolo[3,2-c]pyridin-2-yl]pyridin-2-yl} propanamid;
2-(4-Fluorphenyl)-N-{4-[5-methyl-4-oxo-3-(3,4,5-trifluorphenyl)-4,5-dihydro-1H-pyrrolo[3,2-c]pyridin-2-yl]pyridin-2-yl}propanamid;
4,4-Difluor-2-(4-fluorphenyl)-N-{4-[5-methyl-4-oxo-3-(pyridin-3-yl)-4,5-dihydro-1H-pyrrolo[3,2-c]pyridin-2-yl]pyridin-2-yl}butanamid;
2-(4-Fluorphenyl)-N-{4-[3-(4-hydroxyphenyl)-5-methyl-4-oxo-4,5-dihydro-1H-pyrrolo[3,2-c]pyridin-2-yl]pyridin-2-yl}propanamid;
N-{4-[3-(2,5-Difluorphenyl)-5-methyl-4-oxo-4,5-dihydro-1H-pyrrolo[3,2-c]pyridin-2-yl]pyridin-2-yl}-2-(4-fluorphenyl)propanamid;
N-{4-[3-(3,5-Difluorphenyl)-5-methyl-4-oxo-4,5-dihydro-1H-pyrrolo[3,2-c]pyridin-2-yl]pyridin-2-yl}-2-(4-fluorphenyl)propanamid;
N-{4-[3-(5-Chlor-2-thienyl)-5-methyl-4-oxo-4,5-dihydro-1H-pyrrolo[3,2-c]pyridin-2-yl]pyridin-2-yl}-2-(4-fluorphenyl)propanamid;
N-{4-[3-(2-Cyanophenyl)-5-methyl-4-oxo-4,5-dihydro-1H-pyrrolo[3,2-c]pyridin-2-yl]pyridin-2-yl}-2-(4-fluorphenyl)propanamid;
2-(4-Fluorphenyl)-N-{4-[3-(2-hydroxyphenyl)-5-methyl-4-oxo-4,5-dihydro-1H-pyrrolo[3,2-c]pyridin-2-yl]pyridin-2-yl}propanamid;
2-(4-Fluorphenyl)-N-{4-[5-methyl-3-(5-methyl-2-thienyl)-4-oxo-4,5-dihydro-1H-pyrrolo[3,2-c]pyridin-2-yl]pyridin-2-yl}propanamid;
N-{4-[3-(2-Aminopyridin-4-yl)-5-methyl-4-oxo-4,5-dihydro-1H-pyrrolo[3,2-c]pyridin-2-yl]pyridin-2-yl}-2-(4-fluorphenyl)propanamid;
N-{4-[3-(2-Cyano-5-fluorphenyl)-5-methyl-4-oxo-4,5-dihydro-1H-pyrrolo[3,2-c]pyridin-2-yl]pyridin-2-yl}-2-(4-fluorphenyl)propanamid;
N-{4-[3-(2-Cyano-5-fluorphenyl)-5-methyl-4-oxo-4,5,6,7-tetrahydro-1H-pyrrolo[3,2-c]pyridin-2-yl]pyridin-2-yl}-4,4-difluor-2-(4-fluorphenyl)butanamid;
4,4-Difluor-2-(4-fluorphenyl)-N- {4-[5-methyl-3-(2-methyl-1,3-thiazol-5-yl)-4-oxo-4,5,6,7-tetrahydro-1H-pyrrolo[3,2-c]pyridin-2-yl]pyridin-2-yl}butanamid;
2-(4-Fluorphenyl)-N-{4-[5-methyl-4-oxo-3-(2-thienyl)-4,5-dihydro-1H-pyrrolo[3,2-c]pyridin-2-yl]pyridin-2-yl}propanamid;
2-(4-Fluorphenyl)-N-{4-[5-methyl-4-oxo-3-(3-thienyl)-4,5-dihydro-1H-pyrrolo[3,2-c]pyridin-2-yl]pyridin-2-yl}propanamid;
N-[4-(3,5-Dimethyl-4-oxo-4,5-dihydro-1H-pyrrolo[3,2-c]pyridin-2-yl)pyridin-2-yl]-2-(4-fluorphenyl)propanamid;
N-{4-[3-(5-Chlor-2-thienyl)-5-methyl-4-oxo-4,5-dihydro-1H-pyrrolo[3,2-c]pyridin-2-yl]pyridin-2-yl} -4,4-difluor-2-(4-fluorphenyl)butanamid;
N-{4-[5-Cyclopropyl-3-(4-fluorphenyl)-4-oxo-4,5-dihydro-1H-pyrrolo[3,2-c]pyridin-2-yl]pyridin-2-yl} -4,4-difluor-2-(4-fluorphenyl)butanamid;
(2R)-N-{4-[5-Cyclopropyl-3-(4-fluorphenyl)-4-oxo-4,5-dihydro-1H-pyrrolo[3,2-c]pyridin-2-yl]pyridin-2-yl}-4,4-difluor-2-(4-fluorphenyl)butanamid;
(2S)-N-{4-[5-Cyclopropyl-3-(4-fluorphenyl)-4-oxo-4,5-dihydro-1H-pyrrolo[3,2-c]pyridin-2-yl]pyridin-2-yl}-4,4-difluor-2-(4-fluorphenyl)butanamid;
N-[4-(5-Cyclopropyl-4-oxo-3-phenyl-4,5-dihydro-1H-pyrrolo[3,2-c]pyridin-2-yl)pyridin-2-yl]-4,4-difluor-2-(4-fluorphenyl)butanamid;
4,4-Difluor-2-(4-fluorphenyl)-N-{4-[5-methyl-4-oxo-3-(1,3-thiazol-4-yl)-4,5,6,7-tetrahydro-1H-pyrrolo[3,2-c]pyridin-2-yl]pyridin-2-yl}butanamid;
4,4-Difluor-2-(4-fluorphenyl)-N-{4-[3-(4-fluorphenyl)-5-methyl-4-oxo-4,5,6,7-tetrahydro-1H-pyrrolo[3,2-c]pyridin-2-yl]pyridin-2-yl}butanamid;
4-[2-({4-[3-(4-Fluorphenyl)-5-methyl-4-oxo-4,5,6,7-tetrahydro-1H-pyrrolo[3,2-c]pyridin-2-yl]pyridin-2-yl}amino)-2-oxoethyl]-N-(2-methoxyethyl)benzamid;
4-[2-({4-[3-(4-Fluorphenyl)-5-methyl-4-oxo-4,5,6,7-tetrahydro-1H-pyrrolo[3,2-c]pyridin-2-yl]pyridin-2-yl}amino)-2-oxoethyl]-N-(2-methoxyethyl)-N-methylbenzamid;
4-[2-({4-[3-(4-Fluorphenyl)-5-methyl-4-oxo-4,5-dihydro-1H-pyrrolo[3,2-c]pyridin-2-yl]pyridin-2-yl } amino)-2-oxoethyl]-N-(2-methoxyethyl)benzamid;
4-[2-({4-[3-(4-Fluorphenyl)-5-methyl-4-oxo-4,5-dihydro-1H-pyrrolo[3,2-c]pyridin-2-yl]pyridin-2-yl } amino)-2-oxoethyl]-N-(2-methoxyethyl)-N-methylbenzamid;
N-{4-[3-(4-Fluorphenyl)-5-methyl-4-oxo-4,5,6,7-tetrahydro-1H-pyrrolo[3,2-c]pyridin-2-yl]pyridin-2-yl}-2-[4-(piperazin-1-carbonyl)phenyl]acetamid;
2-(4-Fluorphenyl)-N-{4-[3-(6-fluorpyridin-3-yl)-5-methyl-4-oxo-4,5-dihydro-1H-pyrrolo[3,2-c]pyridin-2-yl]pyridin-2-yl}propanamid;
2-(4-Fluorphenyl)-N-{4-[5-methyl-4-oxo-3-(pyridin-2-yl)-4,5-dihydro-1H-pyrrolo[3,2-c]pyridin-2-yl]pyridin-2-yl}acetamid;
(2RS)-N-{4-[7-(2,2-Difluorethyl)-5-methyl-4-oxo-3-phenyl-4,5-dihydro-1H-pyrrolo[3,2-c]pyridin-2-yl]pyridin-2-yl}-4,4-difluor-2-(4-fluorphenyl)butanamid;
(2R)-N-{4-[7-(2,2-Difluorethyl)-5-methyl-4-oxo-3-phenyl-4,5-dihydro-1H-pyrrolo[3,2-c]pyridin-2-yl]pyridin-2-yl}-4,4-difluor-2-(4-fluorphenyl)butanamid;
(2S)-N-{4-[7-(2,2-Difluorethyl)-5-methyl-4-oxo-3-phenyl-4,5-dihydro-1H-pyrrolo[3,2-c]pyridin-2-yl]pyridin-2-yl}-4,4-difluor-2-(4-fluorphenyl)butanamid;
(2RS)-4,4-Difluor-2-(4-fluorphenyl)-N-[4-(5,7,7-trimethyl-4-oxo-3-phenyl-4,5,6,7-tetrahydro-1H-pyrrolo[3,2-c]pyridin-2-yl)pyridin-2-yl]butanamid;
(2R)-4,4-Difluor-2-(4-fluorphenyl)-N-[4-(5,7,7-trimethyl-4-oxo-3-phenyl-4,5,6,7-tetrahydro-1H-pyrrolo[3,2-c]pyridin-2-yl)pyridin-2-yl]butanamid;
(2S)-4,4-Difluor-2-(4-fluorphenyl)-N-[4-(5,7,7-trimethyl-4-oxo-3-phenyl-4,5,6,7-tetrahydro-1H-pyrrolo[3,2-c]pyridin-2-yl)pyridin-2-yl]butanamid;
(2RS)-N-[4-(5-Methyl-4-oxo-3-phenyl-4,5,6,7-tetrahydro-1H-pyrrolo[3,2-c]pyridin-2-yl)pyridin-2-yl]-2-(pyridin-4-yl)propanamid;
(2R)-N-[4-(5-Methyl-4-oxo-3-phenyl-4,5,6,7-tetrahydro-1H-pyrrolo[3,2-c]pyridin-2-yl)pyridin-2-yl]-2-(pyridin-4-yl)propenamid;
(2S)-N-[4-(5-Methyl-4-oxo-3-phenyl-4,5,6,7-tetrahydro-1H-pyrrolo[3,2-c]pyridin-2-yl)pyridin-2-yl]-2-(pyridin-4-yl)propanamid;
(2RS)-N-(4-(5-Cyclopropyl-4-oxo-3-phenyl-4,5,6,7-tetrahydro-1H-pyrrolo[3,2-c]pyridin-2-yl)pyridin-2-yl)-4,4-difluor-2-(4-fluorphenyl)butanamid;
(2R)-N-[4-(5-Cyclopropyl-4-oxo-3-phenyl-4,5-dihydro-1H-pyrrolo[3,2-c]pyridin-2-yl)pyridin-2-yl]-4,4-difluor-2-(4-fluorphenyl)butanamid;
(2S)-N-[4-(5-Cyclopropyl-4-oxo-3-phenyl-4,5-dihydro-1H-pyrrolo[3,2-c]pyridin-2-yl)pyridin-2-yl]-4,4-difluor-2-(4-fluorphenyl)butanamid;
N-[4-(5-Cyclopropyl-4-oxo-3-phenyl-4,5-dihydro-1H-pyrrolo[3,2-c]pyridin-2-yl)pyridin-2-yl]-2-(4-fluorphenyl)acetamid;
(2RS)-N-{4-[5-Cyclopropyl-4-oxo-3-(pyridin-2-yl)-4,5-dihydro-1H-pyrrolo[3,2-c]pyridin-2-yl]pyridin-2-yl}-4,4-difluor-2-(4-fluorphenyl)butanamid;
(2RS)-N-{4-[7-Chlor-5-cyclopropyl-3-(4-fluorphenyl)-4-oxo-4,5-dihydro-1H-pyrrolo[3,2-c]pyridin-2-yl]pyridin-2-yl}-4,4-difluor-2-(4-fluorphenyl)butanamid;
(2RS)-4,4-Difluor-2-(4-fluorphenyl)-N-{4-[5-methyl-4-oxo-3-(pyridin-2-yl)-7-(2,2,2-trifluorethyl)-4,5-dihydro-1H-pyrrolo[3,2-c]pyridin-2-yl]pyridin-2-yl}butanamid;
oder N-Oxid, Salz, Tautomer oder Stereoisomer der Verbindung oder Salz des N-Oxids, Tautomers oder Stereoisomers.

**6.** Verfahren zum Herstellen einer Verbindung der allgemeinen Formel (I) nach einem der Ansprüche 1 bis 5, wobei das Verfahren Umsetzen einer Zwischenverbindung der allgemeinen Formel (1-3):

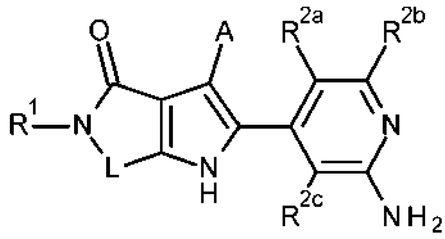

1-3,

worin $R^1$, $R^{2a}$, $R^{2b}$, $R^{2c}$, A und L wie für die Verbindung der allgemeinen Formel (I) nach einem der Ansprüche 1 bis 4 definiert sind,
mit einem Acylierungsreagens ausgewählt aus Folgendem umfasst:

a) einer Carbonsäure der Formel

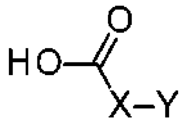

,

b) einem Acylhalid der Formel

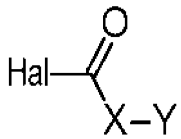

,

wobei Hal F, Cl oder Br darstellt, oder
c) einem Anhydrid der Formel

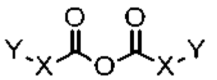
oder
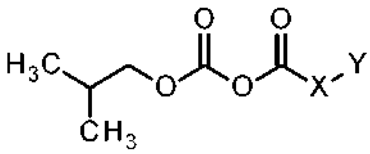
,

worin X und Y wie für die Verbindung der allgemeinen Formel (I) nach einem der Ansprüche 1 bis 4 definiert sind, um eine Verbindung der allgemeinen Formel (I) zu ergeben:

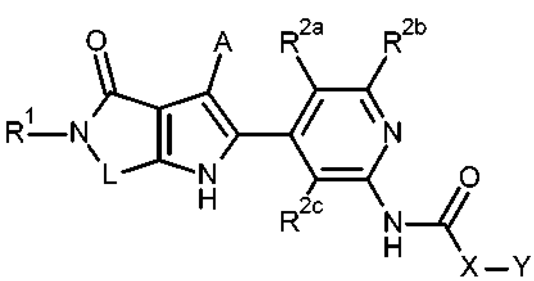

(I),

worin $R^1$, $R^{2a}$, $R^{2b}$, $R^{2c}$, A, L, X und Y wie für die Verbindung der allgemeinen Formel (I) nach einem der Ansprüche 1 bis 4 definiert sind.

7. Verbindung der allgemeinen Formel (I) nach einem der Ansprüche 1 bis 5 zur Verwendung bei der Behandlung oder Prophylaxe einer hyperproliferativen Erkrankung und/oder einer Störung, die auf Induktion von Zelltod anspricht.

8. Verbindung der allgemeinen Formel (I) zur Verwendung nach Anspruch 7, wobei die hyperproliferative Erkrankung und/oder Störung, die auf Induktion von Zelltod anspricht, ein hämatologischer Tumor, solider Tumor und/oder Metastasen davon sind.

9. Verbindung der Formel (I) zur Verwendung nach Anspruch 8, wobei der hämatologische Tumor ein Lymphom und/oder Metastasen davon ist, wobei bevorzugt das Lymphom diffuses großzelliges B-Zell-Lymphom und/oder Metastasen davon ist; der solide Tumor ein Gebärmutterhalstumor, ein Lungentumor, ein Dickdarmtumor und/oder Metastasen davon ist, wobei bevorzugt der Lungentumor ein Lungenkarzinom und/oder Metastasen davon ist und der Dickdarmtumor ein kolorektales Karzinom und/oder Metastasen davon ist.

10. Pharmazeutische Zusammensetzung, umfassend zumindest eine Verbindung der allgemeinen Formel (I) nach einem der Ansprüche 1 bis 5 zusammen mit zumindest einem pharmazeutisch unbedenklichen Hilfsstoff.

11. Kombination, umfassend einen oder mehrere erste Wirkstoffe ausgewählt aus einer Verbindung der allgemeinen Formel (I) nach einem der Ansprüche 1 bis 5 und einen oder mehrere zweite Wirkstoffe ausgewählt aus chemotherapeutischen Antikrebsmitteln und zielspezifischen Antikrebsmitteln.

12. Zwischenverbindung der Formel 1-3, Ia oder 1-9 oder Salz davon:

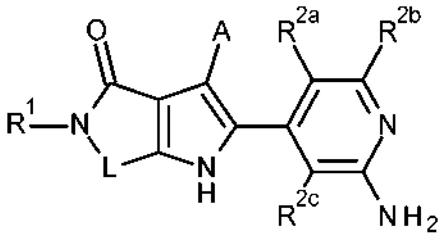

1-3,

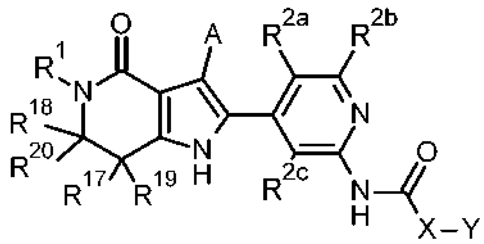

(Ia) ,

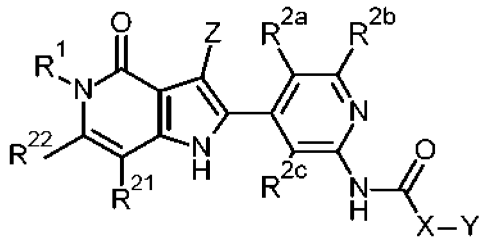

1-9

, ,

wobei $R^1$, $R^{2a}$, $R^{2b}$, $R^{2c}$, $R^{17}$, $R^{18}$, $R^{19}$, $R^{20}$, $R^{21}$, $R^{22}$, A, X, Y und L wie für die Verbindung der allgemeinen Formel (1) nach einem der Ansprüche 1 bis 4 definiert sind und Z Chlor, Brom oder Iod darstellt.

13. Verwendung einer Verbindung der Formel 1-3 oder eines Salzes davon:

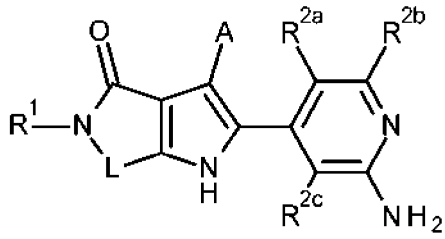

1-3,

worin $R^1$, $R^{2a}$, $R^{2b}$, $R^{2c}$, A und L wie für die Verbindung der allgemeinen Formel (I) nach einem der Ansprüche 1 bis 4 definiert sind, oder

einer Verbindung der allgemeinen Formel (Ia) oder eines Salzes davon:

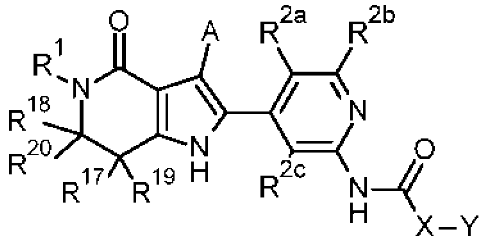

**(Ia)**

,

worin $R^1$, $R^{2a}$, $R^{2b}$, $R^{2c}$, $R^{17}$, $R^{18}$, $R^{19}$, $R^{20}$, A, X und Y wie für die Verbindung der allgemeinen Formel (I) nach einem der Ansprüche 1 bis 4 definiert sind, oder

einer Verbindung der allgemeinen Formel 1-9 oder eines Salzes davon:

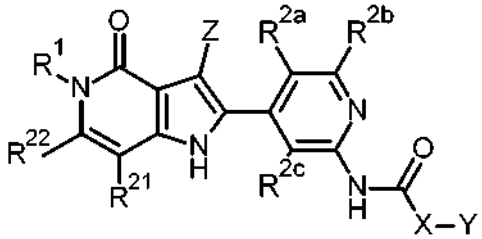

**1-9**

,

worin $R^1$, $R^{2a}$, $R^{2b}$, $R^{2c}$, $R^{21}$, $R^{22}$, X und Y wie für die Verbindung der allgemeinen Formel (I) nach einem der Ansprüche 1 bis 4 definiert sind und Z Chlor, Brom oder Iod darstellt, oder

einer Verbindung der allgemeinen Formel (1-6) oder eines Salzes davon:

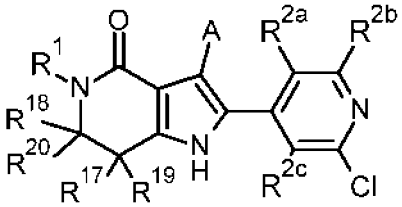

**1-6**

,

worin $R^1$, $R^{2a}$, $R^{2b}$, $R^{2c}$, $R^{17}$, $R^{18}$, $R^{19}$, $R^{20}$ und A wie für die Verbindung der allgemeinen Formel (I) nach einem der Ansprüche 1 bis 4 definiert sind, zur Herstellung einer Verbindung der Formel (I) wie in einem der Ansprüche 1 bis 5 definiert.

**14.** Verfahren zum Herstellen einer Verbindung der allgemeinen Formel (Ib) nach einem der Ansprüche 1 bis 5, wobei das Verfahren Umsetzen einer Zwischenverbindung der allgemeinen Formel (1a):

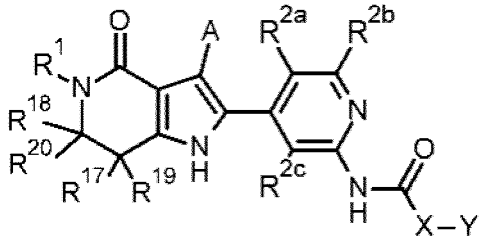

**(Ia)**

,

worin $R^1$, $R^{2a}$, $R^{2b}$, $R^{2c}$, $R^{17}$, $R^{18}$, $R^{19}$, $R^{20}$, A, X und Y wie für die Verbindung der allgemeinen Formel (I) nach einem der Ansprüche 1 bis 4 definiert sind, mit einem Oxidationsmittel umfasst, um eine Verbindung der allgemeinen Formel (Ib) zu ergeben:

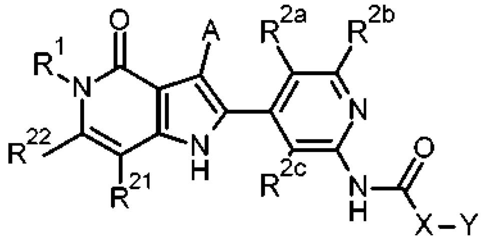

**(Ib)**

.

worin $R^1$, $R^{2a}$, $R^{2b}$, $R^{2c}$, $R^{21}$, $R^{22}$, A, X und Y wie für die Verbindung der allgemeinen Formel (I) nach einem der Ansprüche 1 bis 4 definiert sind; oder wobei das Verfahren Umsetzen einer Zwischenverbindung der allgemeinen Formel (1-9):

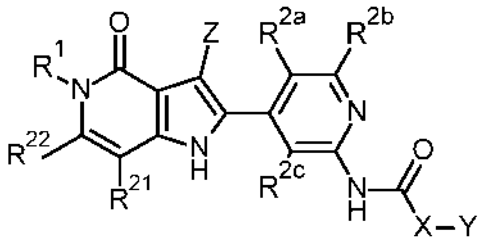

**1-9**

,

worin $R^1$, $R^{2a}$, $R^{2b}$, $R^{2c}$, $R^{21}$, $R^{22}$, X und Y wie für die Verbindung der allgemeinen Formel (I) nach einem der Ansprüche 1 bis 4 definiert sind und Z Chlor, Brom oder Iod darstellt, mit einem Reagens F umfasst:

$Z^2$-A **Reagens** F,

worin A wie für die Verbindung der allgemeinen Formel (I) nach einem der Ansprüche 1 bis 4 definiert ist und $Z^2$ ein Stannan wie etwa A-Sn(nBu)$_3$, Boronsäure-A-B(OH)$_2$ oder einen Boronsäureester davon darstellt, unter Verwendung einer Metall-katalysierten Reaktion, wie zum Beispiel einer Suzuki-Reaktion, um eine Verbindung der allgemeinen Formel (Ib) zu ergeben:

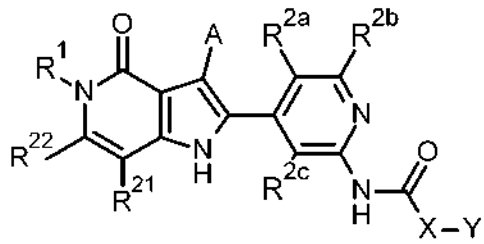

**(Ib)**

,

worin $R^1$, $R^{2a}$, $R^{2b}$, $R^{2c}$, $R^{21}$, $R^{22}$, A, X und Y wie für die Verbindung der allgemeinen Formel (I) nach einem der Ansprüche 1 bis 4 definiert sind.

**15.** Verfahren zum Herstellen einer Verbindung der allgemeinen Formel (Ia) nach einem der Ansprüche 1 bis 5, wobei das Verfahren Umsetzen einer Zwischenverbindung der allgemeinen Formel (1-8):

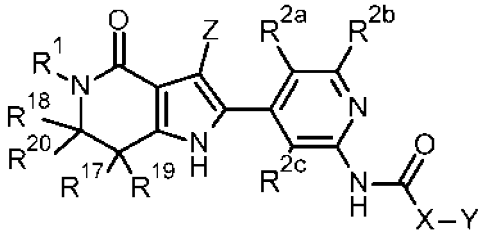

**1-8**

,

worin $R^1$, $R^{2a}$, $R^{2b}$, $R^{2c}$, $R^{17}$, $R^{18}$, $R^{19}$, $R^{20}$, X und Y wie für die Verbindung der allgemeinen Formel (I) nach einem der Ansprüche 1 bis 4 definiert sind und Z Chlor, Brom oder Iod darstellt, mit einem Reagens F umfasst:

$Z^2$-A **Reagens F,**

worin A wie für die Verbindung der allgemeinen Formel (I) nach einem der Ansprüche 1 bis 4 definiert ist und $Z^2$ ein Stannan wie etwa A-Sn(nBu)$_3$, Boronsäure-A-B(OH)$_2$ oder einen Boronsäureester davon darstellt, unter Verwendung einer Metall-katalysierten Reaktion, wie zum Beispiel einer Suzuki-Reaktion, um eine Verbindung der allgemeinen Formel (Ia) zu ergeben:

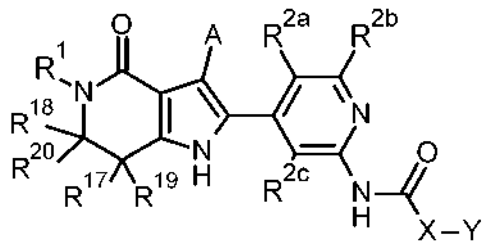

**(Ia)**

worin $R^1$, $R^{2a}$, $R^{2b}$, $R^{2c}$, $R^{17}$, $R^{18}$, $R^{19}$, $R^{20}$, A, X und Y wie für die Verbindung der allgemeinen Formel (I) nach einem der Ansprüche 1 bis 4 definiert sind, oder wobei das Verfahren Umsetzen einer Zwischenverbindung der allgemeinen Formel (1-6):

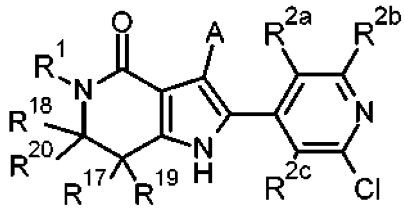

**1-6**

,

worin $R^1$, $R^{2a}$, $R^{2b}$, $R^{2c}$, $R^{17}$, $R^{18}$, $R^{19}$, $R^{20}$ und A wie für die Verbindung der allgemeinen Formel (I) nach einem der Ansprüche 1 bis 4 definiert sind, mit einem Reagens G umfasst:

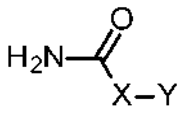

**Reagens G,**

wobei X und Y wie für die Verbindung der allgemeinen Formel (I) nach einem der Ansprüche 1 bis 4 definiert sind, unter Verwendung einer Metall-katalysierten Reaktion, wie zum Beispiel einer Palladium-katalysierten Reaktion in Gegenwart eines Liganden, wie zum Beispiel Xanthphos oder X-Phos, um eine Verbindung der allgemeinen Formel (Ia) zu ergeben:

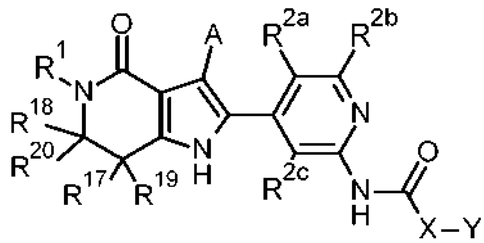

**(Ia)**

,

worin $R^1$, $R^{2a}$, $R^{2b}$, $R^{2c}$, $R^{17}$, $R^{18}$, $R^{19}$, $R^{20}$, A, X und Y wie für die Verbindung der allgemeinen Formel (I) nach einem der Ansprüche 1 bis 4 definiert sind.

## Revendications

1. Composé de formule (I)

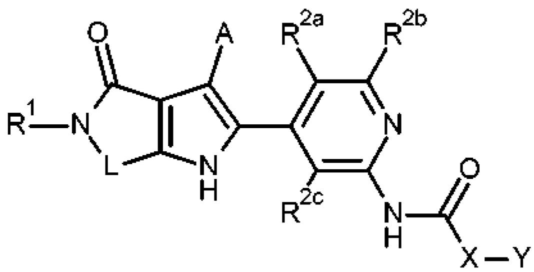

**(I)**

dans lequel :

$R^1$ représente hydrogène ou un groupe sélectionné parmi $C_1$-$C_4$-alkyle, $C_3$-$C_6$-cycloalkyle, $C_3$-$C_6$-cycloalkyl-$C_1$-$C_4$-alkyle-, $C_1$-$C_3$-alkoxy-$C_1$-$C_3$-alkyle-, et $C_1$-$C_5$-hydroxyalkyle, dans lequel lesdits groupes sont chacun indépendamment éventuellement substitués, une ou plusieurs fois, par halogène ;
$R^{2a}$ représente hydrogène, hydroxy, halogène, cyano, $-NH_2$, -NHMe, $-NMe_2$, $C_1$-$C_2$-alkoxy, $C_1$-$C_2$-haloalkyle ou $C_1$-$C_2$-haloalkoxy ;
$R^{2b}$ représente hydrogène, hydroxy, halogène, cyano, $-NH_2$, $-NHR^9$ ou $-NMe_2$ ;
$R^{2c}$ représente hydrogène, halogène, cyano, $-NMe_2$, $C_1$-$C_2$-alkoxy, ou $C_1$-$C_2$-haloalkoxy ;
L représente

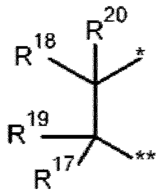

ou

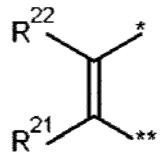

où * représente le point de fixation directe à l'atome N adjacent et ** représente le point de fixation directe à l'atome C adjacent ;
$R^{17}$ représente hydrogène, $C_1$-$C_3$-alkyle, $C_1$-$C_3$-alkoxy, $C_1$-$C_3$-haloalkyle ou $C_3$-$C_6$-cycloalkyl-$C_1$-$C_4$-alkyle- ;
$R^{18}$ représente hydrogène, $C_1$-$C_3$-alkyle, $C_1$-$C_3$-haloalkyle, ou $C_3$-$C_6$-cycloalkyl-$C_1$-$C_4$-alkyle-, ou
$R^{17}$ et $R^{18}$, avec les atomes de carbone auxquels ils sont fixés, forment un anneau $C_3$-$C_6$-cycloalkyle ;
$R^{19}$ représente hydrogène, $C_1$-$C_3$-alkyle, $C_1$-$C_3$-haloalkyle ou $C_3$-$C_6$-cycloalkyl-$C_1$-$C_4$-alkyle- ;
$R^{20}$ représente hydrogène, $C_1$-$C_3$-alkyle, $C_1$-$C_3$-alkoxy, $C_1$-$C_3$-haloalkyle, ou $C_3$-$C_6$-cycloalkyl-$C_1$-$C_4$-alkyle-, ou
$R^{18}$ et $R^{20}$, avec l'atome de carbone auquel ils sont fixés, forment un anneau $C_3$-$C_6$-cycloalkyle ;
$R^{21}$ représente hydrogène, halogène, $C_1$-$C_3$-alkyle, $C_1$-$C_3$-haloalkyle ou $C_3$-$C_6$-cycloalkyl-$C_1$-$C_4$-alkyle- ;
$R^{22}$ représente hydrogène, $C_1$-$C_3$-alkyle, $C_1$-$C_3$-haloalkyle ou $C_3$-$C_6$-cycloalkyl-$C_1$-$C_4$-alkyle- ;
X représente méthylène ou éthylène,
où lesdits groupes méthylène et éthylène sont chacun indépendamment éventuellement substitués, une ou plusieurs fois, par $R^3$ ;
Y représente phényle ou un groupe hétéroaryle,
dans lequel lesdits groupes phényle et hétéroaryle sont chacun indépendamment éventuellement substitués, une ou plusieurs fois, par $R^4$ ;
$R^3$ représente hydroxy, halogène, cyano, $C_1$-$C_3$-alkyle, $C_1$-$C_3$-alkoxy, $C_1$-$C_3$-haloalkyle, $C_1$-$C_2$-haloalkoxy, $C_3$-$C_4$-cycloalkyle, $-NHR^6$, $R^5O$-$C_1$-$C_3$-alkyle, $R^6R^7N$-$C_1$-$C_5$-alkyle ou $R^6R^7N$-X'-$C(R^{10})(R^{11})$-$C_1$-$C_2$-alkyle- ;
X' représente méthylène ou éthylène ;
$R^4$ représente hydroxy, halogène, cyano, $C_1$-$C_4$-alkyle, $C_1$-$C_4$-alkoxy, $C_1$-$C_4$-haloalkyle, $C_1$-$C_4$-haloalkoxy, $-NMe_2$, $-CO_2R^{14}$, $-CONR^{15}R^{16}$, ou $-SO_2Me$ ;
$R^5$ représente hydrogène, $C_1$-$C_2$-alkyle, $C_1$-$C_2$-haloalkyle ou $C_3$-$C_4$-cycloalkyle ;
$R^6$ représente hydrogène, $C_1$-$C_2$-alkyle, $C_1$-$C_2$-haloalkyle ou $C_3$-$C_4$-cycloalkyle ;
$R^7$ représente hydrogène, $C_1$-$C_2$-alkyle, $C_1$-$C_2$-haloalkyle, $C_3$-$C_4$-cycloalkyle ou $-CO_2$-$C_1$-$C_4$-alkyle, ou
$R^6$ et $R^7$, avec l'atome d'azote auquel ils sont fixés, représentent un anneau hétérocyclique à 4 à 7 chaînons, ledit anneau hétérocyclique contenant éventuellement un ou plusieurs autres hétéroatomes sélectionnés parmi N, O, S, ou des groupes sélectionnés parmi C(=O), S(=O) et $SO_2$, et est indépendamment éventuellement substitué, une ou plusieurs fois, par halogène, $C_1$-$C_3$-alkyle, $C_3$-cycloalkyl-$C_1$-$C_2$-alkyle-, $-NH_2$, $-NHR^9$, $-NR^9R^{9a}$, ou hydroxy ;
A représente un groupe sélectionné parmi :

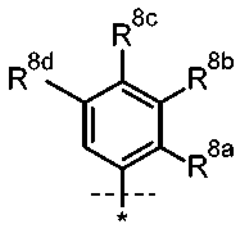 , 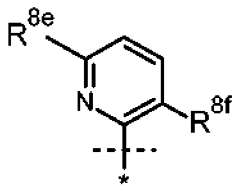 , 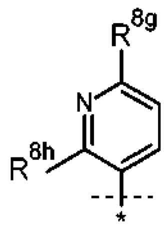 , 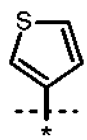 ,

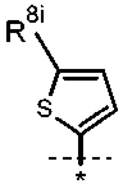 , 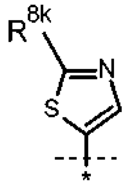 , 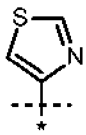 , 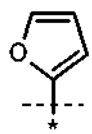 , 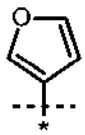 et 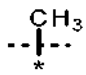 ,

dans lequel * indique le point de fixation dudit groupe au reste de la formule (I) ;
$R^{8a}$ représente hydrogène, hydroxy, halogène, cyano, $C_1$-$C_2$-alkyle, ou $C_1$-$C_2$-haloalkyle ;
$R^{8b}$ représente hydrogène, hydroxy, halogène, cyano, $C_1$-$C_2$-alkyle, ou $C_1$-$C_2$-haloalkyle ;
$R^{8c}$ représente hydrogène, hydroxy, halogène, $C_1$-$C_2$-alkyle, ou $C_1$-$C_2$-haloalkyle ;
$R^{8d}$ représente hydrogène, hydroxy, halogène, cyano, $C_1$-$C_2$-alkyle, ou $C_1$-$C_2$-haloalkyle ;
$R^{8e}$ représente hydrogène, hydroxy, halogène, cyano, $C_1$-$C_2$-alkyle, ou $C_1$-$C_2$-haloalkyle ;
$R^{8f}$ représente hydrogène, hydroxy, halogène, cyano, $C_1$-$C_2$-alkyle, ou $C_1$-$C_2$-haloalkyle ;
$R^{8g}$ représente hydrogène, hydroxy, amino, halogène, cyano, $C_1$-$C_2$-alkyle, ou $C_1$-$C_2$-haloalkyle ;
$R^{8h}$ représente hydrogène, hydroxy, halogène, cyano, $C_1$-$C_2$-alkyle, ou $C_1$-$C_2$-haloalkyle ;
$R^{8i}$ représente hydrogène, hydroxy, halogène, cyano, $C_1$-$C_2$-alkyle, ou $C_1$-$C_2$-haloalkyle ;
$R^{8k}$ représente hydrogène, hydroxy, halogène, cyano, $C_1$-$C_2$-alkyle, ou $C_1$-$C_2$-haloalkyle ;
$R^9$ représente $C_1$-$C_3$-alkyle ou cyclopropyle ;
$R^{9a}$ représente $C_1$-$C_3$-alkyle ou cyclopropyle ;
$R^{10}$ représente méthyle, halogène ou hydroxy ;
$R^{11}$ représente méthyle, halogène ou hydroxy ;
$R^{14}$ représente hydrogène, méthyle ou éthyle ;
$R^{15}$ représente hydrogène, méthyle ou éthyle ;
$R^{16}$ représente hydrogène, méthyle, éthyle, méthoxyéthyle ou diméthylaminoéthyle, ou
$R^{15}$ et $R^{16}$, avec l'atome d'azote auquel ils sont fixés, représentent un anneau hétérocyclique à 5 à 6 chaînons, ledit anneau hétérocyclique contenant éventuellement un ou deux autres hétéroatomes sélectionnés parmi N, O, S, ou des groupes sélectionnés parmi C(=O), S(=O), et $SO_2$ ;
ou un N-oxyde, un sel, un tautomère ou un stéréoisomère dudit composé, ou un sel dudit N-oxyde, tautomère ou stéréoisomère.

**2.** Composé de formule (I) selon la revendication 1, dans lequel :

$R^1$ représente hydrogène ou un groupe sélectionné entre $C_1$-$C_2$-alkyle et $C_3$-$C_4$-cycloalkyle, lesdits groupes étant chacun indépendamment éventuellement substitués, une ou plusieurs fois, par halogène ;
$R^{2a}$ représente hydrogène ou halogène ;
$R^{2b}$ représente hydrogène, halogène, $-NH_2$, ou $-NHR^9$ ;
$R^{2c}$ représente hydrogène ou halogène ;
L représente

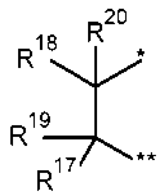

ou

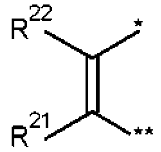

dans lequel * représente le point de fixation directe à l'atome N adjacent et ** représente le point de fixation directe à l'atome C adjacent ;
$R^{17}$ représente hydrogène, $C_1$-$C_3$-alkyle, $C_1$-$C_3$-haloalkyle, ou $C_3$-$C_6$-cycloalkyl-$C_1$-$C_4$-alkyle- ;
$R^{18}$ représente hydrogène, $C_1$-$C_3$-alkyle, $C_1$-$C_3$-haloalkyle ou $C_3$-cycloalkyl-$C_1$-alkyle-, ou
$R^{17}$ et $R^{18}$, avec les atomes de carbone auxquels ils sont fixés, forment un anneau $C_3$-$C_5$-cycloalkyle ;
$R^{19}$ représente hydrogène, $C_1$-$C_3$-alkyle, $C_1$-$C_3$-haloalkyle, ou $C_3$-$C_6$-cycloalkyl-$C_1$-$C_4$-alkyle- ;
$R^{20}$ représente hydrogène ou $C_1$-$C_3$-alkyle, ou
$R^{18}$ et $R^{20}$, avec les atomes de carbone auxquels ils sont fixés, forment un anneau $C_3$-$C_6$-cycloalkyle ;
$R^{21}$ représente hydrogène, halogène, $C_1$-$C_3$-alkyle, $C_1$-$C_3$-haloalkyle, ou $C_3$-$C_6$-cycloalkyl-$C_1$-$C_4$-alkyle- ;
$R^{22}$ représente hydrogène ou $C_1$-$C_3$-alkyle ;
X représente méthylène,
dans lequel ledit groupe méthylène est éventuellement substitué, une ou plusieurs fois, par R 3;
Y représente phényle ou un groupe hétéroaryle,
lesdits groupes phényle et hétéroaryle étant chacun indépendamment éventuellement substitués, une ou plusieurs fois, par $R^4$ ;
$R^3$ représente $C_1$-$C_3$-alkyle, $C_1$-$C_3$-haloalkyle, -$NHR^6$, $R^5O$-$C_1$-$C_3$-alkyle, ou $R^6R^7N$-$C_1$-$C_4$-alkyle ;
$R^4$ représente halogène, $C_1$-$C_4$-alkyle, $C_1$-$C_4$-haloalkyle, $C_1$-$C_4$-haloalkoxy, -$CO_2R^{14}$, - $CONR^{15}R^{16}$ ou -$SO_2Me$ ;
$R^5$ représente hydrogène, $C_1$-$C_2$-alkyle, ou $C_1$-$C_2$-haloalkyle ;
$R^6$ représente hydrogène, $C_1$-$C_2$-alkyle, $C_1$-$C_2$-haloalkyle, ou $C_3$-$C_4$-cycloalkyle ;
$R^7$ représente hydrogène, $C_1$-$C_2$-alkyle, $C_1$-$C_2$-haloalkyle, $C_3$-$C_4$-cycloalkyle, ou -$CO_2$-$C_1$-$C_4$-alkyle, ou
$R^6$ et $R^7$, avec l'atome d'azote auquel ils sont fixés, représentent un anneau hétérocyclique à 4 à 7 chaînons, ledit anneau hétérocyclique contenant éventuellement un ou plusieurs autres hétéroatomes sélectionnés parmi N, O, S, et étant indépendamment éventuellement substitué, une ou plusieurs fois, par halogène, $C_1$-$C_3$-alkyle, $C_3$-cycloalkyl-$C_1$-$C_2$-alkyle-, -$NH_2$, -$NHR^9$, - $NR^9R^{9a}$, ou hydroxy ;
A représente un groupe sélectionné parmi :

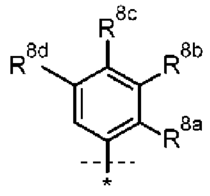
,
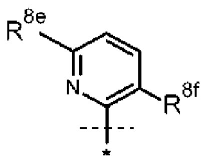
,
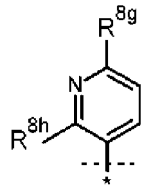
,
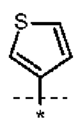
,

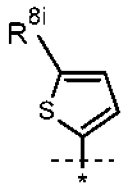 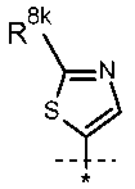 , 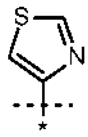 , 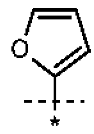 , 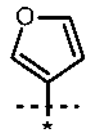 , et 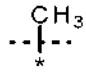 ,

où * indique le point de fixation dudit groupe au reste de la formule (I) ;
$R^{8a}$ représente hydrogène, hydroxy, halogène, cyano ou $C_1$-alkyle ;
$R^{8b}$ représente hydrogène, hydroxy, halogène ou $C_1$-alkyle ;
$R^{8c}$ représente hydrogène, hydroxy, halogène ou $C_1$-alkyle ;
$R^{8d}$ représente hydrogène, hydroxy ou halogène ;
$R^{8e}$ représente hydrogène ;
$R^{8f}$ représente hydrogène ;
$R^{8g}$ représente hydrogène, amino, ou halogène ;
$R^{8h}$ représente hydrogène ou halogène ;
$R^{8i}$ représente hydrogène, halogène, ou $C_1$-$C_2$-alkyle ;
$R^{8k}$ représente hydrogène ou $C_1$-alkyle ;
$R^9$ représente $C_1$-$C_3$-alkyle ou cyclopropyle ;
$R^{9a}$ représente $C_1$-$C_3$-alkyle ou cyclopropyle ;
$R^{14}$ représente hydrogène, méthyle, ou éthyle ;
$R^{15}$ représente hydrogène, méthyle, ou éthyle ;
$R^{16}$ représente hydrogène, méthyle, éthyle, méthoxyéthyle, ou diméthylaminoéthyle, ou
$R^{15}$ et $R^{16}$, avec l'atome d'azote auquel ils sont fixés, représentent un anneau hétérocyclique à 5 à 6 chaînons, ledit anneau hétérocyclique contenant éventuellement un ou deux autres hétéroatomes sélectionnés entre N et O ;
ou un N-oxyde, un sel, un tautomère ou un stéréoisomère dudit composé, ou un sel dudit N-oxyde, tautomère ou stéréoisomère.

**3.** Composé de formule (I) selon la revendication 1 ou 2, dans lequel :

$R^1$ représente hydrogène ou un groupe sélectionné entre $C_1$-$C_2$-alkyle et $C_3$-$C_4$-cycloalkyle ;
$R^{2a}$ représente hydrogène ou halogène ;
$R^{2b}$ représente hydrogène ;
$R^{2c}$ représente hydrogène ou halogène ;
L représente

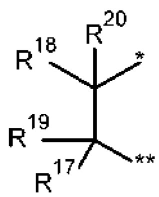

ou

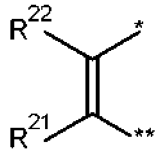

, dans lequel * représente le point de fixation directe à l'atome N adjacent et ** représente le point de fixation directe à l'atome C adjacent ;
$R^{17}$ représente hydrogène, $C_1$-$C_3$-alkyle, $C_1$-$C_3$-haloalkyle, ou $C_3$-$C_4$-cycloalkyl-$C_1$-alkyle- ;

$R^{18}$ représente hydrogène ou $C_1$-$C_3$-alkyle ;
$R^{19}$ représente hydrogène, $C_1$-$C_3$-alkyle, $C_1$-$C_3$-haloalkyle, ou $C_3$-$C_4$-cycloalkyl-$C_1$-alkyle- ;
$R^{20}$ représente hydrogène ou $C_1$-$C_3$-alkyle, ou
$R^{18}$ et $R^{20}$, avec l'atome de carbone auquel ils sont fixés, forment un anneau $C_3$-$C_6$-cycloalkyle ;
$R^{21}$ représente hydrogène, halogène, $C_1$-$C_3$-alkyle, $C_1$-$C_3$-haloalkyle, ou $C_3$-$C_4$-cycloalkyl-$C_1$-alkyle- ;
$R^{22}$ représente hydrogène ;
X représente méthylène,
ledit groupe méthylène étant éventuellement substitué, une ou deux fois, par $R^3$ ;
Y représente phényle,
ledit phényle étant éventuellement substitué, une ou plusieurs fois, par $R^4$ ;
$R^3$ représente $C_1$-$C_3$-alkyle, $C_1$-$C_3$-haloalkyle, ou $R^5O$-$C_1$-$C_3$-alkyle ;
$R^4$ représente halogène, $C_1$-$C_4$-alkyle, $C_1$-$C_4$-haloalkyle, -$CONR^{15}R^{16}$, ou -$SO_2Me$ ;
$R^5$ représente hydrogène ;
A représente un groupe sélectionné parmi :

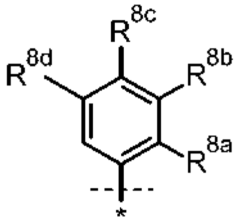 , 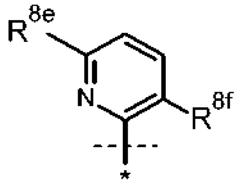 , 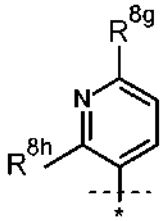 , 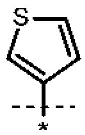 ,

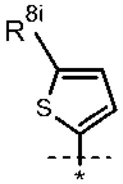 , 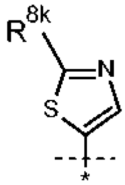 , 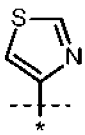 , 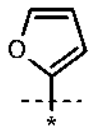 , 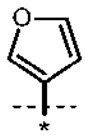 et 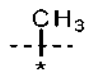 ,

dans lequel * indique le point de fixation dudit groupe au reste de la formule (I) ;
$R^{8a}$ représente hydrogène, hydroxy, halogène ou cyano ;
$R^{8b}$ représente hydrogène, halogène ou $C_1$-alkyle ;
$R^{8c}$ représente hydrogène, halogène ou $C_1$-alkyle ;
$R^{8d}$ représente hydrogène ou halogène ;
$R^{8e}$ représente hydrogène ;
$R^{8f}$ représente hydrogène ;
$R^{8g}$ représente hydrogène ou halogène ;
$R^{8h}$ représente hydrogène ;
$R^{8i}$ représente hydrogène, halogène ou $C_1$-$C_2$-alkyle ;
$R^{8k}$ représente hydrogène ou $C_1$-alkyle ;
$R^{15}$ représente hydrogène ou méthyle ;
$R^{16}$ représente méthyle ou méthoxyéthyle, ou
$R^{15}$ et $R^{16}$, avec l'atome d'azote auquel ils sont fixés, représentent un anneau hétérocyclique à 5 à 6 chaînons, ledit anneau hétérocyclique contenant éventuellement un autre hétéroatome sélectionné entre N et O ;
ou un N-oxyde, un sel, un tautomère ou un stéréoisomère dudit composé, ou un sel dudit N-oxyde, tautomère ou stéréoisomère.

4. Composé de formule (I) selon la revendication 1, 2 ou 3, dans lequel :

$R^1$ représente hydrogène ou un groupe sélectionné entre $C_1$-alkyle et $C_3$-cycloalkyle ;
$R^{2a}$ représente hydrogène ;
$R^{2b}$ représente hydrogène ;
$R^{2c}$ représente hydrogène ;

L représente

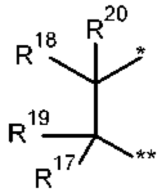

ou

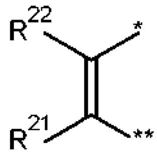

,

dans lequel * représente le point de fixation directe à l'atome N adjacent et ** représente le point de fixation directe à l'atome C adjacent ;

$R^{17}$ représente hydrogène ou $C_1$-alkyle ;

$R^{18}$ représente hydrogène ou $C_1$-alkyle ;

$R^{19}$ représente hydrogène, $C_1$-$C_3$-alkyle, $C_1$-$C_3$-haloalkyle, ou $C_3$-cycloalkyl-$C_1$-alkyle- ;

$R^{20}$ représente hydrogène ou $C_1$-alkyle, ou

$R^{18}$ et $R^{20}$, avec l'atome de carbone auquel ils sont fixés, forment un anneau $C_3$-$C_4$-cycloalkyle ;

$R^{21}$ représente hydrogène, $C_1$-$C_3$-alkyle, $C_1$-$C_3$-haloalkyle ou $C_3$-cycloalkyl-$C_1$-alkyle- ;

$R^{22}$ représente hydrogène ;

X représente méthylène,

ledit groupe méthylène étant éventuellement substitué une fois par $R^3$ ;

Y représente phényle,

ledit phényle étant éventuellement substitué une fois par $R^4$ ;

$R^3$ représente $C_1$-$C_3$-alkyle ou $C_1$-$C_2$-haloalkyle ;

$R^4$ représente halogène ou -CON$R^{15}R^{16}$ ;

A représente un groupe sélectionné entre :

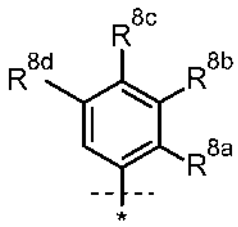

,

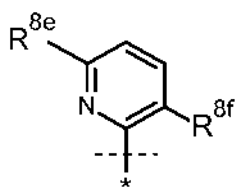

,

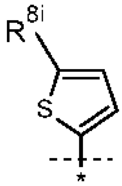

,

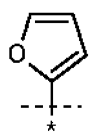

et

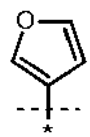

,

où * indique le point de fixation dudit groupe au reste de la formule (I) ;

$R^{8a}$ représente hydrogène ou fluoro ;

$R^{8b}$ représente hydrogène ou fluoro ;
$R^{8c}$ représente hydrogène, fluoro, ou chloro ;
$R^{8d}$ représente hydrogène ;
$R^{8e}$ représente hydrogène ;
$R^{8f}$ représente hydrogène ;
$R^{8i}$ représente hydrogène, chloro, ou $C_1$-alkyle ;
$R^{15}$ représente hydrogène ;
$R^{16}$ représente méthoxyéthyle, ou
$R^{15}$ et $R^{16}$, avec l'atome d'azote auquel ils sont fixés, représentent un anneau hétérocyclique à 5 à 6 chaînons, ledit anneau hétérocyclique contenant éventuellement un autre hétéroatome N ;
ou un N-oxyde, un sel, un tautomère ou un stéréoisomère dudit composé, ou un sel dudit N-oxyde, tautomère ou stéréoisomère.

**5.** Composé de formule (I) selon l'une quelconque des revendications 1 à 4, qui est sélectionné parmi :

2-(4-fluorophényl)-N-[4-(5-méthyl-4-oxo-3-phényl-4,5,6,7-tétrahydro-1H-pyrrolo[3,2-c]pyridin-2-yl)pyridin-2-yl]acétamide ;
4,4-difluoro-2-(4-fluorophényl)-N-[4-(5-méthyl-4-oxo-3-phényl-4,5,6,7-tétrahydro-1H-pyrrolo[3,2-c]pyridin-2-yl)pyridin-2-yl]butanamide ;
(2R)-4,4-difluoro-2-(4-fluorophényl)-N-[4-(5-méthyl-4-oxo-3-phényl-4,5,6,7-tétrahydro-1H-pyrrolo[3,2-c]pyridin-2-yl)pyridin-2-yl]butanamide ;
(2S)-4,4-difluoro-2-(4-fluorophényl)-N-[4-(5-méthyl-4-oxo-3-phényl-4,5,6,7-tétrahydro-1H-pyrrolo[3,2-c]pyridin-2-yl)pyridin-2-yl]butanamide ;
2-(4-fluorophényl)-N-[4-(5-méthyl-4-oxo-3-phényl-4,5-dihydro-1H-pyrrolo[3,2-c]pyridin-2-yl)pyridin-2-yl]acétamide ;
4,4-difluoro-2-(4-fluorophényl)-N-[4-(5-méthyl-4-oxo-3-phényl-4,5-dihydro-1H-pyrrolo[3,2-c]pyridin-2-yl)pyridin-2-yl]butanamide ;
(2R)-4,4-difluoro-2-(4-fluorophényl)-N-[4-(5-méthyl-4-oxo-3-phényl-4,5-dihydro-1H-pyrrolo[3,2-c]pyridin-2-yl)pyridin-2-yl]butanamide ;
(2S)-4,4-difluoro-2-(4-fluorophényl)-N-[4-(5-méthyl-4-oxo-3-phényl-4,5-dihydro-1H-pyrrolo[3,2-c]pyridin-2-yl)pyridin-2-yl]butanamide ;
4,4-difluoro-2-(4-fluorophényl)-N- {4-[5-méthyl-4-oxo-3-(pyridin-3-yl)-4,5,6,7-tétrahydro-1H-pyrrolo[3,2-c]pyridin-2-yl]pyridin-2-yl}butanamide ;
(2R)-4,4-difluoro-2-(4-fluorophényl)-N-{4-[5-méthyl-4-oxo-3-(pyridin-3-yl)-4,5,6,7-tétrahydro-1H-pyrrolo[3,2-c]pyridin-2-yl]pyridin-2-yl}butanamide ;
(2S)-4,4-difluoro-2-(4-fluorophényl)-N- {4-[5-méthyl-4-oxo-3-(pyridin-3-yl)-4,5,6,7-tétrahydro-1H-pyrrolo[3,2-c]pyridin-2-yl]pyridin-2-yl}butanamide ;
2-(4-fluorophényl)-N-{4-[5-méthyl-4-oxo-3-(pyridin-3-yl)-4,5,6,7-tétrahydro-1H-pyrrolo[3,2-c]pyridin-2-yl]pyridin-2-yl}acétamide ;
2-(4-fluorophényl)-N- {4-[5-méthyl-4-oxo-3-phényl-7-(2,2,2-trifluoroéthyl)-4,5,6,7-tétrahydro-1H-pyrrolo[3,2-c]pyridin-2-yl]pyridin-2-yl}propanamide ;
2-(4-fluorophényl)-N- {4-[5-méthyl-4-oxo-3-phényl-7-(2,2,2-trifluoroéthyl)-4,5-dihydro-1H-pyrrolo [3,2-c]pyridin-2-yl]pyridin-2-yl} propanamide ;
(2R)-2-(4-fluorophényl)-N- {4-[5-méthyl-4-oxo-3-phényl-7-(2,2,2-trifluoroéthyl)-4,5-dihydro-1H-pyrrolo[3,2-c]pyridin-2-yl]pyridin-2-yl}propanamide ;
(2S)-2-(4-fluorophényl)-N-{4-[5-méthyl-4-oxo-3-phényl-7-(2,2,2-trifluoroéthyl)-4,5-dihydro-1H-pyrrolo[3,2-c]pyridin-2-yl]pyridin-2-yl}propanamide ;
4,4-difluoro-2-(4-fluorophényl)-N- {4-[5-méthyl-4-oxo-3-phényl-7-(2,2,2-trifluoroéthyl)-4,5,6,7-tétrahydro-1H-pyrrolo[3,2-c]pyridin-2-yl]pyridin-2-yl}butanamide ;
4,4-difluoro-2-(4-fluorophényl)-N- {4-[5-méthyl-4-oxo-3-phényl-7-(2,2,2-trifluoroéthyl)-4,5-dihydro-1H-pyrrolo[3,2-c]pyridin-2-yl]pyridin-2-yl}butanamide ;
(2R)-4,4-difluoro-2-(4-fluorophényl)-N-{4-[5-méthyl-4-oxo-3-phényl-7-(2,2,2-trifluoroéthyl)-4,5-dihydro-1H-pyrrolo[3,2-c]pyridin-2-yl]pyridin-2-yl}butanamide ;
(2S)-4,4-difluoro-2-(4-fluorophényl)-N-{4-[5-méthyl-4-oxo-3-phényl-7-(2,2,2-trifluoroéthyl)-4,5-dihydro-1H-pyrrolo[3,2-c]pyridin-2-yl]pyridin-2-yl}butanamide ;
2-(4-fluorophényl)-N- {4-[3-(4-fluorophényl)-5-méthyl-4-oxo-7-(2,2,2-trifluoroéthyl)-4,5,6,7-tétrahydro-1H-pyrrolo[3,2-c]pyridin-2-yl]pyridin-2-yl}propanamide ;
2-(4-fluorophényl)-N- {4-[3-(4-fluorophényl)-5-méthyl-4-oxo-7-(2,2,2-trifluoroéthyl)-4,5-dihydro-1H-pyrrolo[3,2-c]pyridin-2-yl]pyridin-2-yl}propanamide ;
(2R)-2-(4-fluorophényl)-N-{4-[3-(4-fluorophényl)-5-méthyl-4-oxo-7-(2,2,2-trifluoroéthyl)-4,5-dihydro-1H-pyrrolo[3,2-c]pyridin-2-yl]pyridin-2-yl}propanamide ;
(2S)-2-(4-fluorophényl)-N- {4-[3-(4-fluorophényl)-5-méthyl-4-oxo-7-(2,2,2-trifluoroéthyl)-4,5-dihydro-1H-pyrrolo[3,2-c]pyridin-2-yl]pyridin-2-yl}propanamide ;
4,4-difluoro-2-(4-fluorophényl)-N- {4-[3-(4-fluorophényl)-5-méthyl-4-oxo-7-(2,2,2-trifluoroéthyl)-4,5,6,7-tétrahydro-1H-pyrrolo[3,2-c]pyridin-2-yl]pyridin-2-yl}butanamide ;
4,4-difluoro-2-(4-fluorophényl)-N- {4-[3-(4-fluorophényl)-5-méthyl-4-oxo-7-(2,2,2-trifluoroéthyl)-4,5-dihydro-1H-pyrrolo[3,2-c]pyridin-2-yl]pyridin-2-yl}butanamide ;
(2R)-4,4-difluoro-2-(4-fluorophényl)-N- {4-[3-(4-fluorophényl)-5-méthyl-4-oxo-7-(2,2,2-trifluoroéthyl)-4,5-dihydro-1H-pyrrolo[3,2-c]pyridin-2-yl]pyridin-2-yl}butanamide ;
(2S)-4,4-difluoro-2-(4-fluorophényl)-N- {4-[3-(4-fluorophényl)-5-méthyl-4-oxo-7-(2,2,2-trifluoroéthyl)-4,5-dihydro-1H-pyrrolo[3,2-c]pyridin-2-yl]pyridin-2-yl}butanamide ;
2-(4-fluorophényl)-N-{4-[3-(4-fluorophényl)-5,7-diméthyl-4-oxo-4,5,6,7-tétrahydro-1H-pyrrolo[3,2-c]pyridin-2-yl]pyridin-2-yl}propanamide ;
2-(4-fluorophényl)-N-{4-[3-(4-fluorophényl)-5,7-diméthyl-4-oxo-4,5-dihydro-1H-pyrrolo[3,2-c]pyridin-2-yl]pyridin-2-yl}propanamide ;
(2R)-2-(4-fluorophényl)-N-{4-[3-(4-fluorophényl)-5,7-diméthyl-4-oxo-4,5-dihydro-1H-pyrrolo[3,2-c]pyridin-2-yl]pyridin-2-yl}propanamide ;
(2S)-2-(4-fluorophényl)-N-{4-[3-(4-fluorophényl)-5,7-diméthyl-4-oxo-4,5-dihydro-1H-pyrrolo[3,2-c]pyridin-2-yl]pyridin-2-yl}propanamide ;
4,4-difluoro-2-(4-fluorophényl)-N- {4-[3-(4-fluorophényl)-5,7-diméthyl-4-oxo-4,5,6,7-tétrahydro-1H-pyrrolo[3,2-c]pyridin-2-yl]pyridin-2-yl}butanamide ;
4,4-difluoro-2-(4-fluorophényl)-N- {4-[3-(4-fluorophényl)-5,7-diméthyl-4-oxo-4,5-dihydro-1H-pyrrolo[3,2-c]pyridin-2-yl]pyridin-2-yl}butanamide ;
(2R)-4,4-difluoro-2-(4-fluorophényl)-N- {4-[3-(4-fluorophényl)-5,7-diméthyl-4-oxo-4,5-dihydro-1H-pyrrolo[3,2-c]pyridin-2-yl]pyridin-2-yl}butanamide ;
(2S)-4,4-difluoro-2-(4-fluorophényl)-N- {4-[3-(4-fluorophényl)-5,7-diméthyl-4-oxo-4,5-dihydro-1H-pyrrolo[3,2-c]pyridin-2-yl]pyridin-2-yl}butanamide ;
N-{4-[5,7-diméthyl-4-oxo-3-phényl-4,5,6,7-tétrahydro-1H-pyrrolo[3,2-c]pyridin-2-yl]pyridin-2-yl} -2-(4-fluorophényl)propanamide ;
N-[4-(5,7-diméthyl-4-oxo-3-phényl-4,5-dihydro-1H-pyrrolo[3,2-c]pyridin-2-yl)pyridin-2-yl]-2-(4-fluorophényl)propanamide ;
N-{4-[5,7-diméthyl-4-oxo-3-phényl-4,5,6,7-tétrahydro-1H-pyrrolo[3,2-c]pyridin-2-yl]pyridin-2-yl} -4,4-difluoro-2-(4-fluorophényl)butanamide ;
N-[4-(5,7-diméthyl-4-oxo-3-phényl-4,5-dihydro-1H-pyrrolo[3,2-c]pyridin-2-yl)pyridin-2-yl]-4,4-difluoro-2-(4-fluorophényl)butanamide ;
4,4-difluoro-2-(4-fluorophényl)-N- {4-[5,6,7-triméthyl-4-oxo-3-phényl-4,5,6,7-tétrahydro-1H-pyrrolo[3,2-c]pyridin-2-yl]pyridin-2-yl}butanamide ;
N-[4-(7,7-diméthyl-4-oxo-3-phényl-4,5,6,7-tétrahydro-1H-pyrrolo[3,2-c]pyridin-2-yl)pyridin-2-yl]-2-(4-fluorophényl)acétamide ;
N-[4-(7,7-diméthyl-4-oxo-3-phényl-4,5,6,7-tétrahydro-1H-pyrrolo[3,2-c]pyridin-2-yl)pyridin-2-yl]-4,4-difluoro-2-(4-fluorophényl)butanamide ;
(2R)-N-[4-(7,7-diméthyl-4-oxo-3-phényl-4,5,6,7-tétrahydro-1H-pyrrolo[3,2-c]pyridin-2-yl)pyridin-2-yl]-4,4-difluoro-2-(4-fluorophényl)butanamide ;
(2S)-N-[4-(7,7-diméthyl-4-oxo-3-phényl-4,5,6,7-tétrahydro-1H-pyrrolo[3,2-c]pyridin-2-yl)pyridin-2-yl]-4,4-difluoro-2-(4-fluorophényl)butanamide ;
N-[4-(7,7-diméthyl-4-oxo-3-phényl-4,5,6,7-tétrahydro-1H-pyrrolo[3,2-c]pyridin-2-yl)pyridin-2-yl]-2-(4-fluorophényl)propanamide ;
(2R)-N-[4-(7,7-diméthyl-4-oxo-3-phényl-4,5,6,7-tétrahydro-1H-pyrrolo[3,2-c]pyridin-2-yl)pyridin-2-yl]-2-(4-fluorophényl)propanamide ;
(2S)-N-[4-(7,7-diméthyl-4-oxo-3-phényl-4,5,6,7-tétrahydro-1H-pyrrolo[3,2-c]pyridin-2-yl)pyridin-2-yl]-2-(4-fluorophényl)propanamide ;
4,4-difluoro-2-(4-fluorophényl)-N-[4-(5'-méthyl-4'-oxo-3'-phényl-1',4',5',7'-tétrahydrospiro[cyclobutane-1,6'-pyrrolo[3,2-c]pyridin]-2'-yl)pyridin-2-yl]butanamide ;
(2R)-4,4-difluoro-2-(4-fluorophényl)-N-[4-(5'-méthyl-4'-oxo-3'-phényl-1',4',5',7'-tétrahydrospiro-[cyclobutane-1,6'-pyrrolo[3,2-c]pyridin]-2'-yl)pyridin-2-yl]butanamide ;
(2S)-4,4-difluoro-2-(4-fluorophényl)-N-[4-(5'-méthyl-4'-oxo-3'-phényl-1',4',5',7'-tétrahydrospiro-[cyclobutane-1,6'-pyrrolo[3,2-c]pyridin]-2'-yl)pyridin-2-yl]butanamide ;
2-(4-fluorophényl)-N-[4-(5'-méthyl-4'-oxo-3'-phényl-1',4',5',7'-tétrahydrospiro[cyclobutane-1,6'-pyrrolo[3,2-c]pyridin]-2'-yl)pyridin-2-yl]propanamide ;
(2R)-2-(4-fluorophényl)-N-[4-(5'-méthyl-4'-oxo-3'-phényl-1',4',5',7'-tétrahydrospiro[cyclobutane-1,6'-pyrrolo[3,2-c]pyridin]-2'-yl)pyridin-2-yl]propanamide ;
(2S)-2-(4-fluorophényl)-N-[4-(5'-méthyl-4'-oxo-3'-phényl-1',4',5',7'-tétrahydrospiro[cyclobutane-1,6'-pyrrolo[3,2-c]pyridin]-2'-yl)pyridin-2-yl]propanamide ;
N-{4-[7-(cyclopropylméthyl)-5-méthyl-4-oxo-3-phényl-4,5,6,7-tétrahydro-1H-pyrrolo[3,2-c]pyridin-2-yl]pyridin-2-yl}-2-(4-fluorophényl)acétamide ;
N-{4-[7-(cyclopropylméthyl)-5-méthyl-4-oxo-3-phényl-4,5-dihydro-1H-pyrrolo[3,2-c]pyridin-2-yl]pyridin-2-yl}-2-(4-fluorophényl)acétamide ;
N-{4-[7-(cyclopropylméthyl)-5-méthyl-4-oxo-3-phényl-4,5,6,7-tétrahydro-1H-pyrrolo[3,2-c]pyridin-2-yl]pyridin-2-yl}-2-(4-fluorophényl)propanamide ;
N-{4-[7-(cyclopropylméthyl)-5-méthyl-4-oxo-3-phényl-4,5-dihydro-1H-pyrrolo[3,2-c]pyridin-2-yl]pyridin-2-yl}-2-(4-fluorophényl)propanamide ;
N-{4-[7-(cyclopropylméthyl)-5-méthyl-4-oxo-3-phényl-4,5,6,7-tétrahydro-1H-pyrrolo[3,2-c]pyridin-2-yl]pyridin-2-yl}-4,4-difluoro-2-(4-fluorophényl)butanamide ;
N-{4-[7-(cyclopropylméthyl)-5-méthyl-4-oxo-3-phényl-4,5-dihydro-1H-pyrrolo[3,2-c]pyridin-2-yl]pyridin-2-yl}-4,4-difluoro-2-(4-fluorophényl)butanamide ;
N-{4-[7-(cyclopropylméthyl)-3-(4-fluorophényl)-5-méthyl-4-oxo-4,5,6,7-tétrahydro-1H-pyrrolo[3,2-c]pyridin-2-yl]pyridin-2-yl}-2-(4-fluorophényl)acétamide ;
N-{4-[7-(cyclopropylméthyl)-3-(4-fluorophényl)-5-méthyl-4-oxo-4,5,6,7-tétrahydro-1H-pyrrolo[3,2-c]pyridin-2-yl]pyridin-2-yl}-2-(4-fluorophényl)propanamide ;
N-{4-[7-(cyclopropylméthyl)-3-(4-fluorophényl)-5-méthyl-4-oxo-4,5-dihydro-1H-pyrrolo[3,2-c]pyridin-2-yl]pyridin-2-yl}-2-(4-fluorophényl)propanamide ;
N-{4-[7-(cyclopropylméthyl)-3-(4-fluorophényl)-5-méthyl-4-oxo-4,5,6,7-tétrahydro-1H-pyrrolo[3,2-c]pyridin-2-yl]pyridin-2-yl}-4,4-difluoro-2-(4-fluorophényl)butanamide ;
N-{4-[7-(cyclopropylméthyl)-3-(4-fluorophényl)-5-méthyl-4-oxo-4,5-dihydro-1H-pyrrolo[3,2-c]pyridin-2-yl]pyridin-2-yl}-4,4-difluoro-2-(4-fluorophényl)butanamide ;
N-{4-[3-(2,4-difluorophényl)-5-méthyl-4-oxo-4,5,6,7-tétrahydro-1H-pyrrolo[3,2-c]pyridin-2-yl]pyridin-2-yl}-4,4-difluoro-2-(4-fluorophényl)butanamide ;
(2R)-N-{4-[3-(2,4-difluorophényl)-5-méthyl-4-oxo-4,5,6,7-tétrahydro-1H-pyrrolo[3,2-c]pyridin-2-yl]pyridin-2-yl}-4,4-difluoro-2-(4-fluorophényl)butanamide ;
(2S)-N-{4-[3-(2,4-difluorophényl)-5-méthyl-4-oxo-4,5,6,7-tétrahydro-1H-pyrrolo[3,2-c]pyridin-2-yl]pyridin-2-yl}-4,4-difluoro-2-(4-fluorophényl)butanamide ;
N-{4-[3-(2,4-difluorophényl)-5-méthyl-4-oxo-4,5,6,7-tétrahydro-1H-pyrrolo[3,2-c]pyridin-2-yl]pyridin-2-yl} -2-(4-fluorophényl)propanamide ;
(2R)-N-{4-[3-(2,4-difluorophényl)-5-méthyl-4-oxo-4,5,6,7-tétrahydro-1H-pyrrolo[3,2-c]pyridin-2-yl]pyridin-2-yl}-2-(4-fluorophényl)propanamide ;
(2S)-N-{4-[3-(2,4-difluorophényl)-5-méthyl-4-oxo-4,5,6,7-tétrahydro-1H-pyrrolo[3,2-c]pyridin-2-yl]pyridin-2-yl}-2-(4-fluorophényl)propanamide ;
2-(4-fluorophényl)-N-[4-(4-oxo-3-phényl-4,5,6,7-tétrahydro-1H-pyrrolo[3,2-c]pyridin-2-yl)pyridin-2-yl]acétamide ;
2-(4-fluorophényl)-N-[4-(4-oxo-3-phényl-4,5-dihydro-1H-pyrrolo[3,2-c]pyridin-2-yl)pyridin-2-yl]acétamide ;
2-(4-fluorophényl)-N-[4-(4-oxo-3-phényl-4,5,6,7-tétrahydro-1H-pyrrolo[3,2-c]pyridin-2-yl)pyridin-2-yl]propanamide ;
4,4-difluoro-2-(4-fluorophényl)-N-[4-(4-oxo-3-phényl-4,5,6,7-tétrahydro-1H-pyrrolo[3,2-c]pyridin-2-yl)pyridin-2-yl]butanamide ;
4,4-difluoro-2-(4-fluorophényl)-N-[4-(4-oxo-3-phényl-4,5-dihydro-1H-pyrrolo[3,2-c]pyridin-2-yl)pyridin-2-yl]butanamide ;
(2R)-4,4-difluoro-2-(4-fluorophényl)-N-[4-(4-oxo-3-phényl-4,5-dihydro-1H-pyrrolo[3,2-c]pyridin-2-yl)pyridin-2-yl]butanamide ;
(2S)-4,4-difluoro-2-(4-fluorophényl)-N-[4-(4-oxo-3-phényl-4,5-dihydro-1H-pyrrolo[3,2-c]pyridin-2-yl)pyridin-2-yl]butanamide ;
N-[4-(7-chloro-5-méthyl-4-oxo-3-phényl-4,5-dihydro-1H-pyrrolo[3,2-c]pyridin-2-yl)pyridin-2-yl]-2-(4-fluorophényl)acétamide ;
4,4-difluoro-2-(4-fluorophényl)-N-[4-(5'-méthyl-4'-oxo-3'-phényl-1',4',5',7'-tétrahydrospiro-[cyclopropane-1,6'-pyrrolo[3,2-c]pyridin]-2'-yl)pyridin-2-yl]butanamide ;

4,4-difluoro-2-(4-fluorophényl)-N-{4-[3'-(4-fluorophényl)-5'-méthyl-4'-oxo-1',4',5',7'-tétrahydro-spiro[cyclopropane-1,6'-pyrrolo[3,2-c]pyridin]-2'-yl]pyridin-2-yl}butanamide ;
N-[4-(6,6-diméthyl-4-oxo-3-phényl-4,5,6,7-tétrahydro-1H-pyrrolo[3,2-c]pyridin-2-yl)pyridin-2-yl]-2-(4-fluorophényl)acétamide ;
2-(4-fluorophényl)-N-{4-[3-(4-fluorophényl)-6,6-diméthyl-4-oxo-4,5,6,7-tétrahydro-1H-pyrrolo[3,2-c]pyridin-2-yl]pyridin-2-yl}acétamide ;
2-(4-fluorophényl)-N-{4-[3-(2-fluorophényl)-6,6-diméthyl-4-oxo-4,5,6,7-tétrahydro-1H-pyrrolo[3,2-c]pyridin-2-yl]pyridin-2-yl}acétamide ;
2-(4-fluorophényl)-N-{4-[3-(3-fluorophényl)-6,6-diméthyl-4-oxo-4,5,6,7-tétrahydro-1H-pyrrolo[3,2-c]pyridin-2-yl]pyridin-2-yl}acétamide ;
N-{4-[6,6-diméthyl-3-(3-méthylphényl)-4-oxo-4,5,6,7-tétrahydro-1H-pyrrolo[3,2-c]pyridin-2-yl]pyridin-2-yl}-2-(4-fluorophényl)acétamide ;
2-(4-fluorophényl)-N-{4-[3-(2-fluorophényl)-6,6-diméthyl-4-oxo-4,5,6,7-tétrahydro-1H-pyrrolo[3,2-c]pyridin-2-yl]pyridin-2-yl}propanamide ;
N-{4-[6,6-diméthyl-3-(3-méthylphényl)-4-oxo-4,5,6,7-tétrahydro-1H-pyrrolo[3,2-c]pyridin-2-yl]pyridin-2-yl}-2-(4-fluorophényl)propanamide ;
N-{4-[3-(4-chlorophényl)-6,6-diméthyl-4-oxo-4,5,6,7-tétrahydro-1H-pyrrolo[3,2-c]pyridin-2-yl]pyridin-2-yl}-2-(4-fluorophényl)propanamide ;
N-{4-[3-(4-chlorophényl)-6,6-diméthyl-4-oxo-4,5,6,7-tétrahydro-1H-pyrrolo[3,2-c]pyridin-2-yl]pyridin-2-yl}-2-(4-fluorophényl)acétamide ;
N-{4-[6,6-diméthyl-3-(4-méthylphényl)-4-oxo-4,5,6,7-tétrahydro-1H-pyrrolo[3,2-c]pyridin-2-yl]pyridin-2-yl}-2-(4-fluorophényl)propanamide ;
N-{4-[6,6-diméthyl-3-(4-méthylphényl)-4-oxo-4,5,6,7-tétrahydro-1H-pyrrolo[3,2-c]pyridin-2-yl]pyridin-2-yl}-2-(4-fluorophényl)acétamide ;
N-{4-[6,6-diméthyl-4-oxo-3-(pyridin-3-yl)-4,5,6,7-tétrahydro-1H-pyrrolo[3,2-c]pyridin-2-yl]pyridin-2-yl} -2-(4-fluorophényl)acétamide ;
2-(4-fluorophényl)-N-{4-[3-(4-fluorophényl)-6,6-diméthyl-4-oxo-4,5,6,7-tétrahydro-1H-pyrrolo[3,2-c]pyridin-2-yl]pyridin-2-yl}propanamide ;
(2R)-2-(4-fluorophényl)-N-{4-[3-(4-fluorophényl)-6,6-diméthyl-4-oxo-4,5,6,7-tétrahydro-1H-pyrrolo[3,2-c]pyridin-2-yl]pyridin-2-yl}propanamide ;
(2S)-2-(4-fluorophényl)-N-{4-[3-(4-fluorophényl)-6,6-diméthyl-4-oxo-4,5,6,7-tétrahydro-1H-pyrrolo[3,2-c]pyridin-2-yl]pyridin-2-yl}propanamide ;
N-{4-[6,6-diméthyl-4-oxo-3-(pyridin-3-yl)-4,5,6,7-tétrahydro-1H-pyrrolo[3,2-c]pyridin-2-yl]pyridin-2-yl} -2-(4-fluorophényl)propanamide ;
(2R)-N-{4-[6,6-diméthyl-4-oxo-3-(pyridin-3-yl)-4,5,6,7-tétrahydro-1H-pyrrolo[3,2-c]pyridin-2-yl]pyridin-2-yl}-2-(4-fluorophényl)propanamide ;
(2S)-N-{4-[6,6-diméthyl-4-oxo-3-(pyridin-3-yl)-4,5,6,7-tétrahydro-1H-pyrrolo[3,2-c]pyridin-2-yl]pyridin-2-yl}-2-(4-fluorophényl)propanamide ;
2-(4-fluorophényl)-N-{4-[3-(4-fluorophényl)-5-méthyl-4-oxo-4,5-dihydro-1H-pyrrolo[3,2-c]pyridin-2-yl]pyridin-2-yl}propanamide ;
(2R)-2-(4-fluorophényl)-N-{4-[3-(4-fluorophényl)-5-méthyl-4-oxo-4,5-dihydro-1H-pyrrolo[3,2-c]pyridin-2-yl]pyridin-2-yl}propanamide ;
(2S)-2-(4-fluorophényl)-N-{4-[3-(4-fluorophényl)-5-méthyl-4-oxo-4,5-dihydro-1H-pyrrolo[3,2-c]pyridin-2-yl]pyridin-2-yl}propanamide ;
2-(4-fluorophényl)-N-[4-(5-méthyl-4-oxo-3-phényl-4,5-dihydro-1H-pyrrolo[3,2-c]pyridin-2-yl)pyridin-2-yl]propanamide ;
(2R)-2-(4-fluorophényl)-N-[4-(5-méthyl-4-oxo-3-phényl-4,5-dihydro-1H-pyrrolo[3,2-c]pyridin-2-yl)pyridin-2-yl]propanamide ;
(2S)-2-(4-fluorophényl)-N-[4-(5-méthyl-4-oxo-3-phényl-4,5-dihydro-1H-pyrrolo[3,2-c]pyridin-2-yl)pyridin-2-yl]propanamide ;
N-{4-[3-(3-chlorophényl)-5-méthyl-4-oxo-4,5-dihydro-1H-pyrrolo[3,2-c]pyridin-2-yl]pyridin-2-yl} -2-(4-fluorophényl)propanamide ;
4,4-difluoro-2-(4-fluorophényl)-N- {4-[3-(4-fluorophényl)-5-méthyl-4-oxo-4,5-dihydro-1H-pyrrolo[3,2-c]pyridin-2-yl]pyridin-2-yl}butanamide ;
(2R)-4,4-difluoro-2-(4-fluorophényl)-N- {4-[3-(4-fluorophényl)-5-méthyl-4-oxo-4,5-dihydro-1H-pyrrolo[3,2-c]pyridin-2-yl]pyridin-2-yl}butanamide ;
(2S)-4,4-difluoro-2-(4-fluorophényl)-N- {4-[3-(4-fluorophényl)-5-méthyl-4-oxo-4,5-dihydro-1H-pyrrolo[3,2-c]pyridin-2-yl]pyridin-2-yl}butanamide ;

2-(4-fluorophényl)-N-{4-[3-(2-fluoropyridin-3-yl)-5-méthyl-4-oxo-4,5-dihydro-1H-pyrrolo[3,2-c]pyridin-2-yl]pyridin-2-yl}propanamide ;
2-(4-fluorophényl)-N-{4-[5-méthyl-3-(2-méthylphényl)-4-oxo-4,5-dihydro-1H-pyrrolo[3,2-c]pyridin-2-yl]pyridin-2-yl}propanamide ;
N-{4-[3-(2-chlorophényl)-5-méthyl-4-oxo-4,5-dihydro-1H-pyrrolo[3,2-c]pyridin-2-yl]pyridin-2-yl} -2-(4-fluorophényl)propanamide ;
2-(4-fluorophényl)-N-{4-[3-(3-hydroxyphényl)-5-méthyl-4-oxo-4,5-dihydro-1H-pyrrolo[3,2-c]pyridin-2-yl]pyridin-2-yl}propanamide ;
N-{4-[3-(3,4-difluorophényl)-5-méthyl-4-oxo-4,5-dihydro-1H-pyrrolo[3,2-c]pyridin-2-yl]pyridin-2-yl} -2-(4-fluorophényl)propanamide ;
2-(4-fluorophényl)-N-{4-[3-(3-furyl)-5-méthyl-4-oxo-4,5-dihydro-1H-pyrrolo[3,2-c]pyridin-2-yl]pyridin-2-yl}propanamide ;
2-(4-fluorophényl)-N-{4-[3-(2-furyl)-5-méthyl-4-oxo-4,5-dihydro-1H-pyrrolo[3,2-c]pyridin-2-yl]pyridin-2-yl}propanamide ;
2-(4-fluorophényl)-N-{4-[5-méthyl-4-oxo-3-(3,4,5-trifluorophényl)-4,5-dihydro-1H-pyrrolo[3,2-c]pyridin-2-yl]pyridin-2-yl}propanamide ;
4,4-difluoro-2-(4-fluorophényl)-N-{4-[5-méthyl-4-oxo-3-(pyridin-3-yl)-4,5-dihydro-1H-pyrrolo[3,2-c]pyridin-2-yl]pyridin-2-yl}butanamide ;
2-(4-fluorophényl)-N - {4- [3 -(4-hydroxyphényl)-5 -méthyl-4-oxo-4,5 -dihydro-1H-pyrrolo[3,2-c]pyridin-2-yl]pyridin-2-yl}propanamide ;
N-{4-[3-(2,5-difluorophényl)-5-méthyl-4-oxo-4,5-dihydro-1H-pyrrolo[3,2-c]pyridin-2-yl]pyridin-2-yl} -2-(4-fluorophényl)propanamide ;
N-{4-[3-(3,5-difluorophényl)-5-méthyl-4-oxo-4,5-dihydro-1H-pyrrolo[3,2-c]pyridin-2-yl]pyridin-2-yl} -2-(4-fluorophényl)propanamide ;
N-{4-[3-(5-chloro-2-thiényl)-5-méthyl-4-oxo-4,5-dihydro-1H-pyrrolo[3,2-c]pyridin-2-yl]pyridin-2-yl} -2-(4-fluorophényl)propanamide ;
N- {4-[3-(2-cyanophényl)-5-méthyl-4-oxo-4,5-dihydro-1H-pyrrolo[3,2-c]pyridin-2-yl]pyridin-2-yl} -2-(4-fluorophényl)propanamide ;
2-(4-fluorophényl)-N - {4- [3 -(2-hydroxyphényl)-5 -méthyl-4-oxo-4,5 -dihydro-1H-pyrrolo[3,2-c]pyridin-2-yl]pyridin-2-yl}propanamide ;
2-(4-fluorophényl)-N-{4-[5-méthyl-3-(5-méthyl-2-thiényl)-4-oxo-4,5-dihydro-1H-pyrrolo[3,2-c]pyridin-2-yl]pyridin-2-yl}propanamide ;
N- {4-[3-(2-aminopyridin-4-yl)-5-méthyl-4-oxo-4,5-dihydro-IH-pyrrolo[3,2-c]pyridin-2-yl]pyridin-2-yl} -2-(4-fluorophényl)propanamide ;
N-{4-[3-(2-cyano-5-fluorophényl)-5-méthyl-4-oxo-4,5-dihydro-1H-pyrrolo[3,2-c]pyridin-2-yl]pyridin-2-yl} -2-(4-fluorophényl)propanamide ;
N-{4-[3-(2-cyano-5-fluorophényl)-5-méthyl-4-oxo-4,5,6,7-tétrahydro-1H-pyrrolo[3,2-c]pyridin-2-yl]pyridin-2-yl}-4,4-difluoro-2-(4-fluorophényl)butanamide ;
4,4-difluoro-2-(4-fluorophényl)-N- {4-[5-méthyl-3-(2-méthyl-1,3-thiazol-5-yl)-4-oxo-4,5,6,7-tétrahydro-1H-pyrrolo[3,2-c]pyridin-2-yl]pyridin-2-yl}butanamide ;
2-(4-fluorophényl)-N-{4-[5-méthyl-4-oxo-3-(2-thiényl)-4,5-dihydro-1H-pyrrolo[3,2-c]pyridin-2-yl]pyridin-2-yl}propanamide ;
2-(4-fluorophényl)-N-{4-[5-méthyl-4-oxo-3-(3-thiényl)-4,5-dihydro-1H-pyrrolo[3,2-c]pyridin-2-yl]pyridin-2-yl}propanamide ;
N-[4-(3,5-diméthyl-4-oxo-4,5-dihydro-1H-pyrrolo[3,2-c]pyridin-2-yl)pyridin-2-yl]-2-(4-fluorophényl)propanamide ;
N-{4-[3-(5-chloro-2-thiényl)-5-méthyl-4-oxo-4,5-dihydro-1H-pyrrolo[3,2-c]pyridin-2-yl]pyridin-2-yl}-4,4-difluoro-2-(4-fluorophényl)butanamide ;
N-{4-[5-cyclopropyl-3-(4-fluorophényl)-4-oxo-4,5-dihydro-1H-pyrrolo[3,2-c]pyridin-2-yl]pyridin-2-yl}-4,4-difluoro-2-(4-fluorophényl)butanamide ;
(2R)-N-{4-[5-cyclopropyl-3-(4-fluorophényl)-4-oxo-4,5-dihydro-1H-pyrrolo[3,2-c]pyridin-2-yl]pyridin-2-yl}-4,4-difluoro-2-(4-fluorophényl)butanamide ;
(2S)-N-{4-[5-cyclopropyl-3-(4-fluorophényl)-4-oxo-4,5-dihydro-1H-pyrrolo[3,2-c]pyridin-2-yl]pyridin-2-yl}-4,4-difluoro-2-(4-fluorophényl)butanamide ;
N-[4-(5-cyclopropyl-4-oxo-3-phényl-4,5-dihydro-1H-pyrrolo[3,2-c]pyridin-2-yl)pyridin-2-yl]-4,4-difluoro-2-(4-fluorophényl)butanamide ;
4,4-difluoro-2-(4-fluorophényl)-N-{4-[5-méthyl-4-oxo-3-(1,3-thiazol-4-yl)-4,5,6,7-tétrahydro-1H-pyrrolo[3,2-c]pyridin-2-yl]pyridin-2-yl}butanamide ;

4,4-difluoro-2-(4-fluorophényl)-N- {4-[3-(4-fluorophényl)-5-méthyl-4-oxo-4,5,6,7-tétrahydro-1H-pyrrolo[3,2-c]pyridin-2-yl]pyridin-2-yl}butanamide ;
4-[2-({4-[3-(4-fluorophényl)-5-méthyl-4-oxo-4,5,6,7-tétrahydro-1H-pyrrolo[3,2-c]pyridin-2-yl]pyridin-2-yl}amino)-2-oxoéthyle]-N-(2-méthoxyéthyl)benzamide ;
4-[2-({4-[3-(4-fluorophényl)-5-méthyl-4-oxo-4,5,6,7-tétrahydro-1H-pyrrolo[3,2-c]pyridin-2-yl]pyridin-2-yl}amino)-2-oxoéthyle]-N-(2-méthoxyéthyl)-N-méthylbenzamide ;
4-[2-({4-[3-(4-fluorophényl)-5-méthyl-4-oxo-4,5-dihydro-1H-pyrrolo[3,2-c]pyridin-2-yl]pyridin-2-yl} amino)-2-oxoéthyle]-N-(2-méthoxyéthyl)benzamide ;
4-[2-({4-[3-(4-fluorophényl)-5-méthyl-4-oxo-4,5-dihydro-1H-pyrrolo[3,2-c]pyridin-2-yl]pyridin-2-yl}amino)-2-oxoéthyl]-N-(2-méthoxyéthyl)-N-méthylbenzamide ;
N-{4-[3-(4-fluorophényl)-5-méthyl-4-oxo-4,5,6,7-tétrahydro-1H-pyrrolo[3,2-c]pyridin-2-yl]pyridin-2-yl}-2-[4-(pipérazine-1-carbonyl)phényl]acétamide ;
2-(4-fluorophényl)-N-{4-[3-(6-fluoropyridin-3-yl)-5-méthyl-4-oxo-4,5-dihydro-1H-pyrrolo[3,2-c]pyridin-2-yl]pyridin-2-yl}propanamide ;
2-(4-fluorophényl)-N-{4-[5-méthyl-4-oxo-3-(pyridin-2-yl)-4,5-dihydro-1H-pyrrolo[3,2-c]pyridin-2-yl]pyridin-2-yl} acétamide ;
(2RS)-N-{4-[7-(2,2-difluoroéthyl)-5-méthyl-4-oxo-3-phényl-4,5-dihydro-1H-pyrrolo[3,2-c]pyridin-2-yl]pyridin-2-yl}-4,4-difluoro-2-(4-fluorophényl)butanamide ;
(2R)-N-{4-[7-(2,2-difluoroéthyl)-5-méthyl-4-oxo-3-phényl-4,5-dihydro-1H-pyrrolo[3,2-c]pyridin-2-yl]pyridin-2-yl}-4,4-difluoro-2-(4-fluorophényl)butanamide ;
(2S)-N-{4-[7-(2,2-difluoroéthyl)-5-méthyl-4-oxo-3-phényl-4,5-dihydro-1H-pyrrolo[3,2-c]pyridin-2-yl]pyridin-2-yl}-4,4-difluoro-2-(4-fluorophényl)butanamide ;
(2RS)-4,4-difluoro-2-(4-fluorophényl)-N-[4-(5,7,7-triméthyl-4-oxo-3-phényl-4,5,6,7-tétrahydro-1H-pyrrolo[3,2-c]pyridin-2-yl)pyridin-2-yl]butanamide ;
(2R)-4,4-difluoro-2-(4-fluorophényl)-N-[4-(5,7,7-triméthyl-4-oxo-3-phényl-4,5,6,7-tétrahydro-1H-pyrrolo[3,2-c]pyridin-2-yl)pyridin-2-yl]butanamide ;
(2S)-4,4-difluoro-2-(4-fluorophényl)-N-[4-(5,7,7-triméthyl-4-oxo-3-phényl-4,5,6,7-tétrahydro-1H-pyrrolo[3,2-c]pyridin-2-yl)pyridin-2-yl]butanamide ;
(2RS)-N-[4-(5-méthyl-4-oxo-3-phényl-4,5,6,7-tétrahydro-1H-pyrrolo[3,2-c]pyridin-2-yl)pyridin-2-yl] -2-(pyridin-4-yl)propanamide ;
(2R)-N-[4-(5-méthyl-4-oxo-3-phényl-4,5,6,7-tétrahydro-1H-pyrrolo[3,2-c]pyridin-2-yl)pyridin-2-yl] -2-(pyridin-4-yl)propenamide ;
(2S)-N-[4-(5-méthyl-4-oxo-3-phényl-4,5,6,7-tétrahydro-1H-pyrrolo[3,2-c]pyridin-2-yl)pyridin-2-yl] -2-(pyridin-4-yl)propanamide ;
(2RS)-N-(4-(5-cyclopropyl-4-oxo-3-phényl-4,5,6,7-tétrahydro-1H-pyrrolo[3,2-c]pyridin-2-yl)pyridin-2-yl)-4,4-difluoro-2-(4-fluorophényl)butanamide ;
(2R)-N-[4-(5-cyclopropyl-4-oxo-3-phényl-4,5-dihydro-1H-pyrrolo[3,2-c]pyridin-2-yl)pyridin-2-yl] -4,4-difluoro-2-(4-fluorophényl)butanamide ;
(2S)-N-[4-(5-cyclopropyl-4-oxo-3-phényl-4,5-dihydro-1H-pyrrolo[3,2-c]pyridin-2-yl)pyridin-2-yl]-4,4-difluoro-2-(4-fluorophényl)butanamide ;
N-[4-(5-cyclopropyl-4-oxo-3-phényl-4,5-dihydro-1H-pyrrolo[3,2-c]pyridin-2-yl)pyridin-2-yl]-2-(4-fluorophényl)acétamide ;
(2RS)-N-{4-[5-cyclopropyl-4-oxo-3-(pyridin-2-yl)-4,5-dihydro-1H-pyrrolo[3,2-c]pyridin-2-yl]pyridin-2-yl}-4,4-difluoro-2-(4-fluorophényl)butanamide ;
(2RS)-N-{4-[7-chloro-5-cyclopropyl-3-(4-fluorophényl)-4-oxo-4,5-dihydro-1H-pyrrolo[3,2-c]pyridin-2-yl]pyridin-2-yl}-4,4-difluoro-2-(4-fluorophényl)butanamide ;
(2RS)-4,4-difluoro-2-(4-fluorophényl)-N-{4-[5-méthyl-4-oxo-3-(pyridin-2-yl)-7-(2,2,2-trifluoroéthyl)-4,5-dihydro-1H-pyrrolo[3,2-c]pyridin-2-yl]pyridin-2-yl}butanamide ;
ou un N-oxyde, un sel, un tautomère ou un stéréoisomère dudit composé, ou un sel dudit N-oxyde, tautomère ou stéréoisomère.

**6.** Procédé de préparation d'un composé de formule générale (I) selon l'une quelconque des revendications 1 à 5, ledit procédé comprenant la réaction d'un composé intermédiaire de formule générale (1-3) :

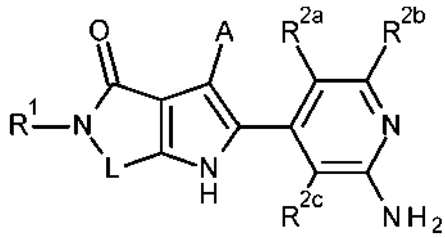

1-3,

dans lequel $R^1$, $R^{2a}$, $R^{2b}$, $R^{2c}$, A, et L sont tels que définis pour le composé de formule générale (I) selon l'une quelconque des revendications 1 à 4,
avec un réactif d'acylation sélectionné parmi :

a) un acide carboxylique de formule

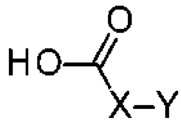

,

b) un halogénure d'acyle de formule

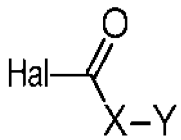

,

dans lequel Hal représente F, Cl ou Br, ou
c) un anhydride de formule

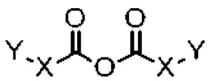

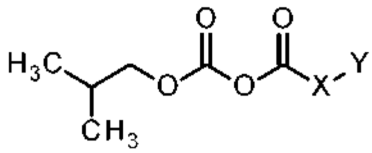

ou

dans lequel X et Y sont tels que définis pour le composé de formule générale (I) selon l'une quelconque des revendications 1 à 4,
pour donner un composé de formule générale (I) :

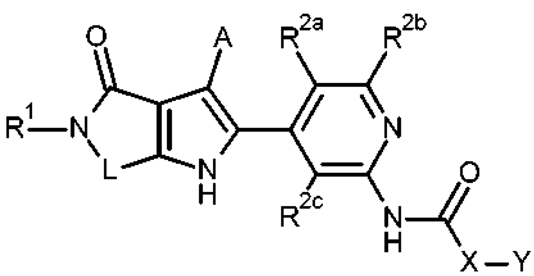

(I),

dans lequel $R^1$, $R^{2a}$, $R^{2b}$, $R^{2c}$, A, L, X, et Y sont tels que définis pour le composé de formule générale (I) selon l'une quelconque des revendications 1 à 4.

**7.** Composé de formule générale (1) selon l'une quelconque des revendications 1 à 5, destiné à être utilisé dans le traitement ou la prophylaxie d'une maladie hyperproliférative et/ou d'un trouble répondant à l'induction de la mort cellulaire.

**8.** Composé de formule générale (I) destiné à être utilisé selon la revendication 7, dans lequel la maladie hyperproliférative et/ou le trouble répondant à l'induction de la mort cellulaire sont une tumeur hématologique, une tumeur solide et/ou des métastases de celles-ci.

**9.** Composé de formule (I) destiné à être utilisé selon la revendication 8, dans lequel la tumeur hématologique est un lymphome et/ou des métastases de celui-ci, de préférence dans lequel le lymphome est un lymphome diffus à grandes cellules B et/ou des métastases de celui-ci, la tumeur solide est une tumeur cervicale, une tumeur du poumon, une tumeur du côlon et/ou des métastases de celles-ci, de préférence dans lequel la tumeur du poumon est un carcinome du poumon et/ou des métastases de celui-ci et la tumeur du côlon est un carcinome colorectal et/ou des métastases de celui-ci.

**10.** Composition pharmaceutique comprenant au moins un composé de formule générale (I) selon l'une quelconque des revendications 1 à 5, avec au moins un excipient de qualité pharmaceutique.

**11.** Combinaison comprenant un ou plusieurs premiers ingrédients actifs sélectionnés entre un composé de formule générale (I) selon l'une quelconque des revendications 1 à 5, et un ou plusieurs deuxièmes ingrédients actifs sélectionnés entre des agents anti-cancer chimio-thérapeutiques et des agents anti-cancer spécifiques à une cible.

**12.** Composé intermédiaire de formule 1-3, Ia ou 1-9, ou sel de celui-ci :

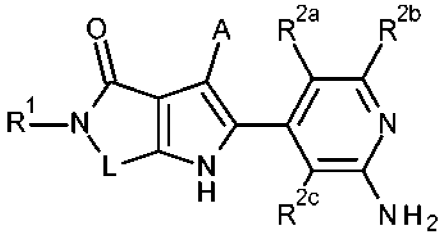

1-3,

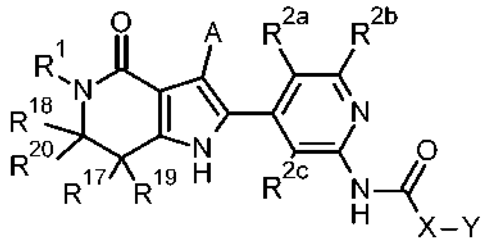

**(Ia)**

,

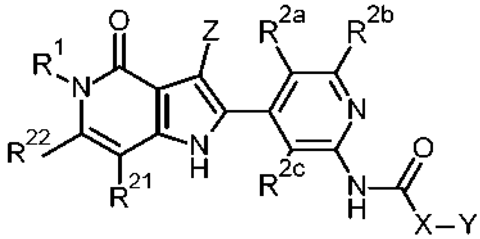

**1-9**

dans lequel $R^1$, $R^{2a}$, $R^{2b}$, $R^{2c}$, $R^{17}$, $R^{18}$, $R^{19}$, $R^{20}$, $R^{21}$, $R^{22}$, A, X, Y, et L sont tels que définis pour le composé de formule générale (I) selon l'une quelconque des revendications 1 à 4, et Z représente chloro, bromo ou iodo.

**13.** Utilisation d'un composé de formule 1-3 ou d'un sel de celui-ci :

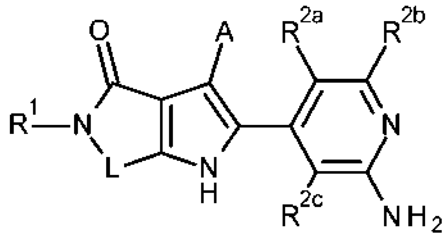

1-3,

dans lequel $R^1$, $R^{2a}$, $R^{2b}$, $R^{2c}$, A et L sont tels que définis pour le composé de formule générale (I) selon l'une quelconque des revendications 1 à 4, ou
d'un composé de formule générale (Ia) ou d'un sel de celui-ci :

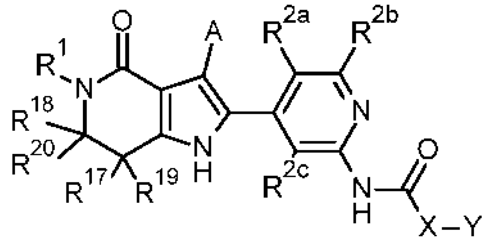

**(Ia)**

,

dans lequel $R^1$, $R^{2a}$, $R^{2b}$, $R^{2c}$, $R^{17}$, $R^{18}$, $R^{19}$, $R^{20}$, A, X, et Y sont tels que définis pour le composé de formule générale (1) selon l'une quelconque des revendications 1 à 4, ou
d'un composé de formule générale 1-9 ou d'un sel de celui-ci :

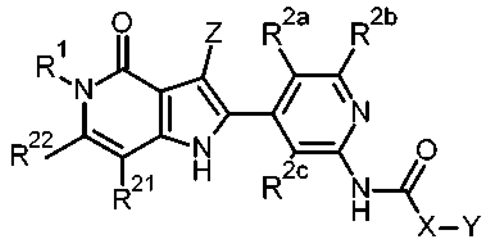

**1-9**

,

dans lequel $R^1$, $R^{2a}$, $R^{2b}$, $R^{2c}$, $R^{21}$, $R^{22}$, X et Y sont tels que définis pour le composé de formule générale (I) selon l'une quelconque des revendications 1 à 4, et Z représente chloro, bromo ou iodo, ou
d'un composé de formule générale (1-6) ou d'un sel de celui-ci :

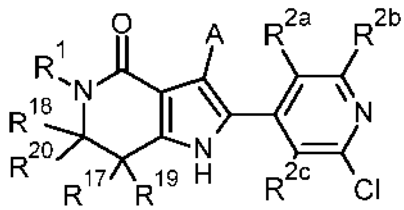

**1-6**

,

dans lequel $R^1$, $R^{2a}$, $R^{2b}$, $R^{2c}$, $R^{17}$, $R^{18}$, $R^{19}$, $R^{20}$ et A sont tels que définis pour le composé de formule générale (I) selon l'une quelconque des revendications 1 à 4, pour la préparation d'un composé de formule (I) tel que défini selon l'une quelconque des revendications 1 à 5.

**14.** Procédé de préparation d'un composé de formule générale (Ib) selon l'une quelconque des revendications 1 à 5, ledit procédé comprenant la réaction d'un composé intermédiaire de formule générale (Ia) :

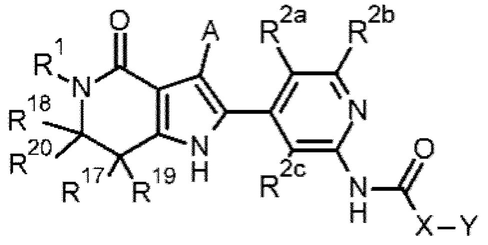

**(Ia)**

,

dans lequel $R^1$, $R^{2a}$, $R^{2b}$, $R^{2c}$, $R^{17}$, $R^{18}$, $R^{19}$, $R^{20}$, A, X et Y sont tels que définis pour le composé de formule générale (I) selon l'une quelconque des revendications 1 à 4, avec le réactif oxydant pour donner un composé de formule générale (Ib) :

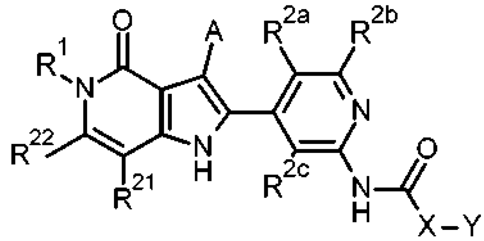

**(Ib)**

,

dans lequel $R^1$, $R^{2a}$, $R^{2b}$, $R^{2c}$, $R^{21}$, $R^{22}$, A, X et Y sont tels que définis pour le composé de formule générale (I) selon l'une quelconque des revendications 1 à 4 ; ou ledit procédé comprenant la réaction d'un composé intermédiaire de formule générale (1-9) :

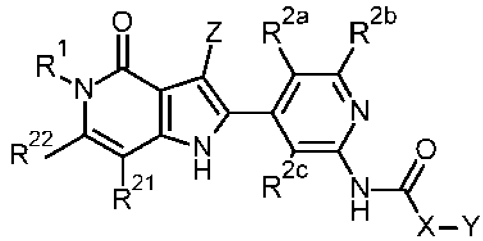

**1-9**

,

dans lequel $R^1$, $R^{2a}$, $R^{2b}$, $R^{2c}$, $R^{21}$, $R^{22}$, X et Y sont tels que définis pour le composé de formule générale (I) selon l'une quelconque des revendications 1 à 4, et Z représente chloro, bromo ou iodo avec un réactif F :

$Z^2$-A **réactif F**

dans lequel A est tel que défini pour le composé de formule générale (I) selon l'une quelconque des revendications 1 à 4 et $Z^2$ représente un stannane tel que A-Sn(nBu)$_3$, acide boronique A-B(OH)$_2$ ou ester boronique

de celui-ci,
utilisation d'une réaction catalysée par métal, telle que, par exemple, une réaction de Suzuki pour donner un composé de formule générale (Ib) :

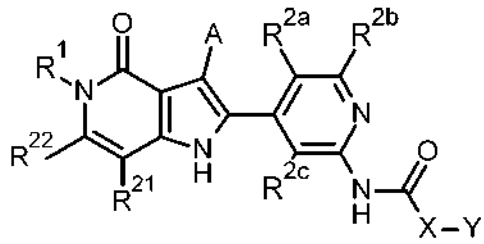

**(Ib)**

,

dans lequel $R^1$, $R^{2a}$, $R^{2b}$, $R^{2c}$, $R^{21}$, $R^{22}$, A, X, et Y sont tels que définis pour le composé de formule générale (I) selon l'une quelconque des revendications 1 à 4.

**15.** Procédé de préparation d'un composé de formule générale (Ia) selon l'une quelconque des revendications 1 à 5, ledit procédé comprenant la réaction d'un composé intermédiaire de formule générale (1-8) :

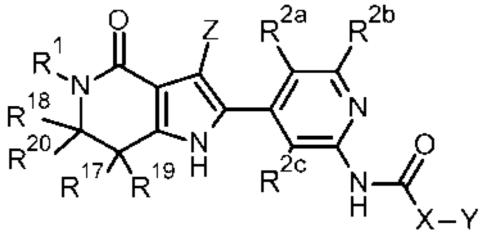

**1-8**

,

dans lequel $R^1$, $R^{2a}$, $R^{2b}$, $R^{2c}$, $R^{17}$, $R^{18}$, $R^{19}$, $R^{20}$, X, et Y sont tels que définis pour le composé de formule générale (I) selon l'une quelconque des revendications 1 à 4 et Z représente chloro, bromo ou iodo, avec un réactif F :

$Z^2$-A **réactif F**

dans lequel A est tel que défini pour le composé de formule générale (1) selon l'une quelconque des revendications 1 à 4 et $Z^2$ représente un stannane tel que A-Sn(nBu)$_3$, acide boronique A-B(OH)$_2$ ou ester boronique de celui-ci
utilisation d'une réaction catalysée par métal telle que, par exemple, une réaction de Suzuki pour donner un composé de formule générale (Ia) :

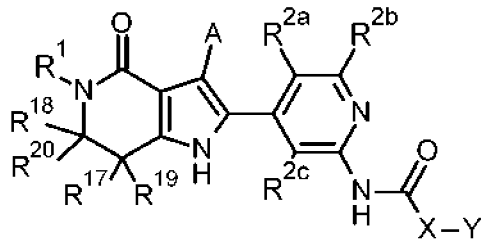

**(Ia)**

.

dans lequel $R^1$, $R^{2a}$, $R^{2b}$, $R^{2c}$, $R^{17}$, $R^{18}$, $R^{19}$, $R^{20}$, A, X et Y sont tels que définis pour le composé de formule générale (I) selon l'une quelconque des revendications 1 à 4, ou ledit procédé comprenant la réaction d'un

composé intermédiaire de formule générale (1-6) :

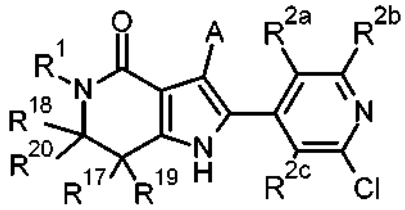

**1-6**

,

dans lequel $R^1$, $R^{2a}$, $R^{2b}$, $R^{2c}$, $R^{17}$, $R^{18}$, $R^{19}$, $R^{20}$ et A sont tels que définis pour le composé de formule générale (I) selon l'une quelconque des revendications 1 à 4 avec un réactif G :

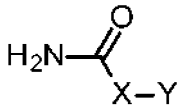

**Réactif G**

dans lequel X et Y sont tels que définis pour le composé de formule générale (I) selon l'une quelconque des revendications 1 à 4 en utilisant une réaction catalysée par métal, telle que, par exemple, une réaction catalysée par palladium en présence d'un ligand, tel que, par exemple, Xanthphos ou X-Phos, pour donner un composé de formule générale (Ia) :

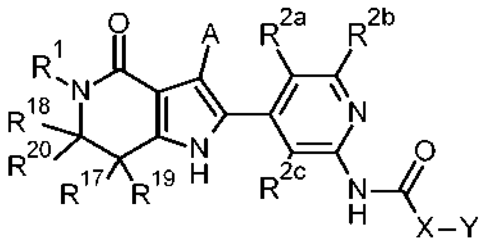

**(Ia)**

,

dans lequel $R^1$, $R^{2a}$, $R^{2b}$, $R^{2c}$, $R^{17}$, $R^{18}$, $R^{19}$, $R^{20}$, A, X et Y sont tels que définis pour le composé de formule générale (I) selon l'une quelconque des revendications 1 à 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016120196 A
- WO 2016100166 A
- WO 2014149164 A
- WO 9920624 A
- US 3966781 A, J. G. Atkinson
- WO 2012112363 A, K. Kassahun
- WO 201472220 A1, Menichincheri
- WO 201422752 A1, Cee
- WO 200739740 A2
- WO 200940399 A1, Marchionni
- WO 201362344 A1, Kim
- WO 201522073 A1, Voss
- US 5011472 A
- US 20190322673 A1, Lauffer


### Non-patent literature cited in the description


- **HANAHAN D ; WEINBERG RA.** *Cell,* 2000, vol. 100, 57
- **HANAHAN D ; WEINBERG RA.** *Cell,* 2011, vol. 144, 646
- **KNIPPSCHILD.** *Onkologie,* 2005, vol. 28, 508-514
- **KNIPPSCHILD et al.** *Front Oncol.,* 19 May 2014, vol. 4, 96
- **SCHITTEK ; SINNBERG.** *Molecular Cancer,* 2014, vol. 13, 231
- **HUTCHINSON et al.** *JBC,* 2011, vol. 286 (10), 8688
- **JARAS et al.** *J. Exp. Med.,* 2014, vol. 211 (4), 605
- **BIDERE.** *Nature,* 05 March 2009, vol. 458
- Isotopic Compositions of the Elements 1997. *Pure Appl. Chem.,* 1998, vol. 70 (1), 217-235
- **H. J. LEIS et al.** *Curr. Org. Chem.,* 1998, vol. 2, 131
- **ESAKI et al.** *Tetrahedron,* 2006, vol. 62, 10954
- **ESAKI et al.** *Chem. Eur. J.,* 2007, vol. 13, 4052
- **J. R. MORANDI et al.** *J. Org. Chem.,* 1969, vol. 34 (6), 1889
- **N. H. KHAN.** *J. Am. Chem. Soc.,* 1952, vol. 74 (12), 3018
- **S. CHANDRASEKHAR et al.** *Tetrahedron,* 2011, vol. 52, 3865
- **HANZLIK et al.** *J. Org. Chem.,* 1990, vol. 55, 3992-3997
- **R. P. HANZLIK et al.** *Biochem. Biophys. Res. Commun.,* 1989, vol. 160, 844
- **P. J. REIDER et al.** *J. Org. Chem.,* 1987, vol. 52, 3326-3334
- **M. JARMAN et al.** *Carcinogenesis,* 1993, vol. 16 (4), 683-688
- **J. ATZRODT et al.** *Angew. Chem., Int. Ed.,* 2007, vol. 46, 7744
- **K. MATOISHI et al.** *J. Chem. Soc, Chem. Commun.,* 2000, 1519-1520
- **A. STREITWIESER et al.** *J. Am. Chem. Soc.,* 1963, vol. 85, 2759
- **C. L. PERRIN et al.** *J. Am. Chem. Soc.,* 2007, vol. 129, 4490
- **C. L. PERRIN et al.** *J. Am. Chem. Soc.,* 2003, vol. 125, 15008
- **C. L. PERRIN.** *Advances in Physical Organic Chemistry,* vol. 44, 144
- **C. L. PERRIN et al.** *J. Am. Chem. Soc.,* 2005, vol. 127, 9641
- **B. TESTA et al.** *Int. J. Pharm.,* 1984, vol. 19 (3), 271
- **D. J. KUSHNER et al.** *Can. J. Physiol. Pharmacol.,* 1999, vol. 77, 79
- **A. E. MUTLIB et al.** *Toxicol. Appl. Pharmacol.,* 2000, vol. 169, 102
- **A. M. SHARMA et al.** *Chem. Res.Toxicol.,* 2013, vol. 26, 410
- **UETRECHT et al.** *Chemical Research in Toxicology,* 2008, vol. 21 (9), 1862
- **A. J. MORALES et al.** Abstract 285. *The 15th North American Meeting of the International Society of Xenobiotics, San Diego, CA,* 12 October 2008
- **C. J. WENTHUR et al.** *J. Med. Chem.,* 2013, vol. 56, 5208
- **F. SCHNEIDER et al.** *Arzneim. Forsch. Drug. Res.,* 2006, vol. 56, 295
- **F. MALTAIS et al.** *J. Med. Chem.,* 2009, vol. 52, 7993
- *Pure Appl Chem,* 1976, vol. 45, 11-30
- **S. M. BERGE et al.** Pharmaceutical Salts. *J. Pharm. Sci.,* 1977, vol. 66, 1-19

- **SMITH et al.** *Bioorg. Med. Chem. Lett.,* 2007, vol. 17, 673-678
- **ANDERSON et al.** *Journal of Medicinal Chemistry,* 2007, vol. 50 (11), 2647-2654
- **T.W. GREENE ; P.G.M. WUTTS.** Protective Groups in Organic Synthesis. Wiley, 1999
- **S. MIWATASHI et al.** *J. Med. Chem.,* 2005, vol. 48, 5966-5979
- **J. ZHAO et al.** *Bioorg. Med. Chem. Lett.,* 2014, vol. 24, 2802-2806
- **M. P. HAY et al.** *J. Med. Chem.,* 2010, vol. 53, 787-797
- **J. M. KEITH et al.** *Med. Chem. Lett,* 2012, vol. 3, 823-827
- **J. LIANG et al.** *Eur. J. Med. Chem.,* 2013, vol. 67, 175-187
- **T. W. GREENE.** Protective Groups in Organic Synthesis. John Wiley & Sons, 1999
- **P. KOCIENSKI.** Protecting Groups. Thieme Medical Publishers, 2000
- **AIELLO et al.** *New Engl. J. Med.,* 1994, vol. 331, 1480
- **PEER et al.** *Lab. Invest.,* 1995, vol. 72, 638
- **LOPEZ et al.** *Invest. Opththalmol. Vis. Sci.,* 1996, vol. 37, 855
- **POWELL, M.F. et al.** Compendium of Excipients for Parenteral Formulations. *PDA Journal of Pharmaceutical Science & Technology,* 1998, vol. 52 (5), 238-311
- **STRICKLEY, R.G.** Parenteral Formulations of Small Molecule Therapeutics Marketed in the United States (1999)-Part-1. *PDA Journal of Pharmaceutical Science & Technology,* 1999, vol. 53 (6), 324-349
- **NEMA, S. et al.** Excipients and Their Use in Injectable Products. *PDA Journal of Pharmaceutical Science & Technology,* 1997, vol. 51 (4), 166-171
- **GOODMAN ; GILMAN'S et al.** The Pharmacological Basis of Therapeutics. McGraw-Hill, 1996, 1225-1287
- *CHEMICAL ABSTRACTS,* 1160861-53-9
- *CHEMICAL ABSTRACTS,* 1536473-72-9
- *CHEMICAL ABSTRACTS,* 564483-18-7
- *CHEMICAL ABSTRACTS,* 118263-97-1
- *CHEMICAL ABSTRACTS,* 5468-37-1
- *CHEMICAL ABSTRACTS,* 128-08-5
- *CHEMICAL ABSTRACTS,* 1445085-55-1
- *CHEMICAL ABSTRACTS,* 51746-82-8
- *CHEMICAL ABSTRACTS,* 1195995-72-2
- *CHEMICAL ABSTRACTS,* 105-56-6
- *CHEMICAL ABSTRACTS,* 6226-25-1
- *CHEMICAL ABSTRACTS,* 456-00-8
- *CHEMICAL ABSTRACTS,* 115497-23-9
- *CHEMICAL ABSTRACTS,* 100-46-9
- *CHEMICAL ABSTRACTS,* 609-14-3
- *CHEMICAL ABSTRACTS,* 36239-09-5
- *CHEMICAL ABSTRACTS,* 141-52-6
- *CHEMICAL ABSTRACTS,* 118263-81-3
- *CHEMICAL ABSTRACTS,* 27741-65-7
- *CHEMICAL ABSTRACTS,* 7051-34-5
- *CHEMICAL ABSTRACTS,* 37517-81-0
- *CHEMICAL ABSTRACTS,* 786719-60-6
- *CHEMICAL ABSTRACTS,* 50607-30-2
- *CHEMICAL ABSTRACTS,* 5239-39-4
- *CHEMICAL ABSTRACTS,* 93102-97-7
- *CHEMICAL ABSTRACTS,* 93102-96-6
- *CHEMICAL ABSTRACTS,* 70785-83-0
- *CHEMICAL ABSTRACTS,* 5467-71-0
- *CHEMICAL ABSTRACTS,* 5467-70-9
- *CHEMICAL ABSTRACTS,* 23794-16-3
- *CHEMICAL ABSTRACTS,* 1501330-86-4
- *CHEMICAL ABSTRACTS,* 148893-10-1
- *CHEMICAL ABSTRACTS,* 22744-12-3
- *CHEMICAL ABSTRACTS,* 57260-71-6
- *CHEMICAL ABSTRACTS,* 23794-15-2
- *CHEMICAL ABSTRACTS,* 332-29-6
- *CHEMICAL ABSTRACTS,* 74427-22-8
- *CHEMICAL ABSTRACTS,* 97518-80-4
- *CHEMICAL ABSTRACTS,* 74-89-5
- *CHEMICAL ABSTRACTS,* 25895-60-7
- *CHEMICAL ABSTRACTS,* 84249-14-9
- *CHEMICAL ABSTRACTS,* 108-24-7
- *CHEMICAL ABSTRACTS,* 97674-02-7
- *CHEMICAL ABSTRACTS,* 13965-03-2
- *CHEMICAL ABSTRACTS,* 631-61-8
- *CHEMICAL ABSTRACTS,* 516-12-1

- *CHEMICAL ABSTRACTS,* 84-58-2
- *CHEMICAL ABSTRACTS,* 98-80-6
- *CHEMICAL ABSTRACTS,* 584-08-7
- *CHEMICAL ABSTRACTS,* 72287-26-4
- *CHEMICAL ABSTRACTS,* 1310-73-2
- *CHEMICAL ABSTRACTS,* 1765-93-1
- *CHEMICAL ABSTRACTS,* 17997-47-6
- *CHEMICAL ABSTRACTS,* 14221-01-3
- *CHEMICAL ABSTRACTS,* 7447-41-8
- *CHEMICAL ABSTRACTS,* 534-17-8
- *CHEMICAL ABSTRACTS,* 7646-69-7
- *CHEMICAL ABSTRACTS,* 405-50-5
- *CHEMICAL ABSTRACTS,* 128625-52-5
- *CHEMICAL ABSTRACTS,* 1538957-14-0
- *CHEMICAL ABSTRACTS,* 75908-73-5
- *CHEMICAL ABSTRACTS,* 128-09-6
- *CHEMICAL ABSTRACTS,* 1075198-30-9
- *CHEMICAL ABSTRACTS,* 192863-37-9
- *CHEMICAL ABSTRACTS,* 38256-93-8
- *CHEMICAL ABSTRACTS,* 109-85-3
- *CHEMICAL ABSTRACTS,* 68957-94-8
- *CHEMICAL ABSTRACTS,* 7664-41-7